# EUROPEAN PATENT APPLICATION

(11) **EP 2 520 661 A1**
(43) Date of publication of application: **07.11.2012**
(21) Application number: 11164471.2
(22) Date of filing: 02.05.2011
(51) Int. Cl.: C12Q 1/68

(54) **Blood-based gene expression signatures in lung cancer**

(71) Applicant: Rheinische Friedrich-Wilhelms-Universität Bonn, 53113 Bonn (DE); UNIVERSITÄT ZU KÖLN, 50923 Köln (DE)
(72) Inventor: Hofmann, Andrea, 53111 Bonn (DE); Schultze, Joachim L., Prof. Dr., 53639 Königswinter (DE); Staratschek-Jox, Andrea, Dr., 50733 Köln (DE); Wolf, Jürgen, Prof. Dr., 50733 Köln (DE); Zander, Thomas, Dr., 53115 Bonn (DE)
(74) Representative: Hoppe, Georg Johannes

(57) **Abstract**

The invention pertains to a method for diagnosing or detecting lung cancer in human subjects based on ribonucleic acid (RNA), in particular based on RNA from blood.

## Description

### Introduction

Lung cancer is still the leading cause of cancer related death worldwide. Prognosis has remained poor with a disastrous 2 year survival rate of only about 15 % due to diagnosis of the disease in late, i.e. incurable stages in the majority of patients (Jemal A, Siegel R, Ward E, et al. Cancer statistics, 2008. CA Cancer J Clin 2008;58: 71-96) and still disappointing therapeutic regimens in advanced disease (Sandler A, Gray R, Perry MC, et al. Paclitaxel-carboplatin alone or with bevacizumab for non-small-cell lung cancer. N Engl J Med 2006;355: 2542-50). Today, the only way to detect lung cancer is by means of imaging technologies detecting morphological changes in the lung in combination with biopsy specimens taken for histological examination. However, these screening approaches are not easily applied to secondary prevention of lung cancer in an asymptomatic population (Henschke CI, Yankelevitz DF, Libby DM, Pasmantier MW, Smith JP, Miettinen OS. Survival of patients with stage I lung cancer detected on CT screening. N Engl J Med 2006;355: 1763-71).
Thus, there is an urgent need in the art to establish reliable tools for the identification of lung cancer patients at early stages of the disease e.g. prior to the development of clinical symptoms.

### Brief description of the invention

The inventors have surprisingly found means to satisfy this need. Accordingly, the present invention provides methods and kits for diagnosing, detecting, and screening for lung cancer. Particularly, the invention provides for preparing RNA expression profiles of patient blood samples, the RNA expression profiles being indicative of the presence or absence of lung cancer. The invention further provides for evaluating the patient RNA expression profiles for the presence or absence of one or more RNA expression signatures that are indicative of lung cancer.

In one aspect, the invention provides a method for preparing RNA expression profiles that are indicative of the presence or absence of lung cancer. The RNA expression profiles are prepared from patient blood samples. The number of transcripts in the RNA expression profile may be selected so as to offer a convenient and cost effective means for screening samples for the presence or absence of lung cancer with high sensitivity and high specificity. Generally, the RNA expression profile includes the expression level or "abundance" of from 4 to about 3000 transcripts. In certain embodiments, the expression profile includes the RNA levels of 2500 transcripts or less, 2000 transcripts or less, 1500 transcripts or less, 1000 transcripts or less, 500 transcripts or less, 250 transcripts or less, 100 transcripts of less, or 50 transcripts or less.

In such embodiments, the profile may contain the abundance or expression level of at least 4 RNAs that are indicative of the presence or absence of lung cancer, and specifically, as selected from Table 3, or may contain the expression level of at least 9, at least 10, at least 13 or at least 29 RNAs selected from Table 3. Where larger profiles are desired, the profile may contain the expression level or abundance of at least about 60, at least 100, at least 157, or 161 RNAs that are indicative of the presence or absence of lung cancer, and such RNAs may be selected from Table 3. The identities and/or combinations of genes and/or transcripts that make up or are included in expression profiles are disclosed in Tables 3 and 5.

Such RNA expression profiles in accordance with this aspect may be evaluated for the presence or absence of an RNA expression signature indicative of lung cancer. Generally, the sequential addition of transcripts from Table 3 to the expression profile provides for higher sensitivity and/or specificity for the detection of lung cancer. For example, the area under the ROC curve (AUC) may be at least at least 0.8, or at least 0.82, or at least 0.85 or at least 0.9. The AUC is a quantitative parameter for the clinical utility (specificity and sensitivity) of the detection method described herein. An AUC of 1.0 refers to a sensitivity and specificity of 100 %.

In a second aspect, the invention provides a method for detecting, diagnosing, or screening for lung cancer. In this aspect, the method comprises preparing an RNA expression profile by measuring the abundance of at least 4, at least 9, at least 10, or at least 13, or at least 29 RNAs in a patient blood sample, where the abundance of such RNAs are indicative of the presence or absence of lung cancer. The RNAs may be selected from the RNAs listed in Table 3, and exemplary sets of such RNAs are disclosed in Tables 3 to 5. The method further comprises evaluating the profile for the presence or absence of an RNA expression signature indicative of lung cancer, to thereby conclude whether the patient has or does not have lung cancer. The method generally provides a sensitivity for the detection of lung cancer of at least about 70 %, while providing a specificity of at least about 70 %.

In various embodiments, the method comprises determining the abundance of at least 4 RNAs, at least 60 RNAs, at least 100 RNAs, at least 157, or of at least 161 RNAs chosen from the RNAs listed in Table 3, and as exemplified in Tables 3 and 6, and classifying the sample as being indicative of lung cancer, or not being indicative of lung cancer.

In other aspects, the invention provides kits and custom arrays for preparing the gene expression profiles, and for determining the presence or absence of lung cancer.

### Detailed description of the invention

The invention provides methods and kits for screening, diagnosing, and detecting lung cancer in human patients (subjects). "Lung cancer" (LC) refers to both non-small cell lung cancer (NSCSC) and small cell lung cancer (SCSC).

Lung cancer is composed of two major different histologies: non-small cell lung cancer and small cell lung cancer. Within the group of non-small cell lung cancer, three main histological subgroups are described: adenocarcinoma, squamous cell carcinoma and large cell carcinoma. All subtypes are described in the WHO classification of 2004 (Travis et al., 2004). Lung cancer clinically presents in different stages that are defined by UICC. (Goldstraw, Peter; Crowley, John; Chansky, Kari; Giroux, Dorothy J; Groome, Patti A; Rami-Porta, Ramon; Postmus, Pieter E; Rusch, Valerie; Sobin, Leslie MD; on behalf of the International Association for the Study of Lung Cancer International staging committee and participating institutions (2007); The IASLC Lung Cancer Staging Project: proposals for the revision of the TNM stage groupings in the forthcoming (seventh) edition of the TNM Classification of Malignant Tumours. Journal of Thoracic Oncology 2(8):706-714).

A synonym for a patient with lung cancer is "LC-case" or simply "case."

As disclosed herein, the present invention provides methods and kits for screening patient samples for those that are positive for LC, e.g., in the absence of surgery or any other diagnostic procedure.

The invention relates to the determination of the abundance of RNAs to detect a lung cancer in a human subject, wherein the determination of the abundance is based on RNA obtained (or isolated) from whole blood of the subject or from blood cells of the subject.

In various aspects, the invention involves preparing an RNA expression profile from a patient sample. The method may comprise isolating RNA from whole blood, and detecting the abundance or relative abundance of selected transcripts. The "RNAs" may be defined by reference to an expressed gene, or by reference to a transcript, or by reference to a particular oligonucleotide probe for detecting the RNA (or cDNA derived therefrom), each of which is listed in Table 3 for 161 RNAs that are indicative of the presence or absence of lung cancer.

The number of transcripts in the RNA expression profile may be selected so as to offer a convenient and cost effective means for screening samples for the presence or absence of lung cancer with high sensitivity and high specificity. For example, the RNA expression profile may include the expression level or "abundance" of from 4 to about 3000 transcripts. In certain embodiments, the expression profile includes the RNA levels of 2500 transcripts or less, 2000 transcripts or less, 1500 transcripts or less, 1000 transcripts or less, 500 transcripts or less, 250 transcripts or less, 200 transcripts of less, 100 transcripts of less, or 50 transcripts or less. Such profiles may be prepared, for example, using custom microarrays or multiplex gene expression assays as described in detail herein.

Such RNA expression profiles in accordance with this aspect may be evaluated for the presence or absence of an RNA expression signature indicative of lung cancer. Generally, the sequential addition of transcripts from Table 3 to the expression profile provides for higher sensitivity and/or specificity for the detection of lung cancer, as indicated by the AUC. A clinical utility is reached if the AUC is at least 0.8.

The inventors have surprisingly found that an AUC of 0.8 is reached if and only if at least 4 RNAs are measured that are chosen from the RNAs listed in table 3. In other words, measuring 4 RNAs is necessary and sufficient for the detection of lung cancer in a human subject based on RNA from a blood sample obtained from said subject by measuring the abundance of at least 4 RNAs in the sample, that are chosen from the RNAs listed in table 3, and concluding based on the measured abundance whether the subject has lung cancer or not. An analysis of 1, 2 or 3 RNAs chosen from the RNAs listed in table 3, however, does not allow for this detection.

For example, the area under the ROC curve (AUC) may be at least 0.8, or at least 0.82, or at least 0.85 or at least 0.9. The AUC is a quantitative parameter for the clinical utility (specificity and sensitivity) of the detection method described herein. An AUC of 1.0 refers to a sensitivity and specificity of 100 %.

In such embodiments, the profile may contain the expression level of at least 4 RNAs that are indicative of the presence or absence of lung cancer, and specifically, as selected from table 3, or may contain the expression level of at least 9, 10, 13 or 29 RNAs selected from table 3. Where larger profiles are desired, the profile may contain the expression level or abundance of at least 60, 100, 200, 500, 1000 RNAs, or 2000 RNAs that are indicative of the presence or absence of lung cancer, and such RNAs may be (at lest in part) selected from table 3. Such RNAs may be defined by gene, or by transcript ID, or by probe ID.

The identities of genes and/or transcripts that make up, or are included in exemplary expression profiles are disclosed in Tables 3 and 5. As shown herein, profiles selected from the RNAs of table 3 support the detection of lung cancer with high sensitivity and high specificity. Exemplary selections of RNAs for the RNA expression profile are shown in table 6.

Thus, in various embodiments, the abundance of at least 4, at least 9, at least 29, at least 60, at least 100, at least 157, or at least 161 distinct RNAs are measured, in order to arrive at a reliable diagnosis of lung cancer. The set of RNAs may comprise, consist essentially of, or consist of, a set or subset of RNAs exemplified in any one of Tables 3 and 5. The term "consists essentially of" in this context allows for the expression level of additional transcripts to be determined that are not differentially expressed in lung cancer subjects, and which may therefore be used as positive or negative expression level controls or for normalization of expression levels between samples.

Such RNA expression profiles may be evaluated for the presence or absence of an RNA expression signature indicative of lung cancer. Generally, the sequential addition of transcripts from Table 3 to the expression profile provides for higher sensitivity and/or specificity and stability (i.e. independence from the sample analyzed) for the detection of lung cancer. For example, the sensitivity and specificity of the methods provided herein may be equivalent to an area under the ROC curve (AUC) of at least at least 0.8, or at least 0.82, at least 0.85, or of at least 0.9.

The present invention provides an in-vitro diagnostic test system (IVD) that is trained (as described further below) for the detection of a lung cancer. For example, in order to determine whether a patient has lung cancer, reference RNA abundance values for lung cancer positive and negative samples are determined. The RNAs can be quantitatively measured on an adequate set of training samples comprising cases and controls, and with adequate clinical information on carcinoma status, applying adequate quality control measures, and on an adequate set of test samples, for which the detection is yet to be made. With such quantitative values for the RNAs and the clinical data for the training samples, a classifier can be trained and applied to the test samples to calculate the probability of the presence or non-presence of the lung carcinoma. Therefore, in one embodiment of the present method, a sample can be classified as being from a patient with lung cancer or from a healthy individual without the necessity to run a reference sample of known origin (i.e. from a lung cancer patient or a healthy individual) at the same time.

Various classification schemes are known for classifying samples between two or more classes or groups, and these include, without limitation: Naive Bayes, Support Vector Machines, Nearest Neighbors, Decision Trees, Logistics, Articifial Beural Networks, and Rule-based schemes. In addition, the predictions from multiple models can be combined to generate an overall prediction. Thus, a classification algorithm or "class predictor" may be constructed to classify samples. The process for preparing a suitable class predictor is reviewed in R. Simon, Diagnostic and prognostic prediction using gene expression profiles in high-dimensional microarray data, British Journal of Cancer (2003) 89, 1599-1604, which review is hereby incorported by reference.

In this context, the invention teaches an in-vitro diagnostic test system (IVD) that is trained in the detection of a lung cancer referred to above, comprising at least 4 RNAs, which can be quantitatively measured on an adequate set of training samples comprising cases and controls, with adequate clinical information on carcinoma status, applying adequate quality control measures, and on an adequate set of test samples, for which the detection yet has to be made. Given the quantitative values for the RNAs and the clinical data for the training samples, a classifier can be trained and applied to the test samples to calculate the probability of the presence or absence of the lung carcinoma.

The present invention provides methods for detecting, diagnosing, or screening for lung cancer in a human subject with a high sensitivity and specificity. Specifically, the sensitivity of the methods provided herein is equivalent to an area under the ROC curve (AUC) of at least at least 0.8, or at least 0.82, at least 0.85,of or at least 0.9.

Without wishing to be bound by any particular theory, the above finding may be due to the fact that an organism such as a human systemically reacts to the development of a lung tumor by altering the expression levels of genes in different pathways. Although the change in expression (abundance) might be small for each gene in a particular signature, measuring a set of at least 4 genes, preferably even larger numbers such as 9, 10, 13, 29, 100, 157, 161 or even more RNAs, for example at least 5, at least 8, at least 120, at least 160 RNAs at the same time allows for the detection of lung cancer in a human with high sensitivity and high specificity.

In this context, an RNA obtained from a subject's blood sample, i.e. an RNA biomarker, is an RNA molecule with a particular base sequence whose presence within a blood sample from a human subject can be quantitatively measured. The measurement can be based on a part of the RNA molecule, namely a part of the RNA molecule that has a certain base sequence, which allows for its detection and thereby allows for the measurement of its abundance in a sample. The measurement can be by methods known in the art, for example analysis on a solid phase device, or in solution (for example, by RT-PCR). Probes for the particular RNAs can either be bought commercially, or designed based on the respective RNA sequence.

In the method of the invention, the abundance of several RNA molecules (e.g. mRNA or prespliced RNA, intron-lariat RNA, micro RNA, small nuclear RNA, or fragments thereof) is determined in a relative or an absolute manner, wherein an absolute measurement of RNA abundance is preferred. The RNA abundance is, if applicable, compared with that of other individuals, or with multivariate quantitative thresholds.

The determination of the abundance of the RNAs described herein is performed from blood samples using quantitative methods. In particular, RNA is isolated from a blood sample obtained from a human subject that is to undergo lung cancer testing, i.e. a smoker. Although the examples described herein use microarray-based methods, the invention is not limited thereto. For example, RNA abundance can be measured by in situ hybridization, amplification assays such as the polymerase chain reaction (PCR), sequencing, or microarray-based methods. Other methods that can be used include polymerase-based assays, such as RT-PCR (e.g., TAQMAN), hybridization-based assays, such as DNA microarray analysis, as well as direct mRNA capture with branched DNA (QUANTIGENE) or HYBRID CAPTURE (DIGENE).

In certain embodiments, the invention employs a microarray. A "micoroarray" includes a specific set of probes, such as oligonucleotides and/or cDNAs (*e.g.,* expressed sequence tags, "ESTs") corresponding in whole or in part, and/or continuously or discontinuously, to regions of RNAs that can be extracted from a blood sample of a human subject. The probes are bound to a solid support. The support may be selected from beads (magnetic, paramagnetic, etc.), glass slides, and silicon wafers. The probes can correspond in sequence to the RNAs of the invention such that hybridization between the RNA from the subject sample (or cDNA derived therefrom) and the probe occurs. In the microarray embodiments, the sample RNA can optionally be amplified before hybridization to the microarray. Prior to hybridization, the sample RNA is fluorescently labeled. Upon hybridization to the array and excitation at the appropriate wavelength, fluorescence emission is quantified. Fluorescence emission for each particular RNA, is directly correlated with the amount of the particular RNA in the sample. The signal can be detected and together with its location on the support can be used to determine which probe hybridized with RNA from the subject's blood sample.

Accordingly, in certain aspects, the invention is directed to a kit or microarray for detecting the level of expression or abundance of RNAs in the subject's blood sample, where this "profile" allows for the conclusion of whether the subject has lung cancer or not (at a level of accuracy described herein). In another aspect, the invention relates to a probe set that allows for the detection of the RNAs associated with LC. If these particular RNAs are present in a sample, they (or corresponding cDNA) will hybridize with their respective probe (i.e, a complementary nucleic acid sequence), which will yield a detectable signal. Probes are designed to minimize cross reactivity and false positives.

Thus, the invention in certain aspects provides a microarray, which generally comprises a solid support and a set of oligonucleotide probes. The set of probes generally contains from 4 to about 3,000 probes, including at least 4 probes deduced from Table 3 or 5. In certain embodiments, the set contains 2000 probes or less, or 1000 probes or less, 500 probes or less, 200 probes or less, or 100 probes or less,.

The conclusion whether the subject has lung cancer or not is preferably reached on the basis of a classification algorithm, which can be developed using e.g. a random forest method, a support vector machine (SVM), a K-nearest neighbor method (K-NN), such as a 3-nearest neighbor method (3-NN), a linear discrimination analysis (LDA), or a prediction analysis for microarrays (PAM), as known in the art.

Preferably, F-statistics (ANOVA) is used to identify specific difference of the abundance of the at least 4 RNAs in healthy individuals versus the abundance of the at least 4 RNAs in individuals with lung cancer.

"Sensitivity" (S+ or true positive fraction (TPF)) refers to the count of positive test results among all true positive disease states divided by the count of all true positive disease states. "Specificity" (S- or true negative fraction (TNF)) refers to the count of negative test results among all true negative disease states divided by the count of all true negative disease states. "Correct Classification Rate" (CCR or true fraction (TF)) refers to the sum of the count of positive test results among all true positive disease states and count of negative test results among all true negative disease states divided by all the sum of all cases. The measures S+, S-, and CCR address the question: To what degree does the test reflect the true disease state?

"Positive Predictive Value" (PV+ or PPV) refers to the count of true positive disease states among all positive test results dived by the count of all positive test results. "Negative Predictive Value" (PV- or NPV) refers to the count of true negative disease states among all negative test results dived by the count of all negative test results. The predictive values address the question: How likely is the disease given the test results?

The preferred RNA molecules that can be used in combinations described herein for diagnosing and detecting lung cancer in a subject according to the invention can be found in table 3. The inventors have shown that the selection of at least 4 or more RNAs of the markers listed in table 3 can be used to diagnose or detect lung cancer in a subject using a blood sample from that subject. The RNA molecules that can be used for detecting, screening and diagnosing lung cancer are selected from the RNAs provided in Table 3 or 5.

Specifically, the method of the invention comprises at least the following steps: measuring the abundance of at least 4 RNAs (preferably 9 RNAs or 10 RNAs) in the sample, that are chosen from the RNAs listed in table 3, and concluding, based on the measured abundance, whether the subject has lung cancer or not. Measuring the abundance of RNAs may comprise isolating RNA from blood samples as described, and hybridizing the RNA or cDNA prepared therefrom to a microarray. Alternatively, other methods for determining RNA levels may be employed.

Examples for sets of 4 RNAs that are measured together, i.e. sequentially or preferably simultaneously, are shown in table 6. The sets of at least 4 RNAs of table 6 are defined by a common threshold of AUC>0.8.

In a preferred embodiment of the invention as mentioned herein, the abundance of at least 4 RNAs (preferably 9, 10, or 13 RNAs) in the sample is measured, wherein the at least 4 RNAs are chosen from the RNAs listed in table 3. Examples for sets of 4 RNAs that can be measured together, i.e. sequentially or preferably simultaneously, to detect lung cancer in a human subject are shown in table 6. The sets of RNAs of table 6 (4, 9, 10, 13, 29 RNAs) are defined by a common threshold of AUC>0.8.

Similarly, the abundance of at least 9 RNAs (preferably up to 29 RNAs), of at least 30 RNAs (preferably up to 59 RNAs), of at least 60 RNAs (preferably up to 99 RNAs), of at least 100 RNAs (preferably up to 160 RNAs), of at least 16 RNAs that are chosen from the RNAs listed in table 3 can be measured in the method of the invention.

An example for a set of 161 RNAs of which the abundance can be measured in the method of the invention is listed in table 3.

When the wording "at least a number of RNAs" is used, this refers to a minimum number of RNAs that are measured. It is possible to use up to 10,000 or 20,000 genes in the invention, a fraction of which can be RNAs listed in table 3. In preferred embodiments of the invention, abundance of up to 5.000, 2.500, 2.000, 1,000, 500, 250, 100, 80, 70, 60, 50, 40, 30, 20, 10, 5, 4, 3, 2, or 1 RNA of randomly chosen RNAs that are not listed in table 3 is measured in addition to RNAs of table 3 (or subsets thereof).

In a preferred embodiment, only RNAs that are mentioned in table 3 are measured.

The expression profile or abundance of RNA markers for lung cancer, for example the at least 4 RNAs described above, (or more RNAs as disclosed above and herein), is determined preferably by measuring the quantity of the transcribed RNA of the marker gene. This quantity of the mRNA of the marker gene can be determined for example through chip technology (microarray), (RT-) PCR (for example also on fixated material), Northern hybridization, dot-blotting, sequencing, or in situ hybridization.

The microarray technology, which is most preferred, allows for the simultaneous measurement of RNA abundance of up to many thousand RNAs and is therefore an important tool for determining differential expression (or differences in RNA abundance), in particular between two biological samples or groups of biological samples. In order to apply the microarray technology, the RNAs of the sample need to be amplified and labeled and the hybridization and detection procedure can be performed as known to a person of skill in the art.

As will be understood by those of ordinary skill in the art, the analysis can also be performed through single reverse transcriptase-PCR, competitive PCR, real time PCR, differential display RT-PCR, Northern blot analysis, sequencing, and other related methods. In general, the larger the number of markers is that are to be measured, the more preferred is the use of the microarray technology. However, multiplex PCR, for example, real time multiplex PCR is known in the art and is amenable for use with the present invention, in order to detect the presence of 2 or more genes or RNAs simultaneously.

The RNA whose abundance is measured in the method of the invention can be mRNA, cDNA, unspliced RNA, or its fragments. Measurements can be performed using the complementary DNA (cDNA) or complementary RNA (cRNA), which is produced on the basis of the RNA to be analyzed, e.g. using microarrays. A great number of different arrays as well as their manufacture are known to a person of skill in the art and are described for example in the U.S. Patent Nos. 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,331; 5,554,501; 5,561,071; 5,571,639; 5,593,839; 5,599,695; 5,624,711; 5,658,734; and 5,700,637.

Preferably the decision whether the subject has lung cancer comprises the step of training a classification algorithm on an adequate training set of cases and controls and applying it to RNA abundance data that was experimentally determined based on the blood sample from the human subject to be diagnosed. The classification method can be a random forest method, a support vector machine (SVM), or a K-nearest neighbor method (K-NN), such as 3-NN.

For the development of a model that allows for the classification for a given set of biomarkers, such as RNAs, methods generally known to a person of skill in the art are sufficient, i.e., new algorithms need not be developed.

The major steps of such a model are:
1) condensation of the raw measurement data (for example combining probes of a microarray to probeset data, and/or normalizing measurement data against common controls);
2) training and applying a classifier (i.e. a mathematical model that generalizes properties of the different classes (carcinoma vs. healthy individual) from the training data and applies them to the test data resulting in a classification for each test sample.

For example, the raw data from microarray hybridizations can first be condensed with FARMS as shown by Hochreiter (2006, Bioinformatics 22(8): 943-9). Alternative methods for condensation such as Robust Multi-Array Analysis (RMA, GC-RMA, see Irizarry et al (2003). Exploration, Normalization, and Summaries of High Density Oligonucleotide Array Probe Level Data. Biostatistics. 4, 249-264.) can be used. Similar to condensation, classification of the test data set through a support-vector-machine or other classification algorithms is known to a person of skill in the art, like for example classification and regression trees, penalized logistic regression, sparse linear discriminant analysis, Fisher linear discriminant analysis, K-nearest neighbors, shrunken centroids, and artificial neural networks (see Wladimir Wapnik: The Nature of Statistical Learning Theory, Springer Verlag, New York, NY, USA, 1995; Berhard Schölkopf, Alex Smola: Learning with Kernels: Support Vector Machines, Regularization, Optimization, and Beyond, MIT Press, Cambridge, MA, 2002; S. Kotsiantis, Supervised Machine Learning: A Review of Classification Techniques, Informatica Journal 31 (2007) 249-268).

The key component of these classifier training and classification techniques is the choice of RNA biomarkers that are used as input to the classification algorithm.

In a further aspect, the invention refers to the use of a method as described above and herein for the detection of lung cancer in a human subject, based on RNA from a blood sample.

In a further aspect, the invention also refers to the use of a microarray for the detection of lung cancer in a human subject based on RNA from a blood sample. According to the invention, such a use can comprise measuring the abundance of at least 4 RNAs (or more, as described above and herein) that are listed in table 3. Accordingly, the microarray comprises at least 3 probes for measuring the abundance of the at least 3 RNAs. Commercially available microarrays, such as from Illumina or Affymetrix, may be used.

In another embodiment, the abundance of the at least 4 RNAs is measured by multiplex RT-PCR. In a further embodiment, the RT-PCR includes real time detection, e.g., with fluorescent probes such as Molecular beacons or TaqMan® probes.

In a preferred embodiment, the microarray comprises probes for measuring only RNAs that are listed in table 3 (or subsets thereof).

In yet a further aspect, the invention also refers to a kit for the detection of lung cancer in a human subject based on RNA obtained from a blood sample. Such a kit comprises a means for measuring the abundance of at least 4 RNAs that are chosen from the RNAs listed in table 3.. The means for measuring expression can be probes that allow for the detection of RNA in the sample or primers that allow for the amplification of RNA in the sample. Ways to devise probes and primers for such a kit are known to a person of skill in the art.

Further, the invention refers to the use of a kit as described above and herein for the detection of lung cancer in a human subject based on RNA from a blood sample comprising means for measuring the abundance of at least 4 RNAs that are chosen from the RNAs listed in table 3. Such a use may comprise the following steps: contacting at least one component of the kit with RNA from a blood sample from a human subject, measuring the abundance of at least 4 RNAs (or more as described above and herein) that are chosen from the RNAs listed in table 3 using the means for measuring the abundance of at least 4 RNAs, and concluding, based on the measured abundance, whether the subject has lung cancer.

In yet a further aspect, the invention also refers to a method for preparing an RNA expression profile that is indicative of the presence or absence of lung cancer, comprising: isolating RNA from a whole blood sample, and determining the level or abundance of from 4 to about 3000 RNAs, including at least 4 RNAs selected from Table 3.

Preferably, the expression profile contains the level or abundance of 161 RNAs or less, 157 or less, of 150 RNAs or less, or of 100 RNAs or less. Further, it is preferred that at least 10 RNAs, at least 30 RNAs, at least 100 RNAs are listed in Table 3 or Table 6.

In yet a further aspect, the invention also refers to a microarray, comprising a solid support and a set of oligonucleotide probes, the set containing from 4 to about 3,000 probes, and including at least 4 probes selected from Table 3 or 5. Preferably, the set contains 161 probes or less (such as e.g. 157 probes, or less). At least 10 probes can be those listed in Table 3 or Table 6. At least 30 probes can be those listed in Table 3 or Table 5. In another embodiment, at least 100 probes are listed in Table 3 or Table 6.

Features of the invention that were described herein in combination with a method, a microarray, a kit, or a use also refer, if applicable, to all other aspects of the invention.

### Figures

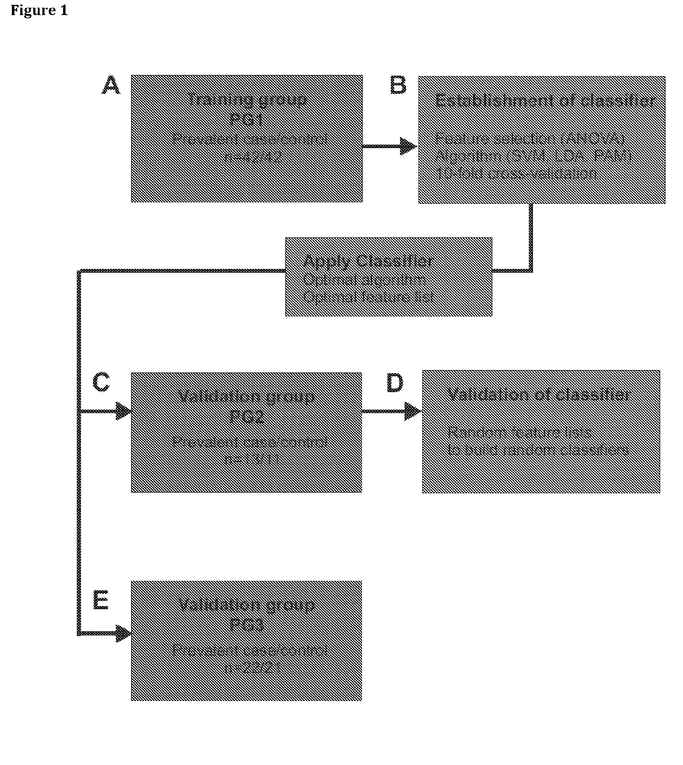

**Fig. 1** shows the experimental design of the study that lead to aspects of the invention. In a training group (**A**) feature selection and classifier detection was performed. Read-out was performed using the area under the curve (AUC) for each cut-off of F-statistics and each algorithm. (**B**) A classifier was applied to the validation groups (PG2, PG3). (**C, E**) A random permutation was performed (n=1000) (**D**) to test specificity.

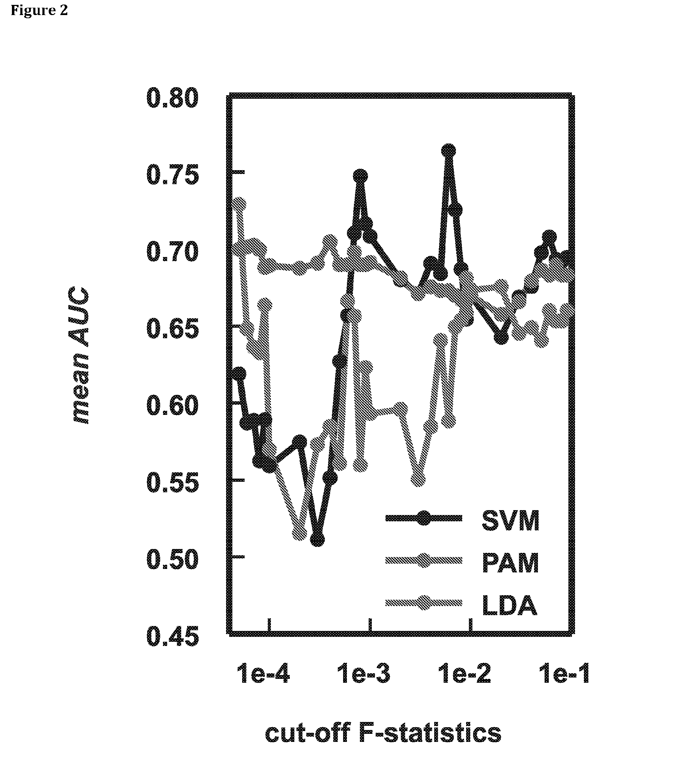

**Fig. 2** shows the mean area under the receiver operator curve (AUC) plotted against the cut-off of the F-statistics for feature selection for all three algorithms (SVM, LDA, PAM) obtained in the 10-fold cross-validation in PG1. An SVM leads to the highest single mean AUC and is therefore preferred. (SVM: dark; LDA: lower curve shown, green; PAM: upper curve shown, red).

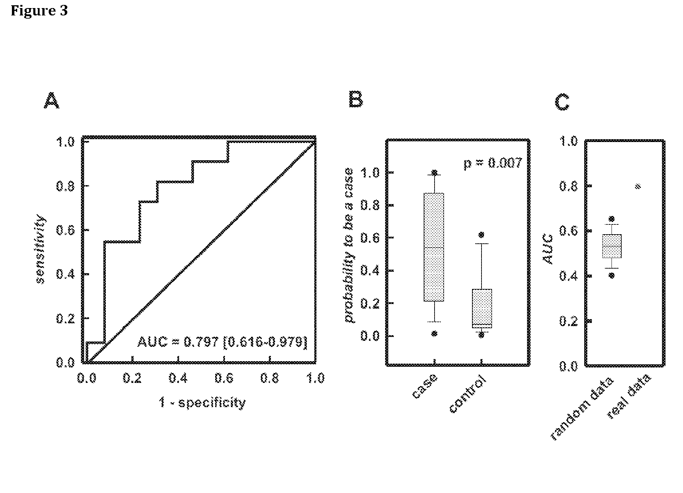

**Fig. 3** shows the classifier for prevalent lung cancer. The AUC and the 95 % confidence interval are given (**A**). Box plots visualizing the SVM probabilities for cases and controls in the validation group (box = 25-75 percentile; whisker = 10-90 percentile; dot = 5-95 percentile) (**B**). The box plot comprises permuted AUCs. The real AUC (real data) is depicted in red (**C**).

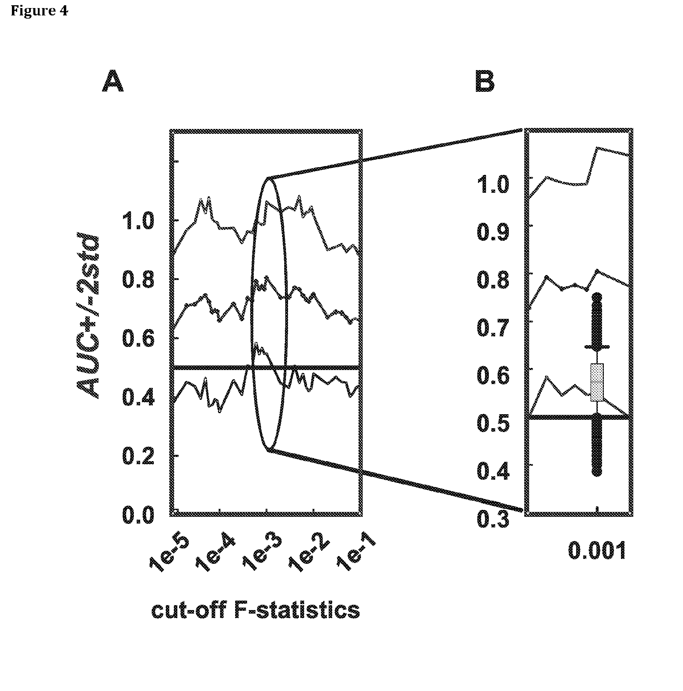

**Fig. 4** shows the mean area under the receiver operator curve (AUC) is plotted against the cut-off of the F-statistics for feature selection in PG1 and PG2. Both prevalent groups (PG1 & PG2) were pooled and a 10-fold cross-validation (9:1 dataset splitting) was performed. The cut-off for the F-statistics for feature selection was continuously increased from 0.00001 - 0.1. For each cross-validation, the AUC for the receiver operator curve was calculated. The mean +/- 2 standard deviation is plotted. For better visualization, a line is drawn at 0.5 (AUC obtained by chance). (**A**) A detailed view in the area of the maximum AUC is shown. An additional box blot visualizes the AUC obtained by random lists at the respective cut-off of the F-statistics (box = 25-75 percentile; whisker = 10-90 percentile; dot = all outliers). (**B**) The overlap in the genes extracted for the respective classifier is depicted (**C**).

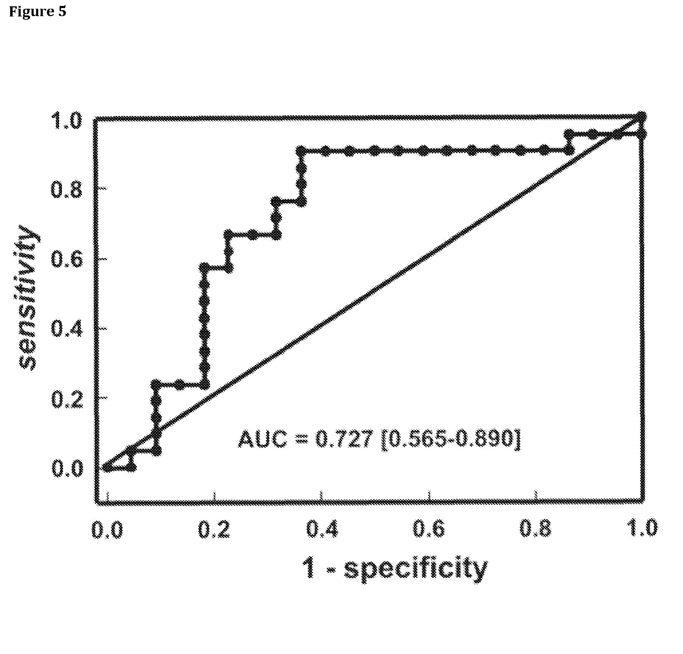

**Fig. 5** shows the receiver operator curve of unmatched cases with prevalent lung cancer (n = 22) and controls (n = 21) (PG3). The false discovery rate (1 - specificity) is plotted against the true discovery rate (sensitivity). The diagonal with an area under the curve of 0.5 is plotted for better visualization. The AUC was calculated with 0.727. At the maximum Youden index, the sensitivity was 0.90 and the specificity 0.64. SVM probabilities for cases and controls were significantly different (Student's T test p = 0.0047). The AUC and the 95 % confidence interval are given.

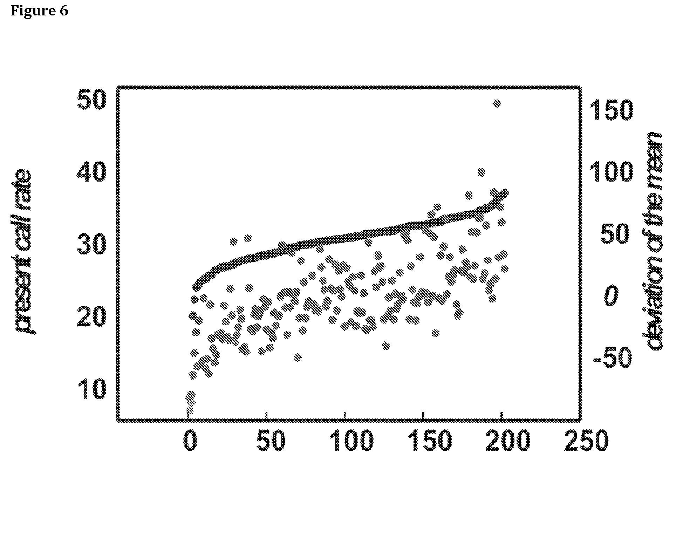

**Fig. 6****:** All samples were ordered by the present call rate. The present call rate (darker; dark blue) for each sample and the respective deviation of the mean from the overall mean (lighter;
dark red) is plotted. Those samples declared of low quality (present call rate = light blue; light;
deviation from the mean = light red; dark) are highlighted.

### Tables

**Table 1: Clinical and epidemiological characteristics of cases with lung cancer and respective controls.**
   Clinical and epidemiological characteristics of cases and controls in the three groups with prevalent lung cancer (PG1, PG2, PG3) are given.
**Table 2: Detailed clinical and epidemiological characteristics of cases with lung cancer and controls recruited for the study**
   Clinical and epidemiological characteristics of all patients are given. Age, gender and pack years of smoked cigarettes are given. For lung cancer cases, the histopathological diagnosis is displayed. Finally, co-morbidity was documented using the ICD-10 code. NA = Not analyzed.
**Table 3: Annotation of features used for the classifiers**
   The feature list used in the classifier is demonstrated: The 161 features selected in the ten-fold cross-validation in PG1 and applied to PG2. In the column up vs. down 1= upregulation in lung cancer patients; -1= downregulation in lung cancer patients. The RNAs listed in this table can be used for the detection of lung cancer according to the invention. Each RNA is identified by SEQ ID NO, gene symbol, gene name, refseq ID, and entrez ID, as used elsewhere in the application.
**Table 4: Annotation of features differentially expressed most robustly**
   31 transcripts demonstrating a stable differential expression over all data-set splitting between cases and controls.
**Table 5: Annotation of features differentially expressed**
   1000 features with differential expression between NSCLC, SCLC and controls.
**Table 6:**
   Table 6 show exemplary sets of RNAs whose abundance in a blood sample from a human individual can be determined according to the invention to detect lung cancer in the individual. Each list shows a set of RNAs (defined by probe set and gene name) with an area under the curve (AUC) of at least 0.8. The AUC is a quantitative parameter for the clinical utility (specificity and sensitivity) of the detection method described herein. An AUC of 1.0 refers to a sensitivity and specificity of 100 %.

**Table 1:**

| | **test group** | | **validation group** | | **validation group** | |
|---|---|---|---|---|---|---|
| | **PG1** | | **PG2** | | **PG3** | |
| | **case** | **control** | **case** | **control** | **case** | **control** |
| total number | 42 | 42 | 13 | 11 | 22 | 21 |
| female | 13 | 14 | 6 | 5 | 7 | 8 |
| male | 29 | 28 | 7 | 6 | 15 | 13 |
| NSCLC | 35 | NA | 11 | NA | 17 | NA |
| SCLC | 7 | NA | 2 | NA | 5 | NA |
| median age (years) | 62 | 61 | 62 | 61 | 63 | 57 |
| stage=I | 5 | NA | 4 | NA | 3 | NA |
| stage>1 | 37 | NA | 9 | NA | 19 | NA |

**Table 2:**

| | | | | | | | | | | |
|---|---|---|---|---|---|---|---|---|---|---|
| Columns from left to right are: GEO_Name V2; groups; case/control [case=1; control=0]; age (years); gender [male=M; female=F]; histology [Adenoc.=AD; squamous cell c. =SQ; large cell c. = LC; small cell c. =SC; small cell lung c. =SCLC; not applicable=NA]; stage [UICC stage 1-4; not applicable=NA]; latency to clinical manifest lung cancer [months; not applicable=NA]; smoking status [current/ever=CU=2; ex-smoker=EX=1; never-smoker=NEV]; packyears; comorbidity ICD-10 | | | | | | | | | | |
| GSM389918 | PG1 | 0 | 63 | F | NA | NA | NA | 1 | 31 | M53.26, M54.4, I10, E03.9 D22.7, 189.0, I10, E66, M35.3, E78.0, |
| GSM389920 | PG1 | 0 | 64 | F | NA | NA | NA | 2 | 27 | Z88.0, J45 M48.06, M54.4, E78.0, M06.0, J45, |
| GSM389922 | PG1 | 0 | 73 | M | NA | NA | NA | 1 | 87 | Z95.0, I49.9 |
| GSM389956 | PG1 | 0 | 70 | M | NA | NA | NA | 1 | 54 | T84.0, Z96.6, I25.1, 110, E87.6 |
| GSM389958 | PG1 | 0 | 45 | M | NA | NA | NA | 1 | 35 | C43.4, E04.9, E11.9, I10 |
| GSM389960 | PG1 | 0 | 62 | F | NA | NA | NA | 1 | 28 | |
| GSM389962 | PG1 | 0 | 71 | F | NA | NA | NA | 1 | 30 | |
| GSM389964 | PG1 | 0 | 57 | F | NA | NA | NA | 1 | 26 | M48.06, M54.4 |
| GSM389966 | PG1 | 0 | 58 | M | NA | NA | NA | 1 | 28 | C44.9, E78.0, I10, I20, M10 M48.06, M54.4, 173.9, E10, 149.9, E79.0, |
| GSM389968 | PG1 | 0 | 73 | M | NA | NA | NA | 1 | 34 | E78, I10 |
| GSM389970 | PG1 | 0 | 58 | F | NA | NA | NA | 1 | 20 | |
| GSM389972 | PG1 | 0 | 68 | M | NA | NA | NA | 2 | 25 | C44.2, 110, L30, M51.2 |
| GSM389973 | PG1 | 0 | 60 | F | NA | NA | NA | 1 | 49 | C43, G43, M79.7, E03.9 |
| GSM389985 | PG1 | 0 | 65 | F | NA | NA | NA | 1 | 22 | M48.06, M54.4, E03.9, F32 |
| GSM389987 | PG1 | 0 | 63 | M | NA | NA | NA | 1 | 54 | |
| GSM389988 | PG1 | 0 | 59 | M | NA | NA | NA | 1 | 73 | D04.3, L57.0 |
| GSM389938 | PG1 | 0 | 71 | M | NA | NA | NA | 1 | 58 | L40, I10, L27.0 |
| GSM389940 | PG1 | 0 | 61 | M | NA | NA | NA | 2 | 62 | M48.02, M50.0+ |
| GSM389942 | PG1 | 0 | 71 | M | NA | NA | NA | 1 | 16 | B86, L02.0, 110, L40 |
| GSM389989 | PG1 | 0 | 68 | M | NA | NA | NA | 1 | 53 | C44.3, E11, E78.0, 110, 173.9 |
| GSM389991 | PG1 | 0 | 59 | M | NA | NA | NA | 2 | 57 | L30.1, B95.6, Z88.0 |
| GSM389975 | PG1 | 0 | 61 | M | NA | NA | NA | 2 | 38 | M16.1, E11, H40 |
| GSM389978 | PG1 | 0 | 63 | M | NA | NA | NA | 1 | 28 | C44.3, E04.0, N40 |
| GSM389979 | PG1 | 0 | 58 | F | NA | NA | NA | 1 | 43 | |
| GSM389924 | PG1 | 0 | 54 | M | NA | NA | NA | 2 | 43 | C43, E78.0, Z86.7, Z92.1 |
| GSM389926 | PG1 | 0 | 47 | F | NA | NA | NA | 2 | 34 | L25, L29 |
| GSM389910 | PG1 | 0 | 66 | M | NA | NA | NA | 1 | 14 | C44.3, I10, E78.0, E79.0, T81.4 |
| GSM389912 | PG1 | 0 | 54 | F | NA | NA | NA | 2 | 26 | M42, M47, M99.3 |
| GSM389914 | PG1 | 0 | 61 | M | NA | NA | NA | 1 | 62 | M16.1, I10, E78.0, E79.0 |
| GSM389929 | PG1 | 0 | 55 | M | NA | NA | NA | 1 | 33 | M16.1, K50, L89 |
| GSM389930 | PG1 | 0 | 60 | M | NA | NA | NA | 1 | 65 | M61.5, F32 |
| GSM389932 | PG1 | 0 | 57 | F | NA | NA | NA | 2 | 62 | M50.2, E11, I10, E66, 149.9, 150 |
| GSM389934 | PG1 | 0 | 63 | M | NA | NA | NA | 1 | 30 | M48.06, M54.4, I10, E79.0 |
| GSM389936 | PG1 | 0 | 53 | M | NA | NA | NA | 1 | 3 | S42.2, Z47.0 |
| GSM389944 | PG1 | 0 | 43 | M | NA | NA | NA | 2 | 28 | |
| GSM389946 | PG1 | 0 | 64 | M | NA | NA | NA | 1 | 54 | M54.4, M99.7, I49.9, N40, E78.0 |
| GSM389948 | PG1 | 0 | 69 | M | NA | NA | NA | 1 | 49 | L20, L29, 110, 150, D50, Z85.0 |
| GSM389950 | PG1 | 0 | 67 | M | NA | NA | NA | 1 | 14 | M17.1, M99.3, G95.1, N40, 135.0, I10 |
| GSM389952 | PG1 | 0 | 57 | M | NA | NA | NA | 1 | 32 | M30.1, I10, J45, N40 |
| GSM389954 | PG1 | 0 | 68 | M | NA | NA | NA | 2 | 36 | S32.0 |
| GSM389915 | PG1 | 0 | 58 | F | NA | NA | NA | 2 | 1 | M16, E03 9 E78 0, H33, N80, D25 |
| GSM389983 | PG1 | 0 | 55 | F | NA | NA | NA | 2 | 21 | M48 06, M54 4 |
| GSM389917 | PG1 | 1 | 65 | F | AD | Ia | 0 | 2 | 33 | I10, Z86 1, Z85 5 |
| GSM389919 | PG1 | 1 | 64 | F | AD | IIb | 0 | 2 | 29 | J44, E78, I70, I10 K70 3, G62 1, K25 -, I10, I50, I73 9, |
| GSM389921 | PG1 | 1 | 70 | M | AD | IIIa | 0 | 1 | 87 | H27 1, H629, J44 |
| GSM389955 | PG1 | 1 | 76 | M | SCLC | IIIa | 0 | 2 | 56 | |
| GSM389957 | PG1 | 1 | 46 | M | AD | IV | 0 | 1 | 34 | Z88 0 |
| GSM389959 | PG1 | 1 | 59 | F | AD | IIIa | 0 | 2 | 28 | E05 8, Y57 9, M03 6, K21 9, K43 9 |
| GSM389961 | PG1 | 1 | 75 | F | SCLC | IV | 0 | 2 | 39 | |
| GSM389963 | PG1 | 1 | 56 | F | AD | IIIa | 0 | 1 | 28 | I10, E03 |
| GSM389965 | PG1 | 1 | 58 | M | AD | IV | 0 | 2 | 30 | F17 2 |
| GSM389967 | PG1 | 1 | 75 | M | AD | IIb | 0 | 1 | 34 | I25 9, I20 9, I10, N40,E79 0 |
| GSM389969 | PG1 | 1 | 61 | F | SCLC | IV | 0 | 1 | 20 | |
| GSM389971 | PG1 | 1 | 71 | M | LC | IIIa | 0 | 1 | 26 | Z85 5, N18 82, I15 1, R53, J96 1 |
| GSM389974 | PG1 | 1 | 60 | F | AD | IIIa | 0 | 2 | 48 | E89 0, E51 |
| GSM389986 | PG1 | 1 | 62 | M | SCLC | IV | 0 | 2 | 79 | |
| GSM389937 | PG1 | | 1 72 M | | AD | Ib | 0 | 1 | 60 | I10, I25 |
| GSM389939 | PG1 | 1 | 61 | M | SQ | IV | 0 | 2 | 60 | I73 9, I10 |
| GSM389941 | PG1 | 1 | 70 | M | SCLC | IIIb | 0 | 1 | 21 | 110, G52 2 |
| GSM389990 | PG1 | 1 | 57 | M | SQ | IIIA | 0 | 1 | 58 | |
| GSM389992 | PG1 | | 1 72 M | | AD | IIIa | 0 | 2 | 50 | K25, N28 1, Z89 0 0 |
| GSM389976 | PG1 | 1 | 60 | M | SQ | Ib | 0 | 2 | 40 | M48 0, N31 9, C05 2, Z85 8, T78 4 |
| GSM389977 | PG1 | 1 | 62 | M | SQ | IV | 0 | 1 | 28 | I25 2, I69 3 |
| GSM389980 | PG1 | 1 | 59 | F | SCLC | IV | 0 | 1 | 20 | |
| GSM389923 | PG1 | 1 | 62 | M | SQ | IIIa | 0 | 2 | 65 | J44 2, I25 9, I25 2 |
| GSM389925 | PG1 | 1 | 55 | M | SCLC | IIIa | 0 | 1 | 34 | |
| GSM389909 | PG1 | 1 | 65 | M | AD | IIIb | 0 | 1 | 14 | 110, E89 0 |
| GSM389911 | PG1 | 1 | 52 | F | AD | IIIb | 0 | 2 | 27 | M41, K21 9 J42, E66 9, I25 12, E14 9, 174 0, E78 2, |
| GSM389913 | PG1 | 1 | 61 | M | SQ | Ia | 0 | 2 | 60 | I10, I50 12, I73 9 |
| GSM389927 | PG1 | 1 | 60 | F | AD | IIb | 0 | 1 | 2 | K31 88, M10 0 |
| GSM389928 | PG1 | 1 | 47 | F | AD | IIIa | 0 | 2 | 35 | K22 7, C37, Z90 8, K44, T78 4 |
| GSM389931 | PG1 | 1 | 60 | F | SQ | IV | 0 | 2 | 59 | 110, E89 0, J44, Z90 3, H40 |
| GSM389933 | PG1 | 1 | 63 | M | SQ | IIa | 0 | 1 | 32 | I10, I25 19, I71 4 |
| GSM389935 | PG1 | 1 | 52 | M | AD | IV | 0 | 1 | 2 | |
| GSM389943 | PG1 | 1 | 45 | M | AD | IV | 0 | 2 | 31 | |
| GSM389945 | PG1 | 1 | 63 | M | AD | IIIB | 0 | 1 | 52 | |
| GSM389947 | PG1 | 1 | 68 | M | AD | IIb | 0 | 1 | 45 | Z86 1, Z90 3, Z90 4 |
| GSM389949 | PG1 | 1 | 68 | M | AD | IIIa | 0 | 1 | 14 | |
| GSM389951 | PG1 | 1 | 58 | M | SQ | IIIa | 0 | 2 | 35 | None |
| GSM389953 | PG1 | 1 | 71 | M | AD | Ia | 0 | 2 | 36 | I10, E89 0, Z85 8 |
| GSM389916 | PG1 | 1 | 53 | M | AD | IV | 0 | 2 | 45 | J37 0 |
| GSM389981 | PG1 | 1 | 57 | M | AD | IIIa | 0 | 1 | 74 | J38 0 |
| GSM389982 | PG1 | 1 | 67 | M | SQ | IIIb | 0 | 2 | 53 | J44 |
| GSM389984 | PG1 | 1 | 54 | F | AD | IIIa | 0 | 2 | 24 | I25 9, I64, I25 3, I51 3 |
| GSM320333 | PG2 | 0 | 71 | M | NA | NA | NA | 2 | 9 | M65, M25 4, Z96 6, N40, N39 4 |
| GSM320330 | PG2 | 0 | 61 | M | NA | NA | NA | 2 | 114 | C44 3, J45, Z88 0 |
| GSM320332 | PG2 | 0 | 62 | F | NA | NA | NA | 1 | 7 | M16 1, D17 0, Z98 1, T81 0 C43, C79 3, C61, Z95 2, 110, E78 0, K80, |
| GSM320361 | PG2 | 0 | 72 | M | NA | NA | NA | 1 | 26 | Z88 0, |
| GSM320362 | PG2 | 0 | 50 | F | NA | NA | NA | 2 | 30 | L23, L40, I10 |
| GSM320363 | PG2 | 0 | 67 | F | NA | NA | NA | 1 | 17 | M48 06, M54 4, M16, J45 |
| GSM320365 | PG2 | 0 | 54 | M | NA | NA | NA | 2 | 37 | M53 26, M96 1, 110, J42 |
| GSM320328 | PG2 | 0 | 53 | F | NA | NA | NA | 1 | 25 | M16 1, Z96 6, E78 0 |
| GSM320329 | PG2 | 0 | 58 | M | NA | NA | NA | 2 | 37 | Z01 5, I50, I10, E79 0, Z88 0, Z88 4 |
| GSM320339 | PG2 | 0 | 70 | F | NA | NA | NA | 1 | 28 | M48 06, M54 4, M42, I35 1, M17, I10, |
| GSM320331 | PG2 | 0 | 48 | M | NA | NA | NA | 2 | 29 | M42, M51 2 |
| GSM320319 | PG2 | 1 | 69 | M | AD | Ib | 0 | 2 | 64 | K38 9, E14, J42, J96 9, I42 0, I20 9, I10 |
| GSM320337 | PG2 | 1 | 63 | M | SQ | IIIa | 0 | 1 | 35 | I10, M 15 9, L40 0, N 18 9, K25, Z96 6 170 2, 110, 127 0, 125 2, I25 1, Z95 0, |
| GSM320338 | PG2 | 1 | 71 | F | SQ | IIb | 0 | 1 | 49 | Z95 2, E11, J44 |
| GSM320325 | PG2 | 1 | 56 | F | AD | IIIb-IV | 0 | 1 | | 42 I11 9, J44 1, I10, E87 6 |
| GSM320326 | PG2 | 1 | 48 | F | AD | IV | 0 | 2 | 37 | I30 9 |
| GSM320323 | PG2 | 1 | 62 | F | AD | IIb | 0 | 1 | 23 | J44 8 |
| GSM320324 | PG2 | 1 | 67 | F | AD | I | 0 | 2 | 56 | H34 2, K25, F32 9, C08 0 I20, I70 2, E78 2, 125 12, 125 22, N18, |
| GSM320336 | PG2 | 1 | 50 | M | SQ | Ia | 0 | 2 | 27 | F12 |
| GSM320335 | PG2 | 1 | 68 | M | SCLC | IV | 0 | 2 | 44 | C79 3, E03 9, H74 8, F17 1 |
| GSM320320 | PG2 | 1 | 69 | M | AD | IV | 0 | 2 | 40 | I10, I69.3, J44, |
| GSM320334 | PG2 | 1 | 52 | F | SCLC | Ib | 0 | 2 | 32 | C56 |
| GSM320321 | PG2 | 1 | 73 | M | AD | IIIA | 0 | 1 | 35 | I64, I10, I71.4 |
| GSM320322 | PG2 | 1 | 53 | M | LC | IV | 0 | 2 | 14 | K08.9, T78.1 |
| GSM320385 | PG3 | 0 | 76 | M | NA | NA | NA | 1 | 39 | C44.3, 110, E78.0, L80, D33.3 |
| GSM320376 | PG3 | 0 | 67 | M | NA | NA | NA | 1 | 60 | B02.7, C90.0, Z94.8, 110 |
| GSM320377 | PG3 | 0 | 65 | M | NA | NA | NA | 2 | 11 | M48.06, M54.4, G95.1, M42 |
| GSM320379 | PG3 | 0 | 70 | F | NA | NA | NA | 2 | 26 | L40, E66, I10, M17, K45, E61.1 |
| GSM320380 | PG3 | 0 | 76 | M | NA | NA | NA | 1 | 45 | M17, E11, 110, C61 |
| GSM320382 | PG3 | 0 | 67 | M | NA | NA | NA | 1 | 37 | T84.5, Z96.6, M17, 163.9, G20, F32 |
| GSM320383 | PG3 | 0 | 46 | M | NA | NA | NA | 2 | 27 | C49.2, H66.9, H90 |
| GSM320369 | PG3 | 0 | 65 | F | NA | NA | NA | 1 | 49 | |
| GSM320370 | PG3 | 0 | 69 | M | NA | NA | NA | 1 | 105 | |
| GSM320371 | PG3 | 0 | 73 | M | NA | NA | NA | 1 | 53 | |
| GSM320372 | PG3 | 0 | 50 | F | NA | NA | NA | 2 | 33 | L50, L23.0, M81 |
| GSM320373 | PG3 | 0 | 51 | F | NA | NA | NA | 1 | 33 | Q82.2, Z88.1, Z88.6, E89.0 |
| GSM320375 | PG3 | 0 | 62 | M | NA | NA | NA | 1 | 38 | M46.4, M42, K26, 110, E78 |
| GSM320366 | PG3 | 0 | 68 | M | NA | NA | NA | 1 | 37 | M19.9, Z98.1, I10 |
| GSM320367 | PG3 | 0 | 61 | F | NA | NA | NA | 1 | 7 | M51.2, M54.4, G57.6 |
| GSM390036 | PG3 | 0 | 30 | F | NA | NA | NA | 2 | NA | |
| GSM390035 | PG3 | 0 | 45 | F | NA | NA | NA | 0 | NA | |
| GSM390033 | PG3 | 0 | 44 | M | NA | NA | NA | 0 | NA | |
| GSM390038 | PG3 | 0 | 32 | M | NA | NA | NA | 1 | NA | |
| GSM390034 | PG3 | 0 | 37 | M | NA | NA | NA | 0 | NA | |
| GSM390037 | PG3 | 0 | 36 | F | NA | NA | NA | 1 | NA | |
| GSM320350 | PG3 | 1 | 55 | M | AD | IV | 0 | 2 | 41 | K80.2 |
| GSM320351 | PG3 | 1 | 71 | M | AD | IIIB | 0 | 2 | 34 | |
| GSM320352 | PG3 | 1 | 62 | M | AD | IIIB | 0 | 2 | 40 | |
| GSM320353 | PG3 | 1 | 61 | M | SQ | IIIA | 0 | 1 | 68 | E11, I10, E79.0 |
| GSM320354 | PG3 | 1 | 69 | M | SQ | Ib | 0 | 2 | 62 | M42.16, I21.9, R09.1, N20.0, I10 |
| GSM320355 | PG3 | 1 | 62 | M | SQ | IIIB | 0 | 1 | 10 | |
| GSM320318 | PG3 | 1 | 68 | F | SQ | IIIA | 0 | 2 | NA | D32, J44, M51.2 |
| GSM320327 | PG3 | 1 | 63 | F | AD | IV | 0 | 2 | 48 | K38.9, E02, N15.10 |
| GSM320340 | PG3 | 1 | 72 | M | SCLC | IV | 0 | 1 | 94 | 110, E1, N08.3, N40 |
| GSM320341 | PG3 | 1 | 53 | F | SCLC | IIIB | 0 | 2 | 54 | None |
| GSM320342 | PG3 | 1 | 72 | M | SQ | IIIB | 0 | 2 | 104 | S68.1, D35.0 |
| GSM320343 | PG3 | 1 | 78 | M | SQ | IIIA | 0 | 1 | 53 | |
| GSM320344 | PG3 | 1 | 61 | M | AD | Ia | 0 | 1 | 50 | |
| GSM320345 | PG3 | 1 | 52 | F | AD | IV | 0 | 2 | 30 | |
| GSM320346 | PG3 | 1 | 62 | F | AD | IV | 0 | 2 | 11 | |
| GSM320347 | PG3 | 1 | 57 | M | AD | IB | 0 | 2 | 85 | F10.2 |
| GSM320348 | PG3 | 1 | 67 | F | SCLC | IV | 0 | 2 | 29 | J42, E89.0, 140, J44 |
| GSM320349 | PG3 | 1 | 47 | M | SCLC | IV | 0 | 2 | 26 | 169.3, A16.8, A17,B90.0, G45, J15, M81, |
| GSM320356 | PG3 | 1 | 77 | M | AD | IIIA | 0 | 1 | 37 | M24.66, I10 |
| GSM320357 | PG3 | 1 | 61 | M | AD | IV | 0 | 0 | NA | Z96.6, I10 |
| GSM320358 | PG3 | 1 | 50 | F | SCLC | II | 0 | 2 | 31 | |
| GSM320359 | PG3 | 1 | 69 | M | SQ | IIIA | 0 | 2 | 39 | J44, I35.0 |

**Table 3: RNAs for prevalent LC of all stages**

| SEQ ID NO. | ID | Symbol | Gene name | refseq | entrez | p-value | Over-(1) / under-(-1) expression |
|---|---|---|---|---|---|---|---|
| 1 | 10541 | TM6SF1 | Homo sapiens transmembrane 6 superfamily member 1 (TM6SF1), transcript variant 1, mRNA | NM_023003 | 53346 | 0,000242963 | 1 |
| 2 | 10543 | ANKRD13A | Homo sapiens ankyrin repeat domain 13A (ANKRD13A), mRNA | NM_033121 | 88455 | 9,80737E-05 | 1 |
| 3 | 70022 | LCOR | Homo sapiens ligand dependent nuclear receptor corepressor (LCOR), transcript variant 1, mRNA | NM_032440 | 84458 | 5,86843E-05 | 1 |
| 4 | 110706 | CTBS | Homo sapiens chitobiase, di-N-acetyl- (CTBS), mRNA. | NM_004388 | 1486 | 0,000125747 | 1 |
| 5 | 130113 | SLC25A25 | Homo sapiens solute carrier family 25 (mitochondrial carrier;phosphate carrier), member 25 (SLC25A25), nuclear gene encoding mitochondrial protein, transcript variant 2, mRNA. | NM_001006641 | 114789 | 0,00055226 | -1 |
| 6 | 160132 | CREB5 | Homo sapiens cAMP responsive element binding | NM_001011666 | 9586 | 0,000670388 | 1 |
| 7 | 270717 | PELI2 | protein 5 (CREB5), transcript variant 4, mRNA Homo sapiens pellino homolog 2 (Drosophila) (PELI2), mRNA | NM_021255 | 57161 | 0,000145125 | 1 |
| 8 | 430382 | UBE2G1 | Homo sapiens ubiquitin-conjugating enzyme E2G 1 (UBC7 homolog, yeast) (UBE2G1), mRNA | NM_003342 | 7326 | 1,32288E-05 | -1 |
| 9 | 450037 | LY9 | Homo sapiens lymphocyte antigen 9 (LY9), transcript variant 2,mRNA | NM_001033667 | 4063 | 0,00051284 | -1 |
| 10 | 460608 | TNFSF13B | Homo sapiens tumor necrosis factor (ligand) superfamily, member 13b (TNFSF13B), transcript variant 1, mRNA | NM_006573 | 10673 | 0,000795241 | 1 |
| 11 | 510450 | RCC2 | Homo sapiens regulator of chromosome condensation 2 (RCC2), transcript variant 1, mRNA | NM_018715 | 55920 | 5,45662E-05 | -1 |
| 12 | 520332 | GALT | Homo sapiens galactose-1-phosphate undylyltransferase (GALT), mRNA | NM_000155 | 2592 | 3,80634E-05 | -1 |
| 13 | 610563 | HMGB2 | Homo sapiens high mobility group box 2 (HMGB2), transcript variant 1, mRNA | NM_002129 | 3148 | 0,000645674 | 1 |
| 14 | 650164 | CYP4F3 | Homo sapiens cytochrome P450, family 4, subfamily F, polypeptide 3 (CYP4F3), transcript variant 1, mRNA | NM_000896 | 4051 | 0,000132547 | -1 |
| 15 | 650767 | PPP2R5A | Homo sapiens protein phosphatase 2, regulatory subunit B', alpha (PPP2R5A), transcript variant 1, mRNA | NM_006243 | 5525 | 0,000372093 | 1 |
| 16 | 670041 | IL23A | Homo sapiens Interleukin 23, alpha subunit p19 (IL23A), mRNA | NM_016584 | 51561 | 0,000435839 | -1 |
| 17 | 870370 | XPO4 | Homo sapiens exportin 4 (XPO4), mRNA | NM_022459 | 64328 | 0,000714824 | -1 |
| 18 | 940132 | FNIP1 | Homo sapiens folliculin interacting protein 1 (FNIP1 ), transcript variant 2, mRNA | NM_001008738 | 96459 | 0,000398007 | 1 |
| 19 | 1110215 | ESYT1 | Homo sapiens extended synaptotagmin-like protein 1 (ESYT1 ), transcript variant 2, mRNA | NM_015292 | 23344 | 0,00030992 | -1 |
| 20 | 1110600 | EIF4E3 | Homo sapiens eukaryotic translation initiation factor 4E family member 3 (EIF4E3), transcript variant 2, mRNA | NM_173359 | 317649 | 2,05726E-05 | 1 |
| 21 | 1240603 | ITGAX | Homo sapiens Integrin, alpha X (complement component 3 receptor 4 subunit) (ITGAX), mRNA | NM_000887 | 3687 | 0,000164472 | 1 |
| 22 | 1400762 | CPA3 | Homo sapiens carboxypeptidase A3 (mast cell) (CPA3), mRNA | NM_001870 | 1359 | 7,19574E-05 | 1 |
| 23 | 1430292 | SLC11A1 | Homo sapiens solute carrier family 11 (proton-coupled divalent metal ion transporters), member 1 (SLC11A1), mRNA | NM_000578 | 6556 | 0,000556791 | 1 |
| 24 | 1440601 | CDK5RAP1 | Homo sapiens CDK5 regulatory subunit associated protein 1 (CDK5RAP1), transcript variant 2, mRNA | NM_016082 | 51654 | 0,000651058 | -1 |
| 25 | 1450184 | C5orf41 | Homo sapiens chromosome 5 open reading frame 41 (C5orf41), transcript variant 1, mRNA | NM_153607 | 153222 | 0,000417493 | -1 |
| 26 | 1580168 | PRSS12 | Homo sapiens protease, serine, 12 (neurotrypsin, motopsin) (PRSS12), mRNA | NM_003619 | 8492 | 0,000226126 | -1 |
| 27 | 1690189 | HSPA8 | Homo sapiens heat shock 70kDa protein 8 (HSPA8), transcript variant 1, mRNA | NM_006597 | 3312 | 0,000720224 | -1 |
| 28 | 1770131 | TSPAN2 | Homo sapiens tetraspanin 2 (TSPAN2), mRNA | NM_005725 | 10100 | 7,36749E-05 | 1 |
| 29 | 1780348 | IMP3 | Homo sapiens IMP3, U3 small nucleolar ribonucleoprotein, homolog (yeast) (IMP3), mRNA | NM_018285 | 55272 | 0,000632967 | -1 |
| 30 | 1820255 | NA | Homo sapiens cDNA FLJ46626 fis, clone TRACH2001612 | AK_128481 | NA | 5,49124E-06 | -1 |
| 31 | 1820598 | ICAM2 | Homo sapiens Intercellular adhesion molecule 2 (ICAM2), transcript variant 5, mRNA | NM_000873 | 3384 | 0,000398072 | -1 |
| 32 | 2000390 | CDK14 | Homo sapiens cyclin-dependent kinase 14 (CDK14), mRNA | NM_012395 | 5218 | 0,000382729 | 1 |
| 33 | 2030482 | RPS6KA5 | Homo sapiens ribosomal protein S6 kinase, 90kDa, polypeptide 5 (RPS6KA5), transcript variant 1, mRNA | NM_004755 | 9252 | 0,000159455 | 1 |
| 34 | 2060279 | PAK2 | Homo sapiens p21 protein (Cdc42/Rac)-activated kinase 2 (PAK2), mRNA | NM_002577 | 5062 | 6,07456E-05 | -1 |
| 35 | 2070152 | CMTM6 | Homo sapiens CKLF-like MARVEL transmembrane domain containing 6 (CMTM6), mRNA | NM_017801 | 54918 | 0,000549761 | 1 |
| 36 | 2100035 | STK17B | Homo sapiens serine/threonine kinase 17b (STK17B), mRNA | NM_004226 | 9262 | 0,00022726 | 1 |
| 37 | 2100427 | RUNX1 | Homo sapiens runt-related transcription factor 1 (RUNX1), transcript variant 2, mRNA | NM_001001890 | 861 | 0,000684129 | -1 |
| 38 | 2260239 | MXD1 | Homo sapiens MAX dimerization protein 1 | NM_002357 | 4084 | 0,000517481 | 1 |
| 39 | 2370524 | TNFAIP6 | (MXD1), transcript variant 1, mRNA Homo sapiens tumor necrosis factor, alpha-induced protein 6 (TNFAIP6), mRNA | NM_007115 | 7130 | 1,41338E-07 | 1 |
| 40 | 2450064 | ZFP91 | Homo sapiens zinc finger protein 91 homolog (mouse) (ZFP91), transcript variant 1, mRNA | NM_053023 | 80829 | 0,000223977 | -1 |
| 41 | 2450497 | NA | FB22G11 Fetal brain, Stratagene Homo sapiens cDNA clone FB22G11 3-end, mRNA sequence | T03068 I | NA | 0,000709867 | -1 |
| 42 | 2510639 | UBE2Z | Homo sapiens ubiquitin-conjugating enzyme E2Z (UBE2Z), mRNA | NM_023079 | 65264 | 0,000247902 | -1 |
| 43 | 2570703 | C17orf97 | Homo sapiens chromosome 17 open reading frame 97 (C17orf97), mRNA | NM_001013672 | 400566 | 5,19791E-06 | -1 |
| 44 | 2630154 | GABARAPL1 | Homo sapiens GABA(A) receptor-associated protein like 1 (GABARAPL1), mRNA | NM_031412 | 23710 | 0,000412487 | 1 |
| 45 | 2630451 | HIST2H2BE | Homo sapiens histone cluster 2, H2be (HIST2H2BE), mRNA | NM_003528 | 8349 | 0,000520526 | 1 |
| 46 | 2630484 | ATP10B | Homo sapiens ATPase, class V, type 10B (ATP10B), mRNA | NM_025153 | 23120 | 0,000154203 | 1 |
| 47 | 2650075 | AP1S1 | Homo sapiens adaptor-related protein complex 1, sigma 1 subunit (AP1S1), mRNA | NM_001283 | 1174 | 0,000171672 | -1 |
| 48 | 2680010 | EPC1 | Homo sapiens enhancer of polycomb homolog 1 (Drosophila) (EPC1), mRNA | NM_025209 | 80314 | 0,000552857 | -1 |
| 49 | 2690609 | CUTA | Homo sapiens cutA divalent cation tolerance homolog (E coli) (CUTA), transcript variant 1, mRNA | NM_001014433 | 51596 | 0,000701804 | -1 |
| 50 | 2710544 | C3orf37 | Homo sapiens chromosome 3 open reading frame 37 (C3orf37), transcript variant 1, mRNA | NM_001006109 | 56941 | 0,000548537 | -1 |
| 51 | 2760563 | EIF2B1 | Homo sapiens eukaryotic translation initiation factor 2B, subunit 1 alpha, 26kDa (EIF2B1), mRNA | NM_001414 | 1967 | 0,000638551 | 1 |
| 52 | 2850100 | DTX3L | Homo sapiens deltex 3-like (Drosophila) (DTX3L), mRNA | NM_138287 | 151636 | 0,000689534 | -1 |
| 53 | 2850377 | ITPR2 | Homo sapiens inositol 1,4,5-triphosphate receptor, type 2 (ITPR2), mRNA | NM_002223 | 3709 | 0,000636057 | 1 |
| 54 | 2940224 | APH1A | Homo sapiens anterior pharynx defective 1 homolog A (C elegans) (APH1A), transcript variant 1, mRNA | NM_001077628 | 51107 | 0,000390857 | -1 |
| 55 | 3120301 | L3MBTL2 | Homo sapiens I(3)mbt-like 2 (Drosophila) (L3MBTL2), mRNA | NM_031488 | 83746 | 0,000296114 | -1 |
| 56 | 3140039 | CYB5R4 | Homo sapiens cytochrome b5 reductase 4 (CYB5R4), mRNA | NM_016230 | 51167 | 4,50809E-05 | 1 |
| 57 | 3140093 | LZTR1 | Homo sapiens leucine-zipper-like transcription regulator 1 (LZTR1), mRNA | NM_006767 | 8216 | 0,000628324 | -1 |
| 58 | 3180041 | TOR1AIP1 | Homo sapiens torsin A interacting protein 1 (TOR1AIP1), mRNA | NM_015602 | 26092 | 0,000606148 | -1 |
| 59 | 3290162 | LAMP2 | Homo sapiens lysosomal-associated membrane protein 2 (LAMP2), transcript variant C, mRNA | NM_001122606 | 3920 | 8,05131 E-05 | -1 |
| 60 | 3290296 | ANKDD1A | Homo sapiens ankyrin repeat and death domain containing 1A (ANKDD1A), mRNA | NM_182703 | 348094 | 0,000470888 | -1 |
| 61 | 3360364 | MORC2 | Homo sapiens MORC family CW-type zinc finger 2 (MORC2), mRNA | NM_014941 | 22880 | 0,000389771 | -1 |
| 62 | 3360433 | IGF2BP3 | Homo sapiens insulin-like growth factor 2 mRNA binding protein 3 (IGF2BP3), mRNA | NM_006547 | 10643 | 0,000454733 | 1 |
| 63 | 3370402 | LOC401284 | PREDICTED Homo sapiens hypothetical LOC401284 (LOC401284), mRNA | XM_379454 | NA | 0,000393422 | 1 |
| 64 | 3460189 | STXBP5 | Homo sapiens syntaxin binding protein 5 (tomosyn) (STXBP5), transcript variant 1, mRNA | NM_139244 | 134957 | 0,000187189 | 1 |
| 65 | 3460674 | SRPK1 | Homo sapiens SRSF protein kinase 1 (SRPK1), transcript variant 1, mRNA | NM_003137 | 6732 | 3,88823E-05 | 1 |
| 66 | 3520082 | RUVBL1 | Homo sapiens RuvB-like 1 (E coli) (RUVBL1), mRNA | NM_003707 | 8607 | 0,000217416 | -1 |
| 67 | 3610504 | GNE | Homo sapiens glucosamine (UDP-N-acetyl)-2-epimerase/N-acetylmannosamine kinase (GNE), transcript variant 2, mRNA | NM_005476 | 10020 | 3,36864E-05 | -1 |
| 68 | 3780689 | NT5C3 | Homo sapiens 5'-nucleotidase, cytosolic III (NT5C3), transcript variant 2, mRNA | NM_001002009 | 51251 | 6,68713E-06 | 1 |
| 69 | 3800270 | CCR2 | Homo sapiens chemokine (C-C motif) receptor 2 (CCR2), transcript variant A, mRNA | NM_001123041 | 1231 | 0,000167225 | -1 |
| 70 | 3830341 | LYRM1 | Homo sapiens LYR motif containing 1 (LYRM1), transcript variant 1, mRNA | NM_020424 | 57149 | 0,000124551 | -1 |
| 71 | 3830390 | KIAA0692 | PREDICTED Homo sapiens KIAA0692 protein, transcript variant 12 (KIAA0692), mRNA | XM_930898 | NA | 0,000774647 | 1 |
| 72 | 3870754 | FBXO28 | Homo sapiens F-box protein 28 (FBXO28), transcript variant 1, mRNA | NM_015176 | 23219 | 0,000764424 | -1 |
| 73 | 3990176 | PROSC | Homo sapiens proline synthetase co-transcribed | NM_007198 | 11212 | 0,000546284 | -1 |
| 74 | 3990639 | IL23A | homolog (bacterial) (PROSC), mRNA Homo sapiens Interleukin 23, alpha subunit p19 (IL23A), mRNA | NM_016584 | 51561 | 0,000463322 | -1 |
| 75 | 4010048 | ACOX1 | Homo sapiens acyl-CoA oxidase 1, palmitoyl (ACOX1), transcript variant 1, mRNA | NM_004035 | 51 | 0,000452803 | 1 |
| 76 | 4050195 | NA | Homo sapiens genomic DNA, cDNA DKFZp564O0862 (from clone DKFZp564O0862) | AL080095 | NA | 0,000112435 | 1 |
| 77 | 4050270 | NA | UI-E-CK1-afm-g-09-0-UI s2 UI-E-CK1 Homo sapiens cDNA clone UI-E-CK1-afm-g-09-0-UI 3-, mRNA sequence | BM668555 1 | NA | 0,000226572 | 1 |
| 78 | 4060131 | LPXN | Homo sapiens leupaxin (LPXN), transcript variant 2, mRNA | NM_004811 | 9404 | 0,000789377 | -1 |
| 79 | 4060138 | NA | PREDICTED Homo sapiens similar to Transcriptional regulator ATRX (ATP-dependent helicase ATRX) (X-linked helicase II) (X-linked nuclear protein) (XNP) (Znf-HX) (LOC652455), mRNA | XM_941904 | NA | 0,000295927 | -1 |
| 80 | 4060605 | CD44 | Homo sapiens CD44 molecule (Indian blood group) (CD44), transcript variant 1, mRNA | NM_000610 | 960 | 0,000351524 | -1 |
| 81 | 4220138 | SDHAF1 | Homo sapiens succinate dehydrogenase complex assembly factor 1 (SDHAF1), nuclear gene encoding mitochondrial protein, mRNA | NM_001042631 | 644096 | 0,000531803 | -1 |
| 82 | 4230253 | MLL5 | Homo sapiens myeloid/lymphoid or mixed-lineage leukemia 5 (trithorax homolog, Drosophila) (MLL5), transcript variant 2, mRNA | NM_018682 | 55904 | 0,000183207 | -1 |
| 83 | 4260102 | NA | UI-H-BI3-ajz-b-11-0-UI s1 NCI_CGAP_Sub5 Homo sapiens CDNA clone IMAGE 2733285 3, mRNA sequence | AW444880 1 | NA | 0,000278766 | 1 |
| 84 | 4280047 | RNF13 | Homo sapiens ring finger protein 13 (RNF13), transcript variant 3, mRNA | NM_183383 | 11342 | 0,000775091 | 1 |
| 85 | 4280056 | C12orf49 | Homo sapiens chromosome 12 open reading frame 49 (C12orf49), mRNA | NM_024738 | 79794 | 0,000774408 | 1 |
| 86 | 4280332 | DDX24 | Homo sapiens DEAD (Asp-Glu-Ala-Asp) box polypeptide 24 (DDX24), mRNA | NM_020414 | 57062 | 0,000120548 | -1 |
| 87 | 4280373 | SVIL | Homo sapiens supervillin (SVIL), transcript variant 1, mRNA | NM_003174 | 6840 | 0,000309748 | 1 |
| 88 | 4290477 | NA | Homo sapiens sperm associated antigen 9 (SPAG9), transcript variant 2, mRNA | NM_172345 | NA | 0,000362839 | 1 |
| 89 | 4540082 | PHF19 | Homo sapiens PHD finger protein 19 (PHF19), transcript variant 2, mRNA | NM_001009936 | 26147 | 0,000502643 | -1 |
| 90 | 4560039 | PRUNE | Homo sapiens prune homolog (Drosophila) (PRUNE), mRNA | NM_021222 | 58497 | 4,24358E-05 | 1 |
| 91 | 4570730 | LOC645232 | PREDICTED Homo sapiens hypothetical protein LOC645232 (LOC645232), mRNA | XM_928271 | NA | 0,000739672 | -1 |
| 92 | 4640044 | BCL6 | Homo sapiens B-cell CLL/lymphoma 6 (zinc finger protein 51) (BCL6), transcript variant 2, mRNA | NM_138931 | 604 | 0,000484396 | 1 |
| 93 | 4730195 | HIST1H4H | Homo sapiens histone cluster 1, H4h (HIST1H4H), mRNA | NM_003543 | 8365 | 3,12382E-05 | 1 |
| 94 | 4730577 | NA | UI-E-EJ1-aka-f-15-0-UI s1 UI-E-EJ1 Homo sapiens cDNA clone UI-E-EJ1-aka-f-15-0-UI 3-, mRNA sequence | CK300859 1 | NA | 0,000578904 | -1 |
| 95 | 4760543 | NUP62 | Homo sapiens nucleoporin 62kDa (NUP62), transcript variant 4, mRNA | NM_012346 | 23636 | 0,000292124 | -1 |
| 96 | 4810204 | CYSLTR1 | Homo sapiens cysteinyl leukotriene receptor 1 (CYSLTR1), mRNA | NM_006639 | 10800 | 8,94451 E-05 | 1 |
| 97 | 4850711 | LOC644474 | PREDICTED Homo sapiens hypothetical protein LOC644474 (LOC644474), mRNA | XM_930098 | NA | 0,000527041 | -1 |
| 98 | 4900053 | PUF60 | Homo sapiens poly-U binding splicing factor 60KDa (PUF60), transcript variant 2, mRNA | NM_014281 | 22827 | 0,000279673 | -1 |
| 99 | 4920142 | LAMP2 | Homo sapiens lysosomal-associated membrane protein 2 (LAMP2), transcript variant C, mRNA | NM_001122606 | 3920 | 0,000430427 | 1 |
| 100 | 4920575 | ZNF740 | Homo sapiens zinc finger protein 740 (ZNF740), mRNA | NM_001004304 | 283337 | 0,00068817 | -1 |
| 101 | 5090477 | PIP4K2B | Homo sapiens phosphatidylinositol-5-phosphate 4-kinase, type II, beta (PIP4K2B), mRNA | NM_003559 | 8396 | 2,45489E-05 | -1 |
| 102 | 5290289 | YIPF4 | Homo sapiens Yip1 domain family, member 4 (YIPF4), mRNA | NM_032312 | 84272 | 0,000514659 | 1 |
| 103 | 5290452 | CPEB3 | Homo sapiens cytoplasmic polyadenylation element binding protein 3 (CPEB3), transcript variant 1, mRNA | NM_014912 | 22849 | 0,000159172 | 1 |
| 104 | 5310754 | METTL13 | Homo sapiens methyltransferase like 13 (METTL13), transcript variant 3, mRNA | NM_001007239 | 51603 | 0,000419383 | -1 |
| 105 | 5340246 | CD9 | Homo sapiens CD9 molecule (CD9), mRNA | NM_001769 | 928 | 0,000216455 | -1 |
| 106 | 5390131 | MCM3AP | Homo sapiens minichromosome maintenance | NM_003906 | 8888 | 0,000419553 | -1 |
| 107 | 5390504 | BIRC3 | complex component 3 associated protein (MCM3AP), mRNA Homo sapiens baculoviral IAP repeat containing 3 (BIRC3), transcript variant 1, mRNA | NM_001165 | 330 | 0,000174761 | 1 |
| 108 | 5490064 | OTUD1 | PREDICTED Homo sapiens OTU domain containing 1 (OTUD1), mRNA | XM_939698 | NA | 0,000361512 | -1 |
| 109 | 5690037 | PRSS50 | Homo sapiens protease, serine, 50 (PRSS50), mRNA | NM_013270 | 29122 | 0,000112016 | |
| 110 | 5720681 | TIPARP | Homo sapiens TCDD-inducible poly(ADP-ribose) polymerase (TIPARP), transcript variant 2, mRNA | NM_015508 | 25976 | 0,000373542 | -1 |
| 111 | 5860196 | NA | UI-E-CI1-afs-e-04-0-UI s1 UI-E-CI1 Homo sapiens cDNA clone UI-E-CI1-afs-e-04-0-UI 3-, mRNA sequence | BU733214 1 | NA | 0,000141309 | 1 |
| 112 | 5860400 | HIST1H2AE | Homo sapiens histone cluster 1, H2ae (HIST1H2AE), mRNA | NM_021052 | 3012 | 0,000116423 | 1 |
| 113 | 5860500 | EIF2C3 | Homo sapiens eukaryotic translation initiation factor 2C, 3 (EIF2C3), transcript variant 1, mRNA | NM_024852 | 192669 | 0,000613944 | -1 |
| 114 | 5900156 | TUBA1 B | Homo sapiens tubulin, alpha 1b (TUBA1B), mRNA | NM_006082 | 10376 | 0,000118183 | -1 |
| 115 | 5910091 | ANKK1 | Homo sapiens ankyrin repeat and kinase domain containing 1 (ANKK1), mRNA | NM_178510 | 255239 | 0,000308507 | -1 |
| 116 | 5910682 | LOC348645 | Homo sapiens hypothetical protein LOC348645 (LOC348645), mRNA | NM_198851 | NA | 0,000643342 | -1 |
| 117 | 5960128 | TAF15 | Homo sapiens TAF15 RNA polymerase II, TATA box binding protein (TBP)-associated factor, 68kDa (TAF15), transcript variant 2, mRNA | NM_003487 | 8148 | 0,000190658 | -1 |
| 118 | 6020402 | SRP68 | Homo sapiens signal recognition particle 68kDa (SRP68), mRNA | NM_014230 | 6730 | 0,000749867 | -1 |
| 119 | 6110088 | ABCA1 | Homo sapiens ATP-binding cassette, sub-family A (ABC1), member 1 (ABCA1), mRNA | NM_005502 | 19 | 0,000514553 | 1 |
| 120 | 6110537 | LOC284701 | PREDICTED Homo sapiens similar to hypothetical protein LOC284701, transcript variant 2 (LOC642816), mRNA | XM_931928 | NA | 0,000606685 | -1 |
| 121 | 6110768 | ATIC | Homo sapiens 5-aminoimidazole-4-carboxamide ribonucleotide formyltransferase/IMP cyclohydrolase (ATIC), mRNA | NM_004044 | 471 | 0,000286296 | -1 |
| 122 | 6180427 | GPR160 | Homo sapiens G protein-coupled receptor 160 (GPR160), mRNA | NM_014373 | 26996 | 0,000312842 | -1 |
| 123 | 6200563 | ZNF654 | Homo sapiens zinc finger protein 654 (ZNF654), mRNA | NM_018293 | 55279 | 0,000270097 | 1 |
| 124 | 6220022 | RNF38 | Homo sapiens ring finger protein 38 (RNF38), transcript variant 1, mRNA | NM_022781 | 152006 | 0,000362573 | -1 |
| 125 | 6220450 | DHRS9 | Homo sapiens dehydrogenase/reductase (SDR family) member 9 (DHRS9), transcript variant 1, mRNA | NM_005771 | 10170 | 0,00014906 | 1 |
| 126 | 6270128 | CD40LG | Homo sapiens CD40 ligand (CD40LG), mRNA | NM_000074 | 959 | 0,000197683 | -1 |
| 127 | 6270301 | AP1S1 | Homo sapiens adaptor-related protein complex 1, sigma 1 subunit (AP1S1), mRNA | NM_001283 | 1174 | 8,37111 E-05 | -1 |
| 128 | 6280343 | EEF2K | Homo sapiens eukaryotic elongation factor-2 kinase (EEF2K), mRNA | NM_013302 | 29904 | 0,000263638 | -1 |
| 129 | 6290458 | ZNF200 | Homo sapiens zinc finger protein 200 (ZNF200), transcript variant 1, mRNA | NM_003454 | 7752 | 0,000253711 | 1 |
| 130 | 6350452 | APAF1 | Homo sapiens apoptotic peptidase activating factor 1 (APAF1), transcript variant 2, mRNA | NM_001160 | 317 | 0,000611935 | 1 |
| 131 | 6350608 | MYLK | Homo sapiens myosin light chain kinase (MYLK), transcript variant 1, mRNA | NM_053025 | 4638 | 8,97415E-05 | -1 |
| 132 | 6380598 | IMP4 | Homo sapiens IMP4, U3 small nucleolar ribonucleoprotein, homolog (yeast) (IMP4), mRNA | NM_033416 | 92856 | 0,000519664 | -1 |
| 133 | 6420692 | RSBN1L | Homo sapiens round spermafid basic protein 1-like (RSBN1L), mRNA | NM_198467 | 222194 | 0,0005904 | 1 |
| 134 | 6520333 | LOC652759 | PREDICTED Homo sapiens similar to F-box and WD-40 domain protein 10 (LOC652759), mRNA | XM_942392 | NA | 0,000152094 | -1 |
| 135 | 6550520 | LYSMD2 | Homo sapiens LysM, putative peptidoglycan-binding, domain containing 2 (LYSMD2), transcript variant 1, mRNA | NM_153374 | 256586 | 0,000356986 | -1 |
| 136 | 6580445 | ENKUR | Homo sapiens enkurin, TRPC channel interacting protein (ENKUR), mRNA | NM_145010 | 219670 | 0,000361628 | -1 |
| 137 | 6590278 | AP3M1 | Homo sapiens adaptor-related protein complex 3, mu 1 subunit (AP3M1), transcript variant 2, mRNA | NM_012095 | 26985 | 0,000105537 | -1 |
| 138 | 6590386 | FN3KRP | Homo sapiens fructosamine 3 kinase related protein (FN3KRP), mRNA | NM_024619 | 79672 | 0,000254624 | -1 |
| 139 | 6660097 | QKI | Homo sapiens quaking homolog, KH domain RNA | NM_006775 | 9444 | 9,41329E-05 | -1 |
| 140 | 6760441 | OSBP | binding (mouse) (QKI), transcript variant 1, mRNA. Homo sapiens oxysterol binding protein (OSBP), mRNA | NM_002556 | 5007 | 0,000222086 | -1 |
| 141 | 6940524 | PDE5A | Homo sapiens phosphodiesterase 5A, cGMP-specific (PDE5A), transcript variant 1, mRNA. | NM_001083 | 8654 | 0,000118562 | 1 |
| 142 | 6960746 | GIMAP5 | Homo sapiens GTPase, IMAP family member 5 (GIMAP5), mRNA. | NM_018384 | 55340 | 0,000373699 | -1 |
| 143 | 6980070 | B4GALT5 | Homo sapiens UDP-Gal:betaGIcNAc beta 1,4-galactosyltransferase, polypeptide 5 (B4GALT5), mRNA. | NM_004776 | 9334 | 0,000353132 | 1 |
| 144 | 6980129 | PGK1 | Homo sapiens phosphoglycerate kinase 1 (PGK1), mRNA. | NM_000291 | 5230 | 0,000771983 | -1 |
| 145 | 6980274 | NA | 603176844F1 NIH_MGC_121 Homo sapiens cDNA clone IMAGE:5241250 5-, mRNA sequence | B1915661.1 | NA | 0,000455734 | 1 |
| 146 | 6980609 | LRRTM1 | Homo sapiens leucine rich repeat transmembrane neuronal 1 (LRRTM1), mRNA. | NM_178839 | 347730 | 0,000250817 | -1 |
| 147 | 7040187 | ARRDC4 | Homo sapiens arrestin domain containing 4 (ARRDC4), mRNA. | NM_183376 | 91947 | 5,48071 E-05 | -1 |
| 148 | 7050543 | COQ6 | Homo sapiens coenzyme Q6 homolog, monooxygenase (S. cerevisiae) (COQ6), nuclear gene encoding mitochondrial protein, transcript variant 1, mRNA. | NM_182476 | 51004 | 0,000756562 | -1 |
| 149 | 7100136 | SLC36A1 | Homo sapiens solute carrier family 36 (proton/amino acid symporter), member 1 (SLC36A1), mRNA. | NM_078483 | 206358 | 0,000359438 | 1 |
| 150 | 7100520 | WHSC1 | Homo sapiens Wolf-Hirschhorn syndrome candidate 1 (WHSC1), transcript variant 10, mRNA. | NM_001042424 | 7468 | 0,000181408 | 1 |
| 151 | 7150634 | MYO9A | Homo sapiens myosin IXA (MYO9A), mRNA. | N_006901 | 4649 | 0,000141291 | -1 |
| 152 | 7160296 | PDCD11 | Homo sapiens programmed cell death 11 (PDCD11), mRNA. | NM_014976 | 22984 | 0,000581747 | -1 |
| 153 | 7160767 | UBE2Z | Homo sapiens ubiquitin-conjugating enzyme E2Z (UBE2Z), mRNA. | NM_023079 | 65264 | 0,000605771 | -1 |
| 154 | 7200681 | KIAA1618 | PREDICTED: Homo sapiens KIAA1618 (KIAA1618), mRNA. | XM_941239 | NA | 0,000132256 | -1 |
| 155 | 7210372 | UGCGL1 | Homo sapiens UDP-glucose ceramide glucosyltransferase-like 1 (UGCGL1), transcript variant 2, mRNA. | NM_001025777 | 56886 | 1,21875E-05 | -1 |
| 156 | 7320047 | SAMHD1 | Homo sapiens SAM domain and HD domain 1 (SAMHD1), mRNA. | NM_015474 | 25939 | 0,000542851 | -1 |
| 157 | 7380274 | ZMYM6 | Homo sapiens zinc finger, MYM-type 6 (ZMYM6), mRNA. | NM_007167 | 9204 | 0,000385861 | -1 |
| 158 | 7380288 | ANAPC5 | Homo sapiens anaphase promoting complex subunit 5 (ANAPC5), transcript variant 1, mRNA. | NM_016237 | 51433 | 0,000593428 | -1 |
| 159 | 7550537 160 7570603 | SLC25A5 RAB31 | Homo sapiens solute carrier family 25 (mitochondrial carrier; adenine nucleotide translocator), member 5 (SLC25A5), nuclear gene encoding mitochondrial protein, mRNA. Homo sapiens RAB31, member RAS oncogene family (RAB31), mRNA. | NM_001152 NM_006868 | 292 11031 | 7,90327E-05 0,000162417 | -1 1 |
| 161 | 7650379 | TMEM154 | Homo sapiens transmembrane protein 154 (TMEM154), mRNA. | NM_152680 | 201799 | 0,000127518 | -1 |

**Table 4:**

| Probe ID | GeneSymbol |
|---|---|
| 6200563 | ZNF654 |
| 430382 | UBE2G1 |
| 5900156 | TUBA1B |
| 2370524 | TNFAIP6 |
| 5720681 | TIPARP |
| 3460189 | STXBP5 |
| 2100035 | STK17B |
| 3460674 | SRPK1 |
| 5090477 | PIP4K2B |
| 2000390 | PFTK1 |
| 270717 | PELI2 |
| 3780689 | NT5C3 |
| 7200681 | NA |
| 5490064 | NA |
| 4060138 | NA |
| 3830390 | NA |
| 7150634 | MYO9A |
| 6550520 | LYSMD2 |
| 3830341 | LYRM1 |
| 2570703 | LOC400566 |
| 1820598 | ICAM 2 |
| 610563 | HMGB2 |
| 4730195 | HIST1H4H |
| 5860400 | HIST1H2AE |
| 6180427 | GPR160 |
| 3610504 | GNE |
| 520332 | GALT |
| 5860500 | EIF2C3 |
| 4280332 | DDX24 |
| 3140039 | CYB5R4 |
| 5290452 | CPEB3 |

**Table 5:**

| GeneSymbol | probe set | F statistic | p-value |
|---|---|---|---|
| | 1820255 | 14,1185 | 5,49E-06 |
| | 4050195 | 10,1948 | 0,000112435 |
| | 5860196 | 9,90952 | 0,000141309 |
| | 4050270 | 9,3253 | 0,000226572 |
| | 4260102 | 9,07091 | 0,000278766 |
| | 4920142 | 8,54205 | 0,000430427 |
| | 670041 | 8,52692 | 0,000435839 |
| | 6980274 | 8,47291 | 0,000455735 |
| | 3990639 | 8,45296 | 0,000463322 |
| | 4730577 | 8,18449 | 0,000578904 |
| | 2450497 | 7,93996 | 0,000709867 |
| | 1690504 | 7,77255 | 0,000816714 |
| | 4280240 | 7,66009 | 0,00089762 |
| | 5860682 | 7,64396 | 0,000909879 |
| | 4060017 | 7,47457 | 0,0010495 |
| | 1660451 | 7,38896 | 0,00112822 |
| | 5560093 | 7,34668 | 0,00116931 |
| | 3940020 | 7,34348 | 0,00117248 |
| | 6550333 | 7,21047 | 0,00131242 |
| | 1570703 | 7,15098 | 0,00138044 |
| | 4150402 | 7,12535 | 0,00141086 |
| | 1110358 | 7,05133 | 0,00150259 |
| | 4010296 | 6,91861 | 0,00168268 |
| | 2450343 | 6,84874 | 0,00178623 |
| | 2850762 | 6,84117 | 0,00179784 |
| | 4070280 | 6,77187 | 0,00190772 |
| | 650343 | 6,6981 | 0,00203228 |
| | 1990113 | 6,67761 | 0,00206835 |
| | 5130154 | 6,62206 | 0,00216943 |
| | 6840408 | 6,58787 | 0,00223416 |
| | 5390187 | 6,58213 | 0,00224521 |
| | 6940246 | 6,53962 | 0,00232886 |
| | 5270450 | 6,48382 | 0,00244355 |
| | 5420148 | 6,47773 | 0,00245641 |
| | 3930632 | 6,40425 | 0,0026172 |
| | 3940719 | 6,3459 | 0,00275252 |
| | 1940341 | 6,33404 | 0,00278088 |
| | 4610129 | 6,32188 | 0,00281029 |
| | 2060220 | 6,30442 | 0,00285305 |
| | 3450187 | 6,28972 | 0,00288959 |
| | 6060400 | 6,21906 | 0,00307198 |
| | 4050692 | 6,19827 | 0,00312787 |
| | 4560463 | 6,19294 | 0,00314233 |
| | 540390 | 6,18482 | 0,00316455 |
| | 650014 | 6,15899 | 0,00323628 |
| | 4010025 | 6,15102 | 0,00325875 |
| | 6860743 | 6,12387 | 0,00333651 |
| | 4730088 | 6,0908 | 0,00343378 |
| | 5570632 | 6,07838 | 0,00347105 |
| | 4880373 | 6,05754 | 0,00353452 |
| | 2120356 | 6,04802 | 0,00356394 |
| | 240653 | 5,98976 | 0,00374938 |
| | 2100482 | 5,9589 | 0,00385156 |
| | 6220706 | 5,95393 | 0,00386829 |
| | 2000474 | 5,95307 | 0,00387119 |
| | 6040326 | 5,90341 | 0,00404257 |
| | 5360605 | 5,89899 | 0,00405818 |
| | 6650482 | 5,89178 | 0,00408381 |
| | 6510452 | 5,87022 | 0,00416141 |
| | 5130747 | 5,80679 | 0,00439863 |
| | 4900471 | 5,79219 | 0,00445516 |
| | 1500433 | 5,78948 | 0,00446577 |
| | 6660056 | 5,74704 | 0,00463477 |
| | 4560543 | 5,72865 | 0,00471003 |
| | 3520040 | 5,71127 | 0,0047823 |
| | 7210719 | 5,71103 | 0,00478332 |
| | 1410678 | 5,70918 | 0,00479109 |
| | 4900441 | 5,67343 | 0,00494362 |
| | 5130553 | 5,67112 | 0,00495368 |
| | 5960307 | 5,65178 | 0,00503844 |
| | 2510324 | 5,64786 | 0,0050558 |
| | 3800524 | 5,64519 | 0,00506767 |
| | 4560451 | 5,6406 | 0,00508814 |
| | 4200148 | 5,62095 | 0,00517666 |
| | 5050112 | 5,60435 | 0,00525268 |
| | 5810521 | 5,60106 | 0,0052679 |
| | 3390333 | 5,60078 | 0,0052692 |
| | 5560736 | 5,59779 | 0,00528302 |
| | 2810537 | 5,57818 | 0,00537487 |
| | 3180750 | 5,56903 | 0,00541829 |
| | 1070131 | 5,55883 | 0,00546706 |
| | 4670195 | 5,55044 | 0,00550756 |
| | 3370646 | 5,5398 | 0,00555938 |
| | 4780386 | 5,53607 | 0,00557761 |
| | 7650553 | 5,52949 | 0,00561002 |
| | 650431 | 5,52747 | 0,00562002 |
| | 3850020 | 5,49933 | 0,00576095 |
| | 6650576 | 5,48985 | 0,0058092 |
| | 3800192 | 5,48005 | 0,00585956 |
| | 520154 | 5,47519 | 0,0058847 |
| | 2060026 | 5,46904 | 0,00591666 |
| | 4220367 | 5,46097 | 0,00595889 |
| | 5550270 | 5,45728 | 0,00597832 |
| | 4810327 | 5,45721 | 0,0059787 |
| | 5340338 | 5,45264 | 0,00600282 |
| | 6420370 | 5,44511 | 0,0060428 |
| | 4220523 | 5,41643 | 0,00619754 |
| | 5670735 | 5,40263 | 0,00627348 |
| | 4540088 | 5,39708 | 0,00630423 |
| | 7570725 | 5,38871 | 0,00635101 |
| | 1050128 | 5,38517 | 0,00637087 |
| | 6420541 | 5,35224 | 0,0065588 |
| | 3890491 | 5,31733 | 0,00676428 |
| | 4220450 | 5,30399 | 0,00684454 |
| | 2450424 | 5,30056 | 0,00686532 |
| | 2190451 | 5,29585 | 0,00689401 |
| | 5550634 | 5,29177 | 0,00691888 |
| | 150711 | 5,27243 | 0,00703831 |
| | 3850367 | 5,26624 | 0,00707693 |
| | 510653 | 5,22629 | 0,00733174 |
| | 2640441 | 5,22571 | 0,00733549 |
| | 630372 | 5,21527 | 0,00740365 |
| | 840139 | 5,2066 | 0,00746074 |
| | 830482 | 5,1925 | 0,00755456 |
| | 7320041 | 5,18344 | 0,00761544 |
| | 1570424 | 5,17193 | 0,0076936 |
| | 830167 | 5,14976 | 0,00784637 |
| | 2650520 | 5,1314 | 0,00797525 |
| | 5130204 | 5,13014 | 0,0079842 |
| | 5090647 | 5,12576 | 0,00801528 |
| | 4900064 | 5,11547 | 0,00808884 |
| | 4900497 | 5,11362 | 0,0081021 |
| | 6560292 | 5,10836 | 0,00814009 |
| | 5670445 | 5,07695 | 0,00837036 |
| | 50242 | 5,0629 | 0,00847556 |
| | 2650243 | 5,04917 | 0,00857968 |
| | 4490612 | 5,03315 | 0,00870279 |
| | 2710291 | 5,01493 | 0,00884494 |
| | 6370592 | 5,00692 | 0,00890826 |
| | 6400047 | 4,99624 | 0,00899332 |
| | 6280682 | 4,97761 | 0,00914377 |
| | 5420487 | 4,97558 | 0,00916028 |
| AAAS | 870088 | 5,22423 | 0,00734515 |
| AARS | 2490747 | 5,42843 | 0,00613228 |
| ABCA1 | 6110088 | 8,32635 | 0,000514553 |
| ABCC5 | 7610097 | 5,30928 | 0,00681258 |
| ABLIM1 | 1110575 | 6,03632 | 0,00360039 |
| ACO2 | 10068 | 5,1771 | 0,0076584 |
| ACOT1 | 7510224 | 5,55523 | 0,00548442 |
| ACOX1 | 4010048 | 8,48072 | 0,000452803 |
| ACSS1 | 5090047 | 6,15103 | 0,00325872 |
| ADA | 3400328 | 5,2374 | 0,00725998 |
| ADAR | 1410358 | 6,02935 | 0,00362232 |
| ADCK2 | 2630524 | 5,25902 | 0,00712234 |
| ADCY4 | 4230653 | 6,54626 | 0,00231559 |
| AGTPBP1 | 4860132 | 5,10886 | 0,00813646 |
| AHSA1 | 3990192 | 6,40918 | 0,00260606 |
| AKR1A1 | 1300768 | 6,29116 | 0,00288598 |
| AKR1B1 | 5890327 | 6,36525 | 0,00270686 |
| ALB | 2710427 | 6,2086 | 0,00309997 |
| ALDH16A1 | 4050411 | 5,68965 | 0,0048738 |
| ALDH8A1 | 2340358 | 5,11504 | 0,0080919 |
| ALG8 | 4810431 | 5,57112 | 0,00540833 |
| ALG9 | 2120681 | 6,61337 | 0,0021857 |
| ALKBH5 | 2100221 | 5,93864 | 0,0039202 |
| ANAPC5 | 7380288 | 8,15472 | 0,000593428 |
| ANKDD1A | 3290296 | 8,43338 | 0,000470888 |
| ANKHD1 | 540338 | 5,03817 | 0,00866398 |
| ANKK1 | 5910091 | 8,94699 | 0,000308507 |
| ANKMY1 | 4850541 | 6,47239 | 0,00246776 |
| ANKRD13 | 10543 | 10,3662 | 9,81E-05 |
| ANKRD17 | 2600097 | 5,89269 | 0,00408056 |
| ANKRD32 | 3120358 | 5,03412 | 0,00869527 |
| ANXA7 | 6770403 | 5,39807 | 0,00629876 |
| AP1S1 | 6270301 | 10,5654 | 8,37E-05 |
| AP1S1 | 2650075 | 9,66784 | 0,000171672 |
| AP1S1 | 7050072 | 5,5133 | 0,0056905 |
| AP3M1 | 6590278 | 10,2741 | 0,000105538 |
| APAF1 | 6350452 | 8,11784 | 0,000611935 |
| APEX1 | 1190647 | 5,35486 | 0,00654365 |
| APH1A | 2940224 | 8,65896 | 0,000390858 |
| APOBEC3F | 4070132 | 6,68502 | 0,00205524 |
| APOL3 | 5670274 | 5,63688 | 0,00510477 |
| APRT | 650358 | 5,28658 | 0,00695075 |
| AQP9 | 6770564 | 7,13272 | 0,00140204 |
| ARFIP1 | 6480647 | 5,60559 | 0,00524699 |
| ARHGAP17 | 1710100 | 5,18768 | 0,00758689 |
| ARHGAP21 | 3370487 | 5,5288 | 0,00561343 |
| ARID1A | 150148 | 5,28184 | 0,00697992 |
| ARID2 | 3850347 | 7,07364 | 0,00147432 |
| ARIH2 | 5390669 | 7,12379 | 0,00141273 |
| ARL6IP6 | 2710722 | 5,81679 | 0,00436035 |
| ARPC5 | 3930243 | 5,92342 | 0,00397259 |
| ARPC5L | 1770279 | 5,11671 | 0,00807995 |
| ARPP-19 | 2600008 | 6,84895 | 0,00178591 |
| ARPP-21 | 840762 | 5,70693 | 0,00480054 |
| ARRDC4 | 7040187 | 11,1023 | 5,48E-05 |
| ASB8 | 4280114 | 7,51678 | 0,00101277 |
| ASXL2 | 60750 | 5,09956 | 0,00820393 |
| ATIC | 6110768 | 9,0383 | 0,000286296 |
| ATP10B | 2630484 | 9,80095 | 0,000154203 |
| ATP11B | 540053 | 6,66493 | 0,00209099 |
| ATP1A1 | 1240440 | 5,42734 | 0,00613818 |
| ATP5A1 | 3130300 | 5,72996 | 0,00470464 |
| ATP5G2 | 6350360 | 5,25137 | 0,00717071 |
| ATP5I | 4570095 | 5,66902 | 0,00496281 |
| ATP7A | 1110259 | 5,74309 | 0,00465082 |
| ATXN1 | 3310470 | 6,06884 | 0,00349998 |
| B4GALT5 | 6980070 | 8,78232 | 0,000353132 |
| B4GALT7 | 6100220 | 6,51419 | 0,00238043 |
| BAZ1A | 4920204 | 6,08814 | 0,00344174 |
| BCL10 | 6290343 | 5,12073 | 0,00805115 |
| BCL11B | 7000133 | 7,56123 | 0,000975516 |
| BCL6 | 4640044 | 8,39922 | 0,000484396 |
| BIRC3 | 5390504 | 9,64575 | 0,000174761 |
| BLR1 | 1440291 | 5,74509 | 0,00464269 |
| BTBD10 | 4860296 | 6,23031 | 0,00304217 |
| BTN3A2 | 4920577 | 5,88592 | 0,00410478 |
| C10orf42 | 6480717 | 5,4839 | 0,00583975 |
| C10orf63 | 6580445 | 8,7534 | 0,000361627 |
| C11orf53 | 1240278 | 6,26539 | 0,00295111 |
| C12orf49 | 4280056 | 7,83599 | 0,000774407 |
| C14orf130 | 1690543 | 5,03059 | 0,00872262 |
| C14orf138 | 5360132 | 5,0372 | 0,00867146 |
| C14orf32 | 2750162 | 5,56789 | 0,00542371 |
| C16orf30 | 4210647 | 7,36075 | 0,00115547 |
| C18orf17 | 2070241 | 6,70539 | 0,00201962 |
| C1orf117 | 3170070 | 5,66336 | 0,00498751 |
| C1orf119 | 2680671 | 6,66674 | 0,00208774 |
| C1orf151 | 6380358 | 5,78235 | 0,00449372 |
| C1orf55 | 2680739 | 6,58853 | 0,00223289 |
| C1orf93 | 6250338 | 5,12037 | 0,00805373 |
| C20orf3 | 3610634 | 5,32484 | 0,00671954 |
| C20orf4 | 3140022 | 5,04173 | 0,00863662 |
| C20orf42 | 2350209 | 6,49994 | 0,00240983 |
| C21orf2 | 610653 | 5,08616 | 0,0083022 |
| C21orf33 | 5960301 | 5,72653 | 0,00471878 |
| C2orf25 | 5090204 | 5,67239 | 0,00494816 |
| C2orf28 | 6220487 | 6,63835 | 0,00213927 |
| C3orf37 | 2710544 | 8,24931 | 0,000548537 |
| C6orf108 | 7160164 | 5,11554 | 0,00808831 |
| C6orf149 | 2630181 | 7,52935 | 0,00100209 |
| C6orf150 | 4850370 | 6,2728 | 0,00293222 |
| C6orf66 | 7150601 | 6,20336 | 0,00311408 |
| C6orf72 | 4560474 | 5,17458 | 0,00767554 |
| C8orf1 | 6330471 | 6,32648 | 0,00279912 |
| C8orf33 | 3830278 | 6,16979 | 0,00320608 |
| C9orf10OS | 2100215 | 6,21688 | 0,00307778 |
| C9orf23 | 4200332 | 5,50703 | 0,00572203 |
| C9orf66 | 2710458 | 5,29041 | 0,00692723 |
| CA4 | 3990296 | 5,7511 | 0,00461833 |
| CA5B | 7560162 | 6,64643 | 0,00212447 |
| CACHD1 | 3140142 | 6,30178 | 0,00285957 |
| CALM1 | 1780035 | 5,95878 | 0,00385197 |
| CALU | 1430243 | 5,1151 | 0,00809149 |
| CAMKV | 4610619 | 5,55584 | 0,00548147 |
| CANX | 2230360 | 5,16219 | 0,00776036 |
| CASC2 | 1230753 | 7,35586 | 0,00116026 |
| CASC4 | 6960044 | 5,08649 | 0,00829973 |
| CASP4 | 3610048 | 6,04984 | 0,00355829 |
| CCDC28A | 1050253 | 5,6573 | 0,00501411 |
| CCDC64 | 3420343 | 6,51108 | 0,00238681 |
| CCDC71 | 4850484 | 7,74444 | 0,000836209 |
| CCNA1 | 1660309 | 6,32497 | 0,00280278 |
| CCPG1 | 6960707 | 4,98721 | 0,00906594 |
| CCR2 | 3800270 | 9,70036 | 0,000167225 |
| CCT7 | 7150017 | 6,6909 | 0,00204489 |
| CCT7 | 5050390 | 5,64899 | 0,00505079 |
| CD40LG | 6270128 | 9,49339 | 0,000197683 |
| CD44 | 4060605 | 8,78787 | 0,000351524 |
| CD44 | 1410189 | 6,06527 | 0,00351085 |
| CD55 | 10025 | 5,40211 | 0,00627632 |
| CD58 | 4150161 | 7,26541 | 0,00125265 |
| CD59 | 4040672 | 5,02136 | 0,00879453 |
| CD6 | 4850192 | 7,40781 | 0,00111038 |
| CD9 | 5340246 | 9,38153 | 0,000216455 |
| CD96 | 2100333 | 6,42674 | 0,00256687 |
| CDADC1 | 6660671 | 6,24842 | 0,0029948 |
| CDC25B | 460754 | 7,48683 | 0,00103869 |
| CDCA4 | 2640278 | 5,11739 | 0,00807509 |
| CDK2AP1 | 60575 | 5,49794 | 0,005768 |
| CDK4 | 5270500 | 6,35263 | 0,00273655 |
| CDK5RAP1 | 1440601 | 8,0435 | 0,000651058 |
| CDK5RAP2 | 7000600 | 5,05632 | 0,00852528 |
| CDK9 | 60468 | 5,35508 | 0,00654237 |
| CDS1 | 1240739 | 5,61 | 0,00522667 |
| CEACAM1 | 1780152 | 7,69898 | 0,00086875 |
| CECR1 | 5560280 | 5,86424 | 0,00418323 |
| CENTB2 | 6040152 | 7,16437 | 0,00136482 |
| CEP350 | 6290719 | 7,72495 | 0,000850005 |
| CHIC2 | 3440431 | 7,11256 | 0,00142628 |
| CHMP7 | 5550746 | 7,13009 | 0,00140518 |
| CKAP4 | 6770348 | 6,27439 | 0,00292818 |
| CKAP5 | 2650164 | 5,55448 | 0,00548804 |
| CKLF | 2000551 | 6,07946 | 0,00346781 |
| CLEC4D | 3990328 | 5,49923 | 0,00576145 |
| CLEC4E | 940754 | 6,5595 | 0,00228934 |
| CMTM6 | 2070152 | 8,24662 | 0,000549761 |
| CNTNAP1 | 6350017 | 5,85848 | 0,00420433 |
| CNTNAP2 | 5890273 | 7,11747 | 0,00142034 |
| COG2 | 2810767 | 5,47603 | 0,00588037 |
| COL11A1 | 2750070 | 6,8737 | 0,00174851 |
| COPS7B | 5310050 | 7,49393 | 0,00103249 |
| COPZ1 | 6650403 | 5,66714 | 0,00497101 |
| COQ6 | 7050543 | 7,86382 | 0,000756562 |
| CPA3 | 1400762 | 10,7566 | 7,20E-05 |
| CPA5 | 2030300 | 5,99082 | 0,0037459 |
| CPD | 6590553 | 7,39642 | 0,00112113 |
| CPEB3 | 5290452 | 9,76158 | 0,000159172 |
| CPEB4 | 1690360 | 5,91218 | 0,00401174 |
| CR1 | 610687 | 5,08424 | 0,00831634 |
| CREB5 | 160132 | 8,00844 | 0,000670388 |
| CRELD1 | 10338 | 5,8767 | 0,00413796 |
| CRNKL1 | 1430441 | 6,61176 | 0,00218871 |
| CROCC | 2970440 | 5,32901 | 0,00669484 |
| CRY2 | 1450082 | 5,64809 | 0,00505479 |
| CSNK1A1 | 4850092 | 7,76326 | 0,000823103 |
| CTBS | 110706 | 10,055 | 0,000125747 |
| CUGBP2 | 6110672 | 6,00801 | 0,00369023 |
| CUTA | 2690609 | 7,95362 | 0,000701805 |
| CYB5R4 | 3140039 | 11,3518 | 4,51E-05 |
| CYBASC3 | 1090048 | 6,00055 | 0,00371429 |
| CYP4F3 | 650164 | 9,98926 | 0,000132547 |
| CYSLTR1 | 4810204 | 10,482 | 8,94E-05 |
| DAZAP2 | 1740735 | 5,10312 | 0,00817801 |
| DCXR | 1410369 | 6,73417 | 0,00197038 |
| DDEF1 | 2760349 | 5,18007 | 0,00763827 |
| DDX21 | 6280474 | 5,0642 | 0,00846576 |
| DDX24 | 4280332 | 10,1077 | 0,000120548 |
| DDX39 | 160240 | 5,7799 | 0,00450336 |
| DEGS1 | 6510209 | 7,43248 | 0,00108747 |
| DERPC | 4290673 | 5,20676 | 0,00745972 |
| DFFA | 3520192 | 7,75351 | 0,000829862 |
| DGKA | 6550390 | 4,99306 | 0,00901882 |
| DHPS | 1850541 | 5,524 | 0,00563717 |
| DHPS | 1990390 | 5,00253 | 0,00894309 |
| DHRS9 | 6220450 | 9,8431 | 0,00014906 |
| DICER1 | 6510575 | 6,83755 | 0,00180341 |
| DIP2B | 3990671 | 7,04466 | 0,00151115 |
| DIRC2 | 6020575 | 7,58839 | 0,000953451 |
| DKFZP586D0919 | 4540301 | 7,77622 | 0,000814203 |
| DLX5 | 3360139 | 6,96721 | 0,0016143 |
| DNAJB1 | 2360092 | 6,13066 | 0,00331687 |
| DNAJC3 | 2760064 | 6,87348 | 0,00174883 |
| DNHD1 | 6110142 | 5,77563 | 0,00452023 |
| DPH2 | 2900524 | 6,59316 | 0,00222401 |
| DREV1 | 460142 | 5,28085 | 0,00698604 |
| DSC2 | 7650025 | 6,13809 | 0,00329555 |
| DSTN | 1340689 | 5,10841 | 0,00813973 |
| DTX3L | 2850100 | 7,97473 | 0,000689534 |
| E2F1 | 3940338 | 5,70275 | 0,00481815 |
| ECH1 | 770458 | 5,32501 | 0,00671855 |
| ECHS1 | 3840022 | 7,16026 | 0,00136961 |
| EEF2 | 2750626 | 4,97769 | 0,00914309 |
| EEF2K | 6280343 | 9,13925 | 0,000263638 |
| EFHC2 | 6270129 | 5,45153 | 0,00600869 |
| EIF2AK2 | 1190349 | 5,38063 | 0,00639646 |
| EIF2B1 | 2760563 | 8,06675 | 0,000638551 |
| EIF2C3 | 5860500 | 8,1139 | 0,000613945 |
| EIF3S2 | 7320576 | 7,05567 | 0,00149705 |
| EIF3S4 | 6290431 | 6,62095 | 0,00217151 |
| EIF3S6IP | 4180142 | 7,01892 | 0,00154466 |
| EIF3S7 | 2970468 | 5,19624 | 0,00752953 |
| EIF4A1 | 2970768 | 5,53526 | 0,0055816 |
| EIF4E3 | 1110600 | 12,366 | 2,06E-05 |
| ELF1 | 4250382 | 6,77049 | 0,00190998 |
| EML2 | 2030450 | 5,87838 | 0,00413188 |
| EPB41L2 | 1030189 | 5,59415 | 0,00529995 |
| EPC1 | 2680010 | 8,23987 | 0,000552857 |
| ERO1L | 5270563 | 5,6931 | 0,00485912 |
| EWSR1 | 4780743 | 6,62882 | 0,00215686 |
| EXOC6 | 1940543 | 5,496 | 0,00577784 |
| EXOC7 | 2260520 | 5,56387 | 0,00544292 |
| EXOSC10 | 5490142 | 7,26988 | 0,00124792 |
| EXOSC10 | 5130142 | 5,14398 | 0,0078867 |
| FAIM3 | 2760092 | 5,864 | 0,00418412 |
| FAM102B | 4390468 | 5,23116 | 0,00730015 |
| FAM38A | 2710253 | 4,98385 | 0,00909311 |
| FAM62A | 1110215 | 8,94141 | 0,00030992 |
| FAM91A1 | 4890681 | 5,95153 | 0,00387639 |
| FARS2 | 130403 | 7,40804 | 0,00111017 |
| FBXL13 | 5050653 | 4,97912 | 0,00913145 |
| FBXL15 | 3930687 | 5,67534 | 0,00493537 |
| FBXL20 | 3710484 | 5,53534 | 0,00558121 |
| FBXL5 | 2070377 | 6,25879 | 0,00296803 |
| FBXO11 | 990474 | 5,80831 | 0,0043928 |
| FBXO11 | 6180497 | 5,58782 | 0,00532953 |
| FBXO21 | 3400372 | 6,09855 | 0,00341072 |
| FBXO28 | 3870754 | 7,85148 | 0,000764424 |
| FCRL3 | 4590646 | 5,10631 | 0,00815493 |
| FEZ1 | 360343 | 5,56591 | 0,00543317 |
| FHL1 | 2320475 | 5,4267 | 0,00614167 |
| FU10099 | 7100291 | 6,79933 | 0,00186338 |
| FU10379 | 5080056 | 5,88741 | 0,00409942 |
| FU11795 | 4670056 | 5,4947 | 0,00578447 |
| FU20186 | 6940612 | 6,89405 | 0,00171836 |
| FU21127 | 3840221 | 5,18932 | 0,00757585 |
| FU32028 | 7650379 | 10,0375 | 0,000127518 |
| FU32154 | 3940368 | 6,8509 | 0,00178294 |
| FU33641 | 5340128 | 5,50724 | 0,00572096 |
| FU33790 | 620215 | 5,15728 | 0,00779422 |
| FU36268 | 2350372 | 5,37856 | 0,00640813 |
| FU38379 | 3870240 | 5,2842 | 0,00696541 |
| FLRT2 | 540358 | 6,58309 | 0,00224336 |
| FN3KRP | 6590386 | 9,18191 | 0,000254624 |
| FNBP1 | 1190470 | 5,4243 | 0,0061547 |
| FNBP1L | 2480255 | 5,58258 | 0,00535414 |
| FNDC3B | 870148 | 6,2997 | 0,00286474 |
| FOXO1A | 270754 | 5,28562 | 0,00695665 |
| FPR1 | 10343 | 5,01605 | 0,00883613 |
| FPRL1 | 3140114 | 5,70404 | 0,00481271 |
| FXYD5 | 6760487 | 5,05011 | 0,00857247 |
| FZD7 | 110343 | 5,83314 | 0,00429847 |
| GABARAPL1 | 2630154 | 8,59363 | 0,000412487 |
| GAGE8 | 6960450 | 5,66586 | 0,0049766 |
| GALNTL5 | 5670747 | 5,81459 | 0,00436875 |
| GALT | 520332 | 11,5689 | 3,81E-05 |
| GATA2 | 3990553 | 5,36069 | 0,00651006 |
| GCA | 940348 | 6,10606 | 0,00338854 |
| GCN5L2 | 130451 | 5,7371 | 0,00467531 |
| GEMIN4 | 4200538 | 6,79701 | 0,00186708 |
| GGA2 | 6270364 | 5,71835 | 0,00475272 |
| GIMAP5 | 6960746 | 8,71348 | 0,000373699 |
| GIMAP6 | 6590523 | 7,40118 | 0,00111662 |
| GIMAP8 | 4540487 | 5,07895 | 0,00835551 |
| GLTSCR2 | 3170092 | 6,54567 | 0,00231676 |
| GNA13 | 2340445 | 5,6436 | 0,00507475 |
| GNAI3 | 5810598 | 5,41555 | 0,00620237 |
| GNAQ | 4760095 | 6,48838 | 0,00243396 |
| GNB1 | 240554 | 5,80351 | 0,00441127 |
| GNB4 | 2470653 | 5,49845 | 0,00576541 |
| GNE | 3610504 | 11,7262 | 3,37E-05 |
| GOLGA3 | 7000041 | 5,16389 | 0,00774865 |
| GOT2 | 1440546 | 7,32571 | 0,00119026 |
| GPBAR1 | 5960035 | 7,31518 | 0,00120092 |
| GPR137B | 7150364 | 5,89303 | 0,00407938 |
| GPR160 | 6180427 | 8,92995 | 0,000312842 |
| GSTM3 | 3940386 | 5,02242 | 0,00878623 |
| GSTM4 | 1030070 | 5,43911 | 0,00607485 |
| GSTP1 | 5420538 | 5,19398 | 0,00754464 |
| GTDC1 | 1990450 | 7,09942 | 0,00144231 |
| H3F3A | 5890307 | 7,44334 | 0,00107754 |
| HAPLN3 | 5360674 | 6,28792 | 0,00289409 |
| HBP1 | 5890494 | 7,1897 | 0,00133577 |
| HDAC1 | 6940242 | 7,58873 | 0,000953175 |
| HELZ | 6290377 | 5,7103 | 0,00478638 |
| HIAT1 | 4060494 | 6,84822 | 0,00178702 |
| HIF1A | 2850288 | 7,36898 | 0,00114746 |
| HIST1H2AC | 4890192 | 7,57549 | 0,00096387 |
| HIST1H2AE | 5860400 | 10,1512 | 0,000116423 |
| HIST1H2BG | 630091 | 5,71961 | 0,0047475 |
| HIST1H2BJ | 5360504 | 5,23354 | 0,00728478 |
| HIST1H3D | 7380241 | 5,50599 | 0,00572724 |
| HIST1H4E | 1780113 | 5,11766 | 0,00807315 |
| HIST1H4H | 4730195 | 11,8236 | 3,12E-05 |
| HIST1H4K | 520097 | 5,95759 | 0,00385597 |
| HIST2H2AA | 1030039 | 5,80666 | 0,00439915 |
| HIST2H2AC | 5860075 | 5,64664 | 0,00506121 |
| HIST2H2BE | 2630451 | 8,31244 | 0,000520526 |
| HLA-DPA1 | 6480500 | 7,56623 | 0,000971415 |
| HLA-DQA1 | 6290561 | 7,38736 | 0,00112975 |
| HLA-DRA | 2680370 | 6,59614 | 0,00221832 |
| HMFN0839 | 7610546 | 6,3451 | 0,00275441 |
| HMG20A | 1710491 | 6,54741 | 0,0023133 |
| HMGB2 | 610563 | 8,05345 | 0,000645674 |
| HNRPF | 2060471 | 5,20123 | 0,00749634 |
| HNRPM | 6270021 | 7,61749 | 0,00093038 |
| HNRPU | 2710026 | 5,97508 | 0,00379761 |
| HPD | 5090554 | 5,83958 | 0,00427435 |
| HSD17B8 | 3130019 | 5,05429 | 0,00854066 |
| HSDL2 | 1340382 | 5,19002 | 0,00757116 |
| HSP90AB1 | 5130082 | 6,59784 | 0,00221508 |
| HSPA1L | 780255 | 6,9121 | 0,00169206 |
| HSPA8 | 1690189 | 7,92263 | 0,000720225 |
| HSPA8 | 6350376 | 5,50264 | 0,00574415 |
| HSPA9B | 4560497 | 5,0627 | 0,00847704 |
| HSPC159 | 4150768 | 6,57979 | 0,00224973 |
| HTATIP2 | 6620674 | 5,3885 | 0,00635215 |
| IBRDC2 | 5910037 | 5,87604 | 0,00414033 |
| ICA1 | 650735 | 5,22617 | 0,00733249 |
| ICAM2 | 1820598 | 8,63677 | 0,000398072 |
| IDH3B | 7380170 | 6,14118 | 0,0032867 |
| IFRD1 | 3780243 | 5,83685 | 0,00428454 |
| IGF2BP3 | 3360433 | 8,47558 | 0,000454733 |
| IGSF8 | 5690576 | 5,08579 | 0,00830487 |
| IL18R1 | 1500328 | 6,13016 | 0,00331834 |
| IL18RAP | 5130475 | 6,18189 | 0,0031726 |
| IL1RAP | 2360398 | 5,59758 | 0,00528402 |
| IL2RB | 1170307 | 6,34396 | 0,00275714 |
| ILF2 | 7400431 | 5,21115 | 0,00743074 |
| ILF3 | 2070494 | 6,00434 | 0,00370205 |
| IMP3 | 1780348 | 8,07729 | 0,000632967 |
| IMP4 | 6380598 | 8,31444 | 0,000519664 |
| IMPDH2 | 3400504 | 6,2529 | 0,0029832 |
| IPO7 | 510746 | 5,09435 | 0,00824198 |
| IRAK3 | 1430762 | 6,40092 | 0,00262472 |
| IRS2 | 6980095 | 5,24759 | 0,00719472 |
| ITCH | 7400369 | 5,17556 | 0,00766886 |
| ITGAM | 6660709 | 5,09625 | 0,0082281 |
| ITGAX | 1240603 | 9,72094 | 0,000164472 |
| ITM2B | 2760358 | 5,41472 | 0,00620692 |
| ITPR2 | 2850377 | 8,07145 | 0,000636057 |
| IVNS1ABP | 2100519 | 5,605 | 0,00524969 |
| JMJD1B | 4120681 | 4,981 | 0,00911615 |
| K-ALPHA-1 | 5900156 | 10,1324 | 0,000118184 |
| KARS | 5900414 | 7,19714 | 0,00132736 |
| KBTBD7 | 2030747 | 6,73079 | 0,0019761 |
| KCMF1 | 4730747 | 4,99997 | 0,00896348 |
| KCNJ15 | 3390458 | 5,79194 | 0,00445614 |
| KCTD17 | 7650605 | 5,68191 | 0,004907 |
| KIAA0174 | 3520168 | 6,5086 | 0,00239193 |
| KIAA0195 | 2190673 | 7,74333 | 0,000836989 |
| KIAA0232 | 1260156 | 5,7984 | 0,00443104 |
| KIAA0692 | 3830390 | 7,83562 | 0,000774646 |
| KIAA0701 | 4060056 | 6,08938 | 0,00343802 |
| KIAA0703 | 1300332 | 6,84384 | 0,00179373 |
| KIAA0859 | 5310754 | 8,57354 | 0,000419383 |
| KIAA0888 | 2490730 | 5,39891 | 0,00629409 |
| KIAA1267 | 2320280 | 5,01711 | 0,00882783 |
| KIAA1344 | 5260674 | 5,96177 | 0,00384194 |
| KIAA1600 | 1770598 | 5,1338 | 0,00795827 |
| KIAA1618 | 7200681 | 9,992 | 0,000132256 |
| KIAA1914 | 4040309 | 6,13932 | 0,00329203 |
| KIAA1961 | 940132 | 8,63697 | 0,000398007 |
| KIF1B | 610465 | 7,07252 | 0,00147572 |
| KLHL8 | 1010754 | 6,18385 | 0,00316722 |
| KPNA4 | 6560377 | 5,20852 | 0,00744804 |
| KREMEN1 | 1440612 | 6,52578 | 0,00235679 |
| KRTAP19-1 | 2710292 | 5,4295 | 0,00612654 |
| KRTAP19-6 | 1450561 | 5,05057 | 0,00856896 |
| L3MBTL2 | 3120301 | 8,99707 | 0,000296114 |
| L3MBTL3 | 5820025 | 5,72882 | 0,00470934 |
| LAMP2 | 3290162 | 10,6146 | 8,05E-05 |
| LARS | 1470762 | 5,0467 | 0,0085985 |
| LAS1L | 1010612 | 5,56266 | 0,00544873 |
| LAX1 | 7000768 | 7,67889 | 0,000883543 |
| LCK | 2230661 | 6,76237 | 0,00192332 |
| LFNG | 3890095 | 5,73123 | 0,0046994 |
| LGR6 | 4760364 | 5,44188 | 0,00606002 |
| LMBRD1 | 4590301 | 5,22412 | 0,00734584 |
| LMNB2 | 5550343 | 5,02955 | 0,00873068 |
| LOC133993 | 4200451 | 5,30894 | 0,00681462 |
| LOC153222 | 1450184 | 8,57901 | 0,000417494 |
| LOC284701 | 20068 | 6,06749 | 0,00350407 |
| LOC285636 | 7610168 | 5,30429 | 0,00684273 |
| LOC343384 | 840347 | 5,51636 | 0,00567521 |
| LOC348645 | 5910682 | 8,05779 | 0,000643342 |
| LOC374395 | 840730 | 6,20241 | 0,00311666 |
| LOC387841 | 3800253 | 5,25844 | 0,00712597 |
| LOC387867 | 1820692 | 5,38172 | 0,00639027 |
| LOC389833 | 6960328 | 6,89012 | 0,00172414 |
| LOC390378 | 6020341 | 5,50622 | 0,0057261 |
| LOC392364 | 770452 | 5,80681 | 0,00439858 |
| LOC400566 | 2570703 | 14,1926 | 5,20E-06 |
| LOC400566 | 3520685 | 7,68717 | 0,000877415 |
| LOC400793 | 3930221 | 5,06119 | 0,00848848 |
| LOC401284 | 3370402 | 8,65103 | 0,000393422 |
| LOC401957 | 2900019 | 5,74489 | 0,00464349 |
| LOC440261 | 3990465 | 6,76775 | 0,00191447 |
| LOC440503 | 940450 | 5,7466 | 0,00463656 |
| LOC441097 | 3800382 | 5,00666 | 0,00891032 |
| LOC51035 | 6480386 | 5,17889 | 0,00764629 |
| LOC51149 | 2750035 | 5,58142 | 0,00535958 |
| LOC57149 | 3830341 | 10,0669 | 0,000124551 |
| LOC642196 | 2030544 | 6,51072 | 0,00238756 |
| LOC642267 | 2970278 | 5,64252 | 0,00507955 |
| LOC642718 | 1940307 | 5,35587 | 0,00653784 |
| LOC642780 | 1580746 | 6,41772 | 0,00258692 |
| LOC642816 | 6110537 | 8,12818 | 0,000606685 |
| LOC642816 | 160458 | 5,29963 | 0,00687096 |
| LOC643060 | 2350121 | 5,3473 | 0,00658753 |
| LOC643300 | 770369 | 5,40606 | 0,00625448 |
| LOC643401 | 2370341 | 7,67203 | 0,000888651 |
| LOC643707 | 3130524 | 5,65439 | 0,00502693 |
| LOC644474 | 4850711 | 8,29745 | 0,000527041 |
| LOC644838 | 2260575 | 6,2576 | 0,00297107 |
| LOC645232 | 4570730 | 7,89079 | 0,000739672 |
| LOC646144 | 2750152 | 5,28085 | 0,00698607 |
| LOC646200 | 6650086 | 5,48605 | 0,00582867 |
| LOC646836 | 5910343 | 5,98222 | 0,00377408 |
| LOC646920 | 1470014 | 5,58907 | 0,00532368 |
| LOC647649 | 2810674 | 5,77173 | 0,00453567 |
| LOC647649 | 2600102 | 4,99758 | 0,00898262 |
| LOC647784 | 7050196 | 5,40878 | 0,00623948 |
| LOC647841 | 1450209 | 7,07418 | 0,00147364 |
| LOC648732 | 7210044 | 5,27582 | 0,00701725 |
| LOC649242 | 3440040 | 5,19455 | 0,00754085 |
| LOC649379 | 540131 | 5,1876 | 0,00758746 |
| LOC649461 | 4150711 | 5,1957 | 0,00753314 |
| LOC650058 | 940706 | 5,29434 | 0,00690319 |
| LOC650557 | 4590563 | 5,59735 | 0,00528509 |
| LOC650849 | 1780543 | 5,84948 | 0,00423752 |
| LOC651076 | 4220138 | 8,28662 | 0,000531802 |
| LOC651131 | 2470092 | 5,05558 | 0,00853092 |
| LOC652025 | 2320309 | 5,13843 | 0,00792564 |
| LOC652219 | 5670121 | 6,92696 | 0,00167072 |
| LOC652455 | 4060138 | 8,99784 | 0,000295927 |
| LOC652458 | 3390725 | 7,11098 | 0,0014282 |
| LOC652578 | 3180192 | 7,76558 | 0,000821499 |
| LOC652759 | 6520333 | 9,81806 | 0,000152094 |
| LOC653063 | 6370463 | 5,34234 | 0,00661643 |
| LOC653181 | 2850274 | 5,43219 | 0,00611199 |
| LOC653492 | 7100092 | 6,10568 | 0,00338965 |
| LOC653518 | 3800082 | 5,52039 | 0,00565514 |
| LOC653610 | 5670544 | 5,39034 | 0,00634187 |
| LOC653832 | 2260446 | 7,31857 | 0,00119748 |
| LOC654123 | 3170491 | 7,01523 | 0,00154953 |
| LOC654123 | 7160477 | 6,54947 | 0,0023092 |
| LOC654123 | 5220112 | 5,91646 | 0,00399679 |
| LOC654126 | 730148 | 6,81786 | 0,00183406 |
| LOC88523 | 3140246 | 6,58819 | 0,00223354 |
| LOC90355 | 10477 | 5,07193 | 0,00840782 |
| LPGAT1 | 870403 | 5,22463 | 0,00734255 |
| LPXN | 4060131 | 7,81314 | 0,000789378 |
| LRDD | 5050307 | 5,63142 | 0,00512928 |
| LRMP | 20553 | 5,26766 | 0,00706808 |
| LRRC42 | 2490397 | 5,32931 | 0,00669307 |
| LRRC4B | 2230019 | 5,35857 | 0,00652225 |
| LRRC8C | 3870102 | 5,62491 | 0,00515871 |
| LRRK2 | 1450523 | 5,57077 | 0,00540998 |
| LRRN1 | 3360156 | 5,28974 | 0,00693133 |
| LRRTM1 | 6980609 | 9,20039 | 0,000250818 |
| LSM4 | 4830563 | 6,26955 | 0,0029405 |
| LUM | 1780215 | 6,16715 | 0,00321345 |
| LXN | 3850669 | 5,5038 | 0,00573829 |
| LY9 | 450037 | 8,33037 | 0,00051284 |
| LY9 | 5310136 | 5,61446 | 0,00520625 |
| LYCAT | 2320241 | 5,8673 | 0,00417206 |
| LYSMD2 | 6550520 | 8,76911 | 0,000356986 |
| LZTR1 | 3140093 | 8,08612 | 0,000628324 |
| MAGED1 | 6480170 | 6,20557 | 0,00310812 |
| MAMDC1 | 670376 | 5,64168 | 0,00508328 |
| MAN1A1 | 4010110 | 5,16872 | 0,00771553 |
| MAN2A2 | 4570612 | 5,60761 | 0,00523765 |
| MAP2K3 | 4150632 | 5,06337 | 0,00847199 |
| MAP2K4 | 6350309 | 7,24078 | 0,00127909 |
| MAP3K4 | 7320594 | 5,47076 | 0,00590772 |
| MAP4K1 | 3420630 | 6,36405 | 0,00270966 |
| MAP7 | 60255 | 7,57404 | 0,000965048 |
| MAPK14 | 6280427 | 5,07097 | 0,00841495 |
| MAX | 1010102 | 5,99342 | 0,00373743 |
| MBP | 2600520 | 6,53858 | 0,00233096 |
| MCM3AP | 5390131 | 8,57304 | 0,000419553 |
| MFNG | 1710286 | 7,46419 | 0,00105873 |
| MGC15619 | 6400563 | 5,92712 | 0,0039598 |
| MGC17624 | 20224 | 6,06151 | 0,00352235 |
| MGC2474 | 4150435 | 5,28563 | 0,0069566 |
| MGC32020 | 7150189 | 5,29166 | 0,00691959 |
| MGC33887 | 1470332 | 5,25207 | 0,00716624 |
| MGC35048 | 7050240 | 5,3186 | 0,00675672 |
| MGC39518 | 5910730 | 5,39515 | 0,006315 |
| MGC57346 | 1340338 | 6,68821 | 0,00204961 |
| MIER1 | 5360575 | 6,61331 | 0,0021858 |
| MIF4GD | 6520241 | 6,53173 | 0,00234475 |
| MIZF | 3400176 | 5,09125 | 0,00826474 |
| MLL2 | 6510349 | 5,16978 | 0,00770831 |
| MLL5 | 4230253 | 9,58735 | 0,000183206 |
| MLLT6 | 2630719 | 6,59288 | 0,00222454 |
| MLR2 | 70022 | 11,0153 | 5,87E-05 |
| MMD | 360671 | 5,48456 | 0,00583633 |
| MME | 240608 | 5,78848 | 0,00446967 |
| MMP9 | 4150224 | 5,11823 | 0,00806906 |
| MNDA | 6380228 | 5,45004 | 0,00601659 |
| MORC2 | 3360364 | 8,66234 | 0,000389771 |
| MRPL34 | 6660253 | 5,09937 | 0,00820532 |
| MRPL44 | 7560014 | 6,03043 | 0,00361891 |
| MRPL49 | 1030692 | 6,7503 | 0,00194331 |
| MRPL9 | 2070131 | 5,46907 | 0,00591651 |
| MRPS26 | 4830435 | 6,08864 | 0,00344022 |
| MS4A2 | 6770427 | 5,60327 | 0,00525768 |
| MSRB3 | 4850414 | 7,72221 | 0,000851962 |
| MTMR11 | 7320195 | 5,19043 | 0,00756841 |
| MTPN | 4880670 | 5,80233 | 0,00441584 |
| MUM1 | 6040259 | 5,02876 | 0,00873684 |
| MUSTN1 | 1260487 | 5,02225 | 0,00878755 |
| MXD1 | 2260239 | 8,31951 | 0,000517481 |
| MYL9 | 4730114 | 5,37621 | 0,00642147 |
| MYLIP | 6370209 | 5,89208 | 0,00408274 |
| MYLK | 6350608 | 10,4778 | 8,97E-05 |
| MYO9A | 7150634 | 9,90968 | 0,000141291 |
| NAP1L4 | 2600286 | 5,30322 | 0,00684918 |
| NBN | 1030398 | 6,47215 | 0,00246826 |
| NCOA1 | 6760121 | 5,29553 | 0,00689592 |
| NDUFA1 | 150132 | 5,18493 | 0,00760541 |
| NDUFA10 | 6480603 | 7,21575 | 0,00130656 |
| NFE2L2 | 6580075 | 7,13461 | 0,00139978 |
| NFIL3 | 6100228 | 5,54302 | 0,00554365 |
| NFKB1 | 4810181 | 7,2131 | 0,00130949 |
| NIPA2 | 270093 | 5,35835 | 0,00652352 |
| NMNAT2 | 1580348 | 7,79232 | 0,000803283 |
| NOLA1 | 1430309 | 5,2716 | 0,00704349 |
| NOLA2 | 1510224 | 5,09396 | 0,00824483 |
| NOSIP | 380685 | 6,3308 | 0,00278869 |
| NOVA1 | 5490133 | 5,25178 | 0,0071681 |
| NPAL3 | 520360 | 5,23525 | 0,00727376 |
| NR2C2 | 5810326 | 6,02484 | 0,00363656 |
| NRBF2 | 5670133 | 5,00611 | 0,00891468 |
| NSUN5 | 5310270 | 5,39767 | 0,00630099 |
| NSUN5C | 2710711 | 5,94789 | 0,00388873 |
| NT5C2 | 520647 | 5,21729 | 0,00739044 |
| NT5C3 | 3780689 | 13,8534 | 6,69E-06 |
| NUBPL | 3840131 | 5,61263 | 0,00521463 |
| NUFIP2 | 5260091 | 6,42347 | 0,00257413 |
| NUMB | 7210692 | 5,65077 | 0,00504289 |
| NUP153 | 1050711 | 5,56317 | 0,00544627 |
| NUP205 | 2750521 | 7,4387 | 0,00108177 |
| NUP210 | 6020500 | 5,6771 | 0,00492774 |
| NUP214 | 730180 | 5,53384 | 0,00558861 |
| NUP43 | 670487 | 5,47064 | 0,00590833 |
| NUP62 | 4760543 | 9,01365 | 0,000292124 |
| NUP85 | 2510132 | 5,30155 | 0,00685933 |
| NUP93 | 50164 | 6,08068 | 0,0034641 |
| OGFOD1 | 2750242 | 5,34015 | 0,00662925 |
| OPLAH | 5820348 | 7,25434 | 0,00126447 |
| OR2D2 | 1500176 | 7,23711 | 0,00128309 |
| OSBP | 6760441 | 9,34991 | 0,000222086 |
| OSTM1 | 6860376 | 5,32043 | 0,00674579 |
| OTUD1 | 5490064 | 8,75379 | 0,000361512 |
| P15RS | 4390768 | 5,23825 | 0,00725449 |
| P2RX4 | 2060332 | 5,08025 | 0,00834588 |
| P2RY11 | 7330487 | 5,78673 | 0,00447653 |
| P2RY2 | 5900446 | 5,10762 | 0,00814544 |
| PABPC4 | 6550142 | 6,71407 | 0,00200464 |
| PADI2 | 6110133 | 5,85279 | 0,00422528 |
| PADI4 | 5310653 | 7,07126 | 0,00147731 |
| PAK2 | 2060279 | 10,9714 | 6,07E-05 |
| PAK2 | 4060722 | 6,38767 | 0,00265494 |
| PBEF1 | 3800243 | 6,95946 | 0,00162501 |
| PCDHGB7 | 1190139 | 5,09031 | 0,00827161 |
| PCNT2 | 2480082 | 5,33655 | 0,00665035 |
| PCSK2 | 7150273 | 5,93808 | 0,00392212 |
| PCSK7 | 1400270 | 6,42419 | 0,00257251 |
| PDCD11 | 7160296 | 8,17861 | 0,000581747 |
| PDE5A | 6940524 | 10,1285 | 0,000118562 |
| PDLIM5 | 520730 | 7,30039 | 0,00121606 |
| PDLIM7 | 2680682 | 5,1749 | 0,00767336 |
| PDZD8 | 5720398 | 7,77266 | 0,000816636 |
| PELI1 | 1780672 | 6,67833 | 0,00206706 |
| PELI2 | 270717 | 9,87636 | 0,000145125 |
| PEX19 | 1450414 | 6,11524 | 0,0033616 |
| PFTK1 | 2000390 | 8,68448 | 0,000382729 |
| PGK1 | 6980129 | 7,83973 | 0,000771983 |
| PGM2 | 2710528 | 5,6691 | 0,00496244 |
| PHF10 | 4260053 | 5,51189 | 0,00569759 |
| PHF15 | 3420735 | 6,81634 | 0,00183644 |
| PHF19 | 4540082 | 8,35459 | 0,000502643 |
| PHF20L1 | 430246 | 5,08044 | 0,00834448 |
| PHTF1 | 1070189 | 4,97601 | 0,00915682 |
| PIGR | 6940333 | 5,51367 | 0,00568866 |
| PIP5K2B | 5090477 | 12,1358 | 2,45E-05 |
| PITPNB | 2100615 | 5,9563 | 0,00386031 |
| PLOD2 | 3710228 | 5,08258 | 0,00832865 |
| PLP2 | 2320717 | 6,91779 | 0,00168386 |
| PLSCR1 | 1260228 | 6,81029 | 0,00184597 |
| PMS2CL | 5560484 | 5,15595 | 0,0078034 |
| PMVK | 3460242 | 5,34356 | 0,00660932 |
| PNPO | 3780220 | 5,54949 | 0,00551216 |
| POLE3 | 4260154 | 5,23073 | 0,00730295 |
| POMT1 | 4880681 | 5,2558 | 0,00714264 |
| PPBP | 6350364 | 7,50401 | 0,00102374 |
| PPP1R16B | 6040196 | 5,18776 | 0,00758634 |
| PPP2R5A | 650767 | 8,71872 | 0,000372093 |
| PPP4R1 | 610408 | 5,77944 | 0,00450515 |
| PPP6C | 4040278 | 5,15807 | 0,00778875 |
| PPRC1 | 20647 | 7,34854 | 0,00116747 |
| PRG1 | 650541 | 6,42167 | 0,00257811 |
| PRKAR1A | 4260035 | 5,05363 | 0,00854571 |
| PRKCB1 | 4070215 | 7,50526 | 0,00102266 |
| PRO0149 | 4230463 | 6,1469 | 0,00327043 |
| PROSC | 3990176 | 8,25426 | 0,000546284 |
| PRPF8 | 4590082 | 5,72624 | 0,00472001 |
| PRPS2 | 360685 | 5,56784 | 0,00542396 |
| PRR3 | 7000408 | 7,18471 | 0,00134144 |
| PRRG4 | 6980100 | 5,24229 | 0,00722857 |
| PRSS12 | 1580168 | 9,32773 | 0,000226126 |
| PRSS15 | 1820341 | 5,02946 | 0,00873134 |
| PRUNE | 4560039 | 11,4293 | 4,24E-05 |
| PSD3 | 650059 | 5,60351 | 0,00525656 |
| PSMD2 | 5720497 | 6,50393 | 0,00240156 |
| PSRC2 | 5700164 | 6,32396 | 0,00280522 |
| PTEN | 1500717 | 5,95378 | 0,00386878 |
| PTPLAD1 | 1110110 | 6,42377 | 0,00257344 |
| PTPN1 | 2760603 | 6,12264 | 0,00334007 |
| PTPRC | 2570379 | 5,50824 | 0,00571592 |
| PUM2 | 2490037 | 7,03768 | 0,00152017 |
| PURA | 2360367 | 6,13054 | 0,00331722 |
| QKI | 6660097 | 10,4177 | 9,41E-05 |
| QPCT | 4780672 | 6,79636 | 0,00186812 |
| RAB22A | 6400372 | 6,32792 | 0,00279564 |
| RAB31 | 7570603 | 9,73654 | 0,000162417 |
| RAB3GAP2 | 6400292 | 7,04517 | 0,00151049 |
| RAB9B | 1580626 | 7,42679 | 0,00109271 |
| RAD50 | 7100059 | 5,58997 | 0,00531948 |
| RAG2 | 150100 | 5,03447 | 0,00869253 |
| RAMP2 | 6620612 | 6,77163 | 0,00190812 |
| RANGAP1 | 3710189 | 5,68851 | 0,0048787 |
| RAP1A | 3060692 | 5,33052 | 0,0066859 |
| RARRES3 | 5720458 | 4,98807 | 0,00905894 |
| RARS | 7150739 | 5,13801 | 0,00792862 |
| RASSF3 | 7160494 | 6,25654 | 0,00297381 |
| RBBP5 | 1740133 | 5,94426 | 0,00390105 |
| RBM14 | 2970332 | 6,40973 | 0,00260482 |
| RBM21 | 360402 | 5,68763 | 0,00488246 |
| RBM4 | 620722 | 7,14981 | 0,00138182 |
| RBM4 | 3990072 | 5,3392 | 0,0066348 |
| RBMX | 5690673 | 6,09306 | 0,00342704 |
| RCC2 | 510450 | 11,1079 | 5,46E-05 |
| REPS1 | 3420725 | 5,11173 | 0,00811578 |
| REPS2 | 6590349 | 5,40985 | 0,0062336 |
| RFC5 | 730592 | 5,05912 | 0,00850412 |
| RFFL | 5870551 | 6,14532 | 0,00327494 |
| RFWD2 | 3870543 | 5,3497 | 0,00657357 |
| RFX4 | 3120181 | 5,07575 | 0,00837933 |
| RFX5 | 2640373 | 5,78564 | 0,00448078 |
| RINT-1 | 50709 | 6,25339 | 0,00298192 |
| RIPK2 | 5690093 | 6,77601 | 0,00190097 |
| RNASEL | 4180079 | 5,13886 | 0,00792264 |
| RNF122 | 5900333 | 5,2518 | 0,00716798 |
| RNF13 | 4280047 | 7,83493 | 0,000775091 |
| RNF149 | 10082 | 6,99312 | 0,00157901 |
| RNF38 | 6220022 | 8,75023 | 0,000362573 |
| ROCK2 | 50521 | 7,68464 | 0,000879281 |
| RPAP1 | 6860243 | 6,53879 | 0,00233053 |
| RPL8 | 6380148 | 5,57074 | 0,00541014 |
| RPS5 | 4280326 | 5,00491 | 0,00892418 |
| RPS6KA5 | 2030482 | 9,75937 | 0,000159455 |
| RPUSD3 | 1990673 | 6,11708 | 0,00335624 |
| RRM2B | 5390100 | 6,63373 | 0,00214778 |
| RSBN1L | 6420692 | 8,16086 | 0,000590399 |
| RTN3 | 4280463 | 5,50049 | 0,00575505 |
| RUNX1 | 2100427 | 7,98415 | 0,000684129 |
| RUTBC3 | 2690576 | 7,20305 | 0,00132072 |
| RUVBL1 | 3520082 | 9,37607 | 0,000217416 |
| RUVBL1 | 2750408 | 6,86356 | 0,00176373 |
| S100A8 | 6280576 | 6,21243 | 0,00308969 |
| S100P | 2640609 | 5,18716 | 0,00759042 |
| SAE1 | 5690008 | 5,52614 | 0,00562656 |
| SAMHD1 | 7320047 | 8,26185 | 0,000542851 |
| SAMM50 | 990273 | 6,25095 | 0,00298823 |
| SAMSN1 | 150632 | 7,20813 | 0,00131504 |
| SAP30 | 2510133 | 6,81248 | 0,00184252 |
| SCAMP3 | 3370687 | 6,69057 | 0,00204547 |
| SDCCAG3 | 1340731 | 6,01652 | 0,00366301 |
| SDHD | 6650754 | 5,08243 | 0,00832975 |
| SEC31L1 | 6040037 | 7,76422 | 0,00082244 |
| SEC31L2 | 4010673 | 5,57648 | 0,0053829 |
| SEH1L | 430142 | 7,53442 | 0,000997814 |
| SEL1L | 4280661 | 5,5022 | 0,00574641 |
| SERPINC1 | 7400240 | 6,84944 | 0,00178516 |
| SF3B3 | 160682 | 5,74597 | 0,00463913 |
| SFRS15 | 7320273 | 5,89798 | 0,00406178 |
| SHMT2 | 2710278 | 6,02934 | 0,00362234 |
| SIAHBP1 | 4900053 | 9,06693 | 0,000279673 |
| SIGIRR | 7380328 | 6,31133 | 0,00283606 |
| SIPA1L2 | 3370605 | 5,96247 | 0,00383959 |
| SIRPB2 | 6280754 | 7,50861 | 0,00101977 |
| SLC10A5 | 840332 | 5,5181 | 0,00566652 |
| SLC11A1 | 1430292 | 8,23133 | 0,000556792 |
| SLC17A5 | 1570543 | 6,16491 | 0,00321972 |
| SLC22A4 | 2710397 | 6,02835 | 0,00362547 |
| SLC24A5 | 1660392 | 5,38386 | 0,00637822 |
| SLC25A25 | 130113 | 8,24117 | 0,00055226 |
| SLC25A3 | 4050398 | 6,23269 | 0,00303589 |
| SLC25A5 | 7550537 | 10,638 | 7,90E-05 |
| SLC27A2 | 6110328 | 6,18193 | 0,0031725 |
| SLC2A11 | 2750091 | 6,6354 | 0,00214469 |
| SLC36A1 | 7100136 | 8,76079 | 0,000359438 |
| SLC36A4 | 2350195 | 5,00374 | 0,00893352 |
| SLC37A3 | 2230008 | 4,99148 | 0,00903155 |
| SLC39A1 | 2630400 | 5,44178 | 0,00606057 |
| SLC40A1 | 840427 | 6,31551 | 0,00282581 |
| SLC7A6 | 2480402 | 5,78558 | 0,00448103 |
| SLC9A3R1 | 6060324 | 6,16182 | 0,00322836 |
| SMAD3 | 5130767 | 6,04884 | 0,00356138 |
| SMAP1 | 4040747 | 5,04835 | 0,00858592 |
| SMARCA3 | 7380576 | 5,29651 | 0,00688997 |
| SMC1L1 | 1500040 | 5,89165 | 0,00408427 |
| SMCHD1 | 5700136 | 6,32478 | 0,00280324 |
| SMOC1 | 7100685 | 6,5461 | 0,00231591 |
| SNRP70 | 2070468 | 5,18375 | 0,00761339 |
| SOD1 | 2120324 | 5,66781 | 0,00496808 |
| SPAG9 | 4290477 | 8,74933 | 0,000362839 |
| SPAG9 | 380541 | 6,49845 | 0,00241294 |
| SPAST | 4830082 | 6,1523 | 0,00325515 |
| SPATA20 | 4120133 | 7,02394 | 0,00153806 |
| SPTBN1 | 4480091 | 6,5738 | 0,00226136 |
| SRP68 | 6020402 | 7,87443 | 0,000749867 |
| SRPK1 | 3460674 | 11,5415 | 3,89E-05 |
| SRRM1 | 290707 | 7,63769 | 0,000914697 |
| SSR2 | 2650240 | 5,6578 | 0,00501189 |
| SSX2 | 4120088 | 5,46733 | 0,00592563 |
| STK17B | 2100035 | 9,32157 | 0,00022726 |
| STK25 | 1820142 | 5,31426 | 0,00678266 |
| STK4 | 2680209 | 7,26555 | 0,00125251 |
| STX3A | 3290192 | 5,22428 | 0,0073448 |
| STXBP5 | 3460189 | 9,56076 | 0,000187189 |
| SVIL | 4280373 | 8,94209 | 0,000309748 |
| SYT17 | 730725 | 5,49774 | 0,00576898 |
| TACC1 | 1050605 | 6,76996 | 0,00191084 |
| TAF15 | 5960128 | 9,53807 | 0,000190658 |
| TAF1C | 3850025 | 5,70477 | 0,00480966 |
| TARSL1 | 6480328 | 6,05332 | 0,00354754 |
| TDRD7 | 7200682 | 6,13568 | 0,00330244 |
| TFEC | 990377 | 5,20654 | 0,00746112 |
| TFF3 | 5550224 | 4,99123 | 0,00903355 |
| TGIF2 | 4850438 | 5,97846 | 0,00378645 |
| THBD | 5490348 | 6,35169 | 0,00273877 |
| TIPARP | 5720681 | 8,71399 | 0,000373542 |
| TLE4 | 6290170 | 7,77483 | 0,000815154 |
| TLN2 | 6520086 | 6,18316 | 0,00316913 |
| TLR4 | 4390615 | 6,55034 | 0,00230748 |
| TLR8 | 6550307 | 6,25081 | 0,0029886 |
| TLR8 | 510338 | 5,21895 | 0,00737959 |
| TM6SF1 | 10541 | 9,23945 | 0,000242963 |
| TM9SF2 | 2810110 | 5,11562 | 0,00808776 |
| TMCC3 | 2650152 | 6,73706 | 0,00196549 |
| TMCO3 | 3130091 | 5,70812 | 0,00479554 |
| TMED2 | 7560445 | 6,6213 | 0,00217084 |
| TMED7 | 4230504 | 6,34151 | 0,00276298 |
| TMEM109 | 4880364 | 6,8448 | 0,00179226 |
| TMEM127 | 670079 | 5,17027 | 0,00770495 |
| TMEM49 | 4010358 | 5,21371 | 0,00741391 |
| TMEM87B | 7320669 | 6,27742 | 0,00292052 |
| TMEM99 | 1090041 | 5,36207 | 0,00650214 |
| TNFAIP6 | 2370524 | 19,2839 | 1,41E-07 |
| TNFRSF10A | 4150739 | 5,42505 | 0,00615059 |
| TNFRSF10B | 6450767 | 6,74205 | 0,0019571 |
| TNFSF13B | 460608 | 7,80431 | 0,000795241 |
| TNFSF4 | 1440341 | 7,43781 | 0,00108258 |
| TNRC6B | 2750386 | 6,77666 | 0,00189992 |
| TOR1AIP1 | 3180041 | 8,12925 | 0,000606148 |
| TRA16 | 6650541 | 5,54381 | 0,00553978 |
| TRAF3IP3 | 4640528 | 6,8101 | 0,00184628 |
| TRAP1 | 160736 | 7,4632 | 0,00105962 |
| TRAPPC6A | 1980424 | 5,75576 | 0,00459954 |
| TRFP | 460524 | 5,09703 | 0,00822237 |
| TRIAD3 | 2190524 | 5,81043 | 0,00438465 |
| TRIB1 | 2710044 | 5,41006 | 0,00623249 |
| TRIM25 | 2850576 | 5,54455 | 0,00553618 |
| TSP50 | 5690037 | 10,1995 | 0,000112016 |
| TSPAN2 | 1770131 | 10,7267 | 7,37E-05 |
| TTC4 | 7100504 | 5,24019 | 0,00724201 |
| TXNDC13 | 1940259 | 6,28066 | 0,00291234 |
| TXNDC14 | 380315 | 5,8647 | 0,00418154 |
| TXNRD1 | 7050372 | 5,15744 | 0,00779313 |
| TYRP1 | 3360491 | 5,46528 | 0,00593633 |
| UBC | 4780609 | 5,79074 | 0,00446082 |
| UBE2C | 2450603 | 6,82453 | 0,00182361 |
| UBE2G1 | 430382 | 12,9455 | 1,32E-05 |
| UBE2G2 | 1440382 | 6,41917 | 0,00258368 |
| UBE21 | 460273 | 6,89336 | 0,00171937 |
| UBE2J1 | 3840446 | 5,22299 | 0,00735319 |
| UBE2Z | 2510639 | 9,21474 | 0,000247902 |
| UBE2Z | 7160767 | 8,12999 | 0,000605771 |
| UBL3 | 130609 | 6,5499 | 0,00230835 |
| UBLCP1 | 1500202 | 6,45134 | 0,00251296 |
| UBQLN3 | 7100392 | 6,00542 | 0,00369856 |
| UBQLN4 | 990224 | 5,79508 | 0,00444393 |
| UGCGL1 | 7210372 | 13,0538 | 1,22E-05 |
| UIP1 | 1070377 | 5,01976 | 0,00880705 |
| UMPS | 3990196 | 6,2988 | 0,00286697 |
| UNC84B | 5570750 | 5,13179 | 0,00797253 |
| USP10 | 1980021 | 5,78018 | 0,00450224 |
| USP3 | 7570112 | 5,50183 | 0,00574825 |
| USP37 | 6840646 | 5,38618 | 0,00636519 |
| USP52 | 1740576 | 6,06563 | 0,00350976 |
| USP8 | 270750 | 6,47431 | 0,00246367 |
| USP9X | 2680064 | 7,11503 | 0,00142329 |
| UTX | 270731 | 6,79812 | 0,00186531 |
| VCL | 6840039 | 5,52267 | 0,00564376 |
| VDAC2 | 1770379 | 7,37165 | 0,00114487 |
| VPREB3 | 360066 | 5,35198 | 0,00656035 |
| VPS11 | 6330634 | 5,1734 | 0,00768355 |
| VPS13A | 6900392 | 5,69895 | 0,00483424 |
| WDFY3 | 5360349 | 5,3433 | 0,00661081 |
| WDR54 | 5700403 | 7,0291 | 0,00153132 |
| WDR6 | 5900021 | 5,61681 | 0,00519549 |
| WHSC1 | 7100520 | 9,59956 | 0,000181408 |
| WNT3A | 270402 | 7,20424 | 0,00131938 |
| WRB | 6420138 | 5,939 | 0,00391897 |
| WSB1 | 5260673 | 5,23028 | 0,00730589 |
| WWP2 | 1190100 | 6,28449 | 0,00290269 |
| XPO4 | 870370 | 7,93163 | 0,000714824 |
| XPO5 | 3130711 | 6,49421 | 0,00242177 |
| XPR1 | 5910093 | 5,2559 | 0,007142 |
| XTP3TPA | 1430156 | 6,17176 | 0,00320062 |
| YARS2 | 1010341 | 5,217 | 0,00739229 |
| YIPF4 | 5290289 | 8,3261 | 0,000514659 |
| YTHDF3 | 1400484 | 5,00651 | 0,00891149 |
| YWHAZ | 7210056 | 6,06452 | 0,00351313 |
| ZBTB24 | 6660689 | 6,16942 | 0,00320714 |
| ZBTB34 | 4070286 | 5,63575 | 0,00510983 |
| ZBTB40 | 6380687 | 5,44163 | 0,00606134 |
| ZBTB9 | 3290019 | 6,02095 | 0,00364891 |
| ZFP91 | 2450064 | 9,33947 | 0,000223977 |
| ZFYVE20 | 4050273 | 5,15079 | 0,0078392 |
| ZMPSTE24 | 5490408 | 5,82224 | 0,00433961 |
| ZMYM6 | 7380274 | 8,67458 | 0,000385862 |
| ZNF161 | 6960390 | 6,82473 | 0,00182331 |
| ZNF200 | 6290458 | 9,18631 | 0,000253711 |
| ZNF207 | 4230373 | 5,54984 | 0,00551046 |
| ZNF268 | 6020132 | 5,41912 | 0,00618285 |
| ZNF313 | 4490747 | 6,13144 | 0,00331463 |
| ZNF416 | 6250047 | 5,63131 | 0,0051298 |
| ZNF589 | 3170468 | 6,04231 | 0,00358169 |
| ZNF599 | 150360 | 5,5351 | 0,00558241 |
| ZNF654 | 6200563 | 9,10959 | 0,000270097 |
| ZNF654 | 2060370 | 5,56102 | 0,00545658 |
| ZNF740 | 4920575 | 7,9771 | 0,000688171 |

**Table 6:**

| Probe set | gene name |
|---|---|
| 3780689 | NT5C3 |
| 3830341 | LYRM1 |
| 2370524 | TNFAIP6 |
| 7200681 | XM_941239.1 |
| AUC = 0.8, n=4 | |
| | |
| 3780689 | NT5C3 |
| 1110600 | EIF4E3 |
| 430382 | UBE2G1 |
| 2370524 | TNFAIP6 |
| 3830341 | LYRM1 |
| 1770131 | TSPAN2 |
| 3360433 | IGF2BP3 |
| 2570703 | LOC400566 |
| 3460674 | SRPK1 |
| AUC = 0.82, n=9 | |
| | |
| 3780689 | NT5C3 |
| 1110600 | EIF4E3 |
| 430382 | UBE2G1 |
| 2370524 | TNFAIP6 |
| 3830341 | LYRM1 |
| 1770131 | TSPAN2 |
| 3360433 | IGF2BP3 |
| 2570703 | LOC400566 |
| 3460674 | SRPK1 |
| 6180427 | GPR160 |
| AUC = 0.85, n=10 | |
| | |
| 3780689 | NT5C3 |
| 3830341 | LYRM1 |
| 2370524 | TNFAIP6 |
| 7200681 | XM_941239.1 |
| 6180427 | GPR160 |
| 6200563 | ZNF654 |
| 3140039 | CYB5R4 |
| 430382 | UBE2G1 |
| 5490064 | OTUD |
| 4290477 | SPAG9 |
| 6550520 | LYSMD2 |
| 3460189 | STXBP5 |
| 4280332 | DDX24 |
| AUC = 0.9, n=13 | |
| | |
| 3780689 | NT5C3 |
| 3830341 | LYRM1 |
| 2370524 | TNFAIP6 |
| 7200681 | NA |
| 6180427 | GPR160 |
| 6200563 | ZNF654 |
| 3140039 | CYB5R4 |
| 430382 | UBE2G1 |
| 5490064 | NA |
| 4290477 | NA |
| 6550520 | LYSMD2 |
| 3460189 | STXBP5 |
| 4280332 | DDX24 |
| 2570703 | LOC400566 |
| 3610504 | GNE |
| 270717 | PELI2 |
| 3180041 | TOR1AIP1 |
| 520332 | GALT |
| 2850100 | DTX3L |
| 4730195 | HIST1H4H |
| 3360433 | IGF2BP3 |
| 6420692 | RSBN1L |
| 6220450 | DHRS9 |
| 4060138 | NA |
| 7040187 | ARRDC4 |
| 5860500 | EIF2C3 |
| 460608 | TNFSF13B |
| 5860400 | HIST1H2AE |
| 3460674 | SRPK1 |
| AUC = 0.9, n=29 | |

### Examples

### Material and Methods

### Cases and controls

Lung cancer cases and controls were recruited at the University Hospital Cologne and the Lung Clinic Merheim, Cologne, Germany. Prevalent lung cancer cases and controls were recruited in two hospitals in Cologne, Germany (University Hospital Cologne, Lung Clinic Merheim) within two genetic-epidemiological case control trials (Lung Cancer Study (LuCS) and Cologne Smoking Study (CoSmoS)). A case was defined by the pathological diagnosis of non-small-cell lung cancer or small-cell lung cancer by histology or cytology. A control was defined by the absence of lung cancer at any time-point of the patient's history. Individuals were not accepted as controls if they actually suffered from a cancer of the upper respiratory tract, the upper gastrointestinal tract or the urogenital system, since smoking represents a risk factor for the development of these cancer entities. An individual was not accepted for the control group if the reason for admission was an acute exacerbation of a chronic obstructive pulmonary disease or an acute cardiovascular event (heart attack, cerebral ischemia). These exclusion criteria were due to the simultaneous analysis of risk factors for acute cardiovascular events in this epidemiological study.

Lung cancer cases were primarily recruited in the Department of Haematology and Oncology (Department I for Internal Medicine, University Hospital Cologne) and in the Department of Thoracic Surgery (Lung Clinic Merheim). In order to recruit individuals with comparable comorbidity, the inventors used in-patient controls that were primarily recruited in the Department of Dermatology and Venerology and in the Department of Orthopaedics and Trauma Surgery at the University Hospital Cologne. Comorbidity of cases and controls was assessed using the medical records of the patients without performing additional examinations. Overall, the median age in this study was 65.74 years for the lung cancer patients and 63.92 years for the controls, respectively.

Initially, PAXgene stabilized blood samples from two independent groups of prevalent lung cancer cases and controls (prevalent groups; PG1: n = 84, PG2: n = 24) were used to establish and validate a lung cancer specific classifier. Blood was taken prior chemotherapy in all patients. Matching was performed for age (+/- 5 years), gender and pack years (+/- 5) (**Tables 1 and 2**). An additional prevalent group of cases and controls (PG3, n = 43) was built without matching and used for further validation of the classifier. Analyses were approved by the local ethics committee and all probands gave informed consent (**Tables 1 and 2**). Overall, in the group of controls, the inventors recruited 12 individuals suffering from advanced chronic obstructive lung disease as typically seen in a population of heavily smoking adults. Other diseases such as hypertension (n=28) or cardiac diseases (n=6) were observed in the control group. The inventors further included patients with other malignancies (n=13) (skin =10, prostate=2, brain=1). The mean age was 60 for the individuals without lung cancer and 62 for those with lung cancer, respectively (T test: p=0.12).

### Blood collection and cRNA synthesis and array hybridization

2.5 ml blood were drawn into PAXgene vials. After RNA isolation biotin labeled cRNA preparation was performed using the Ambion^{®} Illumina RNA amplification kit (Ambion, UK) and Biotin-16-UTP (10 mmol/l; Roche Molecular Biochemicals) or Illumina^{®} TotalPrep RNA Amplification Kit (Ambion, UK). 1.5 µg of biotin labeled cRNA was hybridized to Sentrix^{®} whole genome bead chips WG6 version 2, (Illumina, USA) and scanned on the Illumina^{®} BeadStation 500x. For data collection, the inventors used Illumina^{®} BeadStudio 3.1.1.0 software. Data are available at http://www.ncbi.nlm.nih.gov/geo/GSE12771).

### Quality control

For RNA quality control, the ratio of the OD at wavelengths of 260 nm and 280 nm was calculated and only samples with an OD between 1.85 and 2.1 were further processed. To determine the quality of cRNA, a semi-quantitative RT-PCR amplifying a 5'prime and a 3'prime product of the β-actin gene was used as previously described (Zander T, Yunes JA, Cardoso AA, Nadler LM. Rapid, reliable and inexpensive quality assessment of biotinylated cRNA. Braz J Med Biol Res 2006;39: 589-93). Quality of RNA expression data was controlled by different separate tools. First, the inventors performed quality control by visual inspection of the distribution of raw expression values. Therefore, the inventors constructed pairwise scatterplots of expression values from all arrays (R-project Vs 2.8.0) (Team RDC. R: A language and environment for statistical computing. R Foundation for Statistical Computing, 2006.). For data derived from an array of good quality a high correlation of expression values is expected leading to a cloud of dots along the diagonal. Secondly, the inventors calculated the present call rate. Finally, the inventors performed quantitative quality control. Here, the absolute deviation of the mean expression values of each array from the overall mean was determined (R-project Vs 2.8.0) (Team RDC. R: A language and environment for statistical computing. R Foundation for Statistical Computing, 2006). In short, the mean expression value for each array was calculated. Next the mean of these mean expression values (overall mean) was taken and the deviation of each array mean from the overall mean was determined (analogous to probe outlier detection used by Affymetrix before expression value calculation) (Affymetrix. Statistical algorithms description document. 2002; http://www.affymetrix.com/support/technical/whitepapers/sadd_whitepaper.pdf.). Arrays were only included in the study, if all three quality control methods confirmed sufficient quality. Two samples did not pass these quality controls (e.g. **Fig. 6**).

### Classification algorithm

Expression values were independently quantile normalized. A classifier for lung cancer was built using the following machine learning algorithms: support vector machine (SVM), linear discrimination analysis (LDA), and prediction analysis for microarrays (PAM) using a 10-fold cross-validation design as described below. A schematic view of this approach is depicted in Figure 1. Eighty-four samples were used in the training set (**Fig. 1A**). In the 10-fold cross-validation, the inventors randomly split the training group 10 times in a ratio 9:1. Differentially expressed transcripts between non-small-cell lung cancer, small-cell lung cancer and controls were identified using F-statistics (ANOVA) for each data set splitting in the larger data set split. Thirty six different feature lists were obtained as input for the classifier by sequentially increasing the cut-off value for the F-statistics (p = 0.00001, p = 0.00002, p = 0.00003 ...-...p = 0.08, p = 0.09, p = 0.1). The maximum feature size was restricted to 5 times the sample size to control for overfitting in this step (**Fig. 1B**) (Allison DB, Cui X, Page GP, Sabripour M. Microarray data analysis: from disarray to consolidation and consensus. Nat Rev Genet 2006;7: 55-65). These selected features were used as input for each of the three machine learning algorithms (LDA, PAM, SVM). The optimal cut-off of the F-statistics and the optimal classification algorithm were selected according to the mean area under the receiver operator curve in this 10-fold cross-validation design in the training group (**Fig. 1B**). The inventors subsequently built a classifier using this cut-off value of the F-statistics and the selected algorithm in the whole prevalent training group (PG1). To further control for overfitting (Lee S. Mistakes in validating the accuracy of a prediction classifier in high-dimensional but small-sample microarray data. Stat Methods Med Res 2008;17: 635-42), the classifier was validated in an independent group of matched cases and controls (PG2) (**Fig. 1C**). The area under the receiver operator curve was used to measure the quality of the classifier. Sensitivity and specificity were calculated at the maximum Youden-index (sensitivity + specificity - 1) within the SVM probability range from 0.1-0.9. In addition, the inventors analyzed the single SVM probabilities for each case. To test the specificity of the classifier the whole analysis was repeated thousand times using random feature sets of equal size (**Fig. 1D**). A second validation group (PG3) was additionally used (**Fig. 1E**).

### Computational data analysis:

*Cross-validation:* For 10-fold cross-validation the whole initial training group (PG1) was split 10 times in a ratio of 9:1 into an internal cross-validation training and validation group. Each sample was used only once for each internal validation group. As the number of samples is discrete, the inventors generated 6 internal validation sets with 8 samples and 4 validation sets with 9 samples. The calculation of the F-statistics was performed separately for each internal data set splitting. Based on the identified differentially expressed genes a classifier was built for each internal data set splitting and applied to the remaining internal validation group. For each internal validation group the given SVM scores of samples were used to build a receiver operator curve and calculate the area under this curve (AUC). After separate calculation of 10 AUCs the mean of these 10 AUCs was calculated. This mean AUC was used as read-out for the quality of the classifier. The settings of the best classifier as defined by the maximum mean AUC was used to then build a classifier on the whole training group and apply this classifier to an external independent validation group (PG2).
To avoid artificial optimization due to data set splitting into training (PG1) and independent validation group (PG2), the inventors performed the above described procedure of 10-fold cross-validation in 10 distinct random data-set splitting of a merged data-set from PG1 and PG2. For this random data-set splitting each sample was taken only once for the validation group. The whole test procedure described above was performed for each new data set splitting into test and validation group. For each of these data-set splittings into training and validation group the AUC of the classifier in the validation group was calculated. Finally, the mean and the standard deviation of these 10 AUCs were calculated.
A priori the optimal set of genes for the classifier is not known. The inventors used F-statistics to identify differentially expressed genes. This F-statistics was calculated separately for each single data-set in each cross-validation (n=100). In the next step, the inventors obtained 36 different lists of genes from each F-statistic by step-wise increase of the cut-off for the p-value of the F-statistic (p = 0.00001, p = 0.00002, p = 0.00003 ...-...p = 0.08, p = 0.09, p = 0.1). Two rules were used to choose the optimal set of genes. (i) The optimal set of genes should lead to the maximum AUC. (ii) The number of genes involved in the classifier should be as low as possible to avoid overfitting.
To underline the specificity of the lung cancer specific transcripts extracted, the inventors performed a permutation analysis using 1000 randomly chosen feature lists of the same length as used for the classifiers.

*Algorithms for classification:* The inventors used three different machine learning algorithms (support vector machine (SVM), linear discrimination analysis (LDA), and prediction analysis for microarrays (PAM)) for classification. All three machine learning algorithms were used as implemented in R. The following settings were used for these algorithms:
**SVM**: SVM is a well-established machine learning algorithm for distinction between two groups. Using the Kernel function it allows the identification of an optimal hypergeometric plane. scale = default, leading to an internal scaling of the x and y variable to 0 and unit variance; type = C-classification; kernel = linear; probability = true, allowing for probability predictions.
**LDA:** prior = default, no indication of prior probability of class membership was used leading to a probability equally to the class distribution in the training set; no additional argument was indicated.
**PAMR:** nfold = 10, a 10-fold cross-validation was used; folds = default, a balanced random cross-validation was used; no further argument was added.

### Datamining:

To investigate gene ontology of transcripts used for the classifier, the inventors performed GeneTrail analysis for over- and underexpressed genes (Backes C, Keller A, Kuentzer J, et al. GeneTrail-advanced gene set enrichment analysis. Nucleic Acids Res 2007;35: W186-92). To this end, the inventors analyzed the enrichment in genes in the classifier compared to all genes present on the whole array. The inventors analyzed under- respectively over-expressed genes using the hypergeometric test with a minimum of 2 genes per category.
In addition, the inventors performed datamining by Gene Set Enrichment Analysis (GSEA) (Subramanian A, Tamayo P, Mootha VK, et al. Gene set enrichment analysis: a knowledge-based approach for interpreting genome-wide expression profiles. Proc Natl Acad Sci U S A 2005;102: 15545-50). As indicated, the inventors compared the respective list of genes obtained in the inventors' expression profiling experiment with datasets deposited in the Molecular Signatures Database (MSigDB). The power of the gene set analysis is derived from its focus on groups of genes that share common biological functions. In GSEA an overlap between predefined lists of genes and the newly identified genes can be identified using a running sum statistics that leads to attribution of a score. The significance of this score is tested using a permutation design, which is adapted for multiple testing (Subramanian A, Tamayo P, Mootha VK, et al. Gene set enrichment analysis: a knowledge-based approach for interpreting genome-wide expression profiles. Proc Natl Acad Sci U S A 2005;102: 15545-50). Groups of genes, called gene sets were deposited in the MSigDB database and ordered in different biological dimensions such as cancer modules, canonical pathways, miRNA targets, GO-terms etc. (http://www.broadinstitute.org/gsea/msigdb/index.jsp). In the analysis, the inventors focused on canonical pathways and cancer modules. The cancer modules integrated into the MSigDB are derived from a compendium of 1975 different published microarrays spanning several different tumor entities (Segal E, Friedman N, Koller D, Regev A. A module map showing conditional activity of expression modules in cancer. Nat Genet 2004;36: 1090-8).] The gene sets used for the canonical pathway analysis were derived from several different pathway databases such as KEGG, Biocarta etc (http://www.broadinstitute.org/gsea/msigdb/collection_details.jsp#CP).

### Results

### Expression profiling-based detection of prevalent lung cancer

In the first case-control group of lung cancer patients (PG1) the highest accuracy for diagnosing prevalent lung cancer from blood-based transcription profiles was reached using a support vector machine (SVM-) based algorithm (**Fig. 2**). The highest mean AUC values in this 10-fold cross-validation were 0.747 (+/-0.206 standard deviations (std)) with a cut-off value for the F-statistic of 0.0008 and 0.763 (+/-0.189 std) with a cut-off for the F-statistic of 0.006, respectively. (**Fig. 2**). The inventors subsequently used a cut-off of 0.0008 to control for overfitting. Using this cut-off value, the inventors selected 161 transcripts as best performing feature set in the whole PG1 data set (**Table 3**) and used SVM to build a classifier. The inventors then used these transcripts and the same SVM model to classify samples from an independent validation case-control group (PG2). When using this classifier to build a receiver operator curve, the inventors calculated the AUC for the diagnostic test to be 0.797 [95% confidence interval (Cl) = 0.616-0.979] (**Fig. 3A**). In the PG2 validation cohort, the sensitivity for diagnosis of lung cancer was calculated to be 0.82 and the specificity 0.69 at the point of the maximum Youden index. Given the continuous nature of the SVM score additional use can be made from this score e.g. to increase specificity which might be useful depending on the potential application. E.g. using a cut-off of the SVM score of >0.9 leads to a specificity of 91 % reducing the number of false positives by 27 %.
In addition, the inventors observed a significant difference between the SVM scores of lung cancer cases and respective controls in the validation group (p = 0.007, T test) (**Fig. 3B**). To underline the specificity of this test, the inventors used 1000 random lists each comprising 161 transcripts to build the classifier in PG1 and apply it to PG2. The mean AUC obtained by these random lists was 0.53 and not a single permutation (AUC range 0.31 to 0.78) reached the AUC of 0.797 of the lung cancer classifier (**Fig. 3C**). This translated into a p-value of less than 0.001 for the permutation test confirming the specificity of the lung cancer classifier.
Next, the inventors excluded that the high AUC of the lung cancer classifier might be due to the elected splitting of the groups PG1 and PG2 into test and validation cohort. To this end, the inventors performed 10 random data set splittings of the merged PG1 and PG2 data sets and repeated the analysis for each data set splitting independently. For cut-off values of the F-statistics from 0.0006-0.001 the mean AUC of the 10 data set splittings was significantly above the expected random AUC of 0.5 (> 2 standard deviations) (**Fig. 4A**), demonstrating that the results obtained were not due to specific splitting of the data set. The specificity of these findings is highlighted by the fact that none of the 1000 random feature lists of equal size led to an AUC as high as the mean AUC obtained by disease specific transcripts (**Fig. 4B**). To further underline the stability of the extracted feature list the differential expression of the extracted features was analyzed in each of the 10 random data set splittings in the merged PG1 and PG2 data set. 45% of all the transcripts of the initially extracted transcripts were differentially expressed in at least one random data set splitting at a p-value below 0.0008 in the F-statistics with 19.3 % demonstrating a p-value below 0.0008 in all data set splittings (**Table 4**). Furthermore, 97 % of the transcripts selected demonstrated a significant differential expression in all other dataset splitting, whereas only 7.6 % of all random features were significantly different between the cases and controls at a p-value of below 0.05.
Additionally, the inventors tested the classifier built in PG1 in a third group of unmatched prevalent cases and controls (PG3). The AUC determined for this group was 0.727 [95 %Cl = 0.565-0.890]. Thus, the performance of the classifier is independent of the presence of matched controls in the data set analyzed, further supporting the validity of these findings (**Fig. 5**).

### Mining of expression profiles

To analyze the biological significance of the differentially expressed transcripts different strategies were used. First, the inventors used GeneTrail (Backes C, Keller A, Kuentzer J, et al. GeneTrail--advanced gene set enrichment analysis. Nucleic Acids Res 2007;35: W186-92) to analyze an enrichment in GO-terms of the genes specific for lung cancer in the inventors' study (n=161) (**Table 3**). The inventors observed 10 GO categories demonstrating a significant (p-value FDR corrected < 0.05) enrichment of genes in this classifier (GO:0002634: regulation of germinal center formation; GO:0043231: intracellular membrane-bounded organelle, GO:0000166: nucleotide binding, GO:0043227: membrane-bounded organelle, GO:0042100: B cell proliferation, GO:0002377: immunoglobulin production, GO:0046580: negative regulation of Ras protein signal transduction, GO:0002467, GO:0051058: germinal center formation, GO:0017076: purine nucleotide binding). Six of these GO categories are part of the biological subtree comprising 4 categories of genes associated with the immune system. (GO:0002634, GO:0042100, GO:0002377, GO:0051058) These data indicate an impact of immune cells to the genes involved in the classifier.
Second, the inventors analyzed the 1000 transcripts most significantly changed within the dataset between NSCLC, SCLC and controls (**Table 5**). The inventors computed overlaps between these annotated transcripts and the gene set collection deposited in the Molecular Signature Database focusing on the canonical pathways. The pathway gene sets are curated sets of genes from several pathway databases (http://www.broadinstitute.org/gsea/msigdb/ collection_details.jsp#CP). These pathways point to potential biological functions the group of genes is involved in. Of the 1000 transcripts differentially expressed in the inventors' study, 776 were present in the Molecular Signature Database. When calculating the overlap between the inventors' lung cancer specific gene set and the canonical pathways gene set, the inventors observed 11 canonical pathway gene sets with significant (corrected p-value<0.05, p<2.9*10⁻⁵ uncorrected) overlap 4 of which can be partly attributed to interaction of immune cells (HSA04060 cytokine cytokine receptor interaction (uncorrected p-value = 5.11 x 10⁻⁸), HSA04010 MAPK signaling pathway (uncorrected p-value = 6 x 10⁻⁷), HSA01430 cell communication (uncorrected p-value = 7.8 10⁻⁷), HSA04510 focal adhesion (uncorrected p-value = 2.9 * 10⁻⁵). These data further underline an enrichment of immune associated genes in the lung cancer specific expression profile.
Third, the inventors performed a gene set enrichment analysis (Subramanian A, Tamayo P, Mootha VK, et al. Gene set enrichment analysis: a knowledge-based approach for interpreting genome-wide expression profiles. Proc Natl Acad Sci U S A 2005;102: 15545-50; Segal E, Friedman N, Koller D, Regev A. A module map showing conditional activity of expression modules in cancer. Nat Genet 2004;36: 1090-8) with a focus on cancer modules which comprise groups of genes participating in biological processes related to cancer. Initially, the power of such modules has been demonstrated exemplarily for single genes such as cyclin D1 or PGC-1alpha (Lamb J, Ramaswamy S, Ford HL, et al. A mechanism of cyclin D1 action encoded in the patterns of gene expression in human cancer. Cell 2003;114: 323-34; Mootha VK, Lindgren CM, Eriksson KF, et al. PGC-1alpha-responsive genes involved in oxidative phosphorylation are coordinately downregulated in human diabetes. Nat Genet 2003;34: 267-73) and a more comprehensive view on such modules has been introduced recently (Segal E, Friedman N, Koller D, Regev A. A module map showing conditional activity of expression modules in cancer. Nat Genet 2004;36: 1090-8). This comprehensive collection of modules allows the identification of similarities across different tumor entities such as the common ability of a tumor to metastasize to the bone e.g. in subsets of breast, lung and prostate cancer (Segal E, Friedman N, Koller D, Regev A. A module map showing conditional activity of expression modules in cancer. Nat Genet 2004;36: 1090-8). Overall 456 such modules are described in the database spanning several biological processes such as metabolism, transcription, cell cycle and others. For this analysis, the inventors explored only those genes, which were identified to be discriminative between cases and controls in the inventors' data set independent of the data set splitting (n=31) (**Table 4**). Within this set of 31 genes the inventors observed a significant enrichment of the genes related to modules 543, 552, 168, 222, 421. Interestingly, these specific modules are also mainly observed in lung cancer samples in the original sample collection of 1975 samples. Although the lung cancer samples account only for 13% of the deposited samples the above mentioned modules are preferentially present in these lung cancer samples (average 8.6 samples). In contrast, in non-lung cancer samples accounting for 87% of the deposited samples these modules were rarely observed (average 3.6) (Segal E, Friedman N, Koller D, Regev A. A module map showing conditional activity of expression modules in cancer. Nat Genet 2004;36: 1090-8). This indicates that genes differentially expressed in peripheral blood between lung cancer cases and controls in the inventors' study are part of biologically cooperating genes that are also differentially expressed in primary lung cancer but not in other cancer entities. Many of the genes within these cancer modules have phosphotransferase activity (GNE, GALT, SRPK1, PFTK1, STK17B, PIP4K2B) and are involved in cell signaling. To underline this specificity for lung cancer of the genes extracted in the analysis, the inventors further calculated the overlap between the inventors' extracted gene set (n=161) and the genes differentially expressed in blood of patients with renal cell cancer (Twine NC, Stover JA, Marshall B, et al. Disease-associated expression profiles in peripheral blood mononuclear cells from patients with advanced renal cell carcinoma. Cancer Res 2003;63: 6069-75). Only CD9 was present in both gene sets. Similarly no overlap was observed between the inventors' gene set that was used for classification of samples (n=161) and blood based expression profiles for melanoma (Critchley-Thorne RJ, Yan N, Nacu S, Weber J, Holmes SP, Lee PP. Down-regulation of the interferon signaling pathway in T lymphocytes from patients with metastatic melanoma. PLoS Med 2007;4: e176), breast (Sharma P, Sahni NS, Tibshirani R, et al. Early detection of breast cancer based on gene-expression patterns in peripheral blood cells. Breast Cancer Res 2005;7: R634-44) and bladder (Osman I, Bajorin DF, Sun TT, et al. Novel blood biomarkers of human urinary bladder cancer. Clin Cancer Res 2006;12: 3374-80). In summary, these data point to a lung cancer specific gene set present in the inventors' classifier.

Using RNA-stabilized whole blood from smokers in three independent cohorts of lung cancer patients and controls, the inventors present a gene expression based classifier that can be used to discriminate between lung cancer cases and controls. Applying a classical 10-fold cross-validation approach to a first cohort of patients (PG1), the inventors determined a lung cancer specific classifier. This classifier was successfully applied to two independent cohorts (PG2 and PG3). Extensive permutation analysis as well as random feature set controls and random data set splittings further showed the specificity of the lung cancer classifier.
Overall, the inventors' data demonstrate the feasibility and utility of a diagnostic test for lung cancer based on RNA-stabilized whole blood in smoking patients, in particular with a high degree of comorbidity.

## Claims

1. A method for the detection of lung cancer in a human subject based on RNA from a blood sample obtained from said subject, comprising:
- measuring the abundance of at least 4 RNAs in the sample, that are chosen from the RNAs listed in table 3, and
- concluding based on the measured abundance whether the subject has lung cancer.

2. The method of claim 1, wherein the abundance of at least 9 RNAs, of at least 10 RNAs, of at least 13 RNAs, of at least 29 RNAs that are chosen from the RNAs listed in table 3 is measured.

3. The method of claim 1or 2, wherein the abundance of at least the 161 RNAs of table 3 is measured.

4. The method of claims 1 to 3, wherein the measuring of RNA abundance is performed using a microarray, a real-time polymerase chain reaction or sequencing.

5. The method of claims 1 to 4, wherein the decision whether the subject has lung cancer comprises the step of training a classification algorithm on a training set of cases and controls, and applying it to measured RNA abundance.

6. The method of claims 1 to 5, wherein the classification method is a random forest method, a support vector machine (SVM), or a K-nearest neighbor method (K-NN), such as a 3-nearest neighbor method (3-NN).

7. The method of claims 1 to 6, wherein the RNA is mRNA, cDNA, micro RNA, small nuclear RNA, unspliced RNA, or its fragments.

8. Use of a method of claims 1 to 7 for detection of lung cancer in a human subject based on RNA from a blood sample.

9. A microarray, comprising a solid support and a set of oligonucleotide probes, the set containing from 5 to about 3,000 probes, and including at least 4 probes for detecting an RNA selected from Table 3.

10. Use of a microarray for detection of lung cancer in a human subject based on RNA from a blood sample, comprising measuring the abundance of at least 4 RNAs listed in table 3, wherein the microarray comprises at least 4 probes for measuring the abundance of each of at least 4 RNAs.

11. A kit for the detection of lung cancer in a human subject based on RNA obtained from a blood sample, comprising means for measuring the abundance of at least 4 RNAs that are chosen from the RNAs listed in table 3, preferably comprising means for exclusively measuring the abundance of RNAs that are chosen from table 3.

12. Use of a kit of claim 11 for the detection of lung cancer in a human subject based on RNA from a blood sample, comprising means for measuring the abundance of at least 4 RNAs that are chosen from the RNAs listed in table 3, comprising
- measuring the abundance of at least 4 RNAs in a blood sample from a human subject, wherein the at least 4 RNAs are chosen from the RNAs listed in table 3, and
- concluding based on the measured abundance whether the subject has lung cancer.

13. A method for preparing an RNA expression profile that is indicative of the presence or absence of lung cancer in a subject, comprising:
- isolating RNA from a blood sample obtained from the subject, and
- determining the abundance of from 4 to about 3000 RNAs, including at least 4 RNAs selected from Table 3.


SEQUENCE LISTING

<110>  Universitaet zu Koeln
       Rheinische Friedrich-Wilhelms-Universitaet Bonn

<120>  Blood-based gene expression signature in lung cancer

<130>  PRO001EP

<160>  161

<170>  PatentIn version 3.5

<210>  1
<211>  1909
<212>  DNA
<213>  Homo sapiens

<400>  1
cggctgaggc gcctcgaagg gcaagcgcca agggggcgcg ggcgccgccg gaagctgggg      60

cggggcgccc agcgggatgc ggtgaagggc gagcggcgcg gcggctgcga tgagtgcctc     120

tgcggccacc ggggtcttcg tgctgtccct ctcggccatc ccggtcacct atgtcttcaa     180

ccacctggcg gcccagcatg attcctggac tattgtaggg gttgctgccc tcatcctgtt     240

cctggtagca ctgctggctc gtgtcctcgt caaaagaaaa ccaccccggg acccactgtt     300

ctatgtgtat gcagtttttg gatttaccag cgtggtgaac ctcatcatag gactggagca     360

agatggaatc attgacgggt tcatgacaca ctacttgaga gagggtgaac cgtatctgaa     420

caccgcatat gggcacatga tctgctactg ggatggctct gctcattatc tgatgtacct     480

ggtgatggtg gcagccatag catgggagga aacttataga accattggcc tatattgggt     540

tggatctatt attatgagtg ttgttgtttt tgtgccagga aacattgtag ggaagtatgg     600

aacacgaatt tgccctgctt ttttcttaag cataccatat acttgtcttc ctgtctgggc     660

tggtttcaga atctataatc agccatcaga aaattataat tacccctcaa aggttattca     720

agaagcccaa gcgaaagacc tgctgagaag accatttgat ttaatgttgg ttgtgtgtct     780

cctcctggca actggatttt gcctgttcag aggtttgatt gctttggatt gcccatctga     840

gctctgccga ttatatacgc aatttcaaga gccctatcta aaggatcctg ctgcttatcc     900

taaaattcag atgctggcat atatgttcta ttctgttcct tactttgtga ctgcactgta     960

tggcttagtg gttcctggat gttcctggat gcctgacatc acattgatac atgctggagg    1020

tctggctcag gctcagtttt ctcacattgg tgcatctctt catgctagaa ctgcttatgt    1080

ctacagagtc cctgaagaag caaaaatcct ttttttagca ttaaacatag catatggagt    1140

tcttcctcag ctcttggcct atcgttgtat ctacaaacca gagttcttca taaaaacaaa    1200

ggcagaagaa aaagtggaat aaaaatatta cttcatgttc ctcctttcta aattactaac 1260

ttttgttata ctggtactga tattttgtcc catttcactc tcttctcata cgtgagtact 1320

taagaatatg tacattcttg ctctgcactg tatgtgtgag ctatatggta ttgtgtaaat 1380

tttttttgaa ggaaaatgga aattcttgag aaacagtttg tttaaagaaa tatattcaaa 1440

atcatttgtg aataaacttg atcatccatc tcaatattgt ttgacatata aaataattat 1500

aagtgtaaaa aattacaatt tagtgccaac agtagtgagc atgaaatgaa actattcaaa 1560

agagaatatg gcctgtgcat attaaaaaat tcaaaacagt gaatgcagac tggaggagta 1620

acttttgcaa ataagatgaa tatgcttcat tattaaactc aatataaaag gcaaatcatc 1680

agaatatttt taaatgttgt ttgaaaaatg ttttcccaag gaaagtttat tatttgctgc 1740

tgtttcaaga aaattacttt tactaaattt ttttgtgtga atttaaacag ctaaataggg 1800

atcagtaact ttatctctat ccttaatgaa catttgtttt attggtggct ggaaatattt 1860

ctattgtatt tctgtgtata tttttaataa aattattttt ggcctctta 1909

<210> 2
<211> 3917
<212> DNA
<213> Homo sapiens

<400> 2
agaagtctgt ccgcgagctg tcagcgcggg cgggaacgcc gcggggcgcg gggtgggcgc 60

ggccgacctg gtccctgagc cggccggcga ccccggacct cccgcgcgcc ccgcacccga 120

ccggctcagc cggccggcag cgtaacacgc cctacgctcg cttgctcgcc ggcctcaggg 180

caggcaggcg ggcgcgggag accccgccgg ggccgagact tggggcgggc gacgaggacc 240

aggttacggc ctcctcgcca tgtcctcggc ctgcgacgcg ggcgaccact accccctgca 300

cctcctagtc tggaaaaacg actaccggca gctcgagaag gagctgcagg gccagaatgt 360

ggaggctgtg gacccacgag gtcgaacatt attgcatctt gctgtttcct tgggacattt 420

ggaatctgct cgagtcttac tccgacataa agcagatgtg acaaaagaaa atcgccaggg 480

atggacagtt ttacatgagg ctgtgagcac tggcgatcct gagatggtgt acacagttct 540

ccaacatcga gactaccaca acacatccat ggcccttgag ggagttcctg agctgctcca 600

aaaaattctc gaggctccgg atttctatgt gcagatgaaa tgggaattca ccagctgggt 660

gcccttggtt tctagaatat gcccaaatga tgtctgtcgc atctggaaaa gtggtgccaa 720

actgcgcgtc gatatcacat tgctgggatt tgaaaacatg agctggataa gagggaggcg 780

tagttttata tttaagggag aagacaactg ggcggagtta atggaagtca accatgatga 840

caaagtggtc accaccgaac gcttcgacct ttcccaagaa atggagcgcc tcactctgga 900

cttgatgaag ccaaaaagca gggaagttga gcggcggctc acaagccctg tcattaacac 960

cagcctcgat actaaaaata ttgcttttga aagaactaaa tccggattct ggggctggag 1020

gacagataaa gcagaagttg ttaatggtta cgaagcaaag gtttacacag taaacaatgt 1080

gaatgtgatc accaaaatac gcacagaaca tctgaccgag gaggaaaaaa agagatataa 1140

agcagacagg aacccgctgg aatctttgct gggaactgtg gaacaccaat ttggtgcaca 1200

aggggacctc accacggaat gtgctactgc aaacaacccc acagccatca cgcctgatga 1260

gtacttcaat gaagagtttg atctgaaaga cagggacatt ggaaggccga aagagctgac 1320

gattagaaca cagaagttta aagcaatgtt gtggatgtgt gaagagtttc ccctctctct 1380

ggtggagcag gtcattccca tcattgacct aatggctcga acgagtgctc attttgcaag 1440

actgagagat ttcatcaaat tggaattccc acctggattt cctgtcaaaa tagaaattcc 1500

cttgtttcat gtcttaaatg cacggattac atttggaaat gttaatggct gtagcactgc 1560

cgaagaatct gtatctcaaa atgtggaagg gacccaggct gattcagctt cccacatcac 1620

aaactttgag gttgatcaat ctgtgtttga aattcccgaa tcttactatg ttcaagacaa 1680

tggcagaaat gtgcatttgc aagatgaaga ttacgagata atgcagtttg ccatccagca 1740

aagtctgctg gagtccagca ggagccagga actttcagga ccagcttcga atggagggat 1800

cagccagaca aacacctatg acgcccagta tgagagggcc atccaggaga gcctcctcac 1860

cagcacagaa ggcctgtgcc ccagcgccct gagcgagaca agccgttttg ataatgactt 1920

gcagctagcc atggagctct ctgccaaaga gctggaggaa tgggagctcc ggctccagga 1980

ggaagaggct gagctccagc aagtcttaca gctgtcactc actgacaaat agacctttca 2040

gcctgtgagc ctctgcacaa agcagaggct gtgggctgtc acagatgctg tgtcaaccag 2100

ggccctaggg ctaagggcct gcaccttgcg tgcatgcagc aggcaacaac tgccccttct 2160

ttatgcagag gtgcagaacc agggactcct gggcccatcc aggctgctcc ctggggtgga 2220

gaagggacca gggattgcag gccccatctc caggctaagg ggaggagagc atcatcactt 2280

tccattagct gtattggctt gcaggtcaca tttttactac cagctttaga caaaacccca 2340

atccccgcag gtttgtagat aaatttttat caaggagtga taacaagaca agttattgca 2400

gagtcagggt gcttttatat ataagagcag cagccgcctt tgtaaagtgt gatctatgag 2460

aattggagac acttctttac tgttcaccaa agaaatgggt aatctgtgct gatctcctta 2520

tttttacctt tttacagggt ttctaggaca ttgtcattat tcttcctcca tctatctgat 2580

tccattcctt ccacacccag ctaaagttag aggagataca tatagaatct attttagatt 2640

attgttaact atttgcatca aattaagata cacaaatggc catggatgtt gccttagcct 2700

taaacattac taatagtttc acatcacctc actgcacgca tcgaaggatc tgatttgaat 2760

ttgtaaaggc aattcttttg agagcaggat tctccaggct aaatcaaagg cagctaatcc 2820

tgtccctgaa ggagcagcta ggggaacctg aggcttgttc ttgaagtggg caagctggcc 2880

aaaatattgg aacacacaga accaaaccag gtgtgttcta cacctgcatg agtgaaggat 2940

ttccacgtag acacctagga agagcccgca tgccctagac tcactccaga ggaaggattg 3000

atttgcaacc agaaagggag ctgaaaacca cggagctcca tggctcttca ttcaaaaggg 3060

aaaataatga ttccacgttg ctttttagag ttcaaatcaa catctttctg gataaatcta 3120

ttttttaaca atctttttat tatttgtaaa agatataaaa acaactccca tcagtagcaa 3180

tacaaggtta tacattttaa ccagattttc tcaggccttt tttggatacc tttagtagtt 3240

aactctcttt tgtcaaaccc tcttgtatat aaccatcgca caacatacag aaccctgggg 3300

aatacagcca agggcactgc tcactgtggc ccgtgtattc attcccacag gctgcactga 3360

aataacctgg taaacaacac cctcctccat tttgcgtcta tttcttctat tgtgcttctt 3420

tggggatagg aagagaaaca taactactgt tccaggtaac gtctgtatca tacagttagt 3480

gttgccagtg aaacgttccc agatacaaag aattgtgctt ttaattccat ttcacaatct 3540

gttttgtttt ttttttttgt cattgtctga ccaaaattcc ttctgcacat gaggccagta 3600

taggcaacgt cacatttgtt tgtttcaaat atgcagtgtg gtattttgtt tactttgcat 3660

ttaccgtgag caaagatact tcttggaatg gctgcagtga ggccgtgtca ttggctcaca 3720

aaagtgactc ataggtaatt tctgtgtttg cattccactg ctctgctccc taaggtcctt 3780

gactttctcc ccaggaattc acttaaaaag ggactgagca ttgtgtcaac ctgttgcagt 3840

gtttttcata tatgtgttca ctctaaaaat gcaaataaag actgcttcct tagagggtgc 3900

tcatacaaaa aaaaaaa 3917

<210> 3
<211> 10390
<212> DNA
<213> Homo sapiens

<400> 3
gtgcgaagcg tgaggtggag atgggggcgg agggagctgg agctgtcaca gtccccgggt 60

gcgcctgacc gagccaagtg gggtgtcatg gccgcggggg gcagcggctg cacttcctca 120

gcgggcggcg gtggccgggg agttaatcct cgacgcacgg gtcccgtttc cctctgtgcg 180

gcggccggcg ggaccataag ggcttaactc atatatttaa cccccctcca aaaaggtttg 240

aaagtattct tgaagggctg tttggacctg cattattaaa agatctcagt ttatttaaag 300

actgtgaacc tgaaagcatt tctgattgga cttttgatga aaactgttta ttctgttgct 360

tgagaagaga taaagtaaag ggacacttag tcggtctgga tgaaccagct tcgggagctg 420

ggcaagaagc tctgcttaaa caagagcaag caaaaatcat tcgattcgag agacaagcag 480

aagaattcct caatgcagtc ttttatagaa aagacagtcc ctgggtctcc gaccccaata 540

ttcccctagt ggcccgtgag atcatgcagc gaatgatcca acaatttgct gctgaatata 600

cctcaaaaaa tagctctact caggacccca gccagcccaa tagcacaaag aaccaaagcc 660

tgccgaaagc atctccagtc accacctctc ccacggctgc aactactcag aaccctgtgc 720

tcagcaaact tctcatggct gaccaagact cacctctgga ccttactgtc agaaagtctc 780

agtcagaacc tagcgaacaa gacggtgtac ttgatctgtc cactaagaaa agtccatgtg 840

ctggcagcac ttccctgagc cactctccag gctgctccag tactcaaggg aacgggcgac 900

ctgggagacc cagccagtac cgcccagacg gacttcggag tggtgatggg gtacctccaa 960

gaagcttaca ggatggaacc agggaaggtt ttggacactc cacatcactc aaagttccac 1020

tggctcgatc cctgcagatt agtgaagaac tactgagcag aaaccaattg tccacagctg 1080

ccagccttgg gccatctgga ttacagaatc atggacaaca cttaatatta tccagggaag 1140

cctcttgggc aaaaccacat tacgagttca acctcagccg tatgaagttc aggggaaatg 1200

gtgcactcag caacatcagt gaccttcctt ttcttgcaga aaactctgcc tttccaaaaa 1260

tggcacttca agcaaaacaa gatggaaaaa aggatgtgag ccattcatct cctgtagatt 1320

taaagatacc acaagttcga ggaatggatc tttcttggga gtctcgcact ggtgatcagt 1380

acagctatag ctctttggta atgggttcac aaacggagag cgcgcttagt aaaaaattaa 1440

gggctattct tccaaaacaa agtagaaaaa gcatgttaga tgctggaccc gattcttggg 1500

gctcagatgc tgagcagtct acctctggac agccatatcc cacatcggat caagaaggag 1560

accctggctc caagcagcct cggaagaaaa gagggcgtta cagacagtac aacagtgaga 1620

tactggagga agcaatctca gtggttatga gtggaaaaat gagtgtttcc aaagctcaga 1680

gtatttatgg gattccccac agtacactgg agtacaaagt aaaggagagg ctgggcactt 1740

tgaaaaaccc tccaaagaaa aagatgaaat taatgaggtc ggagggggca gatgtttctg 1800

taaagattga attagatccc cagggagagg cagcacaaag tgcaaatgaa tcaaaaaacg 1860

agtaggaata ctgtagagtg ccaattactg tacaaactgg gtgagcacta ctgcatcatt 1920

gttcagctat cattgcttgc acgtaatttc atttactgtg acacttgctt ctttgcagat 1980

tttgcattga cttgtgtgta cagagatgaa atgtgcattc tgaatgttgc atattttaaa 2040

tttttcatgt gcagtatggc tcggaatatg tttggccttt tgcatgtttc tctacaaaag 2100

agaattgagt tacctcacag agaacagata catggaagtg gactccttgc ctgtagagcc 2160

tgcatgcttt tttgtttttt tttgttgttg ttgttttttc ttaagttata tttgttttta 2220

ctttttaaaa aagaagagga aacaaagccc ccttttagaa actacgtctg tcatactggg 2280

agctttatca ctgcaaattg gaaagccatc ttatacaaaa ttattcacat ttttcatcta 2340

cgattcaact aatcgaagga ttaaactaaa gaaaaaacta agaagaacaa tgaacctttg 2400

actttgagaa atttggttat tcactgacat aattattggg tcatttatag ttacatttta 2460

taattttctt ctcactatta aatttattta aattagcaag ctattagtgc tcacccagag 2520

gggaaggtgg tggaaaatgt gcacacacta ccttcagaaa tgcttcagtt aattactttg 2580

aacactacct ttgctgtaat ttcatttgag attttctaag ggtagaattt ggtctcacca 2640

acaagtgagg atatagcctt atctcatgga ggacgagctc cgtatttact caggagcagt 2700

cagggtacat tacataaaac aatggaggat gcctcatttt agcaaactag gtttctttgt 2760

attcttcagt ccttttacag aattgatgtg ctaactgaat atcattgcag caactagact 2820

aagatattca agatctcttt attggggatg ggagaaatag ggaaagaaat gtgtataagt 2880

aattatgata ttgcaaaagt gatacttaga ttttacagcc tcagtagtct gcccagtgtc 2940

cacattaatg aaggatccat gtttgtagtg agagaaaaaa accccaaggt aacccgatat 3000

gatttaggat gcatatcagt tctaacaatt caatcagaag tcaagctcat tggaattcct 3060

tttttaactg atccaaatac tagtagaagg gggagggaga ggtgttgggt ttttttttta 3120

agtttttatt taattttgtt ggttagaatt tttttctgtt tttggcatcc tacataatac 3180

cccccttctt gactttttct gataattagc tgatattcat ggttgtttag cacacagttc 3240

aggacctttg agatcatgtt tgtataagca ctccttgaag aatatctaag ctttttctga 3300

gatgggcttt taaaattata ataaggaaag tttattttct gcggtttttg caagaataag 3360

caaagcccat ggaatttaat ttttcattgt gatcagatta aaattactat ttgtgcaaaa 3420

caaattccac accacatgta ctgtatattc taatctgctg agaaaggtgg tcaggccctt 3480

ctaaatgctt aaccaaaaac aaacattatt ggagtttcag tgtaaaataa aattaaaatt 3540

ggagggttgg ttagattgat ttcagatgga tactaacagt ttgaggggag gtccagatat 3600

ttccaaggca taaattcttg cctggaaact ttggagcaat taattaaatt aggttaagtt 3660

ttccctcagc aacctgtgtg ttcaaaggtt aggcacacca ttgctgttac taaaatacat 3720

gcactgctca ttcttgacat agcttgggtg tgggttcctc ttgttttggt tattttgtgt 3780

agcagtttaa tcagtggata actgagcctg tttcctctta gctttctaga cttcttaaca 3840

gaaaaaacag tttgggttca atagcatttt atgattctac aatattttgt cacctagctg 3900

gaagttgcaa taaaaattcc ttatgaaata tatggttttc atctgtaatt gaaggaaatc 3960

atagaaagtt tgctgaaagc aaagggctaa gcttatgaag gaatgcctcc tttgttacac 4020

actcaagaat ttgtcagcac acaattaatt gcagccatag tacctttttg cgtaatgttt 4080

gggatattca gtagcccagg attgctacaa aaaggtcctt tgagaaaatt aatttgtgct 4140

tgcttttatc ccctctcaga aagacatcac taaaggccca ttaaaaatct ggccctaaaa 4200

gttcaacttt tgtagagttt attgttcaga gaggtttaat cctatgtggt agtctatata 4260

agtgatgtat gagttgagtt atgtaaattt tgtcattgta gtgtacatgg agtgatcatt 4320

ttactaacat aaatattttc tatgtgtgtt tcagtggatg acaatcgagc agcagaagaa 4380

tggtgtgcct accctttaat gctgcagttt aagagaactt gtattgtgtc atttcagatt 4440

gtaggctgag agttgtaaat agtgaaaatt tgagtacttc tattatttgt tttggttaga 4500

aagtgaattt aaaaaacaaa ccaaactcta aactttctca gattaaaagt cctgagacct 4560

gaagattgta atattcatgt ctgtgaagct tttaaacatt acacttgaga tcagtcatga 4620

cttgatattc aggtaaattt tcttttccaa gaagcttttg aataagcata tttcctccaa 4680

aggtctctct ctttctctct ctttttttga gtgtagatta ttttaaagca ctaattgcat 4740

tacattggta actgcaatat aaaatagcca ttattgtttt gtgaagcatg gttgaaatta 4800

gctagtggtc ccttttgaat ttcatgagcc tctcaaaaaa gaaaaactta aaaaaaaaaa 4860

aaaaaaaaaa caaaaagctg ctggaatatt tcaaaagata tatattttac cttattatag 4920

gttttgtaaa gttagtttgt aatattcagg tgcactttta atgttaaatt ttgtgttcag 4980

agttccatta taccatgtat ttctcggagg tggctcatca cacggctggt ggcagtcttg 5040

gtgccgcagt gattccttaa tatttgattt ctctcttgat ttgccactat tctgtattgg 5100

cactttcagt gtagatattc tagtttgggg atgtcctcac aatgtgtaat agagattggt 5160

ctattagcat gcttaatttc tgctaatcag agttttgcat ggcccaaaca acagttgacc 5220

atttttatgg tatgggaaag tgtagttcac aactgtcttt gtcacgaaag ccgtttttcc 5280

tcacattttc cagtaggcag ataacattac ttaaattatt aatatataac actcttggaa 5340

ctattgatag gaaataatgt atttaaaata attttttgca ttttcagaga ccttgatgtt 5400

atagggtaga taagctatgg cactataaaa tcaaaaacac taattctgtg gaaggatatc 5460

tagttggtat ttttatcttt agctttctca tttctgagac agcatagtca tccagcttga 5520

tggtaatgta gtatattact ttgttcagtg ggcttataag tgactctgaa ttatgtttgt 5580

gatgagctat acactttttt tctgttcccc aaataccatg aaggacaagg tcactctttc 5640

aggaaaaata atttatattt gtgtataata ctttttaata ttaagtttgt tgttctgaag 5700

tttagatgtt gcttgataaa cctttgctgc agcagatcat gaatatgagt aaggctttgt 5760

gtgataatag gggtccttat gtgaagttaa ttactgttca gttctggtga gcaagctcaa 5820

caagtgtgaa gtgtgtatta gctttatttg gtacactttt actttacaat atcgaagtgc 5880

ttttagaact ctggttatct tatataaacc attacctcaa ccaattggca tttcttttcc 5940

ccaacattta ccttagctat aatatgagct atgtgggatc agatggtaaa actataaaag 6000

gcacaatgag aagtttagtt cccaagtttt ggagagaact ctaaattaga ctaaaaacaa 6060

ctacctgtta acaccaatat cataactacc ttctgtcgtt aggcagtaca tagcaacctt 6120

tgagctgcta gtgagctatc tgttctcccc ctacccccaa gtaagcacac agtgccaggc 6180

ctggttctaa gcctttgtga ttctcttaca tctcattgac ccataatagt gaagcatgga 6240

ttcatttatt ttcataagac aaattgagtc agctcgatgt cagaagttga aaatattttg 6300

agtagtttaa cagttatatg agagattggc atcattttct ttaacagctt ttatctttgt 6360

agatttcctt ttgagacatg tatttttgct tttataatcc tttcattaaa tgccagaggt 6420

tgttttcata ttaatcactc ttttctgtat attcaggttg ctgtcattgg tgatagaata 6480

tggcaataga cagaattgtg ctccacttta tgttttttag gagatattga aagttatagg 6540

ttaaattttg gccttcttga agatatcaaa gattgttttc cttgttacct ttaattagaa 6600

tttggtcttc attaatgctt tggtcaggct agcattacta aaaacttgat ggcttcagca 6660

gaacagcccg tttaggagac tattaagcta tctagggatt ttttttcaaa ttaattgctt 6720

tcaaaatctg aaaaggaaaa aagtataatt gtggtacaaa gaagacagat ctccaggctc 6780

tttcttctca gtttattttg gtttaattct gtactggaga cttgaatagg atttgtgaac 6840

atggtgatgg aaagaaaaat agaggacatt atttgtgtac cctcattcct gaataatctt 6900

gcagtcacct tttaaagaag cagacattag ccctgtaaca caacactgta gctataaact 6960

ctaagtgtta ggaaggagat tggggtgggg agggtggagt aggatggaaa aagcatgtag 7020

acttctgaga agaggctaaa aattctgccc tataatacta aaaatgagtc acaagataat 7080

gtggtgaagt taggccatct tgatttgata gaaaaatctt ttacattgtt taaaaatgag 7140

aatcgaggct aattaatgtt gatttgacaa tagaacatga gctaattctg caaccgtttg 7200

gggggtaatt ttatggtttc cagttcattt agagctgtat caagaaaagc taaaaagcaa 7260

gcacttttag ttaatcatag atttattgga tatagcaaat attattataa tatttaatct 7320

ctattatttg aatgtggaaa tgaactcttt taccccatct tgaactgtca ttgaacatgc 7380

tcaagatgat caacactgga aatgacccta cacacatacc aaaaaaatct aaagcagtaa 7440

gacatatttg aagatccata cctcttaaac tttttagatt tgtcttggga aagaatttat 7500

gaacctgtaa ccagtattag tgcagtcttg ggttccttgt tctgcataat tgcacccttt 7560

ctcaggtatg tcctgacaac ataaatttta aaataatctc attagagatt agattctcag 7620

tggatagtgt gtaaggggac taggggtggg gggttggggg tagttggaca caggacactg 7680

acacagtaga gcaggatttt ttaacagtca atagccaaat agaatttcaa tctagaatat 7740

ttttaccccc tcagaatgag tgaaacaact tctattattt ggtgtacccg gtgctacact 7800

cctaaaagtc tttttttttt ttcatccagg tgacgttttt tatttttcct gcttggtggt 7860

ggaaggttat atagtaaact tcactgtcat taagaaagcg cttgtccaaa atgtttaaag 7920

tactagaaaa gggataattc agcacttttt ctgttacaac actaaagtct taatttatct 7980

tcagagtctt aggtactgta agtttttgga tgattattta aaaatataaa acatactaca 8040

taatatgggg tcagatcaca tttatataaa taattcttat tttaaaaaag acaataacat 8100

tttgtacctt tctagcaatt ccaagtgatt gcctcatttt ctcttgttaa gcatttcaag 8160

cttaagaatt tggaatttga gagaaggtga gggggaaaat tccctaaaat aaaaatttgt 8220

ttcaaaccat tttagaaaaa taaatcacta ggaaaatagg tagggattct ctcccccttt 8280

aagtcttcag ggaatgagca gtatttagaa tctttggctg aaattcaagc ctttttactt 8340

tgtaaatttt taatattaat tcactgacat gctttttaca ccagaggact gaaaatggat 8400

cttaatgttt ttaagttgtg gaagggtata tttctaaaat gaggggggat gattaactag 8460

gttagtatta ccagcattag taagtttaag gaattttagt atgtaatagc aaagtgtttg 8520

attaaaagtc taatcctata ttactagtca aaattagtta cattctgatc taggaaagat 8580

aatagtcatt ggatattgat atgctgtgct gtcagaaaac cagactaaga tagaaacctt 8640

tatctgactc ccacatgttg cttgttgtga tgggatgtat actttacagc agcttattgc 8700

tgctttgaaa aaaaatgtat aaactataca tttggagtgt ttgcatataa ttctttataa 8760

cctccactta aagctgtcag acattggtat tttatcagtc cacattgttg aataaaacta 8820

atgttcttag gaatccagct tgtacacact gtttaaaaac cctcagggac agtttacaca 8880

ctcttctcac tcaattcagg tactttgatg ctattcttaa acctaacagt gacttgtatt 8940

tttctgtttt gcttttattt caacgtgctg gtagcacatc tgatgaatgt caacttaaaa 9000

aactcagttc aggtatccag gtataactca gccaaacaga ttttaaagct gcatataact 9060

ctccagcaca tggtgcctca cactcttata gtggcattct atatattcag ttattactac 9120

tgagcagata atatgggggt tcctgttaac agtgtatttt ttaaaaaaag cgcataaatg 9180

tatagccagc acactacaca cacacacaca aacatgcaca gttaaactat atatttttaa 9240

atgccactaa tagccagcac aactaaaaca aaagacattc ctaactgctc tgtaagctgt 9300

taacagatgc agttccttct tgatgtggct cttgcttctt cacgataact actactaaat 9360

tcaagcactg gtccttgggt gtctgacctc tacattctag tttatgcaat gtctttagag 9420

aattttgtgc actggccact gtgatggaac cattgggcca ggagtgcttt gagtttatca 9480

gtagtgattc tgccaaagtt ggtgttgtaa catgagtatg taaaatgtca aaaaaattag 9540

cagaggtcta ggtctgcata tcagcagaca gttttgtccg tgtattttgt agccttgaag 9600

ttctcagtga caagtttttt ctgatgcgaa gttctaattc cagtgtttta gtcctttgca 9660

tctttaatgt taagacttgc ctctttaaaa ttgcttttgt tttctgcagt actatctgtg 9720

gttaacaaaa tagattattt ctctgcttta atatttgata tcttacatct aaaataaatt 9780

ctctccacat ataaaaccca tagcctttgg agatatggaa aatggtatct ttcagatttc 9840

tagaagttca agtgtcatac aacaaaacag gaaccccctt tactcttatg gacctcattt 9900

caatatactg tttacagttt gatggaattg tataatttaa tatttctctt gtactgtagt 9960

ttatatttat ttacagattt ttttgtactg tgtgatttga actttttgtt ccttgctatg 10020

atcaatgttt atgtagtaga gcacttatga tcacaaatta agttttttgg tttgattgca 10080

ctacattaaa ttttttaatg cagttctgat ttttgactgg actaaaactg tgtcttaatg 10140

tatgtgatga gtacttaaaa ttttaatcca tgtggtcccc cccctttttt ttttttttgc 10200

attgtatgtc aaaagcgctt gttctttcgt gcatgtgtaa gatttaatgg ttccattgta 10260

ttatttgacc atgacatttt ggagaaacat tcccagctgt aatgttgtgt atggtagttc 10320

tcactggatg ctagagtttt caaaaccact attcttctaa taaattttgt tgtgaaaaac 10380

tgttaaaaaa 10390

<210> 4
<211> 3152
<212> DNA
<213> Homo sapiens

<400> 4
gacgcgcagc aggccccgcc cacccaggcg gtaggaaccc actccggccc gctagacctg 60

ctgctatgtc ccggccgcag cttcgacgct ggcgcctcgt ctctagcccg ccgagcggcg 120

tcccgggtct agcgctgctg gcgctgctgg cgctgctggc gctgcggctc gcggccggga 180

ccgactgccc atgcccggag cctgagctct gccgcccgat tcgccaccat ccagatttcg 240

aggtctttgt gtttgatgtt ggacagaaaa cttggaaatc ttatgattgg tcacagatta 300

caactgtggc aacatttgga aaatatgact cagaacttat gtgctacgct cattcaaaag 360

gagccagagt agtacttaaa ggagatgtat ccttaaagga tatcattgat cctgctttca 420

gagcatcctg gatagctcaa aaacttaatt tggccaaaac acaatatatg gatggaatta 480

atatagatat agagcaagaa gttaattgtt tatcacctga atatgatgca ttaactgctt 540

tagtcaaaga aactacagac tctttccatc gtgaaattga gggatcacag gtaacctttg 600

atgtagcttg gtctccaaag aacatagaca gaagatgcta taattatact ggaatcgcag 660

atgcttgtga cttcctcttt gtgatgtctt atgatgaaca aagtcagatc tggtcagaat 720

gtattgcagc agccaatgct ccctataatc agacattaac tggatataat gactacatca 780

agatgagcat taatcctaag aaacttgtaa tgggtgttcc ttggtatggt tatgattata 840

cctgcctgaa tctgtctgag gatcatgttt gtaccattgc aaaagtccct ttccgggggg 900

ctccttgtag tgacgctgca ggacgtcagg tgccctacaa aacgatcatg aagcaaataa 960

atagttctat ttctggaaac ctatgggata aagatcagcg ggctccttat tataactata 1020

aagatcctgc tggccacttt catcaagtat ggtatgataa ccctcagagt atttctttaa 1080

aggcaacata tatacaaaac tatcgcttac ggggcattgg catgtggaat gcaaactgtc 1140

ttgactactc tggagatgct gtagccaaac agcaaactga agaaatgtgg gaagtcttaa 1200

agccaaagct gttacagaga tgaacatctt ttgtcaaacc attaagagtt agaaagatga 1260

tctgtatcaa cagatctagt ttcttgcatt tttattatgt tgctatatac ttttgttatc 1320

cgtatactaa aaaaaaagaa taaataaatg ttttgattgt ttgaatttga aaaatacaca 1380

cgaatgtcct cagtatccag gaacataaag gcaagaagca agtcaactta cctattaaat 1440

attcctctat tagatgtttc aacactataa tttaattggg aaaaattgct ttcagaattt 1500

tattatgcca tatttccctt cattatagta aaatatatgc tcacgaatca atgctgattt 1560

ttaaaatatg tataatctga agtggaaatt gtttgcttag agtttttaaa aacctagtct 1620

ttgaaaagca gtttgtgcta tacttttccc ccaaccctcc aataaatctt aaatttaaaa 1680

cctacaatgt agtgtctccc ctttaggttc atcaaactga tgaaaataat tttctgcttt 1740

cctgactcca tatagtattt atgttctctg aggtacttga actaaatctt tggacagtga 1800

gtgagaatta aaaagaatcc agaagattaa atgtattcac aaaatagaag aatgtaacag 1860

taatattttg aattggtaag ttagaactac ctaatatttt ccaactacat tatttttatg 1920

aggctcattt gtaaaatttt taataatagg aataacttgc agttgaactt gattctgttc 1980

actgtcacac ttctaattat tcatttcttc atttatggac atatataatt tatttcttgg 2040

ctagcttgct tacatggtta aatttttctt tctacatctg caatgtatct tttttatttt 2100

tattttttgt agagacaggg tctcgctatg ttgtccagat gggtctccaa ctcctgggct 2160

caagcactcc tcctgccttg gcttcctaaa gagctgggat tacaggcatg agctgcattg 2220

tgcttttaat gatctaaaga cactgattag cttctcaact agttctcttt ctaaaagatc 2280

aattttatac aaataagttt tctaaatgta ataaagtctg ttttctttct ccagggaatt 2340

aagaacaaca ttgtgctgcc cttattagtt cctaattaca gattaaaaat ctgtatcttt 2400

gaactctaga tttacataaa tcctggcttc cctgcacatt ttgcccctgt aaattggaat 2460

acatgatgat tgaaccatct gttatgagaa tattacattt cctatttaca caattgtttg 2520

tgacagagta aaaaccattt tcatagtata tgttggatca ctgttgtctt catatttcta 2580

aaataaatga attaaaaaat ttaaattctt agatgcttat ctctatcata gctcagaatg 2640

caacttcaat acccccattc ttttatattt ccagatttct tttaattaag atataatgaa 2700

caaattaagt taaatgaagt ctgtattttc atgcaaatat attagtattc attaatatac 2760

atttattctt tgcaaataga ttttagttat attagctctt aaattgttaa ataaattatt 2820

attgaatttt attattaaat tattaaaatg aaaattataa aattagcact gtaaaataat 2880

atgtaaagga cttggaatgt taaaaataac cattcatcat tttgctttat attacatcta 2940

atatttcctg aaacattttg taatacccac atggaaagtg gagaaagaga aaccttgtgc 3000

tttttataaa acattgccaa ggagaataat ggttaaacaa atctgttcaa atacatattt 3060

aaattttttt acaaataaaa gttttaaatt aacataccta aaaaaaaaaa aaaaaaaaaa 3120

aaaaaaaaaa aaaaaaaaaa aaaaaaaaaa aa 3152

<210> 5
<211> 3433
<212> DNA
<213> Homo sapiens

<400> 5
ttgccggcgt ctccgagccc gcgctcccag ctgcagagcg cctggcttgc ctcccgcgcg 60

gtcaccgccg gcccgccgcc cccgctcccg cccgcgcccg gagcccctgc ctcgcgcccg 120

atggtgagca gtgtgttgtg ccgctgtgtg gcctccccgc cgccggacgc cgccgccacc 180

gccgcctctt cgtctgcctc atcgccggcg tccgtggggg acccctgcgg cggcgctatc 240

tgcgggggcc cggaccaccg gctgcgcctg tggagactct ttcagacgct cgacgtcaac 300

cgggacggcg gcctgtgtgt caacgacctg gcggtggggc tgcggcgcct gggactgcac 360

cgcaccgagg gcgagctcca gaaaattgta caagctggag ataaggacct tgatgggcag 420

ctagactttg aagaatttgt ccattatctc caagatcatg agaagaagct gaggctggtg 480

tttaagagtt tggacaaaaa gaatgatgga cgcattgacg cgcaggagat catgcagtcc 540

ctgcgggact tgggagtcaa gatatctgaa cagcaggcag aaaaaattct caagagcatg 600

gataaaaacg gcacgatgac catcgactgg aacgagtgga gagactacca cctcctccac 660

cccgtggaaa acatccccga gatcatcctc tactggaagc attccacgat ctttgatgtg 720

ggtgagaatc taacggtccc ggatgagttc acagtggagg agaggcagac ggggatgtgg 780

tggagacacc tggtggcagg aggtggggca ggggccgtat ccagaacctg cacggccccc 840

ctggacaggc tcaaggtgct catgcaggtc catgcctccc gcagcaacaa catgggcatc 900

gttggtggct tcactcagat gattcgagaa ggaggggcca ggtcactctg gcggggcaat 960

ggcatcaacg tcctcaaaat tgcccccgaa tcagccatca aattcatggc ctatgagcag 1020

atcaagcgcc ttgttggtag tgaccaggag actctgagga ttcacgagag gcttgtggca 1080

gggtccttgg caggggccat cgcccagagc agcatctacc caatggaggt cctgaagacc 1140

cggatggcgc tgcggaagac aggccagtac tcaggaatgc tggactgcgc caggaggatc 1200

ctggccagag agggggtggc cgccttctac aaaggctatg tccccaacat gctgggcatc 1260

atcccctatg ccggcatcga ccttgcagtc tacgagacgc tcaagaatgc ctggctgcag 1320

cactatgcag tgaacagcgc ggaccccggc gtgtttgtgc tcctggcctg tggcaccatg 1380

tccagtacct gtggccagct ggccagctac cccctggccc tagtcaggac ccggatgcag 1440

gcgcaagcct ctattgaggg cgctccggag gtgaccatga gcagcctctt caaacatatc 1500

ctgcggaccg agggggcctt cgggctgtac agggggctgg cccccaactt catgaaggtc 1560

atcccagctg tgagcatcag ctacgtggtc tacgagaacc tgaagatcac cctgggcgtg 1620

cagtcgcggt gacgggggga gggccgcccg gcagtggact cgctgatcct gggccgcagc 1680

ctggggtgtg cagccatctc attctgtgaa tgtgccaaca ctaagctgtc tcgagccaag 1740

ctgtgaaaac cctagacgca cccgcaggga gggtggggag agctggcagg cccagggctt 1800

gtcctgctga ccccagcaga ccctcctgtt ggttccagcg aagaccacag gcattcctta 1860

gggtccaggg tcagcaggct ccgggctcac atgtgtaagg acaggacatt ttctgcagtg 1920

cctgccaata gcgagcttgg agcctggagg ccggcttagt tcttccattt cacccttgca 1980

gccagctgtt ggccacggcc cctgccctct ggtctgccgt gcatctccct gtgccctctt 2040

gctgcctgcc tgtctgctga ggtaaggtgg gaggagggct acagcccaca tcccaccccc 2100

tcgtccaatc ccataatcca tgatgaaagg tgaggtcacg tggcctccca ggcctgactt 2160

cccaacctac agcattgacg ccaacttggc tgtgaaggaa gaggaaagga tctggccttg 2220

tggtcactgg catctgagcc ctgctgatgg ctggggctct cgggcatgct tgggagtgca 2280

gggggctcgg gctgcctggc ctggctgcac agaaggcaag tgctggggct catggtgctc 2340

tgagctggcc tggaccctgt caggatgggc cccacctcag aaccaaactc actgtcccca 2400

ctgtggcatg agggcagtgg agcaccatgt ttgagggcga agggcagagc gtttgtgtgt 2460

tctggggagg gaaggaaaag gtgttggagg ccttaattat ggactgttgg gaaaagggtt 2520

ttgtccagaa ggacaagccg gacaaatgag cgacttctgt gcttccagag gaagacgagg 2580

gagcaggagc ttggctgact gctcagagtc tgttctgacg ccctgggggt tcctgtccaa 2640

ccccagcagg ggcgcagcgg gaccagcccc acattccact tgtgtcactg cttggaacct 2700

atttattttg tatttatttg aacagagtta tgtcctaact atttttatag atttgtttaa 2760

ttaatagctt gtcattttca agttcatttt ttattcatat ttatgttcat ggttgattgt 2820

accttcccaa gcccgcccag tgggatggga ggaggaggag aaggggggcc ttgggccgct 2880

gcagtcacat ctgtccagag aaattccttt tgggactgga ggcagaaaag cggccagaag 2940

gcagcagccc tggctccttt cctttggcag gttggggaag ggcttgcccc cagccttagg 3000

atttcagggt ttgactgggg gcgtggagag agagggagga acctcaataa ccttgaaggt 3060

ggaatccagt tatttcctgc gctgcgaggg tttctttatt tcactctttt ctgaatgtca 3120

aggcagtgag gtgcctctca ctgtgaattt gtggtgggcg ggggctggag gagagggtgg 3180

gggactggct ccgtccctcc cagccttctg ctgcccttgc ttaacaatgc cggccaactg 3240

gcgacctcac ggttgcactt ccattccacc agaatgacct gatgaggaaa tcttcaatag 3300

gatgcaaaga tcaatgcaaa aattgttata tatgaacata taactggagt cgtcaaaaag 3360

caaattaaga aagaattgga cgttagaagt tgtcatttaa agcagccttc taataaagtt 3420

gtttcaaagc tga 3433

<210> 6
<211> 7772
<212> DNA
<213> Homo sapiens

<400> 6
   agttaagctt tggctcaaat tctacacaca ctcattccag agctctcatg ttctgcacct 60
   caggagggaa ttcagcctca gtgatgtcca tgaggcctgt cccaggctct ctatcttctc 120
   tgctacatct ccacaacaga cagagacagc ccatgccagc ctccatgcct gggaccctgc 180
   ccaaccctac aatgccagga tcttccgccg tcttgatgcc aatggagcga caaatgtcag 240
   tgaactccag catcatgggg atgcaaggtc caaatctcag caacccctgt gcttctcccc 300
   aggtccagcc aatgcattca gaagccaaaa tgaggttgaa ggctgcattg actcaccacc 360
   ctgctgccat gtcaaatggg aacatgaaca ccatggacac catgatggag atgatgggct 420
   cccggcagga ccagacgcca caccatcaca tgcactcgca cccgcatcag caccagacac 480
   tgccacccca tcacccttac ccacaccagc accagcaccc agcacaccat cctcaccctc 540
   aaccccatca ccagcagaac catccacatc accactccca ttcccacctt catgcacacc 600
   cagcacatca ccagacctcg ccacatccgc ccctgcacac cggcaaccaa gcacaggttt 660
   caccagcaac acaacagatg cagccaaccc agacaataca gccaccccag cccacagggg 720
   ggcgccggcg aagggtggta gacgaggatc cggacgagag gcggcggaaa tttctggaac 780
   ggaaccgggc agctgccacc cgctgcagac agaagaggaa ggtctgggtg atgtcattgg 840
   aaaagaaagc agaagaactc acccagacaa acatgcagct tcagaatgaa gtgtctatgt 900
   tgaaaaatga ggtggcccag ctgaaacagt tgttgttaac acataaagac tgcccaataa 960
   cagccatgca gaaagaatca caaggatatc taagtccaga gagtagccct cctgctagtc 1020
   ctgtcccagc ttgctcccag caacaagtca tccagcataa taccatcact acttcctcat 1080
   cggtcagcga ggtggtagga agctccaccc tcagccagct caccactcac agaacagacc 1140
   tgaatccgat tctttaaaat gcaccatcag acctggcctc caagaagagc tgtagcgtac 1200
   catgcgtcct ttcttttaag ggcattttta gaattaactc agacctggaa gactcctcag 1260
   ttcttcaaag actggctttc atttttatag ttattatgga aatgttgtct tttatactta 1320
   gttatataag aaaaaaggga gttatgcaat taatatctat cagcttggga aacgctttgg 1380
   tgcttttctc cagttttctg gtaccagtta cttgtttata aactgaacct tttctgtata 1440
   tagccatggt ttcattctta tcagtccaac cctttgcctg aaacattgaa tcttgttaaa 1500
   ccacagcttt tagctaaaat gaggtatacc tagatgtcaa gtaagacaga tccaaggtaa 1560
   ctgggtagga aatcttttga catcttaact catgttgagt ttgtgctgtg gtgtcaccag 1620
   aattccagat aaacacacag cctttcccat accttttttt ttcttactat aaaatattat 1680
   aagatccatt gatgtccaaa taataccacc aagcatctct tcacctctcc tcctcttggt 1740
   ccacttgcta atgcccagtt ttcttctcca tttccacttt ttcttaggct ccctatttac 1800
   tattcatttt gacttccttc tgttttattt ttttcccttt agcattgcat gtgaataaga 1860
   aaataatgtt taaagaaaaa aaaaaaaaag caaacctcca aaacgtggac ctaaccattg 1920
   cttcacttac acttcaccca cagctggagt tcattcaact cttgcttttc acaaaatagt 1980
   aaccaggaga tgtttaatgt gcctgattta atgtttttaa taatcacagc aaatgaaagg 2040
   tggtttagtt ataagtgaag catggttgaa taccagctgg ggagacacta gggaagggag 2100
   ctttgtaagc cttgattgcg aaagtccaaa ttttgatgtg gggctataac atgacaccct 2160
   tggattgcga ctggttttat acggcctgcc tataacgttg aaaatccatg tactacataa 2220
   taattcagaa gggctctatt cactacacag attacattgt tcaatcatca gctgctaata 2280
   gcctaagatt tatttttttt tttttcttaa gcctatggaa ccggctttgc tgttctgggg 2340
   ggtgaaaata gactaactac tggagaaaca aagagagaaa gaaaacccag tgtttccata 2400
   ggggcacttt tagccttccc acaacagtta agcactcttt gactgctgaa ggaaccccat 2460
   ggatgaggtg caggctactt cactcttttt ttttcttttt tgagacagag tctcacctat 2520
   tgcccagact gaagtgcagt ggtgcgatca tggatcactg cagcagcatc ctccgagttc 2580
   aagctatcct tccacctccg cctcctgagt agctgggacc acaggttcac ataaccatgc 2640
   ctggctaatt tatttttact tttattttaa aataaaagat gaggtctgtc ttatgttgcc 2700
   caggctggtc tcaaactatc ctacttcttc ctcccaaagt gttgggatta taggtgtgag 2760
   ccactgcacc cagcctactt cactcttctg aattattctg atttattttc aacaactttt 2820
   gtgaacttgc ccgtgataca aagcagatag tccctgaacc acagtcgtgc ctccttgaaa 2880
   caagccattc tactgtgcta atgttttaat atcacatctc acaaataaca ggggtgaatg 2940
   tttctctcta gcaatctagg caggtgctgg tgtttcatct ccatttgaat gcttgacctc 3000
   ttaatgtgtg tgtgtgtgtg tgtgtgtgtg tgtgtgttca tgggttttaa aagaacagta 3060
   ttttacaaaa ggtgtagctt ttataagagt gcagaaaagg gaaggatgtg tttttttctc 3120
   tcactatagt ataagaatct attttggaga aaaaaagaaa atatgagggt ctcgaagcat 3180
   gatttttata taactagttt cagttttatc taataactta ctttttaaat caatatttat 3240
   caacaatctt tccttgtatg cagtgctttc aaaagatggt tttgagtgtc cagtgaaact 3300
   tatgacttgg atatatggtt gaagaatcaa aacaaaagca aaaaaaaaaa gcaaaaaaag 3360
   aaaagagaaa aaaagaaaaa atgcaaatgg aataattttc tattatattt tagacaaaca 3420
   tatcattttc gagtatttta aatactgaat tcatagttgt tgttttttaa attccaacag 3480
   taacagctga atggtttaat ctgactggct tcctaagaaa tgtttaagac tcagctttaa 3540
   aaagaagtta acattcatat ctctgttttg aaatcaaaaa tcatatttca aaattctttc 3600
   ctaggaccat ctatgtgtct cccctcccct ccacaaaaag gagaaagagt gcattaaaat 3660
   gtttagttgg gttttttaat ttttaatttt tatgttatgt tttgctttgt tttaagtaaa 3720
   caaaaatttt tctttcttta ctgcatgcat agcacttaat aaaatggatt ttttaaaaat 3780
   ccactagtaa tatcagaatg tccagggagt gactgtcact acaatgatgg tttagtttac 3840
   ttctgttcca ccttttgatt gaaatattta gttgttaggc tgaaagcctc ggcagttaag 3900
   aacttgcctg agttttcttc gttcagcaac ttgacagttt gactgatgtg cattatatat 3960
   agctcaatta tgtctgtttt ttatgctaag taggaaaacc aaccacacac attagcaaac 4020
   cggcctcaac atataattag aataaactgt cttcttgttc tactcagggc ctttaggtgt 4080
   gttcattcac ggtatggaaa tacagtaaat gaaagattcc aactagttgt cagtgcttct 4140
   tgaaattcca aacagaaaga tacattggtc aaatccaaca cttggcttat caatattaag 4200
   tcttttacct aaaggcccag ccgtcaccag acaacagaat aatcaatctg cctgaaaatc 4260
   cctcctcctt gtcctacact ttttgcctgt ttgggagaat atctttgtac tccattctcc 4320
   tccctcagcc agttactggg tcacccatcc atgtgttcat gaatcaatca tcacggcctg 4380
   cagagcacct gtcctaagga gggaaaatcc tgtcacactg cctctcccca ttcgtgtgtg 4440
   gttttcttga tcggtgagat ctgtctctga agtcactgcc agcctccctg ggaacgtcta 4500
   tagtgcctcc cctgccttat gtgatgggag ttaacaactc agataagtac acctgagagc 4560
   atttctatca ggtaaactgt cacttaaatg gaggtgtcca catcttaatt gtttctcctt 4620
   gacacatttc tcaatccacg aagccaggag aggtagagtg aaaatcccag ccatggatga 4680
   atgtactaat ttgaaagcca agtgttaagt cggatgtttt cccgttacac tactactcag 4740
   ccctctcctg cggccacatc aacggatgca agtcacagtc ttaacacagc ctgtgggaga 4800
   caagcagttt gtgtgctcac agtatatatt atagtaatta gggtgactta gagcaaatac 4860
   tcttcagatc ctatgtagtc agtgaaacaa aatggagagc gtattctgat agaaggacgt 4920
   cgacggtgaa tgttctggtg gttgttgcct gttaagtaaa ctttagtgtg taagttgagt 4980
   ttgtcattaa aatcataaac cagctgcggt aacagacaag cctttggctg ggagttttta 5040
   agcctcggta actgctataa aactagccat ccagttagga tagaatgtgt ttctttctgg 5100
   ttaaaaaaag gaaaaaccat ctaagaaaat atatatgtat gtatgtgtgt atacagtgga 5160
   attcaaagga ccaaagcaaa atttgaacag gaatctatta atttagaatt ttataagata 5220
   tttattaata aatgttattt ttaaacattc catttgaaca gtattctgta ggatctactt 5280
   gtttttaaag tgttagtcca taataaacta ctatagttat gtgtattttc atttttcagg 5340
   gtttcaaatg gctattctcc atcatttggt ggaaatgttt gcttagatct ctgtgcatag 5400
   acatttcaag gatttttatt gctctgtgag ttatttttta atcaacattc tgaacagttt 5460
   tttttaaaca tttatttctg tgtgttcatt tttaaagtaa gctctttcat ttaggaagca 5520
   gagttcagct aaagggaatc agtaactcta actggaacag ctttcttgta gaagtgtaaa 5580
   aacagcttca tctctgcctc tctccacccc accccaattt cctagaaagc cttgcactat 5640
   tcagctccct tagtgctttt tgtcccttcc cgaacaatat gcagtagctt taagccattc 5700
   aagctccatt atgcagtata tctgagaagg gaaaggaaac aacccattta aatttgaata 5760
   aaaccgtgcc tatgcgaaca gtagcaattt agaatctctt ttctgctttt aaaataattt 5820
   atatttaaaa attgcacttt agctttttga tccctttgta tttctcttat tctctttcta 5880
   acctcttctc tgtcctcaaa cttgcctttg ctctccttta caataccccc cacccctcct 5940
   ccaaggctct gagcggcatc atttaaaata ctttacagat atttgcacca ggtacattta 6000
   tgtgcgtcca ttggtagcac agctgagacc tgtgtctcac atcagcctag gtgaagccta 6060
   ctacaagaat gccaaggaga agagccagta cactatatgg tttatactct ttatcccttt 6120
   attcatagca tgttttttaa aaatgttata ttatgcaaca gatgtgaggc agcagctaag 6180
   ctatacttaa gaattttctc tcaccttcca aaccaaagtg tcctgaataa gccaggagac 6240
   ttattctttt gtgcaccctg gtgcacatct gactgttgtc ctagccatag actctctgag 6300
   gccactgaaa gaacagtggc cctatcgatt tcattcctag gtctcaaaaa tacaatgttg 6360
   ccttgtaaca taattaggga cagcacctct atttcacaat tataatctaa ggtaggataa 6420
   gacgacacag cagcaataaa cttacaagta aaattcaata ccaaaacaaa cacaaagaaa 6480
   tttaaaaaac aaaaaaccta gctcatcatg ttgtgaaaat gaaaaagtga atgtccattc 6540
   aaaatatttt actatttctt gtggagtttt tcagtgatgt aatgcttgta gccaaattgc 6600
   ttaaagagtg tttatatatt tttttcctta taaattgtct atttttaaaa aaagctattt 6660
   aaccacagct gaagtggggg gtaaggccaa attgccaaca cttgttaaaa gattaatact 6720
   cttaagtggc actctgatac ctttccaact tgtcatcaga aaggaatcaa taattaccaa 6780
   ctgttgtatt tagaccaact tacaatatct agctcattag aagccaggat ctagaaagct 6840
   ccttctaagc catttaagat attcttacat tgagcttcat attatagaac tttataggat 6900
   tggatatttt acaatagaat aatttagcct caggactgag aatgtggaag ctgaataaat 6960
   tagctttaaa tacatcatta aaatcttatg cacaataagc tcattagatt ctagttttct 7020
   cctttagaat accaatgcca cagacactac aggagataat gaaaggtatc agttgtgttg 7080
   agtggaggga gtttaagaga aaggaccctt cccaaccagc agccagtaga aaatacaacc 7140
   tactcacctt tttcccttct aagttctgct aaatcacatc tgcctcatag agaaaggaat 7200
   gttgcctttg agaactgtct tggagaacag ataagcttga aatgttctct ctagagagga 7260
   catagggttt gggatcctct gaaaaggccc agaaaaatag ctcagttcaa atacaatgtt 7320
   ctaggacaat tggaatataa atattgtcca aaaatataat taaaagaaaa aagtttagca 7380
   ctgtgtaaag taagtgttaa ctgaggaagt cccaaaaagg tgctgtcact ttaagttctg 7440
   gacttggggt tctttgtatt tgtaaacagc aaagcatttg tgtttgtttg tctatttgta 7500
   aagcaaccac cttccttatt ggaaggagaa aaaaaggggt acatacatgt aaatacttgc 7560
   tgcagcattt aatatgttta attttgtgtt aagctttttg ttgcatcgtg aacacattta 7620
   ttgttaccaa tggacaatga gttcattaag actgttcaac taggtcagat ttttacatct 7680
   ctttctagca agaagagaca agattttgtg catttgtaca aatgttaata tcactgcaat 7740
   tccaatataa taaagcactc aaatgcaaat aa 7772

<210> 7
<211> 5755
<212> DNA
<213> Homo sapiens

<400> 7
ggcgctcagc gcaggcaggt ccccctgctg ccgggtccca tttgttgccg gctctgactc 60

ggggcggccg cggcgcgcgg agctccgggg agtcaggcgg agcagccgcg cagccacgac 120

ggagcagcag cgggactggc cgccccgcgc ccccttcgcc gccgtgccct tccccggcgc 180

gctcaccccg ttctcgggat gggattgtag cggcggcgcg gactcggcgg ggatcgcggc 240

ggaggcggcg gcgtcggcgg cggcgtcggc ggccgagcgg ggctccatgt tttcccctgg 300

ccaggaggaa cactgcgccc ccaataagga gccagtgaaa tacggggagc tggtggtgct 360

cgggtacaat ggtgctttac ccaatggaga tagaggacgg aggaaaagta gatttgccct 420

ctacaagcgg cccaaggcaa atggtgtcaa acccagcacc gtccatgtga tatccacgcc 480

ccaggcatcc aaggctatca gctgcaaagg tcaacacagt atatcctaca ctttgtcaag 540

gaatcagact gtggtggtgg agtacacaca tgataaggat acggatatgt ttcaggtggg 600

cagatcaaca gaaagcccta tcgacttcgt tgtcacagac acgatttctg gcagccagaa 660

cacggacgaa gcccagatca cacagagcac catatccagg ttcgcctgca ggatcgtgtg 720

cgacaggaat gaaccttaca cagcacggat attcgccgcc ggatttgact cttccaaaaa 780

catatttctt ggagaaaagg cagcaaagtg gaaaaacccc gacggccaca tggatgggct 840

cactactaat ggcgtcctgg tgatgcatcc acgagggggc ttcaccgagg agtcccagcc 900

cggggtctgg cgcgagatct ctgtctgtgg agatgtgtac accttgcgag aaaccaggtc 960

ggcccagcaa cgaggaaagc tggtggaaag tgagaccaac gtcctgcagg acggctccct 1020

cattgacctg tgtggggcca ctctcctctg gagaacagca gatgggcttt ttcatactcc 1080

aactcagaag cacatagaag ccctccggca ggagattaac gccgcccggc ctcagtgtcc 1140

tgtggggctc aacaccctgg ccttccccag catcaacagg aaagaggtgg tggaggagaa 1200

gcagccctgg gcatatctca gttgtggcca cgtgcacggg taccacaact ggggccatcg 1260

gagtgacacg gaggccaacg agagggagtg tcccatgtgc aggactgtgg gcccctatgt 1320

gcctctctgg cttggctgtg aggcaggatt ttatgtagac gcaggaccgc caactcatgc 1380

tttcactccc tgtggacacg tgtgctcgga gaagtctgca aaatactggt ctcagatccc 1440

gttgcctcat ggaactcatg catttcacgc tgcttgccct ttctgtgcta cacagctggt 1500

tggggagcaa aactgcatca aattaatttt ccaaggtcca attgactgac gcccttgaca 1560

gccatctacg actttattaa caggttactg tgaagatttt gccactaact ctagatttta 1620

cctttttgta atgctgttta tcagaggagg gtgacagggg ctggaaataa agagagggga 1680

catggtgatg aaacatggca ggagtgtaac agataccagt ggtgtgttgc atgctcaaaa 1740

cagcagcgtc gtcattgaag tctgcttgat taaaccataa tatctttgta ataattggat 1800

ttaaaatgct atgcttctat ttttaacctt gggtttttaa ccaagttttt ttttttttgt 1860

aatcttggac aagactttaa atcatatttt acagatgtag aagaaattta ttcaaaagtg 1920

tgggctcatg aagttcactt cagtgcagtg tggtgtaggt gttacgcgaa gggcgcacag 1980

tgtctagaaa tacttgatcg tggctcaaac ctgaccagac agcagagggg cggctctgta 2040

cagtgtgact ggtggacaga tggccttagg cacaggtggt tttgaaatct ggggcttttt 2100

ctgatttatt tttctgactt gttgggggag agaatattca taacttgtgg gctttttttt 2160

tttttaactt cagtggaatt tactttagat attcattcat caaatacatg ggacttcaca 2220

aacaattttc cataactttt tagcctgtct tttgttattt ctgcctaata tgatttgccc 2280

cgatactcat cttgcacggc cagaactgtt tggttgatta aaatacatca gctcttaaaa 2340

actcattaac tgagggtaat tacagtagta gacatggtct gggtactata ctaccatgtt 2400

tatttgctga ctgaattaag atttaagaat gattaaaaat aagcttttac tttttaaaac 2460

cacttgaggt ttcataaagc ttggggtttt tttttttcct ttgttaagaa agccaaccaa 2520

tcacaatgat atagtcattg ttgtgcactc ccttttcacc atctgtcacc ttcccttgca 2580

gcttaaggag cccagtaagt tttgaaaatg tttgcgaatc aaactaaatt taagtgggat 2640

gattagatat acacaacacc aagtggtaca tctgcagaga taattcaaaa ttcctgcttt 2700

tgagagagca aatgagtgtt gctgaggaat aattaaatga gaatttcata ggagctccac 2760

cattcctgtt actttcattt cattttgatt aataattctt ggatgcttgg catcgatcgt 2820

atcactgctc ctagaaggta aagatccttt aggacatgag actggtagaa gctggctgag 2880

ataaaataag tatttattta aactaatgtt ctcttaattt gaccattgca gatttgggtg 2940

actttttttt aacctttgta catatacgta atttatatga ttctaatgta ctatatccat 3000

acttgaattg gttttttcgt attttgccta ctggcaaata ttttgcctat tttcagtcgt 3060

tctaacctat ttgaatacgc ttttccttaa agtgatacga taatattact cttgattgct 3120

gctgcttaat ttgatgtaat atgtttaaag ttcagcctct cagttttaat atagctttat 3180

ttttcagtgg agatcattgt ttaggatgag acatttttgg ttttggtttt gtttgggtaa 3240

attttaaatg gtgtgaaaat cgatgacaac agtcctctta cagatagctt gctgtattct 3300

gtatagctta ctctacctgc agacagaaaa tgaaagaaaa aaatggactt gcctagaata 3360

atatattgaa tgccttttga tttagccaga gtctctgatg attagctttc actgatagag 3420

tatgtctttt cagcctgtaa ttctttgggc cccaaagaat gacaaaggag gcactcgttc 3480

tcttttcttg ctgtatgcct agaaagtggt tgaaggattc ttgatgccct aaaaccatct 3540

tgtaagctaa atggtcttgc atccagaaag gccagatttt acctaccaag aaaaaaagat 3600

atttttccag agagttaggt atatcataat tttccatttc aagtcctgtt tataagtcta 3660

gtcattctgc aacgtgacat atcccccaaa atgaagttac cttccaagtt ggacacgtcc 3720

cgtagttggg catatgtcta actaaaagtt tctgactttt agtaaattca gcttaaatat 3780

aagttgaaat ttgggaaata atttccaagc tcttggaagg ggtaacagtg aaccgccctc 3840

catgggctcc acatcttttc ctttggcttc caaagtcagg tcccgcccac cctgcctaag 3900

gaactgcaga gaggtggcaa atcagcaaaa aggacaccag gctcttcttg gccacttgta 3960

ggaagatccc tttacaattt tgactaagga gatttttttt ttcacagttg agttagtttg 4020

tgaaaataaa gaactctgta gctcaccaag gtggagaaac gcaattcaga aaagtaattt 4080

ctccaaggtc acttcttttt ttatgtcttg ccatcacttt aaaggactag ccccactccc 4140

ccatgtgtat acacaaggaa attgcagacc aattagttgt cttggcctga ctctaatgcc 4200

ttttgcaagt agctttccag aagtaaaagt cccagtgatg tattcccata gaaatatttt 4260

tcagttgttt atgtcgttta ctacaaaaaa aaagattcag agtggatgga gtacaactct 4320

gagtattttt ctagtccgga atttttattt aataatcggt gctgccgggt catgcatgct 4380

gcaactctca acatttccct tatttggttc agcttttagc aaaaagggct acagttcacc 4440

ctgcagagta ttaaggtttc tggatttttt tctcccaact gtggcccaaa agaattaaaa 4500

tctgttaata taaatagaga acatatttat cattcctcga tagttaatta tagactttgg 4560

tacctttgtg cctcagggaa gccacgtgat ataactggtt atagaatttc agggttaggg 4620

tttaaagaaa ggagaaagcc attggaaaaa tgatgggctc cattaaggag actaatgaat 4680

ctggatgcag aaatatgtca gaaactggca taaacatgat tgtagtagaa tttattttcc 4740

agtaccaata gggaaattat tttaagttat tacatttact gtattgggaa acttgaggag 4800

aactctttag ttcataaagc ttcaatgtct tttttttttt tttcatggaa aaactcaaac 4860

ctctgttatt tgggagctca gtattgtgtg gacacttacg agagttttct gcttaattga 4920

agtgtaatat aggttgtaga attgttacct gcagttctat ggttttgttt cacttctttt 4980

cttttttaaa gccattctgt tctttggatg tgcttgaaag ggtgtgtgat tacaccattg 5040

ttaatgctgg gtaaaaacta tcttcttgca gccttgcctc ataacagtgg aatttctgat 5100

agacaaacca caggactttg attttaagcc aaatccatct ccatcccttt actgtcaatc 5160

ttctgtccca gtagtttagc ctttgtggct taggttatga tgcgcctcct tctgtgcgac 5220

caatgagacg acttcagcat ctttttaaaa taatctaagc atcattgaag cagtaacaca 5280

aaaaaaaggt tcagtatttt ctttttagta taacttacat cctttcaaat aagtctttgc 5340

cctcatgaag aatccctaga ggaagataag gaaaataagt attttccagt tttgcttgac 5400

agtttctaaa caaacaaaaa taaactcaat gaaaggaaag atgtttcttt ttagctgaga 5460

tgacagattg cttctctgta ttaaatagtc tagaagttaa ggggatggtc acatttacca 5520

tgtattgtgt tattagcagt taaattttat gaatatgttt gtaaaattgt tgttttatat 5580

ttcatgtcaa attgaaaagt ttatttcttc actattgtac ctgtggaaat acaagccatt 5640

ttacaggaaa aaatcttcaa aaactattaa atggatatca gcctgtttgt gagccaaaaa 5700

aaaaaaaaaa aaaaaaaaaa aaaaaaaaaa aaaaaaaaaa aaaaaaaaaa aaaaa 5755

<210> 8
<211> 4208
<212> DNA
<213> Homo sapiens

<400> 8
gtctcagcgc gcctgcgccg agcggccctg cgcgcagtga ggcagtggcg ggggaaggca 60

ccggtggggc cgacgggcgg gttgaaggag ggaagcgggc gagcgaagtc ccagtgcgcg 120

ccgcggcagc ccgggcaccc tccccttccg ggcgtgagtc gctgtgaaaa gagctgaagc 180

gagcggactc gcaccggcag cgaggcgccg ctcccgccgc ctcagcccgg ccttcctcgg 240

ctccggcgct ccggtcgcgg ggcccgggtt cctcggcaca ccccgctcca gccgccccca 300

gagcctgtcc ccagcccttc ggaagccccg gcgccagccc gggccctcgg cagggaggat 360

gacggagctg cagtcggcac tgctactgcg aagacagctg gcagaactca acaaaaatcc 420

agtggaaggc ttttctgcag gtttaataga tgacaatgat ctctaccgat gggaagtcct 480

tattattggc cctccagata cactttatga aggtggtgtt tttaaggctc atcttacttt 540

cccaaaagat tatcccctcc gacctcctaa aatgaaattc attacagaaa tctggcaccc 600

aaatgttgat aaaaatggtg atgtgtgcat ttctattctt catgagcctg gggaagataa 660

gtatggttat gaaaagccag aggaacgctg gctccctatc cacactgtgg aaaccatcat 720

gattagtgtc atttctatgc tggcagaccc taatggagac tcacctgcta atgttgatgc 780

tgcgaaagaa tggagggaag atagaaatgg agaatttaaa agaaaagttg cccgctgtgt 840

aagaaaaagc caagagactg cttttgagtg acatttattt agcagctagt aacttcactt 900

atttcagggt ctccaattga gaaacatggc actgtttttc ctgcactcta cccacctatt 960

gctggacttc tgttgtacaa gttggcaaac actggctgga actgggctgc aataaaacat 1020

gccagttatc aatgctgaca agagcctaac aagtgccaac ttacagatga ttacgcattt 1080

tgaattctaa tgaactgttt taaccttcag gaagaattgt aaagacctgt acatagcaca 1140

acatgatccg gataatatat atactgttca tgtacatcca caaatacacc ttgtaccaaa 1200

taatgctttc ttgtagtaga ataagaatcg tgtaaattct aagagatttt agcaggtttt 1260

ctttcctatt cattgtttct tatcagttta aaaggattcc tttaagcatg tcagatgaaa 1320

agcaattagg attaaaagtt tccatttaat ttcccttaaa cccttgaggc ttcattaaac 1380

tcttttcact tactaaactt ttgtatcttc tttgttttga cacactcccc tttgctttta 1440

tctcttacct gccagaatgt tctcaaatga tttagttcaa atactgaaat acttaatgag 1500

caattacttg atttttaatg atgacttcga aggagtcatc actaggtgct ttgtcctttt 1560

tgtattctag ttgcacccac ctcttggatt ggatatagca ataacattta ttggccgttg 1620

tgagctcttg atcccagtca ttacccctga gaactaaaaa tagatggttc ttaattcaac 1680

ttactgaaaa tttccccaaa caatagcaaa tctgactttt ccctcttcag ttgcctggta 1740

ttaaggttgg ataaatgaag catgcacagc tacaggcttt ctacttaact tctgggtttg 1800

ctattacaaa tcctatttac tctcataccc ttctccttag tccttcatat ttctctgcct 1860

ctattcttct atactgcaga tttttctcac ctattgtaca aagaaattgc gatgtatatt 1920

ttcatgtaat ttgattttgg aattctgtca ccttatgtag tgagttcttc caaaatataa 1980

ttttttttca ataattgtca agttgttggc ttttattgta ttgaatgaag gctataatac 2040

tgagtgccag agaagtggtt taggaaaatc tcaggttgat tccttatgca aatgaacttt 2100

taatacttga aaatcacatg gccatggcag tatatgtatt tggttctatc tagattcttc 2160

tgtgaatcta aaagcattac aggggtaaat gctttgctat ttgacgtata gatcccgtca 2220

ctaacaatag tacacttgga tgtgattaat gtttgagctt caatatattt catatcatac 2280

agttttctaa aacaacttca gcaaatggta aaatgaacat gtgcagtgtt aaaggcaggc 2340

cttaggctcc ttcatgtttg ttgtgaggtt gtgtgtggga agtagtcttt ggcttataag 2400

ggatagaact tgagacagta gcagatggga catggtgttt gattgtgaga atcagtgaga 2460

attcgtgcat ctctgctctg tggggtttgg agaaatgctt tggcagaaga gtgaaagaac 2520

tcctgccaag agcccagacc tctacaaacg ttgtatgtcc ttttttaagc agaaataaaa 2580

tggttgagga tgtagtcaca gtagagagtg atttttttct aagtccctgt cctctactct 2640

gaagcgttat aaaaacctgt aaacattata caaacccaca aaccttatag aaactcgtaa 2700

gtgtgttgtg actggaaatt gattcattag aacccagttt tctttaagaa ctttgtgact 2760

tggttttttt tttccttttc caaagactgt aaaaatagtt gccccaaaat gtcagcactg 2820

cacaccctcc agggacttgg aatacaatcc tttttacttt tttttttttt tttttaagaa 2880

actgggtctc tctgtcaccc aggctggagt gcagtggcaa cgatcatagc taactgcagc 2940

tttgacctcc tgggctcaag tgatcctcct gcctcagcct cctgagtagc taggactaca 3000

ggtgtatgcc accatgcctg gctaatacaa aaaaattctt ttaagagatg ggatccctcc 3060

cttttaaaat cagaacttgt tcacatggtg gttgcttgtg gcaaaacgga gttcaaattt 3120

tgctctccta ttgctataat tctgctagca atctgttgag gtgaaacttg ggatctgact 3180

cttcagcaag cagcaaatga cctagtaact cagggacaac tatttttgaa ctttaagtgc 3240

cactttaatg cagttagttt gataaaacca tgtgggtttt ttttttaggg ctagctctac 3300

gggagtggaa gtgagagcca ggcatgagtg cgtctccaca tgcttttcca cctgccctga 3360

gtgtgttaca tactgaaaca ggcctacata gatgttacaa cttcccttcc tctgtcggag 3420

atgtcatctg tgcctttctc agtgttcatc tgataatgta aatttaaatg cctctacatt 3480

tgatacgaaa cccacattca ggtgacactg aacgaggtgg cttttgtccc accagtgcct 3540

catcagtgtg aggcgattcc tctctgcttt aggaaaatga tttttccccc taaacttgtg 3600

ccaaccatca acaacatctc catagatctt atggattgta gaactgttgg ctgtttccta 3660

aatttattcc aagttctcgt agaggcatat agatttcagt ctgtgcttgt atgggataga 3720

tgatctgagt ggctttctgg cctctttttt gagtttaaaa tccatatgag gttgacgtgt 3780

catactaagg taacatgttt gtgaggttat tccactagta ctgtgatcac gtgggtgtca 3840

gtatctttaa cggccttcat tcttggttgt gagattttat ttgatatgcc cactcaccct 3900

cgacgaatct gcccgctttg ggctgtggtg cctgtgtatc tttgcccgtc tggtctccag 3960

ttggtggaat tacctttttt gtactgccac ttctcagcat ctttgaaatt tgacataatg 4020

ttgcttcatt tcagtttttt tagttctgta atttgttgat tgtatttaac tatgtgagtt 4080

ctgttgtgat gtttactgta ttgtaaagca cctcattcat gtgatgagtg ctctataaat 4140

caataaatga tgacttagag gctgtatcac gagctatttt ggttttagga tgcaggtctc 4200

aaaagcaa 4208

<210> 9
<211> 1424
<212> DNA
<213> Homo sapiens

<400> 9
gctgtggtaa gtgcatcctc cttcagtctc agttctgaaa atagatcatc atggtggcac 60

caaagagtca cacagatgac tgggctcctg ggcctttctc cagtaagcca cagaggagtc 120

agctgcaaat attctcttct gttctacaga cctctctcct cttcctgctc atgggactaa 180

gagcctctgg aaaggactca gccccaacag tggtgtcagg gatcctaggg ggttccgtga 240

ctctccccct aaacatctca gtagacacag agattgagaa cgtcatctgg attggtccca 300

aaaatgctct tgctttcgca cgtcccaaag aaaatgtaac cattatggtc aaaagctacc 360

tgggccgact agacatcacc aagtggagtt actccctgtg catcagcaat ctgactctga 420

atgatgcagg atcctacaaa gcccagataa accaaaggaa ttttgaagtc accactgagg 480

aggaattcac cctgttcgtc tatgcaccat ttattgaaaa gttgtccgtc cacgtcatcg 540

agggtgacca ccgcacactc ctggagggca gcggcctgga gtccatcatc agcaccctgg 600

ctgagccacg tgtgagcgtg cgggagggct aggccctcgc ccccacctgc cactggagac 660

cgctccgcca tccccacctc accgccgcgc agcagagctg gaagggtcct gccgatggga 720

ccctgccagg cccagtgcca ctgccccccg aggctgctag acgtgggcgt taggcgtgtc 780

ccacccaccc gccgcctccc atggcacgtc gggaacaccg gagccgccaa cttggagact 840

cctggtctgt gaagagccgc tgacgcccgc aggaaccggg ctgggccttg tgtgccagtg 900

gggtttgtgc ttggtctttc tccgcttgga tttgcttatt tattgcattg ctggtagaga 960

ctcccaagcc tgtccaccct gcaaagactc ctcgggcagc atgcgggtcc cgcacactgc 1020

acccatttcc tggatgtccc ctgcaggcgc gggaggccat ccgggcctgc tggctgcggc 1080

cccctctcag ccaggcctgg ctcagcccac tgcgtgggag gtcaccggcc actccccgag 1140

gagctgggat ccccgggatg caggcccaca gtgcggggct gcacccatga tgcggagctg 1200

gcctccaacc ctgcgggccg cgccaggcac caactcagtg tttgtcagtg tttgtttttc 1260

caagaaatgg ttcaaattgc tgctcagatt tttaaattta ctgtagctgc cagtgtacac 1320

gtgtggaccc cattttattt ttacaccaat ttggtgaaaa tgctgctttc ctcagcctcc 1380

ccacaattaa actgcacatg gtctctaaaa aaaaaaaaaa aaaa 1424

<210> 10
<211> 2675
<212> DNA
<213> Homo sapiens

<400> 10
gaaattctta caaaaactga aagtgaaatg aggaagacag attgagcaat ccaatcggag 60

ggtaaatgcc agcaaaccta ctgtacagta ggggtagaga tgcagaaagg cagaaaggag 120

aaaattcagg ataactctcc tgaggggtga gccaagccct gccatgtagt gcacgcagga 180

catcaacaaa cacagataac aggaaatgat ccattccctg tggtcactta ttctaaaggc 240

cccaaccttc aaagttcaag tagtgatatg gatgactcca cagaaaggga gcagtcacgc 300

cttacttctt gccttaagaa aagagaagaa atgaaactga aggagtgtgt ttccatcctc 360

ccacggaagg aaagcccctc tgtccgatcc tccaaagacg gaaagctgct ggctgcaacc 420

ttgctgctgg cactgctgtc ttgctgcctc acggtggtgt ctttctacca ggtggccgcc 480

ctgcaagggg acctggccag cctccgggca gagctgcagg gccaccacgc ggagaagctg 540

ccagcaggag caggagcccc caaggccggc ctggaggaag ctccagctgt caccgcggga 600

ctgaaaatct ttgaaccacc agctccagga gaaggcaact ccagtcagaa cagcagaaat 660

aagcgtgccg ttcagggtcc agaagaaaca gtcactcaag actgcttgca actgattgca 720

gacagtgaaa caccaactat acaaaaagga tcttacacat ttgttccatg gcttctcagc 780

tttaaaaggg gaagtgccct agaagaaaaa gagaataaaa tattggtcaa agaaactggt 840

tactttttta tatatggtca ggttttatat actgataaga cctacgccat gggacatcta 900

attcagagga agaaggtcca tgtctttggg gatgaattga gtctggtgac tttgtttcga 960

tgtattcaaa atatgcctga aacactaccc aataattcct gctattcagc tggcattgca 1020

aaactggaag aaggagatga actccaactt gcaataccaa gagaaaatgc acaaatatca 1080

ctggatggag atgtcacatt ttttggtgca ttgaaactgc tgtgacctac ttacaccatg 1140

tctgtagcta ttttcctccc tttctctgta cctctaagaa gaaagaatct aactgaaaat 1200

accaaaaaaa aaaaaaaaaa aaaaaaaaaa aaaagtagtt accattgcct tttctgtgag 1260

ctatttgttt tggtttgctg aaactagtcc aaaacaggaa atttaacaga cagccacagc 1320

caaagagtgt catgtgaatt acaagaaata gagcccattt agggaaagat agaactagaa 1380

aggcttttca ttataattcc atgttgaaca attgagtcat agcttcttat cttggaggaa 1440

ggacacaatt caaaggggca gtaaggattt tgtaaaacgt ggcatccata atttactatg 1500

gagcaagtgc ccacatctct aggacattaa gacatttatg agaaatctca ggattcatct 1560

tctgttttta tgttaaatgc actccctcct tttcagttaa cattataaaa agtaaaaaat 1620

gaaaatttta gaaatcttgc attagacaca tgaaaaaata actaaaagtt taaatttaaa 1680

tatgaaacaa ttttgctgaa aatagtatcc atatactatt taagtctttt atggttattt 1740

caagtataca atttctatct gtaatgtaat atattaccca cacatttttt tcacaggaga 1800

gagagaatat cctcatttgt ttatgctcat gtgtattttc tatagtgaat ttcagaaact 1860

tttaatatca ggtaatttca atttatgcct ataaagcatt gattgaaaaa taactagaat 1920

tgtgcatata taacacataa tctccaacag aagttactga atacattcat actaatgtaa 1980

tgtaatttcc ctttatttct tgctcttctg tttcaaactg ctgctattgt agtttacata 2040

tcccaacctt taaaaatatt cctcttatta gctttatatt cactttatag aagttgagtt 2100

ttaattaaaa ttcttggcat cctgaagtat gtcacatagc atgtgctcct tataaatatg 2160

ttgatatctc agaagacagc atcccggttt tcattttata aagtaccata cttaagaatg 2220

ctgtaatact tatcttttat aacatgtttc cttcgctttg cttgtctttt atgtcatcag 2280

ttttaactgt ttacttcatt taacagttta catcattcaa cagtttactt cattaaacag 2340

taggtggaaa aatagatgcc agtctatgaa aatcttccca tctatatcaa aatacttttc 2400

aaggatatac ttttcaaaac aaacgattta aattttatgt ttaaaatata aactttagat 2460

ttaaacttta tttaaatatc tggttcctat gattttgact tcagtaagtt caaataaaat 2520

atattttgca attcattttt acattataat ttaaaaagaa gaagcgataa gtggagtcag 2580

tttcaatgct aggtggggtg gttaatgatt tttctggtgt tgctgctaat gtggattaac 2640

aaataaaaac attcattgcc ttttgcctca taaaa 2675

<210> 11
<211> 4109
<212> DNA
<213> Homo sapiens

<400> 11
cgcacatgtg tttctgtttt gtgttgtagc atttgttctg gaagctcgta tttacatttt 60

aagtgtatct ggtgagtggg ctggagccct cgtctgggcc ggaaaaaaaa aagccctccg 120

atccgtcttt tagttgcttc tcttcctttt ttctctccgg tttctcatca ctccaaccag 180

ccgcgaccat gcccaggaag aaggcggcgg cggcggcctg ggaggagccg agctcgggca 240

acggcactgc ccgcgccggg cccaggaaac gcggcggccc ggcgggcagg aagcgcgagc 300

ggcccgagcg ctgcagtagc agcagcggcg gcggcagcag cggcgacgag gacggcctgg 360

agctcgacgg ggcccccggc gggggcaagc gcgcggcgcg gccggcgaca gcaggcaagg 420

cgggcggcgc ggccgtggtc atcaccgaac ccgagcacac caaggagcgc gtcaaacttg 480

aagggtcaaa gtgcaaaggg cagcttttga tttttggggc aaccaactgg gacttgattg 540

gtcgaaaaga agtgcctaaa cagcaagctg cttaccgcaa tctcggtcag aatttgtggg 600

ggccccacag atatgggtgc ctggcggggg tccgggtgcg gacagtggtc tcgggctcgt 660

gtgctgcaca cagcctcctc atcaccacgg aagggaagct gtggagctgg ggtcgaaatg 720

agaaggggca gctgggacat ggtgacacca agagagtaga agcccctaga ctcatcgagg 780

gtcttagcca cgaagtgatt gtgtctgcag catgtgggcg gaaccacacc ttggccttga 840

cggaaacggg ctccgtgttt gcgtttgggg aaaacaagat ggggcagctg ggccttggca 900

accagacaga cgctgttccc agccccgcgc agataatgta caacggccag ccaattacca 960

aaatggcctg tggggctgaa ttcagtatga taatggactg caaaggaaac ctctattcct 1020

ttgggtgccc tgaatatggt cagctgggac acaactcaga tgggaagttc atcgcccggg 1080

cacagcggat agagtacgac tgtgaactag ttccccggcg agtggccatc ttcattgaga 1140

agacgaaaga tggacagatt ctgcctgtac caaacgtggt tgtacgagac gtggcctgtg 1200

gcgctaacca cacgctggtc ctggactccc agaagcgagt cttctcctgg ggctttggtg 1260

gctatggccg gctgggccac gcagagcaga aggatgagat ggtcccccgc ctggtgaagc 1320

tgtttgactt ccctgggcgt ggggcttccc agatctatgc tggttacacc tgctcctttg 1380

ctgtcagtga agtgggtggt ctgtttttct ggggggccac caacacctcc cgtgaatcta 1440

ccatgtaccc aaaagcagtg caggacctct gcggctggag aatccggagc ctggcttgtg 1500

ggaagagcag catcattgtg gccgccgatg agagcaccat cagctggggt ccgtcaccga 1560

cctttgggga actgggctac ggggaccaca agcccaagtc ttccactgca gcccaggagg 1620

taaagactct ggatggcatt ttctcagagc aggtcgccat gggctactca cactccttgg 1680

tgatagcaag agatgaaagt gagactgaga aagagaagat caagaaactg ccagaataca 1740

acccccgaac cctctgatgc tcccggagac tcctccgact ccacacctct cgcggcagct 1800

gtcatttcca tgtgcactgg gacgggaagt caaacgagga atttaaaaaa gcaaaagttg 1860

accgaaggtg catttttgtt tagactccct gaggttccgt tttacacatg atccaacgtt 1920

aactaccttt ttttctgtat gctttccaaa gtcctttttt ttcccttaat gttgaattaa 1980

aatacttgct catagttgat ttaccattcc tacaaaagag gcagaaactt tgagcaatct 2040

aggttttttt tttttttaag ttttttcttt cttcctctcc tgaatacact ccccaaaaca 2100

cccctttcca gttacaatta gcatcgtgat ccaagcagat gccacatgga agaggaatcg 2160

ccatttactc agaaaaaatg tcccttacag gaaccggcag cagctaggca gtcaccggcc 2220

cgcctccatc caaaatcacg ctcgcgtgct tcggaagcat ccgggtcact ccttctccgc 2280

tttttcttgc agatgggcct aggccggtgt cggttctgtt tctccccttg gctgcctgta 2340

cgcccacagc cttctggctg cgacattata gaatcggccg tgtcccccct ggtgggggat 2400

tggggatctg tgtttagcca tttatatcta ctttagctgt taaagaggtc caaatgaaaa 2460

tcaggtgatt gtggaaccat ggggacttgg gggtggggca gaggtgggaa catttgtatc 2520

agttgagtca gcttggtggc tccctgtgga gccagggctg agccttgtca cgcgcactcg 2580

ccaattaaga gatggaccag ccagcagtca agtgcattct ccagtccttg caagaaggat 2640

cagccctttc tgtgccagcc tcgatcgcct tgtgctttgg tctctttttc tcccccccgc 2700

ctggatcctg cctcgcgcgg gccgtcctgt tgctgagact cggggtaccg ttctgctgac 2760

ccagctccct ttagtcacgt ttgcttggct ctggtaccaa atagttggga ttaccgaaga 2820

gtccccttcc ttgcgtgtca gcacggatgc tgtgactgcc acctgcgtcc tcgtcaagtg 2880

cccgagctcg ccgccgtgtg tgctgcgctg agtgagttat gaggtgcctt tcccggaacc 2940

ctcctctcgc ctggacccaa gagaggcgac agctgtggct ggggctcttg gtttccagag 3000

ggtctggact ggtttgggtg ctttaaaata gatatttagt tcagtggtgc ttatggggga 3060

gatgggacta gaacttaagt gtgagacttg ggtggatggg aaagttaaat attggtctct 3120

tcaagttttt tttttctttt gctttgttac cacttgtcac tgtctccatg ttaaaatgcc 3180

aaaaatgatg tagttgttgt tgcttttttc cctattttcc accccagtcg ctccttaccg 3240

tgactcctgc ccttggaggg catgtagcag tgtctgtcct gccagtccca aggccctgtg 3300

ggaggagact ggcctgcatc tctctaagac ttagtctgac gccacgcgca tctcttgttc 3360

tgtgttcaat cagtagtcca ggggagaagc ttctgctact tcagagcttt gctaaactaa 3420

cctaatttgt ccaaatcacc ccaaaaccac catctctgac gtaagcttcc atgcgacagc 3480

ctgatccgtt tccctggaca ggtctctttc ctggaatgca gcccaggcac ctgtgctcct 3540

ggcacccttg aggtctctcc tttgagccgt ggtcaccgag agggttgagg acgcagcacc 3600

cgaggtccca gcctttgcag gagcctccct gggcttagct ggacttagat cttcggtggc 3660

ctcatgtaaa cgtggcagcc agcctcttct agaaccctag cccagggact ggagcaggaa 3720

agggaccttc aaagtgaaga ctgccttgtc ccgcagctcc ttctggctta gattgaaaaa 3780

tgggcttcct aatgggttaa atcctttaaa acaaggagtt gtgggggaag ggtgtcgtgc 3840

actcctagag aaaggtacac agttgcccgg ttgggaatgt gcttggcgct gaccctgcgg 3900

gcatctgact ggtcttccag ctcaggaaaa agaatttgaa agaggcttag cgtgaagggg 3960

aatcaaagag gaggttgtga tttggtcgaa ggtgcctggt ttagtgctgt aattgtctta 4020

ttattttttt tatatatata tttcttggag taaacatttt aaataaacaa caacattgtc 4080

tactgtcttg gaaaaaaaaa aaaaaaaaa 4109

<210> 12
<211> 1347
<212> DNA
<213> Homo sapiens

<400> 12
gtggacggag aaagtgaaag gtgaggcacg gccctgcaga ttttccagcg gatcccccgg 60

tggcctcatg tcgcgcagtg gaaccgatcc tcagcaacgc cagcaggcgt cagaggcgga 120

cgccgcagca gcaaccttcc gggcaaacga ccatcagcat atccgctaca acccgctgca 180

ggatgagtgg gtgctggtgt cagctcaccg catgaagcgg ccctggcagg gtcaagtgga 240

gccccagctt ctgaagacag tgccccgcca tgaccctctc aaccctctgt gtcctggggc 300

catccgagcc aacggagagg tgaatcccca gtacgatagc accttcctgt ttgacaacga 360

cttcccagct ctgcagcctg atgcccccag tccaggaccc agtgatcatc cccttttcca 420

agcaaagtct gctcgaggag tctgtaaggt catgtgcttc cacccctggt cggatgtaac 480

gctgccactc atgtcggtcc ctgagatccg ggctgttgtt gatgcatggg cctcagtcac 540

agaggagctg ggtgcccagt acccttgggt gcagatcttt gaaaacaaag gtgccatgat 600

gggctgttct aacccccacc cccactgcca ggtatgggcc agcagtttcc tgccagatat 660

tgcccagcgt gaggagcgat ctcagcaggc ctataagagt cagcatggag agcccctgct 720

aatggagtac agccgccagg agctactcag gaaggaacgt ctggtcctaa ccagtgagca 780

ctggttagta ctggtcccct tctgggcaac atggccctac cagacactgc tgctgccccg 840

tcggcatgtg cggcggctac ctgagctgac ccctgctgag cgtgatgatc tagcctccat 900

catgaagaag ctcttgacca agtatgacaa cctctttgag acgtcctttc cctactccat 960

gggctggcat ggggctccca caggatcaga ggctggggcc aactggaacc attggcagct 1020

gcacgctcat tactaccctc cgctcctgcg ctctgccact gtccggaaat tcatggttgg 1080

ctacgaaatg cttgctcagg ctcagaggga cctcacccct gagcaggctg cagagagact 1140

aagggcactt cctgaggttc attaccacct ggggcagaag gacagggaga cagcaaccat 1200

cgcctgacca cgccgaccac agggccttga atcctttttt gttttcaaca gtcttgctga 1260

attaagcaga aagggccttg aatcctggcc tggaatttgg gcagatatag cattaataaa 1320

actgtgcatc tcaaaaaaaa aaaaaaa 1347

<210> 13
<211> 1527
<212> DNA
<213> Homo sapiens

<400> 13
ggggatgtgg cccgtggcct agctcgtcaa gttgccgtgg cgcggagaac tctgcaaaac 60

aagaggctga ggattgcgtt agagataaac cagttcacgc cggagccccg tgagggaagc 120

gtctccgttg ggtccggccg ctctgcggga ctctgaggaa aagctcgcac caggtggacg 180

cggatctgtc aacatgggta aaggagaccc caacaagccg cggggcaaaa tgtcctcgta 240

cgccttcttc gtgcagacct gccgggaaga gcacaagaag aaacacccgg actcttccgt 300

caatttcgcg gaattctcca agaagtgttc ggagagatgg aagaccatgt ctgcaaagga 360

gaagtcgaag tttgaagata tggcaaaaag tgacaaagct cgctatgaca gggagatgaa 420

aaattacgtt cctcccaaag gtgataagaa ggggaagaaa aaggacccca atgctcctaa 480

aaggccacca tctgccttct tcctgttttg ctctgaacat cgcccaaaga tcaaaagtga 540

acaccctggc ctatccattg gggatactgc aaagaaattg ggtgaaatgt ggtctgagca 600

gtcagccaaa gataaacaac catatgaaca gaaagcagct aagctaaagg agaaatatga 660

aaaggatatt gctgcatatc gtgccaaggg caaaagtgaa gcaggaaaga agggccctgg 720

caggccaaca ggctcaaaga agaagaacga accagaagat gaggaggagg aggaggaaga 780

agaagatgaa gatgaggagg aagaggatga agatgaagaa taaatggcta tcctttaatg 840

atgcgtgtgg aatgtgtgtg tgtgctcagg caattatttt gctaagaatg tgaattcaag 900

tgcagctcaa tactagcttc agtataaaaa ctgtacagat ttttgtatag ctgataagat 960

tctctgtaga gaaaatactt ttaaaaaatg caggttgtag ctttttgatg ggctactcat 1020

acagttagat tttacagctt ctgatgttga atgttcctaa atatttaatg gtttttttaa 1080

tttcttgtgt atggtagcac agcaaacttg taggaattag tatcaatagt aaattttggg 1140

ttttttagga tgttgcattt cgttttttta aaaaaaattt tgtaataaaa ttatgtatat 1200

tatttctatt gtctttgtct taatatgcta agttaatttt cactttaaaa aagccatttg 1260

aagaccagag ctatgttgat ttttttcggt atttctgcct agtagttctt agacacagtt 1320

gacctagtaa aatgtttgag aattaaaacc aaacatgctc atatttgcaa aatgttcttt 1380

aaaagttaca tgttgaactc agtgaacttt ataagaattt atgcagtttt acagaacgtt 1440

aagttttgta cttgacgttt ctgtttatta gctaaattgt tcctcaggtg tgtgtatata 1500

tatatacata tatatatata tatatat 1527

<210> 14
<211> 2988
<212> DNA
<213> Homo sapiens

<400> 14
   agaagaaggg gagaggaggt tgtgtgggac aaggtgctcc tgacagaagg atgccacagc 60
   tgagcctgtc ctcgctgggc ctttggccaa tggcagcatc cccgtggctg ctcctgctgc 120
   tggttggggc ctcctggctc ctggcccgca tcctggcctg gacctatacc ttctatgaca 180
   actgctgccg cctccggtgt ttcccgcaac ccccgaaacg gaattggttc ttgggtcacc 240
   tgggcctgat tcacagctcg gaggaaggtc tcctatacac acaaagcctg gcatgcacct 300
   tcggtgatat gtgctgctgg tgggtggggc cctggcacgc aatcgtccgc atcttccacc 360
   ccacctacat caagcctgtg ctctttgctc cagctgccat tgtaccaaag gacaaggtct 420
   tctacagctt cctgaagccc tggctggggg atgggctcct gctgagtgct ggtgaaaagt 480
   ggagccgcca ccgtcggatg ctgacgcctg ccttccattt caacatcctg aagccctata 540
   tgaagatttt caatgagagt gtgaacatca tgcatgccaa gtggcagctc ctggcctcag 600
   agggtagtgc ccgtctggac atgtttgagc acatcagcct catgaccttg gacagtctgc 660
   agaaatgtgt cttcagcttt gacagccatt gccaggagaa gcccagtgaa tatattgccg 720
   ccatcttgga gctcagtgcc cttgtgacaa aaagacacca gcagatcctc ctgtacatag 780
   acttcctgta ttatctcacc cctgatgggc agcgtttccg cagggcctgc cgcctggtgc 840
   acgacttcac agatgccgtc atccaggagc ggcgccgcac cctccctagc cagggtgttg 900
   atgacttcct ccaagccaag gccaaatcca agactttgga cttcattgat gtactcctgc 960
   tgagcaagga tgaagatggg aagaagttgt ccgatgagga cataagagca gaagctgaca 1020
   cctttatgtt tgagggccat gacaccacag ccagtggtct ctcctgggtc ctgtaccacc 1080
   ttgcaaagca cccggaatac caggagcgct gtcggcagga ggtgcaagag cttctgaagg 1140
   accgtgagcc taaagagatt gaatgggacg acctggccca gctgcccttc ctgaccatgt 1200
   gcattaagga gagcctgagg ctgcatcccc cagtccctgc cgtctctcgc tgctgcaccc 1260
   aagacattgt gctcccagac ggccgggtca tccccaaagg cattatctgc ctcatcagtg 1320
   tttttggaac ccatcacaac ccagccgtgt ggccggaccc tgaggtctat gacccctttc 1380
   gctttgaccc aaagaacatc aaggagaggt cacctctggc ttttattccc ttctcagcag 1440
   ggcccaggaa ctgcatcggg caggcgttcg cgatggcgga gatgaaggtg gtcctggggc 1500
   tcacgctgct gcgcttccgc gtcctgcctg accacaccga gccccgcagg aagccggagc 1560
   tggtcctgcg cgcagagggc ggactttggc tgcgggtgga gcccctgagc tgagttctgc 1620
   agagacccac tctgacccca ctaaaatgac ccctgattca tcaaaagtga ggcctagaat 1680
   taccctaaga ccctgttcca cagtcctgta ttccatccta gatatctact caaaataatt 1740
   gagacaagtg ttcaaacaga aagacgcttg tgcgtgaatg ttcatggcag ccctattcac 1800
   agtagccaaa cgatgaaaac aaccccaagc tatatattac cagatgaaag gataaacaaa 1860
   atatggtcca tccatacaat ggagtattac acagccataa aaaggaatga agcagtgatc 1920
   cccactacac tgtggatgaa ccttgaatgc atgatactga atgaaagaca tcagatgcaa 1980
   aaggtcacat agtgtactgt ccttttatat gaaatttcca gaacaggcca atctgaagag 2040
   atgtatagtg gattggtggc tttcagcagc tgtggggagg tgggactgag gagcgactgc 2100
   taatcaggat ggggtttcct cctgggatgg tgaaaatgtt ccggacctag atagtgatga 2160
   aggtagcacg acactgtgag tgcactaaat gctattgaat tggacacttt agaatggttg 2220
   aaatagtgat ttttatgtga attctaccta aacatgctat tacagctcat atatactttt 2280
   tccatctgga ttcttcacaa aagaatatgt tgtgagcatc tttccatgat attaaatcat 2340
   cttaggaaac attattttgt gttcttcaaa atgtgcatgt taagtattca aatcagtctt 2400
   aaatttttaa aaatatgtaa ttttagaaaa taatttaaaa ggttttgttt cagtttgtaa 2460
   gatttctttt ctggcacttt aatggcttga ggtatcatta tcagttacaa attgagttat 2520
   tcttcatcaa atgacttttg gagtagagat tttattttta tagcaataga tgcacagata 2580
   ttcctgtaag atacaggtgt ggttagacac ttttctagaa caggcatgcc ctgcaaactc 2640
   cacagacact gactgttttt gtcctattaa gaagtagacc actgagaagg gagaaggtga 2700
   cattttagct ttcccaggta aaagtggttt tcatcctcac accaatttta tggactggac 2760
   gttaactctc ttgctcaagg tcactctgag tggaagagtg gggataaatc tggttcgttt 2820
   ggcatcagag gccatgactt ttcctaccac agaagtaatt ttcaaagtaa gtctctgccc 2880
   taggcacatc agatcacctg gggaccactc cagagtgagt agacaagact ttgacagggg 2940
   tgcctaattt tttttttttt ttgagatgga gtctcgctct gttgccca 2988

<210> 15
<211> 3135

<212>  DNA
<213>  Homo sapiens

<400>  15
   cgcagagggc cggggctacg gggcagcgcc ccgggcgatg aggggccggc gttgaccggg      60
   aagagcgggc accgcggcag tggctccgag gggacccgcg atggcagcgc cctgagagga     120
   ggctccaggc agggcgggct gcgctggcag cggccgctga ggtgctggcc ggccggctgg     180
   ctggcgacgg gggcagaagc gacgagaggc gcgctcggca cccgcacccc cgtgcccccg     240
   cctcagttgt ctaaacttcg ggctctcttc cacccgctct gcgcgcccag agtcaacaac     300
   ttcttcaccc ccctccgccc ccgcccttcc ctccgtcagc cccgggagct cgccgcgcgc     360
   cggggaccag gaacctccag cgctgagatg tggccgtgag gcgttggcgg gcggcgagga     420
   gaagctcggc ggcgtcccgg ggccggaggg ccgtggggcc ggggcgcagg ggcgcgagca     480
   ccccgcgcct ctcccccgcc tcctcctgcc gtctccgccg ctgcccgtgc cttgcaagca     540
   gcagccggag ctgccaagcg tcagggccgc ggagatgtcg tcgtcgtcgc cgccggcggg     600
   ggctgccagc gccgccatct cggcctcgga gaaagtggac ggcttcaccc ggaaatcggt     660
   ccgcaaggcg cagaggcaga agcgctccca gggctcgtcg cagtttcgca gccagggcag     720
   ccaggcagag ctgcacccgc tgccccagct caaagatgcc acttcaaatg aacaacaaga     780
   gcttttctgt cagaagttgc agcagtgttg tatactgttt gatttcatgg actctgtttc     840
   agacttgaag agcaaagaaa ttaaaagagc aacactgaat gaactggttg agtatgtttc     900
   aactaatcgt ggtgtaattg ttgaatcagc gtattctgat atagtaaaaa tgatcagtgc     960
   taacatcttc cgtacacttc ctccaagtga taatccagat tttgatccag aagaggatga    1020
   acccacgctt gaggcctctt ggcctcacat acagttggta tatgaattct tcttgagatt    1080
   tttggagagc cctgatttcc agcctagcat tgcaaaacga tacattgatc agaaattcgt    1140
   acaacagctc ctggagcttt ttgatagtga agatcccaga gaacgtgact tcctgaagac    1200
   tgttctgcac cgaatttatg ggaaatttct tggattaaga gcattcatca gaaaacaaat    1260
   taacaacatt ttcctcaggt ttatatatga aacagaacat ttcaatggtg ttgctgaact    1320
   tcttgaaata ttaggaagta ttatcaatgg ctttgcattg ccactgaaag cagaacataa    1380
   acaatttcta atgaaggttc ttattcctat gcatactgca aaaggattag ctttgtttca    1440
   tgctcagcta gcatattgtg ttgtacagtt cctggagaaa gatacaacac taacagagcc    1500
   agtgatcaga ggactgctga aattttggcc aaaaacctgc agtcagaaag aggtgatgtt    1560
   tttaggagaa attgaagaaa tcttagatgt cattgaacca acacagttca aaaaaattga    1620
   agagccactt ttcaagcaga tatccaagtg tgtatccagt tctcattttc aggttgcaga    1680
   aagggcattg tacttctgga ataacgaata tattcttagt ttgattgagg agaacattga 1740
   taaaattctg ccaattatgt ttgccagttt gtacaaaatt tccaaagaac actggaatcc 1800
   gaccattgta gcactggtat acaatgtgct gaaaacccta atggaaatga atggcaagct 1860
   tttcgatgac cttactagct catacaaagc tgaaagacag agagagaaaa agaaggaatt 1920
   ggaacgtgaa gaattatgga aaaaattaga ggagctaaag ctaaagaaag ctctagaaaa 1980
   acagaatagt gcttacaaca tgcacagtat tctcagcaat acaagtgccg aataaaaaaa 2040
   aagcctccca cctctgccgg ataggcagag ttttgtatgc ttttttgaaa tatgtaaaaa 2100
   ttacaaaaca aacctcatca gtataatata attaaaaggc caattttttc tggcaactgt 2160
   aaatggaaaa atatatggac taaacgtagc cctgtgctgt atcatggcca tagtatattg 2220
   taacctttgt ctaatcattg gatttattgt gtcacttctg aagtttcaca gaaatgaatg 2280
   aattttatca tctatgatat gagtgagata attatgggag tggtaagaat tatgacttga 2340
   attcttcttt gattgtgttg cacatagata tggtagtctg ctctgtatat ttttcccttt 2400
   tataatgtgc ttttcacact gctgcaaacc ttagttacat cctaggaaaa aatacttcct 2460
   aaaataaaac taaggtatca tccttaccct tctctttgtc tcacccagaa atatgatggg 2520
   gggaattacc tgccctaacc cctccctcaa taaatacatt actgtactct ggaatttagg 2580
   caaaacctta aatctccagg ctttttaaag cacaaaatat aaataaaagc tgggaaagta 2640
   aaccaaaatt cttcagattg ttcctcatga atatccccct tcctctgcaa ttctccagag 2700
   tggtaacaga tgggtagagg cagctcaggt gaattaccca gcttgcctct caattcattc 2760
   ctcctcttcc tctcaaaggc tgaaggcagg gcctttccag tcctcacaac ctgtccttca 2820
   cctagtccct cctgacccag ggatggaggc tttgagtccc acagtgtggt gatacagagc 2880
   actagttgtc actgcctggc tttatttaaa ggaactgcag taggcttcct ctgtagagct 2940
   ctgaaaaggt tgactatata gaggtcttgt atgtttttac ttggtcaagt atttctcaca 3000
   tcttttgtta tcagagtacc attccaatct cttaacttgc agttgtgtgg aaaactgttt 3060
   tgtaatgaaa gatcttcatt gggggattga gcagcattta ataaagtcta tgtttgtatt 3120
   ttgccttaaa aaaaa 3135

<210> 16
<211> 1049
<212> DNA
<213> Homo sapiens

<400> 16
aaaacaacag gaagcagctt acaaactcgg tgaacaactg agggaaccaa accagagacg 60

cgctgaacag agagaatcag gctcaaagca agtggaagtg ggcagagatt ccaccaggac 120

tggtgcaagg cgcagagcca gccagatttg agaagaaggc aaaaagatgc tggggagcag 180

agctgtaatg ctgctgttgc tgctgccctg gacagctcag ggcagagctg tgcctggggg 240

cagcagccct gcctggactc agtgccagca gctttcacag aagctctgca cactggcctg 300

gagtgcacat ccactagtgg gacacatgga tctaagagaa gagggagatg aagagactac 360

aaatgatgtt ccccatatcc agtgtggaga tggctgtgac ccccaaggac tcagggacaa 420

cagtcagttc tgcttgcaaa ggatccacca gggtctgatt ttttatgaga agctgctagg 480

atcggatatt ttcacagggg agccttctct gctccctgat agccctgtgg gccagcttca 540

tgcctcccta ctgggcctca gccaactcct gcagcctgag ggtcaccact gggagactca 600

gcagattcca agcctcagtc ccagccagcc atggcagcgt ctccttctcc gcttcaaaat 660

ccttcgcagc ctccaggcct ttgtggctgt agccgcccgg gtctttgccc atggagcagc 720

aaccctgagt ccctaaaggc agcagctcaa ggatggcact cagatctcca tggcccagca 780

aggccaagat aaatctacca ccccaggcac ctgtgagcca acaggttaat tagtccatta 840

attttagtgg gacctgcata tgttgaaaat taccaatact gactgacatg tgatgctgac 900

ctatgataag gttgagtatt tattagatgg gaagggaaat ttggggatta tttatcctcc 960

tggggacagt ttggggagga ttatttattg tatttatatt gaattatgta cttttttcaa 1020

taaagtctta tttttgtggc taaaaaaaa 1049

<210> 17
<211> 9902
<212> DNA
<213> Homo sapiens

<400> 17
agaatgcagc gcatggcgcg tcattgaaga gagaccatga tggcggcggc gctggggccc 60

ccagaagtga tcgctcagct ggagaacgcg gctaaagttc tgatggcacc accttccatg 120

gtcaataatg aacaacgcca gcatgcagag cacatattct tatcatttag gaaatcaaaa 180

tcaccatttg cagtttgcaa gcatattttg gaaactagta aagtggacta tgtcctcttt 240

caagctgcca cagccataat ggaagcagtt gtccgagagt ggattctctt ggaaaaaggt 300

agcatcgagt ctctgcgaac attcctttta acctatgtct tacaaaggcc caaccttcaa 360

aagtatgttc gggaacagat tctactagca gtagcagtaa ttgtaaaaag aggatcatta 420

gataaatcaa ttgactgcaa aagcattttt catgaagtca gccagttgat tagtagtggc 480

aatcccactg tgcaaactct ggcctgttct attctgactg cgctattgag tgaattttca 540

agttcaagta aaactagcaa cattggattg agcatggaat tccatggtaa ctgcaaaaga 600

gtttttcagg aagaagacct tcgtcagatc ttcatgttaa ctgttgaagt tctgcaggag 660

ttcagcaggc gggaaaacct caatgctcag atgtcttcag tatttcagcg ttaccttgca 720

ctcgccaatc aagtcttgag ctggaacttt cttcctccaa atttgggcag acattatata 780

gctatgtttg aatcctcgca aaatgtgctg ttgaagccaa cagagtcctg gcgggagact 840

cttctggaca gcagagttat ggagcttttc ttcacagtac atcgaaaaat cagagaagat 900

tcagatatgg cacaagattc tctgcagtgc cttgcccagt tagcttctct tcatggaccc 960

atcttcccag atgaaggatc acaagttgat tatctagcac acttcattga gggattactg 1020

aatactatca atggaattga aatagaagat tctgaagctg tggggatctc cagcattatc 1080

agcaacctga taaccgtgtt cccacgaaat gttttaactg ccattccaag tgaacttttc 1140

tcctcctttg ttaactgcct cacacacctc acttgttctt ttgggcgaag tgctgcattg 1200

gaagaagtgc ttgataaaga tgacatggta tacatggaag catatgataa attgttggag 1260

tcctggttaa ctttggttca agatgacaaa catttccata aaggcttttt tacccaacat 1320

gcagttcaag ttttcaattc ctatattcag tgccacctag ctgctccaga tggcacaaga 1380

aatttgactg ccaatggtgt ggcctctcgt gaggaggaag aaataagtga acttcaagag 1440

gatgatcgag accagttttc tgatcaactg gccagtgtag gaatgctagg aagaattgct 1500

gcagaacact gtatacctct tctgacaagt ttattagaag aaagagtaac aagactccat 1560

ggtcagttac aacgacatca gcaacagtta cttgcttcac cgggttcaag cactgttgac 1620

aacaaaatgc ttgatgatct ctatgaagat attcactggc ttattttagt tacaggctac 1680

ctcttagctg atgatactca gggagagact ccgctaatac ctccagaaat aatggaatat 1740

tccattaagc attcatctga agttgacatt aatacaacac ttcaaatttt gggatctcca 1800

ggagaaaagg cttcttccat cccagggtac aacagaacag attctgtgat taggctgttg 1860

tctgccattc tcagagtttc agaagttgaa tctcgagcaa taagagcaga tctcactcat 1920

ctactaagtc cccagatggg caaagatatt gtttggtttt taaaacgctg ggcaaagact 1980

tatctcctgg tggatgaaaa actgtatgat cagataagtc tgccattcag tacagcgttc 2040

ggagcagata cagagggttc tcagtggata attggctacc tcttacaaaa agtcatcagt 2100

aacctctcag tctggagtag tgagcaggac cttgcaaatg acactgtgca gctccttgtc 2160

actttggtgg aaagaagaga aagggcaaac ttagtaattc aatgtgagaa ctggtggaat 2220

ttagctaagc agtttgcaag ccgaagccca cctcttaatt tcttgtcaag tcctgtgcag 2280

aggacattga tgaaggctct agtcttagga ggttttgcac atatggacac agaaaccaaa 2340

cagcagtatt ggacagaggt tcttcagcca cttcagcagc gattcttaag agtgataaac 2400

caagaaaact tccagcagat gtgtcagcaa gaggaagtca agcaggaaat cactgccaca 2460

ctagaggccc tgtgtggcat tgctgaggct acccagattg acaacgtagc aatcctgttt 2520

aattttttaa tggacttcct taccaattgc attggattga tggaagttta caagaatacc 2580

ccagagactg tcaatctcat tatagaagtt tttgttgaag ttgcacataa acagatatgc 2640

tatcttggag agtccaaagc tatgaactta tatgaagcct gccttacttt gttgcaagtg 2700

tattctaaga ataatttagg gcggcaaaga atagatgtta cagcagaaga agagcaatac 2760

caagacctgc ttctcattat ggaacttctt actaacctgc tgtcaaaaga attcatagat 2820

ttcagtgata cagatgaagt gtttagagga catgagccag gtcaagcagc aaacagatct 2880

gtgtcagcag cggatgttgt gttgtatgga gtaaacctaa ttctgccctt gatgtcacag 2940

gatctcttga agtttccaac cctttgtaat cagtactaca aattaatcac atttatctgt 3000

gagatttttc ctgaaaaaat accacagctt cctgaggatc tgtttaaaag tctgatgtac 3060

tccctagaat taggaatgac atcaatgagt tcggaggttt gccagctttg cctggaggcc 3120

ttgacaccgt tagctgaaca gtgtgcaaaa gcacaagaaa cagactcacc actttttcta 3180

gcaacacggc actttcttaa gctggttttt gatatgctgg ttttgcaaaa gcacaacaca 3240

gagatgacca ctgcggctgg cgaagctttc tacacgttgg tgtgtttgca ccaggctgaa 3300

tattctgaac tggtcgaaac attactatca agtcagcaag acccagttat ttaccagaga 3360

ttagcagatg ccttcaacaa gctcactgca agcagcactc ctcctacgct ggatcggaag 3420

cagaagatgg ccttcttaaa gagtttagaa gaatttatgg caaatgttgg tggtctcctt 3480

tgtgtaaaat aaacaacaga actttatgct taatttagat cctttctgca aagtgcactg 3540

aattgctgaa agttgacttg agtcttgtcc tattcctcag ttcatttggc cattttggat 3600

tttggagagc ctgaaacttt gatatgtatg taatacagtg aaacaggaga ggtcaacttg 3660

gcatcagctt ctgctgttaa gtgttagcca caatctgtca tatatatgtc ttttagattc 3720

tgaatggtga tttaaaattt tcaaaatgaa attccatata tgtgcaaaca gatatgggca 3780

ccacgaaata catatgcagt gccttttttc cttttaacat aggtggctag ccaaagttta 3840

gaatttttgt cattaaatat gaaatggata tatgctaggc agtgtttctc aaaatctcca 3900

tagatcgcct gcatcacttg aggagctggt gaaaaggcag attcttaggc ccaactgtag 3960

accttcagag tcagaatgtc tggttgttgg gcccaggagt cttcatgtta ataagcttct 4020

ccctttcgtc accccaaaag ttttgaatca atgaaagaga cattgaaaac tcttaagagg 4080

ttttgtgctt tctagctttt cctccctttg atgattgggt tttataattc agcaggaagg 4140

ggaaacatca ccaggggttt gttggctttt tcttagcttg ctttcttgct tgcttgcttt 4200

cttgcttttc ttgctttctg tctctctctt tcttttctct ctctctctca catcaaccca 4260

gtgctgcagg ttttgtgtaa tacaagtcac taatcatact ctgatgcctg aacttgagga 4320

ggaaaataca tgtatatttt tgttccgtaa aaataacctt aggaactgta gccatttcat 4380

tgccttaatt ttaagaggaa aatacaaaaa cagctgattt gttttagtaa gaaaccacgt 4440

cttgatgctt cagagttggt ttagggtgtt agctgctatg aacctgttgc ccctttcgat 4500

cgtgtattta tgtaggttta tcagtgaaat gaaaggcttg tttccgtcta gtctaacttt 4560

ttgagtgtgt ttctatccag ccacatagcc catatctact ctaaatggct tgcttaagca 4620

ataattattt taaaggatgt gaatcactga ttcacacaga ctattgcacg ttggggcatt 4680

aggggcaata attcttatcc agacatggga gccagtgaat ttaatttcag agattaaaaa 4740

ttcactttag atcctctagt ttgatctctt aatcaggatt tttatacagc tgccaggctc 4800

ccctaattca gtgtgccagc ttacaatgtg gaaatgaaag ctaatttata cacagcaggc 4860

atatgaaact ccactcattg cagtactttc acagcacagt gacaggtaga ggactctggc 4920

acaggtgcac tcatgaaact ctgcttccac catgttcctg acacctatct attaaaccat 4980

tctgcaaata cggtttttct acctgattgc atatagcata tgtgtcatta catgtgatgc 5040

tgtgcaaaac tttgtataat tctgtgttat taacagttaa caaaactgga gcatctgaat 5100

tacatccaac ctgtgcatgt gatgttaggt agatgtgaat gcagggcctt gggccataac 5160

ttacatttct ctcaatttga ttagctttga gtcacaatta aggggaagca aaaacatctt 5220

gaaaagactg ctaggaagga aattaatatc agtcatccag aagtacacgt ttctgtattt 5280

taaaaaatac tttgatgcat ttatttttag gtgttttttt tttcccctta aaaaacttga 5340

agtgatatgc agcagtaatc tatttgtttt gcattgttct tggtgttttg tgtttcccag 5400

atccctcaag ctttctcagc tgttgtgaat tatgtgtatc tgtgtgtgtg ctaagtacag 5460

tctctttacc aaagggcact gaaacacaca attgactgga caggtccacg cgccatgaca 5520

aaactataat caagttatta aaactaaaga ggagtgggaa aggaatgcct tggtaagtaa 5580

aaaggcatct atatttaata acttttatcc agatggcaac atatttgcaa aatttgccca 5640

gatcctatta caatactaaa aatagaaaat ttcacctcca tattcctgag gtgtaatttc 5700

attagactag ttttagttta aaaagacctt cttcagattg gaccaaataa tacttataag 5760

atcagcagaa tgttgaatat tagctcactg gggtggggag aagccactac cattttttag 5820

gtgatgggga tgccactgag ttgcaacggc tagacctttt cagggtggtt gtgtccatgt 5880

ttgcctgatt ggatgcttat tcactttgtg ttttcttttg ttttattttg tccaattttg 5940

tctttagctg tgtttattaa cttctccggt cttgttttgt tttaatgctc ttggcccagt 6000

gggtgtcaag aacactggct taattcaagt cagttgattt tttttctatt aaaactgttg 6060

ttaaaatatt ttttaaaaca aaaacattat ttgtgccctc ttttatatat gtcaaaggga 6120

cactgtcaag tatttcattt ttagattttt gttttataaa atttctgttg ttcatatagt 6180

atcctttaac ctctagtttt ccatacatcc tttgtttgtt tctcatttta ttttccttga 6240

cccatttatt tcccaaggca caatcactaa agactttgta ctttcacagt ctgttaatgt 6300

ggtagcacct gtaactgtgt tcttgttctg ttaaaaggat tgatttgctt ttatagtcct 6360

tgtgctggat gagtggctgc ctcagtagca aaactacctg acagtatttg acagtgtcct 6420

ttccagcacc attatttggg tctttcaggg tggccatctc tgttagaaga cagtagcatg 6480

ttaacatcac tgcattgagt ttttgtctgg tgtaaagtat gacttttaat gtaaacaaac 6540

tgcaggtttt tttcaaacta attttaagaa tttagtctta tttcgttgta aactgtgtat 6600

ctaattatat tacattactc tgttcagatg ggatggttac taccacttgt ccatgatttt 6660

catttgaaaa gcaagtatct atatcatttc cccccagtca gcattattta acactcccct 6720

taactgtctt tgaactttct cttttaacaa aaatgtcaag tctttatagt tgtaatatca 6780

ccatgtttcc catttctgtt aatacttcta tgaaccccta aagtattgaa gggaactagc 6840

tgtcagtttc aaggattaca agtttgagtc tcctagtatt caacatcatt ctgaaccctg 6900

aaataatatt tttctctgtt aaacaatttt tatctgtttg ccacctctgt tgttagaggt 6960

ggttgtcaat tgaccttact aagttagctg tctttgatga ggaattattg ttattggttc 7020

ctgaataaaa cattaacctt ttaagtcaga aggaacctcg gtacttctta aggtttgttt 7080

gtgttttcta aaaccagaga ataaggaact gatttggcta tgaggtttaa cattataatt 7140

ttctgtaagc tttcccacaa aaaaacattg ttgatttgag gatataataa tgttttaatc 7200

tttttaaaat ataagtggtt attctctgac ttggtaacta tgttctgaaa acactgcatt 7260

taagaatttt taaaaattgg ttttctaaaa ttaaaatgtc caaattaggc atattgctga 7320

gctcaaattg atgtgaaatg ccatggttcc agttgaattt taagcatatt ttcatttaga 7380

tataaaatat atgaagtatg ctttgttgat tatagtgaga acccatgaca tagttaacca 7440

aagaatatgt ttggttcaaa taaaaataga agcttaatac tgggcattca tactttttaa 7500

agagaatgaa tgaagaaatc ggtttcctgc tgtagttctc tatgggtaag tcttagtaaa 7560

gacgagaatg ctgaagtcgg ccgtggcgat tccctcctag gaactgggag gtgtggcttg 7620

cccattaccc gcttgaagct cacatcttta ccctcctctc ccactgtggt ttgatcttca 7680

cctattccca ggccctccca gcaattggag aggtgtcttt ttttttggtt ttggtttttt 7740

ttctccccgt ctgcattctt aggcctctta gctattagga actgtcagat acatactagt 7800

agctaatttt cctagcctga aattatatac tgcatctgca ctatgtacct actagggatc 7860

tgacctcaag tgttttctga gcccaggctt cctggtgtgg tgtcttttac cacataaaat 7920

tattacaaat tgcaaatgtt ggtattgtga tttgattatc tgtacaaaga aagaagctct 7980

atgcagtgag tttgtggttt aatggtcaca aaaatgttag cactgctacc actcagcacg 8040

tgtaaaattt tttaaattta taaatattaa aattttaaac ttacactaag acttttcagt 8100

tttatttaaa gacccaggga tgagtgtact gtttaaatat ttacctctat taacataact 8160

aatgaaggta taaaattgca tttagttttt cagaagatgc tgcaatatga ttttaggaaa 8220

taaggctatg tattgagcca gttataggct gaatatcagg ttgataaaat tttatttgta 8280

tttttaaaat tcataaatgg gagttaaaat gtgtcttttc actaaatatt tttattacat 8340

ttttgttttg accatgtgtg atttgttcat gggtatttag ctagactctt ttatgaaatg 8400

cattgaaatg gtgctagttt gtttaccatt ataggaagat tgacactggg gacattacac 8460

ggtcctaaaa taattttaaa tttaaaaaaa tgaagacatc tgtacagata aaaatcaaga 8520

tttgcatcac cttctgggag aacccgtggt actcacagaa tatccagtat tagaaatata 8580

aaaattttgt ctatatttta taatatttat ctcagcccac agtcttagaa acagtttcac 8640

agttactagt taagtcatta tctttggtgc atatttagta aatactttta gcaagtcatt 8700

ctttttggcc tttcagggaa agtggtgaat taccatatta ctgtgtgcta gatagatcca 8760

ccctttgccc agtgttaaat cttgtaaaca tatgaagtga aaagttaatt tttaaagaaa 8820

atgcatcttt aaaatgttca tttttaacct gaatttgctt tgaatattct ttgatttgta 8880

atgttgttat ttagcttata ttgagattta ttccactcca aaggtggata aagaaaagtt 8940

gtttaagaaa accaattaga gtgtattttg aatttttgtt tctttcatta ccaagtgtgg 9000

ggccatacac acaggctaat catctgtcat ttgagaccca cagatgattt caaaggtccc 9060

aaataaatac acatctataa ctgtttttta aaagacatct ttaatggtgc tctccaaaaa 9120

tacaattcgg ttgcttatga gtgatgcctt tgacaggctg attagattat aaaacagttt 9180

tgttttttta aacatagggt ctcagattga gtcttgtgca ttctatgtaa tctcaaatgg 9240

cagttcagtt tgactttctt ttaaaactaa gtggaaatgg tatatttggt gaaactaatg 9300

aaagaatctt ttgctgggaa attccataca gcacccccag cagcatcctg tctcagtctg 9360

gcgtataatt caacctccta ctgagtgtcc ctgtaaatta tttttaatat atagcatgag 9420

agtgaactga ggtaagtcat tgatgtttcc cagagatgat gaatgaacaa aagcaaatag 9480

gatctgctaa acatttttat tcccacagta gtgagcagtt ttgttcttgt tttgataatg 9540

ttcatttctg cctaatctta ggcttcaaat acacctttaa gaaagcccaa gatgtattga 9600

gtagtagaac ttcttatttg tacacctgtt tataactgca ctgccctgga tgttatggtt 9660

atttctaagg actgacctgg taaagctact tgaactggta gactgcaggg tagaggcaga 9720

cctagattca atgtctaact aagctgctta gggcttacat taatttccac aggtctgtta 9780

tccatatcat gagtttataa gtgttcctcc caaagaaaaa atttaaaagt gatggatgaa 9840

atataatagt agatgctcaa taaattccat tgtatttaaa gattaaaaaa aaaaaaaaaa 9900

aa 9902

<210> 18
<211> 6571
<212> DNA
<213> Homo sapiens

<400> 18
   gccccgcccc gcgcgccgtg gccgtttgaa gtgactaatt tctgtcatat gactgaggcg 60
   cccatggggg tggcggggcg gctgtaggag caggggccta gcaagcgccc agcggagcga 120
   cccctgcctg gccgtggcta gcatggcccc tacgctgttc cagaagctct tcagcaagag 180
   gaccgggctg ggcgcgcccg gccgcgacgc ccgggaccca gattgcgggt tcagttggcc 240
   tttaccagag tttgatccaa gccagattcg actgattgta tatcaagact gtgaaagacg 300
   agggagaaat gttttgtttg actccagtgt taagagaaga aatgaggaca tatcagtatc 360
   gaaactctgc agtgatgctc aagttaaagt ctttgggaaa tgctgccaac tgaaacctgg 420
   aggagacagt tcttcctctt tagatagttc tgtgacttca tcttctgata taaaagacca 480
   gtgtcttaag taccagggtt ctcggtgctc ttctgatgcc aatatgcttg gagagatgat 540
   gtttggctca gtagcaatga gctacaaagg atccacctta aaaattcatc agattcgttc 600
   ccctccacag ctcatgctca gcaaagtgtt tactgctcgg actggcagca gtatttgtgg 660
   gagtctcaat acgctacaag atagtcttga attcatcaat caggacaaca atacattaaa 720
   ggctgataat aacacagtta ttaatggact gcttggaaat atagttcaca gcaacccaat 780
   ggacatgcct ggaagagagc tcaatgagga cagagacagt ggcatagcac ggtctgcatc 840
   tctcagcagc ttgctgatca ctccatttcc ttccccaaac tcctcactta cccgaagttg 900
   tgccagcagc taccagcgac gttggcgacg cagccaaaca acaagtttgg aaaatggggt 960
   atttcctaga tggtctatag aagaaagctt taatctctca gatgaaagct gtggccctaa 1020
   cccaggaatt gtgcggaaaa agaagattgc aattggggta atcttttcat tgtccaaaga 1080
   tgaagatgaa aataacaaat ttaatgaatt ctttttttca cattttcctc tctttgaaag 1140
   ccacatgaac aaattaaaga gtgcaataga acaggctatg aaaatgagcc ggagatcagc 1200
   tgatgccagt cagagaagtt tggcatataa tcgaatagtt gatgccctaa atgaattcag 1260
   aacaacaatt tgtaatcttt acacgatgcc acgaattgga gaacctgtct ggcttacaat 1320
   gatgtcgggg actccagaaa agaaccacct ttgctatcgt ttcatgaagg agttcacctt 1380
   tctaatggaa aatgcttcca aaaatcaatt cttgccagct ctcattactg cagttctgac 1440
   caatcatctt gcctgggttc caacagtcat gccaaatgga caaccaccta taaaaatatt 1500
   tttagaaaag cattcctctc agagtgtgga catgttggca aagactcatc catataaccc 1560
   actttgggca caactgggag acttgtatgg cgctattggc tctcccgtac ggttagcaag 1620
   gactgtggta gttggcaaac gacaagacat ggtccagagg ctactttatt ttcttactta 1680
   ttttataaga tgctctgaac ttcaagaaac gcatctttta gaaaatggag aagatgaagc 1740
   catcgttatg ccaggcacag taattactac cactttagag aaaggtgaaa tagaagaatc 1800
   agagtatgtc cttgtcacaa tgcatagaaa caaaagcagt ttgctcttta aagagtcaga 1860
   agaaattaga actcccaatt gtaactgtaa atattgcagt catccactcc ttggcaaaaa 1920
   tgtagagaac atttcacaac aagagagaga agatattcaa aacagctcta aggagctgct 1980
   aggaatttca gatgagtgcc agatgatttc tccttctgac tgccaagaag aaaatgctgt 2040
   tgatgttaaa cagtacagag ataaattaag aacttgcttt gacgccaagt tagagacagt 2100
   tgtttgcaca ggatctgttc cagtagacaa atgtgcattg tcagagtcag gcttagagtc 2160
   aacagaggaa acatggcaga gtgagaagtt gctggattca gacagtcaca caggcaaagc 2220
   aatgagatcc acaggaatgg ttgtggaaaa aaaacctcca gataagattg tgcctgcttc 2280
   attttcttgt gaggctgccc agacaaaggt tactttcctg attggggatt ctatgtcacc 2340
   tgattcagat actgagcttc gaagtcaggc agtggtggat cagattacca gacatcacac 2400
   caaaccattg aaggaagaaa gaggggctat tgatcagcat caagaaacta aacaaacaac 2460
   caaggaccaa tctggagagt ctgatacaca gaacatggtt tctgaagagc cctgtgaact 2520
   tccctgttgg aatcattcag acccagaaag catgagctta ttcgacgaat attttaatga 2580
   tgattcaatc gaaaccagga ctattgatga tgttccattt aaaacaagta cagatagtaa 2640
   agaccattgc tgtatgttag agttttcaaa aatattgtgt acaaaaaata acaagcagaa 2700
   caatgaattt tgtaaatgta tagaaacagt tccccaagat tcatgtaaaa cctgctttcc 2760
   tcagcaggac caaagagata cactctccat tcttgtcccc catggggata aagagagttc 2820
   agataaaaaa attgctgtag gaactgaatg ggacattcca agaaatgaaa gttcagacag 2880
   tgcccttggg gatagtgaaa gtgaagatac aggtcatgat atgactagac aagttagcag 2940
   ttattatgga ggagagcaag aagattgggc agaagaggat gagatacctt ttcctgggtc 3000
   aaagttaatc gaagtgagtg ctgttcagcc caacattgcc aacttcggga ggtccttgct 3060
   gggtggctac tgctcatctt atgtgcctga ctttgttctt caaggaattg ggagtgatga 3120
   gaggttccgt cagtgtctga tgtcagattt atctcatgct gtgcagcatc cagttttgga 3180
   tgaaccaata gcagaagctg tctgtattat agctgacatg gataaatgga ctgttcaagt 3240
   ggccagtagc cagagacgag tgacagataa taaattggga aaggaagtat tggtttccag 3300
   tcttgtttcc aatctgcttc attccacact tcagctttat aagcataact tgtctccaaa 3360
   tttttgtgta atgcatcttg aagaccggtt gcaggagcta tacttcaaaa gtaaaatgct 3420
   gtctgaatac ctgagggggc agatgcgtgt tcatgtcaag gagctgggag tggttctggg 3480
   gattgaatcc agtgatcttc cacttctggc tgctgtagca agcactcact ctccatatgt 3540
   tgcacaaata ctcctttaat atacctaaaa attgttagaa attggtggga aaataggtag 3600
   aaaccaagga agcagacaca acatgcattt atggagattc tttttccctt ttagacttcc 3660
   atctgaatga gtcagtcacc agggtattct gcatagcatt gtatattctg tgtatgtcag 3720
   atggcttttt ctttttgact ggacttttgg gtggtggtag atttttaaac aaatgaaatt 3780
   aaagcaacaa taattttgaa gcatttgaaa aagccaaagt gtacggtaga aatttctaca 3840
   aaatgaatat tatcaagagt ttcatgtgat cactgcagtg ttgtcacagc tcataaatag 3900
   caacagtgtc tcatgattta atggctcaga aatagttatt cattagtttt taatttttaa 3960
   tttctaaggt acagagatct ataaaacctt gattatttgt tagttttgca attcaaaaca 4020
   gctaatgtct ggttatttct caaagtaagt attttaaaca gcctgttaat tataagaaac 4080
   tcagaataat gagtgtaaat gtgttatgtt atccacccaa gtgtacatat gtacctattt 4140
   tttttaaaaa agcagaaata gaaatacaag actggtaaac atgcctttaa aaatatatat 4200
   attttcaact agtattgtct ataatgctga aatattactt attggtgatt tttctgtttc 4260
   acacactcta aaatataagt aaagccaacc ttttttttaa ggctgagatt cccaaaatga 4320
   gaatactact ttataccatt tgtttataag tatgaactgt tcttataaat attaatattt 4380
   acatattcac taatttaaca taaatgaaaa ttaggattaa aaattgcacc aaagcatcgg 4440
   caaaaacaat actatattct ttaaaagtgc tcaggtagcc aaggcccttg cttttggtat 4500
   caaccctcat gaacccatag gagctgaata tttgtttcac tgcttaataa tcctcaattt 4560
   acactattca taactcttaa aattattctc tttttttcta agagtccctc ccttccaaaa 4620
   gtgtattttt ttcaaagatt ttcacttctc aattgttgcc tttgtacata ctatagagtg 4680
   ttgcttgtaa gaaaggctaa tatggaacca aactcttgta agtaatgtaa atagaaaggt 4740
   gggtggataa agttttcaat actttctact acctcagttt acttgagtac tacattatag 4800
   tttattcttt gcttatctgg tctaagagac ttttaatgct agtagtaaag ttggtttctg 4860
   ctttcattga ctattttcat cataatttca tcattgatta aaaaaagaaa accacttgtt 4920
   tattcagtta ttaaatatat ttactatata acacatccat tcttgctgtt taaattttca 4980
   atagttaatg gaaagttgtc tttgaccttg aatttacagc attgggtcac attttgcctt 5040
   gctgtgtatg tattcaagag acttccaact agacaaagaa aaaattgttg ttttaatgga 5100
   atgtaaacct gaaattggtg tgtctgcaat ctgtttggcc catgaccttt tacctagtcc 5160
   cagttattac ctgagtctcc catggatgac ttgctgccaa ggagtgtttg tggatatatt 5220
   ttctttggct taattttctt attctgtgca ttaacaaaat tatccagttg tctgattttg 5280
   gaattctatg agtcaatctt tttggcagaa ttcagaatat taaaaagttc atacatttgc 5340
   gggccattgt accttttttt tttttttttt ttttttgacg gagtttcact cttgttaccc 5400
   aggctggagt gcaatggtgc gatctcagct cactgcaacc tccgcctccc agttcaagtg 5460
   attctcctgc ctcagccccc aagtagctgg gattacaagt gtgcgccacc acacccagct 5520
   aattttttat ttttagtaga gatgagattt caccatgtgt tggtcaggct ggtcttgaac 5580
   tcctgaactc aagtgatcca cctgcctcga cctcccaaag tactgggatt acaggcgtga 5640
   gccactgtgc ccagccttgt actttttttt ttttttattg tagctctgta tagcacttgg 5700
   ataatgtctg ggtgtctcct taaccatcaa actgttcttt atttaaaatg tttaattacc 5760
   gtttagaaat tctagtcctc aataagtggg tggctcatgc tattgttgtt cctaaagcta 5820
   agctttgctg ggaaggaaat gacctatagt ttcttaaaga atcattaatc cttataggat 5880
   tcctgagtta ctgttttgtt cctccccact gcttcccatt cctgagtttt gtaattccta 5940
   atccttctat aatttctatt accttcacca tagtcattct ttccttacgc aaagcccaag 6000
   gaatgagctg ctgctacttt aaagtgtggt cattatgatg aatgtgaaaa gagttttggc 6060
   ttgttctaaa taatttttac aagttatggt acagacagtt gttacatatc aaaaaaaacc 6120
   tgtttattga aacaggaaat aaaaaaggga tcttttacat tatagaatta aaactaattt 6180
   tttcttgtat ataaactaat tggtttgatt ttaaatattt ctggctttta ttaatatgtc 6240
   ttaattttga gtttgaaaat gttaagtgca ataaaaacat actagtacag attttgtttt 6300
   gttccaattg gcatactctg gggatgatca cttaaaaaaa cagattttac ataatgccta 6360
   cttctggtag atgtcttatg agattgttct gctttttcta agttacttag atgttggata 6420
   tgtacatagc tgtttcttgt tctgtataca tttctcaaat gtacacttgt attataataa 6480
   cctcccagtt ctaggggata tttgtgcaat aaatacacat gtcaacttga aaaaaaaaaa 6540
   aaaaaaaaaa aaaaaaaaaa aaaaaaaaaa a 6571

<210> 19
<211> 4255
<212> DNA
<213> Homo sapiens

<400> 19
tggcttgaag tcccctcctc catcccgggg atcacgtttt gcctggggga gctgatcgca 60

agactaggca acctccagcc agtccctggg tcgggcggat cctcccagag gtggcacaat 120

ggagcgatct ccaggagagg gccccagccc cagccccatg gaccagccct ctgctccctc 180

cgaccccact gaccagcccc ccgctgctca cgcaaagcca gacccaggtt ctgggggcca 240

acctgctggc cctggcgcgg cgggtgaggc cctggcggtg ctgacttcat tcgggaggcg 300

gttgctggtg ctgatacctg tgtatttggc cggggcagtg ggactcagcg tgggtttcgt 360

gctcttcggc ctcgccctct acctgggctg gcgccgggtc cgcgacgaga aagaacggag 420

ccttcgagca gcgaggcagc tactggacga cgaggagcag ctcactgcga aaactctcta 480

tatgagtcat cgagagctac ctgcctgggt cagcttccca gacgtggaaa aggctgaatg 540

gctcaataag attgtggccc aggtctggcc cttcctgggc cagtatatgg agaagcttct 600

ggctgaaact gtggctccgg ctgttagggg atctaacccc catctgcaaa catttacatt 660

tacacgagtg gaactgggtg aaaagccatt gcgcatcatt ggagtcaagg ttcacccagg 720

tcagagaaaa gagcagatcc tgctggactt gaacatcagc tatgtaggtg atgtgcagat 780

tgatgtggaa gtgaagaaat atttttgcaa agcaggagtc aagggcatgc agctacatgg 840

cgttttgcgg gtgatactgg agccactcat tggggacctt cccttcgtgg gggctgtgtc 900

aatgttcttc atccgacgcc cgaccctaga catcaactgg acagggatga ccaacctgct 960

ggatatccca ggacttagct cactctctga caccatgatc atggactcca ttgctgcctt 1020

cctcgtgttg cccaaccgat tactggtgcc ccttgtgcct gaccttcaag atgtggctca 1080

gttgcgttcc cctctgccca ggggcattat tcgaattcac ctgctggctg ctcgaggcct 1140

gagttccaag gacaaatatg tgaagggcct gattgagggc aagtcagacc catatgcact 1200

tgtgcgtttg ggtacccaga cattctgcag tcgtgtcatt gatgaagaac tcaacccaca 1260

gtggggagag acttatgagg tgatggtaca cgaggtccca gggcaggaga ttgaagtgga 1320

ggtgttcgac aaggatccag ataaagatga ctttctgggc agaatgaagc tggatgtagg 1380

gaaggtgtta caggctagcg ttctggatga ttggttccct ctacaaggtg ggcaaggcca 1440

agttcacttg aggctagaat ggctgtcact tttgtcagat gcagagaaac tggagcaggt 1500

tctacagtgg aattggggag tctcctctcg accagatccc ccgtcagctg ccatcttagt 1560

tgtctacctg gatcgggccc aggatcttcc tctgaagaag gggaacaagg aacccaaccc 1620

tatggtacaa ctgtcaattc aggatgtgac tcaggagagc aaggctgtct acagtaccaa 1680

ctgcccagtg tgggaggaag cgttccggtt cttcctacaa gaccctcaaa gccaggagct 1740

cgatgtgcaa gtgaaggatg attccagggc cctgacttta ggagcactga cgctgcctct 1800

ggcccgcctg ctgactgccc cagaactcat cctggaccag tggttccagc tcagcagctc 1860

tggtccaaac tccagactct atatgaaact agtcatgagg atcctgtact tggattcatc 1920

agaaatatgc ttccccacgg tgcctggttg tcctggtgct tgggacgtgg acagtgagaa 1980

tccccagaga ggcagcagtg tggatgcccc acctcgaccc tgtcacacga ctcctgatag 2040

ccagtttggg actgagcatg tgcttcggat ccatgtatta gaggcccagg acctgattgc 2100

caaagaccgt ttcttggggg gactggtgaa gggcaagtca gacccctatg tcaaactaaa 2160

gttggcagga cgaagcttcc ggagccatgt tgttcgggaa gatctcaatc cccgctggaa 2220

tgaggttttt gaggtgatcg tcacatcagt tccaggccaa gagctagagg ttgaagtctt 2280

tgacaaggac ttggacaagg atgattttct gggcaggtgt aaagtgcgtc tcaccacagt 2340

cttaaacagt ggcttccttg atgagtggct gaccctggag gatgtcccat ctggccgcct 2400

gcacttgcgc ctggagcgtc tcaccccccg tcccactgct gctgagttag aggaggtgct 2460

gcaggtgaat agtttgatcc agactcagaa gagtgcggag ctggctgcgg ccctgctatc 2520

catctatatg gagcgggcag aggacctccc gctgcgaaaa ggcaccaagc acctcagccc 2580

ttatgctact ctcactgtgg gagatagttc tcataaaacc aagactattt cgcaaacttc 2640

agcccctgtc tgggatgaga gtgcctcctt tctcatcagg aaaccacaca ctgagagcct 2700

agagttgcag gttcggggtg agggcactgg cgtgctgggc tcattatccc tgcccctctc 2760

agagctcctc gtggctgacc agctctgctt ggaccgctgg tttacactca gcagtggtca 2820

ggggcaggtg ctactgagag cacagctagg gatcctggtg tcccagcact cgggagtgga 2880

agctcatagc cacagctaca gccacagctc ctcatcgctg agtgaagaac cagagctctc 2940

ggggggaccc cctcacatca cctcctcagc cccagagctc cggcagcgcc taacacatgt 3000

tgacagtccc cttgaggctc cagccgggcc tctgggccag gtgaaactga ctctgtggta 3060

ctacagtgaa gaacgaaagc tggtcagcat tgttcatggt tgccggtccc ttcgacagaa 3120

tggacgtgat cctcctgatc cctatgtgtc actgttgcta ctgccagaca agaaccgagg 3180

caccaagagg aggacctcac agaagaagag gaccctgagt cctgaattta atgaacggtt 3240

tgagtgggaa ctccccctgg atgaggccca gagacgaaag ctggatgtct ctgtcaagtc 3300

taattcctcc ttcatgtcaa gagagcgtga gctgctgggg aaggtgcagc tggacctagc 3360

tgagacagac ctttcccagg gtgtagcccg gtggtatgac ctgatggaca acaaggacaa 3420

gggcagctcc taggagctgg cgagtcccag cctgactgct ctgtcttcct gccttcgtct 3480

cgctccatca ccgcctcaat gtgatgagcc taaagctagg gtccaagggc agagcctgtg 3540

cccttcagcc ctttcaccta acaggcccat attcgggcct ttgcctgacc aaagagaaga 3600

accgtatgtt ccctttactg cacggccttt atccttctgg gcccctgggg cggggacctg 3660

agctggctgt ttcctgcttt gcctgcacat tgttctccct tcctcccaac tcctcagggc 3720

cttctgtatc tgtgcctggc cagtggcagc actagcagtg gtattagctt atgccaaata 3780

cagctttgga aggatctttt tttctttaac tagatggtca ccttcttccc taccacacat 3840

gggtgggaag gtggacaggc taacctctcc agctgtgagc ctcttagact actgcatgta 3900

gcaaatgttc agcagctcag gcccccatgt ccagttctgt ccccactgtc ctcaaccctg 3960

tcctgaaaat tctactgctt tgatggctgg ggccagtctc ttgtcacttt ggaaactgag 4020

gacgcgtgga ttctactcaa gcctccaagt agtggcatat cagtcttgga gctcctagct 4080

ggtgatacgg agagggcttt ggaggacttg ggacagcagg gccaattttt ttgcccaagt 4140

gcctaggctg ctaactcact gactagaact taatctggta ctttacagtt ttgcaccaac 4200

tctgccaagc cactggatct tacattaaac atcatactca aaccagcaaa aaaaa 4255

<210> 20
<211> 6185
<212> DNA
<213> Homo sapiens

<400> 20
   agttggcggc cgcctggccg gtggcgcccg ggccgtggaa ggtccagtcg tggctgacgg 60
   cgggcaccag gcgcgcgggc cgcatcttgc ggcggtcgtg gatgaagaag agcaggcgga 120
   ggtagacgag gggctgtggg gtcaccttct cttcctctct tctgccaccc caagtcgtct 180
   gtgttgcgtg tcatgcagtg ggacagcacg ttggtccgga cctcaagcta cccacgttct 240
   agtcccgcct ctgtcatttc ctagagctgt ggcctcggat ccctccctgg tgccacagca 300
   gctgagtgcg catcaaatct gaagaaaatc tacacagtac agacagtaca gatattttgg 360
   agtgtataca ataatatccc tcctgtgact agcctgcctt tgagatgtag ttatcattta 420
   atgagaggag agaggcgacc actttgggaa gaggagagta atgcaaaggg tggcgtatgg 480
   aagatgaaag tccccaagga cagcacgtcc acagtttgga aagagttgct gttagcaacc 540
   atcggggaac agttcacaga ctgtgccgca gcagatgatg aagtaatagg agttagtgtc 600
   agtgttcggg accgagaaga cgtcgtccaa gtctggaatg taaatgcctc tttagtgggt 660
   gaagcgactg ttttagaaaa gatctatgaa cttctgcccc acataacttt taaagcagta 720
   ttttataaac cccatgaaga gcatcatgct tttgaaggtg gacgtggaaa acactaattg 780
   cactctgtaa agaattcttt gtcctttgct gattggtttt ggaaacggtc ttaacaggag 840
   ggagagtgaa gagaagactt gccgaagccg atgctggtca atttagagtg ggtttctgtc 900
   cttgcagatg ggcaattagg actcaaagtg gcaggtgggg tgggggagaa ttgtgtgggt 960
   ttctaccgtc acattacttg cttttaaaaa aaaacacaca caaaaaactt tttttttggc 1020
   tttctaaatt ctctgttctt ttcactctga tttttttttt tttttgccgt ttaaaattcc 1080
   ttattcagcc taattattgt tttctacact cccctttcat tgaggcacaa gaaatgggtt 1140
   tccctttaag tagctctgct gtacctagtt aatgagaata tgcataatcg tgacaatact 1200
   gcttttgaat accacactgt acaaggagga acactagctt ggagaaatct gtcttctgat 1260
   tatttgttgt gacacattcc tatgtactgt gagctgggca ttgttttttt ctgtcccagg 1320
   gaaaagaaaa accatactta atgtgatttt gcactgacag cacacaaatc tttaattttc 1380
   ctttttaaaa catttttttt tagtgataaa aggaaaataa tagttggatt gcataacatg 1440
   aaaactataa tcacaattca gtatcaggat ataaatcaag ctctaagtct ttggaccctc 1500
   tgtggaaaca tttctaaccc gaattttaat ttggcctttt caaccaaagg cttagagtgg 1560
   tagcaaggga tttcttccaa taggaaacct gatgtttctg atttaaagag aaggtgatat 1620
   tttaattgtt tgaattaagc tccttgcaaa gttcgggtgt gtttacccac ttagggcttt 1680
   ctttcccagt caacgctaac acattttgtt aaatgatccc tttccctgct cacattgtgt 1740
   gtcatttctc atcatcagta gtcctccagc ctgggcagct gtccccaccc tttttcatgt 1800
   aggtgcagga agttaaatct catttccagg atgcatgtga acatttacaa agttgaactt 1860
   tgagtgcatt ctgctcatat gaattattgg gattgttgat atatattgta ttatgctacc 1920
   aaagaaatat tggttttatt agaaggaaat ggtcatcctc tggaccatgg agactagctc 1980
   agaatacgct gatttccctc tcctgacttt gccaagcctt tggctgcttt tgcctgataa 2040
   agggcagggc catctgaaga cacttccccc agtcggcttt ggagtcacgg gagctagtgc 2100
   ctgctcacac atttttcaaa agggcagtgc actcagaact tcactgtacc tgggattttt 2160
   aattcctctt gcagtgttga ccagcagaga gacttgaggc tactttaagc ctccactatg 2220
   tgtttgtaga taaaattctc cattcaaaca ttttaaagga ctttgaacat tatctgctta 2280
   tggaagttgt gcccttcact tggttagtaa ccacctcagc cataatactt accatcatag 2340
   gtttcttaaa atgctttttt tttttcccta aacttgagtt tccttagtga tttcaaaatg 2400
   aagtataaga atatcagatc cagttagcaa aagcctagga cttgtttctc caaacattgt 2460
   actaacattc aacttgtttt aaaattatga ctcaagaatt ttaaaaaatt attctggaca 2520
   tgaattaaaa cttttttata atataagtat ttttctgatt gaaaaaagga tataattgac 2580
   ttcactctaa ttgtcatgta tatttccata agtaaatgga ttttgaagta tttttatttt 2640
   ttgaacttta tttaaagcat ttgtgatgac atgttcaact tttgcatgta tgtagccttt 2700
   gaagtaaaaa taaataggaa tgttaggctc acgttaatat ttctagtttc tgtttatgat 2760
   catggaacat gactagcaca caaagtagat ttttgcccca gtgaacttct gcaactgttt 2820
   ctgacccttt agaaaatacc aactgtgaga aggatttaat ttgcctaatg ttaaaaacct 2880
   atcatctaaa ttactttcag agaggatttt gtcagatttt ggtttggaaa ttgttttaat 2940
   tgaatattta atcataatag actttgtatg aactaagctg atttaatcag tttaaaaacc 3000
   tctcattacc ttcttagatt aaagaagcta atactgtttt gagatagaag gaaatatttt 3060
   gtggggcatg tgacttttgt gttaaggcag tttacccatc ttggctgatg tggggaaagt 3120
   gcaaacttcc agtagaatcc cggtcttttc atccaagtgc ttgcaggggc agccttgtag 3180
   tctgcaaaga accatgcaga aaccaaagag cttgcttttt tgtctttgta attccttagg 3240
   gtctgtgata tatgccagga aatctgattg aaactaatat gctttttatt ctcccattat 3300
   tccctaaatt gttatatcac aggcacttgc ctacattggg aaagtaagga caaataatac 3360
   ccatttaaag taacttgcct atattttgac tgtctttcgt aagcacttgc tgaactgctg 3420
   acttaactca gttttaagat ttcctctttc cccccaactt ttccacattt cttaaatgag 3480
   ggtgtaattt actgcaaaca tgtacattta atataatatt gctgttagat tcgtggtaca 3540
   ctttatgaag caaaaacatg aaaaggaaac cacaactcta ataaacttta aattaaagat 3600
   taagatgact ctttctcacc cctcttcttg agtttagccc atgtctacac catgcacaca 3660
   gtcacaagac tttatagctt agaaacttta ggtataattg ggtcctatag acataatata 3720
   caatccacca actgagcctt ggccgaactt gtcccttgtt aaagacgtag ggctagccag 3780
   ccacagagct gggtattgaa gtcggcaagt tgtatcccgg tgcaggcgtc cttaaatgga 3840
   gagtactcag aatctgccag ccttaacctg cccactagcc accctaatca agcattaaat 3900
   atctgatcca atctggccac tggtgtaact ccgtaatcag acatcctgtt tgtaaacagc 3960
   tcctccttct ctccctctcc ctctcagaga cagaagtggt gctggggttt ggagtttgaa 4020
   aataaccttc ctatgacagg aagtgggctt ttgctattag ttgcccaact cttagaggat 4080
   cccagcttgt gtctttagac aaaactgaca gcctttggaa cgatcgcatg gtggcgctgc 4140
   cttccagcca acagttctgg ctcagcagca gaatttatcc agctatgttt gtagaatttt 4200
   tttaagatcc tactgcattt tgctcttaag cttttataaa aatttgacga gaaaatgtaa 4260
   aaatgatact tggcaataag acatttagtt ttcatgcacc taataaaaat gtgtcaaagc 4320
   tagatcgctc cagatgtgct taaaatatct caaatcctag gctctcattg gccttataag 4380
   tgattgggaa gaaaaagaat aaaataaact cacatatagg agatatggaa aatatttcat 4440
   caaatattat gcccaaagag agaaaattag gagttgattt tttttagcac tctgaatatg 4500
   gaacttcctt ttaaaaatgt aattctaccc ttcacctttc ctacattgta tgtttctgtg 4560
   agattttact cattacaagt gaaagtagag gactgcctgt gaattgttca cttctgaaaa 4620
   gaatatattg agatcagtta aaattactca agagtttggt gactcaggat tgtaaaaagc 4680
   ctggatctca acagctggga ggagtcagag ggtgaagtgg gaaaaaatag ggactgagag 4740
   gcaacgaaaa gtccctttga acctgtggtc atttatagca attagagtgg gttgcccctc 4800
   tacttctccc aagaagaaat cccaaataga ttcctttcat gtcagattat actgttctga 4860
   ttttattttc tattattttt acatatacat ccaaatctaa gaatacttat tcagattggc 4920
   ccactcaggc caaaagtcac tgtcttgtgt tctagaaatt ctttcatgtt gctgacataa 4980
   atccattttt aaaagaacat tcagggactc ttagaatttt agagtggaag ggacctttga 5040
   gatcatctac cagtgcttct caccccttag catgcagcaa aatcactcag atacttactt 5100
   attattcaca tttcttgacc ccagcactag aaatctggga ggatatctgc atttttaaca 5160
   agtgccctag tgattctaat gctgatggtg ctcagatcgc tcttggagaa cccctgattt 5220
   tatccaactc ccacatatga caaatgagga aagcctccca accctaagct ctgtcaggtg 5280
   acctgattcc aatgtatgat accaacaatc ttatgaaggc tggagaaaac cattaatact 5340
   gagacttgag tagatgatgc tcgtgaataa gaactttatg gctgggacca cagcagtcat 5400
   gggagcccat ccctggaagt ccataatata aagcctttaa tacagagtgg tggtttttct 5460
   cttttctaaa taccatttca gtctgttaag agaaaagaaa ttagtgttca gaaaagtagt 5520
   tactccatat gtgcagtaac tacttttcta taatgtctta tggcatctgg ggttcagcat 5580
   cattgaacaa gattaaatga acgttataga gctataaaat tgcatgtaaa ataacacaaa 5640
   actgttcaaa tgactatcaa agtagcctgg gaatgaattt ccactaaaat taattgtcac 5700
   tttcaaaacg tagcttagca aagctgttac taaactggag agatccttta atacatagaa 5760
   atgtcaaaaa aatcaaataa gtattcccta aacgatgtga gtgataagca aaagtcattg 5820
   caaagtcagt atttttttgt tcatcattgc tactgaaatt cagagttcca ttgtatatgt 5880
   gtgtcttgag tgtaccttat tagaatatcc aatacaatag cttttggata ttctatgctt 5940
   ttgtgtcctt cagaaatcat gagattgaag gaatctgtgt taaatgtttt tgttttggtt 6000
   gctgcctgaa aaaagttacc aactcattta gtgaaatgca agatttcttt atgaaaattt 6060
   caatttctaa tatcgaagtc agataatttt tcttgaactt ttattttgga acagatgttt 6120
   atttcggatc tattaaagtg tcaactttgc attatagaac tgaacatgaa aaaaaaaaaa 6180
   aaaaa 6185

<210> 21
<211> 4666
<212> DNA
<213> Homo sapiens

<400> 21
agtaccttgg tccagctctt cctgcaacgg cccaggagct cagagctcca catctgacct 60

tctagtcatg accaggacca gggcagcact cctcctgttc acagccttag caacttctct 120

aggtttcaac ttggacacag aggagctgac agccttccgt gtggacagcg ctgggtttgg 180

agacagcgtg gtccagtatg ccaactcctg ggtggtggtt ggagcccccc aaaagataac 240

agctgccaac caaacgggtg gcctctacca gtgtggctac agcactggtg cctgtgagcc 300

catcggcctg caggtgcccc cggaggccgt gaacatgtcc ctgggcctgt ccctggcgtc 360

taccaccagc ccttcccagc tgctggcctg cggccccacc gtgcaccacg agtgcgggag 420

gaacatgtac ctcaccggac tctgcttcct cctgggcccc acccagctca cccagaggct 480

cccggtgtcc aggcaggagt gcccaagaca ggagcaggac attgtgttcc tgatcgatgg 540

ctcaggcagc atctcctccc gcaactttgc cacgatgatg aacttcgtga gagctgtgat 600

aagccagttc cagagaccca gcacccagtt ttccctgatg cagttctcca acaaattcca 660

aacacacttc actttcgagg aattcaggcg cagctcaaac cccctcagcc tgttggcttc 720

tgttcaccag ctgcaagggt ttacatacac ggccaccgcc atccaaaatg tcgtgcaccg 780

attgttccat gcctcatatg gggcccgtag ggatgccgcc aaaattctca ttgtcatcac 840

tgatgggaag aaagaaggcg acagcctgga ttataaggat gtcatcccca tggctgatgc 900

agcaggcatc atccgctatg caattggggt tggattagct ttttcaaaca gaaattcttg 960

gaaagaatta aatgacattg catcgaagcc ctcccaggaa cacatattta aagtggagga 1020

ctttgatgct ctgaaagata ttcaaaacca actgaaggag aagatctttg ccattgaggg 1080

tacggagacc acaagcagta gctccttcga attggagatg gcacaggagg gcttcagcgc 1140

tgtgttcaca cctgatggcc ccgttctggg ggctgtgggg agcttcacct ggtctggagg 1200

tgccttcctg taccccccaa atatgagccc taccttcatc aacatgtctc aggagaatgt 1260

ggacatgagg gactcttacc tgggttactc caccgagctg gccctctgga aaggggtgca 1320

gagcctggtc ctgggggccc cccgctacca gcacaccggg aaggctgtca tcttcaccca 1380

ggtgtccagg caatggagga tgaaggccga agtcacgggg actcagatcg gctcctactt 1440

cggggcctcc ctctgctccg tggacgtaga cagcgacggc agcaccgacc tggtcctcat 1500

cggggccccc cattactacg agcagacccg agggggccag gtgtctgtgt gtcccttgcc 1560

cagggggtgg agaaggtggt ggtgtgatgc tgttctctac ggggagcagg gccacccctg 1620

gggtcgcttt ggggcggctc tgacagtgct gggggatgtg aatggggaca agctgacaga 1680

cgtggtcatc ggggccccag gagaggagga gaaccggggt gctgtctacc tgtttcacgg 1740

agtcttggga cccagcatca gcccctccca cagccagcgg atcgcgggct cccagctctc 1800

ctccaggctg cagtatttgg ggcaggcact gagcgggggt caagacctca cccaggatgg 1860

actggtggac ctggctgtgg gggcccgggg ccaggtgctc ctgctcagga ccagacctgt 1920

gctctgggtg ggggtgagca tgcagttcat acctgccgag atccccaggt ctgcgtttga 1980

gtgtcgggag caggtggtct ctgagcagac cctggtacag tccaacatct gcctttacat 2040

tgacaaacgt tctaagaacc tgcttgggag ccgtgacctc caaagctctg tgaccttgga 2100

cctggccctc gaccctggcc gcctgagtcc ccgtgccacc ttccaggaaa caaagaaccg 2160

gagtctgagc cgagtccgag tcctcgggct gaaggcacac tgtgaaaact tcaacctgct 2220

gctcccgagc tgcgtggagg actctgtgac ccccattacc ttgcgtctga acttcacgct 2280

ggtgggcaag cccctccttg ccttcagaaa cctgcggcct atgctggccg ccgatgctca 2340

gagatacttc acggcctccc taccctttga gaagaactgt ggagccgacc atatctgcca 2400

ggacaatctc ggcatctcct tcagcttccc aggcttgaag tccctgctgg tggggagtaa 2460

cctggagctg aacgcagaag tgatggtgtg gaatgacggg gaagactcct acggaaccac 2520

catcaccttc tcccaccccg caggactgtc ctaccgctac gtggcagagg gccagaaaca 2580

agggcagctg cgttccctgc acctgacatg tgacagcgcc ccagttggga gccagggcac 2640

ctggagcacc agctgcagaa tcaaccacct catcttccgt ggcggcgccc agatcacctt 2700

cttggctacc tttgacgtct cccccaaggc tgtcctggga gaccggctgc ttctgacagc 2760

caatgtgagc agtgagaaca acactcccag gaccagcaag accaccttcc agctggagct 2820

cccggtgaag tatgctgtct acactgtggt tagcagccac gaacaattca ccaaatacct 2880

caacttctca gagtctgagg agaaggaaag ccatgtggcc atgcacagat accaggtcaa 2940

taacctggga cagagggacc tgcctgtcag catcaacttc tgggtgcctg tggagctgaa 3000

ccaggaggct gtgtggatgg atgtggaggt ctcccacccc cagaacccat cccttcggtg 3060

ctcctcagag aaaatcgcac ccccagcatc tgacttcctg gcgcacattc agaagaatcc 3120

cgtgctggac tgctccattg ctggctgcct gcggttccgc tgtgacgtcc cctccttcag 3180

cgtccaggag gagctggatt tcaccctgaa gggcaacctc agctttggct gggtccgcca 3240

gatattgcag aagaaggtgt cggtcgtgag tgtggctgaa attacgttcg acacatccgt 3300

gtactcccag cttccaggac aggaggcatt tatgagagct cagacgacaa cggtgctgga 3360

gaagtacaag gtccacaacc ccacccccct catcgtaggc agctccattg ggggtctgtt 3420

gctgctggca ctcatcacag cggtactgta caaagttggc ttcttcaagc gtcagtacaa 3480

ggaaatgatg gaggaggcaa atggacaaat tgccccagaa aacgggacac agacccccag 3540

cccgcccagt gagaaatgat cccctctttg ccttggactt cttctccccc gcgagttttc 3600

cccacttact taccctcacc tgtcaggcct gacggggagg aaccactgca ccaccgagag 3660

aggctgggat gggcctgctt cctgtctttg ggagaaaacg tcttgcttgg gaaggggcct 3720

ttgtcttgtc aaggttccaa ctggaaaccc ttaggacagg gtccctgctg tgttccccaa 3780

aggacttgac ttgcaatttc tacctagaaa tacatggaca atacccccag gcctcagtct 3840

cccttctccc atgaggcacg aatgatcttt ctttcctttc tttttttttt tttttctttt 3900

cttttttttt tttttgagac ggagtctcgc tctgtcaccc aggctggagt gcaatggcgt 3960

gatctcggct cactgcaacc tccgcctccc gggttcaagt aattctgctg tctcagcctc 4020

ctgagtagct gggactacag gcacacgcca cctcgcccgg cccgatcttt ctaaaataca 4080

gttctgaata tgctgctcat ccccacctgt cttcaacagc tccccattac cctcaggaca 4140

atgtctgaac tctccagctt cgcgtgagaa gtccccttcc atcccagagg gtgggcttca 4200

gggcgcacag catgagaggc tctgtgcccc catcaccctc gtttccagtg aattagtgtc 4260

atgtcagcat cagctcaggg cttcatcgtg gggctctcag ttccgatttc ccaggctgaa 4320

ttgggagtga gatgcctgca tgctgggttc tgcacagctg gcctcccgcg ttgggcaaca 4380

ttgctggctg gaaggaggga gcgccctcta gggagggaca tggccccggt gcggctgcag 4440

ctcacccagc cccaggggca gaagagaccc aaccacttct attttttgag gctatgaata 4500

tagtacctga aaaaatgcca agacatgatt atttttttaa aaagcgtact ttaaatgttt 4560

gtgttaataa attaaaacat gcacaaaaag atgcatctac cgctcttggg aaatatgtca 4620

aaggtctaaa aataaaaaag ccttctgtga aaaaaaaaaa aaaaaa 4666

<210> 22
<211> 1699
<212> DNA
<213> Homo sapiens

<400> 22
agggctgagg cataaaactg ccagagggtc tcaaggcagg caaagaagaa ccatgaggct 60

catcctgcct gtgggtttga ttgctaccac tcttgcaatt gctcctgtcc gctttgacag 120

ggagaaggtg ttccgcgtga agccccagga tgaaaaacaa gcagacatca taaaggactt 180

ggccaaaacc aatgagcttg acttctggta tccaggtgcc acccaccacg tagctgctaa 240

tatgatggtg gatttccgag ttagtgagaa ggaatcccaa gccatccagt ctgccttgga 300

tcaaaataaa atgcactatg aaatcttgat tcatgatcta caagaagaga ttgagaaaca 360

gtttgatgtt aaagaagata tcccaggcag gcacagctac gcaaaataca ataattggga 420

aaagattgtg gcttggactg aaaagatgat ggataagtat cctgaaatgg tctctcgtat 480

taaaattgga tctactgttg aagataatcc actatatgtt ctgaagattg gggaaaagaa 540

tgaaagaaga aaggctattt ttacggattg tggcattcac gcacgagaat gggtctcccc 600

agcattctgc cagtggtttg tctatcaggc aaccaaaact tatgggagaa acaaaattat 660

gaccaaactc ttggaccgaa tgaattttta cattcttcct gtgttcaatg ttgatggata 720

tatttggtca tggacaaaga accgcatgtg gagaaaaaat cgttccaaga accaaaactc 780

caaatgcatc ggcactgacc tcaacaggaa ttttaatgct tcatggaact ccattcctaa 840

caccaatgac ccatgtgcag ataactatcg gggctctgca ccagagtccg agaaagagac 900

gaaagctgtc actaatttca ttagaagcca cctgaatgaa atcaaggttt acatcacctt 960

ccattcctac tcccagatgc tattgtttcc ctatggatat acatcaaaac tgccacctaa 1020

ccatgaggac ttggccaaag ttgcaaagat tggcactgat gttctatcaa ctcgatatga 1080

aacccgctac atctatggcc caatagaatc aacaatttac ccgatatcag gttcttcttt 1140

agactgggct tatgacctgg gcatcaaaca cacatttgcc tttgagctcc gagataaagg 1200

caaatttggt tttctccttc cagaatcccg gataaagcca acgtgcagag agaccatgct 1260

agctgtcaaa tttattgcca agtatatcct caagcatact tcctaaagaa ctgccctctg 1320

tttggaataa gccaattaat ccttttttgt gcctttcatc agaaagtcaa tcttcagtta 1380

tccccaaatg cagcttctat ttcacctgaa tccttctctt gctcatttaa gtcccatgtt 1440

actgctgttt gcttttactt actttcagta gcaccataac gaagtagctt taagtgaaac 1500

cttttaacta cctttctttg ctccaagtga agtttggacc cagcagaaag cattattttg 1560

aaaggtgata tacagtgggg cacagaaaac aaatgaaaac cttcagtttc tcacagattt 1620

tcaccatgtg gcttcatcaa tttatgtgct aatacaataa aataaaatgc acttaatgct 1680

ttaaaaaaaa aaaaaaaaa 1699

<210> 23
<211> 3865
<212> DNA
<213> Homo sapiens

<400> 23
acagaacacg gggtgcctgg aaggggaaca gatgtgttgt ggggcacagg gcaggctggg 60

aggggaacaa aggtccactc catgggtaac cagacccttc cgccagggct ggccacttct 120

gcctttggaa aatgtttcac aacgccccat gttgtgtgtg tgtgtgaatc ggccgatgtg 180

aaccgaatgt tgatgtaaga ggcagggcac tcggctgcgg atgggtaaca gggcgtgggc 240

tggcacactt acttgcacca gtgcccagag agggggtgca ggctgaggag ctgcccagag 300

caccgctcac actcccagag tacctgaagt cggcatttca atgacaggtg acaagggtcc 360

ccaaaggcta agcgggtcca gctatggttc catctccagc ccgaccagcc cgaccagccc 420

agggccacag caagcacctc ccagagagac ctacctgagt gagaagatcc ccatcccaga 480

cacaaaaccg ggcaccttca gcctgcggaa gctatgggcc ttcacggggc ctggcttcct 540

catgagcatt gctttcctgg acccaggaaa catcgagtca gatcttcagg ctggcgccgt 600

ggcgggattc aaacttctct gggtgctgct ctgggccacc gtgttgggct tgctctgcca 660

gcgactggct gcacgtctgg gcgtggtgac aggcaaggac ttgggcgagg tctgccatct 720

ctactaccct aaggtgcccc gcaccgtcct ctggctgacc atcgagctag ccattgtggg 780

ctccgacatg caggaagtca tcggcacggc cattgcattc aatctgctct cagctggacg 840

aatcccactc tggggtggcg tcctcatcac catcgtggac accttcttct tcctcttcct 900

cgataactac gggctgcgga agctggaagc tttttttgga ctccttataa ccattatggc 960

cttgaccttt ggctatgagt atgtggtggc gcgtcctgag cagggagcgc ttcttcgggg 1020

cctgttcctg ccctcgtgcc cgggctgcgg ccaccccgag ctgctgcagg cggtgggcat 1080

tgttggcgcc atcatcatgc cccacaacat ctacctgcac tcggccctgg tcaagtctcg 1140

agagatagac cgggcccgcc gagcagacat cagagaagcc aacatgtact tcctgattga 1200

ggccaccatc gccctgtccg tctcctttat catcaacctc tttgtcatgg ctgtctttgg 1260

gcaggccttc taccagaaaa ccaaccaggc tgcgttcaac atctgtgcca acagcagcct 1320

ccacgactac gccaagatct tccccatgaa caacgccacc gtggccgtgg acatttacca 1380

ggggggcgtg atcctgggct gcctgttcgg ccccgcggcc ctctacatct gggccatagg 1440

tctcctggcg gctgggcaga gctccaccat gacgggcacc tacgcgggac agttcgtgat 1500

ggagggcttc ctgaggctgc ggtggtcacg cttcgcccgt gtcctcctca cccgctcctg 1560

cgccatcctg cccaccgtgc tcgtggctgt cttccgggac ctgagggact tgtcgggcct 1620

caatgatctg ctcaacgtgc tgcagagcct gctgctcccg ttcgccgtgc tgcccatcct 1680

cacgttcacc agcatgccca ccctcatgca ggagtttgcc aatggcctgc tgaacaaggt 1740

cgtcacctct tccatcatgg tgctagtctg cgccatcaac ctctacttcg tggtcagcta 1800

tctgcccagc ctgccccacc ctgcctactt cggccttgca gccttgctgg ccgcagccta 1860

cctgggcctc agcacctacc tggtctggac ctgttgcctt gcccacggag ccacctttct 1920

ggcccacagc tcccaccacc acttcctgta tgggctcctt gaagaggacc agaaagggga 1980

gacctctggc taggcccaca ccagggcctg gctgggagtg gcatgtatga cgtgactggc 2040

ctgctggatg tggagggggc gcgtgcaggc agcaggatag agtgggacag ttcctgagac 2100

cagccaacct gggggcttta gggacctgct gtttcctagc gcagccatgt gattaccctc 2160

tgggtctcag tgtcctcatc tgtaaaatgg agacaccacc acccttgcca tggaggttaa 2220

gcactttaac acagtgtctg gcacttggga caaaaacaaa caaacgaaaa acatttcaaa 2280

aggtatttat tgagcacctg caggcgtgac ctgacagccc aagggtgggt ggggtgaggg 2340

cttgaggact tgggcgggac acaggctcca aactggagct tgaaatagtg tctgatgaat 2400

gttaaattat ctatctatct atttatttat ttatttgaga cagggaaagg gtctccctct 2460

gttgccaagg ctggagtgca gtggcgcaat cttaactcat tgcaacctcc accttctggg 2520

ttcaagcgat tctctttatt cagccccggg agtggcgcgc gccaccacgc ccagctaatt 2580

tgtgtatttt cagcagagac ggggtttgcc atgctggcca ggctggtctc gaactgctgg 2640

attcaagtga tccgcccatc tccgtctccc aaagtgctgg gaattacagg cgtgagccac 2700

caaacccggc ctgattaaag ttaaataaat actagttccc ttctcgtcca aaggagcagg 2760

gaatgggaac cgggaaggca cgaagtctct aaagcatcca gaagacccct acaccagggt 2820

ctggtccgct cctattcgcc gcagcctttc tgttccgcct gcaacccatt ttccagacag 2880

taaaacggcg gcgcacttct ttctccgtca ggcaccaggt cataaggaac ccaagagtct 2940

gtgcctctga ggcccaaatt atttgctgtt tcctcagggg agccggcggc cgcgactccc 3000

acgccgcgcc gttaccgctc cctctctgct gactgctccc cctaggggca gagacggtcc 3060

cgacgcccgc catcccgccc cggcctcacc cctccccgcc aggcggaacg acgcggggag 3120

gcgggcgctc ggggctcgcg ccaggggccc cagaatcctt cggggagagt gggtgggagg 3180

aagctgtgtg ggcggggagc cccctctgcc ttagggagcg gctgggcacc cattcgcccc 3240

attcaggggc tgcactttat agacgttccc taggctgttt ctaggctccc ccaagtccct 3300

cctccagcct cgtcgggtcc ctcagacccc agcccaggac ctgcggaggg ccgcagcgag 3360

gagaggccaa caggcctttc cctagagttg aacctgggcc gggtgttgca cctggaagaa 3420

cccccgattt cctggggacc cagcagggca ggcggcctgg ctccgcgctc aggtccggac 3480

gcttgtttat gagaagaatt tcctctttct taaaagggca acgatgcgag tgggtccctc 3540

aaggagagaa gagatgggac cggtctggtg cgacctgggc aagcgctgca gagggtacct 3600

gggcaagagg gccgcccgcc tcctctgggt ttggcactgg agaagatggg tccatgccag 3660

ctgaaggagg agatggatgg gggacgttta gcgaagaaag gcatctccca gatcctttag 3720

cctcctggaa gtgcccccgt tgtaccccct acacacccct cttggcattg agtgccagtc 3780

ctctgccagg ctctgtgtta caagttgggg agggcggcaa agtcccgaat taaagatgtc 3840

agttctcaag gaaaaaaaaa aaaaa 3865

<210> 24
<211> 1944
<212> DNA
<213> Homo sapiens

<400> 24
cggaagtcgc ttgtgtatga acgcagcggc ggacctgtga ggggatccga cttgccggca 60

gaacttacgc tgcgggaccc cgggcactgt tgctgctgcg ggagtccaga gaggcaggag 120

gatggagctc ggaaggattt cagctccagg ctggctgctg gaccgacttt tcaacatttt 180

ttaaaaagtg cctcagctcc tcaggagaag ctgtcttcag aagtggaaga cccacctccc 240

tatctcatga tggatgaact tcttggaagg cagagaaaag tctacctcga gacctatggc 300

tgccagatga atgtgaatga cacagagata gcctggtcca tcttacagaa gagtggctac 360

ctgcggacca gtaacctcca agaggcagat gtgattctcc ttgtcacatg ctctatcagg 420

gagaaggctg agcagaccat ctggaaccgt ttacatcagc ttaaagcctt gaagacaagg 480

cggccccgct cccgggttcc tctgaggatt ggaattctag gctgcatggc tgagaggttg 540

aaggaggaga ttctcaacag agagaaaatg gtagatattt tggctggtcc tgatgcctac 600

cgggaccttc cccggctgct ggctgttgct gagtcgggcc agcaagctgc caacgtgctg 660

ctctctctgg acgagaccta tgctgatgtc atgccagtcc agacaagcgc cagtgccacg 720

tctgcctttg tgtcaatcat gcgaggctgt gacaacatgt gtagctactg cattgttcct 780

ttcacccggg gcagggagag gagtcggcct attgcctcca ttctagagga agtgaagaag 840

ctttctgagc aggggctgaa agaagtgaca cttcttggtc agaatgttaa tagttttcgg 900

gacaattcgg aggtccagtt caacagtgca gtgcctacca atctcagtcg tggctttacc 960

accaactata aaaccaagca aggaggactt cgttttgctc atcttctgga tcaggtctcc 1020

agagtagatc ctgaaatgag gatccgtttt acctctcccc accccaagga ttttcctgat 1080

gaggttctgc agctgattca tgagagagat aacatctgta aacagatcca cctgccagcc 1140

cagagtggaa gcagccgtgt gttggaggcc atgcggaggg gatattcaag agaagcttat 1200

gtggagttag ttcaccatat tagagaatct attccaggtg tgagcctcag cagcgatttc 1260

attgctggct tttgtggtga gacggaggaa gatcacgtcc agacagtctc tttgctccgg 1320

gaagttcagt acaacatggg cttcctcttt gcctacagca tgagacagaa gacacgggca 1380

tatcataggc tgaaggatga tgtcccggaa gaggtaaaat taaggcgttt ggaggaactc 1440

atcactatct tccgagaaga agcaacaaaa gccaatcaga cctctgtggg ctgtacccag 1500

ttggtgctag tggaagggct cagtaaacgc tctgccactg acctgtgtgg caggaatgat 1560

ggaaacctta aggtgatctt ccctgatgca gagatggagg atgtcaataa ccctgggctc 1620

agggtcagag cccagcctgg ggactatgtg ctggtgaaga tcacctcagc cagttctcag 1680

acacttaggg gacatgttct ctgcaggacc actctgaggg actcttctgc atattgctga 1740

cctgagagga tggcctcaga gctgacttgg gcaatcctcc ccaacaggaa ggggagacat 1800

tgcctgccac tgaggaaaca ggtcatgaag gtggagataa gctgcaaggg gcgaagcaac 1860

tttatgtcag tggaaaacgt gtctctttaa agctgctatg tgaacagctt ttacagtcat 1920

taaatttacc taaactaagg ttaa 1944

<210> 25
<211> 7782
<212> DNA
<213> Homo sapiens

<400> 25
gagtcacgcg atttccggga acccgtcagg aaggacataa acaaaacaaa cccgaggcag 60

catggagagg ggccgtggcc cctgcagcgg aaccggaccc agtccctgag ccgcccctac 120

acccacagac agcatcgcac agaattattt taaaaaaaag cagtgatcca agcaattgaa 180

ttggaagcac tctggggaaa cctgctgttt attgtggaaa tcatcttcga tcttggaatt 240

gaaagtaaag ctggaaagga atttacaaac aagaaaaaaa agaagtttgg aatcggattc 300

acaggatctg ggcttggaaa tgcctcagcc tagtgtaagc ggaatggatc cgcctttcgg 360

ggatgccttt cgaagccaca ccttttcgga acaaactctg atgagcacag atctcttagc 420

aaacagttcg gatccagatt tcatgtatga actggataga gagatgaact accaacagaa 480

tcctagagac aactttcttt ctttggagga ctgcaaagac attgaaaatc tggagtcttt 540

cacagatgtc ctggataatg agggtgcttt aacctcaaac tgggaacagt gggatacata 600

ctgtgaagac ctaacgaaat ataccaaact aaccagctgt gacatctggg gaacaaaaga 660

agtggattac ttgggtcttg atgacttttc tagtccttac caagatgaag aggttataag 720

taaaactcca actttagctc aacttaatag tgaggactca cagtctgttt ctgattccct 780

ttattacccc gattcacttt tcagtgtcaa acaaaatccc ttaccctctt cattccctgg 840

taaaaagatc acaagcagag cagctgctcc tgtgtgttct tctaagactc tgcaggctga 900

ggtccctttg tcagactgtg tccaaaaagc aagtaaaccc acttcaagca cacaaatcat 960

ggtgaagacc aacatgtatc ataatgaaaa ggtgaacttt catgttgaat gtaaagacta 1020

tgtaaaaaag gcaaaggtaa agatcaaccc agtgcaacag agccggccct tgttgagcca 1080

gattcacaca gatgcagcaa aggagaacac ctgctactgt ggtgcagtgg caaagagaca 1140

agagaaaaaa gggatggagc ctcttcaagg tcatgccact cccgctttgc cttttaaaga 1200

aacccaggaa ctattactaa gtcccctgcc ccaggaaggt cctgggtcac ttgcagcagg 1260

agagagcagc agtctttctg ccagtacatc agtctcagat tcatcccaga aaaaagaaga 1320

gcacaattat tctctttttg tctccgacaa cttgggtgaa cagccaacta aatgcagtcc 1380

tgaagaagat gaggaggacg aggaggatgt tgatgatgag gaccatgatg aaggattcgg 1440

cagtgagcat gaactgtctg aaaatgagga ggaggaagaa gaggaagagg attatgaaga 1500

tgacaaggat gatgatatta gtgatacttt ctctgaacca ggctatgaaa atgattctgt 1560

agaagacctg aaggaggtga cttcaatatc ttcacggaag agaggtaaaa gaagatactt 1620

ctgggagtat agtgaacaac ttacaccatc acagcaagag aggatgctga gaccatctga 1680

gtggaaccga gatactttgc caagtaatat gtatcagaaa aatggcttac atcatggaaa 1740

atatgcagta aagaagtcac ggagaactga tgtagaagac ctgactccaa atcctaaaaa 1800

actcctccag ataggcaatg aacttcggaa actgaataag gtgattagtg acctgactcc 1860

agtcagtgag cttcccttaa cagcccgacc aaggtcaagg aaggaaaaaa ataagctggc 1920

ttccagagct tgtcggttaa agaagaaagc ccagtatgaa gctaataaag tgaaattatg 1980

gggcctcaac acagaatatg ataatttatt gtttgtaatc aactccatca agcaagagat 2040

tgtaaaccgg gtacagaatc caagagatga gagaggaccc aacatggggc agaagcttga 2100

aatcctcatt aaagatactc tcggtctacc agttgctggg caaacctcag aatttgttaa 2160

ccaagtgtta gagaagactg cagaagggaa tcccactgga ggccttgtag gattaaggat 2220

accaacatca aaggtgtaat cagcctcatt ggaccactgg tcagaaatgt ctgcgttttg 2280

tcacgttatc cattgtaaat tttcattctg ttttgcatgt cagttagcat tatgtaaaca 2340

tttacaatta ggttacattg ttttaagaac taagtagcat aagtgaagca tgatccaaaa 2400

tacttgatta ttgcattttc agagcataaa ccatgattaa aactgctact ggcatcagaa 2460

ttgaaaatca tatgtttaag taaatgttag gtacagatta caaaaatctg ttaaagcaaa 2520

acattttgga ggagtgaaat agtaaaatgc caagtattgt ggcagattta tgctctgaac 2580

cacacaaaaa aattgaggaa gcattttttt aaacagtcgg tttaaattgt ttttagaatt 2640

attgcttttt gttctaattt tccacaacca ttaatctcac ttgtatatgg cacacccagc 2700

acttgtgcct gtgggccata ttagatgttc attgtcagag ctcaagatga tatatataaa 2760

tatatatata tatatatata tatacacaca cacacacaaa tgtctgtgca agtaagaaaa 2820

aaaaagcata ttctttgtgc cttgtatttt ggggaaactc taaaactggt aatattttgt 2880

atgatgaaaa ccctaatgag aaaaaacaag atatatagat ggaaaaatta tggggtttaa 2940

atgttttttt gttccaactc tttttcagat tttttgaatg tatataggac tatgttgaaa 3000

tgtagatata tgccacagag tctgtgtatt gtataaaaaa caaaacaaaa aacaacaaaa 3060

aaaagatggc tctagaaaac tcatatttcg gtacttgacc ggaagaagac aaatacttgc 3120

acattattgc gattgtttta ttttttgtac caaagacaaa tgcaactgat atggcaaact 3180

gccagtctaa gtaaagtttt gcacagctta catgatactg tatgaatgta tgaaaaaaaa 3240

ggagaaaaaa aagaaaaaaa aaggtcaggg ttagggatct tactgaactg tgaattttat 3300

ttctgtttgg gtccaattat ctacagaagg agcatccata catacaaata ttattttgct 3360

gttcctctag ttcgcttcca tagtagataa gttggtggcc atttagatgt cttttatttc 3420

tgcacttatt gtaggaaatt ttaatatatt tcattttagt aagctattga taaaatagtt 3480

tttgactttg aaaattaaaa tgtttattta gcttattgta gtatacttcc accagacaac 3540

aaaatagatt atttttattg tattatgtat atatatatat gtaaagaaag aaaaaagcta 3600

aaaatatcta attctttagt tgccactttt ccgattgatg tattattgtg catgtaatat 3660

tttcaaagat caacacaggc taaaacaaaa acaatttata gatttttata tttttgtaca 3720

ggtattttca aactagcttc ttcaaactta acatgtgact tattcttcta tagtttctag 3780

aattgagaaa cattaacaca tttagttttt aggtgctctt ttttgctcat ataaaacagc 3840

ttcattagtc agtgttttaa ctgtgttcaa gctttacctc ttgatgagaa atttcttatg 3900

tcaaggcagc attataaacc ttcccccaca gatttttcca tcctgtctct cttactgttt 3960

tattctcaaa tcttgtgctt tgaactctga aaactggtgg cttaaaaact aaaaaaagaa 4020

aaaaagcata tttagcaagg aaaaaaatac caaaaatttc aggcatagct gctggaaaaa 4080

ttatctattt ctccattacc cactgtagga tttctttttt aattatactt tgactataaa 4140

gtgtcaaagt ataatttgtt cttttctttt actttgttac cccatttgta agctatagca 4200

tatgaagcta tatatatagc ttgtgaaggt ttgatctaga acacccagta acaaatgaac 4260

aatgttgctt acctgcttct ttgacatctt aaaaaagaaa tccaaggagg attgtaagga 4320

ttgtcttacc accttagctg aactgtgatg cacaagattt ttctatgtgt ttggtggaaa 4380

tgtacctggt ttgtacattc acgctaaaca gatgataagc tcaagtctga tggtttaata 4440

gaatgtaagt tcatcgttta aagcttttcc tttttaggtt ggagaaggca aaacacaggc 4500

ttgcaagttg gaagtatatg aagtcttgac agagtgtgtc tggtaaattg aaaagtgttt 4560

caaactatgg cagttttgca atcaggtgaa aatcacctca tgatattcag ctgataaggt 4620

ttataaaatt gcccctttct agctgctctg ttaggaattc tggtttttga tacttttttc 4680

ctgtctgcaa accagaattt gattttttgg tcttgcattt caaaaaaaaa aagactttga 4740

atctgtttag tagattccat atctttgagt ttcagtgttt tatatgtact acttaagtta 4800

aatagttaaa agcttttaaa tagttgagct ttttaatgtt gacactttat tttgtaccta 4860

tttatatatg tatgtatatc ttagaaaagc actttgttaa aaaaaaattg cattttatat 4920

gattcctgcc atttgctgct aaatctgggc tggtcagaat gctgcagcga tacttgatct 4980

atataaaaac ctggcagtaa aatgtagagt gaaagttaaa tcctcttgct gttttaactt 5040

tatcataaag atgacatagg caagctgtgc agctttacat tttaaccagg ggactctgtg 5100

gcatttaaaa ccgtctagaa atggttgtac tttaatgcca gtaataatct gcttcctcta 5160

ttgtcattaa aatatatacg tttagtgtat cacacaaacc aatcttataa gggtaatgta 5220

aaaaccccaa caattgtaca tgttctgttt ttgaaaattg tggcatgtat ttttgggtga 5280

agatcattag agaagagttc tctaaaggtt ttctgtgttc atacatggta tacagatagc 5340

tcataatgaa gtccagaatc ttacttttaa gtgaaggcat tgtgaattca cctcaagtaa 5400

acccattgtt ccaaagcaat tataaacttt gactctagta ctactatgat ttaaaaaaaa 5460

aaaaaaccaa caaaaacctt ttttcctagt ttcagataca ctggattctt tatagagttt 5520

gtctccatat gaaagcatgc tgtccagtcg ctcttgttaa gatcttgtct gagttttgaa 5580

ttgggtgcca cacttttcca gtcaatataa ttgcttgttc tactgtacca tgtatgattc 5640

ttgtcctttc ctatatcctt catgacagat tatgatgtgg ctttatattg tgccttactt 5700

gtacatttaa aactaaacgt cttcattccc ttccacttcc tacatcttta actttgacct 5760

ttttggtaag agaatcagaa ctattacaaa agcatcatga aggatttcag atgggtatgg 5820

tttcaaattc cctctcttta tagttatttt atatttgtat gaaagaccag ttttggatgg 5880

tctttgaata taggggggaa agattagcag taatttcact acatcccttt tctctgactt 5940

tcatgcattt ctcatacatc ttctttctga tgcttgactt tatttgcttc ctagcaatag 6000

tctgcattta aagaaaggtg tgttcaattc atcagcttga aattgactat ttcatttttc 6060

caggattttt taggagaaga gtacccattt tgttttataa aaacagatga caagtctctt 6120

taaaagaaac agaagtacag tacttttgaa atacaatgct gttagtttgg atttcttttt 6180

atatatatat ataatattca tacaatgatc tgatgtttgc cttcattaat aaagctgtta 6240

gtttattcac caaaatgtca agaatggatg tgcttttctt tattccacac atttaaaaaa 6300

atttagctgc taagatttaa tgttataaga aatgaattca agttgccttc agcaagaatt 6360

aacaaaaact tatgttccct ttctttatat agtttcctaa aattctgttc aagtattttc 6420

tagttaatta tgtaacagaa tgttagcatc tctccatatc ttgaaacttg aattttgaga 6480

atgcattgaa ttatgctttc agtgttaaag taaaaggttt caattatcct tctagtgaag 6540

tctgttgtgg aataccattt cccatggaac tgaggccatt tccacaactt tgcacagaac 6600

tgcagtcttg ttcttccctt ggatcatgac aaataagtct cacacagtgc cgtaatactt 6660

gtggattctt ttgtaatctt tgtaatctta ataagggcat tatgagaaga cgactccatg 6720

tttttttaat acttcaaaca cattgggatg taacaatgaa tgtcaactgt aggaatggtg 6780

gtttcgtttt aaggaataag catgttgggg aaagatgatg aaaatgtact actgaaagtt 6840

atacacttcc ataggcaaat gggattatgt gttgaagcat agtcctcatg cttaataaac 6900

tgactgaaat cgtagaaatt acacctagga actgagctag gccaaattgc catttttgtt 6960

tagagagttt tggaggtagt agtgagggga cagagcctta aaactacttc caaacagtat 7020

tttggaattg aagacttggt aactagtgaa gaacatcaaa gttgggtatt tcaatgtgcc 7080

aagtttgggt gaactaggtt cggtttgcct ctttcataac aatgtaaaca caatggtgta 7140

gttaattaaa ttctgggtgg ataggagcag gactgattac tatgtcttgc ccttcgccct 7200

ttgttttttt cagaaccaaa taacagaaat gtgtatgtgt gtactgtatc tgcctttcca 7260

ccacattttt atgacactgt attccactgc ctgctttttt accttctttc cctaggattt 7320

gtcctacagc ttagtattgt ggttgacagc gatactaggg ctgacagcac agaagtcaca 7380

agagaagagt ggaagggcaa gaattcaaag catttgttca tacaatgtgg caacctcttt 7440

tgcatagttg cgtaggatcc tgtttgtaat gctatcataa atattctgta gttttttttt 7500

tttctctccc aactggagct atgacacttt ttattggatt cagtcttgtc tcttgtctag 7560

aaagaacttt atcttgttga cgcatgagct gtttaaaaat tatcctatta aatgttggtt 7620

aatagttgtg cagtttttca tttcagatgg aaaggcaatg caaattttgc ctttgttttc 7680

tgtcaccttc caacccctga gcacttctag tcagatacag attcatcagt gtatgcaaca 7740

tcctttgtaa tttaaaataa aaaaagatga aaagaaaacg tt 7782

<210> 26
<211> 4573
<212> DNA
<213> Homo sapiens

<400> 26
gcccgagaag ctggggagca tggaccagac cccgcagcgc tggcaccatg acgctcgccc 60

gcttcgtgct agccctgatg ttaggggcgc tccccgaagt ggtcggcttt gattctgtcc 120

tcaatgattc cctccaccac agccaccgcc attcgccccc tgcgggtccg cactacccct 180

attaccttcc cacccagcag cggcccccga ggacgcgtcc gccgccgcct ctcccgcgct 240

tcccgcgccc cccgcgggcg ctccctgccc agcgcccgca cgccctccag gccgggcaca 300

cgccccggcc gcacccctgg ggctgccccg ccggcgagcc atgggtcagc gtgacggact 360

tcggcgcccc gtgtctgcgg tgggcggagg tgccaccctt cctggagcgg tcgcccccag 420

cgagctgggc tcagctgcga ggacagcgcc acaacttttg tcggagcccc gacggcgcgg 480

gcagaccctg gtgtttctac ggagacgccc gtggcaaggt ggactggggc tactgcgact 540

gcagacacgg atcagtacga cttcgtggcg gcaaaaatga gtttgaaggc acagtggaag 600

tatatgcaag tggagtttgg ggcactgtct gtagcagcca ctgggatgat tctgatgcat 660

cagtcatttg tcaccagctg cagctgggag gaaaaggaat agcaaaacaa accccgtttt 720

ctggactggg ccttattccc atttattgga gcaatgtccg ttgccgagga gatgaagaaa 780

atatactgct ttgtgaaaaa gacatctggc agggtggggt gtgtcctcag aagatggcag 840

ctgctgtcac gtgtagcttt tcccatggcc caacgttccc catcattcgc cttgctggag 900

gcagcagtgt gcatgaaggc cgggtggagc tctaccatgc tggccagtgg ggaaccgttt 960

gtgatgacca atgggatgat gccgatgcag aagtgatctg caggcagctg ggcctcagtg 1020

gcattgccaa agcatggcat caggcatatt ttggggaagg gtctggccca gttatgttgg 1080

atgaagtacg ctgcactggg aatgagcttt caattgagca gtgtccaaag agctcctggg 1140

gagagcataa ctgtggccat aaagaagatg ctggagtgtc ctgtacccct ctaacagatg 1200

gggtcatcag acttgcaggt gggaaaggca gccatgaggg tcgcttggag gtatattaca 1260

gaggccagtg gggaactgtc tgtgatgatg gctggactga gctgaataca tacgtggttt 1320

gtcgacaatt gggatttaaa tatggtaaac aagcatctgc caaccatttt gaagaaagca 1380

cagggcccat atggttggat gacgtcagct gctcaggaaa ggaaaccaga tttcttcagt 1440

gttccaggcg acagtgggga aggcatgact gcagccaccg cgaagatgtt agcattgcct 1500

gctaccctgg cggcgaggga cacaggctct ctctgggttt tcctgtcaga ctgatggatg 1560

gagaaaataa gaaagaagga cgagtggagg tttttatcaa tggccagtgg ggaacaatct 1620

gtgatgatgg atggactgat aaggatgcag ctgtgatctg tcgtcagctt ggctacaagg 1680

gtcctgccag agcaagaacc atggcttact ttggagaagg aaaaggaccc atccatgtgg 1740

ataatgtgaa gtgcacagga aatgagaggt ccttggctga ctgtatcaag caagatattg 1800

gaagacacaa ctgccgccac agtgaagatg caggagttat ttgtgattat tttggcaaga 1860

aggcctcagg taacagtaat aaagagtccc tctcatctgt ttgtggcttg agattactgc 1920

accgtcggca gaagcggatc attggtggga aaaattcttt aaggggtggt tggccttggc 1980

aggtttccct ccggctgaag tcatcccatg gagatggcag gctcctctgc ggggctacgc 2040

tcctgagtag ctgctgggtc ctcacagcag cacactgttt caagaggtat ggcaacagca 2100

ctaggagcta tgctgttagg gttggagatt atcatactct ggtaccagag gagtttgagg 2160

aagaaattgg agttcaacag attgtgattc atcgggagta tcgacccgac cgcagtgatt 2220

atgacatagc cctggttaga ttacaaggac cagaagagca atgtgccaga ttcagcagcc 2280

atgttttgcc agcctgttta ccactctgga gagagaggcc acagaaaaca gcatccaact 2340

gttacataac aggatggggt gacacaggac gagcctattc aagaacacta caacaagcag 2400

ccattccctt acttcctaaa aggttttgtg aagaacgtta taagggtcgg tttacaggga 2460

gaatgctttg tgctggaaac ctccatgaac acaaacgcgt ggacagctgc cagggagaca 2520

gcggaggacc actcatgtgt gaacggcccg gagagagctg ggtggtgtat ggggtgacct 2580

cctgggggta tggctgtgga gtcaaggatt ctcctggtgt ttataccaaa gtctcagcct 2640

ttgtaccttg gataaaaagt gtcaccaaac tgtaattctt catggaaact tcaaagcagc 2700

atttaaacaa atggaaaact ttgaaccccc actattagca ctcagcagag atgacaacaa 2760

atggcaagat ctgtttttgc tttgtgttgt ggtaaaaaat tgtgtacccc ctgctgcttt 2820

tgagaaattt gtgaacattt tcagaggcct cagtgtagtg gaagtgataa tccttaaatg 2880

aacattttct accctaattt cactggagtg acttattcta agcctcatct atcccctacc 2940

tatttctcaa aatcattcta tgctgatttt acaaaagatc atttttacat ttgaactgag 3000

aacccctttt aattgaatca gtggtgtctg aaatcatatt aaatacccac atttgacata 3060

aatgcggtac cctttactac actcatgagt ggcatattta tgcttaggtc ttttcaaaag 3120

acttgacaag aaatcttcat attctctgta gcctttgtca agtgaggaaa tcagtggtta 3180

aagaattcca ctataaactt ttaggcctga ataggagtag taaagcctca aggacatctg 3240

cctgtcacaa tatattctca aagtgatctg atatttggaa acaagtatcc ttgttgagta 3300

ccaagtgcta cagaaaccat aagataaaaa tactttctac ctacagcgta taaactagca 3360

aaatttgaaa ggatttctgg taactaaatg ccagctggac atacaaccta ttttatccaa 3420

cacattaaca tatctactat atttgctttt aacaagcacc tcatcatagt ccacgttccc 3480

cgaccatgat gtaagagtgc cacccactga cacttccctg taactgactt atacctgcag 3540

gttttcccaa gcccaacctg atacaatcca atcttaaaat atactagtca aaatgctttc 3600

ctggcctaca cattgaaaag ttctaataca tacagcagtt ataacatatg ccaggcactg 3660

cattaggcag ctttgcacat cactgacaat ttgcaaaagg ccagtaaatc ttatggtagt 3720

agcccttgtt ttcaaatggg ggaaaacagg cttgcctcaa agtgagacca gttggtgaaa 3780

ctgattccaa actccagaaa aaatgctgat ttttttcccc atggttctcc tacctaaatg 3840

attggtttcc ctatggctat ttctccaaga gttaagggat agctgcttaa ctatgcacct 3900

ttacagaaca ttcttagcag taatgctcaa atttaaaagg cacactcaag atacttccat 3960

gtcatggatc cttccccagg tcccagtagt ataaatatag ggaagaggtt agcatgaact 4020

tacaaattgt tttaagtaat cctcttgaat gccagtcatt aaaggacttt gcccttctac 4080

atcaaattac atccttttca caaatcccca tttctgtaat aactggtgca aacctaaagg 4140

tgctttatag ttttactact ttgcagattt gcaatgctgc atataatgca gaagagcatt 4200

aaaaactttt gtaaaaactc atgattttga taaactttta aagtagcgtt tatatgtaaa 4260

tagaactaca catggcacac acacttgcac aagggcttca gaaaaacgtg caatataggt 4320

gagaaaaatg tctattgaaa ctttctcaca ggctgccctt ataattaaac tagtgttggg 4380

gcaagcaaca tctgtttcaa gtagatcagg gactagcaaa cctaaaaggc agcaagcagc 4440

ctgcagtttg ctcaatcctt aagaatacat ggttcataca acatgctttt actatgtctt 4500

aagtgtgact tttgaaagat acaagtgttc tgtgcatatt tgtgtaaata aatacataat 4560

tttccacctt gta                                                    4573

<210>  27
<211>  2276
<212>  DNA
<213>  Homo sapiens

<400>  27
ctcattgaac tcgcctgcag ctcttgggtt ttttgtggct tccttcgtta ttggagccag      60

gcctacaccc cagcaaccat gtccaaggga cctgcagttg gtattgatct tggcaccacc     120

tactcttgtg tgggtgtttt ccagcacgga aaagtcgaga taattgccaa tgatcaggga     180

aaccgaacca ctccaagcta tgtcgccttt acggacactg aacggttgat cggtgatgcc     240

gcaaagaatc aagttgcaat gaaccccacc aacacagttt ttgatgccaa acgtctgatt     300

ggacgcagat ttgatgatgc tgttgtccag tctgatatga aacattggcc ctttatggtg     360

gtgaatgatg ctggcaggcc caaggtccaa gtagaataca agggagagac caaaagcttc     420

tatccagagg aggtgtcttc tatggttctg acaaagatga aggaaattgc agaagcctac     480

cttgggaaga ctgttaccaa tgctgtggtc acagtgccag cttactttaa tgactctcag     540

cgtcaggcta ccaaagatgc tggaactatt gctggtctca atgtacttag aattattaat     600

gagccaactg ctgctgctat tgcttacggc ttagacaaaa aggttggagc agaaagaaac     660

gtgctcatct ttgacctggg aggtggcact tttgatgtgt caatcctcac tattgaggat     720

ggaatctttg aggtcaagtc tacagctgga gacacccact tgggtggaga agattttgac     780

aaccgaatgg tcaaccattt tattgctgag tttaagcgca agcataagaa ggacatcagt     840

gagaacaaga gagctgtaag acgcctccgt actgcttgtg aacgtgctaa gcgtaccctc     900

tcttccagca cccaggccag tattgagatc gattctctct atgaaggaat cgacttctat     960

acctccatta cccgtgcccg atttgaagaa ctgaatgctg acctgttccg tggcaccctg    1020

gacccagtag agaaagccct tcgagatgcc aaactagaca agtcacagat tcatgatatt    1080

gtcctggttg gtggttctac tcgtatcccc aagattcaga agcttctcca agacttcttc    1140

aatggaaaag aactgaataa gagcatcaac cctgatgaag ctgttgctta tggtgcagct    1200

gtccaggcag ccatcttgtc tggagacaag tctgagaatg ttcaagattt gctgctcttg    1260

gatgtcactc ctctttccct tggtattgaa actgctggtg gagtcatgac tgtcctcatc    1320

aagcgtaata ccaccattcc taccaagcag acacagacct tcactaccta ttctgacaac    1380

cagcctggtg tgcttattca ggtttatgaa ggcgagcgtg ccatgacaaa ggataacaac    1440

ctgcttggca agtttgaact cacaggcata cctcctgcac cccgaggtgt tcctcagatt    1500

gaagtcactt ttgacattga tgccaatggt atactcaatg tctctgctgt ggacaagagt 1560

acgggaaaag agaacaagat tactatcact aatgacaagg gccgtttgag caaggaagac 1620

attgaacgta tggtccagga agctgagaag tacaaagctg aagatgagaa gcagagggac 1680

aaggtgtcat ccaagaattc acttgagtcc tatgccttca acatgaaagc aactgttgaa 1740

gatgagaaac ttcaaggcaa gattaacgat gaggacaaac agaagattct ggacaagtgt 1800

aatgaaatta tcaactggct tgataagaat cagactgctg agaaggaaga atttgaacat 1860

caacagaaag agctggagaa agtttgcaac cccatcatca ccaagctgta ccagagtgca 1920

ggaggcatgc caggaggaat gcctggggga tttcctggtg gtggagctcc tccctctggt 1980

ggtgcttcct cagggcccac cattgaagag gttgattaag ccaaccaagt gtagatgtag 2040

cattgttcca cacatttaaa acatttgaag gacctaaatt cgtagcaaat tctgtggcag 2100

ttttaaaaag ttaagctgct atagtaagtt actgggcatt ctcaatactt gaatatggaa 2160

catatgcaca ggggaaggaa ataacattgc actttataaa cactgtattg taagtggaaa 2220

atgcaatgtc ttaaataaaa ctatttaaaa ttggcaccat aaaaaaaaaa aaaaaa 2276

<210> 28
<211> 3213
<212> DNA
<213> Homo sapiens

<400> 28
ggagcctgcc gctccccgcg ctcgtagcgc gggcctgggg actggggatc ccgccgccgg 60

gccgcagcat ggggcgcttc cgcgggggcc tgcggtgcat caagtacctg ctgcttggct 120

tcaacctgct cttctggctg gctggatcgg ccgtcattgc ttttggacta tggtttcggt 180

tcggaggtgc cataaaggag ttatcatcag aggacaagtc cccagagtat ttctatgtgg 240

ggctgtatgt tctggttgga gccggggccc tgatgatggc cgtggggttc ttcgggtgct 300

gcggagccat gcgggagtcg caatgtgtgc ttggatcatt ttttacctgc ctcctggtga 360

tatttgctgc tgaagtaacc actggagtat ttgcttttat aggcaagggg gtagctatcc 420

gacatgttca gaccatgtat gaagaggctt acaatgatta ccttaaagac aggggaaaag 480

gcaatgggac actcatcacc ttccactcaa catttcagtg ctgtggaaaa gaaagctccg 540

aacaggtcca acctacatgc ccaaaggagc ttctaggaca caagaattgc atcgatgaaa 600

ttgagaccat aatcagtgtt aagctccagc tcattggaat tgtcggtatt ggaattgcag 660

gtctgacgat ctttggcatg atattcagca tggtcctctg ctgtgcgata cgaaactcac 720

gagatgtgat atgaagctac ttctacatga aaattgcaat ctaaagcttt cataccaaat 780

gtcacaggag ctgtctccca gctcattttt aacactgaaa tgacattagg atctaaaata 840

atttgctgtc aattgtacat ttgcatgagt acgtatgttt ggctcattac tggtttaccc 900

cttgagtgaa tgcctgttta tgatgactga gagcatattc atgtgtgatc tgcgtgtttc 960

tggaatatgc tttatacgta atgaaatctg tttgctggga attcctgatt cttgttatat 1020

aagaagaaca acctatttcg ctcccagaaa aaaaagatca aagagctttc agaaactttg 1080

agaacttggc tatttagaaa aagtgataat gggtcagttt ctcagactgt agccattgaa 1140

aattagatgc agagaattca gagatttctt cttaatggaa gtaataagct gtaagaattg 1200

agagatcaca atggagtgtt aaaactgact gtgtctaagt tgggtgtaag ggtttcctgg 1260

gtttttttat atacatgctc tccccagaat acagtaaacc acagttttag aactaaacac 1320

atctgtaaaa ctaaatatag catggaaaat ccaatttgaa taagtcatgc tttcctagaa 1380

tttaaaaata aaaaagtctt cctctggaaa gagaagtcac acagacaatc atgtgcccta 1440

taaaagtgag tgtttatagg actaaaaaac ttttaacaac tttttaagga aatatttttg 1500

ttcttataca aaaacatgta aatattgctt tattactttc attttctgac cctgctgtaa 1560

actactgcaa ccctcacatc ctcaaaggga cttttatgtc aaactcttct gtttctccaa 1620

atataaggaa aaaagactaa agcaagagat ctggcagttg aaaattgtgg gaaagagaat 1680

ttgtatgggc actgtatcta tgaaatacct cataacttac gtttacatgt tttcctaact 1740

ttttgtattt ttcttgtata gccacctaga gaattcttca tagattaaga actacagttt 1800

tcaccactta acataagtaa aacaaagtcc ttcataattt aaccattagc atctttggcc 1860

aaaccaaaat aaagaaaagc atcttctcct agttgtgtgt gggcaacaga aacaagttaa 1920

ggaaacaaaa atacttatat atacacagaa caaaaataat gttcttttta tgcaaatccc 1980

ctgtgaaaat aaaattttca atgttttttt ggtcttaacg cttcatttta acttagtaaa 2040

cccaaattta ggatttacat tggcaaattt ttcagtgatt tttaacgtgt tacatttagt 2100

aaaccataga ttttttaaaa actacatttt gtaaggccac acataagtga aaatacggaa 2160

ggctttggaa acccccagat gaacttctat acatgtgaaa acacaagtct ttgagtgtgc 2220

actaaaagtg tcacactccc aaaaaactca caaaacattt tgctaaaaag atatgactgc 2280

ttgaaatagt ctctctccca tggagaacca catttctctt tctgccatac ttgctactgc 2340

tactgatttt ggaggaaatt tttctagaag aacaaaggtc atatttgagg caagctaaaa 2400

acttaattat acatggctac tttactgtgt ctgtagctaa agaaaaatta ttttacaaaa 2460

attacttcat gaaatatagt ttgtacccca gtagttgaga aatgtgacct tttaggcaaa 2520

gaacaggcag tccatctctc ttagctttag tttatctctc tgcctgaaga caggaaacag 2580

gcccaagtgc atactcgggt tctttccaac tcagaatcat ctctgattcc acaaaagtga 2640

gtttagtttc ctatctgaat taacaacttt aaaggagact ataatagtta aaagtggaag 2700

aatagaaata aataaattta aaatgaaatt aattaaagta gaagagaagg gttctgttcc 2760

atgtacgatt aatgtgcccc ttttggagat aagctttcca aaatatgcgt atgagtaaaa 2820

ttagagaatt ccaccactaa gcaacaatag attttatatg tgtgtgtgga tatatataca 2880

cacacacaca gaaagatgaa tatatatata tatatatctg tatagacaca catataatat 2940

atatagatat ataatcccta tatatcttga atctacaaat atctttatat gtatataatt 3000

tgaatatata catgtatgta tatacagatg tgtgtgtata tatatatata catacacata 3060

tacataaatt ttcagctatt tctgttcccc tgaactaatt tgagattaga cccaaacatg 3120

cttttaaatg tctcatgcca aggcactgaa ttaagacttc ttaatgttga aatattttaa 3180

gtgatttcat attaaatttt ctatatactg aaa 3213

<210> 29
<211> 1255
<212> DNA
<213> Homo sapiens

<400> 29
ccctggccaa tgggaagtga gggcgtgact ttaagtcttt ctttcattgg cccagggtcc 60

caggccgtcc aggtcttggg gcgccgcggc ggaaatcgcg cggatgccag aacgcgctct 120

cagcttcggg tcctgcggct gcggctgccg ccatcatggt gcggaagctt aagttccacg 180

agcagaagct gctgaagcag gtggacttcc tgaactggga ggtcaccgac cacaacctgc 240

acgagctgcg cgtgctgcgg cgttaccggc tgcagcggcg ggaggactac acgcgctaca 300

accagctgag ccgtgccgtg cgtgagctgg cgcggcgcct gcgcgacctg cccgaacgcg 360

accagttccg cgtgcgcgct tcggccgcgc tgctggacaa gctgtatgct ctcggcttgg 420

tgcccacgcg cggttcgctg gagctctgcg acttcgtcac ggcctcgtcc ttctgccgcc 480

gccgcctccc caccgtgctc ctcaagctgc gcatggcgca gcaccttcag gctgccgtgg 540

cctttgtgga gcaagggcac gtacgcgtgg gccctgacgt ggttaccgac cccgccttcc 600

ttgtcacgcg cagcatggag gactttgtca cttgggtgga ctcgtccaag atcaagcggc 660

acgtgctaga gtacaatgag gagcgcgatg acttcgatct ggaagcctag cggatctccc 720

actttgcatg gctgtctttt acagatggga aaactgaggc ctgatgctgg agattctatg 780

agggtgctct cctcaagggt atcagacggt cgtaggttct taagaatttg attcatcagt 840

ggcaggccat gcatagagcc acgggaggtg cgtccttgtt ttccagggaa tgttcttaga 900

acttggacta ctgattatta attgactgtg ccttgggaaa cagtgggaag taacttggtg 960

cagcactggg gtattgttgg cttcttgtgt tggaaacttt gtaatgtaaa aggaaaaact 1020

ggaaatcccc acgccctgtt tccctttatc gtcttgtggt tggactggtt caattcgttt 1080

aactcgaatt cttgctcctg gccgtggtta agctgtgtac agatgatgga gagtttggcc 1140

tcaagttttt ataaactgag cgagactagt gttcaggatc tcctcccttg tttaaatgtc 1200

aataaatgcc ccaactgctt tgtaagtgca actttttaaa aaaaaaaaaa aaaaa 1255

<210> 30
<211> 2218
<212> DNA
<213> Homo sapiens

<400> 30
ttttatgaag gttaagaagt ctacagcttg ggctggccgg tatttattat tctgtggcca 60

gaggatttta aattaatgta aattttttaa acaaaataag cagttttcct tccctgttgg 120

agtaaaaaaa tttttttccc tttgttgttt tacgtttttg ttcatagtct tctttattcc 180

acgctcctct tccccttctc ccagtgctga cttgggacaa aacagttcag cctttgtctc 240

gacgcagata aaagcacctg taagcaatgc tgtccagtca ttttatgtgc tgatttctca 300

aatctgcaat aatccatcag gttaatgttt ttacctgaaa ataaagaaag tttgcttcac 360

ctttttgttt atagttttgt gctgtatgca taactttcaa ttgtatagat gtatttcaat 420

gcaaggaaat ccacatatct ccatgattcg ggtcatggct gattttgcta ttcaaacaat 480

ttataatagc aacttgaaga aactaaaatg tgaagagaaa atgggaggag attccttcct 540

aaggtccaaa actaggcctg tggcataaat ttgaaggaat tttccataat tcatctcctt 600

gaaaataaga gatgagctag tttttttttt ttaattgaac tttgagaaat caattttttt 660

gactgttctg taagttttaa ttaattgaca caaaagccct tttccctatc ccaccaccca 720

aaagaaactc agtggctcgt ttgacataga ttgctgtgtc aatttacact taaatggcct 780

tatatggaaa tgtcccctcc tgagagttca cttgggcagt gaccccatta agtggaaggg 840

aactgtcaga ggagttgggg gcagagaatt ctctccctgt ctctgcagag actgacaagt 900

gaatggcttt tccctgaaat ccaagaaggg aattgtgttt ctgcagtttt caccactaac 960

agcacagttg ctgagcagct tcatcaagtg aagttaaaga ctgaaacaat ttgttttcaa 1020

ctgttctgat atcttcctgc aaattaagca caaggatttt tgtgtgaaca tagcattttg 1080

ttcctgattt ttaaagggtt ctgttaaaat ttagacatgt ataaacaaaa taatgtttca 1140

tctgtacttg acattttatc catgttgcag ccctggcaaa tttggcctca ttgattccag 1200

catctaataa gtatatatga tgattttgac tcaaactcta aagtcttgag tgtttcaaag 1260

tcagtcgtta cctgtttaaa agcctcagcc tttagcttat tcctccttca atacacggga 1320

cctttggtta atttggggca ggaaaactct taaagtaatc tctcttggca gaggccttat 1380

tgcaccagag ggaaaaagta tatacttcat ttgctgttac tccagttatg ccttaaattc 1440

atttgcttgg taatcctatc aacgagcact aacttcttag tatactttaa acacttagtt 1500

gggtaacaac tgagattttg ttgtccttta ttttttgctg agatggagtc agtcagatgt 1560

tagtcatagc taacaccgaa tttgtgttgt catttagaca gttactgatt cgatctgctt 1620

tatatatgag aacgtatttt taactattcc aagaaggaag aggtagctaa atgtaatccc 1680

ctcttcctat ccccccagaa aactgaactg taagttctag gtagactaat tgggagcaga 1740

cacggagttt tagatgcctt agccaaaccc agcagaaacc tttcacacag ccactcatcg 1800

taagaaacgc agatttttct cttctcatgc ttgtctctgg ttccctgcat ttgtagtgac 1860

agaactttca ctagcaggat ataaagaaag taattatgct tggagtccct ctttactggg 1920

tttgagttag gtgcataaca tggaaaggag tggtgccttc aaatgaatgt gaccactccg 1980

tattgtggag tgacttccct agggcatcct atacatccta ccacagaagg ccaagggaca 2040

gagcaccaac ttcagtatcc aagaaattag atccacaact cttgattttc cacactgagg 2100

actgtcgcga gtaagttgta agtttgccgt cttccttctg gcttagcagg tgctgcagct 2160

gtactctcga ctcctgtctg tgagcgtgag ctcaggaaat gagagtggag tctattcc 2218

<210> 31
<211> 1229
<212> DNA
<213> Homo sapiens

<400> 31
gccctgctta tcagcagctt cccagcttcc tctgcctgga ttcttagagg cctggggtcc 60

tagaacgagc tggtgcacgt ggcttcccaa agatctctca gataatgaga ggaaatgcag 120

tcatcagttt gcagaaggct agggattctg ggccatagct cagacctgcg cccaccatct 180

ccctccaggc agcccttggc tggtccctgc gagcccgtgg agactgccag agatgtcctc 240

tttcggttac aggaccctga ctgtggccct cttcaccctg atctgctgtc caggatcgga 300

tgagaaggta ttcgaggtac acgtgaggcc aaagaagctg gcggttgagc ccaaagggtc 360

cctcgaggtc aactgcagca ccacctgtaa ccagcctgaa gtgggtggtc tggagacctc 420

tctagataag attctgctgg acgaacaggc tcagtggaaa cattacttgg tctcaaacat 480

ctcccatgac acggtcctcc aatgccactt cacctgctcc gggaagcagg agtcaatgaa 540

ttccaacgtc agcgtgtacc agcctccaag gcaggtcatc ctgacactgc aacccacttt 600

ggtggctgtg ggcaagtcct tcaccattga gtgcagggtg cccaccgtgg agcccctgga 660

cagcctcacc ctcttcctgt tccgtggcaa tgagactctg cactatgaga ccttcgggaa 720

ggcagcccct gctccgcagg aggccacagc cacattcaac agcacggctg acagagagga 780

tggccaccgc aacttctcct gcctggctgt gctggacttg atgtctcgcg gtggcaacat 840

ctttcacaaa cactcagccc cgaagatgtt ggagatctat gagcctgtgt cggacagcca 900

gatggtcatc atagtcacgg tggtgtcggt gttgctgtcc ctgttcgtga catctgtcct 960

gctctgcttc atcttcggcc agcacttgcg ccagcagcgg atgggcacct acggggtgcg 1020

agcggcttgg aggaggctgc cccaggcctt ccggccatag caaccatgag tggcatggcc 1080

accaccacgg tggtcactgg aactcagtgt gactcctcag ggttgaggtc cagccctggc 1140

tgaaggactg tgacaggcag cagagacttg ggacattgcc ttttctagcc cgaatacaaa 1200

cacctggact taaaaaaaaa aaaaaaaaa 1229

<210> 32
<211> 4953
<212> DNA
<213> Homo sapiens

<400> 32
gcatcccctt gattaaatgt ttttcctcct atgcaatcac cattagctgt ttggctccca 60

ttctgtattc ttctgaagca gccctgcatt gcaaatcaat atctttctga aaagacagtg 120

tgttgtgaat tgccttgaca gcatatgcac ggttactttg gctgcaatgc tgctgcagag 180

cccggttact ctgccttcgt gggaactcca cagatatgtg tcacaaagat gtctacacgg 240

aactgccagg gaatggactc agtgatcaaa cccctggaca caattcctga ggataaaaaa 300

gtcagagttc agaggacaca gagcactttt gacccatttg agaaaccagc taatcaagta 360

aagagggtgc attctgagaa caatgcttgc attaacttta agacctcctc cactggcaaa 420

gagtcaccta aagttaggcg gcactccagc cccagctcgc caacaagtcc caaatttgga 480

aaagctgact catatgaaaa gctggaaaaa ctaggggaag gatcttatgc tacagtatac 540

aaagggaaaa gcaaggtaaa tgggaagttg gtagctctga aggtgatcag gctgcaggaa 600

gaagaaggga cacctttcac agctatcagg gaagcttctc ttttaaaagg actaaaacat 660

gctaacatag tgctacttca tgacatcatc cataccaagg agacgctgac acttgtgttt 720

gaatatgtgc acactgattt atgtcagtac atggacaagc accctggggg gctgcatcca 780

gataatgtga agttgttttt atttcagttg ctgcgaggtc tgtcttacat ccaccagcgt 840

tatattttgc acagagacct gaaaccacag aaccttctga tcagtgacac gggggagtta 900

aagctggcag atttcggtct tgcaagagca aaatccgtcc ctagccacac atactccaac 960

gaagtggtta ccttgtggta cagacctcca gatgtccttc taggctcaac agaatattcc 1020

acctgccttg acatgtgggg agtaggttgc atctttgttg aaatgatcca aggagttgct 1080

gcttttccag gaatgaaaga cattcaggat caacttgaac gaatatttct ggttcttgga 1140

acaccaaatg aggacacatg gcctggagtt cattctttac cacattttaa gccagaacgc 1200

tttaccctgt acagctctaa aaaccttaga caagcatgga ataagctcag ctatgtgaac 1260

catgcagagg acctggcctc caagctccta caatgttccc caaagaacag actgtcggca 1320

caggctgcct tgagccacga gtattttagt gacctgccgc cacggctatg ggaactcacc 1380

gacatgtctt ctatttttac tgtcccaaat gtgagattgc aaccagaagc tggagaaagc 1440

atgcgggcct ttgggaaaaa caatagttat ggcaaaagtc tatcaaacag caagcactga 1500

caagcagcac attctcaaga gcacacagga ttaagttgtc atcattctgg gaagaaaaaa 1560

aaaacattaa tgaagaggcc aataatatga agggaatcat ggatcagttt tctttcgctc 1620

cctgtggtgg atttcactta caagaaaatt gaagctggca agaccctgtt ttctctgcaa 1680

tttatttaaa accttgcacg catttggata ccttgtgatt tccaagaact acgtgaagat 1740

taagctttgc ttactgatac atggcatgta ttcttttcag tcttttgtgt ttgattttgt 1800

ttgatttccc tctgcagcac agcgtctctg taaaggtttt tatgctttca ccagccatgt 1860

cttaaataca ttaagacaac acatttggtg ttcacacttc ttcagtaatg tctgaacttg 1920

aaagccacag agtggcataa aacaatgtgt gttttctttg agagcagtgc acattttgca 1980

accactagga aggaaatttt ctgctaaagc aaacccctgt tctctgactt gacaacttgg 2040

ccccggactg tggggcccca cctgttgctt accttttgag gtaattttgc aaatgtggtt 2100

tttttacttg gaaataactg cacatttata tataggatat tggactctgc ttagcatttt 2160

caagccacat agcatgactg ttttttgaat aggttggaat tgaaaaaaca attatcaaac 2220

gttaagaaca aagacaggga taaattgctt acatttcaac ctctagagat tgaggtaact 2280

ttttgtgtct gggtcttgtc aacatctaat ttttttccat ccattctgtt acactttgta 2340

ttttctaact ggagaaaaga gtgaggaaca gaatgtttta aatctggtgc aaaagaacta 2400

tatctgctgg atgagccttg aaagcagtct tggcctgtta gggcttacaa agtaaattac 2460

aaagtgatcc agttcaaagt ttgcttagtt acaacaaagc acctttaaaa aaaatacatt 2520

ttaaaaaaac attccaagcc aattggaaga catcattggg ttcttacttt aagacatctc 2580

ctggaataac tgttcaaatg caggttttag aaacaatgca ggaatcttgc tttaaagatg 2640

aaaaagggaa tgggccagct tcccttactc aaggagttga gggaccttgg aggatgaagg 2700

cgagtatgtg acactggaga aaagtggacc aggcatgtct tttgctttga tctggaggga 2760

gggctgcctg atgcaggccg gctcccagtg gggcaggcct cgctgcagaa tgcccagtag 2820

tactgcggcc aaggggacag ttaggagact tcatctaaag catgaaacct agctcctcta 2880

cacacaaatt cctatggaaa tacctttgtg tacagtgtct tacattttcc tattagtcag 2940

aaagaaggag agaatgagtg agtgcttgaa atgtgtcata ctgttttagg atcaagacta 3000

ggaattagga gccaggttga caaggacttt ttctgagagt tgggtgaggg taaagctttt 3060

ctataatcaa gctcaataca ccaaggaaac tggatccaga attcctaaac tttaaaatgg 3120

tactgtctgc ggagtggagt atggatggtt atgtcaaagt catagttcat cctatccaga 3180

tgtagcattc atggtaaact tttaagtgct aagcaaggaa ttatttactg attggtttta 3240

aagagagcag aaaacaccca agtgtagaat gtctactgtt tgctacctag aaatcttttc 3300

cattcctctt tcatacattc caacccactg gaagtcttta gaggtatttt gatttaaagt 3360

atacttaaat taggatttct taaagaaaac atagggagaa aactttacat gcaattaaaa 3420

atggactttc ctgtgatttg tttttaatca ttcatttgga gaagaggcat gacctttgta 3480

tttcactaag tttaaagcaa gagcaactga tgattaaatg ttgcttttta ataaggtttt 3540

taacttgaaa atttgaaaat atttaatgtt gaaagacttc aattagggct attagagtta 3600

tatctccctg tcgtaggcag cttcttcgga gaagtgaaat ataacattac tcagtggacg 3660

gagaagtctg ttttgttaca gagacatgcc tctcagaagg tcaggaggtt ttgagtacct 3720

atccttgcca cccatacagg aaatccaaag tttggtgtct ctctctctct gtctctttct 3780

ttctctttct ccccccaaac ccctctcact ccctccctcc ctctctcctt cccctatttg 3840

caatcatatt ctccctctgc ttcttttctc ttctgccctc cttgtgggca gtcatgaaaa 3900

tcaattcaga ctgtgttcat tagcagattt attattctat tgagaaagca ctggaatgtt 3960

ttgtgagatt atttttatat gaaggaatag cctgaactca aacagatggt aagaatagta 4020

caaacacctt agcacatcac tgcacacaca gtattctgaa aggagatttg acacttaatt 4080

cccattttct taaaataaca gttttgttga cttaaaaata tgagatacat aggatgtgaa 4140

aaaaaatgtt tgcagtactc agcaaaaaat agggtacata aagcagggtg gctgtccatc 4200

cactgattct ggggtgagaa gcgatttcta cctcgcaaga gtgactagaa agtttctagg 4260

agcacctcca ggcttgcaaa gaaagtgagg cctcttggta tcctttcctc agtgtgtata 4320

tgacagccag tataatcaat accctaggtt atgcgtctat atgatactca tctgtgaata 4380

ttattggttt tgtaatcttt gttatataag aggatgttta ggctgtatat actggggtag 4440

attattgcct gccccttata cataggaata tgctgcataa ttgcgcataa cttccatctc 4500

ccttactggc ttgtaggcag aggaaactgt atatgttact gccttgtact tttctcatac 4560

accaaaaaca caccaaaaaa atcaataaaa taagcaatct tctattctca ttccttttcc 4620

cacagcagca tattttagag gcacatacaa aacctacatt ctctagttgg gagtggattt 4680

ttaaagtttt ccttttatct tttatttttt tttgtatgat gcactgagat gtgtactttc 4740

taacagggga ttggtaccta agaaatgtgg tagcattatt cagaaaacta ttatactttc 4800

aaatgacaca tagtaaggag aatggaataa tacatgttgc atatttgtta ccagttgtaa 4860

tttgtctgta ttatgaaaga tgtaatggtt tgtcagctgt cactgttgtt ttcttgtaac 4920

atgatatgga ataaagtata gcagaatctc cgt 4953

<210> 33
<211> 3883
<212> DNA
<213> Homo sapiens

<400> 33
ttggttgcgc ggtactagcg gtgcccgccg aaggggaggg aggcgaggag cgagccgtgc 60

ggccagagcg ggaaagagac tcgtctttgc gtccgagttc tggagccgcc gcaccccgac 120

tcctggggcc gcggcagcgg ctgcgagggg acggcgtccg gctgtctcct gggttcccct 180

cgtagcgacc cgcgggatcg gaaaaaaagg agaagatgga ggaggagggt ggcagcagcg 240

gcggcgccgc ggggaccagc gcggacggcg gcgacggagg agagcagctc ctcactgtca 300

agcacgagct gcggactgct aatttgacag gacatgctga gaaggtggga atagaaaatt 360

ttgagctcct gaaggtccta ggaactggag cttatggaaa agtatttcta gttcgtaaaa 420

taagtggcca tgatactgga aagctgtatg ccatgaaagt tttgaaaaag gcaacaatcg 480

ttcaaaaggc caaaaccaca gagcatacaa ggacagaacg acaagtcctg gaacacatta 540

ggcagtcgcc atttttggta acattacatt atgctttcca gacagaaacc aaacttcatc 600

tcattttaga ttatataaat ggtggtgaac tttttactca tctttctcaa agagagcgtt 660

tcacagagca tgaggtgcag atttatgttg gagagattgt gcttgccctc gaacatctcc 720

acaagttggg gattatatat cgtgatatta agcttgagaa tattctactt gattctaatg 780

gccatgtggt gctgacagat tttggtctga gtaaggagtt tgtggctgat gaaactgaaa 840

gagcatattc cttttgtgga actattgaat acatggcacc agatattgtc agagggggag 900

attcaggaca tgacaaggca gttgactggt ggagtttggg tgttctaatg tatgaattac 960

taactggagc atctcctttc actgttgatg gagaaaaaaa ttcccaagct gagatatcta 1020

ggagaatatt aaaaagtgag cctccatatc cccaagaaat gagtgcttta gcgaaagacc 1080

taattcagcg tcttttgatg aaagatccca agaagagatt gggatgtggt ccacgtgatg 1140

cagatgaaat caaagaacat ctcttctttc agaaaataaa ttgggatgat ttagccgcca 1200

aaaaagtgcc tgcaccattt aagccagtca ttcgagatga attagatgtg agtaactttg 1260

cagaagagtt cacagaaatg gatcccactt attctcccgc agccctgccc cagagttctg 1320

agaagctgtt tcagggctat tcctttgttg ctccttccat cctattcaag cgtaatgcag 1380

ctgtcataga ccctcttcag tttcacatgg gagttgaacg tcctggagtg acaaatgttg 1440

ccaggagtgc aatgatgaag gactctccat tctatcaaca ctatgaccta gatttgaagg 1500

acaaacccct gggagaaggt agtttttcaa tttgtcgaaa gtgtgtgcat aaaaaaagta 1560

accaagcttt tgcagtcaaa ataatcagca aaaggatgga agccaatact caaaaggaaa 1620

taacagctct gaaactctgt gaaggacacc ccaatattgt gaagttgcat gaagtttttc 1680

atgatcagct tcacacgttt ctagtgatgg aacttctgaa tggaggagaa ctgtttgagc 1740

gcattaagaa aaagaagcac ttcagtgaga cggaagccag ctacatcatg aggaagcttg 1800

tttcagctgt aagccacatg catgatgttg gagtggtgca cagggatctg aaacctgaga 1860

atttattgtt caccgatgaa aatgacaatt tggaaattaa aataattgat tttggatttg 1920

cacggctaaa gccaccggat aatcagcccc tgaagactcc atgcttcacc cttcattatg 1980

ccgccccaga gctcttgaat cagaacggct acgatgagtc ctgtgacctg tggagcttgg 2040

gcgtcatttt gtacacaatg ttgtcaggac aggttccctt ccaatctcat gaccgaagtt 2100

tgacgtgtac cagcgcggtg gaaatcatga agaaaattaa aaagggagat ttctcctttg 2160

aaggagaagc ctggaagaat gtatcccaag aggctaaaga tttgatccaa ggacttctca 2220

cagtagatcc aaacaaaagg cttaaaatgt ctggcttgag gtacaatgaa tggctacaag 2280

atggaagtca gctgtcctcc aatcctctga tgactccgga tattctagga tcttccggag 2340

ctgccgtgca tacctgtgtg aaagcaacct tccacgcctt taacaaatac aagagagagg 2400

ggttttgcct tcagaatgtt gataaggccc ctttggctaa gagaagaaaa atgaaaaaga 2460

ctagcaccag taccgagacg cgcagcagtt ccagtgagag ttcccattct tcttcctctc 2520

attctcacgg taaaactaca cccaccaaga cactgcagcc cagcaatcct gccgacagca 2580

ataacccgga gaccctcttc cagttctcgg actcagtagc ttaggcatgg taggagtgta 2640

tcagtgatcc attgcacctt tattccctca gcatatgcct gaggcgatct tttatgcttt 2700

taaaaatgtt tcccgttggt ctcattggaa tctgcctcct aatgattttt ttcaggaaaa 2760

cctgtttggt tatcctcatt caaaagcact ggacagagaa tgttactgtg aatagagcac 2820

atattactct ttttagcaac ctagcatgat gccaacaaga ctattcttga aagagcaaag 2880

gttcctgtaa atttaattag ggctagattt gagctgcttg taagtcacag gttttccaga 2940

tgtctgccaa caagaaatga ctcatactgt gatgatacct tttgctttgc cttgtggaca 3000

atgtgggttt ttgaaatttg cacccttcaa acaatgattt atcagagaaa ggggtctgtt 3060

ttcaaaaaag attctgtaat gaattttatg tgtggcatat acttatttct tgagagaaga 3120

ttttaactta ttgtttttat tttatggtta catatgatga taacctgcta ttattaaact 3180

ttttctaaaa agtgaaaaaa aaaaaaaaag atataagaac tcaaggtccc atactctgta 3240

ttcgggatcc atctgagatg catgctaagc tatgtgtatg tttttaattt tgcactgctc 3300

tttcctggca atttgtttta atggttattg cagaatatta aggtacatgt ctctctgttt 3360

taagtaatat tgcactttat aaaaaagtat gaataaagca aactatttta taaagtgcac 3420

tgtttaaagc atttgcactg tatttttgcc atttattttc attttctact ttaaatttgt 3480

cctcacattc ctcttctact ttgtatgcaa caagtagaat ggggcatgtt gtgtcatgta 3540

gtcagccact tatgcaccaa tgtgaggaaa acctaaaggg aaattaaact aaacactgtg 3600

cttcatattt gtacactgtg ttgtactaca gtgaggaatt tcctcctgta gtcatatatt 3660

atgtacataa tattttagaa tcatacctat gacttgtttg gaaatttttc tgttaaattt 3720

taaatccaga aagcatattt tataaactta tgcagagcac ttttattgct caaaagttct 3780

gaattcatac agaaaacaag tactatgtga tgaaaacatt tcattaaaag attgctgcat 3840

ttaaaaatac aattaattcg ttccctatgc aaaaaaaaaa aaa 3883

<210> 34
<211> 6139
<212> DNA
<213> Homo sapiens

<400> 34
gtcttcctcc cccagggttg tggccacgcg cagcggcggc ggttgttccg cttcccctcc 60

ggcccgggcc gtcgccattg ccgaaggctc cctcccctcc cctccctggc gtgcgcagga 120

ctccgccgcc gctgggccta gcggtagcag cggctgctcc agcgcggcgt ctcttcccgc 180

cccgcttccc cttccctccc ctcccctccc cgcaccgcgc gctagcccgg ggcggctccg 240

cagcccgccg ggagctctga ccgaggcgcc tcgctggggc ggggaccttg ccttgcccgg 300

ggccatttca taattctgaa tcatgtctga taacggagaa ctggaagata agcctccagc 360

acctcctgtg cgaatgagca gcaccatctt tagcactgga ggcaaagacc ctttgtcagc 420

caatcacagt ttgaaacctt tgccctctgt tccagaagag aaaaagccca ggcataaaat 480

catctccata ttctcaggca cagagaaagg aagtaaaaag aaagaaaagg aacggccaga 540

aatttctcct ccatctgatt ttgagcacac catccatgtt ggctttgatg ctgttactgg 600

agaattcact ggcatgccag aacagtgggc tcgattacta cagacctcca atatcaccaa 660

actagagcaa aagaagaatc ctcaggctgt gctggatgtc ctaaagttct acgactccaa 720

cacagtgaag cagaaatatc tgagctttac tcctcctgag aaagatggct ttccttctgg 780

aacaccagca ctgaatgcca agggaacaga agcacccgca gtagtgacag aggaggagga 840

tgatgatgaa gagactgctc ctcccgttat tgccccgcga ccggatcata cgaaatcaat 900

ttacacacgg tctgtaattg accctgttcc tgcaccagtt ggtgattcac atgttgatgg 960

tgctgccaag tctttagaca aacagaaaaa gaagactaag atgacagatg aagagattat 1020

ggagaaatta agaactatcg tgagcatagg tgaccctaag aaaaaatata caagatatga 1080

aaaaattgga caaggggctt ctggtacagt tttcactgct actgacgttg cactgggaca 1140

ggaggttgct atcaaacaaa ttaatttaca gaaacagcca aagaaggaac tgatcattaa 1200

cgagattctg gtgatgaaag aattgaaaaa tcccaacatc gttaactttt tggacagtta 1260

cctggtagga gatgaattgt ttgtggtcat ggaatacctt gctggggggt cactcactga 1320

tgtggtaaca gaaacgtgca tggatgaagc acagattgct gctgtatgca gagagtgttt 1380

acaggcattg gagtttttac atgctaatca agtgatccac agagacatca aaagtgacaa 1440

tgtacttttg ggaatggaag gatctgttaa gctcactgac tttggtttct gtgcccagat 1500

cacccctgag cagagcaaac gcagtaccat ggtcggaacg ccatactgga tggcaccaga 1560

ggtggttaca cggaaagctt atggccctaa agtcgacata tggtctctgg gtatcatggc 1620

tattgagatg gtagaaggag agcctccata cctcaatgaa aatcccttga gggccttgta 1680

cctaatagca actaatggaa ccccagaact tcagaatcca gagaaacttt ccccaatatt 1740

tcgggatttc ttaaatcgat gtttggaaat ggatgtggaa aaaaggggtt cagccaaaga 1800

attattacag catcctttcc tgaaactggc caaaccgtta tctagcttga caccactgat 1860

catggcagct aaagaagcaa tgaagagtaa ccgttaacat cactgctgtg gcctcatact 1920

cttttttcca ttttctacaa gaagcctttt agtatatgaa aattattact ctttttgggg 1980

tttaaagaaa tggtctgcat aacctgaatg aaagaagcaa atgactattc tctgaagaca 2040

accaagagaa aattgcaaaa agacaagtat gacttttata tgaacccctt ctttagggtc 2100

cagaaggaat tgtggactga atcactagcc ttaggtcttt cagcaaacag cctatcaggg 2160

ccatttatca tgtgtgagat ttgcatttta ctttgctgac tttgttgtaa tagatcccat 2220

tcattgtccc ctttggggta tttccaatac ttgaatggca gattggagtt tttcagagta 2280

tgtgtttcat ctgctagtct ttctctcctt catagctttt cttttcctgg acttgctcct 2340

tttgagttgc ttttgcgttt ctcatgccta ggcaagtgta atagaaatta tgtagctcct 2400

tatgttggca aaggagctct atatagtttc actttgtata aaagttagga ccagctgttg 2460

ttacatgtaa tattttagtt cagaacttga cctgaaggaa gggaagaaaa gtatgtgatt 2520

tttacctttt ttaacaaatg tgaaaaagtc agttttagaa atttcgtggt agtaagttcg 2580

gcatttgtta catgtataga gagaagacta ataatctcta tttataacta aatcattgag 2640

atagaaaaag attcccattg actgtagact tcttcccatt ttgtcttccc ttctgcctgt 2700

ttccccttca ggcttggctc taggaaccaa agtgatttgt tgttgttcca acctgggctt 2760

tgtgactttg gttagtgcca ctaccttctt ccctcctttc ccccttcaat ttggaaataa 2820

atttctgtat atgttgcaat tttaggttta ggtttgttct ttttcttttt cattaatcct 2880

ctctcacctc acagataccc cctcccatgg caaataatat aataaccagt gaattttcag 2940

gaatttaaaa attagctttt ttccacttaa aggagaaaaa tatttgggac tagcagcaga 3000

ggcagtaaga gatgtgaacc ttggtgagct ctgatacagt gagaagagat tatactcatg 3060

aaagagaatg ttagtgttac agagaagcag ccgatagcaa atcgactgta gagacttggc 3120

ggcggtggca ttgccccagg tcgtcagcag tgtggtatta tctatgagaa cttgagcgac 3180

agagtatttc ttgatgaatt tatagatcat ttgagatgtt gagttacttt agtttagttt 3240

tgttttgttt tttcaaataa gtagagacta ttgtaaaaaa cgagaaagga aaatgaaatg 3300

tgcgtgttga tagcaataat ttgtttcttt taaagattct aaaaggtctg agacctgtag 3360

cattaattat ttgagtgccc tcccttctcc cctcccctcc cttttctctt ctcttttttc 3420

ctctcctctt cttctccttt attcattgtt ttgcttttgg agtgggtgtt gttcaagtat 3480

ctgtggtttg gttctggcat tttgttccca ccatcccctt cccccattaa cttcccccct 3540

gcttgccatc ctgcagtagt ataaatcatg aataaaaaat aattttgctg ttgtagtata 3600

cattggagaa actggcaggt tttatttcca ttattttatt tccactatat ctatgataag 3660

atgcaattat aaggagagaa gtgactgttt tttattgata aggcaagatt ttcagaaaaa 3720

tgagtaaaat aattaatgaa acatatttag agcacttaat ggtctctgtt ttcaatataa 3780

ttcttgattt catttttctc tggaatatat tggccttcta cagctattac tgaattatag 3840

aaactggttt atttctggca gaaagctgca gtgccacctg agttccaaat tttaccattc 3900

tttgtaaaca gttggatgga ttatgataaa gaagatgcta ccaatgaaat agaaaaccaa 3960

cgagatgaga agactgtgat cctcatgtac tcagaggcac ttccctccta agtcaaagac 4020

catcctcact gactatgtgc caacgcctcg tttcaggctt gtgactcaac aaagggcttt 4080

tccattgata gaagcagttt gggatttgta gttgcgactt cttcgatagt tacctgcacg 4140

tccattgctg gcaactgact tgtcattaaa acctggctct ttggttaagg gagctacgct 4200

gtggtttatt cttaagttac gtggataaac taacctctaa cagaaatata ctttggttaa 4260

ttttgaaatg tgtcattttt aaacaatctt aaaagtaata cagaattgtg atttattaat 4320

tttaaaacat tcagaacttg ttgaaagaaa aattatatct gaatcaagat tcatgttttt 4380

tatttttatt ttttttgata cagagtctca ctctgtcact caggctggag tgcagtgaca 4440

tgatctcagc tcactgcaac ctccgcttcc tgggttcaag caattctcat gcctcagcct 4500

cctgagtagc tgagaccaca ggcacccgcc accacaccca gctatttttt tgtattttta 4560

gtagagacag agtctcacca cattgcccag gctggtctcc aactcctgag ctcaggcagt 4620

ctgcccacct tggcctccca aagtgctgca gttacaggcg tgagccactg cacctggcct 4680

catgtttttt aaataattgc cttttatatt tacccttttt gtcatcactt tagaatgaaa 4740

attcccattt aaatctgaaa gttaccttaa tagtcctctt gtgttattag gacagtatta 4800

ttatagtact tatttatttt attttagatt taaagttatc ttctcttttt ctttttcttt 4860

ctgctgcttt tagggacaat taaaactggg aaactatgaa acatggaaca ttttatccta 4920

cctgaaagta aacgagtaat tgtgaagcat aagacactga ggctaataca actctgtctt 4980

catgtgttga ctgcctggca catagtattc attctcttcc ctttaacata gaagtgtcca 5040

gctgcgtaca gtctagtaac cagcaactgt aaacgaacct gtgcctctaa caagcgattc 5100

taaaccacct atgagtattt cttttagggc tcacttaaat acatgtttgt atatactgta 5160

ttctagccag aataatttta gatctgatca ggtagtagct aaaattagaa aaaaacaaaa 5220

tagatgctta aagaatttgc atccattttt gagtctaaat cttttaaaat atactgagat 5280

ccacatctag tgaaatgtca gtgtcaaaat attatagatt atagctaaaa tccagattaa 5340

tactcatttg gggtttttta tagtggaact tcatagtaat acaaaaagca gattgtcttc 5400

ctgtctccgc tgctcccaca gtaggtattg aaactggtaa aatcagtttt ttgatagtgt 5460

gtgtatataa gaaaaaatag atacacacat tcttttttct cagtcaacac attgattgaa 5520

cactctggca aagatgctgt ggtggatgag gttggagttc gaaagaagaa gcaagcgctg 5580

gcctggcctt gaaagaaccg aagtctttcc cattcacttc tctagaaagc tgccaagaca 5640

gaggcagaaa gaaatggatg atagttctgt caagcacact tctgttctct tagaacttag 5700

aagtgtttct aagagaacag aagtaataag agaaacagtt acgtgtggaa ttcaacatct 5760

ttggttggaa cgcattggct ttttttttct tgttttgata gaaatggaat taagcaaaag 5820

tagtttttgt cttttctgtt gtcttcaaat tttatgcctt ttatttttaa tttaatcccg 5880

ttcaattatt taattgttat acattgacat taactgctgt attttgactt tgttcaataa 5940

ttttgttctt tcagggctag aaataaactt tttaaaaaaa gtgtgcattt ttccctttcc 6000

taaactttta ttctttcttt tgatcagcgt aaaagaatat tttaatgtct tttgatagca 6060

taaaagaata tttaaatgtc ttaataggtt ttcaaagaac atttagtatt tttagtgata 6120

aatgttttaa accttttaa 6139

<210> 35
<211> 3384
<212> DNA
<213> Homo sapiens

<400> 35
agggggcggg gcgggccaag ggcgggggcg ggaaggggcg gagtcaggcg gaagccgggg 60

agaaggccca ggaagtgacg gccgcctccc ggctaccggg gacttctgga gtccgagaag 120

tcaacggcgc ggttgctgcg gccgccgcgc tccccggccc gaggcgatgg agaacggagc 180

ggtgtacagc cccactacgg aggaggaccc gggccccgcc agaggccccc ggagcggcct 240

cgctgcctac tttttcatgg gccggctccc attgctccgg cgcgttctca agggcttgca 300

gctgttgctg tctctgctgg ccttcatctg tgaagaagtt gtatcacaat gtactttatg 360

tggaggactt tatttttttg agtttgtaag ctgcagtgcc tttcttctga gtctccttat 420

actgattgtg tattgcactc cattttatga gagagttgat accacaaaag taaaatcatc 480

ggatttttat attactttgg gaacaggatg tgtgtttttg ttggcatcca tcatttttgt 540

ttccacacat gacaggactt cagctgagat tgctgcaatt gtgtttggat ttatagcaag 600

ttttatgttc ctacttgact ttatcactat gctgtatgaa aaacgacagg agtcccagct 660

gagaaaacct gaaaatacca ctagggctga agccctcact gagccactta atgcctaaag 720

actctgggga gcagatgtta cctaaggtag tgaccctgca ttgtggtgcc tgagccctgg 780

cagaagctct tgtaaaattt gttaattgtt taaaccactt cttttggaga gcaaggggaa 840

ggtcaagaag gcagttttat caatattgtg tcagtcacca caaagtaggc cagataagtt 900

aaaaaaaatt tttttttaaa taataattga aacttatctc aaatggagat tttggtggga 960

ggaggagaaa acaattgttt ttaaatcaca cagctcaacg gttgataaat gattctgtca 1020

ttctgttaca ggtcattctt ttactaggct tagcttccaa attatgcttt atagctgtat 1080

aaacatcgtg attatattca tctacttaga aattgtttta tttttaaatt aatttgctta 1140

gctgtttgtt ttgatgctta gattatgttc tgttaatggg aatttaacat atttaagaaa 1200

ccaatattta aaatgttggt ctaggttttt ttccttaaca tatattacca ggctttactg 1260

tatttcactc agccttaaat gttataatat ttttggataa cggttattaa ttctttgaga 1320

ccttcgtata gcctataaaa tgtatgggag atgttggtat tttatgtgta taaaagcaac 1380

aatatcagca acttcgtgtt tatactgcac cttggttgtt gatgtcaagt aaaaaaaaga 1440

ttgttttgta acacataaaa aaatggaaga aactgatacc acacctaagg accaaagata 1500

agaaagactt tttgcccaag acagtgaaag taattataaa aacaagcttt gaccacttac 1560

caagtatctg aagagatgag ttcatactat gatttagaaa gtggttcaat tcccctgttg 1620

gcatatgatt atttttacta aaattaatac agctctgtgg gtcttcctta gtgttttctt 1680

tgaagccaat ctgttttttt taggacacca gcctttggtt tttcatctgt tcgagatgcc 1740

tcttctctgt ctccttatca gatagaaatg gagtcatgtg ctgctgcttc atctagcaga 1800

ggttggcctc tggctctgac actttttgtc agttgtcttt aggtggtcct gaatcttggg 1860

cccttttgat tgtgaatact gtgtagcagg atcttgagag tccttgttct tacataggca 1920

ttgctctagt ttgtctttgg caaaaaaaaa aaaaaaaaaa agtaaatatc cagggaaccc 1980

tgcccagact aatactgttg gtggcataag agaatcaagc cattctcaag agataacttc 2040

ataaccagaa ttgtctgttg gctagcagct gtcacagata ggcagggcac ttgggatatg 2100

acctttctgt ccaggtgatt cacagactag acctttctta tcctcctcct agagttttga 2160

cttgggactc tagtgttaag atgatgagcc cgtgcatcag gtccttctgc actttggtgg 2220

aagtctccca gggtaggttt cctatttgaa acagtggaat catgtttcca gtgataaagt 2280

ttaatgacct catccttttt tttttttctc atctgccatt tgtgtgtctt agatgggttt 2340

taattgcatg aatgtggcta atgtggttct cagaaattgg tcagtatggc ccaacatagc 2400

ttctgctctg tcttactgac tcaatacctt taggatttgt atcagagttt ggatactagt 2460

gttagtggtg gtgtcaccac tacttaattg ggagataatg aaaccaatca tggatgctgt 2520

ttttattggg catgtcatct aagagaggag aaatagctgg gttttgggtc taattatgaa 2580

taaggactga ttcagaaaac gagtttatgg taggtagact aaagtttcac atcagactgt 2640

accattgtga tttagaccta tctaaaattc agagcatatc atctgggcta cctcagggtc 2700

accacccatg tattgggctt agtcaggatt gacagataca ttctcagctg gcctgtcata 2760

taaaacatac tgtcattgag cttaagctcc gcttgttctg aggtttcacc tccatgtgtt 2820

tcattggtgc aaaagtggat ctcttagttg gtcacttaat tctttctttt tcagaaagat 2880

agtatgttca ctggtatatt tggtcactct tagaaccttc cttcacattg ttttttatgg 2940

gacccatgaa tggttagcct ttcttttcta ttgtagaagg aaataaatag gagtaaaaag 3000

accattgtag taaataagtt caaggggaac ttgggaccag aaaccactgt tatgtacaaa 3060

aaaatggcaa attcaataaa ctcaaattta aaataatttt taaattaaca gttatgataa 3120

attttatatt ttatacaaat agattgctta gaatggttct caagaattat aagagaaatg 3180

aactcacagt acaaaaattt tataattact atacttgtgt tttgtttggg ggctgggaaa 3240

tgtattttta cattgtagcc aatcatttta tatttgtcaa tttaaatctt atgggtcttt 3300

tttttttatc tctcttgatg tcagatttta tagtcttttt aaataaatcc atttaattaa 3360

aacgttaaaa aaaaaaaaaa aaaa 3384

<210> 36
<211> 5324
<212> DNA
<213> Homo sapiens

<400> 36
gaacggcgat gccccagacg cggctgcagt tttcaaaccg cgactgcaag cttcggtagt 60

cctctccgct gctgtcgcca ggagtcactt cacgagaagc caggtcacaa ccgtcggccc 120

ttgtctggaa aagtaaaagt ggatcctgcc acgttcggag ctccctggcg cctcgcccgg 180

ctggagctag agaactcgtc ctgtggcggc ccccggcgtg gggcgggaca gcggccccct 240

ggagggggca gtcccgggag aacctgcggc ggccggagcg gtaaaaataa gtgactaaag 300

aagcagacct gggaatcacc taacatgtcg aggaggagat ttgattgccg aagtatttca 360

ggcctactaa ctacaactcc tcaaattcca ataaaaatgg aaaactttaa taatttctat 420

atacttacat ctaaagagct agggagagga aaatttgctg tggttagaca atgtatatca 480

aaatctactg gccaagaata tgctgcaaaa tttctaaaaa agagaagaag aggacaggat 540

tgtcgagcag aaattttaca cgagattgct gtgcttgaat tggcaaagtc ttgtccccgt 600

gttattaatc ttcatgaggt ctatgaaaat acaagtgaaa tcattttgat attggaatat 660

gctgcaggtg gagaaatttt cagcctgtgt ttacctgagt tggctgaaat ggtttctgaa 720

aatgatgtta tcagactcat taaacaaata cttgaaggag tttattatct acatcagaat 780

aacattgtac accttgattt aaagccacag aatatattac tgagcagcat ataccctctc 840

ggggacatta aaatagtaga ttttggaatg tctcgaaaaa tagggcatgc gtgtgaactt 900

cgggaaatca tgggaacacc agaatattta gctccagaaa tcctgaacta tgatcccatt 960

accacagcaa cagatatgtg gaatattggt ataatagcat atatgttgtt aactcacaca 1020

tcaccatttg tgggagaaga taatcaagaa acatacctca atatttctca agttaatgta 1080

gattattcgg aagaaacttt ttcatcagtt tcacagctgg ccacagactt tattcagagc 1140

cttttagtaa aaaatccaga gaaaagacca acagcagaga tatgcctttc tcattcttgg 1200

ctacagcagt gggactttga aaacttgttt caccctgaag aaacttccag ttcctctcaa 1260

actcaggatc attctgtaag gtcctctgaa gacaagactt ctaaatcctc ctgtaatgga 1320

acctgtggtg atagagaaga caaagagaat atcccagagg atagcagcat ggtttccaaa 1380

agatttcgtt tcgatgactc attacccaat ccccatgaac ttgtttcaga tttgctctgt 1440

tagcactttt ttctttgact catttggact gaatttgaaa ttttatatcc actccagtga 1500

gattatgatt tgtagcttca tatatgacat gtttatattg taaatgcact tttccatgga 1560

ataatttagg gaagtgtttt aatgttaaat tactagttgc tagcatgtta tgatttcata 1620

tcctgagata gctctgcaga taagaaaata tttaaatata tgacaaaaag taaaattgta 1680

catgtgagtt tacatgttaa tgaaataatt caacttcaaa tgaacttacc agaatgtttt 1740

gcatatcaac aaaaaaagtg gcttgagttt tattatagtt ggtgtaaact gaacacagtg 1800

aagacattgg aatttaatag gttctctctc taaggtgact cttataccat gcctctatca 1860

acataatttg tttaggaaag cagtatgaag tttaagccaa aataatttct actttataga 1920

tgctcaagag acattttaca attgaaaatg tctttcaatt acaaatattt tgaaacttcg 1980

taagattttc attctctgtg gtctgttata tgagagagat cctttaacta gagcaaagag 2040

ggagttagaa acctgatcag ggatattctt tacaagttgg agcagaggaa agagtagcat 2100

gccttcgtat tttaacgcaa atgtcttttt cctcctccca acctacttga gatctgataa 2160

ggtctggaag atggagatat ttggtatgca agtgtagagt tttttaatcc tccagaattt 2220

ctagagtaga agatacttag gtatagttaa atattctgta tttttagtca aacatattta 2280

ttaattgaat atagaagaaa atgttgacac actcagacag cttactgaat tttagatgtc 2340

ttctgcatct tagaatacaa gccagtcatt cagagttcta aaagtatgca taaaaaatta 2400

cagcaccggt aggtctatta acacagtgcc cgagtcagcg gtagcaagac tgatgtgatc 2460

ataaaacatg acatcaggct cgtctgaagt tcttgtgtga aattcctagt gagtgaggag 2520

gctcagctta aagccatctg cagagtggcc cctcattgtg gtcttttgct gggaccaatg 2580

caagagacta gggagagcaa aatgtttgct tatggctaga gactatatcc agccctaatg 2640

atggggaaag ttagtccttt tcgggtaatc ttttatgaat tttcacctga tgaccgttat 2700

attggtctgt tatcatgtta cgataactgt gatctcatga ccatgttgct gtatcagaag 2760

aaatagtttg acaaatggta acaacaacct gatgttcccc ctttagacct ttaacttctc 2820

aaaattttgg taagtttcca aattctttaa taataactta aaactttttg aataactatc 2880

aggtcacttt atttgaccac atggtgaatt cctttaatgt cttcagcatt tgttaaggaa 2940

aagttttctc tacttgtgtg tgtatgtgtg cacatgtgtg tatgtacagg tgtatgtata 3000

tatctataga tagatacaat acattcttta gacacttttc aagattcttt gctgtggtat 3060

attgtgctca actcaggtgc caaaggagct tttttttttt tttttttttt ttgagatgga 3120

gttttgctct gtctctcagg ctggagtgca gtggcatgat ctcagctcac ggcaacctct 3180

gcctcccggg ttcaagcaat tctcctgtct cagcctcctg agtagttggg attacaggcg 3240

catgccaccg tgcccagcta atttttgtat ttttagtaga gacggggttt caccatgttg 3300

gccaggctgg tcacaaactc ctgacttcaa gtgatccacc cgcctcggcc tcccaaagtg 3360

ctgggattac aggcgtgagc cactgcgccc ccgcccagga gctcttttct tatgacatat 3420

aaattatgac atttatattc tttatatgac tttatgttct cttcttatga catttaaatt 3480

ctttaagtag tttgttggtc caataaacta gacgttgtat aatctaaatt gagcccttgt 3540

atatctaaaa ctgatgagtt gtttctaaat tgttgattgt ccatttactt gcctttggta 3600

ttaagataat gcaagtaaag tttagtaagt cattggataa tgaaatgatt atgtttctga 3660

agaccatatt atatttttaa tttttagagg aatcatgcca tcccccaaaa aatcaagaaa 3720

tatttgaatt ttaaattata agttcatttg ttaaaagaca tttttacaaa tgtctgaaaa 3780

tcttaaaata ctttacatct acctttaagt agtagaatac agagctgtaa atttccatgc 3840

cttttttcct gatattaagt tttatagtaa aaaagcaact agtgattgca caaagaatat 3900

aaaaatccac tctttttaca aaggtgtgaa tttaaataac gttattgatt ggaatatgaa 3960

aatagaccaa tcatttaaga gctttttagc aaatgattca attcttactc tttttctccc 4020

aagattgaaa agcataatgt atttctctaa agtaggaatc tagagagccc ctgtgagtgg 4080

acaaatgtca gtaacacttg aacacatgag aagataagtg ttatgttgtg ataatttaaa 4140

gttaaatttg ctttttgggt aggatcccta aatagatggg atttttaaat agatgatata 4200

tagatgacaa ttgcaattgt cattttaatt attttcccta cagtaaagaa cctagctctg 4260

agcagtgaaa ttgtaatggc actttaaagg aagtaagccg ttaactgttc tctagtggag 4320

cgatctccaa ctgttttggc actagggacg ggttttgtgg aagaaaattt ttccacagga 4380

ctgggggttt agggggatgg tttcaggatg attcaagtac attacattta tcattagatt 4440

ctcataagga gcatgcaacc tagatctctt gcacgtgtgg ttcacagcag gattcgagct 4500

cctttgagaa tctaatgcca tggctgatct aacaggaaac tgagctcagg cagtaatgct 4560

tggcaccgcc ccccaccttc tatgcagccc ggtcgtggcc tggggactgg ggacccctgc 4620

tctagtcagt aataaggtac ttgtgccaga atataaatca acacattgct tcctttatca 4680

aagaagtctt gttatttaaa aaaagtcaac tgagccagta tgattagtga tgtaattgat 4740

tttcattctg gcacaagcct ctttcattct ggacagctca caaatagtta atggaccatg 4800

ctttgaatag ccttcctcta agcaacattt ataaatactg atattttaga actgtttaca 4860

tttcttctgt ttatttttga attttcagtt tgatatcttg tccttattca ttgttgtata 4920

aacaactgta ctttaatttc aagtagtatt aaaagtattt cacttcagtt tggggggatt 4980

attatcaatt tataatttta taaaagtatt ttaaagaata attgtaaatt ttccataaat 5040

tacaacttcc tgccatattt tattaaataa taatcttgct taaggcatat agacagacat 5100

tattatgagt attccagtaa aaaaaatcta catcaacttg accattctgg ctaaaaatta 5160

aaaagcactt ttttatatct gtggttgtca tttgtttcaa agcatttcta aatttattgt 5220

tcttaaaagt atgtctgcat gttctagcct ttgacctagg tcatctatga accctctttg 5280

tgtctaataa acatatctgt aaaggcaaaa aaaaaaaaaa aaaa 5324

<210> 37
<211> 7274
<212> DNA
<213> Homo sapiens

<400> 37
catagagcca gcgggcgcgg gcgggacggg cgccccgcgg ccggacccag ccagggcacc 60

acgctgcccg gccctgcgcc gccaggcact tctttccggg gctcctaggg acgccagaag 120

gaagtcaacc tctgctgctt ctccttggcc tgcgttggac cttccttttt ttgttgtttt 180

tttttgtttt tcccctttct tccttttgaa ttaactggct tcttggctgg atgttttcaa 240

cttctttcct ggctgcgaac ttttccccaa ttgttttcct tttacaacag ggggagaaag 300

tgctctgtgg tccgaggcga gccgtgaagt tgcgtgtgcg tggcagtgtg cgtggcagga 360

tgtgcgtgcg tgtgtaaccc gagccgcccg atctgtttcg atctgcgccg cggagccctc 420

cctcaaggcc cgctccacct gctgcggtta cgcggcgctc gtgggtgttc gtgcctcgga 480

gcagctaacc ggcgggtgct gggcgacggt ggaggagtat cgtctcgctg ctgcccgagt 540

cagggctgag tcacccagct gatgtagaca gtggctgcct tccgaagagt gcgtgtttgc 600

atgtgtgtga ctctgcggct gctcaactcc caacaaacca gaggaccagc cacaaactta 660

accaacatcc ccaaacccga gttcacagat gtgggagagc tgtagaaccc tgagtgtcat 720

cgactgggcc ttcttatgat tgttgtttta agattagctg aagatctctg aaacgctgaa 780

ttttctgcac tgagcgtttt gacagaattc attgagagaa cagagaacat gacaagtact 840

tctagctcag cactgctcca actactgaag ctgattttca aggctactta aaaaaatctg 900

cagcgtacat taatggattt ctgttgtgtt taaattctcc acagattgta ttgtaaatat 960

tttatgaagt agagcatatg tatatattta tatatacgtg cacatacatt agtagcacta 1020

cctttggaag tctcagctct tgcttttcgg gactgaagcc agttttgcat gataaaagtg 1080

gccttgttac gggagataat tgtgttctgt tgggacttta gacaaaactc acctgcaaaa 1140

aactgacagg cattaactac tggaacttcc aaataatgtg tttgctgatc gttttactct 1200

tcgcataaat attttaggaa gtgtatgaga attttgcctt caggaacttt tctaacagcc 1260

aaagacagaa cttaacctct gcaagcaaga ttcgtggaag atagtctcca ctttttaatg 1320

cactaagcaa tcggttgcta ggagcccatc ctgggtcaga ggccgatccg cagaaccaga 1380

acgttttccc ctcctggact gttagtaact tagtctccct cctcccctaa ccacccccgc 1440

ccccccccac cccccgcagt aataaaggcc cctgaacgtg tatgttggtc tcccgggagc 1500

tgcttgctga agatccgcgc ccctgtcgcc gtctggtagg agctgtttgc agggtcctaa 1560

ctcaatcggc ttgttgtgat gcgtatcccc gtagatgcca gcacgagccg ccgcttcacg 1620

ccgccttcca ccgcgctgag cccaggcaag atgagcgagg cgttgccgct gggcgccccg 1680

gacgccggcg ctgccctggc cggcaagctg aggagcggcg accgcagcat ggtggaggtg 1740

ctggccgacc acccgggcga gctggtgcgc accgacagcc ccaacttcct ctgctccgtg 1800

ctgcctacgc actggcgctg caacaagacc ctgcccatcg ctttcaaggt ggtggcccta 1860

ggggatgttc cagatggcac tctggtcact gtgatggctg gcaatgatga aaactactcg 1920

gctgagctga gaaatgctac cgcagccatg aagaaccagg ttgcaagatt taatgacctc 1980

aggtttgtcg gtcgaagtgg aagagggaaa agcttcactc tgaccatcac tgtcttcaca 2040

aacccaccgc aagtcgccac ctaccacaga gccatcaaaa tcacagtgga tgggccccga 2100

gaacctcgaa gacatcggca gaaactagat gatcagacca agcccgggag cttgtccttt 2160

tccgagcggc tcagtgaact ggagcagctg cggcgcacag ccatgagggt cagcccacac 2220

cacccagccc ccacgcccaa ccctcgtgcc tccctgaacc actccactgc ctttaaccct 2280

cagcctcaga gtcagatgca ggatacaagg cagatccaac catccccacc gtggtcctac 2340

gatcagtcct accaatacct gggatccatt gcctctcctt ctgtgcaccc agcaacgccc 2400

atttcacctg gacgtgccag cggcatgaca accctctctg cagaactttc cagtcgactc 2460

tcaacggcac ccgacctgac agcgttcagc gacccgcgcc agttccccgc gctgccctcc 2520

atctccgacc cccgcatgca ctatccaggc gccttcacct actccccgac gccggtcacc 2580

tcgggcatcg gcatcggcat gtcggccatg ggctcggcca cgcgctacca cacctacctg 2640

ccgccgccct accccggctc gtcgcaagcg cagggaggcc cgttccaagc cagctcgccc 2700

tcctaccacc tgtactacgg cgcctcggcc ggctcctacc agttctccat ggtgggcggc 2760

gagcgctcgc cgccgcgcat cctgccgccc tgcaccaacg cctccaccgg ctccgcgctg 2820

ctcaacccca gcctcccgaa ccagagcgac gtggtggagg ccgagggcag ccacagcaac 2880

tcccccacca acatggcgcc ctccgcgcgc ctggaggagg ccgtgtggag gccctactga 2940

ggcgccaggc ctggcccggc tgggccccgc gggccgccgc cttcgcctcc gggcgcgcgg 3000

gcctcctgtt cgcgacaagc ccgccgggat cccgggccct gggcccggcc accgtcctgg 3060

ggccgagggc gcccgacggc caggatctcg ctgtaggtca ggcccgcgca gcctcctgcg 3120

cccagaagcc cacgccgccg ccgtctgctg gcgccccggc cctcgcggag gtgtccgagg 3180

cgacgcacct cgagggtgtc cgccggcccc agcacccagg ggacgcgctg gaaagcaaac 3240

aggaagattc ccggagggaa actgtgaatg cttctgattt agcaatgctg tgaataaaaa 3300

gaaagatttt ataccettga cttaactttt taaccaagtt gtttattcca aagagtgtgg 3360

aattttggtt ggggtggggg gagaggaggg atgcaactcg ccctgtttgg catctaattc 3420

ttatttttaa tttttccgca ccttatcaat tgcaaaatgc gtatttgcat ttgggtggtt 3480

tttattttta tatacgttta tataaatata tataaattga gcttgcttct ttcttgcttt 3540

gaccatggaa agaaatatga ttcccttttc tttaagtttt atttaacttt tcttttggac 3600

ttttgggtag ttgttttttt ttgttttgtt ttgttttttt gagaaacagc tacagctttg 3660

ggtcattttt aactactgta ttcccacaag gaatccccag atatttatgt atcttgatgt 3720

tcagacattt atgtgttgat aattttttaa ttatttaaat gtacttatat taagaaaaat 3780

atcaagtact acattttctt ttgttcttga tagtagccaa agttaaatgt atcacattga 3840

agaaggctag aaaaaaagaa tgagtaatgt gatcgcttgg ttatccagaa gtattgttta 3900

cattaaactc cctttcatgt taatcaaaca agtgagtagc tcacgcagca acgtttttaa 3960

taggattttt agacactgag ggtcactcca aggatcagaa gtatggaatt ttctgccagg 4020

ctcaacaagg gtctcatatc taacttcctc cttaaaacag agaaggtcaa tctagttcca 4080

gagggttgag gcaggtgcca ataattacat ctttggagag gatttgattt ctgcccaggg 4140

atttgctcac cccaaggtca tctgataatt tcacagatgc tgtgtaacag aacacagcca 4200

aagtaaactg tgtaggggag ccacatttac ataggaacca aatcaatgaa tttaggggtt 4260

acgattatag caatttaagg gcccaccaga agcaggcctc gaggagtcaa tttgcctctg 4320

tgtgcctcag tggagacaag tgggaaaaca tggtcccacc tgtgcgagac cccctgtcct 4380

gtgctgctca ctcaacaaca tctttgtgtt gctttcacca ggctgagacc ctaccctatg 4440

gggtatatgg gcttttacct gtgcaccagt gtgacaggaa agattcatgt cactactgtc 4500

cgtggctaca attcaaaggt atccaatgtc gctgtaaatt ttatggcact atttttattg 4560

gaggatttgg tcagaatgca gttgttgtac aactcataaa tactaactgc tgattttgac 4620

acatgtgtgc tccaaatgat ctggtggtta tttaacgtac ctcttaaaat tcgttgaaac 4680

gatttcaggt caactctgaa gagtatttga aagcaggact tcagaacagt gtttgatttt 4740

tattttataa atttaagcat tcaaattagg caaatctttg gctgcaggca gcaaaaacag 4800

ctggacttat ttaaaacaac ttgtttttga gttttcttat atatatattg attatttgtt 4860

ttacacacat gcagtagcac tttggtaaga gttaaagagt aaagcagctt atgttgtcag 4920

gtcgttctta tctagagaag agctatagca gatctcggac aaactcagaa tatattcact 4980

ttcatttttg acaggattcc ctccacaact cagtttcata tattattccg tattacattt 5040

ttgcagctaa attaccataa aatgtcagca aatgtaaaaa tttaatttct gaaaagcacc 5100

attagcccat ttcccccaaa ttaaacgtaa atgttttttt tcagcacatg ttaccatgtc 5160

tgacctgcaa aaatgctgga gaaaaatgaa ggaaaaaatt atgtttttca gtttaattct 5220

gttaactgaa gatattccaa ctcaaaacca gcctcatgct ctgattagat aatcttttac 5280

attgaacctt tactctcaaa gccatgtgtg gagggggctt gtcactattg taggctcact 5340

ggattggtca tttagagttt cacagactct taccagcata tatagtattt aattgtttca 5400

aaaaaaatca aactgtagtt gttttggcga taggtctcac gcaacacatt tttgtatgtg 5460

tgtgtgtgtg cgtgtgtgtg tgtgtgtgtg aaaaattgca ttcattgact tcaggtagat 5520

taaggtatct tttattcatt tgccctcagg aaagttaagg tatcaatgag acccttaagc 5580

caatcatgta ataactgcat gtgtctggtc caggagaagt attgaataag ccatttctac 5640

tgcttactca tgtccctatt tatgatttca acatggatac atatttcagt tctttctttt 5700

tctcactatc tgaaaataca tttccctccc tctcttcccc ccaatatctc cctttttttc 5760

tctcttcctc tatcttccaa accccacttt ctccctcctc cttttcctgt gttctcttaa 5820

gcagatagca cataccccca cccagtacca aatttcagaa cacaagaagg tccagttctt 5880

cccccttcac ataaaggaac atggtttgtc agcctttctc ctgtttatgg gtttcttcca 5940

gcagaacaga gacattgcca accatattgg atctgcttgc tgtccaaacc agcaaacttt 6000

cctgggcaaa tcacaatcag tgagtaaata gacagccttt ctgctgcctt gggtttctgt 6060

gcagataaac agaaatgctc tgattagaaa ggaaatgaat ggttccactc aaatgtcctg 6120

caatttagga ttgcagattt ctgccttgaa atacctgttt ctttgggaca ttccgtcctg 6180

atgattttta tttttgttgg tttttatttt tggggggaat gacatgtttg ggtcttttat 6240

acatgaaaat ttgtttgaca ataatctcac aaaacatatt ttacatctga acaaaatgcc 6300

tttttgttta ccgtagcgta tacatttgtt ttgggatttt tgtgtgtttg ttgggaattt 6360

tgtttttagc caggtcagta ttgatgaggc tgatcatttg gctctttttt tccttccaga 6420

agagttgcat caacaaagtt aattgtattt atgtatgtaa atagatttta agcttcatta 6480

taaaatattg ttaatgccta taactttttt tcaatttttt tgtgtgtgtt tctaaggact 6540

ttttcttagg tttgctaaat actgtaggga aaaaaatgct tctttctact ttgtttattt 6600

tagactttaa aatgagctac ttcttattca cttttgtaaa cagctaatag catggttcca 6660

atttttttta agttcacttt ttttgttcta ggggaaatga atgtgcaaaa aaagaaaaag 6720

aactgttggt tatttgtgtt attctggatg tataaaaatc aatggaaaaa aataaacttt 6780

caaattgaaa tgacggtata acacatctac tgaaaaagca acgggaaatg tggtcctatt 6840

taagccagcc cccacctagg gtctatttgt gtggcagtta ttgggtttgg tcacaaaaca 6900

tcctgaaaat tcgtgcgtgg gcttctttct ccctggtaca aacgtatgga atgcttctta 6960

aaggggaact gtcaagctgg tgtcttcagc cagatgacat gagagaatat cccagaaccc 7020

tctctccaag gtgtttctag atagcacagg agagcaggca ctgcactgtc cacagtccac 7080

ggtacacagt cgggtgggcc gcctcccctc tcctgggagc attcgtcgtg cccagcctga 7140

gcagggcagc tggactgctg ctgttcagga gccaccagag ccttcctctc tttgtaccac 7200

agtttcttct gtaaatccag tgttacaatc agtgtgaatg gcaaataaac agtttgacaa 7260

gtacatacac cata 7274

<210> 38
<211> 5630
<212> DNA
<213> Homo sapiens

<400> 38
   aagagaaggg gctgggggcg gggagatggt gagtcgggga taaacactcc gcggcggtgg 60
   cggctgctgc tctgctccgg gttctgtcac tgtgtcggcg gtgcccagct cactggcccc 120
   ctccctctct tgtcgagcgt ggttgccaga gaggctccct cagccctgct ccgcggggtc 180
   cacagcgggc tccacagcgg gctccatagc gggctccaca gcggtccggc ggcggcagcg 240
   agcccgtggg cagtgggggt tggtcccgtg gctccggccc ccggtgcaga atggcggcgg 300
   cggttcggat gaacatccag atgctgctgg aggcggccga ctatctggag cggcgggaga 360
   gagaagctga acatggttat gcctccatgt taccatacaa taacaaggac agagatgcct 420
   taaaacggag gaacaaatcc aaaaagaata acagcagtag cagatcaact cacaatgaaa 480
   tggagaagaa tagacgggct catcttcgct tgtgcctgga gaagttgaag gggctggtgc 540
   cacttggacc cgaatcaagt cgacacacta cgttgagttt attaacaaaa gccaaattgc 600
   acataaagaa acttgaagat tgtgacagaa aagccgttca ccaaatcgac cagcttcagc 660
   gagagcagcg acacctgaag aggcagctgg agaagctggg cattgagagg atccggatgg 720
   acagcatcgg ctccaccgtc tcctcggagc gctccgactc cgacagggaa gaaatcgacg 780
   ttgacgtgga gagcacggac tatctcacag gtgatctgga ctggagcagc agcagtgtga 840
   gcgactctga cgagcggggc agcatgcaga gcctcggcag tgatgagggc tattccagca 900
   ccagcatcaa gagaataaag ctgcaggaca gtcacaaggc gtgtcttggt ctctaagaga 960
   gtgggcactg cggctgtctc cttgaaggtt ctccctgttg gttctgatta ggtaacgtat 1020
   tggacctgcc cacaactccc ttgcacgtaa acttcagtgt cccaccttga ccaaaatcag 1080
   ctttgtaact gttttcaagg aggtgcttag gattgtgggt ttctgattgc atccactagc 1140
   ttctcttttt ctcgccataa aaatttgtct ctgagagact atacattcca atcaatttga 1200
   agcatccaag aattcttaga ccgaataagc aatgtccaca tctcagcaat ccccctcttt 1260
   gctctcctcg tgtcctctcc cagacctttc ttccagtttt ctggcttact gtgttacttg 1320
   cctaggagga atgtccaagt gtgtcttgta cttaggaaac tgaaggaaac aaacttttga 1380
   aattgagatc ctgatctcag aactccaaag taagctttga aagcagcatt taagagcact 1440
   taaccatgga cctcaccacc agtgaggaag tcaggaatac ctctagaaaa cacgcccttc 1500
   acaccgcgca tgctcattcc cccgacgcgc cgcgtgtgga tgggagcagt atttctcact 1560
   ttaaaatgga caccttgata gtgtgtctct ggggttgcac agctcatcaa agttccaaat 1620
   tgtttgttct cacaaacaaa acggtacaat ctatttttgt gcgatgttct tgggacctcg 1680
   ctgtgttctg ctatctcgag gcacattctc ccctccaact ttgttaatgc tacttgtctc 1740
   tggaacattt ttttttccta cctcagagat aaatgagatc tccatttccc acttaacaca 1800
   gtgattatcc ttttttcccc ccccaaaata gtgacatttg gtccaattat aatctatttt 1860
   cctattgaac tttcagcaat aactgagctg tggcactagc agagctagta gccattatca 1920
   gtgaaagaaa aagtccacat ttctactctc tgttgcaggt gaacaggaga ggatggtaca 1980
   ctgttactag aactcctctc cttattgatt atttttggac acacccagaa acttctttat 2040
   ggaggctttt gggttgatag tttaaaaggc tgatttttct ttttggttat gatttctccc 2100
   ttaagtggtc tccgactttg tagcattttt atttaagcta aaacagagca catgtatatg 2160
   tacataagac acattaaatc tataaatact atttattcat tttatataaa ctaatgtaat 2220
   ggaaaacaaa ttcttatgac tttgtggttt tatagatgtt ctagaaactt tgtatgtagg 2280
   tatctacaaa attagttcat tcccctgaat atttttgcat tcatattttt gaggtcttga 2340
   tgttttcagc ctctggcgaa tctttttcat tgaatttgaa ccatttgtaa aatctgtgat 2400
   gctgaagcag agtgtgtcac aaagtgatga gaacattact aaaatccacg gacgcactgc 2460
   gacctaaggg ctcaacggct gactcggcag cgggcagcca ccccacgctc ccctgcggtc 2520
   actcgcacac cacagcctga agctccccca gcgcctgcac ctcgcacaca gctaaggtca 2580
   aagttcaaac gcactccaca cggaagctca ttctataccc gaagagcagt ctcagaaagc 2640
   aagattactt ttgtgttttt taaaaaatga ttctttaatg tatttttcta aacattctga 2700
   ttggaagtag tggattccta aatgattcca aagtcacctg taattcttct gtttttgttt 2760
   tgttctgtct tttcttcatt ttggctttgg gtggggggag gggcaggtga cacaaaggat 2820
   tttttttttt tttttttaat ttttggaatc ttttccaata accagctaaa gatttgcact 2880
   gaaatacaac ttgtatgcct tttgcatttt taaagcctgc ttcctggatt taagcagagt 2940
   gatagtgttc aaagagccag ttcagcctgt aacatatttg aaaaagatat gtctgcactt 3000
   tgaggtccct tttgaatgcc attcactaga cctctcaagc attttgtttc attgctacat 3060
   ccaagcgcct cacaagtcca caatgcggga cagcatcaaa agctcaagac tttggaaaaa 3120
   gcttgtgggc ttgcactggg ggagggaagg gaacaaaatt tgtgtacttc tttgtttaat 3180
   ttagaaataa ggcatccaag agatgccatt attttctgtg tttcaattgt tgtgcctttg 3240
   agttaaactg catttttgtc ttttggttga aatctgaaat gtactgtccc aatataaaac 3300
   agtaattatt tgacctttgc actgtttgtc tggtcctttt cagtttgatt gcatataaat 3360
   gtggaacttg atagatctct atatttttaa tgcacttgtg ataaactggc agcagggtta 3420
   gacattactt tcaaagcttg aggtagaccg agtcagcatg ctagacaggc ttctctctct 3480
   aaccaaaact gtaatcttca ggaccagcaa actcagccca aggcagctaa tccccccaac 3540
   cccatcctcc gcgccccgtg cggctgatcg gcagccctga ttcgccaatt tgtcctctct 3600
   cattcactga tccaccagcc tgactgctaa gagctatagt ctttttagtt gttttgtctt 3660
   tttaagcaag atgaaaacct ttctattagg gattttgggg ttgggagggg atgggcagag 3720
   atataaaccc cagcctttaa gactttgaca attgtacgta aatacagatg tgtataaata 3780
   taggcacatg catattttta tgtgaaagtt gattttaaaa aactaaaaaa atctaaactg 3840
   cactcttatt gataccatca taacgcaagt gggaaaaata agagtacgca gtctaattta 3900
   atttcatgca gtgggaaaat atatatgtgt gcttctgtaa catcctgaaa acatagcttt 3960
   ccatcccctg ttggctttga atggtgggcc gagcacccag gtcgtctgta ttttggtttt 4020
   cttttgctaa gcagagatct tgaattcttc aaggtgctga tagcaactgc tggctccttt 4080
   ctgtagtcac acacctaatg ctagtttagt gattcaaaat gcatcacatt tttaggcagg 4140
   acctagattt tccctgtcaa cctaagatga aaataatttc agtgttgatt cagaactgaa 4200
   cattaagtag gccctcgtcc tgcagttggc cacttgagtg ttttgttttg tttttatttt 4260
   ttaaggtggg cattttcctt taacctctac tttttcaaaa gcaacaaagg ggcctcaacc 4320
   tgagtttcct atgggcctct cttctgcatc cccaaagcgg ccaagagcaa atcctggggg 4380
   ataagaaaaa agtgtaaact aggtaggatt gtgatgctca aaataaccat ctagcaatat 4440
   cttggagctt gagaatagat tttgtgggct tatttcttct tgcctcttcc ccatcctttc 4500
   aagagagaac ttatttttga aaagtatcta tatatacaca cacacacaca cacacacata 4560
   ttattattta ggtttttata ccatactgta ttggcgagaa taccactatc attgtccttt 4620
   acagtctatt tcttccccca agtcttggtc tttttttatt ttctattttt tcatgaacca 4680
   cacaggagac tttaacatcc tggtcttttc tgtttcttct ttgtttcccc aagtttgtct 4740
   gtcccccttt gccttccctg agtgttgaac atcaggtagt aaaaggctaa acgcaatttc 4800
   ttgcatgtca atctattctt tttctatgtt tgactctgat gcagtgtgtt tagcgtgtct 4860
   agtagctggc tactcctatt taaaaactct tcctggtaga agacaaccca aagacccttt 4920
   tcgatgaggt ggtttctcat tctacatcct ctgatctcta tagactgtag gatgctttgc 4980
   tttcaaagat aactgggtta gagggtgggg tgtgcaatag gtgatttatc atggtttttt 5040
   tcattatcaa tattacatgg atgattttct cagattcttc tgaaagaaga aattgacagg 5100
   cactgctaga ttcagctatt gaatggctga agagattgag tatttgacct tctctcaaaa 5160
   tcataaagtg agaattcata aggcacccaa tgttaagatt tatccagatt tttacatttt 5220
   gatttcttct ctctgtgggg tggcaagttg agggagcatt cttcatttta gcttttacct 5280
   gacaaccaaa cttgccttta ccccatccct agaattggtg ctcttggaat attgctgtta 5340
   ccatcatttt tgggggccca tcttcctaat gctacacaca gcctgacagg ggagcagcag 5400
   atgaaagggt atgctattct gtttccagat gtttctttat gtaaatatga cgccaatgta 5460
   aatcctgtgt caagatcata gagaatggtg ctttttacta cagttagcac atgcattttt 5520
   agaaactact acatgtttta gagaatcttt gctgtgtata tgtaaactgt attgttcaac 5580
   tgttaacaaa taataaatta tttcattatt aaagaaaaaa aaaaaaaaaa 5630

<210> 39
<211> 1439
<212> DNA
<213> Homo sapiens

<400> 39
agtcacattt cagccactgc tctgagaatt tgtgagcagc ccctaacagg ctgttacttc 60

actacaactg acgatatgat catcttaatt tacttatttc tcttgctatg ggaagacact 120

caaggatggg gattcaagga tggaattttt cataactcca tatggcttga acgagcagcc 180

ggtgtgtacc acagagaagc acggtctggc aaatacaagc tcacctacgc agaagctaag 240

gcggtgtgtg aatttgaagg cggccatctc gcaacttaca agcagctaga ggcagccaga 300

aaaattggat ttcatgtctg tgctgctgga tggatggcta agggcagagt tggatacccc 360

attgtgaagc cagggcccaa ctgtggattt ggaaaaactg gcattattga ttatggaatc 420

cgtctcaata ggagtgaaag atgggatgcc tattgctaca acccacacgc aaaggagtgt 480

ggtggcgtct ttacagatcc aaagcaaatt tttaaatctc caggcttccc aaatgagtac 540

gaagataacc aaatctgcta ctggcacatt agactcaagt atggtcagcg tattcacctg 600

agttttttag attttgacct tgaagatgac ccaggttgct tggctgatta tgttgaaata 660

tatgacagtt acgatgatgt ccatggcttt gtgggaagat actgtggaga tgagcttcca 720

gatgacatca tcagtacagg aaatgtcatg accttgaagt ttctaagtga tgcttcagtg 780

acagctggag gtttccaaat caaatatgtt gcaatggatc ctgtatccaa atccagtcaa 840

ggaaaaaata caagtactac ttctactgga aataaaaact ttttagctgg aagatttagc 900

cacttataaa aaaaaaaaaa aggatgatca aaacacacag tgtttatgtt ggaatctttt 960

ggaactcctt tgatctcact gttattatta acatttattt attatttttc taaatgtgaa 1020

agcaatacat aatttaggga aaattggaaa atataggaaa ctttaaacga gaaaatgaaa 1080

cctctcataa tcccactgca tagaaataac aagcgttaac attttcatat tttttctttt 1140

cagtcatttt tctatttgtg gtatatgtat atatgtacct atatgtattt gcatttgaaa 1200

ttttggaatc ctgctctatg tacagttttg tattatactt tttaaatctt gaactttata 1260

aacattttct gaaatcattg attattctac aaaaacatga ttttaaacag ctgtaaaata 1320

ttctatgata tgaatgtttt atgcattatt taagcctgtc tctattgttg gaatttcagg 1380

tcattttcat aaatattgtt gcaataaata tccttgaaca cacaaaaaaa aaaaaaaaa 1439

<210> 40

<211>  5735
<212>  DNA
<213>  Homo sapiens

<400>  40
gtgggggggg cgccctcgga gccgggcgga ggggaggggg gaaagaggag cgcagggtga      60

gagtgagccg caggcttcgg gaggcgaggg ggcgggggga gcagcgccga ggccgccgcc     120

tccgcctccg ccgcctagga ctagggggtg ggggacggac aagccccgat gccgggggag     180

acggaagagc cgagaccccc ggagcagcag gaccaggaag ggggagaggc ggccaaggcg      240

gctccggagg agccccaaca acggccccct gaggcggtcg cggcggcgcc tgcagggacc     300

actagcagcc gcgtgctgag gggaggtcgg gaccgaggcc gggccgctgc ggccgccgcc      360

gccgcagctg tgtcccgccg gaggaaggcc gagtatcccc gccggcggag gagcagcccc     420

agcgccaggc ctcccgacgt ccccgggcag cagccccagg ccgcgaagtc cccgtctcca     480

gttcagggca agaagagtcc gcgactccta tgcatagaaa aagtaacaac tgataaagat     540

cccaaggaag aaaaagagga agaagacgat tctgccctcc ctcaggaagt ttccattgct     600

gcatctagac ctagccgggg ctggcgtagt agtaggacat ctgtttctcg ccatcgtgat     660

acagagaaca cccgaagctc tcggtccaag accggttcat tgcagctcat ttgcaagtca     720

gaaccaaata cagaccaact tgattatgat gttggagaag agcatcagtc tccaggtggc     780

attagtagtg aagaggaaga ggaggaggaa gaagagatgt taatcagtga agaggagata     840

ccattcaaag atgatccaag agatgagacc tacaaacccc acttagaaag ggaaacccca     900

aagccacgga gaaaatcagg gaaggtaaaa gaagagaagg agaagaagga aattaaagtg     960

gaagtagagg tggaggtgaa agaagaggag aatgaaatta gagaggatga ggaacctcca    1020

aggaagagag gaagaagacg aaaagatgac aaaagtccac gtttacccaa aaggagaaaa    1080

aagcctccaa tccagtatgt ccgttgtgag atggaaggat gtggaactgt ccttgcccat    1140

cctcgctatt tgcagcacca cattaaatac cagcatttgc tgaagaagaa atatgtatgt    1200

ccccatccct cctgtggacg actcttcagg cttcagaagc aacttctgcg acatgccaaa    1260

catcatacag atcaaaggga ttatatctgt gaatattgtg ctcgggcctt caagagttcc    1320

cacaatctgg cagtgcaccg gatgattcac actggcgaga agccattaca atgtgagatc    1380

tgtggattta cttgtcgaca aaaggcatct cttaattggc acatgaagaa acatgatgca    1440

gactccttct accagttttc ttgcaatatc tgtggcaaaa aatttgagaa gaaggacagc    1500

gtagtggcac acaaggcaaa aagccaccct gaggtgctga ttgcagaagc tctggctgcc    1560

aatgcaggcg ccctcatcac cagcacagat atcttgggca ctaacccaga gtccctgacg    1620

cagccttcag atggtcaggg tcttcctctt cttcctgagc ccttgggaaa ctcaacctct 1680

ggagagtgcc tactgttaga agctgaaggg atgtcaaagt catactgcag tgggacggaa 1740

cgggtgagcc tgatggctga tgggaagatc tttgtgggaa gcggcagcag tggaggcact 1800

gaagggctgg ttatgaactc agatatactc ggtgctacca cagaggttct gattgaagat 1860

tcagactctg ccggacctta gtggacagga agacttgggg catgggacag ctcagacttt 1920

gtatttaaaa gttaaaaagg acaaaaaaaa aatctaaagc atttaaaatc tagtgaaata 1980

actgaagggc ctgctctttc cattgtggat cacagcacac acatacatac accctccacc 2040

tccccatccc ctgttctccc tctgttgctc cccttataaa attgatgttg tctttaccag 2100

aaaggtagac aaaaaagaag cagcagcagc tcttaaagtg agggttattc tcatactcgg 2160

ttccagccat cagcagactt cctgctcatc ggcagatccc cctttccaac ctgtaactct 2220

gatgtgctct ggatcagctt ttaactttta atcatatatt actgtcttct aaatcccttc 2280

tcctcctcta ctgctgccct atggttctgg ctcctacccc ctgcggcaca cttatcttca 2340

aataccatag aattctaatc tctggaggct ggcagcttga cttggcactt tagggcccct 2400

tagcagggtg agctgttaaa acagcacaca tctctcatcc cctcttcctt tattcccccc 2460

tgggtttcag aaaggaagga tatatgggga ccacctcccc cttctttgat cccagcatct 2520

cagtccccct cccaaccctc catatggctc tcaatggtgc tcacttgctt ggaagcaggc 2580

tcccaatagg gagggggctg ccctctacag tctctttgac tgtaagacag ggctctgtat 2640

cagtgagacg atgagaaaag tcccaggcta atggcagaaa tttgcacttt gaacatgtgt 2700

gtttttgtgt tgtggaacct gagattcctt atttattaac aggaagtctg attttttttt 2760

tttggagtct ttgttgctat attttgtggg gctgggagag agagattaga ttattttgac 2820

atgggatccc ttccataaca ggtactttga aggcaagaca tagggttgaa gaagcacagc 2880

cagcctctga aatcatagct ctccagtggc ttttaaagaa agctggtcct cagcactaac 2940

aaaatcacta caatagccta gtgctttttt ggaagccttt ttagggaaga atgttaggtt 3000

catggtaact agtatgctct ttgagatttt tacagtgttg aaacttaaga attttgagag 3060

ggtgaggagg gttgttcaga atctaaatta cagatagatg attgtttctt gtgaatttgt 3120

ttcttttcct ttttttttgt ccctaccatt tccttacatt tcccttgggg cccatctctg 3180

gctccttgct tttgtttctt tgctttgctt tatcagttca ttccagctcc ctgttagtga 3240

aggacactgc tgttagtgaa ggaacaaagt ctatgagtcc taaaatttta agtcaaagaa 3300

aactgctctg tttccccttt agtaacactt ctgaagagga aaaacttcaa tagccaaagt 3360

taataatcct atataataat tgctttggct ttcacctaaa attctgggca tcacaatttc 3420

cttgggatag aggttgtgtt ggggaataga ttgcttattg ctgttcactg gagagaaaag 3480

gtagtgtttt tgtacaaggt cataccgcca gaagccccaa atcctatttt ggctcatctt 3540

caggtaaaga gtaattccta tcctgtgtgc ctcagaagct agaatcgaag gcttacccta 3600

ttcattgttt attgtcagaa atgcatgatg gctcttggaa agaatgacgt tttgctggaa 3660

aaaaaaaaaa gaacagtttg tgtttcacaa acatggctta tcaatttttt caaagaattc 3720

ttttttccca aaaagaggag taacaaaatg tcatttctga aagaggctta ctttatacca 3780

actagtgtca gcatttggga tgccagggaa cagagagtga gacacctaca atcaccagtc 3840

tcaaatgcgc tattgtttct tttcagagtg ttgcagattt gccatttctc cataatatgg 3900

ggatagaaaa tggaataaag atagaaggga tgtagaatat gctttcctgc caacatggtt 3960

tggagtcgac tttggtatat tgactagatt tgaaaataca agattgatta gatgaatcta 4020

caaaaaagtt gtcctcctct caggtccctt ttacactttt tgactaacta gcatctatat 4080

tccacactta gcttttttgt cacacttatc ctttgtctcc gtaaatttca tttgcagtgg 4140

ttagtcatca gatattttag ccacctacac aaaagcaaac tgcattttta aaaatctttc 4200

tgagatggga gaaaatgtat tctcctttcc tataccgctc tcccaacaaa aaaacaacta 4260

gttagttcta ctaattagaa acttgctgta cttttctttt tcttttaggg gtcaaggacc 4320

ctctttatag ctaccatttg cctacaataa attattgcag cagtttgcaa tactaaaata 4380

ttttttatag actttatatt tttccttttg ataaagggat gctgcatagt agagttggtg 4440

taattaaact atctcagccg tttccctgct ttcccttctg ctccatatgc ctcattgtcc 4500

ttccagggag ctcttttaat cttaaagttc tacatttcat gctcttagtc aaattctgtt 4560

acctttttaa taactcttcc cactgcatat ttccatcttg aattggtggt tctaaattct 4620

gaaactgtag ttgagataca gctatttaat atttctggga gatgtgcatc cctcttcttt 4680

gtggttgccc aaggttgttt tgcgtaactg agactccttg atatgcttca gagaatttag 4740

gcaaacactg gccatggccg tgggagtact gggagtaaaa taaaaatatc gaggtataga 4800

ctagcatcca catagagcac ttgaacctcc tttgtacctg tttggggaaa aagtataatg 4860

agtgtactac caatctaact aagattatta tagtctggtt gtttgaaata ccattttttt 4920

ctccttttgt gtttttccca ctttccaatg tactcaagaa aattgaacaa atgtaatgga 4980

tcaatttaaa atattttatt tcttaaaagc cttttttgcc tgttgtaatg tgcaggaccc 5040

ttctcctttc atgggagaga caggtagtta cctgaatata ggttgaaaag gttatgtaaa 5100

aagaaattat aataaaaggg atactttgct tttcaaatct ttgttttctc ttattctagg 5160

taaggcatat taaaaataaa tatgtaaaga agaaaaataa aagttgtctt catggaagca 5220

acttgtcttc cttggttgta ctgagttaca gttatcctag gggtgaaaca tgtgatgctg 5280

ctaagcaaac caaatgccct cagaacaggt gttatgtggg gcatactatt gtttgctttt 5340

gttgagaatc aggtggttaa tttttgactg ttcttgattt ctaatgctga aatgacatga 5400

ttctgttatt cagcaaactt ggaaatcttg atgttttgac aactgcctcc taggaaaact 5460

ggccatatgt taattaacct agtagatgga aaattaagga ttatgtgagg ttaattttac 5520

cctgataatg acaaaacctt gatagcattt aatattaata cttcttctca aaattgaatg 5580

tttatatcaa gtactgattt ttattttaaa aaagaaaaaa ctataatcct tctgccttcc 5640

aaaagccatg ctgtgatagc tgcccaggct gctctgttac atctcccatt tattgtttac 5700

ttttataaat ttgcttctaa gatggaaaaa aaaaa 5735

<210> 41
<211> 325
<212> DNA
<213> Homo sapiens

<220>
<221> misc_feature
<222> (275)..(277)
<223> n is a, c, g, t or u

<400> 41
gtgcccgaac cttggctgcc tcactcgagc cagcagatgc cttcctgaga acaggcggca 60

cccaggggcc aaccacgctt ggctctgacg tctctgtcct gcagactgtc cccgcttccc 120

cacgcacctc ctcccagctc tgtggacgcc tctttctaaa agagacgcac tatcccctta 180

ccctggagaa gcctgcagcc ctttggtgag gcgggggcat tctccgactg gattgccctt 240

ccaatccagt gtcaactgtg gcgacagccg attannncat tggaagtaaa ccctcttgaa 300

taaacggcta tccttaccag gtttg 325

<210> 42
<211> 3137
<212> DNA
<213> Homo sapiens

<400> 42
agcaccgtct gggctgtgga agcggagggg gtggggacac tctggcccgg ttctcggtgg 60

tgcgggagcg ggcgggagca gcggccgctc tggtcggcgg acgtgctgcc gagtagtccc 120

ggaagcgaag cagcgatggc ggagagtccg actgaggagg cggcaacggc gggcgccggg 180

gcggcgggcc ccggggcgag cagcgttgct ggtgttgttg gcgttagcgg cagcggcggc 240

gggttcgggc cgcctttcct gccggatgtg tgggcggcgg cggcggcagc gggcggggcc 300

gggggcccgg ggagcggcct ggctccgctg cccggctccc cgccctcagc cgctgcccac 360

ggggccgcgc tgcttagcca ctgggacccc acgctcagct ccgactggga cggcgagcgc 420

accgcgccgc agtgtctact ccggatcaag cgggatatca tgtccattta taaggagcct 480

cctccaggaa tgttcgttgt acctgatact gttgacatga ctaagattca tgcattgatc 540

acaggcccat ttgacactcc ttatgaaggg ggtttcttcc tgttcgtgtt tcggtgtccg 600

cccgactatc ccatccaccc acctcgggtc aaactgatga caacgggcaa taacacagtg 660

aggtttaacc ccaacttcta ccgcaatggg aaagtctgct tgagtattct aggtacatgg 720

actggacctg cctggagccc agcccagagc atctcctcag tgctcatctc tatccagtcc 780

ctgatgactg agaaccccta tcacaatgag cccggctttg aacaggagag acatccagga 840

gacagcaaaa actataatga atgtatccgg cacgagacca tcagagttgc agtctgtgac 900

atgatggaag gaaagtgtcc ctgtcctgaa cccctacgag gggtgatgga gaagtccttt 960

ctggagtatt acgacttcta cgaggtggcc tgcaaagatc gcctgcacct tcaaggccaa 1020

actatgcagg acccttttgg agagaagcgg ggccactttg actaccagtc cctcttgatg 1080

cgcctgggac tgatacgtca gaaagtgctg gagaggctcc ataatgagaa tgcagaaatg 1140

gactctgata gcagttcatc tgggacagag acagaccttc atgggagcct gagggtttag 1200

accctgctcc catctcccct tcccccactc aagagtccca gcagaatccc ttccccccac 1260

cccagggatg gagaggcact gtgtatctcc ctccagactc gaagtcatcc tgcaagatgg 1320

caagaaccaa gcaagctccg atcccagggt gtgggagtgg gggcctgttc ccggtctgac 1380

ctccttggca ctggagcatc tggggcttcg ttcatccatt catcccgtat caggggccaa 1440

ggtaccttta caggagcacc tagagcgagg gcctttggca aaaacaaaac aaccaacaca 1500

cctctccaca gggccagctc cttagggata agtggaagat ggaaattgca attccaagag 1560

ggagtgtgcc caaatgattt atggggatac ctggaaggga gcttggggtg ggggctgtct 1620

gtgacactta agcagtctgg gtggttgtct atttgtctgt cttcagtctt gaagcagggc 1680

ttcccaatgc ccttttcctc cctgccttcc ttcccccatt atttcccaca ggccagcata 1740

attttgtttt tcctaattta tagtcactgt tctagacaga ccaaagagaa ggaacagtgg 1800

tggagtctag gctgctgatc agtaagcttt acctagcacc tgagcacctt tctcccctcc 1860

cctctttcct cacccttttc tagatgtaag acagaaagta aatgtgactg ggacttaacc 1920

aaggtcttgg taaagcctgc atggcaccgt aagaagctga aaatactgtt tgttcccgca 1980

atcactgatt tgaaaagttc ccaacacagg cagctgctgt gtatatggga ttagagccac 2040

tacatagaat agtctcttac agattttcat aaatactagt cacaataagg gtatttttct 2100

tgggggtgga gtaaggggga gactgatgct agtccttgtt gtattttgtt gggctgtcct 2160

tgtgtatttt caccccagcc tgtagtcctc ctcacttcaa ccccagggat tttgggggag 2220

caagggtagc caatggcaga gggggttggg gctgggactc tggaggctcc tccccttctt 2280

tctcttcctt ccgcctcccc cgtgccccca gctgctcttg tcactgtctc tgatgggtat 2340

ttgcctggct ttgttgcttc tctatctgta tttagctgca gtgatccttt agctggttgg 2400

ctcagaaaaa aaaaaatgtg ctttaggtgc cctgtaatcc tgggcatcaa gggaatccat 2460

ccttcccctt tttgatatgt tctccccgta cttccagatt tattgttatg gctcccagtg 2520

ggtattggcg attcttgtga tgcagggcct cagtcagtgt ccagccatgc ataaggagag 2580

ggatagtgtg tacctgccct gccctctgct atgaaggtct ctgccttgtg gatcatggga 2640

ctccccttgg aggatctgtg caaagggggg ctgggcacaa aggagaatgt cctatttggg 2700

agggcaggaa gcaaaggaac tggacaggga ttggtgggct tggggaacgg aagtttatct 2760

tggataccct tgaagaggct gggtctcttc acatgaagat cgaaaaggga ccctgcttcc 2820

aatttccctc ttccattcct cgagctactc cagggcttag aagaatgctc ttggtctgtg 2880

ggtccagtgt tgtctgtcat ccatttaagt gttcccactt tcaagtgaca atcctctcct 2940

tggccctgcc atagggcaga gcatgtctgg catagcagcc tgacttttat gccctaatct 3000

tgagttgagg aaatatatgc acaggagtca aagagatgtc tttatatctg actgtatata 3060

aatgaagttt ttttgttttt tttgttttcc tttttggtgc aataaagttt gttttggcag 3120

aaggagaaaa aaaaaaa 3137

<210> 43
<211> 1839
<212> DNA
<213> Homo sapiens

<400> 43
agcaggggtg gtcgccatgg agacgcgtgg ccctggcctg gcggtccgcg ctgagagtcg 60

ccgattagtc ggcatcgggc ctcggcgccc cccggggcgg gttgggttgc agcccagcgg 120

gcggctggac cgccgcggtg gggcggggac aatggggtac aaggacaacg acggcgagga 180

ggaggagcgg gagggcggcg ccgcgggccc gcgggggtct agactgcccc ccatcacagg 240

cggcgcctcc gagctggcca aacggaaggt gaagaagaaa aaaaggaaga agaagaccaa 300

ggggtctggc aagggggacg ataaacatca gagtcagagc ctgaagagcc agccgctttc 360

ttcctccttt catgacatct taagtccttg caaagagcgt ggcccgaaac cagagcacag 420

acagagcaaa gtggaaaaga agcacctccc ttctgactcc tccaccgtca gcctccccga 480

ctttgccgaa atagagaacc ttgcgaatcg gatcaacgaa agtctgcgtt gggatggaat 540

tcttgctgac ccggaggcag agaaggaaag gattcgcatc tataaactga atcggagaaa 600

acggtaccgg tgcttggccc tcaagggctt ccaccccgac cccgaggccc tcaagggctt 660

ccaccccgac cccgacgccc tcaagggctt ccaccccgac cccgaggccc tcaagggctt 720

ccaccccgac cccgaggccc tcaagggcat ccaccccgac cccgaggctc tcaagggctt 780

ccaccccgac cccgaggccc tcaagggctt ccaccccgac cctgaggccc tcaagggttt 840

ccacactgac cccgaggccc tcaagggttt ccacattgac cccgaggccc tcaagggctt 900

ccaccccgac cccaaggccc tcaagggctt ccaccccgac cccaaggccc tcaagggttt 960

ccacactgac cccgaggccc tcaagggctt ccaccccgac cccaaggccc tcaagggctt 1020

ccaccccgac cccgaggccc tcaagggctt ccaccccgac cccgaggccc tcaagggctt 1080

ccaccccgac cccgaggccc tcaagggctt ccacactgac cccaatgccg aggaggcccc 1140

tgagaactta ccctacctct cggacaaaga tggcagctcc agccacaggc agcccacgtc 1200

gaaagccgag tgccccaatc tctgttttga aggaaacctt accccaaagc ttctacactc 1260

tgacttagct cctactctgc tggagtgaaa atctacccac gacgctcaca aacttataat 1320

tcctcctcac cacaagttta tgtgttaaaa accatcatca taaaaggaaa tgagacccgt 1380

atctgaagga aacagatgtt cggtacacgg acgacgccga ctctcccatc accaagctgc 1440

cctcggttgc ccaggagagc cacagtgcct tgagaacata agcaatttag tgaacagagt 1500

tcttttcaga atttcctttt tcttaagtaa gcatctctgt tacttaattt ctcaccacag 1560

ctagatgtct ataatctgcc ccaaaaagaa aagaaagcgt gtagatgatt tacctcttaa 1620

gtctcaatct catggtgaaa ctcctcacac ggtacctgct tctgttggcc ggaggacgat 1680

gccctgaacc tcttggagcc tttttctgtt ttaattaaat gcatggcctg cctttatatg 1740

gcctcaaata agtgggttcc agaactccat ccctgggaag ttaccatggg aagtcccaag 1800

aggaatactg cttctttcat aaacatggtc ctctcctaa 1839

<210> 44
<211> 1885
<212> DNA
<213> Homo sapiens

<400> 44
cagctctagc gaaaagccgc cggtatttct ccatctggct ctcctctacc tccaggcagg 60

ctcacccgag atccccgccc cgaacccccc ctgcacactc ggcccagcgc tgttgccccc 120

ggagcggacg tttctgcagc tattctgagc acaccttgac gtcggctgag ggagcgggac 180

agggtcagcg gcgaaggagg caggccccgc gcggggatct cggaagccct gcggtgcatc 240

atgaagttcc agtacaagga ggaccatccc tttgagtatc ggaaaaagga aggagaaaag 300

atccggaaga aatatccgga cagggtcccc gtgattgtag agaaggctcc aaaagccagg 360

gtgcctgatc tggacaagag gaagtaccta gtgccctctg accttactgt tggccagttc 420

tacttcttaa tccggaagag aatccacctg agacctgagg acgccttatt cttctttgtc 480

aacaacacca tccctcccac cagtgctacc atgggccaac tgtatgagga caatcatgag 540

gaagactatt ttctgtatgt ggcctacagt gatgagagtg tctatgggaa atgagtggtt 600

ggaagcccag cagatgggag cacctggact tgggggtagg ggaggggtgt gtgtgcgcga 660

catggggaaa gagggtggct cccaccgcaa ggagacagaa ggtgaagaca tctagaaaca 720

ttacaccaca cacaccgtca tcacattttc acatgctcaa ttgatatttt ttgctgcttc 780

ctcggcccag ggagaaagca tgtcaggaca gagctgttgg attggctttg atagaggaat 840

ggggatgatg taagtttaca gtattcctgg ggtttaattg ttgtgcagtt tcatagatgg 900

gtcaggaggt ggacaagttg gggccagaga tgatggcagt ccagcagcaa ctccctgtgc 960

tcccttctct ttgggcagag attctatttt tgacatttgc acaagacagg tagggaaagg 1020

ggacttgtgg tagtggacca tacctgggga ccaaaagaga cccactgtaa ttgatgcatt 1080

gtggcccctg atcttccctg tctcacactt cttttctccc atcccggttg caatctcact 1140

cagacatcac agtaccaccc caggggtggc agtagacaac aacccagaaa tttagacagg 1200

gatctcttac ctttggaaaa taggggttag gcatgaaggt ggttgtgatt aagaagatgg 1260

ttttgttatt aaatagcatt aaactggaat tgacaagagt gttgagcatc cctgtctaac 1320

ctgctctttc tctttggtgc cccttatctc accccttcct tggaatttaa taagtctcag 1380

gcatttccaa ttgtagacta aaaccactct tagcatctcc tctagtattt tccatgtatc 1440

aggacagagg tgtcttatgt agggaggggg caagtatgaa gtaaggtaat tatatactac 1500

tctcattcag gattcttgct cccatgctgc tgtcccttca ggctcacatg cacaggaatg 1560

ctacatgatg gccagctgct tccctccttg gttatcatcc actgcagctg ctagttagaa 1620

aggtttggag ggatgacttt tagtaaatca tggggatttt attgatttat tttcactttt 1680

gggattttgt ggggtgggag tggggagcag gaattgcact cagacatgac atttcaattc 1740

atctctgcta atgaaaaggg ttctttctct tgggggaaat gtgtgtgtca gttctgtcag 1800

ctgcaagttc ttgtataatg aagtcaatgc catcaggcca aggaaataaa ataattgctt 1860

accttaaaaa aaaaaaaaaa aaaaa 1885

<210> 45
<211> 2223
<212> DNA
<213> Homo sapiens

<400> 45
   acttcttttc ttggctaagc cgcgtttgta ctgtgtctta ccatgcctga accggcaaaa 60
   tccgctccgg cccctaaaaa gggctccaag aaagccgtca ccaaagccca gaagaaagac 120
   ggcaagaagc gcaagcgcag ccgcaaagag agctactcca tctacgtgta caaggtgctg 180
   aagcaggtcc accccgacac cggcatctcg tccaaggcca tgggcatcat gaactccttc 240
   gtcaacgaca tcttcgagcg catcgcggga gaggcttccc gcctggcgca ctacaacaag 300
   cgctccacca tcacatcccg cgagatccag acggccgtgc gcctgctgct gcccggcgag 360
   ctggccaagc acgccgtgtc cgagggcacc aaggcggtca ccaagtacac cagctccaag 420
   tgagtccctg ccgggacctg gcgctcgctc gctcgagtcg ccggctgctt gactccaaag 480
   gctcttttca gagccaccca cctaatcact agaaaagagc ttgttcactt attcccttag 540
   tttcttttca taaagtaagt tattttagtg tgaaggtcat gggaaatggc atacgtagct 600
   ttttaactat ttggaactcg aggtccccag tgcgtcattg gatttgcttt tgaatctaga 660
   gcgtgtcttt actcattgtg ctgcttagcc ttcccaggag tcggttctca attaggctgt 720
   tgggaatccg cctctttacc cgcccccact cccgccccac acgcgccctg gtggctcctt 780
   gggtctgttt cattctaaaa cgaagtggct gagttcggct gtcatttaag agaactccag 840
   gacacaattc agcccgggtt ccgcaaacac tgcgtgacag ctctgtatga ctgacgcttg 900
   gcagcagctt ttgtgtccgg tcaccagttc tgccgtgcga tggggcctcc tgtggatacc 960
   agccgttctg tgtattttgg acgaaggccg cgcagccggg tcccagcctt gtcctgattg 1020
   ggcgacaaga atattcaaaa ttctgcgcct tttctaattt tgtagatttc agtttccgtc 1080
   gttcactttg agactttgaa attcctattt ctcattttgt tgataatttc tgcatttaat 1140
   ggtctgtgct ttaaatggta acgctacggc cccaggtcac tgcgaggcac ttaccatgta 1200
   gatacgggct caaaagtcac ctctcagaga cctacgtcat ccactcagga attcgcgcct 1260
   ctcatacttg cctgtctcat tttatcttcc ttctagcagc tgtctgaaat tggttcgtct 1320
   gttttcttgt ttatggtatt ctcaagccct tgacagaccg gctagtgtgg ttttcccgtg 1380
   catcttcagc ctggcacatt atggacactt aaatactacg tattgatcta atattgttgg 1440
   gttaattttt ccatcccacc cttttcttaa tcgcttccgt ggatggatga agggtgctgt 1500
   tcatttccat tagatgtatg tgaaggcaca gtgaaaatgg aaatgttctt ggagctactt 1560
   cctcaaaatg tatccttagt cacctcagtg caacagctgg gagggggccg tgttaagatt 1620
   ttttttgcta caaagaggag gtggcaatgg tagatccacc cttatgcttc tcagtttagc 1680
   ataacctctt atggattttc atcaaattca gcgtgttggt cactggaaag agccttttcc 1740
   ttctcctttt cttactctcc cctcatggtg ttcccctctt aaaggagagg agcttttaat 1800
   ttacacttac cacctcattt gcttttctgg aggccatgca atataggcgg gactacagag 1860
   ttaatctcct ttttacaaat gaggccaaga gaagcctcat tggttcacag tcatgcagct 1920
   catactgtcc acccttgtat tctcagatgc aggacaattg cattttagtt ttattttgtg 1980
   gaggtgcaga atatttactc tttctgtcca acccttgatt ctgccgagga agacactgat 2040
   ggtttgatga gtgattcagc tgtttttggc taagggcttt tggagctgat ggcaggggtt 2100
   tgatgaatcc aaatgagctc tagacattat cacagactga atagatctta actgtctcct 2160
   acatgtgtgt tttcaaatgt gtatagatgc tattgttatt aataaagtta ccaattaatt 2220
   taa 2223

<210> 46
<211> 7582
<212> DNA
<213> Homo sapiens

<400> 46
tctttcactc ttgggcctag cgaggcagct tatttttcct ttttctttca ttggtcacag 60

ttatttcttt tcagtgtgtc atcttctcta agtttctgag ggagtgttag cttgaggcct 120

tgccttgaca taatcagata taatcagaaa aatgaaaaat tccataggaa agagaactat 180

tttagccaag gtgtgcgaga gaaataccgc cactttcaag cactgttttc ttctactgga 240

gtctgctcaa tagggacgtc agctttgctg gggcttcctt tgacaagaga atcagaaccg 300

actggtgaca tttgtttcaa tgaaagcaac agtgtgaaga gtaagtagtt tttcctatta 360

ttcatctcac tcagtggaga taatgaactc ctctccactg ccaagatgag aaactaccat 420

ttcttacaca tggacacaaa gatgagaaca ataaacactg agacttcaaa aaggagggaa 480

ggagaaagag gaacaagact tgaaatctac ctatcagaac ttgcaattta ttctgatcaa 540

caatttgccc agctaaagta ctacatctgc cccctcttcc tgtggctgta ggggcacagc 600

aaaggtcact ggtctaacct ccttaaaggg actccgctaa cagaaaccac caaatggagt 660

ggagaaaaag aaaagggatc ccctatcccc aactccagcc atctagatta agaaaagcca 720

gctgactgga cagtagcaca gcccagtcac ctcatggaca aatttcctag gaaagaacct 780

ctcccatcta ttctacttat cactctcctt tgaggttccg gccacagatc ttcgcctgct 840

gctggaaatg gccctctcag tggactcatc gtggcatcgg tggcagtgga gagtcagaga 900

tggcttcccc cattgtccat cggaaaccac accgctgctc tctccagaga aagggagaca 960

gagctacaac ttgacacagc agcgggtcgt gttccccaac aacagcatat tccatcaaga 1020

ttgggaagag gtctccagga gataccctgg caacagaacc tgcacaacca aatacaccct 1080

cttcaccttc ctgccccgga atctctttga gcaatttcat agatgggcta acctctattt 1140

cctgttcctg gtgattttga actggatgcc ctccatggaa gtcttccaca gagaaatcac 1200

catgttacca ttggccattg tcctgttcgt catcatgatc aaggatggca tggaggactt 1260

caagagacac cgctttgata aagcaataaa ctgctccaac attcgaattt atgaaagaaa 1320

agagcagacc tatgtgcaga agtgctggaa ggatgtgcgc gtgggagact tcatccaaat 1380

gaaatgcaat gagattgtcc cagcagacat actcctcctt ttttcctctg accccaatgg 1440

gatatgccat ctggaaactg ccagcttgga tggagagaca aacctcaagc aaagatgtgt 1500

cgtgaagggc ttctcacagc aggaggtaca gttcgaacca gagcttttcc acaataccat 1560

cgtgtgtgag aaacccaaca accacctcaa caaatttaag ggttatatgg agcatcctga 1620

ccagaccagg actggctttg gctgtgagag tcttctgctt cgaggctgca ccatcagaaa 1680

caccgagatg gctgttggca ttgtcatcta tgcaggccat gagacgaaag ccatgctgaa 1740

caacagtggc ccccggtaca aacgcagcaa gattgagcgg cgcatgaata tagacatctt 1800

cttctgcatt gggatcctca tcctcatgtg ccttattgga gctgtaggtc acagcatctg 1860

gaatgggacc tttgaagaac accctccctt cgatgtgcca gatgccaatg gcagcttcct 1920

tcccagtgcc cttgggggct tctacatgtt cctcacaatg atcatcctgc tccaggtgct 1980

gatccccatc tctttgtatg tctccattga gctggtgaag ctcgggcaag tgttcttctt 2040

gagcaatgac cttgacctgt atgatgaaga gaccgattta tccattcaat gtcgagccct 2100

caacatcgca gaggacttgg gccagatcca gtacatcttc tccgataaga cggggaccct 2160

gacagagaac aagatggtgt tccgacgttg caccatcatg ggcagcgagt attctcacca 2220

agaaaatgct aagcgactgg agaccccaaa ggagctggac tcagatggtg aagagtggac 2280

ccaataccaa tgcctgtcct tctcggctag atgggcccag gatccagcaa ctatgagaag 2340

ccaaaaaggt gctcagcctc tgaggaggag ccagagtgcc cgggtgccca tccagggcca 2400

ctaccggcaa aggtctatgg ggcaccgtga aagctcacag cctcctgtgg ccttcagcag 2460

ctccatagaa aaagatgtaa ctccagataa aaacctactg accaaggttc gagatgctgc 2520

cctgtggttg gagaccttgt cagacagcag acctgccaag gcttccctct ccaccacctc 2580

ctccattgct gatttcttcc ttgccttaac catctgcaac tctgtcatgg tgtccacaac 2640

caccgagccc aggcagaggg tcaccatcaa accctcaagc aaggctctgg ggacgtccct 2700

ggagaagatt cagcagctct tccagaagtt gaagctattg agcctcagcc agtcattctc 2760

atccactgca ccctctgaca cagacctcgg ggagagctta ggggccaacg tggccaccac 2820

agactcggat gagagagatg atgcatctgt gtgcagtgga ggtgactcca ctgatgacgg 2880

tggctacagg agcagcatgt gggaccaggg cgacatcctg gagtctgggt caggcacttc 2940

cttggaggag gcattggagg ccccagccac agacctggcc aggcctgagt tctgttacga 3000

ggctgagagc cctgatgagg ccgccctggt gcacgctgcc catgcctaca gcttcacact 3060

agtgtcccgg acacctgagc aggtgactgt gcgcctgccc cagggcacct gcctcacctt 3120

cagcctcctc tgcaccctgg gctttgactc tgtcaggaag agaatgtctg tggttgtgag 3180

gcacccactg actggcgaga ttgttgtcta caccaagggt gctgactcgg tcatcatgga 3240

cctgctggaa gacccagcct gcgtacctga cattaatatg gaaaagaagc tgagaaaaat 3300

ccgagcccgg acccaaaagc atctagactt gtatgcaaga gatggcctgc gcacactatg 3360

cattgccaag aaggttgtaa gcgaagagga cttccggaga tgggccagtt tccggcgtga 3420

ggctgaggca tccctcgaca accgagatga gcttctcatg gaaactgcac agcatctgga 3480

gaatcaactc accttacttg gagccactgg gatcgaagac cggctgcagg aaggagttcc 3540

agatacgatt gccactctgc gggaggctgg gatccagctc tgggtcctga ctggagataa 3600

gcaggagaca gcggtcaaca ttgcccattc ctgcagactg ttaaatcaga ccgacactgt 3660

ttataccatc aatacagaga atcaggagac ctgtgaatcc atcctcaatt gtgcattgga 3720

agagctaaag caatttcgtg aactacagaa gccagaccgc aagctctttg gattccgctt 3780

accttccaag acaccatcca tcacctcaga agctgtggtt ccagaagctg gattggtcat 3840

cgatgggaag acattgaatg ccatcttcca gggaaagcta gagaagaagt ttctggaatt 3900

gacccagtat tgtcggtccg tcctgtgctg ccgctccacg ccactccaga agagtatgat 3960

agtcaagctg gtgcgagaca agttgcgcgt catgaccctt tccataggtg atggagcaaa 4020

tgatgtaagc atgattcaag ctgctgatat tggaattgga atatctggac aggaaggcat 4080

gcaggctgtc atgtccagcg actttgccat cacccgcttt aagcatctca agaagttgct 4140

gctcgtgcat ggccactggt gttactcgcg cctggccagg atggtggtgt actacctcta 4200

caagaacgtg tgctacgtca acctgctctt ctggtatcag ttcttctgtg gtttctccag 4260

ctccaccatg attgattact ggcagatgat attcttcaat ctcttcttta cctccttgcc 4320

tcctcttgtc tttggagtcc ttgacaaaga catctctgca gaaacactcc tggcattgcc 4380

tgagctatac aagagtggcc agaactctga gtgctataac ctgtcgactt tctggatttc 4440

tatggtggat gcattctacc agagcctcat ctgtttcttt atcccttacc tggcctataa 4500

gggctctgat atagatgtct ttacctttgg gacaccaatc aacaccatct ccctcaccac 4560

aatccttttg caccaggcaa tggaaatgaa gacatggacc attttccacg gagtcgtgct 4620

cctcggcagc ttcctgatgt actttctggt atccctcctg tacaatgcca cctgcgtcat 4680

ctgcaacagc cccaccaatc cctattgggt gatggaaggc cagctctcaa accccacttt 4740

ctacctcgtc tgctttctca caccagttgt tgctcttctc ccaagatact ttttcctgtc 4800

tctgcaagga acttgtggga agtctctaat ctcaaaagct cagaaaattg acaaactccc 4860

cccagacaaa agaaacctgg aaatccagag ttggagaagc agacagaggc ctgcccctgt 4920

ccccgaagtg gctcgaccaa ctcaccaccc agtgtcatct atcacaggac aggacttcag 4980

tgccagcacc ccaaagagct ctaaccctcc caagaggaag catgtggaag agtcagtact 5040

ccacgaacag agatgtggca cggagtgcat gagggatgac tcatgctcag gggactcctc 5100

agctcaactc tcatccgggg agcacctgct gggacctaac aggataatgg cctactcaag 5160

aggacagact gatatgtgcc ggtgctcaaa gaggagcagc catcgccgat cccagagttc 5220

actgaccata tgaggagctg cagaaatctg tacaaactca acagaggcca cctagtcact 5280

ggtccacata acccttgacc ccttcttctt catagaggaa acaatgtgcc agtcttattc 5340

ttttcttcaa caaccttgac ttccatggag gaagtgctgg ccccaagggg tctgacacaa 5400

agacgggaaa cccagtcggc ctctagtttt ctgctgctct caggcagcac atcttgcaaa 5460

cagtttggag aaggaggctg tttttgttga atcgagttct caaatcggtt tagaccaaag 5520

ccattcttct gaccctctag ataagcgtag cctacaaccc agtgccgtaa gtttccaaga 5580

ttcaagaagt gtatcaaccc aggcaatatc tcaggatatg gaagtttctg ggtttattta 5640

cccctcagtg cccagagtta aagtttcaga agagacttgt gcacataagg gcttcatctc 5700

aagtgtattg cagtaatggc tgaatcgggg ttaacatccc ttccaggcac agcgagttgg 5760

ttctgctttt tgcctgtaag ccaaagaaaa gccacatcta aaaagctact actaaaagcc 5820

agaaagaaaa gtggatttga actcagtgtc acagactctt ctgagtgttt tagggtcaca 5880

gctagtgtaa gaggcatgaa gaatagacat gcaaaaggga acgggtgcac cagagacccc 5940

tgttttggct gacagaccat atgtcccacc agctggggaa tctgacaaga ggacataggt 6000

ggcactcttt ttttaaagct atttattgta tctattttta aataaaattg cccatcctca 6060

ttcagctctt agaacaaaag caaaaaaccc tgtaaatcag gagatataag cacatctgca 6120

cccagaatag gcccatatga tagggcaacc ctgagcttaa acaatgacat cttcaagggt 6180

agaactaatc tgaaacccca ttcagcctat tccagaatgg ggataggctg aaaccccctt 6240

ccagcctctg gaagacactg gcctgcatca gttagagtca gagcaagtgt cacttcacag 6300

ggaaaagaag gattatatag acttcctatc cctagagttt ataaatgtca actatataaa 6360

aaaagctcaa aacagtgtta aaggaatgaa cagtagaatt ttaataggct gtccaaagaa 6420

gccaggtctg ctgtgggcaa gtatagccta accctagtct tgtaaaataa gccagaaagg 6480

gttactgagc caccttaagc tagtacctat atagtaggca aaaagtacag aaatagatgc 6540

aataagtgtg gtgagtcttt gagcctacga gtcatgccac cagccataag ttgacctatc 6600

acttgagaac ctcctcagca aagatgccag aaaacattca atcaagttgg caaatgacac 6660

agggagctgg ccctctgacc atcttcctgg caaacctgga ctggaagggc catttgcagc 6720

actgtcctgg agctaataca ctgtttcact gcctctgcca tataatgatg ccagcactag 6780

ccagctggtg ggtatttgga ggaatcctgc atgaggattg cccaataagg ggcaggtaca 6840

catacctggc aaagtgatga tgatgtgaat tgtttccagt gaggggattg agtcaaaact 6900

tggatctcag gtacctcaat tttcccccca atttctggct actactaaaa gccagaaaga 6960

acagaacagt ggcctcagga gatctgagtt tgaatccttg ctctctagga tgcaggtggc 7020

ttgaagcaga atgccacacc tgcaagttga ttagaactgc ctttcttccc aggcttgaca 7080

taggtattaa gtcaaaatta catgaaaccc agtggtaaaa aagcctctga aagctgtaac 7140

accctcagta ataacaaaag ggatttttat ttcacagcta aagggaaaat aggtggagaa 7200

gttaaaaaat aatgtctgat cctgttccta agttccaaac tatagccaac actctgatgc 7260

tgctcttttt cttgtaggac caaccgtccc agtttgcctg ggactttctc atttttacag 7320

agtcccaaat cctaggaaac tggagcaact ggtacaactg gtcacctact cttgcccctc 7380

tgtaaatcaa gccaactgtg accatccaat gtgccatctt acagggaaaa gttataacca 7440

ctattcccct ataacataat gctaatgatt gtacttagta catttttata cttttatgat 7500

attttactga ttggaaatgt catcctttat taaaaataaa catggttttc catagttgcc 7560

tgccaaaaaa aaaaaaaaaa aa 7582

<210> 47
<211> 1297
<212> DNA
<213> Homo sapiens

<400> 47

ggggcggggc ttcgggcgag agggagggca gcaggagaaa ggataagaaa gggagtggga 60

ctggcgccta cggtggccga agtgggacgc gccgagccgg aggctgcagg atgatgcggt 120

tcatgctatt attcagccgg cagggaaaac tgcggctgca aaaatggtac ctggccactt 180

cggacaagga acggaagaag atggtgcgcg agctcatgca ggttgtcctg gctcgaaagc 240

ccaagatgtg cagcttcctg gagtggaggg acctcaaagt tgtctataag agatatgcca 300

gcctctactt ctgctgcgcc atcgagggcc aagacaatga gctcatcaca ctggagctga 360

tccaccgata cgtggagctc ttagacaaat actttggcag tgtgtgcgag ctggacatca 420

tcttcaactt tgagaaggcc tacttcatcc tggatgagtt tttgatgggg ggggatgtcc 480

aggacacctc caagaagagt gtgctgaaag ccatcgagca ggctgaccta ctgcaagagg 540

aggatgagtc gccacggagt gtgctggagg agatgggttt ggcatagccc ctgctgggcc 600

ggggtgtggc gatggggtcc tggcagcgtg gcgggaacgg ctgcttctcc tctgcccagg 660

gccctgttct tggtgggact cggctgcccc tcctctgctg cctcaccttt cggagtgagc 720

tgtgggctca ggcccttcaa acattccctc cctccacccc ctacctccac tttccccttt 780

tcccactgaa ggttttagaa gctaggaggc aggaaaatgt gacccagatg ggggtgctat 840

ttggctttta ttccctgcct ttgcagaact gatgtcaccc cagatgtcct tccctcccta 900

ataactgtaa atatataaat atgtcaggtt aaagggaaaa ggtgttcagg gcacttcttg 960

tcctctctgt cccataacct acctccaccc tccccctagc cagccaggca gcttctctgc 1020

ctgggagggg agcctggacc cccctctttc tccttggctg cagtggggcc tttatccagt 1080

gccagggagg aacaacatag ttaatttttt tctaaccttg ccactttgag ggaaggaagg 1140

gttgggggaa gggcaagctt tatgggaccc tggtcctggc cctggccttt cactccagtt 1200

ctgggtgagg caggagctgg gaggggtggg gagggggagg gggaagtgtc tgcctttatg 1260

tcctttcttc tgaaataaaa gtaaaagcat ttctgga 1297

<210> 48
<211> 2913
<212> DNA
<213> Homo sapiens

<400> 48
gaatggctgg ggttggctga agagcgcaca gtggagtttt aatgtccgcc atgttggcca 60

tggcgtattg aagagagcga gcgagagagg agcggagcgg cacagcctcc caccctcccg 120

gctggtgtta gtgcccggac ggcgggctct gcgctccgcc cctcaagtcc ccggcagcgg 180

ttggcgagtg ggggccgaac ccccggttct ccatgatccc gctggccggg gccgtttccc 240

cagagcggag aggtatctgc tgcgcctgag atgagtaaac tgtcgtttcg ggcgcgggcg 300

ctagacgcct cgaagccgct gccggttttc cgctgtgagg atctgcccga cctgcacgaa 360

tacgcctcga taaacagggc cgtgccgcag atgcccaccg gaatggagaa ggaagaggag 420

tcggaacatc atcttcagcg ggctatttca gcacagcagg tgtatggcga gaagagggat 480

aatatggtta taccggtccc agaggcagaa agtaatattg cttactatga gtctatatat 540

cctggggaat ttaagatgcc aaagcagctc attcacatac agccttttag tttggatgct 600

gaacagcctg attatgattt ggattctgaa gatgaagtat ttgtgaataa actgaaaaag 660

aaaatggaca tctgcccatt gcaatttgag gagatgattg accgcctaga aaaaggcagt 720

ggtcagcagc cagtcagtct gcaggaagcc aaactactgc taaaagaaga tgatgaacta 780

attagagaag tttatgaata ttggattaaa aagagaaaaa actgtcgagg gccatctctt 840

attccatcag taaaacaaga gaagcgagat ggttccagca caaatgatcc ttatgtggct 900

tttagaaggc gtactgaaaa aatgcagact cgaaaaaatc gcaaaaatga tgaagcctct 960

tacgaaaaaa tgcttaagct gcgacgagat ctaagtcgag ctgttactat tctagagatg 1020

ataaaaagaa gagaaaaaag taaaagagag ctattgcact taacactgga aattatggaa 1080

aagaggtata atttgggcga ctacaatgga gagatcatgt ctgaggttat ggcacagaga 1140

cagccaatga aacctactta tgccatcccc atcatcccta ttactaatag cagtcaattt 1200

aaacaccagg aagcaatgga tgtgaaggag ttcaaagtta ataagcaaga taaagccgat 1260

cttatccgac cgaaacggaa atatgaaaag aagcccaaag tcttaccatc gtctgccgct 1320

gctactcccc aacagacgag tcctgctgca ctgccagtct tcaatgctaa agatctgaat 1380

cagtatgact ttcccagctc agacgaagaa cctctctccc aggttttgtc tggctcttcg 1440

gaagctgagg aagacaatga tcctgatggt ccttttgctt tccgtaggaa agcaggctgt 1500

cagtactatg ctcctcactt agaccaaact ggcaactggc cttggactag tcctaaagat 1560

ggaggattag gggatgtgcg atatagatac tgcttaacta ctctcaccgt accccaaagg 1620

tgtattggat ttgcacgaag acgggttggg cgcggtggaa gggtcttact ggacagagct 1680

cattcagact atgacagtgt gtttcaccat ctggatttgg aaatgctttc ctcaccacaa 1740

cattctccag tcaatcagtt tgccaatacc tcagaaacaa atacctcgga caaatctttc 1800

tctaaagacc tcagtcagat actagtcaat atcaaatcat gtagatggcg gcattttagg 1860

cctcggacac catccctaca tgacagtgac aatgatgaac tctcctgtag aaaattatat 1920

aggagtataa accgaacagg aacagcacaa cctgggaccc agacatgcag tacctctacg 1980

caaagtaaaa gtagcagtgg ttcagcacac tttgcattta cagccgaaca ataccagcaa 2040

catcaacagc aactggcact catgcagaaa cagcagcttg cacaaattca gcaacagcaa 2100

gcaaatagta attcctccac caacacatca cagaaccttg catctaacca gcagaaaagt 2160

ggctttcgcc tgaatataca gggtttagaa agaacactac agggttttgt ttctaagact 2220

ttggattctg ctagtgcaca gtttgctgct tctgctttgg tgacatcaga acaactgatg 2280

ggattcaaga tgaaggatga tgtggtgctt ggaatcgggg tgaatggcgt ccttccagcc 2340

tcaggagtat acaagggctt acacctcagt agtactacac caacagcact tgtacataca 2400

agtccatcaa cggcaggttc agctttgtta cagccttcaa atattacaca gacttcaagt 2460

tcccacagtg cactgagtca tcaagtaact gctgccaatt ctgcaacaac tcaggttctg 2520

attgggaaca acattcgatt aactgtacct tcatcagttg ccactgtaaa ctctattgcc 2580

ccaataaatg cacgacatat acctaggact ttaagtgctg ttccatcatc tgccttaaag 2640

ctggccgctg cagcaaactg tcaagtttcc aaggtcccat cttcatcctc tgtagattca 2700

gttccaaggg aaaatcatga atcagaaaag ccagcactga acaacatagc agacaacaca 2760

gtagcgatgg aggtgacgta gcttcctccg agtggaactg cagcctgggg acttgattag 2820

gtgctatgca tcagtagcat tttgaatgca aggatgatgg gaaagcagca catgcgtttc 2880

tgtggcagct gtggggactt gacagcaatg ctc 2913

<210> 49
<211> 823
<212> DNA
<213> Homo sapiens

<400> 49
acggaaacgg gcgtgccatt tccgcgcacg tctgcagatg cggtagtcga ttggtcaagt 60

ctcccatggc tcctccttca tcaggaggtg ggcaaaccgc gccatgatag ggtcgggatt 120

ggctggctct ggaggcgcag gtggtccttc ttctactgtc acatggtgcg cgctgttttc 180

taatcacgtg gctgccaccc aggcctctct gctcctgtct tttgtttgga tgccggcgct 240

gctgcctgtg gcctcccgcc ttttgttgct accccgagtc ttgctgacca tggcctctgg 300

aagccctccg acccagccct cgccggcctc ggattccggc tctggctacg ttccgggctc 360

ggtctctgca gcctttgtta cttgccccaa cgagaaggtc gccaaggaga tcgccagggc 420

cgtggtggag aagcgcctag cagcctgcgt caacctcatc cctcagatta catccatcta 480

tgagtggaaa gggaagatcg aggaagacag tgaggtgctg atgatgatta aaacccaaag 540

ttccttggtc ccagctttga cagattttgt tcgttctgtg cacccttacg aagtggccga 600

ggtaattgca ttgcctgtgg aacaggggaa ctttccgtac ctgcagtggg tgcgccaggt 660

cacagagtca gtttctgact ctatcacagt cctgccatga tgagccctgt tcctgctcat 720

catgaagatc cccgcgatac ttcaacgcct tctgacttcc aggtgatgac tgggccccca 780

ataaatcccg tctttgggtc tctctgccaa aaaaaaaaaa aaa 823

<210> 50
<211> 1809
<212> DNA
<213> Homo sapiens

<400> 50
gcgagctgac agaggggagc agaggcccga gcggacgcga ggcgacgcgg agagggcggc 60

ctgggccgaa gtgaggcgac gcggggcggc ggagtctgag ggccggagcg gagcggaggc 120

gacgcgggga ggggcgaggg atcgcggccg gtggctgggg gccacctgct ccccgagcac 180

cggcccccgc tccggggagc agagtccgga gcgggatccg cggcccacag gttcgcaggt 240

tgcgaggggc ggtgttgaag aatgtgtggg cgaacatcct gtcacttacc tagagatgtt 300

ctcacgagag cttgcgccta ccaggatcgg cggggccagc agcggctccc ggagtggagg 360

gaccctgata agtactgccc ctcttacaac aagagtcctc aatccaacag cccagtgctt 420

ctgtctcgac tgcactttga gaaggatgca gactcatctg agcgtatcat tgctcccatg 480

cgctggggct tggtcccttc ttggttcaaa gaaagtgatc cttccaagct gcagttcaat 540

actaccaact gtcgtagtga taccgtaatg gagaaacggt catttaaggt gcctctggga 600

aagggaagac gctgtgtcgt tttagcagat ggattctatg agtggcagcg atgtcaggga 660

acaaaccaga ggcagccata cttcatctat tttcctcaaa tcaagacaga gaagtcaggt 720

agcattggtg ctgcagatag tcctgagaac tgggagaaag tctgggacaa ctggaggctg 780

ctgacaatgg ccgggatctt tgactgctgg gagcccccag agggaggaga tgtcctgtat 840

tcctatacca tcatcacagt ggattcctgc aaaggcttga gtgacatcca ccacaggatg 900

cctgccatat tagatggaga ggaggcagtt tctaaatggc ttgactttgg tgaagtctca 960

actcaggaag ctctgaaatt aatccaccca acagagaaca tcaccttcca tgcagtctct 1020

tctgtggtga acaactcgcg aaacaacact cctgagtgtc tggctcctgt cgacttggtg 1080

gtcaaaaagg agctcagggc aagtggcagt agccagagga tgttgcagtg gttggccaca 1140

aagtcaccca aaaaggaaga ctcaaaaaca cctcaaaagg aagagtcaga tgttccccag 1200

tggtccagtc agttcctgca gaagagtcca ctccccacca agagaggcac tgcaggactc 1260

ctagagcaat ggctgaagcg ggagaaggag gaggaacctg tggccaagcg tccttacagc 1320

cagtgacaca ggactttcag agaccaaggc cagggtctgc tgcactgctg ttctgataat 1380

aggttcttaa cattgtatgt atatgtgttt gctttgggag gaggtggcac tgtgttagtt 1440

gacagttgtg ggctcatgta gtcttttttg ccatgagtag gagcccctag tggggctggt 1500

ggacagcttt ggaagaggtg tcctgctgct gttaccagcc atgtgggccc cataggggca 1560

ctgcgcctgc tgccctttcc tggcagggct ggtggagtct tccctcaaag catgccttac 1620

ccagctggga agtctctgcc ctgatctggt actccttgta gtaagctgtt ttctgctcag 1680

ccactgggct ctttcacttt tttagttctt aaaaatttat ttttaagttc taaaataaaa 1740

taaaaataag ttcttaaaat ttattttttt cctgaataaa ttgtatttgg taaaaaaaaa 1800

aaaaaaaaa 1809

<210> 51
<211> 1886
<212> DNA
<213> Homo sapiens

<400> 51
aagtgacgca cgaggtgctg tgactcgtgc cagcccccta atctgcggaa gtggagtgcg 60

gggagtgcgc cggaagaggg gtacggaagt gcgccggaag tggggtgcgg aggtgtgcag 120

cgcgctgtca gactggctcg caggcggcgc ggccggcgga cccgttcgag acagcgcggg 180

cggctcgggt cccctggggc tccgcagcag gaggacgcca tggacgacaa ggagttaatt 240

gaatacttta agtctcagat gaaagaagat cctgacatgg cctcagcagt ggctgccatc 300

cggacgttgc tggagttctt gaagagagat aaaggggaga caatccaggg tctgagggcg 360

aatctcacca gtgccataga aaccctgtgt ggtgtggact cctctgtggc agtgtcctct 420

ggcggggagc tcttcctccg cttcatcagt cttgcctccc tggaatactc cgattactcc 480

aaatgtaaaa agatcatgat tgagcgggga gaactttttc tcaggagaat atcactgtca 540

agaaacaaaa ttgcagatct gtgccatact ttcatcaaag atggagcgac aatattgact 600

cacgcctact ccagagtggt cctgagagtc ctggaagcag ccgtggcggc caagaagcga 660

tttagtgtat acgtcacaga gtcacagcct gatttgtcag gtaagaaaat ggccaaagcc 720

ctctgccacc tcaacgtccc tgtcactgtg gtgctagatg ctgctgtcgg ctacatcatg 780

gagaaagcag atcttgtcat agttggtgct gaaggagttg ttgaaaacgg aggaattatt 840

aacaagattg gaaccaacca gatggctgtg tgtgccaaag cacagaacaa acctttctat 900

gtggttgcag aaagtttcaa gtttgtccgg ctctttccac taaaccagca agacgtccca 960

gataagttta agtataaggc agacactctc aaggtcgcgc agactggaca agacctcaaa 1020

gaggagcatc cgtgggtcga ctacactgcc ccttccttaa tcactctgct gtttacagac 1080

ctgggcgtgc tgacaccctc agcagtcagc gatgagctca tcaagctcta tctgtaacct 1140

gtgagccctt tcctgccaag gtgcagctta cgtagttgag gcagggtgag tagctgcttg 1200

acaccccagt gagtcaggcc aaaactgaga tgtgtttaat gaagatttat ggagtaagga 1260

cttaaaatca tacatcttgg agaacctttc ttactcattt cagtcccatc taaaaatgtg 1320

tcagctattc taaatcccaa cttaaattgt tcttacggtt tctagaaact ttccttttca 1380

gtttccagaa atacaagtta gataattggc tacttaactg atgaaagatg agcccaagtc 1440

cacctgtctt catcctcccc tgcactccag actgatctgc ctaaagcacg cgagatgcag 1500

gcgaaaagca gccacacccc tctgccacaa atgaccaaca gctggtcagg acgttacacg 1560

cggtgccttg taagaggcaa gaaacacttg ccgaatctgc atctggcttc cagtggtaag 1620

cacattcctc agcaggatca agccaaacag taaaaactac caagagaaca cgaggaaggc 1680

agaaacgatg tttagcaaca gtattctgca tggttcactg cttaagaaaa tgccttctgg 1740

aatatttgta aactgaaatt ctgtatgtgt aagagatgtt taaatatgtt tgggtcctaa 1800

acagcttttt aaaattatac ttgggaataa attcagcatc ttttcaaata aactctatga 1860

aagacgctga aaaaaaaaaa aaaaaa 1886

<210> 52
<211> 5768
<212> DNA
<213> Homo sapiens

<400> 52
gaaactttgc gcccagtccg cagggcgggc cgcgccttta ccgcccagct gcctcccgga 60

gcccccgcgc cctcccgacg cgcagagcca tggcctccca cctgcgcccg ccgtccccgc 120

tcctcgtgcg ggtgtacaag tccggccccc gagtacgaag gaagctggag agctacttcc 180

agagctctaa gtcctcgggc ggcggggagt gcacggtcag cacccaggaa cacgaagccc 240

cgggcacctt ccgggtggag ttcagtgaaa gggcagctaa ggagagagtg ttgaaaaaag 300

gagagcacca aatacttgtt gacgaaaaac ctgtgcccat tttcctggta cccactgaaa 360

attcaataaa gaagaacacg agacctcaaa tttcttcact gacacaatca caagcagaaa 420

caccgtctgg tgatatgcat caacatgaag gacatattcc taatgctgtg gattcctgtc 480

tccaaaagat ctttcttact gtaacagctg acctgaactg taacctgttc tccaaagagc 540

agagggcata cataaccaca ctgtgcccta gtatcagaaa aatggaaggt cacgatggaa 600

ttgagaaggt gtgtggtgac ttccaagaca ttgaaagaat acatcaattt ttgagtgagc 660

agttcctgga aagtgagcag aaacaacaat tttccccttc aatgacagag aggaagccac 720

tcagtcagca ggagagggac agctgcattt ctccttctga accagaaacc aaggcagaac 780

aaaaaagcaa ctattttgaa gttcccttgc cttactttga atactttaaa tatatctgtc 840

ctgataaaat caactcaata gagaaaagat ttggtgtaaa cattgaaatc caggagagtt 900

ctccaaatat ggtctgttta gatttcacct caagtcgatc aggtgacctg gaagcagctc 960

gtgagtcttt tgctagtgaa tttcagaaga acacagaacc tctgaagcaa gaatgtgtct 1020

ctttagcaga cagtaagcag gcaaataaat tcaaacagga attgaatcac cagtttacaa 1080

agctccttat aaaggagaaa ggaggcgaat taactctcct tgggacccaa gatgacattt 1140

cagctgccaa acaaaaaatc tctgaagctt ttgtcaagat acctgtgaaa ctatttgctg 1200

ccaattacat gatgaatgta attgaggttg atagtgccca ctataaactt ttagaaactg 1260

aattactaca ggagatatca gagatcgaaa aaaggtatga catttgcagc aaggtttctg 1320

agaaaggtca gaaaacctgc attctgtttg aatccaagga caggcaggta gatctatctg 1380

tgcatgctta tgcaagtttc atcgatgcct ttcaacatgc ctcatgtcag ttgatgagag 1440

aagttctttt actgaagtct ttgggcaagg agagaaagca cttacatcag accaagtttg 1500

ctgatgactt tagaaaaaga catccaaatg tacactttgt gctaaatcaa gagtcaatga 1560

ctttgactgg tttgccaaat caccttgcaa aggcgaagca gtatgttcta aaaggaggag 1620

gaatgtcttc attggctgga aagaaattga aagagggtca tgaaacaccg atggacattg 1680

atagcgatga ttccaaagca gcttctccgc cactcaaggg ctctgtgagt tctgaggcct 1740

cagaactgga caagaaggaa aagggcatct gtgtcatctg tatggacacc attagtaaca 1800

aaaaagtgct accaaagtgc aagcatgaat tctgcgcccc ttgtatcaac aaagccatgt 1860

catataagcc aatctgtccc acatgccaga cttcctatgg tattcagaaa ggaaatcagc 1920

cagagggaag catggttttc actgtttcaa gagactcact tccaggttat gagtcctttg 1980

gcaccattgt gattacttat tctatgaaag caggcataca aacagaagaa cacccaaacc 2040

caggaaagag ataccctgga atacagcgaa ctgcatactt gcctgataat aaggaaggaa 2100

ggaaggtttt gaaactgctt tatagggcct ttgaccaaaa gctgattttt acagtggggt 2160

actctcgcgt attaggagtc tcagatgtca tcacttggaa tgatattcac cacaaaacat 2220

cccggtttgg aggaccagaa atgtatggct atcctgatcc ttcttacctg aaacgtgtca 2280

aagaggagct gaaagccaaa ggaattgagt aagacaactg ctggaagatg tcttaaatca 2340

agctttcaaa aaaatatatt ttaggaggct gatttaatgc cagtctaaat ccttatgtag 2400

aaaggacttt gaaatttttc ttctcaagaa atggtttgta taagaataac aatctgctag 2460

tctgtcattt ctggagtgat actttttttt ttgagacgga gtctgctctg tcgctcgcgc 2520

tggagtgcag tggcatgatc tcggctcact gcaagctccg cctcccaggt tcatgccatt 2580

ctcctacctc agcctcccga gtagctggga ctacaggcgc ccaccaccat gcccggctaa 2640

tttttgtttt tgtattttta gtagagacag ggtttcactg tgttagccag gatggtctcg 2700

atctcctgac ctcgtgatcc gcccgcctca gccttccaaa gtgttgggat tataggcgtg 2760

agccaccgcg cccagccctg gagtgatact ttttatggaa gacaaaagcc ccccaaatct 2820

gtgtaaaatc tgctgcaaag gtgtcatccc tcttgtgtca tcactggggt tagaggtggg 2880

tccgaaataa tcttctgtgt ccttcagttg gactctcggc tgccaattga tctctttttc 2940

attgccatct ctggggtggt tctttggttt tttgtgtgtt ttccccttca tctctacctg 3000

tgaaagtgaa attctattgt aaatggaggg aaaaagggtt ggttgtgaaa aattaaagac 3060

ccacattctg ctttcttact catggtaaga aaagtggcca tgagtagaga ttgggcaagc 3120

attggtaata aatggaataa gactattatt attattattt gagatggagt ctcactctgt 3180

cacccaggct ggaatgcagt ggtgtgatct tggctcactg caacctccac ttcccgggtt 3240

caagcgattc tcctgcctca gcctcctgag tagctgggat tacaggtgtg tgcctccaca 3300

cccggctaat tttttgtatt tttagtagag acggggtttt gccatgttgg ccaggctggt 3360

ttcaaactcc tgagctcaaa tgatcctcct gccttggcct cccaaagtgc tggaattaca 3420

ggcatgagcc accacaccca cacaagacta tcatttttaa tgaccaagag cctagtatat 3480

agttggtgcc tgtcttagtc tgtttgtgtt gctataaaag aacacctgag actgggtaat 3540

tgataaagaa aaaggtttgt ttggctcaca attttgctgg ctagaaggtt gggcatccgg 3600

tgaaagcctc aggctgcttc cattcatagc aaagggcagc cagtgtgtgc agaaatcaaa 3660

tgacagagag gaagtgagag agagaggtgt cggggaggtg ccaggctctt tttaacaagc 3720

agttcttcag gaactaagag tgagtcactc ccatgagaac agcaccaagc cattcatggg 3780

ggaatctgcc cccatgaccc agacccctcc cgttaggctt cacctccaac actgaggatc 3840

aaatttcaac atgagatttg gaggaggtca aacaaactaa actgtagcag tgtttcataa 3900

aattgtttgc ctgactcagg ttgctagtaa gccagcagag ggatatttgc ctcctaaatc 3960

tttggcagag gcaggagtaa ggaagccatt tctggagtcc ttgctactaa tttggaaaac 4020

tgagcttctt tctttcattg ctttttccct taagagacaa gtccttacta tattgccctg 4080

tctctcaagg gaagacatca agactggact tgaactcctg ggctcaagcc atcccccaac 4140

cttggcctct cgagtagatg ggattatagg catgtgccac ggtgcctgac ttgagtttct 4200

tattctagaa cacttggagc ctgaactctg accaggcccc tcacttgagc ctttgctttc 4260

tgctccttgt aaactgccat attgggtgca cttgccctgc cacagtaatg ctatatattt 4320

ctgagcattg tttttctcta gataatttta tatttttgag tataccccac ttccaagtgt 4380

tttttgtttt gttttgcttt gtttttgttg ttgttgtttt gagacagggt ctcactgtgt 4440

cccccaggct ggagtgcagt ggcacaatga cgactcactg cagcctcaac ctcctggggc 4500

caagtgatcc tcccacctca gcctctcaag tggctgggac cacagaagtg caccaccatg 4560

cctggctttt tttttttttt ttggtcgaga tggggtgtcc ctgtgttgcc cagactggtc 4620

ttgaactcct ggactcaagg gatcctcctg tcttgggctc ccaaagtgtt gggattacag 4680

gcgtgagtga ccatgcctag ctcacttcca ggtttaacag acaaaataaa cttactctag 4740

tttccatctc tatcatttta taataaccgt agcccacatt gtagtagttt ttcagctctt 4800

tactaagtcc caccaattca tgttttcacc cttaaaatct ttctcactga tactctctct 4860

ggacagaaaa aaggtgaaat aagcctacta taaggaatat atgacatgct aaattttatt 4920

tttaaacggt tcttcaagtc agattaaagt aataatagca aattatgtga ttatccatgt 4980

cccagcctct ctccaaaaaa atagtaaaca agatgtcttc ttcttttccc aaagatacac 5040

atacacacat gtacaaattt ttttatcaga taataatagc taatatttaa tgagtactta 5100

ccttagtttg tcccctttac aacagcttta catctgtgtg attgatacag ttcatattcc 5160

cattttataa ctgagaaaac tggtgcacag agaggataag caacttgcca aaggtcacac 5220

agttaataag tggaaatgct ggggtatgaa ccaggtagtc tgcccccata gctctgcccc 5280

ccagagctgt actgtctccc atgagggtac ttctccatgg agcagcctga ggcgatccct 5340

ttattctggg cttctctcag aaatggattc ccacacagta ttcaaagcaa atttccccag 5400

aggaaatcct attggaagaa cttaaaaact cagaatcttt ttctttgtcc agagagttga 5460

ggaagcttaa gctaaatgat acatgttttt aaaaaaaaat cagattataa atttagtttt 5520

tggtgattca ttaaattctt tactattata gttattttct agctgttcat cttttagcta 5580

aatttgttcc aaagaagcaa aagtttggtt tctactaagt tctggattct ggatgggaga 5640

ttgcactgtg tgtgacatgc aagtttcatg gtgtgggaga ttgcagagca tttgggttac 5700

tgcttttact ctttggaagc tgttatcatc tgtatctgct ttaaataaag ttaaagattt 5760

ggaacaaa 5768

<210> 53
<211> 12568
<212> DNA
<213> Homo sapiens

<400> 53
cttccagctc ccgccgggcg gctgtgacgg ccgcagcggg tcgcagagca gggagtggac 60

acggagtggg gagcggagag ggaaggagga ggaggaggag caaaggtgtt ggagagaaaa 120

cttcagaaag gaacaggaaa cctgccgggg aggcggcggc ggctgcggct tctctgggcg 180

cctgggctgc gctcttcgcg gggtgtccgc agctgcggct tggccccggt ctggcttccc 240

cggcgcgcac gcacggctga gccgcgacgc tcagtggctc gggccgtgcc ctccgccgcg 300

gctcttggcc gctgtagtcc cgcgatccga tccgcttctg ccgcggcggc tccctggaga 360

gagggcggcg agggcgcagg gaagaagagg gacgtccact gtggaacatg aagcagcatg 420

actgagaaaa tgtccagctt cctctacata ggggacatcg tgtccctgta cgcggagggc 480

tcggtcaacg gcttcatcag caccttgggg ttagtggatg acagatgtgt ggtgcaccca 540

gaggccgggg accttgccaa ccctcccaag aagttcagag actgcctttt caaggtgtgc 600

cctatgaaca gatattctgc ccagaagcaa tattggaaag caaagcaagc caaacaaggg 660

aaccacaccg aggcagcctt gctgaagaaa ctacagcacg ctgcagaact ggaacaaaaa 720

caaaatgaat cggagaataa gaaactgttg ggagaaattg taaaatacag taatgttata 780

caactactgc atataaaaag caacaaatat cttactgtca acaagagatt acctgcttta 840

ctggagaaga atgccatgcg tgtgtccttg gatgctgcag gaaatgaagg gtcttggttt 900

tatattcatc cgttctggaa actgagaagc gagggtgaca atattgttgt aggagataaa 960

gttgttttga tgcctgtgaa tgcagggcag ccactacatg ccagcaacat agagcttctt 1020

gataacccag ggtgtaaaga ggtgaatgct gtcaattgca acaccagctg gaaaatcact 1080

ttattcatga aatatagttc ctatcgagag gatgtattaa aaggagggga cgttgttaga 1140

ttatttcatg cggaacaaga gaagtttttg acttgtgatg aatatgagaa aaaacagcac 1200

attttccttc gtacgacctt gcgccaatca gctacttctg ctactagttc taaagcactc 1260

tgggaaatag aggtggttca tcatgaccca tgccgtgggg gtgcaggaca gtggaacagc 1320

ttgttcagat ttaagcatct tgcaactgga aactatttag ctgcagagct taatcctgat 1380

tatcgagatg cccaaaatga aggaaaaaat gtgagagatg gagtccctcc aacttcaaag 1440

aaaaaacgcc aggcagggga gaagatcatg tatactttgg tttcagtccc gcatggcaat 1500

gacattgcat ccctttttga actagatgcc acaactcttc agagagctga ctgcctggtt 1560

ccaaggaact catatgttcg gttaaggcat ttatgcacca acacatgggt aaccagtact 1620

agtatcccca tagacacaga tgaagagagg cctgttatgt taaagattgg aacctgccaa 1680

accaaagaag ataaagaagc gttcgcaatc gtgtctgttc cactgtctga agttcgagac 1740

ttagactttg ccaatgatgc caataaagta ctagcgacca cagttaaaaa gctagaaaac 1800

ggcacaataa ctcagaatga aaggaggttt gtaaccaaat tattggaaga tctcatattc 1860

tttgttgctg atgtgcctaa taatggacaa gaagttctgg atgtggttat cactaagcca 1920

aaccgagagc gtcaaaaatt gatgagggaa caaaacatac tggcacaggt atttggaatt 1980

cttaaagcac cctttaaaga gaaagcagga gaaggctcga tgctgagact tgaagatctg 2040

ggggatcaaa gatatgcacc ctacaagtac atgctgcggc tctgttaccg cgtcctgaga 2100

cactcgcagc aggattaccg gaaaaatcag gaatatattg ctaagaattt ctgtgtcatg 2160

cagtcccaga ttggctatga tattttggca gaagatacta tcacagcttt gttgcacaac 2220

aacagaaaac tactagagaa acatatcaca gcaaaagaaa tagaaacatt tgtcagttta 2280

ctcaggagaa atcgggagcc aaggtttttg gattatttgt cagatctgtg tgtgtctaat 2340

accactgcta tccctgtaac tcaagaactc atctgtaaat ttatgttgag tccaggcaat 2400

gcagacattc tcattcaaac taaggtggtc tcaatgcaag cagacaaccc catggagagc 2460

tccatccttt cagatgacat tgatgatgaa gaagtttggc tctattggat tgacagcaac 2520

aaggaacctc atggcaaagc tatcaggcac cttgctcaag aggcaaaaga aggcaccaaa 2580

gctgacttag aagttcttac ctattacagg taccagctaa acctctttgc aaggatgtgc 2640

ttggatcgcc agtatctggc cataaaccag atttctacac agctgtctgt agacctgatc 2700

ctgcggtgtg tgtcggatga gagcctgccg ttcgacctcc gagcgtcctt ctgtcgcctc 2760

atgctccaca tgcacgttga ccgggatccc caggagtccg tggtgcctgt tcgctatgcc 2820

aggctctgga cagaaatccc cacaaagatc acaattcatg aatatgattc tataacagac 2880

tcttccagaa atgatatgaa gaggaaattt gccctgacaa tggaatttgt tgaagaatat 2940

ttgaaagaag ttgtaaacca gccctttcct tttggggata aagaaaaaaa taaactgaca 3000

tttgaggtgg tccacttggc tcggaatctt atatactttg gattttatag tttcagtgag 3060

ttattaaggc taacaagaac acttctggct attttagaca ttgtacaggc ccccatgtca 3120

tcatactttg aaagattaag caaatttcaa gatggaggaa acaatgtgat gagaaccatt 3180

catggggtgg gagagatgat gacccagatg gtactcagta gaggctccat cttccccatg 3240

agcgtgccgg atgtgccacc cagcatccac ccgagcaagc aagggagccc caccgagcac 3300

gaggatgtga ctgtgatgga caccaagctg aagatcattg agattttgca gtttatcctg 3360

agtgtcagac tggattatag gatctcatat atgctgtcaa tatataagaa ggagtttgga 3420

gaggacaatg acaatgcgga gacatctgcc agtggatctc cagacacttt actaccatca 3480

gctattgttc ctgatataga tgaaattgca gctcaggcag aaactatgtt tgcgggaaga 3540

aaagaaaaaa atccagttca acttgacgat gaaggaggca ggacgttttt acgggtcctc 3600

attcatctga tcatgcacga ctacccgcct ttgctgtctg gagccctgca gctgttgttt 3660

aagcacttca gccagagggc agaggtttta caggcattta agcaggtgca attactggtg 3720

tctaatcaag acgtagataa ctacaagcaa atcaaggcag atctagacca gcttcgactg 3780

acagtagaaa agtctgagct atgggtggag aagagcagca actatgagaa tggagaaata 3840

ggggaaagtc aagtgaaagg tggtgaagag ccaattgagg aatcaaacat tttaagtcca 3900

gtgcaggatg gaacaaagaa acctcagatt gacagcaaca agagcaataa ctaccggatt 3960

gtaaaggaga ttttgatcag gctaagtaaa ctctgtgtgc agaataaaaa gtgtcggaat 4020

caacatcaac gattactgaa aaatatgggg gcgcattcgg tggtgttgga tcttctgcag 4080

ataccctatg aaaagaatga tgaaaagatg aatgaagtaa tgaatctagc ccatacattt 4140

ctgcagaatt tctgtcgagg aaatccacag aatcaagttc ttcttcataa acatctgaat 4200

ttgtttttaa ctccaggtct ccttgaagca gaaaccatgc ggcacatctt catgaacaat 4260

taccatctgt gcaacgaaat tagcgagaga gttgtacaac actttgtgca ctgcattgag 4320

acacatggcc gccacgtgga gtacctgagg tttttgcaaa caattgtaaa agcagatggt 4380

aaatatgtga agaaatgcca ggatatggta atgacagagt tgataaatgg gggtgaagac 4440

gtgctgatat tttacaatga tagagcatca tttccaatcc ttctccatat gatgtgttca 4500

gagagagacc gaggggatga gagtggcccc ttagcctacc acatcaccct ggtggagttg 4560

ctggcagcat gcacagaggg gaaaaatgtc tacactgaaa tcaagtgtaa ttcccttctc 4620

ccgctggacg acatagtgag ggtggtgacc catgacgact gcatccctga ggttaaaatt 4680

gcttatgtga actttgttaa tcactgttat gttgacactg aagtggaaat gaaagaaatc 4740

tatacaagta accacatttg gaaattattt gagaacttct tggtggatat ggcaagggtt 4800

tgcaacacaa ctacagacag gaaacatgca gacatctttt tggaaaagtg tgttactgag 4860

tcaataatga atattgtgag cggcttcttt aattctccct tttcagacaa tagtaccagc 4920

ctccagacac atcagccagt ttttattcag ctactgcaat ctgccttcag aatttacaat 4980

tgcacctggc caaacccagc gcagaaagcc tcagtggaat cctgtatcag aactttggct 5040

gaagtggcaa aaaatcgtgg aattgccatt ccagtggatt tggacagcca agttaatact 5100

cttttcatga agagccattc aaatatggtg cagagagcag caatgggttg gagactatca 5160

gctcgctctg ggccacgctt taaggaagct cttggagggc ctgcttggga ttacagaaat 5220

attattgaaa agttacagga tgtagtggcc tccttggagc accagttcag cccaatgatg 5280

caggctgaat tctcagtgtt ggttgatgta ttgtacagtc cagaactgct gttccctgag 5340

ggaagcgatg caagaataag atgtggcgct ttcatgtcga agttgattaa tcatacaaag 5400

aaactaatgg agaaagaaga aaaactgtgc attaaaattc ttcagacatt acgagaaatg 5460

ttagagaaga aagacagctt tgtggaagag ggtaacacat taagaaagat acttctgaat 5520

cgatacttta aaggtgatta tagtattggt gtgaatggac acctatcagg agcctactcc 5580

aaaactgcac aggtgggagg aagcttttct ggacaagatt cagataagat ggggatatca 5640

atgtcagaca ttcagtgtct gctggataaa gaaggtgcat cagaacttgt catcgatgtt 5700

atagtgaaca ccaaaaatga cagaattttt tcagaaggca ttttcctcgg cattgccttg 5760

cttgaaggag gaaatacaca aacacagtat tctttctacc agcagttgca tgaacaaaaa 5820

aagtcagaaa aattctttaa agttctctat gatcgaatga aggctgctca gaaagaaata 5880

agatcaacag tgacagttaa taccatagat ttaggtaaca aaaaaaggga cgatgacaat 5940

gaattgatga catctggtcc acgaatgaga gtaagagatt caacactaca tttaaaagag 6000

ggaatgaaag ggcaattaac agaagcttct tcagcaacat ccaaagcata ttgtgtatac 6060

agaagagaaa tggatccaga aatagacatt atgtgcacag gaccagaagc gggaaacact 6120

gaggaaaaat ccgcagagga agtaacaatg agtcccgcaa ttgccatcat gcagccaata 6180

ctgagatttc ttcagttact gtgtgagaat cacaaccggg aattgcagaa cttcttgagg 6240

aatcaaaaca acaaaacaaa ttacaaccta gtctgtgaga cccttcagtt tctggactgc 6300

atttgtggaa gtacaaccgg tggcctgggc ctgttgggtc tctacatcaa tgagaagaat 6360

gtagcgctgg tcaaccagaa cctggagagc ttgactgagt attgccaggg cccttgccat 6420

gaaaatcaga cctgtatcgc tacacatgag tctaatggga ttgatatcat cattgctttg 6480

attctgaatg acataaaccc tcttggtaaa taccgaatgg acctggtgct ccagctaaag 6540

aacaatgcat ctaaactttt gctggccatt atggaaagca gacatgacag tgagaatgca 6600

gaaagaattc tttttaacat gagacccaga gaactggtgg atgtgatgaa gaatgcctat 6660

aaccaaggat tggaatgtga ccatggggat gatgagggtg gagatgatgg tgtttctcca 6720

aaagatgttg gacacaatat ctatattctg gcccatcagt tggcccgcca caataaactg 6780

ttgcagcaga tgctcaaacc aggatcggat ccagatgaag gagatgaagc cttaaagtat 6840

tatgccaacc acactgcaca gattgagatt gtccggcatg ataggaccat ggaacaaata 6900

gtttttcctg tccccaatat atgtgaatac ctcactcgag aatccaagtg ccgtgtgttc 6960

aatacaactg aaagggatga acaaggaagt aaagtgaatg actttttcca gcaaacagaa 7020

gatctctaca atgaaatgaa gtggcagaag aaaatcagga ataaccctgc actgttctgg 7080

ttctcgaggc acatctctct ctgggggagc atttccttca acctggctgt gttcatcaat 7140

ttagctgttg ctctcttcta cccatttggg gatgatggag atgaaggtac actttctcca 7200

ttgttctcgg ttcttctttg gatagcagtt gcgatctgca catctatgct gtttttcttc 7260

tccaagcctg tgggtattcg gccgtttctt gtatcaataa tgctcagatc aatatataca 7320

ataggtcttg ggcctacatt aatacttctt ggtgcagcta atctttgtaa taaaattgtt 7380

tttctggtga gttttgttgg aaatcgtggc acgttcaccc gtgggtaccg agcagtcatc 7440

ctggatatgg cctttctcta tcacgtggcg tatgtcctgg tttgcatgct gggccttttt 7500

gtccatgaat tcttctatag cttcctgctt tttgatttgg tgtacaggga agagactttg 7560

ctgaatgtca taaaaagtgt cacacgaaat ggccgctcta ttattctaac tgcagtcctg 7620

gctctcatcc tcgtctacct gttttccatt attgggttcc tttttttgaa ggatgacttc 7680

actatggaag ttgataggct gaaaaaccga actcctgtta caggcagtca tcaagtgcct 7740

actatgactt taactaccat gatggaagca tgtgccaagg agaactgttc acccacaatt 7800

ccagcttcaa atacagctga tgaagagtat gaagatggaa ttgaaaggac gtgtgacact 7860

ctccttatgt gcattgtcac cgtgctgaac cagggcctca ggaatggcgg tggtgtgggg 7920

gatgtgctaa gaaggccatc gaaagatgag cccttgtttg ctgcccgagt ggtttatgac 7980

cttctttttt atttcattgt tatcattatt gttctgaact tgatttttgg tgttatcatc 8040

gatacttttg ctgatctcag aagcgaaaaa cagaaaaaag aagaaattct aaagacaact 8100

tgtttcatct gtggacttga gagagacaag tttgataata aaacggtttc atttgaggag 8160

cacattaagt cagaacacaa tatgtggcat tatttgtact tcatagtcct ggtgaaagtt 8220

aaagacccaa cagaatacac tggacctgaa agttatgtgg ctcaaatgat tgtggagaag 8280

aatttggatt ggtttcctcg gatgcgagcc atgtccctcg ttagcaatga aggcgacagt 8340

gagcaaaatg aaattcggag ccttcaggag aagttggaat cgaccatgag tctggtcaaa 8400

cagctgtcgg gtcagctggc ggagctcaag gagcagatga cagaacaaag gaagaataag 8460

cagagactgg gcttcctcgg atcaaacaca ccccatgtga atcatcacat gccaccacac 8520

tgataccatg gggggaagcc gtgactagcc tttcatcagt gtcctgcctg atcactgaat 8580

aaagaactga gatggagggg agtgaacagt gcctattgtt gaaaagttaa aaacaaccaa 8640

gtgccaagat gttgagtggg ttagctccga gaacaattta taactgtgtt ttcatggttg 8700

cgaagaccta acctcaaatg catctgctag aaagcgtaca tcacacattc gcaatgcatc 8760

aggaagaaaa ggcttgccca aaaggctgga gagggcaggg agcggcagga tggaaggaga 8820

cacggggcag ggagaactct cttctgctaa atcgatagga gtcagttttg tcttaaatgc 8880

tgactacagc cactgacatg gttggctgga atttctttct tttaattgtg gcatataggt 8940

ttgtgacaca agaagtcata ctttggtggc taagttttac taaggaaaat aactgaaaag 9000

attaaaagtg agagctgaaa agagaaatga taatgcttcc aaactgtagc tgtcacaggg 9060

caatttcttt atttataaca tgaagcacaa tggatttaca gctctaggaa cttagtactt 9120

tggagctttt gcctctcaca ctgacaacat aacaggatgt gattgccttc tctgggattc 9180

agacaggctc tgtcaatgtg gagcacaaaa ggagattttc atataacttg ttaaaaacat 9240

gttctaagtc atgtataggc taagatttta agaagatctg ggggaataaa aagccaacag 9300

taagccccag gaaagggttt tttgagacca tatgtatgct attaaaatat attgagatta 9360

atacatgaaa atgcttaaaa gtgataggaa ttattgagaa acttatgatg gtggcattgc 9420

cttttataaa catggagtga aagccatttg actcaacgtt tgctgtgctt aaagaattgc 9480

ttcaggaccc gagcgttttt atgtatgctg ttcctgcagt taggaaaaaa aaatccaaga 9540

aatgtattga atactcaaga aaatgccaca tttcaattac atatttaaaa ctgtatctgt 9600

aagggctttt caaaatgtag caagataaaa atctcttatt caaattgttt ttgtattaaa 9660

tcatgcactc agaatttgtc gagggagaga ataacctggt gtgttcaggt tattcttaga 9720

gactacacat ttggaatagc agagcaaaat atggataatg aaagtgttgg gaaaaaagtg 9780

atgtgacagg gagtgaagac acttgatacc agactctggg agatactctg caagttgacc 9840

tggcctctcc cccacaggaa caaaacactg cctccagagt ctttaaattc tcagttatca 9900

acgccaaggt ttaaggtcta gatagggttt gctatagggt ttgctctgac aatttttaaa 9960

gcttttggat tgttctaaca tagcttaagc tattggttcc taaaaatccc aaatcaagat 10020

ctatgtagaa tataaaggaa gcctgaacca atccttccac atactgttaa gatgtagact 10080

tggaacaaag ctgttgggac ccagagcaat gaatttttga actgaagcta ctggtactcc 10140

ccagcaccac ctcatactaa gaattcctca ctctgacatg acagtatttt ttctgaccag 10200

gagctgaaag accctgacat tcatgatcca aagatataag aaattttgat atgtctgcat 10260

gcagcacaga actttgaacc taggaggcag tcaataaata catgagaaat tcagctgtca 10320

ttcagttact cttaattctt tataaaactt taaatgatac cacatatttg tttgtttaaa 10380

atggctttcc caaaatcaaa gtagactaaa gcagccatct ttaaaatcca ggatcatcaa 10440

tgctattaac agtatcaggt aaaaagtaca ctttaaaata ttttaatcag gcagagtttt 10500

atggatagag aatagaagag aaaggtagta aatattgaac atattccaat ataggaacct 10560

atctctgttt tagtacaaaa tatttctgac atctgaacta gaggtcaaga gaataaattc 10620

atttgtatac atctgagcaa cctgtctttc agatgataaa gtatctagcc ttttctgaca 10680

ccataatagt tcattttgta gggaataagc cattaggtgt atataattgc tttctagaaa 10740

tgacctaatg tccccaacca ctttgtagtg gcagatcact gtttcacagc atattttctc 10800

ccaaggaaag tattcaaaag agactgcaac taacaagact cttatttcat caaaatttaa 10860

atatttctga gttgtatttt taatgcctct ttcttttctg cctaaatgct tagaaattat 10920

aaagcaaaaa aaaaaaaaac agcaacaaaa aatcgaagca gacaaaaaag gcacttttca 10980

gaacatcaaa ttcctaatga agaagaggag aagataatgg ggaaatttga catttgatat 11040

aaatttatat ttgttatgtg tatgtttgtt aatgcaactg gaatatttga cttaggtgag 11100

tatcagttaa catccttgta tttatatagt gacatcaaaa taaatgcaat catcttgaac 11160

tttgatgtta agggagattt tgaaaaaata tttagttatt tcaaattcat attggttcaa 11220

aagtatcagt ttctgctgaa taatattttt aatttcaaaa gggttttgtt ccctctgtgc 11280

tccattctga ggctcaagag cttaatgcca gtatgttttc tgagttaaaa taacactttt 11340

agatgagaaa atgcttgtat catacagggc tataatataa ataaataaat tgtatgtatg 11400

caaaatttat catattttct accccttaaa aaattaagtt agaaataagc ttattttctt 11460

gcacatcaat atttttctgt tggtaaagag gacacaattt ctagtagatt gttaataaca 11520

tcaggaagat atttctttcg tcagaactaa ttgtgtgctt attcccatat tctcagctca 11580

taactccctg ttttggctgc tctctttatt ttacaattgt ttcattgcaa acagcaaggc 11640

agtatagccc acactcagcc actagccccc agtccccagg actgagaatg agtggggagg 11700

ctggggagtt ggtgagagaa ggtggagata gggatttctg cttcccagta ctctatcatt 11760

agagaaaagg aggggtaaat attagctaag aagaagaaaa aagcttattc ttcaggaccc 11820

cagtagattg gacgtaagca agtaaatatt gcacaggcat catgatgtaa tagttgatgg 11880

accccagaga tacccctcac ccatgcagag aacaaagctg tgtggaagag caactaccat 11940

aagatggtgt gttccttcca gcggctgtta aagccatcct gaataacaaa tcagctattc 12000

ctacctacag acccattcca atatgctgtg atacacgaca cggtgcaccg cagaggagac 12060

agacatcttc agcacaaaca cagatgcctg caggagcttg gcaagtcact aaattgcatg 12120

aaattgtgag gtgcacacca aatgcaggga tgggggaggc cgtgggaagc tctgtattct 12180

caaaatttga cagctaattc cggtttttag aaaatgcctg aggccagtag aggccctcta 12240

gtcactcact gctgctgttt ctgatataat tattgagaaa gctatctcac ttaatagaag 12300

aaaacacgca ctatcaaaac cagatagcct aacgtgcatg tgaaaatcga gaaagctgaa 12360

aacaaatcca ggtacccttc tctgaactgg agtgtttcca cagacttgaa taatttatga 12420

aattatcaca ccagtatttc tcatatcacc aagaagactt tctctcctgc agtagaggat 12480

tgttatattt gcctaaaaaa cacgattcca atatatgaca agggcagata atttataagt 12540

gaatgttaat aaaattggat gtgtataa 12568

<210> 54
<211> 1875
<212> DNA
<213> Homo sapiens

<400> 54
ggcgactaca actcccagca ggtcgagcag ctccgcccgc gctgattctc cattggcctt 60

ccgggggtgg ggattagatg ggaggtggcc gtggggctgc ggccgggatt tgtcccctct 120

tcggcttccg tagaggaagt ggcgcggacc ttcatttggg gtttcggttc ccccccttcc 180

ccttccccgg ggtctggggg tgacattgca ccgcgcccct cgtggggtcg cgttgccacc 240

ccacgcggac tccccagctg gcgcgcccct cccatttgcc tgtcctggtc aggcccccac 300

cccccttccc acctgaccag ccatgggggc tgcggtgttt ttcggctgca ctttcgtcgc 360

gttcggcccg gccttcgcgc ttttcttgat cactgtggct ggggacccgc ttcgcgttat 420

catcctggtc gcaggggcat ttttctggct ggtctccctg ctcctggcct ctgtggtctg 480

gttcatcttg gtccatgtga ccgaccggtc agatgcccgg ctccagtacg gcctcctgat 540

ttttggtgct gctgtctctg tccttctaca ggaggtgttc cgctttgcct actacaagct 600

gcttaagaag gcagatgagg ggttagcatc gctgagtgag gacggaagat cacccatctc 660

catccgccag atggcctatg tttctggtct ctccttcggt atcatcagtg gtgtcttctc 720

tgttatcaat attttggctg atgcacttgg gccaggtgtg gttgggatcc atggagactc 780

accctattac ttcctgactt cagcctttct gacagcagcc attatcctgc tccatacctt 840

ttggggagtt gtgttctttg atgcctgtga gaggagacgg tactgggctt tgggcctggt 900

ggttgggagt cacctactga catcgggact gacattcctg aacccctggt atgaggccag 960

cctgctgccc atctatgcag tcactgtttc catggggctc tgggccttca tcacagctgg 1020

agggtccctc cgaagtattc agcgcagcct cttgtgccga cggcaggagg acagtcgggt 1080

gatggtgtat tctgccctgc gcatcccacc cgaggactga gggaacctag gggggacccc 1140

tgggcctggg gtgccctcct gatgtcctcg ccctgtattt ctccatctcc agttctggac 1200

agtgcaggtt gccaagaaaa gggacctagt ttagccattg ccctggagat gaaattaatg 1260

gaggctcaag gatagatgag ctctgagttt ctcagtactc cctcaagact ggacatcttg 1320

gtctttttct caggcctgag ggggaaccat ttttggtgtg ataaataccc taaactgcct 1380

tttttctttt tttgaggtgg ggggagggag gaggtatatt ggaactcttc taacctcctt 1440

gggctatatt ttctctcctc gagttgctcc tcatggctgg gctcatttcg gtccctttct 1500

ccttggtccc agaccttggg ggaaaggaag gaagtgcatg tttgggaact ggcattactg 1560

gaactaatgg ttttaacctc cttaaccacc agcatccctc ctctccccaa ggtgaagtgg 1620

agggtgctgt ggtgagctgg ccactccaga gctgcagtgc cactggagga gtcagactac 1680

catgacatcg tagggaagga ggggagattt ttttgtagtt tttaattggg gtgtgggagg 1740

ggcggggagg ttttctataa actgtatcat tttctgctga gggtggagtg tcccatcctt 1800

ttaatcaagg tgattgtgat tttgactaat aaaaaagaat ttgtaattgt gaaaaaaaaa 1860

aaaaaaaaaa aaaaa 1875

<210> 55
<211> 3205
<212> DNA
<213> Homo sapiens

<400> 55
ggcaggccaa tatggcttcc tgcacctggt gacgcttggc gaaactgagg tctcatggag 60

aagccccgga gtattgagga gaccccatct tcagaaccaa tggaggaaga ggaagatgac 120

gacttggagc tgtttggtgg ctatgatagt ttccggagtt ataacagcag tgtgggcagt 180

gagagcagct cctatctgga ggagtcaagt gaagcagaaa atgaggatcg ggaagcaggg 240

gaactgccga cctccccgct gcatttgctc agccctggga ctcctcgctc cttggatggc 300

agtggttctg agccagctgt ctgtgagatg tgtggtatcg tgggtacaag ggaagccttc 360

ttctccaaga ccaagaggtt ctgcagcgtc tcctgctcca ggagctactc ctccaactcc 420

aagaaagcca gtatcttggc taggttacag ggaaaaccac cgaccaaaaa agccaaagtc 480

ctgcacaagg ctgcctggtc tgccaaaatt ggagccttcc tccactctca agggacagga 540

cagctggcag atgggacacc aacaggacaa gacgctctgg tcttgggctt cgactggggg 600

aagttcctga aggatcacag ttacaaggct gctcccgtca gctgtttcaa gcacgtccca 660

ctctatgacc agtgggagga tgtgatgaaa gggatgaagg tggaggtgct caacagtgat 720

gctgtgctcc ccagccgggt gtactggatc gcctctgtca tccagacagc agggtatcgg 780

gtgctgcttc ggtatgaagg ctttgaaaat gacgccagcc atgacttctg gtgcaacctg 840

ggaacagtgg atgtccaccc cattggctgg tgtgccatca acagcaagat cctagtgccc 900

ccacggacca tccatgccaa gttcaccgac tggaagggct acctcatgaa acggctggtg 960

ggctccagga cgcttcccgt ggatttccac atcaagatgg tggagagcat gaagtacccc 1020

tttaggcagg gcatgcggct ggaagtggtg gacaagtccc aggtgtcacg cactcgcatg 1080

gctgtggtgg acacagtaat cgggggtcgc ctacggctcc tctacgagga tggtgacagt 1140

gacgacgact tctggtgcca catgtggagc cccctgatcc acccagtggg ttggtcacga 1200

cgtgtgggcc acggcatcaa gatgtcagag aggcgaagtg acatggccca tcaccccacc 1260

ttccggaaga tctactgtga tgccgttcct tacctcttca agaaggtacg agcagtctac 1320

acagaaggcg gttggtttga ggaagggatg aagctggagg ccattgaccc cctgaatctg 1380

ggcaacatct gcgtggcaac tgtctgtaag gttctcctgg atggatacct gatgatctgt 1440

gtggacgggg ggccctccac agatggcttg gactggttct gctaccatgc ctcttcccac 1500

gccatcttcc cggccacctt ctgtcagaag aatgacattg agctcacacc gccaaaaggt 1560

tatgaggcac agactttcaa ctgggagaac tacttggaga agaccaagtc gaaagccgct 1620

ccatcgagac tctttaacat ggattgccca aaccatggct tcaaggtggg catgaagctg 1680

gaggccgtgg acctgatgga gccccggctc atctgtgtgg ccacggtgaa acgagtggtg 1740

catcggctcc tcagcatcca ctttgacggc tgggacagcg agtacgacca gtgggtggac 1800

tgcgagtccc cagacatcta ccccgtcggc tggtgtgagc tcaccggcta ccagctccag 1860

cctcctgtgg ccgcagaacc ggccacaccg ctgaaggcca aagaggccac aaagaagaaa 1920

aagaaacagt ttgggaagaa aaggaaaaga atcccgccca ctaagacgcg acccctcaga 1980

caggggtcca agaagcccct gctggaggac gaccctcagg gtgccaggaa gatctcgtcg 2040

gagcctgttc ctggcgagat cattgctgtg cgtgtgaagg aagagcatct agacgtggcc 2100

tcgcccgaca aggcttcaag tccagagctg cctgtctccg tcgagaacat caagcaggaa 2160

acagacgact gagccttcct gcctccagcc tggcttctag ctggaagcca gcccagcgtt 2220

tctctaccac caccaccatg cctccacctg actttggctt ggagactgat cctctctgtg 2280

taaattctgc ccggtgctgt gaaggctgga cggtggagga cctgctgggg tctcctggga 2340

cccgcctgtt gcttctgccc tcccctgtgg aaaggtctat atgacggccc gcctgaggcc 2400

ccagaactcg tctgtgaacc accttttcca gccagagttc ccaaagctgg aacgctagct 2460

gcctgctctt ccttaagatg gcctcccccc gacccgccac ggccctcagt tgccagggat 2520

ggggccacca ctgtcacact gtggaataca agacagtgaa ctctgtctgc ctgaacgagc 2580

catgtaaatt aagttctaga gcagctctct gagcaggata aggtcccctg acagtgagtt 2640

gtgtggtggg ggcagcctct gcctcaaaaa ttcaccaagc agaatgcctc tcagcctcat 2700

gtgttggtcc tctgctcctc ctagctcccc agggatgttg gggacccagc ttgtctcggc 2760

agctaagaag cagtgaccag gatgtggatt ttggcgacct gtgtggtggc cttgagctgc 2820

tttctgtgtt tgtgaggact gactcccatt tcctaaagga aatgcccccg gggaggacat 2880

tgggaggaag atggcctgag tgtgcacttt ggctctgcta cctgctcctg aagccccgct 2940

aaaaataatt catccaagat tcctttgtag ttaaagggtc cagttctgac tggagcctct 3000

agagagctgg gcttgtatgt tcttttggcc ttttgttcct acctaaatga agaaaccatg 3060

cctggagggg ccgtgaacac agaaccctca agacaaggat gacagagctg gaggacacat 3120

ctagctgcca ttgcaacctc actgggctcc ccagactctg tgtgtgagaa attaaacccc 3180

ctgcttgctt gaaaaaaaaa aaaaa 3205

<210> 56
<211> 2259
<212> DNA
<213> Homo sapiens

<400> 56
aatgcgcctc acccctggcg accccggaag tgggtcgggg gcttggcctc tgcccggcca 60

cagagccgga gctggaggtg ctgtcccgtc tggcggcgat ccccgggcag ggcccggggc 120

cggggtttga agatgctgaa cgtcccttcc cagtctttcc cggcccccag gtcgcagcag 180

cgtgtcgcct ccgggggcgg tagcaaggta cctttaaaac agggcagaag ccttatggat 240

tggattcgac tgaccaaaag tggaaaggat ctaacgggat taaaaggcag gttaattgaa 300

gtaactgaag aagaacttaa gaaacacaac aaaaaagatg attgttggat atgcataaga 360

ggtttcgttt ataatgtcag cccttatatg gagtatcatc ctggtggaga agatgaacta 420

atgagagcag caggatcaga tggtactgaa ctttttgatc aggttcatcg ttgggtcaat 480

tatgaatcca tgctgaaaga atgcctggtt ggcagaatgg ccattaaacc tgctgttctg 540

aaagactatc gtgaggagga aaagaaagtc ttaaatggca tgcttcccaa gagccaagtg 600

acagatacac ttgccaaaga aggtcctagt tatccaagct atgattggtt ccaaacagac 660

tctttagtca ccattgccat atatactaaa cagaaggata tcaatttaga ctcaataata 720

gttgatcatc agaatgattc ctttagagca gaaacaatta ttaaggattg tttatatctt 780

atacatattg ggctaagcca tgaggttcag gaagattttt ctgtgcgggt tgttgagagt 840

gtgggaaaaa tagagattgt tctacaaaaa aaagagaata cttcttggga ctttcttggc 900

catcccctga agaatcataa ttcacttatt ccaaggaaag atacaggttt gtactacaga 960

aagtgccagt taatttccaa ggaagatgtt actcatgata cgaggctttt ctgtttgatg 1020

ctgccaccaa gcactcatct tcaagtgccc attgggcaac atgtttacct caagctacct 1080

attacaggta cagaaatagt aaagccatat acacctgtat ctggttcctt actctcagag 1140

ttcaaggaac cagttcttcc caacaataaa tacatctact ttttgataaa aatctatccc 1200

actggactct tcacaccaga gcttgatcgt cttcagattg gagattttgt ttctgtaagc 1260

agtcctgagg gcaattttaa aatatccaag ttccaagaat tagaagatct ctttttgttg 1320

gcagctggaa caggcttcac accaatggtt aaaatactga attatgcttt gactgatata 1380

cccagtctca ggaaagtgaa gctgatgttc ttcaataaaa cagaagatga tataatttgg 1440

agaagccaat tggagaaatt agcatttaaa gataaaagac tggatgttga atttgttctc 1500

tcagcaccta tttctgaatg gaatggcaaa cagggacata tttcaccagc tcttctttct 1560

gaatttttga aaagaaattt ggacaaatcc aaagttctcg tctgcatttg tggaccagtg 1620

ccatttacag aacaaggagt aaggttgctg catgatctca acttttccaa aaatgagatc 1680

catagtttta cagcataatg aagagctgtc attgtccttt attcaactag tttatctaaa 1740

tttgtgattg cttagggttt tttaagagaa catttttgta cataacaaaa ggttaactag 1800

aatccagcct tcagtttctt aaatgaaatc aaatgttcct tcagtacagg taacttcttg 1860

gctttctttt gtaccacaac ttattttact actgatattt gacctggaaa gttaatcatg 1920

gcaacaaata catacaggat tctttgttat gaatcacaaa tttccttgcc atttaaatta 1980

tatcactgtt ctacaataag cacttgtgtt ttatgacatg ataaagtaaa tgatcacttc 2040

gatcatgttt ctatgctgta aaatgtctta ttagcaatac agattaaatt ttaccttgca 2100

ctgttaactc aggaaatgat catttatgtc cttgttatta atgttaaaac atagaatgtt 2160

ataacttatt aagtgatcca aacatttttt tgtgtgtgta tggcattgat gcagaataga 2220

ataaaattat acttaagttc tttttaaaaa aaaaaaaaa 2259

<210> 57
<211> 4332
<212> DNA
<213> Homo sapiens

<400> 57
accgtcagcg cagggctcgc cgggaaatgt ggtttctcca gccggcccgg ggcggtggcc 60

gcaagttggg cttacagcgc ggccgatccg gcgtggaccc gggatggctg gaccgggcag 120

cacggggggg cagatcgggg ctgcggccct ggcaggcggc gcgcggtcca aggtagcccc 180

gagcgtggac ttcgaccata gctgctcgga cagtgtcgag tacctgacgc tcaacttcgg 240

gcccttcgaa acagtgcatc gctggcggcg cctcccgccc tgcgacgagt tcgtgggtgc 300

ccggcgcagc aagcacacag tggtggccta taaagatgcc atttatgtat ttggtggaga 360

caatgggaag accatgctca atgacctcct gcggttcgat gtgaaagact gctcctggtg 420

caggggcttt accactggga ccccaccggc cccccgttac caccactcgg ccgtcgtcta 480

tgggagcagc atgtttgtct ttgggggtta cactggggac atttattcca attctaactt 540

gaagaataaa aacgacctct ttgaatacaa gtttgcaact ggccagtgga cggagtggaa 600

aattgaagga cggttgccag tcgctaggtc agcccatggg gccacggtgt acagtgacaa 660

gctgtggatc tttgctggct atgacggcaa cgccaggttg aatgacatgt ggacaattgg 720

cctccaggac cgagagctca cctgctggga ggaggtggcc cagagtggcg agatcccccc 780

atcttgctgc aacttccccg tggctgtgtg ccgggacaag atgtttgtat tctctgggca 840

aagcggagcc aaaataacca acaacctctt ccagtttgaa ttcaaggaca agacgtggac 900

acgcatccca actgaacacc tgctccgggg ctccccacca cccccgcagc ggcgctacgg 960

gcataccatg gtggcctttg accgccacct ctatgtgttt gggggtgcgg ccgacaacac 1020

gctgcccaac gagctgcact gctatgacgt ggacttccag acctgggagg tcgtccagcc 1080

cagctccgac agcgaggttg gtggggctga agtgcccgag cgagcctgtg cttccgagga 1140

ggtgcccacc ctgacctatg aggagcgggt tggcttcaag aagtcccgag atgtgtttgg 1200

cctggacttt ggcaccacct cagccaagca gcccacccag cctgcctcgg agctgcccag 1260

tgggaggctc ttccacgcgg ctgctgtcat ctcggacgcc atgtacatct tcgggggcac 1320

ggtggacaac aacatccgca gcggggagat gtacaggttc cagttctcct gttaccctaa 1380

atgcacgctg cacgaggact acgggcggct gtgggagagc cgccagttct gcgacgtgga 1440

gttcgtgctg ggtgagaagg aggagtgcgt gcagggccac gtagccattg tcacagcgcg 1500

gagccgctgg cttcgcagga agatcacgca ggcgcgggag aggctggccc agaagctgga 1560

gcaggaggcc gccccagttc ccagggaggc ccccggcgtg gctgctggtg gggcccggcc 1620

gcccctgctg cacgtggcca tccgggaggc cgaggcccgg cccttcgagg tgctcatgca 1680

gttcctctac accgacaaga tcaaataccc acggaaaggc catgtggagg atgtgctgct 1740

catcatggat gtgtacaaac tggcactgag cttccagttg tgccgcctgg agcagctgtg 1800

ccgccagtac atcgaggcct ccgtggacct gcagaacgtg ctggttgtgt gcgagagtgc 1860

cgcccggctg cagctgagcc aactcaagga gcactgcctg aacttcgtgg taaaggagtc 1920

ccacttcaac caggtgatca tgatgaagga gttcgagcgc ctctcctctc cactgatagt 1980

ggagattgtg cggcggaagc agcagccgcc ccctcgcact cccttggacc agccagtgga 2040

cattggcaca tctctgatcc aggacatgaa ggcatacctg gagggagcgg gcgcggaatt 2100

ctgtgacatc actctgttgc ttgacgggca cccacggcca gcccacaagg ctatcctggc 2160

cgcccgctcc agctactttg aagccatgtt ccggtccttc atgcccgaag atgggcaggt 2220

gaacatctcc atcggggaga tggtgcccag caggcaggcc ttcgagtcca tgctgcgcta 2280

catctactac ggcgaggtca acatgccgcc cgaggactcg ctctacttgt ttgcggcccc 2340

ctactactac ggcttctaca acaaccggct gcaggcgtac tgcaagcaga acctggagat 2400

gaacgtgacg gtgcagaacg tgctgcagat cctggaggca gctgacaaaa cgcaggcact 2460

ggacatgaag cggcactgcc tgcacatcat tgtgcaccag ttcaccaagg tctccaagtt 2520

gcccaccctg cggtcgctga gccagcagct gctgctggac atcatagact ccctggcctc 2580

ccacatctca gacaagcagt gcgcagagct gggcgccgac atctgaggcc ctgtggcgcc 2640

tgcccattgt gaagaatcgc cgtgcctgcc tgccctgcct actgagaaga ctaccggcta 2700

tgcgcatgcc tatggcagtg ggtgcacctg ccaggccaag ggtcagggtg cccagagcct 2760

ccaaagagag ctgaggggat gtggggcccc aaactcatta attcactgaa gacacaggtc 2820

ccacagggag cggatgatga agcagacccc ctcctgtcat caccctctcc tggtgtagtg 2880

tggatgcgag gccacggctc agtgatgggc tcaccaccca gaagtgggga gagactttgg 2940

gcctcccacc cagtggggct tggcctggct tctgtggcct gggcgtgttg tggactcagg 3000

cactggggcc tgtcaccaag gctcctccaa catgcgggag gaggcttagc agacttgcgc 3060

tgcaccagcg aatctgcctg ggctgctcct gtcccaccca ccctcactga gatccatgta 3120

aggggctcct cttcccacct ggaacttgtg agtggggacc catgatgtat gggtctcacc 3180

tgacttgagg tgaattttgg agtgaagggc cctgaggtca gctcccaggt cggtcgtgct 3240

gggccaggcc tggttttcac aggggctgaa ggatcccagt ccacctgtgt gcatgtcagg 3300

gctcggccgg gaagaagcca gcaaagtccc ccgtgtccct tgctgagtat tctgtcacag 3360

acaagcctcc attaaagcca cagcagtgct acccaccaca cacaccttgc tggcccggcc 3420

accactgctg gcttcagccc cttgagcagc ccatggctta gcagaccccc agatgtaggt 3480

cagtggcctt acctgtctct atccatgctg tcaactcctg cctccacctg ggtcacccca 3540

gtcacattgg gaagggctgt gaaggcctcc aggctggccc cttccagggg aatcctggag 3600

gcctggggtg ggctcctgcc ccttctgccc tgccttgccc ctgcactatg ctcttggctc 3660

ctgtggaagg agggctgccc tcttgcccta gtgagggccc catgtggatc cactctagtg 3720

ctgggagcca gcgctccctt actggaacag gattccagg accccttct tgttgtggct 3780

gccatgaagc cacagctcct tggggaagtg acctgctctc ctttgggtgt atgcaggtgt 3840

gtgggggccc ctgagtggca agttgcttag ctaacaggag atccataggc agcctgcagg 3900

ctaggaagtg gcctagtgca agatgagctg ggaacaaggg aggagagagc aggagctggg 3960

gcagaggctg agccgggagg cccttgaggt gaggacacag caggcccagg accatggctg 4020

gggaggatat gtcagcacct ggaagtggag tgcaggctgc agcgcccagc catgtgggcc 4080

aggtgcattc actcagagtg gggccacaca cccatctacc cagtttccac aagatgtggc 4140

tcctgccaca cccacagggc agcctcctcc aaatccctcc tggaggggcc tacccagaag 4200

cctccttgaa ccagtctgca accctgctct atgctgaccc ttgtcactga accctgatct 4260

agacttatat gaataaatga aattacatgc caagggccct aaaaagcaaa ttttaaaaaa 4320

aaaaaaaaaa aa 4332

<210> 58
<211> 3824
<212> DNA
<213> Homo sapiens

<400> 58
actgcctctc tcacagccct caagacacac catgggccca gaggcaggtt tgctacacag 60

cagcgacgac gcaggcggcg gccccagcga ctcgcaactg cctccctgac cacagcggcc 120

accgcccaac acccccgaga agccatcgcc accaccggca ggagaaccta gggtccataa 180

agccatcttc gcgatcgact aaagctacgt caacaactat ggcgggcgac gggcggcggg 240

cagaggcggt gcgggaagga tggggtgtgt acgtcacccc cagggccccc atccgagagg 300

gaaggggccg gctcgcccct caaaatggcg gcagcagcga tgcgcctgcg tacagaactc 360

ctccgtcgcg ccagggccgg cgggaagtga ggttctcgga cgagccgcca gaagtgtacg 420

gcgacttcga gcccctggtg gccaaagaaa ggtccccggt gggaaaacga acccggctag 480

aagagttccg gtccgattct gcgaaagagg aagtgagaga aagcgcgtac taccttcggt 540

ctaggcagcg gaggcagccg cgaccccagg aaaccgagga aatgaagacg cgaaggacta 600

cccgccttca gcagcagcac tcagagcagc ctccgctaca gccgtctcct gttatgacca 660

ggagagggct gcgggactct cattcctctg aagaggatga agcatcttcc caaactgatt 720

taagccaaac gatctcaaag aaaactgtca ggagcataca agaggctcca gtgagtgaag 780

atcttgtaat caggttacgt cgaccccctc taagataccc aagatatgaa gccaccagtg 840

tccaacagaa ggtcaatttc tctgaagaag gagaaactga agaagatgat caagacagct 900

ctcacagcag tgtcactact gttaaggcca gatccaggga ttctgatgaa tctggagata 960

aaaccaccag atcatctagt caatatatag aatcattttg gcagtcatca caaagtcaaa 1020

acttcacagc tcatgataag caaccttcag tgctaagctc aggatatcaa aaaactcccc 1080

aggaatgggc cccacaaact gcaagaataa ggaccaggat gcaaaatgac agcattctga 1140

aatcagagct tggaaaccag tcaccatcaa cctccagccg acaagtgact ggacaacccc 1200

aaaatgcatc ttttgtcaag aggaaccggt ggtggctact tcctctgata gctgctcttg 1260

cctctgggag tttttggttc tttagtactc ctgaggtaga aaccactgct gttcaagagt 1320

tccagaacca gatgaatcaa cttaagaata agtaccaagg tcaagatgag aagctgtgga 1380

aaaggagcca aacattcctg gaaaaacatc ttaatagctc ccatcctcgg tctcagcctg 1440

ctatcttact gctcactgct gcccgagatg ctgaagaagc acttaggtgt ctgagtgaac 1500

aaattgctga tgcctattct tcttttcgta gtgtccgtgc catccggatt gatgggacag 1560

ataaagctac tcaagacagt gatactgtca aactagaggt agaccaagaa ctgagcaatg 1620

gatttaagaa tggccagaat gcagctgtgg tacaccgctt tgagtcattt cccgcaggct 1680

ctactttgat cttctacaaa tattgtgacc atgaaaacgc ggccttcaaa gatgtagcct 1740

tagtcctgac tgtcttattg gaggaagaga cacttggaac aagtctaggc ctaaaggaag 1800

ttgaagaaaa agtaagagat tttcttaaag tcaagttcac caattctaac acacccaact 1860

cctacaatca tatggaccca gacaaactga atggcctctg gagccgtatt tctcacttag 1920

ttctgcctgt gcaacctgaa aatgccctga aaaggggcat ctgcttataa gaagtgagag 1980

agaagaaaaa tcatgtccca agttctgaga attgttcaca ctttctaacc agagacagaa 2040

ttcagagctc tttttgaaag aactagtttc tttttaaaga agttaagtgc ttacataaac 2100

atggaacata taaatcattc attcaaccta attatctgtg atatgagaga atcatttcag 2160

tttccattga gagctctgtt aaaggtatct taggagtgca gattatatgc agttccttag 2220

agaatctgtt ttgattctgg gctgagttat tacagttatg gtatgaccag tgagagggat 2280

ttgtgtcctt tcttatgaac cttcctgatt ttttaactta gatttctcac taagtttcct 2340

gagttattag taagattgct ccctaaatgt aaccagggat tttcccatta tgttcccttt 2400

tataccattt aggtgtgagt tccttagctt ctgcctatag gaacataagt aatgaaaggt 2460

catctaggtg tgtgtttgga atttttttct tttgtttttt ggaaaatgga aagaacaagg 2520

caaggaagga aaattaatgg ggaagctgaa gggaggaaat gttacagtaa tccactgaga 2580

tgtagtttag tcaaacatag gtatatgaac tgttaatctt ggtggattcc gttgggattt 2640

gttttttaca tctccttttt ccttccttga agaaaaattc ttggctgtcc caaggattct 2700

tcatgtatat tgcaaaattt aatatatttg ttcacttgag tcttaagagt aggtcaggcc 2760

aaggcaggtg gatcacctga ggtcagaagt tcaagaccag cctggccaac atggtgaaac 2820

cccgtctcta ctaaaaatac aaaaattagc tgggcgtggt ggtgcatgcc tgaaatccca 2880

gctacttggg aggctgaggc aggagaattg cttgaacccg ggaggcggag gttgcagtaa 2940

gccgagacca tgcttattgc cctccagtct gggcaacaaa aatgaaactc cgtctcaaaa 3000

aaaaaaaagt aggtcaattc gtaacagttt attttggaca caccctgaag cttttgtttt 3060

gaattaagaa atgagtttca ggaattgaca aaccatttgt taaccagctg tcctgggcta 3120

ttgaagaaga tttcattttc cgcgcatcca cttgttgcag tccaagtcct ctagtgcaac 3180

gccatagcaa atataaagat ttactatgca cgtgatacat tttatggagt gcctcaagcc 3240

aagatagtga atttatatga agggtgtgaa atgtatttct ttactactat agtagtttag 3300

ttataacttt gcatctataa ttgagctttc tcatctgtca aatgctatgg ttttcttaaa 3360

atgttacaat tctagatcta tccaccttgt ttttttattg tctacaaacc attaaatgta 3420

aatattgttt ttagagtcag tcattggctt tgtcatttac cctttgagag ttccacaagt 3480

ggtagtagag tggtttaacg tctttcctct agtactacca gtattcataa atgtataccc 3540

cttactgtaa tttgttcctc ttagaagtca gatcatctga tttatagagg attatgaaaa 3600

ccagagtttt tgaaaaggct tattttatac atacgtatta tatagagaaa caaatgtttt 3660

tattaaatgt ttcattgacc caagtaattt aaaagtaata tgttacctat gtctttcagg 3720

aaaaataatt atagcagctg atctgtaaat gactttaaaa gctattttag taatttaaat 3780

aaatttactt tcttggttta tactctcaaa aaaaaaaaaa aaaa 3824

<210> 59
<211> 3767
<212> DNA
<213> Homo sapiens

<400> 59
aagaaagagc cccgccccta gtcttatgac tcgcactgaa gcgccgattc ctggcttttg 60

caaggctgtg gtcggtggtc atcagtgctc ttgacccagg tccagcgagc cttttccctg 120

gtgttgcagc tgttgttgta ccgccgccgt cgccgccgtc gccgcctgct ctgcggggtc 180

atggtgtgct tccgcctctt cccggttccg ggctcagggc tcgttctggt ctgcctagtc 240

ctgggagctg tgcggtctta tgcattggaa cttaatttga cagattcaga aaatgccact 300

tgcctttatg caaaatggca gatgaatttc acagtacgct atgaaactac aaataaaact 360

tataaaactg taaccatttc agaccatggc actgtgacat ataatggaag catttgtggg 420

gatgatcaga atggtcccaa aatagcagtg cagttcggac ctggcttttc ctggattgcg 480

aattttacca aggcagcatc tacttattca attgacagcg tctcattttc ctacaacact 540

ggtgataaca caacatttcc tgatgctgaa gataaaggaa ttcttactgt tgatgaactt 600

ttggccatca gaattccatt gaatgacctt tttagatgca atagtttatc aactttggaa 660

aagaatgatg ttgtccaaca ctactgggat gttcttgtac aagcttttgt ccaaaatggc 720

acagtgagca caaatgagtt cctgtgtgat aaagacaaaa cttcaacagt ggcacccacc 780

atacacacca ctgtgccatc tcctactaca acacctactc caaaggaaaa accagaagct 840

ggaacctatt cagttaataa tggcaatgat acttgtctgc tggctaccat ggggctgcag 900

ctgaacatca ctcaggataa ggttgcttca gttattaaca tcaaccccaa tacaactcac 960

tccacaggca gctgccgttc tcacactgct ctacttagac tcaatagcag caccattaag 1020

tatctagact ttgtctttgc tgtgaaaaat gaaaaccgat tttatctgaa ggaagtgaac 1080

atcagcatgt atttggttaa tggctccgtt ttcagcattg caaataacaa tctcagctac 1140

tgggatgccc ccctgggaag ttcttatatg tgcaacaaag agcagactgt ttcagtgtct 1200

ggagcatttc agataaatac ctttgatcta agggttcagc ctttcaatgt gacacaagga 1260

aagtattcta cagctgaaga atgttctgct gactctgacc tcaactttct tattcctgtt 1320

gcagtgggtg tggccttggg cttccttata attgttgtct ttatctctta tatgattgga 1380

agaaggaaaa gtcgtactgg ttatcagtct gtgtaatcag ttaaatctag tgtttgtttg 1440

tttttttcaa ttagaagtta cgtttccatt ggctaaaagc caggacatgc tgtgcaatag 1500

attgtttaag atatgcagac taacttcagt gagttcctag ctaacttggg catgagtaca 1560

cttatttaag acaaaatata ttaggaccaa tttttttctg ttttttttct tcctttgtta 1620

aagtataatt aaaagaaaaa ttgtggctta gaatttttta agtaaataat gattttaagc 1680

ccctggatcc aattatgaaa gcatttttgc tgatgtgtaa ttttatatgt tacagttact 1740

tatattttac tactttgatg ttatttgcaa aatcaaaggt gttaaagaat ttaacttgct 1800

tcaggaaata aattcaagaa catagtggat tcattttcat tggtggcaga cacgaaattt 1860

ggttcatgat aagacttcct ttccccacct cctgatcagc attatttaaa tctgtatttt 1920

tctgttagtt aagaaagaaa tggcttcatg atattgtatt taatagcaaa agtttggctg 1980

tcttcttcat tactgttaat agctactata ttttaacaag gagatttctt tttttgttgt 2040

tgttgttcta gagtttggaa tatactgatt atctcagact tgacatttat actgaaggat 2100

gaagtaagac ctccagcttt ttttaaaaaa ggtgttgatt tggaacacct gtatgggtta 2160

tggtttatta aggttatggt ttagaaagtt tttttccctc agagccttaa cttgttaaga 2220

aggttcattt atcctgcact gaaaacaaaa actctatata ctttgtttgt gtgcctcctg 2280

cactctccca ttccctatgt gaatatgctc tagttgatat ttttaatata ttgatttctt 2340

ttttctcaca gcaacaagtg cttactctag aggttagtgg gccctgatat gtcatcagtc 2400

agatgcctgc ctagccaaag ctggactaag attattctgt acatttgttg atcttgatat 2460

agacttatat ccctgtaggg actgctaatg gctccggctt ctggagtaag gtactggaga 2520

ccactcatcc ctgtgtctgc ttgattggtt cagctgttga attgcccttt tatttggaag 2580

cagtgttgaa gttgtctagg gttcaaatgg ctgctttgta cacctgtcat tagtataagg 2640

cagatgttta ttttatcaag ctattttatc tctacattta actaaaaaca aaagttccca 2700

aagatctgcc ttcacttcag aaattttttt tggattaaaa aaattaagcc tgaaccttaa 2760

ataaagtgag ttggttattc attccaagga ttaagtccca atctacctct cagcacaatg 2820

cagaagctca ccactgtatt gctgccatta actcatgcca gaaccctttg ccaataactg 2880

gaattacaaa tttttgttaa agaaaattta tcaagatctt tctttactgc cttctctata 2940

tgtacatctc aaaaacatgt acatctcaaa aactggagta gaaagttaga ttgctcaact 3000

acaactcctc tagaactcta tagctctgac atacagattc acactctcct ctatttgcta 3060

agtatgtaaa gaatgttttc ttttaaaatg ttctcttttg agaacaactg cttatttgtt 3120

ataaaagcat ttggttaaaa tgatgtcatc ataaaaaaca gtggctttgt ttcaatacat 3180

atttttgaga tgattatcta gaagccagat taataaaatc agcttgtgac cttgctaagc 3240

atataaactg gaaattcaga tacattcaaa attatgggtt catttaaaag tgttctacct 3300

tttgggtatg agactaatat cactaattcc tcaatagtta tcatggctct atcttaatta 3360

attagaaaat atgtgtgttt aattctttga gaattaaaat agagaatatt aacagagggt 3420

taaaaactgc ttcaactcca ataagataaa ggaagctcaa aatctatgag ctgagtgttc 3480

aattagcttt gcctactgag ttcaatttta tgtcaataca acagtggatc agacagtacg 3540

actttgaact ggtgaatgta aacaattgtt tttcacctaa gctgctttgg aagaactgat 3600

gcttgctgct aactaaagtt ttggatgtat cgatttagag aaccaattaa tacctgcaaa 3660

ataaagcata ctgtggtact tctgtttgat ctagtatgtg tgattttaga ttgatggatt 3720

aaaaattaat aaagatcata cattccatac caaaaaaaaa aaaaaaa 3767

<210> 60
<211> 3126
<212> DNA
<213> Homo sapiens

<400> 60
agcgcgcgca ggggctgcgg agcggcagga tgcaggagga gctggcgtgg gagaccgacg 60

gcctgcttcc tctggagagg cagctccacg aggccgcccg ccagaacaat gtcggcagga 120

tgcaggagct gattgggagg agggttaaca ccagggccag aaaccacgtg ggcagggtgg 180

ccctgcactg ggctgcaggt gcagggcacg agcaggctgt gcgtctgctt ctggagcacg 240

aggctgctgt ggacgaggag gatgcggtag gggccctcac agaggcacgt ctgtgttttg 300

ggatgaatgc gcttctcctg tctgcctggt tcggccactt acgaatcctc cagatcttgg 360

taaactcagg ggccaagatc cactgtgaga gcaaggatgg cctgacctta ctgcactgcg 420

cagcccaaaa aggccatgtg cctgtgctgg cgttcataat ggaggacctg gaggatgtgg 480

ccctggacca cgtagacaag ctggggagga cggcgtttca cagggcagct gagcacgggc 540

agctggatgc tctggacttc ctcgtgggct ctggctgtga ccacaatgtc aaagacaagg 600

aggggaacac tgcccttcat ctggctgctg gtcggggcca tatggctgtg ctgcagcgac 660

ttgtggacat cgggctggac ctggaggagc agaatgcgga aggtctgact gccctgcatt 720

cggctgctgg aggatcccac cctgactgtg tgcagctcct cctcagggct gggagcaccg 780

tgaatgccct cacccagaaa aacctaagct gccttcacta tgcagccctc agtggctcgg 840

aggatgtgtc tcgggtcctc atccacgcag gaggctgcgc caacgtggtt gatcatcagg 900

gtgcctctcc tctgcacctc gctgtgaggc acaacttccc tgccttggtc cggctcctca 960

tcaactccga cagtgacgtg aatgccgtgg acaataggca gcagacgccc cttcacctgg 1020

ctgcagagca cgcctggcag gacatagcag atatgctcct cattgctggg gttgacttaa 1080

acctgagaga taagcaggga aaaaccgccc tggcagtggc tgtccgcagc aaccatgtca 1140

gcctggtgga catgatcata aaagctgatc gtttctacag atgggagaag gaccacccca 1200

gtgatccctc tgggaagagc ttgtccttta agcaggacca tcggcaggaa acacagcagc 1260

tccgttctgt gctgtggcgg ctggcctcca ggtatctgca gccccgtgag tggaagaagc 1320

tggcatattc ctgggagttc acggaggcac atgtcgacgc catcgagcaa cagtggacag 1380

gcaccaggag ctatcaggag cacggccacc gaatgctgct catttggctg catggcgtgg 1440

ccacggctgg tgagaacccc agcaaagcgc tgttcgaggg cctcgtggcc attggcagga 1500

gggacctggc tggctggagt acaatggcga gatctcagct cacggcaacc tccgcctccc 1560

gggttcaaat gattctcgtg cctcagcctc ccgagtagct gggattacag gtgcatgcca 1620

tcacagctgg ctaatttttg tatttttagt agagatgggg tttggccatg atggccaggc 1680

tggaaaattg aaacataatt tcacaattat tcctttttcc accttaaata ataagagtag 1740

aatactttct gtgtttttat cttatacaca tgaataaatg ctatggctta tcacagttac 1800

aatgtgtttt ctgaaagtaa agattatttt accttgaaat tacaaaaatt attttcagtt 1860

ttccaaaatt ctatcatctt taaacctaaa taattcaatt tcatggatgc acaaatgttt 1920

attgagagtc tcatattcat gcttttcttc acagcactat aaagttggac ttggaaaatt 1980

tggacagcca tttgccattg taatttttat tctttttctt cctgattatt tgacaaaact 2040

tgtatccaca ttgtagctgt tcatgtgtct gcttctattg catattgtaa aattattaac 2100

tacttcccaa aatagtattt ctctcagcag atatttcttt ggtactacca tgtattgtgt 2160

aacttttggg aaagtaaggt ggcttccctg ctctcagtga agcattttat taaaagaata 2220

attataatta aaaaaacacg atacagatca gtgcatatag tatattacta atagtatgct 2280

gttaattgtg taagaaagaa aggaaattag aaaacatatt tgcataaata aaagctagac 2340

aaaggctaag aaactaatca ggtggtttcc ctggggtagt aggataatgg ggaacagtgg 2400

ggctgggacc gggtgggaga aagagttctc agtggttcct ttctatacag ttttaacttt 2460

tgaaatgtgt gagagtgtta cttactgaaa actgaaagga tatgacatgc tggaacaggc 2520

aaaactacaa agacagtaaa aagatcagtg atttccagga gctgcaaggg cagtgagagg 2580

aagggaggga tgaggaggtg gagtgcaggg gatgttaggg cagtgaaagg ttctgtatga 2640

cactgtgatg gtgggtacat gacattgggc attggtcaaa acccatagca tgtacagcac 2700

aaagcgtttg ctctaatgtg agctatggac tttagttaat agtaacgtgt caatattggt 2760

tcatcagttg tatcaaatgt accacactga tgccagatgt taataatcgg ggaaactgtg 2820

tgtgctgaag agtacgtggg agctctctgt gctatctgct caatttttct ctaaacctaa 2880

aactgttcta aaatagaaag tctgtgaaaa aaaacaaaag taaagttata aaaggaaaag 2940

aaaacagagg ctttaaatta aatcatctat tccttgggaa aggtattttg taccatatgg 3000

gactgaaatc atttctctgg atgaatttta taaaatgaat tttgtaattt ttttctgaga 3060

aaaaaaagtt cttaaataca ataaaattga aatgttgaaa tatatctctc aaaaaaaaaa 3120

aaaaaa 3126

<210> 61
<211> 4467
<212> DNA
<213> Homo sapiens

<400> 61
cgagactcag attgtctgaa ttgagctatc gcaacttaat gctaaaagct ccttaaagct 60

acagatttat gacatagttc cttccaaaat attacatcat aaatcattga gaagattaaa 120

aaaaaacact tgaagaaatt gtagttttaa acatctctgc atatattttg gatagctact 180

aggttacttt aactgtcatt aaggagcaca gacttactga agctttactg gacagaatcc 240

tgggaaatcg atatcattat aaggttatat ttcccagtta gcgggtgaag ggctggagac 300
cttattgcag tcatggcttt cacaaattac agcagtctga atcgagctca gctaaccttt 360
gaatatctgc acacaaattc gtaagtatcc tctaggtgcc actgaggtaa ccagtaactc 420
gttccttgat attatatgga aatcgtttcc ccagaaaatt ttgctttttc actttttgag 480
atgtatccca ctggagtgaa atgtgtcact ggatatcttg agctctgtat tgaagaactg 540
agatcagtga aatacttgtt gctaatccag aagaatctga tttttgttta ttggatcaaa 600
attttctaaa tgcaaacttt agttatttga agtcaatatg ttgagttgtt tcattcaagt 660
gtttatagga atccaacaaa tactgctcta ttggatcgcc aaatgttgga ctattttagt 720
atcaaccgtt tcccctctgt agtgacaacg tcctaaacag ttaggtttat aacaagtgtt 780
tactttctaa caagaaaaca gaagacattt aaatgacaac tttcaagaag aaaattttta 840
ttttttcaga agttggcatt atcttcctgg cagattgctc acatccaata ttatttgtat 900
atgctaaaca ggaaacggca acttgtttat atctctattt agatagtctt tccccaaaat 960
ttccacagaa acatacagtg ttcatggttc ttgagttcat gaaggagtaa tctaatcact 1020
ccaacatggt ctggaatgtt tcaggtttaa tccatatgcc cactctcttg gaggctgtcc 1080
agtagcgtca aaactttagt gttttaatac attcacctgt tacttttgag atgaagttca 1140
cctttcttgg atcacatgca aaggatgttt aggtctgtga agaaaagaat ttctaggccg 1200
ggtgctgtgg ctcacgcctg taatcccagc actttgggag gccgagaacc actcacgaat 1260
tcttgtttgg tgctcttgct gaactggttg ataatgcaag agatgctgat gccaccagaa 1320
tagatattta tgcagaaaga cgagaggacc ttcgaggagg atttatgctt tgctttttgg 1380
atgatggagc aggaatggat ccaagtgatg ctgccagtgt gatccagttt gggaagtcgg 1440
ccaagcgaac acctgagtct actcagattg ggcagtacgg gaatgggtta aaatcgggct 1500
caatgcgcat tgggaaggat tttatcctgt tcaccaagaa ggaagacacc atgacgtgcc 1560
tcttcctgtc tcgcacgttt catgaggaag aaggcattga tgaagtgata gtcccactgc 1620
ccacctggaa tgctcggacc cgggaacctg tcacagacaa tgtagagaaa tttgccattg 1680
agacagaact catctataag tactctccat tccgcactga ggaggaagtg atgacccagt 1740
ttatgaagat tcctggggac agcggaacat tggtgatcat cttcaatctc aaactcatgg 1800
ataatggaga gccagaacta gacataatct caaatccaag agatatccag atggcagaga 1860
cgtccccaga gggcacgaag ccagagcggc gctcgttccg tgcctatgcc gctgtgctct 1920
atattgatcc ccggatgagg atcttcatcc atgggcacaa ggtgcagacc aagaggctct 1980
cctgctgcct gtacaagccc aggatgtaca agtacacgtc aagccgtttc aagacccgtg 2040

cggagcagga ggtgaagaaa gcagagcacg tagcaaggat tgctgaagag aaggcgcggg 2100

aggcagagag caaagctcgg acattagaag tacgcctagg tggagacctc acgcgggact 2160

ccagggtgat gttgcgacag gtccagaaca gagccatcac tctgcgcaga gaagccgatg 2220

tcaagaagag gatcaaggag gccaagcagc gagcacttaa agaacctaag gaactgaatt 2280

ttgtttttgg tgtcaacatt gaacaccggg atctggatgg catgttcatc tacaactgta 2340

gccgactgat caaaatgtat gagaaagtgg gcccacagct ggaagggggc atggcatgtg 2400

gcggggttgt tggggttgtt gatgtgccct acctggtcct ggagcctaca cacaacaaac 2460

aggactttgc tgatgccaag gagtaccggc acctgctccg agcaatgggg gagcacctgg 2520

cgcagtattg gaaggatatt gccatcgccc agaggggaat catcaagttc tgggatgagt 2580

ttggctacct ctctgccaac tggaaccagc ccccgtccag tgagctgcgt tacaaacgcc 2640

ggagagctat ggaaatcccc accaccatcc agtgcgattt gtgtctgaaa tggagaaccc 2700

tccccttcca gctgagttct gtggaaaaag attaccctga cacctgggtt tgctccatga 2760

accctgatcc tgaacaggac cggtgtgagg cttctgaaca aaagcagaag gttcccctgg 2820

gaacattcag aaaggacatg aagacgcagg aagagaagca gaaacaactg acagagaaaa 2880

ttcgccagca gcaggagaag ctggaggccc ttcagaaaac cacacccatc cgctcccaag 2940

cagacctgaa gaaattgccc ttggaagtga ccaccagacc ttccactgag gaacctgtgc 3000

gtagacctca gcgtcctcgg tcgccccctt tacctgctgt gatcaggaac gcccccagca 3060

gacccccttc tttgccaact cctagaccag ccagccagcc ccgaaaggct cctgtcatca 3120

gcagtacccc aaagctccct gctttggcag cccgggagga ggccagcaca tctaggctgc 3180

tccagccacc tgaggcaccc cgaaagcctg ccaacactct cgtcaagact gcatcccgac 3240

ctgcccctct ggtgcagcaa ctgtcaccat ctttactgcc caactccaag agccctcggg 3300

aggttccttc tcccaaagtc atcaagactc cagtggtgaa gaagacagag tcacccatca 3360

aactctcccc ggctacccct agtcggaagc ggagtgtcgc agtttctgat gaggaagaag 3420

ttgaggagga agctgagagg aggaaggaga ggtgcaagcg gggcagattt gttgtgaagg 3480

aggaaaagaa ggactcgaat gagctctcag acagtgctgg ggaagaggac tcggctgacc 3540

tcaagagagc tcagaaagat aaagggctgc acgtggaggt gcgtgtgaac agggagtggt 3600

acacgggccg tgtcacagcc gtggaggtgg gcaagcatgt ggtgcggtgg aaggtgaagt 3660

ttgactacgt gcccacagac acgacaccaa gagaccgctg ggtggagaaa ggcagtgagg 3720

atgtgcggct gatgaaaccc ccttctccgg aacatcagag ccttgataca caacaggagg 3780

gcggggagga ggaggtgggc cctgtggccc agcaggccat agctgtcgca gagccctcca 3840

cttccgaatg cctccgcatt gagcctgaca ccactgccct gagcaccaat cacgagacca 3900

tcgacctgct tgtccagatc ctccggaatt gtttacggta cttcctgcct ccaagtttcc 3960

ccatctccaa gaagcagctg agtgctatga attcagatga gctaatatct tttcctctga 4020

aggagtactt caagcaatat gaagtagggc tccaaaacct gtgcaattcc taccagagcc 4080

gtgctgactc ccgggccaag gcctccgagg aaagcctgcg cacctccgag aggaagctcc 4140

gcgagacgga ggagaagctg cagaagctga ggaccaacat cgtggcactc ctgcaaaagg 4200

tgcaggagga catagacatc aacacagatg atgagctgga cgcctacatt gaggacctca 4260

tcaccaaggg ggactgaagg caggagagag agcagctccc ctgcccacct gcccctcaac 4320

cctgtagctg cagggggagg ggacttcatt catgggttgg tggtcgcacc ttggtttgac 4380

ttacacggga catttgtgtt tttggaggaa aagataccct gattctttga atcttcctta 4440

agtttataaa tatttatttt ttaaaag 4467

<210> 62
<211> 4168
<212> DNA
<213> Homo sapiens

<400> 62
aagacttagg aagactggtg gatgcgtttg ggttgtagct aggctttttc ttttctttct 60

cttttaaaac acatctagac aaggaaaaaa caagcctcgg atctgatttt tcactcctcg 120

ttcttgtgct tggttcttac tgtgtttgtg tattttaaag gcgagaagac gaggggaaca 180

aaaccagctg gatccatcca tcaccgtggg tggttttaat ttttcgtttt ttctcgttat 240

ttttttttaa acaaccactc ttcacaatga acaaactgta tatcggaaac ctcagcgaga 300

acgccgcccc ctcggaccta gaaagtatct tcaaggacgc caagatcccg gtgtcgggac 360

ccttcctggt gaagactggc tacgcgttcg tggactgccc ggacgagagc tgggccctca 420

aggccatcga ggcgctttca ggtaaaatag aactgcacgg gaaacccata gaagttgagc 480

actcggtccc aaaaaggcaa aggattcgga aacttcagat acgaaatatc ccgcctcatt 540

tacagtggga ggtgctggat agtttactag tccagtatgg agtggtggag agctgtgagc 600

aagtgaacac tgactcggaa actgcagttg taaatgtaac ctattccagt aaggaccaag 660

ctagacaagc actagacaaa ctgaatggat ttcagttaga gaatttcacc ttgaaagtag 720

cctatatccc tgatgaaatg gccgcccagc aaaacccctt gcagcagccc cgaggtcgcc 780

gggggcttgg gcagaggggc tcctcaaggc aggggtctcc aggatccgta tccaagcaga 840

aaccatgtga tttgcctctg cgcctgctgg ttcccaccca atttgttgga gccatcatag 900

gaaaagaagg tgccaccatt cggaacatca ccaaacagac ccagtctaaa atcgatgtcc 960

accgtaaaga aaatgcgggg gctgctgaga agtcgattac tatcctctct actcctgaag 1020

gcacctctgc ggcttgtaag tctattctgg agattatgca taaggaagct caagatataa 1080

aattcacaga agagatcccc ttgaagattt tagctcataa taactttgtt ggacgtctta 1140

ttggtaaaga aggaagaaat cttaaaaaaa ttgagcaaga cacagacact aaaatcacga 1200

tatctccatt gcaggaattg acgctgtata atccagaacg cactattaca gttaaaggca 1260

atgttgagac atgtgccaaa gctgaggagg agatcatgaa gaaaatcagg gagtcttatg 1320

aaaatgatat tgcttctatg aatcttcaag cacatttaat tcctggatta aatctgaacg 1380

ccttgggtct gttcccaccc acttcaggga tgccacctcc cacctcaggg cccccttcag 1440

ccatgactcc tccctacccg cagtttgagc aatcagaaac ggagactgtt catctgttta 1500

tcccagctct atcagtcggt gccatcatcg gcaagcaggg ccagcacatc aagcagcttt 1560

ctcgctttgc tggagcttca attaagattg ctccagcgga agcaccagat gctaaagtga 1620

ggatggtgat tatcactgga ccaccagagg ctcagttcaa ggctcaggga agaatttatg 1680

gaaaaattaa agaagaaaac tttgttagtc ctaaagaaga ggtgaaactt gaagctcata 1740

tcagagtgcc atcctttgct gctggcagag ttattggaaa aggaggcaaa acggtgaatg 1800

aacttcagaa tttgtcaagt gcagaagttg ttgtccctcg tgaccagaca cctgatgaga 1860

atgaccaagt ggttgtcaaa ataactggtc acttctatgc ttgccaggtt gcccagagaa 1920

aaattcagga aattctgact caggtaaagc agcaccaaca acagaaggct ctgcaaagtg 1980

gaccacctca gtcaagacgg aagtaaaggc tcaggaaaca gcccaccaca gaggcagatg 2040

ccaaaccaaa gacagattgc ttaaccaaca gatgggcgct gaccccctat ccagaatcac 2100

atgcacaagt ttttacctag ccagttgttt ctgaggacca ggcaactttt gaactcctgt 2160

ctctgtgaga atgtatactt tatgctctct gaaatgtatg acacccagct ttaaaacaaa 2220

caaacaaaca aacaaaaaaa gggtggggga gggagggaaa gagaagagct ctgcacttcc 2280

ctttgttgta gtctcacagt ataacagata ttctaattct tcttaatatt cccccataat 2340

gccagaaatt ggcttaatga tgctttcact aaattcatca aatagattgc tcctaaatcc 2400

aattgttaaa attggatcag aataattatc acaggaactt aaatgttaag ccattagcat 2460

agaaaaactg ttctcagttt tatttttacc taacactaac atgagtaacc taagggaagt 2520

gctgaatggt gttggcaggg gtattaaacg tgcattttta ctcaactacc tcaggtattc 2580

agtaatacaa tgaaaagcaa aattgttcct tttttttgaa aattttatat actttataat 2640

gatagaagtc caaccgtttt ttaaaaaata aatttaaaat ttaacagcaa tcagctaaca 2700

ggcaaattaa gatttttact tctggctggt gacagtaaag ctggaaaatt aatttcaggg 2760

ttttttgagg cttttgacac agttattagt taaatcaaat gttcaaaaat acggagcagt 2820

gcctagtatc tggagagcag cactaccatt tattctttca tttatagttg ggaaagtttt 2880

tgacggtact aacaaagtgg tcgcaggaga ttttggaacg gctggtttaa atggcttcag 2940

gagacttcag ttttttgttt agctacatga ttgaatgcat aataaatgct ttgtgcttct 3000

gactatcaat acctaaagaa agtgcatcag tgaagagatg caagactttc aactgactgg 3060

caaaaagcaa gctttagctt gtcttatagg atgcttagtt tgccactaca cttcagacca 3120

atgggacagt catagatggt gtgacagtgt ttaaacgcaa caaaaggcta catttccatg 3180

gggccagcac tgtcatgagc ctcactaagc tattttgaag atttttaagc actgataaat 3240

taaaaaaaaa aaattagact ccaccttaag tagtaaagta taacaggatt tctgtatact 3300

gtgcaatcag ttctttgaaa aaaaagtcaa aagatagaga atacaagaaa agtttttggg 3360

atataatttg aatgactgtg aaaacatatg acctttgata acgaactcat ttgctcactc 3420

cttgacagca aagcccagta cgtacaattg tgttgggtgt gggtggtctc caaggccacg 3480

ctgctctctg aattgatttt ttgagttttg tttgtaagat gatcacagtc atgttacact 3540

gatctaaagg acatatatat aaccctttaa aaaaaaaatc actgcctcat tcttatttca 3600

agatgaattt ctatacagac tagatgtttt tctgaagatc aattagacat tttgaaaatg 3660

atttaaagtg ttttccttaa tgttctctga aaacaagttt cttttgtagt tttaaccaaa 3720

aaagtgccct ttttgtcact ggattctcct agcattcatg attttttttt catacaatga 3780

attaaaattg ctaaaatcat ggactggctt tctggttgga tttcaggtaa gatgtgttta 3840

aggccagagc ttttctcagt atttgatttt tttccccaat atttgatttt ttaaaaatat 3900

acacataggt gctgcattta tatctgctgg tttaaattct gtcatatttc acttctagcc 3960

ttttagtatg gcaaatcata ttttactttt acttaagcat ttgtaatttg gagtatctgg 4020

tactagctaa gaaataattc tataattgag ttttgtactc accatatatg gatcattcct 4080

catgtataat gtgccccaaa tgcagcttca ttttccagat accttgacgc agaataaatt 4140

ttttcatcat ttaggtgcaa aaaaaaaa 4168

<210> 63
<211> 3883
<212> DNA
<213> Homo sapiens

<400> 63
ctcggacccg gtagtggtcc gtgaagtccg cggagtgggc gctgatggtg gggctatccc 60

ttcccactgt ccgcgaggtg cgccgtggta acgcgcgggg acacagcgcg ggtgtggacc 120

ccctgcccac agctggcgca cagccattga gtgtagaccc gactggggca cgaccactga 180

accctgcact tggcgtcctc cgggccccgc gaggggcctt gctgggcggg tccgcggcac 240

cccgggttcc cagaagtcga ggccccgaat gcgcctcccc tcgcctggaa tcggagagcg 300

gagggctcct gcgggcaggt ggccgtgctc cgaagagcgc cctgccaccc atgaggtggc 360

cctgggggcg tcgctagcct tcccagctgg gtgtcctcat ccggagacac aaacacctgc 420

ctcaggatta aagcgaggag ctgtatttca gaccgtctca ggagaaatag caagaggctg 480

cctcactcct ttgcccttgt tcaagtttga ggcattaaga aggataagac atcaatttga 540

aaagagcccc tgtcagagca acatgacttg aaagcaagaa gaaaaaacaa aaaatgaagc 600

aagaacaaac atcaaattta tgctgagttt ggctgcaaga atggtgaaat cattgatact 660

ttacaagaca ctaatcagga cagtgcccca aaggaatcag cagtttacaa atggatataa 720

cctgttttca gaagagacaa gattaatttg aagatgaagc agtggcggac catccatgtc 780

aatttatgag aaaaaaattc attctgttca tgccctaact gaagaggact gacaacagca 840

caaacaatag ccaacatcat agacatctca actggttcag cttactcaat tctgactgaa 900

agttaaagtt gagcaaactt tccactcaat gagtgtcgaa accctcatgc ccagatcagc 960

tgcagacaag agcagaggtt tcaatggaaa ttttaaacaa gtgagattaa gatcctgaag 1020

catttcttga aagaattgta acaggagata aaccatggct tcaccagtac aatactgaag 1080

actaagcaca atcaaagcaa tggctaccaa gaggccgaag tggtcccatt aaggcgaaag 1140

tggaccagtc aagagcaaag gtcatggcaa cagtgttttg ggatgccaag acattttgct 1200

cgttgacttt ctggagggcc aaagtgcagt aatgtctgtt tattatgttt tgagaaagtt 1260

ggccaaacct ttagtagaaa aatgcccagg agaacttcac tagagtgacc ttcgttcaat 1320

ttcagatcac cacaataaag caaaaaattg caattataaa ttttttggtt tcccagtaca 1380

tgtaaaagtt ataattacac tatactatag tctattaagg gtgcaatagc attatgtctg 1440

aaaaaaacaa tggtcatgcc ttaatttaaa aatactttat tactaaaaaa tgctagcaat 1500

catctgaacc ttcagtgagt cataatattt ttgctagtgg agggccttgc ctggatgttg 1560

atggctgctg actgatcagg atggtggttg ctgaaggtta gggtggctgc agcaatttct 1620

taacataaga caacagtgaa gtttcctgca ttgattgact cttcctttca tgaaatattt 1680

ctctatagca tgtaatgctg tttgatagca ttttacccac agtagaattt atttcagaat 1740

tgaagtcagt cctcttaaac cctgtcactt ctttattaac taagttcacg caatattcta 1800

aatcctttgt tgtcatttca acaatgttca caacatcttc accagggata gattctgtct 1860

caagaaacca ctttctttgt tcattcgtaa gaagcaaccc ctcatctgtt caagttttct 1920

catgagattg gagcaattca gtcacatttt caggctgcat ttttaattct agttctcttg 1980

ctatttctac cacatctgca gttacttcct tcactgaagg cttgaacccc tcaaagtcac 2040

ccatgagggt tggaatcaac ttcttccaaa ttcctgttaa tgttgatgtt ttgacctcct 2100

ctcatgaatg atgaatgttt ttaatgacat ctaaaatggt cagtcctttc cagaaggttt 2160

tcaatttact ttgccccaat ccatcagagg aatcactgtc tatggcaact gtaggcttat 2220

aaaaggtatt tcttaaataa tatgtcttga aagtctaaat gactccttga tccataagct 2280

gcagaatggg tgttgtgtta gcaagcatga aaacaacatt catcttgtgc ctctccatct 2340

gagctcttct atgaccaggt gccttgttga tgagcagtaa tattttgaaa gaaatctttt 2400

ttcctgagca gtgggtctca acaatgggct taaaatgttc attaagccat gctgtaaaca 2460

gatgtgctat catccaggct ttgatgttcc ttatacagag tacaggcaca ggcagagtag 2520

atttagtata attcttaagg gcccttggat tttcagaatg gtaagtgaac attggtgtga 2580

acttaaggtc accagctgca ttagccccta acaagagagt cagcctgtcc tttgaagctt 2640

tgaagctagg cattgacttc tctctagcta tgaaagtcct agatgacatc ttctgattta 2700

aggctgtttt gtctacattg aaagtctatt atttagtgta gccaccttca tcaattatct 2760

tagtaaggtc ttctggataa cttgctgcag cttctacatc agcatttact gcttcacctc 2820

gcacttttat gttatggaga tggcttcatt ccttaaacct catgaaccaa cgagcttcaa 2880

acctttcttc tgcagcttcc tcacctccct caggcttcat agaatttaat agagttaggg 2940

ctttgctatg gattaggttt ttgcttatgg gaggattgtg gctggtttaa tcttctatcc 3000

agactactac aactttcccc atatcaaaaa taaggcactt tcattttttg atcatttgtt 3060

cactggagtg acacttactt ttaatttcct gaaaatttcc ctttgcattt acaatgtagc 3120

taactattca gtggaagagg gctagttttt gacatgcctt cctcaatatg cttaataatt 3180

tctagttttt gatttaaagt gagacacatg tgactcttcc tttcacttga atgccgagag 3240

gccatgcagg gttattaatt agcctaattt caatattgtt atttaagtta tggggaggct 3300

tgaggagagg gagagagaga caggagaatg gccggtcagt ggagcagtca gaatacacac 3360

aacatatatt gattaacttt cctatcttat atgagcacag ttcttggcac tccaaaacaa 3420

ttacagtact aacatcaaag atcactgatc acagatcacc ataactgata ttataataat 3480

ggaaagtctt gaaatattgt gaaaattacc aaaatgtgac agatacaaca tgaacaaatg 3540

ctgttggaag aatggcactg atagatttgc ccaatgcagg gttgccacaa actttcaact 3600

tgtaaaaaga gcagtatctg tgaaagacgg taaagcagaa agcaataaaa tgaagtgtgc 3660

ccatgtatac taataagaca tagttcattt tattaataaa gcacctgtca atttcagttg 3720

agtaattttt atcaggaatg ggttttgaat tttgttcaag gcttttacag catctgtggg 3780

ataactatgt attgtttccc tggatctata aatacataca ggatactaat gacttttcaa 3840

atattgaacc aactttgcat tcttggaata aatctcactt gat 3883

<210> 64
<211> 6325
<212> DNA
<213> Homo sapiens

<400> 64
tgttcggatt atcttttgct tccccccagc tgcctcctta ccctcacact cccactcctc 60

cgtttccgcg gtcgaagctg ccttcggccc cgggtggtct cccccgcccg gggaccccct 120

gtgcctcccc tcccgggctg cgggggagcc cctccgagac catgaggaaa ttcaacatca 180

ggaaggtgct ggacggcctg accgccggct cgtcctcggc gtcgcagcag caacagcagc 240

agcatccgcc tgggaaccgg gagccggaga tccaggaaac gctccagtcc gagcactttc 300

agctctgcaa gactgttcgc catggatttc cctatcaacc ctcagccctg gcctttgatc 360

ctgtacagaa gatcctggca gtgggaactc agactggtgc tttaaggctc tttggtcgtc 420

caggagtaga atgttattgc cagcatgaca gtggagctgc agtaatccag ctccagttcc 480

tgattaatga gggagcgctt gtgagtgcct tggctgatga caccttacac ttatggaatt 540

tacgtcagaa gaggcctgcc atactacatt cgcttaaatt ttgcagagaa agggttacat 600

tttgccatct gcctttccag agtaagtggc tctatgtggg cactgaacga ggtaatatac 660

atattgtcaa tgtggagtcc ttcacactct caggctacgt cattatgtgg aataaagcca 720

ttgaactgtc atctaaatct cacccaggac ctgtggtcca tataagtgat aatccaatgg 780

acgagggaaa gcttttgatt ggctttgaat ctggaacagt agttttatgg gacctcaaat 840

caaagaaagc cgactacaga tacacatatg atgaggctat ccactctgtt gcttggcatc 900

atgaaggaaa acaatttatt tgcagtcatt cagatggcac cttgactata tggaatgtaa 960

ggtcccctgc taaaccagta cagacaatca ctccacatgg aaaacagtta aaggatggga 1020

agaagccaga accatgcaaa cctatcctca aggtggaatt caaaacgact agatctgggg 1080

agccttttat tattttatca ggaggtttgt catatgatac tgtaggaaga agaccttgct 1140

taacagtgat gcatgggaaa agcactgctg tgctagaaat ggactattca attgttgatt 1200

ttctaacgct gtgtgaaaca ccatacccaa atgattttca agaaccatat gctgtggttg 1260

ttcttctaga aaaggattta gtacttatag accttgcaca aaatggatat cctatatttg 1320

aaaatcccta ccctttgagt atacatgagt cccctgttac atgttgcgaa tattttgcgg 1380

attgtcctgt ggaccttatt cctgcacttt attctgttgg agctagacag aaacgtcaag 1440

gttacagcaa aaaggaatgg cccatcaacg gaggtaattg gggcttgggt gctcaaagtt 1500

acccagaaat aattattaca gggcatgctg atgggtcagt taagttctgg gatgcttctg 1560

caataactct acaagtatta tataagctaa agacatctaa agtatttgaa aagtcaagaa 1620

ataaagatga caggccaaac acagacattg tagatgaaga tccatatgcc attcagatca 1680

tctcctggtg tccagaaagt agaatgctgt gcatcgctgg agtttcagct catgtcatta 1740

tttatagatt cagcaagcag gaagtaatca cagaagtcat tccgatgctt gaagttcgat 1800

tattatatga gataaatgat gtggaaactc cggagggtga gcagccacca cctttgccaa 1860

cacccgtggg agggtccaac cctcagccca tccctcctca gtctcatcca tctaccagta 1920

gcagttcatc tgatgggctt cgtgataatg taccttgttt aaaagttaaa aactcaccac 1980

ttaaacagtc tccaggttat caaacagaac tagttattca gttggtttgg gtgggtggag 2040

aaccaccaca acaaataacc agcctggcag tcaattcttc ctatggactg gtggtttttg 2100

gcaattgcaa tggcattgct atggttgact acctccagaa agcagtgctg ctcaacctgg 2160

gcactattga attatatggc tctaatgatc cttatcggag agaaccccga tctcctcgta 2220

aatctcgaca gccttcagga gccggtctgt gtgatattag tgaagggact gttgttccag 2280

aggatcgctg caaatctcca acctctgcaa agatgtcaag gaagttaagc ttacctactg 2340

acctaaagcc tgatttagat gtaaaggata actcctttag ccgatcacgg agttcaagtg 2400

taacaagcat tgacaaagaa tcccgagaag cgatctccgc tcttcatttc tgtgaaacgt 2460

ttactcgaaa gacggactcg tccccttccc cttgtctatg ggttggaaca acgctaggaa 2520

cagtgcttgt cattgcactg aaccttcccc cagggggaga gcaaagactt cttcagccag 2580

taattgtgtc tccaagtggt actatattga ggttaaaagg tgcaatcttg agaatggcat 2640

ttctggatac cacaggctgc ttaataccac ctgcgtatga accctggaga gagcacaatg 2700

ttcctgaaga aaaagacgaa aaggagaaat tgaaaaaacg gcggcctgtc tcagtatccc 2760

cctcctcttc tcaggaaatt agtgaaaacc agtatgcagt gatatgttct gaaaagcaag 2820

caaaagtaat ctcactgcca acccagaact gtgcttataa gcaaaatatt acagagacct 2880

cgtttgtgct tcgtggagat attgtagcat tgagtaacag tatctgcctt gcctgtttct 2940

gtgccaatgg acatataatg acttttagtt tgccaagttt aagacctctg ttggatgtgt 3000

attacttgcc ccttaccaat atgcggatag ccagaacgtt ctgctttacc aacaatggac 3060

aagcattata ccttgtttca cctacagaaa tccagagact tacttatagt caagagacct 3120

gtgaaaatct tcaggaaatg ttgggtgaac tcttcactcc tgtagaaaca cctgaagcac 3180

caaacagggg attctttaaa ggcttatttg gaggtggtgc acaatctctt gacagagaag 3240

aactatttgg agaatcgtcc tcaggaaagg cttcaaggag ccttgcacag catattcctg 3300

gccctggtgg cattgaaggc gtaaaagggg cagcatctgg agttgttggt gaattagcac 3360

gagccaggct ggcactagat gaaagagggc agaaacttgg cgatctggaa gaaagaactg 3420

cggccatgtt atcaagtgca gagtcatttt ctaaacatgc tcatgagatt atgttgaaat 3480

acaaagataa gaagtggtac cagttctgac aaccagaatc caataagtcc aacttcagcc 3540

agaaggaaaa aagttttcca tttttattac attctttagg aaagttaacg ttaaagggat 3600

gttcgtcact gaatactgtt ctttcctagc acagtcatgc actgttttac ctcagtcatg 3660

tggctttaac tgaggagtgt tcacacgcac tcgaaatgga gtatatggtg tgtgccagtt 3720

atgagttgac catttgggaa ttaaacaggt cacacgtgac agatgaagaa accaaggggg 3780

ctgctgagga gacctggtgc agggactaat cctggatcat tcctgtatta aactttcata 3840

tgccaaaagg gtttgtgccg ttttatctgc catcagtgtt tgacctgttt agggcagagg 3900

caataagtca gaagtcttga agttgaaata gttatatgtg tgtcattgga ctggattata 3960

aacagctgtc ttggactttc cctctcttaa cactgactgg tcatcagtat cattagtgaa 4020

aaagaaacaa attgtttgtt atcatctctt tagacagata agctgaatgg tgggctttaa 4080

ataataaaaa catacacata gttgacttgt gtatgagcta ctcttggatt cttgttatta 4140

tagacttgta tttagttcat attttgtcaa aagcaaaaca agaagataca tcacttttca 4200

ttgaaaagaa aagtgtagag catgactgaa ttgctcatca ttctgggagt ttccatgtag 4260

tggctatgca gtgtggaaag tgagaaaaac ctccattgtg gtgaggagaa tacttcaatg 4320

tcccttgtcc ttgttctcat taattcagct aaaggtggat ttgaccaaaa taatgctggt 4380

taaatttgta gaaatgttga aattggctgt gttttaaatt ttgcttgaat ttttataaaa 4440

tgtttaacca aatgtacctt tgcattcttt aattaaaatt gcttaaaaaa aaactttcat 4500

tatttcagta aaatgctcag ctcccttttc aaaatgccct ttatttgcta actgttccaa 4560

tacactcagg tgatgagcca attaaatgtt agtgcacatg ctatcgcatg gaaaattaca 4620

agcatctgtt gtcaataatt atataagagt ttagtctgat gacctacaaa atattttgtg 4680

tagaaatata ctataaatct gtacatatcc tttcaaggtt ttaaaaaacc aaaaagaaag 4740

gaaaatatgt acactgtgat aactaaatta tttctgtatt ggaatataat acaatttgag 4800

accagcaatg gacaatgaat gattgtttca tagaatagca ttacgggcag gaaagaaacc 4860

acctagtaga tctagaagta agcaatctca gaatacagac taatctgaga ttattattga 4920

ttgttgttaa agcaacccag caaaagcaaa atggaagcat taaaattaat gttaatgaaa 4980

tttaaatatt gtcttctata aaaaattcct ttaaatgatt ttgttttttc attaagagat 5040

aatcagagca caaattgata gtaaacagga tggttgtttt tctattctat atgatcatta 5100

aaggaatatg taggacatct taactttttc atacagtctg ttatgcatat tttcctctac 5160

ttttcattaa taaagctttt atgtttacat tttataacat gcactactag atgcaaaagt 5220

ttacatcaaa gtttacttta aatatcattt ggtagggact aaatgtgtgt gatagagata 5280

attgatcaag ccaaaagaaa tattttttaa ataagccctt ttcaaaagtt tttgaattta 5340

tagaaagcac caatgaataa catatttctg tttcgttaat gtcagctgcc tgaacattca 5400

gcagtttata aattgcttaa tttgtgttat ctattatcca gtaaacccat agttccatga 5460

tatgtcacag gaattgttag gtcctatttt aaaggtacag ttttgtgaat gtcatcaata 5520

aaatcaacag ttatggattt gaagaagttg ggaaagcatt atgtagatta atatactggt 5580

tggttcccta tctatgtgga aggtcatatt agctgcaatt atttaatttg ctgtgttatt 5640

ttgtgttata taacacaaat atatttgtat attaacttca tttttactgt catttttcct 5700

gttgtataca aaatgaacta atcttgtaat tattttcaaa tatagaagta tatacattag 5760

atggatttcc aagattttgt aagaaaatct taaatcagtg ttttgagtta tttaattttt 5820

aaattaatct acaaattatg cacaacaaac tagagactca gttaggatta gaaagcttaa 5880

agtatgtttg agtgtaggaa ataagcttct cagagttata cttgattctg tctgtagata 5940

agagacagtc tacagtaata actaacccag ggaatttact gtaatttgag aggtaacatc 6000

tctatttttt tctttttaaa tacagaaagt aatagtcacc agcaagccac gatttcagga 6060

aaactgacta aaaaaaaaag tcaaaataga aaatatcaga aaaatgaaag atttttgtgt 6120

gccctctcca tatcctcatt gtttttgttg catttgtcct tgactctaaa atcactgaag 6180

tattcatgat tagtagtttg taagtaggaa gtggtttttc tgtatgtacc atatatccag 6240

ttcatttcag ttttcaaact aaaatccata tgtatttgct gagaaagtta tttgaaatct 6300

tatattctga caaattctgt acatt 6325

<210> 65
<211> 4380
<212> DNA

<213>  Homo sapiens

<400>  65
atcgtggggc gggggtgggg cgggactgag ggcggagtgt gagcgggctc ggttttgggc      60

cgcggcggga gcgggagtcg ccgccactcg agtgcgcagg cgcctggcga ttaccggtct     120

caccatggag cggaaagtgc ttgcgctcca ggcccgaaag aaaaggacca aggccaagaa     180

ggacaaagcc caaaggaaat ctgaaactca gcaccgaggc tctgctcccc actctgagag     240

tgatctacca gagcaggaag aggagattct gggatctgat gatgatgagc aagaagatcc     300

taatgattat tgtaaaggag gttatcatct tgtgaaaatt ggagatctat tcaatgggag     360

ataccatgtg atccgaaagt taggctgggg acacttttca acagtatggt tatcatggga     420

tattcagggg aagaaatttg tggcaatgaa agtagttaaa agtgctgaac attacactga     480

aacagcacta gatgaaatcc ggttgctgaa gtcagttcgc aattcagacc ctaatgatcc     540

aaatagagaa atggttgttc aactactaga tgactttaaa atatcaggag ttaatggaac     600

acatatctgc atggtatttg aagttttggg gcatcatctg ctcaagtgga tcatcaaatc     660

caattatcag gggcttccac tgccttgtgt caaaaaaatt attcagcaag tgttacaggg     720

tcttgattat ttacatacca agtgccgtat catccacact gacattaaac cagagaacat     780

cttattgtca gtgaatgagc agtacattcg gaggctggct gcagaagcaa cagaatggca     840

gcgatctgga gctcctccgc cttccggatc tgcagtcagt actgctcccc agcctaaacc     900

agctgacaaa atgtcaaaga ataagaagaa gaaattgaag aagaagcaga agcgccaggc     960

agaattacta gagaagcgaa tgcaggaaat tgaggaaatg gagaaagagt cgggccctgg    1020

gcaaaaaaga ccaaacaagc aagaagaatc agagagtcct gttgaaagac ccttgaaaga    1080

gaacccacct aataaaatga cccaagaaaa acttgaagag tcaagtacca ttggccagga    1140

tcaaacgctt atggaacgtg atacagaggg tggtgcagca gaaattaatt gcaatggagt    1200

gattgaagtc attaattata ctcagaacag taataatgaa acattgagac ataaagagga    1260

tctacataat gctaatgact gtgatgtcca aaatttgaat caggaatcta gtttcctaag    1320

ctcccaaaat ggagacagca gcacatctca agaaacagac tcttgtacac ctataacatc    1380

tgaggtgtca gacaccatgg tgtgccagtc ttcctcaact gtaggtcagt cattcagtga    1440

acaacacatt agccaacttc aagaaagcat tcgggcagag ataccctgtg aagatgaaca    1500

agagcaagaa cataacggac cactggacaa caaaggaaaa tccacggctg gaaattttct    1560

tgttaatccc cttgagccaa aaaatgcaga aaagctcaag gtgaagattg ctgaccttgg    1620

aaatgcttgt tgggtgcaca aacatttcac tgaagatatt caaacaaggc aatatcgttc    1680

cttggaagtt ctaatcggat ctggctataa tacccctgct gacatttgga gcacggcatg 1740

catggccttt gaactggcca caggtgacta tttgtttgaa cctcattcag gggaagagta 1800

cactcgagat gaagatcaca ttgcattgat catagaactt ctggggaagg tgcctcgcaa 1860

gctcattgtg gcaggaaaat attccaagga atttttcacc aaaaaaggtg acctgaaaca 1920

tatcacgaag ctgaaacctt ggggcctttt tgaggttcta gtggagaagt atgagtggtc 1980

gcaggaagag gcagctggct tcacagattt cttactgccc atgttggagc tgatccctga 2040

gaagagagcc actgccgccg agtgtctccg gcacccttgg cttaactcct aagcccctgc 2100

ccagcaccac agcagagatc acacactgac cctccgccct tccccttcaa gcattttcct 2160

cttccctttt cagggtgaag ctcttccttc aagagtttct agatcttgtt ttttttttaa 2220

tccaacatgt tcatttgggt ttgcttactt gaccctgtgg agatccccac agccattggg 2280

catcctaggt gaatttggcc ttggttgggc tctgccaaag actaatggac taaaatgtga 2340

aacagcctct tgccctgtac ctttccttcc cattaggaca tcctttaaat tataagcatc 2400

ctttttgaaa agagctatga aggtgtatga gcccatcctt ttattcattg actctaagag 2460

tcaaattttc tagtgcatat cctattgcca gcataaggat gaggaggggg aaagggtctt 2520

aattctatgt acagcagaga cattaaactt gctgtgtccg ggctgcatca tcttcctgga 2580

ctgtttctgt tgttctctgt gttcacattt tttcctgcaa cttttaagct actgtctttt 2640

ttaaatagct atatgaacac caaatttggg taccatttta tcactgttca aagcactgtc 2700

aaattccttt catcctttaa tagttaagat ctttgaatct tcagtctgat ttttaatgta 2760

agcaaaaaca gaaccattga atagtaattt cttgagaacc tcaggtgttc tataaacagt 2820

cctttcctgt atgtcttcta ttaccctaag accagagtta ttttggttgg ttgttttgtt 2880

ttattttttg tttttgtatc catggctggc actttactca ttgcacttga gtttattgcc 2940

ccataactaa aggatcagga tgatggtaga acggagatct gggtttcaga gctttcccat 3000

ttaagaaaaa tagatcttga gattctgatt cttttccaaa cagtcccctg ctttcatgta 3060

cagctttttc tttaccttac ccaaaattct ggccttgaag cagttttcct ctatggcttt 3120

gcctttctga ttttctcaga ggctcgagtc tttaatataa ccccaaatga aagaaccaag 3180

gggaggggtg ggatggcact tttttttgtt ggtcttgttt tgttttgttt tttggttggt 3240

tggttcgtta ttttttaaga ttagccattc tctgctgcta tttccctaca taatgtcaat 3300

ttttaaccat aattttgaca tgattgagat gtacttgagg cttttttgtt ttaattgaga 3360

aaagactttg caattttttt tttaggatga gcctctccta gacttgacct agaatattac 3420

atattcctcc agtaagtaat actgaagagc aaaagagagg caggattggg gtcacagccg 3480

cttcttcagc atggaccaag tgggccttgg ggattgcagc gttctcgaag tggctgtagg 3540

actcgaattt acagaaagcc acagaggtgc aacttgaggc tctgctagca agccaccagt 3600

gaggctattg ggtaaccacc tttctataca ggagattgga atctactttg tcatttatcc 3660

accacagtga caaaggaaaa gtggtgccgt tatgcaatcc atttaactca taaacatatt 3720

actctgagta actggccagc cattcatcgg atccttcatt gggtactcct gaaatcagac 3780

atgttcctgt agaaagaatt ttaagttagg ctttctatgc acctatcaag aatcaagaga 3840

atagattgta tcaaacaacg gcagggaaat ccttcagcaa ttctaatcca ctttgggttt 3900

tcagctgttt ttacatctaa agcaatagac tagaactgaa ttatcttcta catagtaaaa 3960

tcacaattgt ggaattacag gaattctggt gatattaagg tgaaataaca aaacacaaaa 4020

ggccctattt taacagttga tgtgacagta agttttaata gaacctgtaa cttcattttg 4080

gaaatgcttc tccaccaaat aagggctttt tcccctattt aaggagccag atggattgaa 4140

agatgtggaa ataggcagct gtagatcttg atcttccagg taccccatgt acctttattg 4200

agcttaatta taatactgtc aaattgccac gatctcacta aaggatttct atttgctgtc 4260

agttaaaaat aaagccctaa atacattttt attctttcta ctgagggcat tgtctgtttt 4320

ctttgtaaat gccgtacaat aaacaaatta tttaataacc taaaaaaaaa aaaaaaaaaa 4380

<210> 66
<211> 1785
<212> DNA
<213> Homo sapiens

<400> 66
ctttgcaaat tgccctagta acggccgcat ggtaactcag gcgccgggcg cactgtccta 60

gctgctggtt ttccacgctg gttttagctc ccggcgtctg caaaatgaag attgaggagg 120

tgaagagcac tacgaagacg cagcgcatcg cctcccacag ccacgtgaaa gggctggggc 180

tggacgagag cggcttggcc aagcaggcgg cctcagggct tgtgggccag gagaacgcgc 240

gagaggcatg tggcgtcata gtagaattaa tcaaaagcaa gaaaatggct ggaagagctg 300

tcttgttggc aggacctcct ggaactggca agacagctct ggctctggct attgctcagg 360

agctgggtag taaggtcccc ttctgcccaa tggtggggag tgaagtttac tcaactgaga 420

tcaagaagac agaggtgctg atggagaact tccgcagggc cattgggctg cgaataaagg 480

agaccaagga agtttatgaa ggtgaagtca cagagctaac tccgtgtgag acagagaatc 540

ccatgggagg atatggcaaa accattagcc atgtgatcat aggactcaaa acagccaaag 600

gaaccaaaca gttgaaactg gaccccagca tttttgaaag tttgcagaaa gagcgagtag 660

aagctggaga tgtgatttac attgaagcca acagtggggc cgtgaagagg cagggcaggt 720

gtgataccta tgccacagaa ttcgaccttg aagctgaaga gtatgtcccc ttgccaaaag 780

gggatgtgca caaaaagaaa gaaatcatcc aagatgtgac cttgcatgac ttggatgtgg 840

ctaatgcgcg gccccagggg ggacaagata tcctgtccat gatgggccag ctaatgaagc 900

caaagaagac agaaatcaca gacaaacttc gaggggagat taataaggtg gtgaacaagt 960

acatcgacca gggcattgct gagctggtcc cgggtgtgct gtttgttgat gaggtccaca 1020

tgctggacat tgagtgcttc acctacctgc accgcgccct ggagtcttct atcgctccca 1080

tcgtcatctt tgcatccaac cgaggcaact gtgtcatcag aggcactgag gacatcacat 1140

cccctcacgg catccctctt gaccttctgg accgagtgat gataatccgg accatgctgt 1200

atactccaca ggaaatgaaa cagatcatta aaatccgtgc ccagacggaa ggaatcaaca 1260

tcagtgagga ggcactgaac cacctggggg agattggcac caagaccaca ctgaggtact 1320

cagtgcagct gctgaccccg gccaacttgc ttgctaaaat caacgggaag gacagcattg 1380

agaaagagca tgtcgaagag atcagtgaac ttttctatga tgccaagtcc tccgccaaaa 1440

tcctggctga ccagcaggat aagtacatga agtgagatgg ctgaggtttt cagcagtaag 1500

agactcccca ggtgtgcctg gcctgggtcc agcctgtggg cgcttgcccc tgggcttggg 1560

gctgccgtcc ccactcaggc gtggtctgca gcgctgtcag ttcagtgtgg aaagcatttc 1620

tttttaagtt atcgtaactg ttcctgtggt tgctttgaaa gaacccttcc ttacctggtg 1680

tgttttctat aaatcttcat aggttatttt gattctctct ctctctctct ctaagttttt 1740

taaaaataaa cttttcagaa cagttttaaa aaaaaaaaaa aaaaa 1785

<210> 67
<211> 5329
<212> DNA
<213> Homo sapiens

<400> 67
gtgggcgtgg ctgggcgagc gaggagtggg gacaaggtcg agcgacgagt cgtctacccg 60

cgcgcccgag cgcggaaacc gaggggcagg ctcgcgctct gcctgcttcg tggcgcttgg 120

ttcgtccctc gcccgaggag cgcggtggcg gcgtggaggg gagcctctga cggcgtctgg 180

aactctattt taagaacctc tcaaaacgaa acaagcaaat catggagaag aatggaaata 240

accgaaagct gcgggtttgt gttgctactt gtaaccgtgc agattattct aaacttgccc 300

cgatcatgtt tggcattaaa accgaacctg agttctttga acttgatgtt gtggtacttg 360

gctctcacct gatagatgac tatggaaata catatcgaat gattgaacaa gatgactttg 420

acattaacac caggctacac acaattgtga ggggagaaga tgaggcagcc atggtggagt 480

cagtaggcct ggccctagtg aagctgccag atgtccttaa tcgcctgaag cctgatatca 540

tgattgttca tggagacagg tttgatgccc tggctctggc cacatctgct gccttgatga 600

acatccgaat ccttcacatt gaaggtgggg aagtcagtgg gaccattgat gactctatca 660

gacatgccat aacaaaactg gctcattatc atgtgtgctg cacccgcagt gcagagcagc 720

acctgatatc catgtgtgag gaccatgatc gcatcctttt ggcaggctgc ccttcctatg 780

acaaacttct ctcagccaag aacaaagact acatgagcat cattcgcatg tggctaggtg 840

atgatgtaaa atctaaagat tacattgttg cactacagca ccctgtgacc actgacatta 900

agcattccat aaaaatgttt gaattaacat tggatgcact tatctcattt aacaagcgga 960

ccctagtcct gtttccaaat attgacgcag ggagcaaaga gatggttcga gtgatgcgga 1020

agaagggcat tgagcatcat cccaactttc gtgcagttaa acacgtccca tttgaccagt 1080

ttatacagtt ggttgcccat gctggctgta tgattgggaa cagcagctgt ggggttcgag 1140

aagttggagc ttttggaaca cctgtgatca acctgggaac acgtcagatt ggaagagaaa 1200

caggggagaa tgttcttcat gtccgggatg ctgacaccca agacaaaata ttgcaagcac 1260

tgcaccttca gtttggtaaa cagtaccctt gttcaaagat atatggggat ggaaatgctg 1320

ttccaaggat tttgaagttt ctcaaatcta tcgatcttca agagccactg caaaagaaat 1380

tctgctttcc tcctgtgaag gagaatatct ctcaagatat tgaccatatt cttgaaactc 1440

taagtgcctt ggccgttgat cttggcggga cgaacctccg agttgcaata gtcagcatga 1500

agggtgaaat agttaagaag tatactcagt tcaatcctaa aacctatgaa gagaggatta 1560

atttaatcct acagatgtgt gtggaagctg cagcagaagc tgtaaaactg aactgcagaa 1620

ttttgggagt aggcatttcc acaggtggcc gtgtaaatcc tcgggaagga attgtgctgc 1680

attcaaccaa actgatccaa gagtggaact ctgtggacct taggaccccc ctttctgaca 1740

ctttgcatct ccctgtgtgg gtagacaatg atggcaactg tgctgccctg gcggaaagga 1800

aatttggcca aggaaaggga ctggaaaact ttgttacact tatcacaggc acaggaatcg 1860

gtggtggaat tatccatcag catgaattga tccacggaag ctccttctgt gctgcagaac 1920

tgggccacct tgttgtgtct ctggatgggc ctgattgttc ctgtggaagc catgggtgca 1980

ttgaagcata cgcctctgga atggccttgc agagggaggc aaaaaagctc catgatgagg 2040

acctgctctt ggtggaaggg atgtcagtgc caaaagatga ggctgtgggt gcgctccatc 2100

tcatccaagc tgcgaaactt ggcaatgcga aggcccagag catcctaaga acagctggaa 2160

cagctttggg tcttggggtt gtgaacatcc tccataccat gaatccctcc cttgtgatcc 2220

tctccggagt cctggccagt cactatatcc acattgtcaa agacgtcatt cgccagcagg 2280

ccttgtcctc cgtgcaggac gtggatgtgg tggtttcgga tttggttgac cccgccctgc 2340

tgggtgctgc cagcatggtt ctggactaca caacacgcag gatctactag acctccagga 2400

acagacatgg accttctctc cagagctcct gagtggaatc aagttcttgt ctttaggatg 2460

accgtttctt aacaatcaaa tctggtattg aactgcaggt gactttggca gagaaatgtt 2520

ttcacttttg gtctcctctt ccagagtcac ctttccccac tcctattttt gtagatgcta 2580

ttctttctga tgtcttctta ctaggggtca ttttagctca aaccctgtaa gttacagtca 2640

caattttctg tgccaaagca gctacaataa tagagaggaa gccttcttag aactctgctt 2700

actaatgtat taataccact gagaccttca ggccttgcct gggatatcac ttcatcctga 2760

agtttgcatt aataatcctt ccaggccggg cacagtggct cacgcctgta atcccagcac 2820

tttgggaggc cgaggcgggc ggatcacgag gtcaggagat cgagaccgcc ctggctaaca 2880

tggtgaaaca tggtgaaacc ccgtctctac taaaaataca aaaaattagc tgggtgtggt 2940

ggcgggtccc agctactcgg gaggctgagg caggagaatg gcatgaacca gggaggcgga 3000

gctggcagtg aactgagacc gcaccactgc actccagcct gggtgacaga gcaagactcc 3060

atctcaaaaa taataataat aaataataat aataataata ataataataa taatccttcc 3120

agctgggcgc agtggctcac gcctgtaatc ccaacatttt gggaggccga gatgggcgga 3180

tcacctgagg tcaggagttc aagaccagcc tggccaacat ggtgaaaccc catctctact 3240

aaaatacaaa aattagccgg gcgtggtggc atgtgcctgt agtcccagct actcgggagg 3300

ctgaggcagg agaattgctt gaacccggga ggcggaggtt gcagtgagcc gagatcatgc 3360

cactgcactc caccctgggt gacagagcga gactccgtct acacacacac acacacacac 3420

acacacatcc ttcctcctct aaccccaaac taagatcaca gaaggtgatc cagtcagaga 3480

acagagggaa atcttaccag gaagggctta agtacacttt tttttaaaac agctttattg 3540

tttttaaagc ctacaatttg ataagccttg acatatgtat acctgtgaaa gcatcaccac 3600

aatcaagaca ctggacatat ctatcactcc cccatctcag atatcccccc taatcctgga 3660

taccatttgt tgaaagatgt tattactcta gctgaactta caagagactt tagaccaggg 3720

atctaaatta cagtggcctt agtgaccttg tccttatctt cttaggacag ctgagaagcc 3780

actgggactt agagccttta aaaggagatt aactgtccca aaaggatctt tgctactgac 3840

cagcagacac ttcttccttc agtagccttt catactgtgt tgagtaacac cctagggtgt 3900

ccattaaagt tttgagtttt acctagagcc cagagccatg aatcaggatt ctgtctacat 3960

gattcgtgtt ttcattggtg tcaaaataca aaagccaaag ttctggctat gaattgttaa 4020

cttggaagaa atactaactg ccaccactta ttaagtgcct actgtgtgcc aggctctgaa 4080

ctaggtgctt catatacatt atcctaaatt atctcaacat atgaggtagg tgttttaatt 4140

tttattttat agaacttggt gtgtttgact gttaagctat ggggctagag agagggtttg 4200

atcccaggtc cctctgtgct tttgctgctg agccacacaa cctctcattt caaaaacact 4260

ttcaaaatgc taacatattc taattcactc taggccacca aaaactttaa tactaatatc 4320

tgatttgtaa atgacttaat gtatccttga ccctatcagc tgaatttaat gaaatattcc 4380

tctctgctgt gaaattttac cagtatagta tttggtctag tgacaggtgt tctatttttt 4440

tgagacgggt ctcactctgt cacccaggct ggagtgcagt ggctcaatgc aacctctgcc 4500

ccccaagttc aagtgattct cctgcctcag cctcttgagt agctgggatt acaggcacat 4560

gcaccacgcc cggctgattt ttttttgtat ttttagtaga cagggtttca ccatgttggc 4620

caggctggtc ttgaactcct gacctcaggt gatccgcccg cctctgcctc ccaaagtgct 4680

gggattacag gtgtaagcca tcacacctgg ccctagtgac aggtttttat gggtactttt 4740

agatgatcta agaaatcatg tgcatatatc tttcagattt ctattttggg aaaatgaagg 4800

tttctacaac atattgtttc agtgttcaaa taaactgaag gactcaacat tacatttgaa 4860

ctatatcctt cctagtgggt tagtgtgaaa aagagtttgg ctgattccta aaactctgcc 4920

agccctgcag taatctccag ggcctggtta ttgttcagac attccatggt gattcctggg 4980

aaggaagctt ggctgctcag tttctgagtc tggggtgaga taatgttctg ggaagggaca 5040

tctgttcttt ggtgtaatct ctcatggtga aatctgctct gtacatcaga caattgcatt 5100

gctaccaagt ttcataccaa atatttgaaa ggatggtatt gaatctaaaa ccaaatatta 5160

gtttttatta aactcatggg aaggctaata tattccaacg taaattatta catatggtta 5220

agtaattgca tgttaattta ttttaatgta aatatttttg ttactgttct gagccaaatt 5280

ctaaagaaaa aataaataca tttccttgtt gaaaaaaaaa aaaaaaaaa 5329

<210> 68
<211> 1743
<212> DNA
<213> Homo sapiens

<400> 68
cgtgatgctc tgggatcccg cgcttccgag actcgcagtc tacgcgagct gcctgttttt 60

ttcctgcttg gacgcgcatg agggccccgt ccatggaccg cgcggccgtg gcgaggtggg 120

gcgcggtagc gagcgccagc gtgtgcgccc tggtggcggg ggtggtgctg gctcagtaca 180

tattcacctt gaagaggaag acggggcgga agaccaagat catcgagatg aagattggat 240

aaccaagaaa tgactaatca agagtctgcc gtacatgtga aaatgatgcc agaattccag 300

aaaagttcag ttcgaatcaa gaaccctaca agagtagaag aaattatctg tggtcttatc 360

aaaggaggag ctgccaaact tcagataata acggactttg atatgacact cagtagattt 420

tcatataaag ggaaaagatg cccaacatgt cataatatca ttgacaactg taagctggtt 480

acagatgaat gtagaaaaaa gttattgcaa ctaaaggaaa aatattacgc tattgaagtt 540

gatcctgttc ttactgtaga agagaagtac ccttatatgg tggaatggta tactaaatca 600

catggtttgc ttgttcagca agctttacca aaagctaaac ttaaagaaat tgtggcagaa 660

tctgacgtta tgctcaaaga aggatatgag aatttcttgg ataagctcca acaacatagc 720

atccccgtgt tcatattttc ggctggaatc ggcgatgtac tagaggaagt tattcgtcaa 780

gctggtgttt atcatcccaa tgtcaaagtt gtgtccaatt ttatggattt tgatgaaact 840

ggggtgctca aaggatttaa aggagaacta attcatgtat ttaacaaaca tgatggtgcc 900

ttgaggaata cagaatattt caatcaacta aaagacaata gtaacataat tcttctggga 960

gactcccaag gagacttaag aatggcagat ggagtggcca atgttgagca cattctgaaa 1020

attggatatc taaatgatag agtggatgag cttttagaaa agtacatgga ctcttatgat 1080

attgttttag tacaagatga atcattagaa gtagccaact ctattttaca gaagattcta 1140

taaacaagca ttctccaaga agacctctct cctgtgggtg caattgaact gttcatccgt 1200

tcatcttgct gagagactta tttataatat atccttactc tcgaagtgtt ccctttgtat 1260

aactgaagta ttttcagata tggtgaatgc attgactgga agctcctttt ctccacctct 1320

ctcaacacac tcctcaccgt atcttttaac ccatttaaaa aaaaaaaaaa gctaaaatta 1380

gaaaaataac tccctacttt tccaaagtga attttgtagt ttaatgttat catgcagctt 1440

ttgaggagtc ttttacactg ggaaagtttg tagaaatttt aaaataagtt ttatgaaatg 1500

gtgaaataat atgcatgatt ttaagtattg ccatttttgt aatttgggtt attatgctga 1560

tggtatcacc atctcttgaa attgtgttag gtttggttat tttgtctggg gaaaaaatat 1620

ttactggaaa agactagcag ttagtgttgg aaaaacctgg tggtgtttac aatgttgcta 1680

atcattacaa aacattctat attgaagcac tgataataaa tatgaaatgc aaaacctttt 1740

taa 1743

<210> 69
<211> 2689
<212> DNA

<213>  Homo sapiens

<400>  69
tttattctct ggaacatgaa acattctgtt gtgctcatat catgcaaatt atcactagta      60

ggagagcaga gagtggaaat gttccaggta taaagaccca caagataaag aagctcagag     120

tcgttagaaa caggagcaga tgtacagggt ttgcctgact cacactcaag gttgcataag     180

caagatttca aaattaatcc tattctggag acctcaaccc aatgtacaat gttcctgact     240

ggaaaagaag aactatattt ttctgatttt ttttttcaaa tctttaccat tagttgccct     300

gtatctccgc cttcactttc tgcaggaaac tttatttcct acttctgcat gccaagtttc     360

tacctctaga tctgtttggt tcagttgctg agaagcctga cataccagga ctgcctgaga     420

caagccacaa gctgaacaga gaaagtggat tgaacaagga cgcatttccc cagtacatcc     480

acaacatgct gtccacatct cgttctcggt ttatcagaaa taccaacgag agcggtgaag     540

aagtcaccac cttttttgat tatgattacg gtgctccctg tcataaattt gacgtgaagc     600

aaattggggc ccaactcctg cctccgctct actcgctggt gttcatcttt ggttttgtgg     660

gcaacatgct ggtcgtcctc atcttaataa actgcaaaaa gctgaagtgc ttgactgaca     720

tttacctgct caacctggcc atctctgatc tgctttttct tattactctc ccattgtggg     780

ctcactctgc tgcaaatgag tgggtctttg ggaatgcaat gtgcaaatta ttcacagggc     840

tgtatcacat cggttatttt ggcggaatct tcttcatcat cctcctgaca atcgatagat     900

acctggctat tgtccatgct gtgtttgctt taaaagccag gacggtcacc tttggggtgg     960

tgacaagtgt gatcacctgg ttggtggctg tgtttgcttc tgtcccagga atcatcttta    1020

ctaaatgcca gaaagaagat tctgtttatg tctgtggccc ttattttcca cgaggatgga    1080

ataatttcca cacaataatg aggaacattt tggggctggt cctgccgctg ctcatcatgg    1140

tcatctgcta ctcgggaatc ctgaaaaccc tgcttcggtg tcgaaacgag aagaagaggc    1200

atagggcagt gagagtcatc ttcaccatca tgattgttta ctttctcttc tggactccct    1260

ataatattgt cattctcctg aacaccttcc aggaattctt cggcctgagt aactgtgaaa    1320

gcaccagtca actggaccaa gccacgcagg tgacagagac tcttgggatg actcactgct    1380

gcatcaatcc catcatctat gccttcgttg gggagaagtt cagaagcctt tttcacatag    1440

ctcttggctg taggattgcc ccactccaaa aaccagtgtg tggaggtcca ggagtgagac    1500

caggaaagaa tgtgaaagtg actacacaag gactcctcga tggtcgtgga aaaggaaagt    1560

caattggcag agcccctgaa gccagtcttc aggacaaaga aggagcctag agacagaaat    1620

gacagatctc tgctttggaa atcacacgtc tggcttcaca gatgtgtgat tcacagtgtg    1680

aatcttggtg tctacgttac caggcaggaa ggctgagagg agagagactc cagctgggtt 1740

ggaaaacagt attttccaaa ctaccttcca gttcctcatt tttgaataca ggcatagagt 1800

tcagactttt tttaaatagt aaaaataaaa ttaaagctga aaactgcaac ttgtaaatgt 1860

ggtaaagagt tagtttgagt tactatcatg tcaaacgtga aaatgctgta ttagtcacag 1920

agataattct agctttgagc ttaagaattt tgagcaggtg gtatgtttgg gagactgctg 1980

agtcaaccca atagttgttg attggcagga gttggaagtg tgtgatctgt gggcacatta 2040

gcctatgtgc atgcagcatc taagtaatga tgtcgtttga atcacagtat acgctccatc 2100

gctgtcatct cagctggatc tccattctct caggcttgct gccaaaagcc ttttgtgttt 2160

tgttttgtat cattatgaag tcatgcgttt aatcacattc gagtgtttca gtgcttcgca 2220

gatgtccttg atgctcatat tgttccctat tttgccagtg ggaactccta aatcaagttg 2280

gcttctaatc aaagctttta aaccctattg gtaaagaatg gaaggtggag aagctccctg 2340

aagtaagcaa agactttcct cttagtcgag ccaagttaag aatgttctta tgttgcccag 2400

tgtgtttctg atctgatgca agcaagaaac actgggcttc tagaaccagg caacttggga 2460

actagactcc caagctggac tatggctcta ctttcaggcc acatggctaa agaaggtttc 2520

agaaagaagt ggggacagag cagaactttc accttcatat atttgtatga tcctaatgaa 2580

tgcataaaat gttaagttga tggtgatgaa atgtaaatac tgtttttaac aactatgatt 2640

tggaaaataa atcaatgcta taactatgtt gaaaaaaaaa aaaaaaaaa 2689

<210> 70
<211> 1589
<212> DNA
<213> Homo sapiens

<400> 70
tttctggaaa cgacggcggc ccggcgggtc caaaccacct accatccccg ctcgcgtttc 60

caggctccct acccccgccg ccgccgcctc ttcttccacc actgccaccg cctcctcttc 120

atctgcggcc caccggcggc ggccgccccg aagccccgcc ccctccatcg cctcctgcat 180

catcacttcc ggtaaccccg ccggccgcca gctccaggag ggagaatggg agaggggcaa 240

ggagggaacg agtggaagat tgcgtcacga cgcacataaa gacgtacgaa gccgtcgcca 300

aatgacaacg gcaacacgac aagaagtcct tggcctctac cgcagcattt tcaggcttgc 360

gaggaaatgg caggcgacat cagggcagat ggaagacacc atcaaagaaa aacagtacat 420

actaaatgaa gccagaacgc tgttccggaa aaacaaaaat ctcacggaca cagacctaat 480

taaacagtgt atagatgaat gcacagccag gattgaaatt ggactgcatt acaagattcc 540

ttacccaagg ccaattcatc tgcctccaat gggccttacc ccactccgag gccggggact 600

tcgaagccaa gagaaactga ggaaactttc caaaccagta tatctcagat ctcatgatga 660

agtttcctaa tctagaggaa agtttatttc tgcaaatgat gagccaacta gttattgaat 720

gtaaaccaga tggcaaaaca cttcttgatt aggggcaaaa attcaaatgt cttcttaaaa 780

cctccacaat tgattctccc cctgtgatgt aaacttagat atccctagag tttctcagca 840

tctttcttcc tgagtggatg gcattatccc tagaggtcat ggaccttaca tcctcactgc 900

agtcaccttt ggaaacaaga ccgaggcctg tagaaggaaa gcggaaggat gtaagagctg 960

ttcatgggaa ttccattcac cacacatgcc agctgatgca aagaaatttg ccagtcactg 1020

gaagttactg tggctactat aaaactatgt accactctca gaatattctt aggctttttt 1080

tttttcaagt tatagatttg cttaaagtga ggttttttgg tgtgtgcttt tttgttgtct 1140

tttgaccctt ccagtttcca gacaaacttg tcttaaaaag tgcatcattg gattgaccat 1200

ggtttttcat gaaattttat gagatttctg ccttaacaaa atgcatatat tccttatctt 1260

aaagcctgtc attacttagg atgcacttat aggatctgaa aagctcactt taaactcata 1320

ctacattcgt tacgagtatt ttacgttaac ataattgaaa agtacaaggt ccaagctggc 1380

tttcaaatta tgtctaaaca gaaatgggac aaatagactt gaaaatagaa gggatttatt 1440

ccacccctgc aagggtagag tcaggtgaga gtcccttggt gagtcatttg tacatcagtg 1500

tcatttcttc ttaacctctg aagaagatgg gcatcagaaa taaagacaaa gcactatcaa 1560

aaaaaaaaaa aaaaaaaaaa aaaaaaaaa 1589

<210> 71
<211> 4186
<212> DNA
<213> Homo sapiens

<400> 71
acactcttca cacgttttct gagtgtgagc tttattaaat ctttagcatt aagatactat 60

tctactttac tgagtttaaa actatttttc tgaaatcatt gtaatgataa ggaaatgttg 120

cgatcaaata agatttagac acactttttt ttccaatagg taatttgtga aagaagcaaa 180

aataaatctg tggaactgaa ggagcggatc agagagtatt taaagggcca ctactacgtg 240

cccctcctga gagcggaaga gacttcttct ccagtcatcg ggagctgtgg gtccccagac 300

cagacggctg aggcctctca cgtcagccgc tatggaggca gccccagaga cccggtactg 360

accctgagag ccttcgcagg gcccctgagt ccagccaagg cagaagattt tcgcaagctc 420

tggaaaactc cacctcgaga gaaagcaggc ttccttcacc acgtcaagaa gtcggacccg 480

gaaagaggct ttgagagagt gggaaggtac tgaaaggctg agccgcagca gcgtggggca 540

cacagttggt tgcattttga ttaatgacag aaggtcaaat cacagcttca aagtaccaat 600

ttccagaagg cttttctggg agacttggtc cttttcgtga aggtctaatt ccttgtggat 660

gttgtggaat tctagcttaa gccagtcccg tcccagacct gggggatgtc tgcagtgcct 720

tcctttcctt ttctaattgg aattccttgt tggttctggg acatgtttct aaggaacctt 780

aatattttat agaaggtacc atataatgct atggattcct tgggaaaaaa taggccatag 840

ccacaaagct aaattatgct gtaatcaaat aaagcttcgt ggaattgttt agaatatttt 900

ctcaaaatat gtgcccagtg tcaggtgtac aactggaacc cagaaaggac ctccaggctt 960

tcgtgtgcct ggccatgact agaaccacgg gcaagttcac agggctgttc cgtgatcatg 1020

aacacgtccg tctgtgggag ttgaactgtg tgacctgggt tgctggcgtg agggtctgtc 1080

gggctgaggc ctccttcctg cctgcagccg gttcacggct cctgagagct gtgccgggtc 1140

ctgcttacct gtgaggaggc gtgctgtttc agaaaatgca ccgtatgatt tcagctgcca 1200

gttaaggata aaggaaaatt ataaccatga ttgcagacat tgctttggta cttgaagctt 1260

tagtggcttt tttctgtgta gttctgagta ttttttctgg tttcacagct ggttaattgt 1320

gttttgtttc ttacagggag ctagctcatg agctggggta tccctgggtt gaatactggg 1380

aatttctggg ctgttttgtt gatctgtctt cccaggaagg cctgcaaaga ctagaagaat 1440

atctcacaca gcaggaaata ggcaaaaagg ctcaacaaga aacaggagaa cgggaagcct 1500

cctgccgaga taaagccacc acgtctggca gcaattccat ttccgtgagg gcgtttctag 1560

atgaagatga catgagcttg gaagaaataa aaaatcggca aaatgcagct cgaaataaca 1620

gcccgcccac agtcggtgct tttggacata cgaggtgcag cgccttcccc ttggagcagg 1680

aggcagacct catagaagcc gccgagccgg gaggtccaca cagcagcaga aatgggctct 1740

gccatcctct gaatcacagc aggaccctgg cgggcaagag accaaaggcc ccccgtgggg 1800

aggaagccca tctgccacct gtctcggatt tgactgttga gtttgataaa ctgaatttgc 1860

aaaatatagg acgtagcgtt tccaagacac cagatgaaag tacaaaaact aaagatcaga 1920

tcctgacttc aagaatcaat gcagtagaaa gagacttgtt agagccttct cccgcagacc 1980

aactcgggaa tggccacagg aggacagaaa gtgaaatgtc agccaggatc gctaaaatgt 2040

ccttgagtcc cagcagcccc aggcacgagg atcagctcga ggtcaccagg gaaccggcca 2100

ggcggctctt cctttttgga gaggagccat caaaactcga tcaggatgtt ttggccgctc 2160

ttgaatgtgc agacgtcgac ccccatcagt tcccggccgt gcacagatgg aagagtgctg 2220

tcctgtgcta ctcaccctcg gacagacaga gttggcccag tcccgcggtg aaaggaaggt 2280

tcaagtctca gctgccagat ctcagtggcc ctcacagcta cagtccgggg agaaacagcg 2340

tggctggaag caaccccgca aagccaggcc tgggcagtcc tgggcgctac agccccgtgc 2400

acgggagcca gctccgcagg atggcgcgcc tggctgagct tgccgccctg taggcttggc 2460

gctgggctct cggtttgttc ttcattttta aagaaggaag ggtcatatgt ttattgctaa 2520

actgtcaaaa aggaatatat tctgattaaa ttattactcc tcactttgag ggtgtgagaa 2580

ttttagaaga tttaaatgtt ctatataaca cttagatttc tgatattttg gaagaagtta 2640

gaagttaatg aaagcaaact cagttaccaa ttttctggaa aatatccatg tggtaatggt 2700

agacttttta ggtggcaatt tctaggtctg aaatatagca gaggaaaggg cgctgaggca 2760

gttgcaggca ggcagccctg tacttaccct gtactcacct catccgacag acgctgtgga 2820

tgaggagggg cttggcggag gcgtgagcac cgatgtccct ttgataacct gcactcacca 2880

agatgaacta tttgccgccc tgtcttttcc tgggttgggg ggtggcatct gatggtggca 2940

gagtgcctgt tggttcgccc gtgggtctca tggttcagac agagggaggt ggacggcagg 3000

gatcagggag ccaggagcgc gcctcagact tgcagcaacc attgtgattt gggttgttcg 3060

gaatatttaa attactgatc agaagatgaa agtagctttt ctcttgggaa gtcttgcagc 3120

ccgtgggagt gataccagga gcaacacaga gctcagcagc ggcgccaagg tgttccctgt 3180

ttcctcagca cgtgagcctt caccgcctgc ttcattcagg agccagtgca gcagtaatac 3240

agtctataca ttgttctgtt ttcaaattta tcctgaggct ttgttgagca taaatgatta 3300

tacgataaag gtatccgtta ttttggaact catttcagtt gggatctcct gtatgcagag 3360

tgttgcattt agaggtttga gtcccatctt ggtttcttgc cgtgctgact gtagccttca 3420

ccttgacttg aatgaaggtc tgtggttgga atgtgtgagg agccgctgag gtgttcagga 3480

ggtgctgcct ggaggtcggt ttcttcctgg gtgttacggg caactgctca cacagttgtt 3540

tctctgtgaa catttccagt gtttaatcca aaatgaaaac ccaccaatgc ttttgctaac 3600

ttcagtgcct tttataaatc atttttaaat ttcctgaact tgctttttga ggatatacag 3660

ggatattaag tagacgcagg attgtttttg tttgtaaaaa ttctgaattg aaactttgtt 3720

ttaaaaaaag gcttctttct ttcatatgac aagagatagg tcaggaatat tggaatcaag 3780

atttaaatgt taaaattcga ttttgttaca cagggtgtgt tcatttgttt tgtagcagac 3840

aagatctaga tcccagacag aaacaacaca tgctattcta aaaagccgca ttttaaaggg 3900

caccttggtt ctcaaaagaa atcagaatat ggatattcgt agtgatgatc tgttttctct 3960

aaaatcttac catattgtct gtatatggtt gtaaattcaa atggaaagta aaacgttttg 4020

gccctgattt tgtatgtgga ccactgctcc tgatttccca ggtcttaggc cacctttgac 4080

tgtttctccg tttgtttgtg ggcagcgatt ccagtcccaa cggaggcatt ctcgtgtgtc 4140

ccggggggtt atgtccttca caaaacactt aatgaaatga attact 4186

<210> 72
<211> 5457
<212> DNA
<213> Homo sapiens

<400> 72
ctgttcccct tgctgtgggg gtaaggaatc aagcccccaa gatggcggca gcggcggagg 60

agcggatggc agaggaagga ggcggcggcc aaggcgacgg cggttcctct ttggcctccg 120

gctctaccca gcgacagcct ccaccgcccg cgccacagca cccgcagccg gggtcccagg 180

cgctcccagc ccccgcgctg gctccggacc agctgcctca aaacaacacg cttgtggcgc 240

tgcccatcgt agccatcgag aacatcctca gctttatgtc ctacgacgaa attagccagc 300

tccgcctggt ttgtaaaaga atggacttgg tctgccagag aatgttgaat cagggatttc 360

tgaaagtgga gaggtaccat aatctatgtc agaaacaagt taaagcacaa ctcccaagga 420

gagagtcaga aaggagaaac cattcattag ctcgtcatgc agacattctt gctgctgttg 480

aaacaaggct gtcactatta aatatgactt tcatgaaata tgtggattcc aatctctgtt 540

gcttcatccc aggaaaggtg attgatgaga tttatcgtgt gttgagatat gtcaattcta 600

ccagagcccc tcaacgagct catgaagtac ttcaagaatt aagggatata tcctctatgg 660

caatggagta ctttgatgaa aagattgttc caattttaaa gaggaaatta ccaggatcag 720

atgtttctgg aagactcatg ggctctcctc cagttccagg accgtctgca gccctaacaa 780

caatgcagct cttctccaag caaaatcctt caagacaaga ggttaccaaa ctccagcagc 840

aggttaaaac aaatggtgct ggcgtgactg ttctcaggcg tgaaatttct gagcttcgca 900

ccaaagtgca agaacagcaa aaacagcttc aagaccagga ccagaaactg ctagagcaga 960

cccagatcat aggtgaacaa aatgcacggt tggcagagct agaacgcaaa ctacgagaag 1020

taatggaaag tgctgtagga aattcctcag ggtccgggca gaatgaggag tctcctcgga 1080

aacgaaaaaa ggccacggaa gccatagact ctcttaggaa atctaaacgt cttcggaata 1140

gaaagtaaat tggaatgtgg ttctagttcc agaggaagac acagcttagt agcttaagca 1200

gttatgcata tcaggatact gtgagcaaca tggtgtccct taagcttcta ggctttcaga 1260

acacaactta aacttgaact cttatggttg ggacattctt ttcctgcttc tcctgattga 1320

actgatgcac agaatctttt tactttgcaa tagatttgta ctaaccagac ctgttctatc 1380

atgttttgag gagttaactt ttttctgtgc agataatgtt gaaagtaagt taatttgttt 1440

ctgcatatat gcacatacat acgtaaggat cttaaaccca gtcttgacag actagaagtt 1500

aagtttaata cagaaaatgt cctgttcact tgaaagaaaa gacctacttt ttaaatggac 1560

actatcttgg ttcttttttt tttaagtcat ctttttgtta taagtgacct ttgtctggaa 1620

tgtctgaaaa gtagttaatg ctttttggta ttgaagtaat gggtaactaa aatggacttc 1680

catagtattg actgtagaag gagcctctac aatattgact atatattttt ataaactact 1740

ggcaaggaac ttacccagct gttatacatg ttttcagttc tctttggggg ccatgtccta 1800

catttgcatg gatggatgca tgcattgcct agtcaactca cttaaaaagc tcttgcactg 1860

gtattgatct tgaacctcta tacttctcca ctttttagag cggcgtctta gtttatttaa 1920

acgcttaaca tcttccaccg caacagcttg cctctagagg gggaattgtt catccatttg 1980

tcctccagtt ttataggaac aagagactat taaagcccat tgctttatct gctctctgta 2040

gggttcaggt acactggcta aggcagaacc ccaaatttca aggctctttc tatcaatatt 2100

ggacctctgg gagctaatac tcctctttgc tcaccaccat gtgatcattg atcagatgtg 2160

gtgagtgagt aggggactcg acttgcggga tgcagttttg ccattgcagc aaagttcatt 2220

gaggaaaatt gtaccatgaa aaagcatttt gattatttca tttctggagg atgaccttga 2280

gggaagcttt ttaatattag tttcaagctt tccttaccct acaactttaa acaaaagctt 2340

taattttgtt tgcactccta ttttgagctt gtaactggaa aagcatgtct tgcactgttc 2400

aacctcctga gtggtcgctt taacaacctc caaattgtgt acagtggtta ttttccccag 2460

ttgtatagtt ttgagacatt caattatcac tgtcttagtt gtattttaat ttactaaatt 2520

atgtagctat ttgactgtta agttctttta aacaactgtt tccttgggct tcagttattt 2580

aaagtaaatt taggtgtaat atagaaaact gtccctttcc aggtgggagc ttaacacctg 2640

tataaaacct aaggttttgg taagtacctt agttgaataa aagcacaagg ttatcacctt 2700

ttttatccgt ccaccgtgac atggttatgc atcctttaga ttaacctcac caaatgaagc 2760

tttttctatc catttttaat attgtcctta cataatattg tccttagatt ttgatcaatt 2820

ctatgtctga ctttgaaatt ccatttacaa tgtagtatgt tttcaatgaa aaaccataaa 2880

gtaacatcca agtgtttcat ggtttgttgg gaaggtaatt ttaaaataaa acaatttcca 2940

aaaaacattt gtcagccaag gtcatccata aaagtccccg tctggaactc gttttctcag 3000

cacttctcat ttttataatc atagtattta gccatattag catatcttta gaggaggcct 3060

ggtggaatac ccggggcctc tactagtctg cttttccggt cataggaaga cttttttttt 3120

tacgtatcaa agcatgtgtt tacagattta cagagtgtga tccgtatgca tcaaagggat 3180

acagccccag actgtttctt taatggtcat tcatgaacta ttaaaaattt cctgaatttc 3240

tttttaaact acatctagat agcctaaaca tctatgtagt cttccattag ttaacatgtg 3300

tatagggctt tcataataag tagtcacact gttacaaggt aaattttcta ttacaaaatc 3360

caaatttcta ttgcatacac ttataatcat ctattcataa agctaattca ttgtacttgt 3420

taatgcatga atgttccatg cagtaggaga ataaagcact acagttcggt tgttcaaatc 3480

ttaaatctta ggctatttag ctaaaacatg ttaggatatt gccttcacca cttgttagga 3540

cagttatttg atagccatta tgcttaccta tggatagcat aatggtattt aaggttcaac 3600

aaaccgcaga ttattatgat cagctagaac tgacatatgt tacaagaaaa ctgtaaatta 3660

acctcctgag agaaccattc tgccctgcat tttaagtcag gtatttaaat actgcgtgtt 3720

gttaatattg cgttgtctta gtgcaacatt tcttaaagta ttcccgttat cttccttggc 3780

tgtttttcag aggagacgtc tccatgttaa tgggtccttc ccacatagaa atgtattctg 3840

gtacagcgct gcacacatgg atagcaatgt gaaaaggcat gcctaagaga cagaaacggc 3900

tgaccttacc ccaccccagc cggatcctct gcttatctcc cacatgcact tggcttaact 3960

ggttttcaca gattctgagg ttatagaacc tgttgttttt caaactcctg tacagagcct 4020

ggaaataatg gagcaaacgc aaatgtattg agagtcatta tactttaaaa tagttttaaa 4080

gactattggg gtaccatcag gtcaaaagcc actgatttgg gaagcaattt ttttgcaaaa 4140

cacttttttt ggagtactga actttacagg ggcttgcctt aatctctatt tagtagtttc 4200

aagatatatg cgcaattatt ctacgtgtta aaaatgttaa aatattctat gtagccccaa 4260

aggtgggtaa aacagtataa gtaatgccta aattaataag ctaaaaggtg tcactacagc 4320

tggatttttg agagaaaatg gacataaaac taggctatgt gtaataaaaa taatggcacc 4380

ttaagattgc actattttat gcattatttt atatgccatt tcatagcctg cactttaatc 4440

tccaaagaaa cgatgtatga tgtcccactt gttccttatt cttaataaac tttttcctga 4500

gacagggcac ttaattctat tttactatac tgaattagtt tcttggaggt agattcattt 4560

cttgagttct ctgtttcctt agccataata ttttctagga tccgggaact ccctatattg 4620

tctatttcca acattatata attttaattt aatgtaatgt aaatctcata cttggtgatc 4680

agcacattct tatatcctgc tctagcaatg agtacccatt tgttacactt accaaactct 4740

ctaaaaacat atactgtgag taagatttga agccaggaat aaaaggcagt ctgaaagaaa 4800

acgtcctata cgatgcagtt gtttggaaat ggctgatttc tgatatgaaa cccaacttga 4860

tacactgttt ggttctttga cttgggatct atctgaattg ctttcactag tacacaaaag 4920

gttaagttaa aactacttta aacttgtata gcctgccatc tgaggtgaat tattttcaaa 4980

aaatactaaa tctatgaact gagactagca attagttggc tgctccctac catgatttta 5040

cttttaatag caatacagtt atattggtga taaatatgac aggcttaagt actgctgtcc 5100

ttttgcatct tcctaagcat cttggttaaa tttctgaagt ttaaaaatta gatccagatt 5160

tcttctgttg tgtctgaaga gaaccatgac atttagaagt atattggtaa tctattaggt 5220

ccattggcaa agtatattgg tccatattca aacctattcc aaaaggttag aagtgtttaa 5280

gattccttta ttgtggtacc atgtctgttt acctatgctt atgagcaaca ataaattact 5340

agtttacctt ctggaatttt aattgttata accgaatcaa ttattggaaa gtgaaaaaca 5400

cctcccggtc acacaacagg gtacgtaaat aaatgtgttg ttaccagtga aaaaaaa 5457

<210> 73
<211> 2586
<212> DNA
<213> Homo sapiens

<400> 73
gcaagctggg cggaaccgga agatggtgtg agccacgggc tgccgggggc ctggggctcg 60

gcgtcggtcc ccgggggatg tggagagctg gcagcatgtc ggccgagctg ggagtcgggt 120

gcgcattgcg ggcggtgaac gagcgcgtgc agcaggctgt ggcgcggcgg ccgcgggatc 180

tcccagccat ccagccccgg ctagtggcgg tcagcaaaac caaacctgca gacatggtga 240

tcgaggccta tggacatggg cagcgcactt ttggcgagaa ctacgttcag gaactgctag 300

aaaaagcatc aaatcccaaa attctgtctt tgtgtcctga gatcaaatgg cacttcattg 360

gccacctaca gaaacaaaat gtcaacaaat tgatggctgt ccccaatctc ttcatgctgg 420

aaacagtgga ttctgtgaag ttggcagaca aagtgaacag ttcctggcag agaaaaggtt 480

ctcctgaaag gttaaaggtt atggtccaga ttaacaccag cggagaagag agtaaacatg 540

gccttccacc ttcagagacc atagccatcg tggagcacat aaacgccaag tgtcctaacc 600

tggagtttgt ggggctgatg accataggaa gctttgggca tgatcttagt caaggaccaa 660

atccagactt ccagctgtta ttgtccctcc gggaggagct gtgtaaaaag ctgaacatcc 720

ctgctgacca ggttgagctg agcatgggca tgtccgcgga tttccagcat gcggttgaag 780

taggatctac aaatgtccga ataggaagca cgatttttgg agagcgggat tactcaaaga 840

aacccacccc ggacaagtgc gcagcagacg tgaaggcccc gctggaggtg gcacaggagc 900

actgagccag ggaatactga gagcactaac tatgcactaa cctagatttt catttcgata 960

ttccctgtgt cccagcgcag tcctgctctc ctgtgacctg tggagagcac taatgatcac 1020

gtgtgttgat ggaaaccatc tgtgcttagt ctctgacata ggaagcttgc ttcaggcaat 1080

ggctttggat tgagtttgag aaattcaaac atttctgcag aacagatacc aaatcaatag 1140

ctaggaatca tgttcaatat tgaattctgc ccaggagcat gaactgatcc atgaatgcct 1200

tttccaggtt aaaatttggt cactgatgcc tataatcgtg gaagtcagag ggattcccct 1260

ttttcatctc attttaatag gaaaattcct tatggttaac atctccctac aaactcctac 1320

tacgtcgtct aaattgctgc tctggaataa ggtgatttct gcccccagat tcttccctag 1380

ccggtagata cgtgaagata ttcccaactg tggaatggca gtgtaggtag cttcaggaaa 1440

tggctcaggt taattctcaa aacacaaatt gttgctggcc aggcatggtg actcatgcct 1500

gtaatcccag caatttggga gacagaggcg gaaggatcac ctgagcctag gagttcaaga 1560

ccagcctcag caacagcagg agccccaccc cccgtctcta caaaaaaatt taaaaattaa 1620

ctgggcatgg tggctgaggt ggaagaatgg aagaatcact tgagcccagg agtttgaggc 1680

tgcagtgagc tatgattgca ccactgtact cctgccttaa aaaaaaaaaa aaaatcccaa 1740

tagtccatga aggctttgat ctcttgggaa gttcttcata gatgctgtca catttcttaa 1800

agcaaccttt taatatgcag ataatacccc ccaacttttt tagagacagc ctgtctctta 1860

aaaaaaaaat taatttggta gtgagagctt gtgtcactgc cactctgttt tatccctgaa 1920

attaaaggat aacataagga ggacttgggc ctttctgaca tcatcctgaa gagacaggac 1980

tttgcgtttt tcctctggga cctacagtga tgagaattta atgattatct cctccactat 2040

aatcctcttt agggtgattt tttaaatcaa aacccagtga atctcattac tcctaagaaa 2100

cgaaagattc cttcaaagcc ttttcaggca catggtttca acaaagcctg gctttgacat 2160

tccttgtcct gaggagcact ttccaggcat agttacagct tccccactgt atttacaagc 2220

cagaattgtg caactcttct ggatcattaa taaagtagca agatcctcaa aaaacccaaa 2280

aacaccattc tctaatagtc atgacaaatg gcttcagtat ggcttgtttt ttattttcca 2340

gatggctttt tctcttattt tttgaagccc cagtctttga ttttacaggt aactttcaaa 2400

acatcatgat gctgccaaat gtacttttgt aaacttaaac attatgattc ctgtattatt 2460

tcagtgagag ctacagtgtg atatttcaga gtctattaaa taaaaatgtg agtttgaatt 2520

acaccatctg tgccaattac aaagcaatta aaagatttat tttttatgaa aaaaaaaaaa 2580

aaaaaa 2586

<210> 74
<211> 1049
<212> DNA
<213> Homo sapiens

<400>  74
aaaacaacag gaagcagctt acaaactcgg tgaacaactg agggaaccaa accagagacg      60

cgctgaacag agagaatcag gctcaaagca agtggaagtg ggcagagatt ccaccaggac     120

tggtgcaagg cgcagagcca gccagatttg agaagaaggc aaaaagatgc tggggagcag     180

agctgtaatg ctgctgttgc tgctgccctg gacagctcag ggcagagctg tgcctggggg     240

cagcagccct gcctggactc agtgccagca gctttcacag aagctctgca cactggcctg     300

gagtgcacat ccactagtgg gacacatgga tctaagagaa gagggagatg aagagactac     360

aaatgatgtt ccccatatcc agtgtggaga tggctgtgac ccccaaggac tcagggacaa     420

cagtcagttc tgcttgcaaa ggatccacca gggtctgatt ttttatgaga agctgctagg     480

atcggatatt ttcacagggg agccttctct gctccctgat agccctgtgg gccagcttca     540

tgcctcccta ctgggcctca gccaactcct gcagcctgag ggtcaccact gggagactca     600

gcagattcca agcctcagtc ccagccagcc atggcagcgt ctccttctcc gcttcaaaat     660

ccttcgcagc ctccaggcct ttgtggctgt agccgcccgg gtctttgccc atggagcagc     720

aaccctgagt ccctaaaggc agcagctcaa ggatggcact cagatctcca tggcccagca     780

aggccaagat aaatctacca ccccaggcac ctgtgagcca acaggttaat tagtccatta     840

attttagtgg gacctgcata tgttgaaaat taccaatact gactgacatg tgatgctgac     900

ctatgataag gttgagtatt tattagatgg gaagggaaat ttggggatta tttatcctcc     960

tggggacagt ttggggagga ttatttattg tatttatatt gaattatgta cttttttcaa    1020

taaagtctta tttttgtggc taaaaaaaa                                      1049


<210>  75
<211>  7585
<212>  DNA
<213>  Homo sapiens

<400>  75
   aaaaagtgct ttattttccc actccccgga cgcccagcag ggcagtttct tgaccttcgg      60
   agcccctttc ccgaggatcc gctcgggagc ctcccctggc caggagcagg ggattagtct     120
   gccccgcgac cggccccagc cacgacgcgg acatcgcccc ctctgtctgg gccgctgtca     180
   ctcacgcgcc aaagggccac ggagaaagaa ggggcgggcc ggggcgggcc gggcgagcgg     240
   aggcggggac ttgcgccgtc ctgaggctgc ctcctagggt ccggccggcg ctggagctgc     300
   ggatttagat tgtcactgcc acctcggtcg gtgcttactt cgctgccagc tggtcgtcgc     360
   catgaacccg gacctgcgca gggagcggga ttccgccagc ttcaacccgg agctgcttac     420
   acacatcctg gacggcagcc ccgagaaaac ccggcgccgc cgagagatcg agaacatgat 480
   cctgaacgac ccagacttcc agcatgagga cttgaacttc ctcactcgca gccagcgtta 540
   tgaggtggct gtcaggaaaa gtgccatcat ggtgaagaag atgagggagt ttggcatcgc 600
   tgaccctgat gaaattatgt ggtttaaaaa ttttgtgcac cgaggcggcc ctgagcctct 660
   ggatcttcac ttgggcatgt tcctgcccac cttgcttcac caggcaactg cggagcagca 720
   ggagcgcttc ttcatgcccg cctggaactt ggagatcatt ggcacttatg cccagacaga 780
   gatgggtcat ggaactcacc ttcgaggctt ggaaaccaca gccacgtatg accctgaaac 840
   ccaggagttc attctcaaca gtcctactgt gacctccatt aaatggtggc ctggtgggct 900
   tggaaagact tcaaatcatg caatagttct tgcccagctc atcactaagg ggaaatgcta 960
   tggattacat gcctttatcg tacctattcg tgaaatcggg acccataagc ctttgccagg 1020
   aattaccgtt ggtgacatcg gccccaaatt tggttatgat gagatagaca atggctacct 1080
   caaaatggac aaccatcgta ttcccagaga aaacatgctg atgaagtatg cccaggtgaa 1140
   gcctgatggc acatacgtga aaccgctgag taacaagctg acttacggga ccatggtgtt 1200
   tgtcaggtcc ttccttgtgg gagaagctgc tcgggctctg tctaaggcgt gcaccattgc 1260
   catccgatac agcgctgtga ggcaccagtc tgaaatcaag ccaggtgaac cagaaccaca 1320
   gattttggat tttcaaaccc agcagtataa actctttcca ctcctggcca ctgcctatgc 1380
   cttccagttt gtgggcgcat acatgaagga gacctatcac cggattaacg aaggcattgg 1440
   tcaaggggac ctgagtgaac tgcctgagct tcatgccctc accgctggac tgaaggcttt 1500
   cacctcctgg actgcaaaca ctggcattga agcatgtcgg atggcttgtg gtgggcatgg 1560
   ctattctcat tgcagtggtc ttccaaatat ttatgtcaat ttcaccccaa gctgtacctt 1620
   tgagggagaa aacactgtca tgatgctcca gacggctagg ttcctgatga aaagttatga 1680
   tcaggtgcac tcaggaaagt tggtgtgtgg catggtgtcc tatttgaacg acctgcccag 1740
   tcagcgcatc cagccacagc aggtagcagt ctggccaacc atggtggata tcaacagccc 1800
   cgaaagccta accgaagcat ataaactccg tgcagccaga ttagtagaaa ttgctgcaaa 1860
   aaaccttcaa aaagaagtga ttcacagaaa aagcaaggag gtagcttgga acctaacttc 1920
   tgttgacctt gttcgagcaa gtgaggcaca ttgccactat gtggtagtta agctcttttc 1980
   agaaaaactc ctcaaaattc aagataaagc cattcaagct gtcttaagga gtttatgtct 2040
   gctgtattct ctgtatggaa tcagtcagaa cgcgggggat ttccttcagg ggagcatcat 2100
   gacagagcct cagattacac aagtaaacca gcgtgtaaag gagttactca ctctgattcg 2160
   ctcagatgct gttgctttgg ttgatgcatt tgattttcag gatgtgacac ttggctctgt 2220
   gcttggccgc tatgatggga atgtgtatga aaacttgttt gagtgggcta agaactcccc 2280
   actgaacaaa gcagaggtcc acgaatctta caagcacctg aagtcactgc agtccaagct 2340
   ctgaagtgtc acaaggacaa gtttaatctg cttcagaaag cgcctgtgtg caactcaaat 2400
   tttgtggaat ctttttcgaa ttcaaatagc tatagagcaa atgataaatt gacccctttt 2460
   tataaatgga gggaaaaaat gaacagattt cagagattaa atgaaaaaaa gcagatgttt 2520
   taagtgcaat taacactgaa agagacctgt taaaccattc agaaaaagct taagaaatgc 2580
   gatatgactt ccttttgtaa tgctgctgat cccagtagac tatgactttt gataattagc 2640
   agaatttaac tactgagtag ttgattattt tcacatttta attgctaatc actggctata 2700
   taagtgtttt taagcaaagg tatttttgaa gtggtgtaga acccttccaa gctttcctgc 2760
   tcagtgttct accagactta ccctggggcc tggcttaaaa gcaggattga agaaaaggga 2820
   ctgggggaag gaaacttatt ggaaaacttg atgcgaatga gtttctgctt ggcacagtct 2880
   ctgcctgctt gctctccttt gctgatggat tgcatttatc aaactattca tgctagcatt 2940
   tttccaacga gggaacttat tccgcacggg cctactgtag gaccattgtc tcgtgtaatt 3000
   aggaattttc catttgaagg attgctaaat tgtcacagta gtaggaagta tagggaaacc 3060
   tctcagctgt ggcactgttg tagctttgga gtgcagagtg taactctggg acaatcagat 3120
   ttcacatatt ctgtcatctt ggcataagcc attaaaagct tggagattac tgtatttggc 3180
   attaaaaaaa aatgtcactt aggtcagcac tcccagacgt agcacagaaa aaccctttga 3240
   cacaaaccat gtgttctgat ttttggttca gaaaatattg aaactgtgag ttgttttttt 3300
   tttaacaact gggaaaaaac aaaaacaaaa aactatagtt agaaaaatgg aagttccata 3360
   ggttctattt cttactctat gtatggcttt gttttcagtc tatttctagg agctttctct 3420
   gaatcgctaa ttgtcctttc agttgaaatc taatttatac aatcattcta tacttaaagg 3480
   ttaaatacat cttaattaat tttttcttaa agtcaatgta agtcactttg ttttgttttt 3540
   ttttaatcta cgccatatgc ctcatgaaac cagctgttct agaatcagtc ctgagaatat 3600
   ggcttaattc catggaaaca taactcctat cttgggacct gacataatat ctatctatcc 3660
   tggggaactg gtaatatgag acttataggt tacagcagaa atgctacatg ttgacaaaag 3720
   ccttaatcgt tccactggga gaactaattg ataattgtgt taagattgaa gattaaccct 3780
   gtgttaatct cacttgagtc tatcctgaca gtagttcaga ttctggaaaa tgataaactg 3840
   acctgctaga tgtagaattg tttcaaaatt agtgttgaaa taccttgttc acagatgaat 3900
   atctgggcag gatctgaggg tgtttggaat gacacccccc aatccagttg catagatggg 3960
   atgtctttgc aggtttgagg agatcatcga cctgcagagc cccctttgac ccagtacctc 4020
   acgttttatt taaaatctaa atctggggcc aggcgtggtg gctcacgcct gtaatccaag 4080
   cactttggga ggccgaagcg ggtggatcac ctgaggtcag gagtttgaga ccatcctggc 4140
   caacatggtg aaaccccgtc tctactgaaa aaaatacaaa aattagctgg gcatggtggt 4200
   agcacgcgcc tgtagtccca gctactcggg aggctgaggc aggataatcg cttgaacttg 4260
   gcgggtagag gttgcagtga gccaagatgg tgccactaca ctgcagcctg ggtggctgag 4320
   cgagactccg tctcaaaaaa aaaatctaaa tctgacattt gatgctattt ttattaatat 4380
   tggaatgttc tgtcttgaac tttattcaat ataatcaaga ataaagatag agtaaacgtc 4440
   actgatttgt actattaaga gagaaaaaat atgccacaca actaaacata ggtttaaatt 4500
   atgaagaaat ttagaataga ggtttattag atttagggaa cactaagaac aaaaaaggaa 4560
   ggagtgatac ctgcctgagt ggacagctgt aaatcagctg taattactgc agttgtacca 4620
   atagttgtga gtggctccag tcactttagg agtccttgga agtacttggt acacatttgt 4680
   tggctgtacc ttaaaggaag tggcaagtcc agtttgttct ctctaccaca ctagactgcc 4740
   actgacaagt ttgggtctgt tggattcaaa attttgtaag ccattttcac aagtacaaag 4800
   atacatttta accttgtctt ctccaaaatt actgagtagg aattttattt ttatcttttt 4860
   gagacagggt atcactgtca cccagactgg agtgcagtgg tgggatcttg gcttactgtg 4920
   acctctgcct cccgggttca aatggtcctc cctcctcagt ctcctgagta gctgggacga 4980
   caggcacgtg ccaccatgcc cagctaattt gttctatttt ttctgtagag acggggtttt 5040
   gccatgttgc ccaggctggt ctcagactcc tgggctcaag cgatcatttc gcctcagcct 5100
   cccaaagtgc tgagattata ggtgtgagcc acagcatctg gcccagagtg aggagaatta 5160
   atgagatttt ttgtgtgtgt tagataatat tgatttaagc ctttttttaa aaagtactct 5220
   caaccaaata caaaattgaa aatgtgaggt ttaatagaaa tgtgttggct atttgcaatg 5280
   gattttcttc ttgcccaagt gtttggagtt ctactttatg ctctgtattt aaaaattagt 5340
   gacctcaaag cagagttgat gacacaggct ttggggccct catgttcttg tctttagaac 5400
   atatcactac taagtatcag cttatcttca gaacattaca acattcaccg tgttcatatg 5460
   ctttctgaga agtcaccact tgtaatttca gatcacatac acctgaaggc attttatagt 5520
   tcctaaagtt aacatgttag atcttttttt tccaccccat gagggtctca ctctcaccca 5580
   ggctggaatg cagtggtgtg attgtagcac actttggcca ccaactcctg ggctcaagtg 5640
   atcctcctgc tttggcctcc tctgagaagc tgggattact ggtgcacacc accacacctg 5700
   gctaattttt tatttttttt tatttttgga aatagggtat ggctatgttg ccttgggccc 5760
   gtcatgaact cctagcctca ggtgaccctc ccacctcagg cctcccaaag ggttgggatt 5820
   acaggagtga gctactgcac tgggccaaca tgttaatttt tttttttttt tttttgagat 5880
   ggagtctccc aggcgggaat gcaatggtgc gatctcgact tcactgcaat cttcacctcc 5940
   cgggttcgag cgattctccc acctcagcct cctgagtagc tgggattaca ggcacctgcc 6000
   atcacgctcg gctaattttt gtattttgag cagagatgga gtttcaccat gttggccagg 6060
   tgatctgcac tcccactccc cccacacttc ccaaagtgct gggattacag gcataagcca 6120
   caagccacct cacccagcca acatgttaca tcttaattct tggattttct tcactgcagg 6180
   gctttgggtg gagaaataaa actcttcaaa tgcatgatct tggagatccc tgtgaatcaa 6240
   taattcttta gacaactgcg gctcaaaatc cctcctttcc cttttccgag ttattccatc 6300
   catcttatta gaaaggaagt gaattaggtg taggtggtct gtaacacctg cacatctttt 6360
   tatacgtgta gagggtatgc ctgggatata taggttgtct tcaagcagta gctgctacta 6420
   cagctagaga gaggaggagt gccaggaaac tgatgacctg agaccaagag tcttgttgat 6480
   gttctgactt agataaaggt tttgatcatt ttcatgaaat aatgcaggga agtcatttct 6540
   gctgtttctt tactactcca ttcttggagg attagaacaa gtcacactgt aattgactaa 6600
   aacgactttt tattttaaaa tattgatggt ggggttttgc tttttttttt tttttttttt 6660
   ttgagacgtt gtcttgctct ttcacccagg gtagagtgca gtggcgaaat cttggctcac 6720
   tgcaacctct gcctcccggg ttcaagcaat tctctgcctc agcctcctga gaagctggga 6780
   ttacagcgcc tgccaccatg cctgtctagt ttttttgtat ttttagtaga gacaggggtt 6840
   tcaccaagtt ggccaggctg gtcttgaact cctgacctcg tgatccaccc acctcggcct 6900
   cccaaagtgc tgggattaca ggcgtgagcc accgtgccgg gccagtgttt ttaactgtcc 6960
   acacttacta aattttccag tacttcttcc tatggttcat gatagtaaac acagaaacat 7020
   ataaggaaca ttattagagt taccagatgt tagctgttgc atatgtgcac attcctagat 7080
   tcaagggtgt tttgccttaa aatttaagtg ggtcattggt tgtcttggga agatcatgga 7140
   aaattcggga ttttttagaa ttctgaacca aaatatgttt tgagtgtttc ttagtaaatg 7200
   tgtgatcttc caccttccac attcagactg cggactacac ttcataaatg cctttttatt 7260
   tccagttatg gattcaacta aatgactgcc ttgggagcac ataattactt tgctaccttt 7320
   ttcccccttt gctgttgtgg ctcgagtttg gttctcacct gagaagatgc attgagcata 7380
   tgttgttacc cagccctggc ttaatggtgt cctgtggggt aggggtggga ggacgagggg 7440
   cacggggcca gagcatgtga atggatcatg gttggacagc tgtgacctgc cagcactgcg 7500
   ggtaagcaaa actacaaacc gttctttcct ctgtgacatt gaataaacct taataaaatt 7560
   cataattagc acatactaga aaaaa 7585

<210> 76
<211> 2038
<212> DNA
<213> Homo sapiens

<400> 76
ggggagatat agtaatagtc ccagtgtcag taaactgggt cttgaactta aatgtttgtt 60

tcagttctca ttgccttcat ctcttaattg tcggcactta aggccaacca agcagcttag 120

acagcgcagt ccactcaacg gtaactttat ttgctatatt gacaatctgc tctgttctgt 180

aatatagaac attggatcaa tctttatctt tttgctgact taaggaggct gctattgttg 240

caatatccag agatttcaag ttctagccat tgcggggtct ttgtggtgtt ttcaatttct 300

cctctctcca cccctgcccc catctgtggc catctaagtt agcaatacaa tttattttgg 360

ccatttaaaa tttttctgcc tttgttttat ttcttcccaa atatatttga aaatgaatga 420

tctatatagc tgattttctg accacatata taattgcaat ttttatttgc tcttcaaact 480

attacaacat ttttgtcctt ataaggtttt ttgggcagat aatgcgaggt ctgtggtgtt 540

accttttata tactcctatt accttatgtt tgtgaagcat tttacatttt acagagcact 600

ttgattcatc tccctatttg ttcatttatt taactgatca gttcactgaa atatttattg 660

aacacctact ggaaccaagt gactgtgcta gtaacttgtg atatacagta gtgagagtcc 720

aggcatgtaa ccgatcatta tagtacaata acatccctaa accagacttt acaagtagat 780

aggatcaaca atactctatc aattttcaac tccaattttc tattctactt aaaatgggaa 840

ctagaaactg tttgcctgaa gaatgtgtct gaaacataat atatcactgc atgtctgtgc 900

tgtagacctg ttaattttat ctgtgagaaa aaaagttact caaaattctc cctgacctaa 960

gaatactttc tagttcactg cagtcttact gaccagatgc aacagttgaa gtttgatttc 1020

tcgacccaat atttctattg ggttttgaat tattaatatc actgttttga ggtattcaga 1080

aacaccagtg tatcaaaaaa gcatttgcac tttaggtgtg tgtggtggtt atgtcattta 1140

ttagaccatc ccagacataa gacaatcagg gaaatcagaa aactccagcc tcaaatgtgt 1200

ctataatttc ctgttctacc attgtcatat cataacatgg tattacttct taaggttttg 1260

attaagttga tctagcctca acttaaattg taatacatct gcctaattat tgtctggaat 1320

aacttttcaa ccatccaatg cccactgctc tcacaatgat tattgtagag aagtaaaatg 1380

gtaattattc aagtaaagtc acataatttc tggagtcagt ttttcatatc aagtttatgg 1440

aatacatcat cattggcttc ttcataatat atttattatg agtgaccaaa ttttgcctgg 1500

aggagcaaaa tgctcaaaac ttgttattat aggttaattt ccagctcact gttgacactg 1560

aaagattctg tgttacttta aacccaggat aaaaggctgg aaaaaaaaaa ttaaatgtaa 1620

gtcataaact agtactcagc ttttcctagt ttctaaggct tattaacatt tgcaaattac 1680

tcaataaatg tctttcataa tggaataaca taaaagcttt tgatttggca gatagtgata 1740

ttttatttat tttcattctg gttggaaaaa atctcagtgg cttctcttca ttccacgaga 1800

attttgattt ttaacagcag tctctctttt tctcagcatt gcaaatatat atgtatatat 1860

acattcatga ccaaagtatc gcttactgac catgcagctg taaaccttct gtgcctatca 1920

aacaaataca tagcatgaaa ctaattttag aagtttcatg ggggaatttt aggggaaagt 1980

ataaacctaa gagtgagtga atggagatga ttcatggaaa aaaaaaaaaa aaaaaaac 2038

<210> 77
<211> 686
<212> DNA
<213> Homo sapiens

<220>
<221> misc_feature
<222> (580)..(580)
<223> n is a, c, g, t or u

<220>
<221> misc_feature
<222> (613)..(613)
<223> n is a, c, g, t or u

<220>
<221> misc_feature
<222> (657)..(657)
<223> n is a, c, g, t or u

<400> 77
tttttttttt ttttttgtga acttttttta tttcttcagt gttttttcag agttggaccc 60

ccggcgtctt cacgctaatg ccaccgtctt catttctctc tggctgcatc tgctcttcat 120

ctccatgcgg ttatagtttt cacctcgctg taatcagggg tcctgctttt tcacttgatg 180

atatacagag acggcttccc ttgtacgggg acttcctacc catcaggtct cacgggcgca 240

caatatccca tcgagtgccc gccgcatact tcacgtaact atccctttac ggcggacagg 300

taagatgatg ccagaggggt ttcttctgct gctataaata tcttggcacg tccagctttt 360

ttcatctttt gaattatttc tcagggatat ggtcccgggg cgggattatg gaattgaagg 420

gtcgggtggc tcttgtcctg ttctgccagt tgcccttcaa gagatggggc tggggtaggt 480

gatgtggagg agtgccctac caccctccat ctgcacgtct ggtgcctggt ggggatgtgc 540

agcatgggca gagggtgcag cccgcagcag gagctcccan acacaatgag ggtgccgagt 600

tttggagttt cancaccttc tccagcgtcc ggtgatctca gtcctggtct cctctangca 660

gcgagcagtg agcacctctg cagagg 686

<210> 78
<211> 1926
<212> DNA
<213> Homo sapiens

<400> 78
agtgctaagc aaatacagga accaaaaagg tttatagagg ctgccttatg ctgtctcatc 60

cagttccttt gcggtcctct tcttcagcac atgccaaagc tgttcctcac ggcctgtgag 120

acaagagcat cttggatgta ggacaatgga agagttagat gccttattgg aggaactgga 180

acgctccacc cttcaggaca gtgatgaata ttccaaccca gctcctcttc ccctggatca 240

gcattccaga aaggagacta accttgatga gacttcggag atcctttcta ttcaggataa 300

cacaagtccc ttgccggcgc agctcgtgta tactaccaat atccaggagc tcaatgtcta 360

cagtgaagcc caagagccaa aggaatcacc accaccttct aaaacgtcag cagctgctca 420

gttggatgag ctcatggctc acctgactga gatgcaggcc aaggttgcag tgagagcaga 480

tgctggcaag aagcacttac cagacaagca ggatcacaag gcctccctgg actcaatgct 540

tgggggtctg gagcaggaat tgcaggacct tggcattgcc acagtgccca agggccattg 600

tgcatcctgc cagaaaccga ttgctgggaa ggtgatccat gctctagggc aatcatggca 660

tcctgagcat tttgtctgta ctcattgcaa agaagagatt ggctccagtc ccttctttga 720

gcggagtggc ttggcctact gccccaacga ctaccaccaa cttttttctc cacgctgtgc 780

ttactgcgct gctcccatcc tggataaagt gctgacagca atgaaccaga cctggcaccc 840

agagcacttc ttctgctctc actgcggaga ggtgtttggt gcagaaggct ttcatgagaa 900

ggacaagaag ccatattgcc gaaaggattt cttagccatg ttctcaccca agtgtggtgg 960

ctgcaatcgc ccagtgttgg aaaactacct ttcagccatg gacactgtct ggcacccaga 1020

gtgctttgtt tgtggggact gcttcaccag tttttctact ggctccttct ttgaactgga 1080

tggacgtcca ttctgtgagc tccattacca tcaccgccgg ggaacgctct gccatgggtg 1140

tgggcagccc atcactggcc gttgtatcag tgccatgggg tacaagttcc atcctgagca 1200

ctttgtgtgt gctttctgcc tgacacagtt gtcgaagggc attttcaggg agcagaatga 1260

caagacctat tgtcaacctt gcttcaataa gctcttccca ctgtaatgcc aactgatcca 1320

tagcctcttc agattcctta taaaatttaa accaagagag gagaggaaag ggtaaatttt 1380

ctgttactga ccttctgctt aatagtctta tagaaaaagg aaaggtgatg agcaaataaa 1440

ggaacttcta gactttacat gactaggctg ataatcttat tttttaggct tctatacagt 1500

taattctata aattctcttt ctccctctct tctccaatca agcacttgga gttagatcta 1560

ggtccttcta tctcgtccct ctacagatgt attttccact tgcataattc atgccaacac 1620

tggttttctt aggtttctcc attttcacct ctagtgatgg ccctactcat atcttctcta 1680

atttggtcct gatacttgtt tcttttcacg ttttcccatt tccctgtggc tcactgtctt 1740

acaatcactg ctgtggaatc atgataccac ttttagctct ttgcatcttc cttcagtgta 1800

tttttgtttt tcaagaggaa gtagatttta actggacaac tttgagtact gacatcattg 1860

ataaataaac tggcttgtgg tttcaataaa aaaaaaaaaa aaaaaaaaaa aaaaaaaaaa 1920

aaaaaa 1926

<210> 79
<211> 1902
<212> DNA
<213> Homo sapiens

<400> 79
atgaacagcg gcccagaaca aggtctgcaa agaaagcaga gttggaagaa aatcagcgga 60

tctataaaca gaaaaagaaa aggcgacgta ttaaggttca agaagattca tccagtgaaa 120

acaagacacg gcgttagtgg gtttgtcctc ctcaatactg cttttaattg gatgaatgaa 180

tttgagaagt ggcaagaggg attaaaagat gatgagaagc ttgaggtttc tgaattagca 240

actgtgaaac gtcctcagga gagaagctac atgctgcaga ggtggcaaga agatggtggt 300

gttatgatca taggctatga gatgtataga aatcttgctc aaggaaggaa tgtgaagagt 360

cggaaactta aagaaatatt taacaaagct ttggttgatc caggccctga ttttgttgtt 420

tgtgatgaag gccatattct aaaaaatgaa gcatctgctg tttctaaagc tatgaattct 480

atacgatcaa ggaggaggat tattttaaca ggaacaccac ttcaaaataa cctaattgag 540

tatcattgta tggttaattt tatcaaggaa aatttacttg gatccattaa ggagttcagg 600

aatagattta taaatccaat tcaaaatggt cagtgtgcag attctaccat ggtagatgtc 660

agagtgatga aaaaacgtgc tcacattctc tatgagatgt tagctggatg tgttcagagg 720

aaagattata cagcattaac aaaattcttg cctccaaaac acgaatatgt gttagctgtg 780

agaatgactt ctattcagtg caagctctat gcaggtgcaa agcttttcca agattttcag 840

atgttaagta gaatatggac tcatccttgg tgtttgcagc tagactacat tagcaaagaa 900

aataagggtt attttgatga agacagtatg gatgaattta tagcctcaga ttctgatgaa 960

acctccatga gtttaagctc cgatgattat acaaaaaaga agaaaaaagg gaaaaagggg 1020

aaaaaagata gtagctcaag tggaagtggc agtgacaatg atgttgaagt gattaaggtc 1080

tggaattcaa gatctcgggg aggtggtgaa ggaaatgtgg atgaaacagg aaacaatcct 1140

tctgtttctt taaaactgga agaaagtaaa gctacttctt cttctaatcc aagcagccca 1200

gctccagact ggtacaaaga ttttgttaca gatgctgatg ctgaggtttt agagcattct 1260

gggaaaatgc tggtagagag aagagagaag atagaagata aaccccttat ttataaaggt 1320

gaggggaagt ggcttcgaaa cattgactat taccgtttag atggttccac tactgcacag 1380

tcaaggaaga agtgggctga agaatttaat gatgaaacta atgtgagagg acgattattt 1440

atcatttcta ctaaagcagg atctctagga attaatctgg tagctgctaa tcgagtaatt 1500

atattcgacg cttcttggaa tccatcttat gacatccaga gtatattcag agtttatcgc 1560

tttggacaaa ctaagcctgt ttatgtatat aggttcttag ctcagggaac catggaagat 1620

aagatttatg atcggcaagt aactaagcag tcactgtctt ttcgagttgt tgatcagcag 1680

caggtggagc gtcattttac tatgaatgag cttactgaac tttatacttt tgagccagac 1740

ttattagatg accctaattc agaaaagaag aagaaaagga gggatactcc catgctgcca 1800

aaggatacca tacttgcaga gctccttcag atacataaag aacacattgt aggataccat 1860

gaacatgatc tcttttggac cacaaagaag aagaagagtt ga 1902

<210> 80
<211> 5748
<212> DNA
<213> Homo sapiens

<400> 80
gagaagaaag ccagtgcgtc tctgggcgca ggggccagtg gggctcggag gcacaggcac 60

cccgcgacac tccaggttcc ccgacccacg tccctggcag ccccgattat ttacagcctc 120

agcagagcac ggggcggggg cagaggggcc cgcccgggag ggctgctact tcttaaaacc 180

tctgcgggct gcttagtcac agcccccctt gcttgggtgt gtccttcgct cgctccctcc 240

ctccgtctta ggtcactgtt ttcaacctcg aataaaaact gcagccaact tccgaggcag 300

cctcattgcc cagcggaccc cagcctctgc caggttcggt ccgccatcct cgtcccgtcc 360

tccgccggcc cctgccccgc gcccagggat cctccagctc ctttcgcccg cgccctccgt 420

tcgctccgga caccatggac aagttttggt ggcacgcagc ctggggactc tgcctcgtgc 480

cgctgagcct ggcgcagatc gatttgaata taacctgccg ctttgcaggt gtattccacg 540

tggagaaaaa tggtcgctac agcatctctc ggacggaggc cgctgacctc tgcaaggctt 600

tcaatagcac cttgcccaca atggcccaga tggagaaagc tctgagcatc ggatttgaga 660

cctgcaggta tgggttcata gaagggcacg tggtgattcc ccggatccac cccaactcca 720

tctgtgcagc aaacaacaca ggggtgtaca tcctcacatc caacacctcc cagtatgaca 780

catattgctt caatgcttca gctccacctg aagaagattg tacatcagtc acagacctgc 840

ccaatgcctt tgatggacca attaccataa ctattgttaa ccgtgatggc acccgctatg 900

tccagaaagg agaatacaga acgaatcctg aagacatcta ccccagcaac cctactgatg 960

atgacgtgag cagcggctcc tccagtgaaa ggagcagcac ttcaggaggt tacatctttt 1020

acaccttttc tactgtacac cccatcccag acgaagacag tccctggatc accgacagca 1080

cagacagaat ccctgctacc actttgatga gcactagtgc tacagcaact gagacagcaa 1140

ccaagaggca agaaacctgg gattggtttt catggttgtt tctaccatca gagtcaaaga 1200

atcatcttca cacaacaaca caaatggctg gtacgtcttc aaataccatc tcagcaggct 1260

gggagccaaa tgaagaaaat gaagatgaaa gagacagaca cctcagtttt tctggatcag 1320

gcattgatga tgatgaagat tttatctcca gcaccatttc aaccacacca cgggcttttg 1380

accacacaaa acagaaccag gactggaccc agtggaaccc aagccattca aatccggaag 1440

tgctacttca gacaaccaca aggatgactg atgtagacag aaatggcacc actgcttatg 1500

aaggaaactg gaacccagaa gcacaccctc ccctcattca ccatgagcat catgaggaag 1560

aagagacccc acattctaca agcacaatcc aggcaactcc tagtagtaca acggaagaaa 1620

cagctaccca gaaggaacag tggtttggca acagatggca tgagggatat cgccaaacac 1680

ccaaagaaga ctcccattcg acaacaggga cagctgcagc ctcagctcat accagccatc 1740

caatgcaagg aaggacaaca ccaagcccag aggacagttc ctggactgat ttcttcaacc 1800

caatctcaca ccccatggga cgaggtcatc aagcaggaag aaggatggat atggactcca 1860

gtcatagtat aacgcttcag cctactgcaa atccaaacac aggtttggtg gaagatttgg 1920

acaggacagg acctctttca atgacaacgc agcagagtaa ttctcagagc ttctctacat 1980

cacatgaagg cttggaagaa gataaagacc atccaacaac ttctactctg acatcaagca 2040

ataggaatga tgtcacaggt ggaagaagag acccaaatca ttctgaaggc tcaactactt 2100

tactggaagg ttatacctct cattacccac acacgaagga aagcaggacc ttcatcccag 2160

tgacctcagc taagactggg tcctttggag ttactgcagt tactgttgga gattccaact 2220

ctaatgtcaa tcgttcctta tcaggagacc aagacacatt ccaccccagt ggggggtccc 2280

ataccactca tggatctgaa tcagatggac actcacatgg gagtcaagaa ggtggagcaa 2340

acacaacctc tggtcctata aggacacccc aaattccaga atggctgatc atcttggcat 2400

ccctcttggc cttggctttg attcttgcag tttgcattgc agtcaacagt cgaagaaggt 2460

gtgggcagaa gaaaaagcta gtgatcaaca gtggcaatgg agctgtggag gacagaaagc 2520

caagtggact caacggagag gccagcaagt ctcaggaaat ggtgcatttg gtgaacaagg 2580

agtcgtcaga aactccagac cagtttatga cagctgatga gacaaggaac ctgcagaatg 2640

tggacatgaa gattggggtg taacacctac accattatct tggaaagaaa caaccgttgg 2700

aaacataacc attacaggga gctgggacac ttaacagatg caatgtgcta ctgattgttt 2760

cattgcgaat cttttttagc ataaaatttt ctactctttt tgttttttgt gttttgttct 2820

ttaaagtcag gtccaatttg taaaaacagc attgctttct gaaattaggg cccaattaat 2880

aatcagcaag aatttgatcg ttccagttcc cacttggagg cctttcatcc ctcgggtgtg 2940

ctatggatgg cttctaacaa aaactacaca tatgtattcc tgatcgccaa cctttccccc 3000

accagctaag gacatttccc agggttaata gggcctggtc cctgggagga aatttgaatg 3060

ggtccatttt gcccttccat agcctaatcc ctgggcattg ctttccactg aggttggggg 3120

ttggggtgta ctagttacac atcttcaaca gaccccctct agaaattttt cagatgcttc 3180

tgggagacac ccaaagggtg aagctattta tctgtagtaa actatttatc tgtgtttttg 3240

aaatattaaa ccctggatca gtcctttgat cagtataatt ttttaaagtt actttgtcag 3300

aggcacaaaa gggtttaaac tgattcataa taaatatctg tacttcttcg atcttcacct 3360

tttgtgctgt gattcttcag tttctaaacc agcactgtct gggtccctac aatgtatcag 3420

gaagagctga gaatggtaag gagactcttc taagtcttca tctcagagac cctgagttcc 3480

cactcagacc cactcagcca aatctcatgg aagaccaagg agggcagcac tgtttttgtt 3540

ttttgttttt tgtttttttt ttttgacact gtccaaaggt tttccatcct gtcctggaat 3600

cagagttgga agctgaggag cttcagcctc ttttatggtt taatggccac ctgttctctc 3660

ctgtgaaagg ctttgcaaag tcacattaag tttgcatgac ctgttatccc tggggcccta 3720

tttcatagag gctggcccta ttagtgattt ccaaaaacaa tatggaagtg ccttttgatg 3780

tcttacaata agagaagaag ccaatggaaa tgaaagagat tggcaaaggg gaaggatgat 3840

gccatgtaga tcctgtttga catttttatg gctgtatttg taaacttaaa cacaccagtg 3900

tctgttcttg atgcagttgc tatttaggat gagttaagtg cctggggagt ccctcaaaag 3960

gttaaaggga ttcccatcat tggaatctta tcaccagata ggcaagttta tgaccaaaca 4020

agagagtact ggctttatcc tctaacctca tattttctcc cacttggcaa gtcctttgtg 4080

gcatttattc atcagtcagg gtgtccgatt ggtcctagaa cttccaaagg ctgcttgtca 4140

tagaagccat tgcatctata aagcaacggc tcctgttaaa tggtatctcc tttctgaggc 4200

tcctactaaa agtcatttgt tacctaaact tatgtgctta acaggcaatg cttctcagac 4260

cacaaagcag aaagaagaag aaaagctcct gactaaatca gggctgggct tagacagagt 4320

tgatctgtag aatatcttta aaggagagat gtcaactttc tgcactattc ccagcctctg 4380

ctcctccctg tctaccctct cccctccctc tctccctcca cttcacccca caatcttgaa 4440

aaacttcctt tctcttctgt gaacatcatt ggccagatcc attttcagtg gtctggattt 4500

ctttttattt tcttttcaac ttgaaagaaa ctggacatta ggccactatg tgttgttact 4560

gccactagtg ttcaagtgcc tcttgttttc ccagagattt cctgggtctg ccagaggccc 4620

agacaggctc actcaagctc tttaactgaa aagcaacaag ccactccagg acaaggttca 4680

aaatggttac aacagcctct acctgtcgcc ccagggagaa aggggtagtg atacaagtct 4740

catagccaga gatggttttc cactccttct agatattccc aaaaagaggc tgagacagga 4800

ggttattttc aattttattt tggaattaaa tacttttttc cctttattac tgttgtagtc 4860

cctcacttgg atatacctct gttttcacga tagaaataag ggaggtctag agcttctatt 4920

ccttggccat tgtcaacgga gagctggcca agtcttcaca aacccttgca acattgcctg 4980

aagtttatgg aataagatgt attctcactc ccttgatctc aagggcgtaa ctctggaagc 5040

acagcttgac tacacgtcat ttttaccaat gattttcagg tgacctgggc taagtcattt 5100

aaactgggtc tttataaaag taaaaggcca acatttaatt attttgcaaa gcaacctaag 5160

agctaaagat gtaatttttc ttgcaattgt aaatcttttg tgtctcctga agacttccct 5220

taaaattagc tctgagtgaa aaatcaaaag agacaaaaga catcttcgaa tccatatttc 5280

aagcctggta gaattggctt ttctagcaga acctttccaa aagttttata ttgagattca 5340

taacaacacc aagaattgat tttgtagcca acattcattc aatactgtta tatcagagga 5400

gtaggagaga ggaaacattt gacttatctg gaaaagcaaa atgtacttaa gaataagaat 5460

aacatggtcc attcaccttt atgttataga tatgtctttg tgtaaatcat ttgttttgag 5520

ttttcaaaga atagcccatt gttcattctt gtgctgtaca atgaccactg ttattgttac 5580

tttgactttt cagagcacac ccttcctctg gtttttgtat atttattgat ggatcaataa 5640

taatgaggaa agcatgatat gtatattgct gagttgaaag cacttattgg aaaatattaa 5700

aaggctaaca ttaaaagact aaaggaaaca gaaaaaaaaa aaaaaaaa 5748

<210> 81
<211> 1147

<212>  DNA
<213>  Homo sapiens

<400>  81
gtggctttgc cctttggcct ttgctggctg tgtggcggct ccgcggttcg caggtcgttc      60

gctgagcgtc tctgcttagc cgcggtcatg agccggcaca gccggctgca gaggcaggtt     120

ctgagcctgt accgcgatct gctgcgcgcc gggcgtggga agccgggcgc cgaggcgcga     180

gtgcgggcag agttccggca gcatgcgggc ctgccgcggt ccgacgtgct gcgcatcgag     240

tacctgtacc gccgcgggcg gcgccagctg cagctgctac gctcggccca cgccaccgcc     300

atgggcgcct tcgtacgccc gcgggccccg accggggagc ctggcggcgt gggttcccag     360

cctgacgacg gcgacagtcc aaggaacccc cacgacagca cgggggcacc ggagacccgc     420

cccgacggac ggtgacaggc gaagagccga actcgctcga tggcgtggtg gagccaggag     480

gctcgcctga ctgcatgggg ggactgggga acccgcctaa ggtgagaggt cttaagagac     540

tagcttgacg aattggggat gtcagagact cctccttggc gacgcagggg gcctagagag     600

ccccgtgatg gacggcaagg gaggcccgcc ttttccgatg cttggagaca ggtcggtgct     660

cctcccccat gagggcttgg ggcggcctgg gacgctggcg ggctggacag tgtcaagcca     720

agagctactt gcccgaaggt acggggagcc aggacgaccc ccggtggaca gggagagcct     780

gagacgccct tctcttgacc cctgagaaca tacccacttc tggctcctca aggagtctcc     840

cctctcctgt atttaactct gagaagtgca gactttttgc tgagaacgtt ttgggaaggt     900

gccctgatga gcggtgagaa gcccggaatc cccttctgga aaactttccc ccattaattg     960

tgacaagcca ggaccatgag gaaggggtag gggtctatca ccctggttga tcaactgaag    1020

acccccaaag gcccctactt gatggttttg aggggcaaca ttgactcatt tgccccttcc    1080

ctctcggaat gttggacaaa gggaataaaa ttggggatat gtctacttcc caaaaaaaaa    1140

aaaaaaa                                                              1147


<210>  82
<211>  6725
<212>  DNA
<213>  Homo sapiens

<400>  82
cgccgccatt ttcccagagc gagaggcagt gacactgagc gggcgcaggg ggccgagtcg      60

gagaccgtgc cggagttcgg gagcggcaac agagtgggca tagacactcc gagcagcctc     120

gccgtcgtct ctgcgttcct gttgactgcc tggctgcccc ctcccctact cctcggttcc     180

tggtgaagag gctgcgcgct gctgtttggg gagggggtgt gtggagccgg gtcctgtgtc     240

cgcagtggct gctgtcgggg ggtcgcctgt tcgcggaggt gcggagagac tccttggggg 300

tcgagcactg tggctggcat gccccagtgt tttggatacc aatgcatagg actccatagt 360

aatcgaattt accagaggcg aacgtcatga gcatagtgat cccattgggg gttgatacag 420

cagagacgtc atacttggaa atggctgcag gttcagaacc agaatccgta gaagctagcc 480

ctgtggtagt tgagaaatcc aacagttatc cccaccagtt atataccagc agctcacatc 540

attcacacag ttacattggt ttgccctatg cggaccataa ttatggtgct cgtcctcctc 600

cgacacctcc ggcttcccct cctccatcag tccttattag caaaaatgaa gtaggcatat 660

ttaccactcc taattttgat gaaacttcca gtgctactac aatcagcaca tctgaggatg 720

gaagttatgg tactgatgta accaggtgca tatgtggttt tacacatgat gatggataca 780

tgatctgttg tgacaaatgc agcgtttggc aacatattga ctgcatgggg attgataggc 840

agcatattcc tgatacatat ctatgtgaac gttgtcagcc taggaatttg gataaagaga 900

gggcagtgct actacaacgc cggaaaaggg aaaatatgtc agatggtgat accagtgcaa 960

ctgagagtgg tgatgaggtt cctgtggaat tatatactgc atttcagcat actccaacat 1020

caattacttt aactgcttca agagtttcca aagttaatga taaaagaagg aaaaaaagcg 1080

gggagaaaga acaacacatt tcaaaatgta aaaaggcatt tcgtgaagga tctaggaagt 1140

catcaagagt taagggttca gctccagaga ttgatccttc atctgatggt tcaaattttg 1200

gatgggagac aaagatcaaa gcatggatgg atcgatatga agaagcaaat aacaaccagt 1260

acagtgaggg tgttcagagg gaggcacaaa gaatagctct gagattaggc aatggaaatg 1320

acaaaaaaga gatgaataaa tccgatttga ataccaacaa tttgctcttc aaacctcctg 1380

tagagagcca tatacaaaag aataagaaaa ttcttaaatc tgcaaaagat ttgcctcctg 1440

atgcacttat cattgaatac agagggaagt ttatgctgag agaacagttt gaagcaaatg 1500

ggtatttctt taaaagacca tacccttttg tgttattcta ctctaaattt catgggctag 1560

aaatgtgtgt tgatgcaagg acttttggga atgaggctcg attcatcagg cggtcttgta 1620

cacccaatgc agaggtgagg catgaaattc aagatggaac catacatctt tatatttatt 1680

ctatacacag tattccaaag ggaactgaaa ttactattgc ctttgatttt gactatggaa 1740

attgtaagta caaggtggac tgtgcatgcc tcaaagaaaa cccagagtgc cctgttctaa 1800

aacgtagttc tgaatccatg gaaaatatca atagtggtta tgagaccaga cggaaaaaag 1860

gaaaaaaaga caaagatatt tcaaaagaaa aagatacaca aaatcagaat attactttgg 1920

attgtgaagg aacgaccaac aaaatgaaga gcccagaaac taaacaaaga aagctttctc 1980

cactgagact atcagtatca aataatcagg aaccagattt tattgatgat atagaagaaa 2040

aaactcctat tagtaatgaa gtagaaatgg aatcagagga gcagattgca gaaaggaaaa 2100

ggaagatgac aagagaagaa agaaaaatgg aagcaatttt gcaagctttt gccagacttg 2160

aaaagagaga gaaaagaaga gaacaagctt tggaaaggat cagcacagcc aaaactgaag 2220

ttaaaactga atgtaaagat acacagattg tcagtgatgc tgaagttatt caggaacaag 2280

caaaagaaga aaatgctagc aagccaaccc ctgccaaagt aaatagaact aaacagagaa 2340

aaagtttttc tcggagtagg actcacattg gacagcagcg tcggagacac agaactgtca 2400

gcatgtgttc agatatccag ccatcttctc ctgatataga agttacttca caacaaaatg 2460

atattgaaaa tactgtactt acaatagaac cagaaactga aactgcacta gcagaaataa 2520

ttactgaaac tgaagttcca gcacttaata aatgtcctac caagtacccc aaaacaaaga 2580

agcacttggt taatgaatgg ttaagtgaga agaatgagaa gacaggaaaa ccttcagatg 2640

gcctttcaga aaggcctcta cgcataacta cagatcctga agtgttagct acacaactca 2700

attctttacc aggtctcact tacagccccc atgtatactc cactcctaag cattatatta 2760

gatttacttc accattcctt tcagaaaaaa ggagaagaaa agaacctact gaaaacattt 2820

ctggttcatg caagaagcga tggttgaaac aagctctgga agaagaaaat tcagcaattt 2880

tacatagatt taattcaccc tgtcaagaaa gatccagaag tcctgcagtc aatggtgaaa 2940

ataaaagtcc actactatta aatgacagct gttcccttcc agatttaact acaccactaa 3000

aaaaacgaag attttatcag ttgctagatt cggtttactc agaaacctcc acacctactc 3060

cttccccgta tgctacacca actcacaccg atattactcc tatggaccca tcttttgcca 3120

cgcctccacg gataaaatca gatgatgaaa cttgtagaaa tggttataaa cccatatatt 3180

caccagttac cccagtaact cctggtacac caggaaatac catgcacttt gagaatattt 3240

cttccccaga aagttctcca gaaataaaga gacgcactta tagtcaagag ggatatgaca 3300

gatcttcaac catgttaaca ttggggcctt ttagaaattc taatttaact gaactgggtc 3360

tgcaagaaat aaagactatt ggttatacga gccctaggag taggactgaa gtcaacaggc 3420

agtgtcctgg agaaaaggaa cctgtgtcag accttcagct aggactcgat gcagttgagc 3480

caactgccct acataaaacc ctggaaacgc ctgcacatga cagggctgag cccaacagcc 3540

aactggactc gactcactct ggacggggca caatgtattc ttcctgggta aagagccctg 3600

acagaacagg agttaacttc tcagtgaact ccaacttgag ggacctgaca ccctcgcatc 3660

agttggaggt tggaggaggc ttccgaataa gtgagtcaaa gtgcctgatg caggatgata 3720

ctagaggcat gtttatggaa acaactgtgt tttgtacttc cgaagatggg cttgtatctg 3780

gtttcggacg gactgttaat gacaatttga tcgacgggaa ttgcacaccc cagaatccac 3840

cacaaaagaa aaaggtttct ctattagaat accgtaagag acaacgtgaa gctaggaaaa 3900

gtggctctaa gacagagaac tttccactca ttagtgtatc accccatgca agtggaagct 3960

tgagcaacaa tggtgatggc tgtgccagca gtaatgacaa tggggagcag gtggaccaca 4020

ctgctagcct acctttacca acaccagcta cagtttataa tgccacttct gaagaaacta 4080

gcaataactg ccctgttaag gatgctactg ctagtgagaa gaatgaacca gaagttcaat 4140

ggactgcctc aacttcagtg gaacaagtca gagaaaggag ttatcagaga gctttacttc 4200

tcagtgatca ccgaaaagat aaagatagtg ggggagaatc accatgtgtc tcatgttcac 4260

cgagtcatgt tcagtcttca ccttcatctc attcaaatca cataccccag ttgcaagcta 4320

agggcccagt cccttctttc agtgaactta tggaagaccc tgatcctgaa aatccagaac 4380

ccacaactac gaatgaatgt ccatccccag atacttctca aaatacttgt aaaagtcctc 4440

caaaaatgag caagcctggt tcacctggat ctgtaattcc tgctcaagca cacgggaaaa 4500

tattcacaaa accagatccc caatgggact ccacagttag tgcatccgaa gctgaaaatg 4560

gtgttcacct aaaaacagag ctccaacaaa aacagctatc aaataacaac caagcacttt 4620

caaagaatca tcctcctcag acacacgttc gtaattcatc tgagcaactt tcacaaaagc 4680

tgccttctgt gccaacaaag ttgcactgtc ctccatcacc tcacctagaa aatcctccaa 4740

agtcatccac gcctcacaca cctgtacagc atggttatct ttcaccaaag cctccttcac 4800

agcagttagg atctccctac aggcctcatc attcacagtc acctcaagtt ggaacacctc 4860

agcgagagcc tcaaagaaac ttttatccag cagcacagaa ccttccagcc aatactcagc 4920

aggcaacttc tggaacatta tttacacaga caccctcagg acaatcttca gcaacataca 4980

gtcagtttaa ccaacaaagt ctgaacagca cggcaccacc ccctccacct cctccacctc 5040

cttcttcgtc ttactatcaa aaccagcagc cctctgcaaa ctttcagaat tataatcagc 5100

tcaaaggtag tctttctcaa caaactgtgt ttacatcagg accaaatcaa gcacttcctg 5160

gcaccacaag ccagcaaaca gttccaggac accacgtgac tccagggcat tttttgccct 5220

ctcagaaccc taccattcac catcaaactg ctgctgccgt agtcccccct cctcctccac 5280

caccacctgc tccaggaccg caccttgtac aacagccgaa ttcccatcag caacactctg 5340

tagcacatgt agtagggcct gttcatgcgg tcacccctgg gtcgcatatt cattctcaaa 5400

ctgctggaca ccacttaccc ccacccccac cccctcctgg tcctgcccct catcaccatc 5460

caccacccca tccatccaca ggactccaag gtctacaagc acaacaccag catgttgtaa 5520

attcagcacc cccaccaccc cctccgccgc caccttccag tgttttggct tctgggcatc 5580

ataccacatc agctcaagcc ttacaccacc cacctcatca aggacctcca cttttccctt 5640

cgagtgctca tccaactgta ccaccgtatc cctcacaagc tacacatcat accactttgg 5700

gaccgggacc ccagcaccag ccttctggaa cagggccaca ttgtccatta cctgtcacag 5760

gtcctcatct ccagccccaa ggaccaaaca gtattccaac acctactgct tcagggttct 5820

gtcctcatcc tggctctgtg gccctgccac atggggttca aggacctcag caggcatctc 5880

cagtgcctgg acagattcca attcacagag cacaggtgcc accaacattt caaaacaatt 5940

accatgggtc agggtggcat taaaatggac tccaaaaaca tttttttaaa tgttctgtaa 6000

gataaactgt atatttcata tgtacctgtt aaggtacttt ttaaagcttg tacatgaacc 6060

tttgtataaa aaacaccagt gctctttcgt tgtatttttc tcatttttgc tttttaaaat 6120

tcctttaaaa aatgtgctgt taagccagta ttaggtatct ttattttgta agtgaacatt 6180

ccagctgttt ttttctggca gatctgatgc tgatttgatg ctgtatgatc tttttttttt 6240

ttttagttaa attcatttag tgaatgttct attattttat acatacacat taagtactca 6300

gctaagtaat ggcactatga ggattttttt tttctttcct gtcagcagca gttctgtgaa 6360

tgcatcttag gtataaaaat gcaatacaga tttttatatt ttggtgtgga catggctcat 6420

tttgttttac cagttatttg caagcaaaat gtaatttaat gtatagatga tttctaatgt 6480

ctcctgacaa actgtaaata ctgcatttct tttgcgtata taattgctta cagcttttct 6540

catttgatat atagcattgt acatatgaca agtcttttgc aaaactgtgt gatctttgtg 6600

aaagtagtac agtatatgac ctttaatttc ttttttattt taaatatact gtcacactga 6660

agcactggtt gggcatttta attcatgtta ataaatcaca attatgtcag ttttaaaaaa 6720

aaaaa 6725

<210> 83
<211> 594
<212> DNA
<213> Homo sapiens

<400> 83
tttttttttt tttttttgaa agtagagtgt tttatttatt ttattttagc aataacaaaa 60

aaggcactac ataagagtaa tgtaatttca gatgggccag gagcccaccc aggccgagcc 120

atcaaccaat ctggtctcat cttcttttat ccaggcttat tttgttttca ccagaaaaag 180

gccttattag ttatttaata caaagtacat gccaggtaca attctaaata tctcagctgc 240

atcattgttt aaatttcttc atacctaaca atcgtaacaa tttcttcaat cctaacaatc 300

ttatgctgtg ggtatcttgg tttctgtttt tcaggtaaag tctagaaagt tgacatataa 360

tgatttttag agagctgata agtcaaaggg tcaggatttg tacacatttc tgccttattc 420

tgaagttgaa atcataacca caatgctcat gagcatctgt tggggatggt gttcgtggga 480

tgtgtgataa ttaattttat gtgtaaactt ggcttggcca tggtatgcag acatttggtc 540

aaacattatt ttagatgttt ctgtgaaggt ctttttgatg atactaacat tcaa 594

<210> 84
<211> 2743
<212> DNA
<213> Homo sapiens

<400> 84
ggatgaggcc tcgccctcgc ctctttagta ggtcgggtga gtgtagtgtg cagggaagag 60

acgcgtcagc gccagggcca ggcccgcccg ggggcagccc ggcagccgaa tcttgggcta 120

ctctgtccca acagccggag cagatcagac cgaccggccc tgcccgctcg gtcccgcgcc 180

ctccagaccc tacggtctcc gtttctaggg gcacatggtt agcggcaggc gcccacagcc 240

aatccacttt gccagcctgc cccttcctct gccaagagca gcttcttcag ccgcgctcca 300

gttccgcaga cgcctgcccc accctgctct tcccttccag ggaagacgga tcacgcggcc 360

aagaacgaga ctcgcaaact gggcatttct ccgagccggg ctagagcaag tagcgagact 420

ccgcgtgaga gtgggaaaga gccttaacag gcaaccatgt tgcccagtgg gttttctgtg 480

cctttgggtg cggaccaatg aggcgcgtgg ggcgggactt ccgcttcgcc taggtgttgt 540

cgtccctgct agtactccgg gctgtggggg tcggtgcgga tattcagtca tgaaatcagg 600

gtagggactt ctcccgcagc gacgcggctg gcaagactgt ttgtgttgcg ggggccggac 660

ttcaagtata actttgaaaa tgcatctcag acatttgatg acctccctgc aagatttggt 720

tatagacttc cagctgaagg tttaaagggt tttttgatta actcaaaacc agagaatgcc 780

tgtgaaccca tagtgcctcc accagtaaaa gacaattcat ctggcacttt catcgtgtta 840

attagaagac ttgattgtaa ttttgatata aaggttttaa atgcacagag agcaggatac 900

aaggcagcca tagttcacaa tgttgattct gatgacctca ttagcatggg atccaacgac 960

agtaagtaca gttgaggtac taaagaaaat tgacattcca tctgtcttta ttggtgaatc 1020

atcagctaat tctctgaaag atgaattcac atatgaaaaa gggggccacc ttatcttagt 1080

tccagaattt agtcttcctt tggaatacta cctaattccc ttccttatca tagtgggcat 1140

ctgtctcatc ttgatagtca ttttcatgat cacaaaattt gtccaggata gacatagagc 1200

tagaagaaac agacttcgta aagatcaact taagaaactt cctgtacata aattcaagaa 1260

aggagatgag tatgatgtat gtgccatttg tttggatgag tatgaagatg gagacaaact 1320

cagaatcctt ccctgttccc atgcttatca ctgcaagtgt gtagaccctt ggctaactaa 1380

aaccaaaaaa acctgtccag tgtgcaagca aaaagttgtt ccttctcaag gcgattcaga 1440

ctctgacaca gacagtagtc aagaagaaaa tgaagtgaca gaacataccc ctttactgag 1500

acctttagct tctgtcagtg cccagtcatt tggggcttta tcggaatccc gctcacatca 1560

gaacatgaca gaatcttcag actatgagga agacgacaat gaagatactg acagtagtga 1620

tgcagaaaat gaaattaatg aacatgatgt cgtggtccag ttgcagccta atggtgaacg 1680

ggattacaac atagcaaata ctgtttgact ttcagaagat gattggttta tttcccttta 1740

aaatgattag gtatatactg taatttgatt ttttgctccc ttcaaagatt tctgtagaaa 1800

taacttattt tttagtattc tacagtttaa tcaaattact gaaacaggac ttttgatctg 1860

gtatttatct gccaagaata tacttcattc actaataata gactggtgct gtaactcaag 1920

catcaattca gctcttcttt tggaatgaaa gtatagccaa aacataaaaa aaaaaaaatc 1980

ctcagtatag cttgcaatta agacctagat cacagtattt aagtgttttg cgttttatac 2040

atgaggtcag tgctacagcc acctagcatg aactaaccca gcttccacct ccataaagtt 2100

acctagagtt gttgagttgg aatatgttct ggcatttacc tgacctgcca atcattaggg 2160

agaggcaaca aggtaattca gcctttcctc ctatcagcac aaagaaactc aaagctgttt 2220

tttccctttc tgttccaaag cagtcttatc ctgacaggag cggtctatac tagtgcagat 2280

ttcaacactt ttttttaacg ttttaattac tatagtgtta tgtagagatt tgattgagca 2340

gctaatgttt ctgaacttta cttactaatt ttcagtgtcc ttaagggttc tgtagtgtta 2400

tcaaagcaaa aagaaaatgc tgcataaaaa taccaaactt cagcaactgt taatactcag 2460

atcatatacc tcttaataaa tagcatctta tgctaattag ccctgctaaa ctatgtacag 2520

aggaaactgt tcaagtattg gatttgaaag taagtgactt atgtttaaca gaactaatga 2580

tgtattgaaa cactgtatta tgaaaagcta aattatacat cattgtaact atgtagaaag 2640

tgtagactaa tgtataatca aaatgctaag gatttttata tggccttgta tgaggggagt 2700

ttgaatgtta ataaacatgt tttccacttt aagatccagt aaa 2743

<210> 85
<211> 2798
<212> DNA
<213> Homo sapiens

<400> 85
agcagactgg gctgcgggcg gcggggccgg cgcctgcctg gagggatggg gctgccgggc 60

gcgtaggggc catgccgccc gggacccggg cctgccgcgt tccgcgcccc ggccgccgcg 120

ccccacgtcc gcgccgggat ggtgaacctg gcggccatgg tgtggcgccg gcttctgcgg 180

aagaggtggg tgctcgccct ggtcttcggg ctgtcgctcg tctacttcct cagcagcacc 240

ttcaagcagg aggagagggc agtgagagat aggaatctcc tccaggttca tgaccataat 300

cagcccatcc cgtggaaagt gcagtttaac ttgggcaata gcagtcgtcc gagcaatcag 360

tgccgcaact ccattcaagg gaagcacctc atcacggatg aactcggcta cgtttgcgag 420

aggaaggatt tgctggtaaa tggctgctgt aatgtcaacg tccctagcac gaagcagtac 480

tgctgtgatg gctgctggcc caacggctgc tgcagcgcct atgagtactg tgtctcctgc 540

tgcctgcagc ccaacaagca acttctcctg gagcgcttcc tcaaccgggc agccgtggca 600

ttccagaacc tcttcatggc agtcgaagat cactttgagt tgtgcctggc caaatgcagg 660

acctcatctc agagcgtgca gcatgagaac acctaccggg accccatagc aaagtattgc 720

tatggagaaa gcccgcccga gctcttcccc gcttgacggg tgcagcggac ttgctccagc 780

ctgggtgagg aggccccgct gaagaactcg cctcctggga cccagcttca gccatcgggc 840

caggctgcag gaagaagaca aaggcagcgt gaggaaacct tggctttgac cccttctcgt 900

gttgtcatct ttggcttcgc tcaccacccg ggcttaccag atggaactct tctgtaaagc 960

agcttggccc ctccagccag tcccattcgg gaaagatgaa accggaggcc gggctcacgg 1020

tggtggtgga gttcttggat gactcagccc tgggacctgc acagggacct gtgacttgtg 1080

ttcatcgggg gccggtgtca cttccagttt tgatccaggc tctttcactg taaaattatt 1140

tattggattc ctttggagta atgggaacat tctaatgttt tatgtaggaa aatgccttgc 1200

cattctagtt gaatatgttc aaggaagtta tttttgttgt tgttctgtgt tctcgagttt 1260

caggagttaa atcattcttc cacccagata caacattttc tcttttagga cgtgaatatt 1320

ctctctaggc agttattttt gtttgtattt cgacagtatc aagcataggc cctgaacgtg 1380

acctgttagc catatcctga cgtgtaaaat tatctaaaaa ctcagacact cttccattct 1440

aatctaactg cacgatttct aacagtgggc accatgtgcc tgccctcagg ttattttcca 1500

gtggtagtcg aatgtgctca tataccctat ggagagcact gttttagcag aaatctaatt 1560

tctcttcctg gaggaatttg ttctcatttc ttttgccact taaaattaac tgtgggctac 1620

tcagccaggg tacagtggga gcctcaggaa ggtcagaggc aacctcctcc cctgttctat 1680

caatagaaac ccaacgttga ggcaattcct aaacagacgc acctcgtagc ttgctgtatg 1740

tgtttattct ttattgcttt cagctttggg gctgtaacag gtacaaatat ttggtttccc 1800

tatgatttat agagaagaag aagaaaccca gctttctatc agagcactgc aagagaagag 1860

tcttacacct gccctcagtg ggagatgaga atggtcatta tgacttagag aatgctacac 1920

gtgtaggttg ctggtgtgtc ctgaatccac aggcataaag cactccccat ttttcctact 1980

gtaatgcaga ttctccggct caaggtctag aatatttgat cctaagatca agacatcatg 2040

cccttcgaat agtactgctc tttgttttca ggagtcacgt gaacacacaa ctctcctata 2100

ttcctcacaa acctcaggat tgagcaaggt ctttgtaatt tttttggttc actttattga 2160

cctgggagca aggtgctaat tctgtggtca gtattcaatg tttttttcag tggagctttt 2220

tctttgggcc atatttgcct tctaatacat tcctgcaata tgtagtggtg atttccctta 2280

gcttcctcct actacctctt atactcatct ccccaaatta tttgcctccc ttaaataagt 2340

tttcctagaa ggtaagctgg tcaggcaatt tgaaaaatat tagatcccaa gaaatctatt 2400

ccgtttgcat tggacttctc ggattccatg tgtttgcagc aggactacat cgaactctga 2460

tgtgccggat tgtggcatgt ctgcatgtct catccatcta ttgtttttgg taactcagtt 2520

tggaatttca gtgtctgtct tccctgggtt gacattggaa tcagcctctc ctttgagctt 2580

attttaactc ttgagcaaca taacatagat ttaatgtgaa cagtttatac caaagggcag 2640

cctgtgcctg tttatggatc ctctctgcct ttgtacttga agagcgcatt ttacatttcc 2700

agtcctttca cagacaggag ctccaacctt acgatggaga attaaacttg cttgtatttc 2760

cactttgtgg atgagaaaaa aaaaaaaaaa aaaaaaaa 2798

<210> 86
<211> 2967
<212> DNA
<213> Homo sapiens

<400> 86
ccggaactgc agttgctgct gcagctgagg tacagcggcg gtttctgagg ttcttcactc 60

gcgactgacg gagctgcggt ggcgtctcca cacgcaacca tgaagttgaa ggacacaaaa 120

tcaaggccaa agcagtcaag ctgtggcaaa tttcagacaa agggaatcaa agttgtggga 180

aaatggaagg aagtgaagat tgacccaaat atgtttgcag atggacagat ggatgacttg 240

gtgtgctttg aggaattgac agattaccag ttggtctccc ctgccaagaa tccctccagt 300

ctcttctcaa aggaagcacc caagagaaag gcacaagctg tttcagaaga agaggaggag 360

gaggagggaa agtctagctc accaaagaaa aagatcaagt tgaagaaaag taaaaatgta 420

gcaactgaag gaaccagtac ccagaaagaa tttgaagtga aagatcctga gctggaggcc 480

cagggagatg acatggtttg tgatgatccg gaggctgggg agatgacatc agaaaacctg 540

gtccaaactg ctccaaaaaa gaagaaaaat aaagggaaaa aagggttgga gccttctcag 600

agcactgctg ccaaggtgcc caaaaaagcg aagacatgga ttcctgaagt tcatgatcag 660

aaagcagatg tgtcagcttg gaaggacctg tttgttccca ggccggttct ccgagcactc 720

agctttctag gcttctctgc acccacacca atccaagccc tgaccttggc acctgccatc 780

cgtgacaaac tggacatcct tggggctgct gagacaggaa gtgggaaaac tcttgccttt 840

gccatcccaa tgattcatgc ggtgttgcag tggcagaaga ggaatgctgc ccctcctcca 900

agtaacaccg aagcaccacc tggagagacc agaactgagg ccggagctga gactagatca 960

ccaggcaagg ctgaagctga gtctgatgca ttgcctgacg atactgtaat tgagagtgaa 1020

gcactgccca gtgatattgc agccgaggcc agagccaaga ctggaggcac tgtctcagac 1080

caggcgttgc tctttggtga cgatgatgct ggtgaagggc cttcttccct gatcagggag 1140

aaacctgttc ccaaacagaa tgagaatgag gaggaaaatc ttgataaaga gcagactgga 1200

aatctaaaac aggagttgga tgacaaaagc gccacctgta aggcatatcc aaagcgtcct 1260

ctgcttggac tggttctgac tcccactcga gagctggccg tccaggtcaa acagcacatt 1320

gatgctgtgg ccaggtttac aggaattaaa actgctattt tggttggtgg aatgtccacg 1380

cagaaacagc agaggatgct gaaccgtcgt cctgagattg tggttgctac tccaggccgg 1440

ctgtgggaat taattaaaga aaagcattat catttgagga accttcggca gctcaggtgc 1500

ctggtagtgg atgaggctga ccggatggtt gagaaaggcc attttgctga gctctcacag 1560

ctgctagaga tgctcaatga ctcccaatac aacccaaaga gacaaacgct tgttttttct 1620

gccacactca ccctggtgca tcaggctcct gctcgaatcc ttcataagaa gcacaccaag 1680

aaaatggata aaacagccaa acttgacctc cttatgcaga aaattggcat gaggggcaag 1740

cccaaggtca ttgacctcac aaggaatgag gccacggtgg agacgctaac agagaccaag 1800

atccattgtg agactgatga gaaagacttc tacttgtact acttcctgat gcagtatcca 1860

ggccgcagct tagtgtttgc caacagtatc tcctgcatca aacgcctctc tgggctcctc 1920

aaagtccttg atatcatgcc cttgaccctg catgcctgta tgcaccagaa gcagaggctc 1980

agaaacctgg agcagtttgc ccgtctggaa gactgtgttc tcttggcaac agatgtggca 2040

gctcggggtc tggatattcc taaagtccag catgtcatcc attaccaggt cccacgtacc 2100

tcggagattt atgtccaccg aagtggtcga actgctcgag ctaccaatga aggcctcagt 2160

ctgatgctca ttggccctga ggatgtgatc aactttaaga agatttacaa aacgctcaag 2220

aaagatgagg atatcccact gttccccgtg cagacaaaat acatggatgt ggtcaaggag 2280

cgaatccgtt tagctcgaca gattgagaaa tctgagtatc ggaacttcca ggcttgcctg 2340

cacaactctt ggattgagca ggcagcagct gccctggaga ttgagctgga agaagacatg 2400

tataagggag gaaaagctga ccagcaagaa gaacgtcgga gacaaaagca gatgaaggtt 2460

ctgaagaagg agctgcgcca cctgctgtcc cagccactgt ttacggagag ccagaaaacc 2520

aagtatccca ctcagtctgg caagccgccc ctgcttgtgt ctgccccaag taagagcgag 2580

tctgctttga gctgtctctc caagcagaag aagaagaaga caaagaagcc gaaggagcca 2640

cagccggaac agccacagcc aagtacaagt gcaaattaac tggtcaagtg tgtcagtgac 2700

tgcacattgg tttctgttct ctggctattt gcaaaacctc tcccaccctt gtgtttcact 2760

ccaccaccaa ccccaggtaa aaaagtctcc ctctcttcca ctcacaccca tagcgggaga 2820

gacctcatgc agatttgcat tgttttggag taagaattca atgcagcagc ttaatttttc 2880

tgtattgcag tgtttatagg cttcttgtgt gttaaacttg atttcataaa ttaaaaacaa 2940

tggtcagaaa aaaaaaaaaa aaaaaaa 2967

<210> 87
<211> 6725
<212> DNA
<213> Homo sapiens

<400> 87
tcggcgggaa gcggcgatcc tgccaccggg aggtgtggaa gagccgggta gattctggct 60

acattggaga ttggttgctt tctaaaactg aaggagaagc ccatgaagag atggtggatt 120

ctcactgagt tttgactagc ggaagaaaag agagagttca agtggatggc cttgaggact 180

tgaaaagctg agatatgatg attttgaagt catttcacat cgaagccatg atttaaatat 240

cggcgttaag atttcaacaa gaaaaactta agcttccttg gattcccacg tcaaaggaaa 300

gtttcaagct ttcagaagga gttctcactc gaagataaag aacagctcgc taaccacgaa 360

agaggaatcg atgctcagct tttagttgca cttcctaaag ttgcagaatt aagacaaatc 420

tttgaaccaa agaagaaaga attcttagaa atgaaaagaa aagaaagaat tgccaggcgc 480

ctggaaggga ttgaaaatga cactcagccc atcctcttgc agagctgcac aggattggtg 540

actcaccgcc tgctggagga agacacccct cgatacatga gagccagcga ccctgccagc 600

ccccacatcg gccgatcaaa tgaagaggag gaaacttctg attcttctct agaaaagcaa 660

actcgatcca aatactgcac agaaacctcc ggtgtccacg gtgactcacc ctatggttcg 720

ggtaccatgg acacccacag tctggagtcc aaagccgaaa gaattgcaag gtacaaagca 780

gaaagaaggc gacagctggc agagaagtat gggctgactc tggatcccga ggccgactcc 840

gagtatttat cccgctatac caagtccagg aaggagcctg atgctgtcga gaagcgggga 900

ggaaaaagtg acaaacagga agagtcaagc agagatgcta gttctctgta ccccgggacc 960

gagacgatgg ggctcaggac ctgtgccggt gaatccaagg actatgccct ccatgtgggt 1020

gacggctctt ccgacccgga ggtgctgctg aacatagaaa accaaagacg aggtcaagag 1080

ctgagtgcca cccggcaggc ccatgacctg tccccagcag ccgagagttc ctcgaccttc 1140

tctttctctg ggcgagactc ctccttcact gaagtgccac ggtcccccaa gcacgcccac 1200

agctcctccc tgcagcaggc agcctcccgg agcccctcct ttggtgaccc acagctatcc 1260

cctgaggccc gacccaggtg cacttcacat tcagaaacgc caactgtcga tgatgaagaa 1320

aaggtggatg aacgagccaa gctgagcgtc gccgccaaga ggttgctttt cagggagatg 1380

gaaaaatctt ttgatgaaca aaatgttcca aagcgacgct caagaaacac agctgtggag 1440

cagaggctac gccgtctgca ggacaggtcc ctcacccagc ccatcaccac tgaagaggtg 1500

gtcatcgcag ccacattgca ggcctctgct caccaaaagg ccttagccaa ggaccagaca 1560

aatgagggca aagagcttgc tgagcaagga gaacctgatt cctccactct aagcttggcc 1620

gaaaagttgg ccttgtttaa caaattgtcc cagccagtct caaaagcgat ttctacccgg 1680

aacagaatag acacgagaca gaggagaatg aacgctcgct atcaaactca gccagtcaca 1740

ctgggagagg tggagcaggt gcagagtgga aagctcattc ctttctcacc tgccgtgaac 1800

acatcagtgt ctaccgtagc atccacggtt gctccaatgt atgccggaga tcttcgcaca 1860

aagccacctc ttgaccacaa tgcaagtgcc actgactata agttttcttc ttcaatagaa 1920

aattcggact ctccagttag aagcattctg aaatcgcaag cttggcagcc tttggtagag 1980

ggtagcgaga acaaggaaat gttgagagaa tatggagaga cagaaagcaa gagagctttg 2040

acaggtcgag acagtgggat ggagaagtat gggtcctttg aggaagcaga agcatcctac 2100

cccatcctga accgagccag ggaaggagac agccataagg aatctaaata tgctgttccc 2160

agaagaggaa gcctggaacg ggcgaaccct cccatcaccc acctcgggga tgaaccgaag 2220

gaattttcca tggctaaaat gaatgcacaa ggaaacttgg acttgaggga caggctgccc 2280

tttgaagaga aggtggaggt ggagaatgtt atgaaaagga agttttcact aagagcggca 2340

gagttcgggg agcccacttc cgagcagacg gggacagctg ctgggaaaac tattgctcaa 2400

accacagccc ccgtgtcctg gaagccccag gattcttcgg aacagccaca ggagaagctc 2460

tgcaagaatc catgtgcgat gtttgctgct ggagagatca aaacgccgac aggggagggc 2520

cttcttgact cacccagcaa aaccatgtct attaaagaaa gattggcact gttgaagaaa 2580

agcggggagg aagattggag aaacagactc agcaggaggc aggagggcgg caaggcgccg 2640

gccagcagcc tgcacaccca ggaagcaggg cggtccctca tcaagaagcg ggtcacagaa 2700

agtcgagaga gccaaatgac gattgaggag aggaagcagc tcatcactgt gagagaggag 2760

gcctggaaga cgagaggcag aggagcggcc aacgactcga cccagttcac tgtggctggc 2820

aggatggtga agaaaggttt ggcgtcacct actgccataa ccccagtagc ctcacccatt 2880

tgcggtaaaa caagaggcac cacacccgtt tccaaacccc tggaagatat cgaagccaga 2940

ccagatatgc agttagaatc ggacctgaag ttggacaggc tggaaacctt tctaagaagg 3000

ctgaataaca aagttggcgg gatgcacgaa acggtgctca ctgtcaccgg caaatctgtg 3060

aaggaggtga tgaagccaga tgatgatgaa acctttgcca aattttaccg cagcgtggat 3120

tataatatgc caagaagtcc tgtggagatg gatgaggact tcgatgtcat tttcgatcct 3180

tatgcaccca aattgacgtc ttccgtggcc gagcacaagc gggcagttag gcccaagcgc 3240

cgggttcagg cctccaaaaa ccccctgaaa atgctggcgg caagagaaga tctccttcag 3300

gaatacactg agcagagatt aaacgttgcc ttcatggagt caaagcggat gaaagtagaa 3360

aagatgtctt ccaactccaa cttctcagaa gtcaccctgg cgggtttagc cagtaaagaa 3420

aacttcagca acgtcagcct gcggagcgtc aacctgacgg aacagaactc taacaacagc 3480

gccgtgccct acaagaggct gatgctgttg cagattaaag gaagaagaca tgtgcagacc 3540

aggctggtgg aacctcgagc ttcggcgctc aacagtgggg actgcttcct cctgctctct 3600

ccccactgct gcttcctgtg ggtaggagag tttgcaaacg tcatagaaaa ggcgaaggcc 3660

tcagaacttg caactttaat tcagacaaag agggaacttg gttgtagagc tacttatatc 3720

caaaccattg aagaaggaat taatacacac actcatgcag ccaaagactt ctggaagctt 3780

ctgggtggcc aaaccagtta ccaatctgct ggagacccaa aagaagatga actctatgaa 3840

gcagccataa tagaaactaa ctgcatttac cgtctcatgg atgacaaact tgttcctgat 3900

gacgactact ggggaaaaat tccgaagtgc tcccttctgc aacccaaaga ggtactggtg 3960

tttgattttg gtagtgaagt ttacgtatgg catgggaaag aagtcacatt agcacaacga 4020

aaaatagcat ttcagctggc aaagcactta tggaatggaa cctttgacta tgagaactgt 4080

gacatcaatc ccctggatcc tggagaatgc aatccgctta tccccagaaa aggacagggg 4140

cggcccgact gggcgatatt tgggagactt actgaacaca atgagacgat tttgttcaaa 4200

gagaagtttc tggattggac ggaactgaag agatcgaatg agaagaaccc cggggaactt 4260

gcccagcaca aggaagaccc caggactgat gtcaaggcat acgatgtgac acggatggtg 4320

tccatgcccc agacgacagc aggcaccatc ctggacggag tgaacgtcgg ccgtggctat 4380

ggcctggtgg aaggacacga caggaggcag tttgagatca ccagcgtttc cgtggatgtc 4440

tggcacatcc tggaattcga ctatagcagg ctccccaaac aaagcatcgg gcagttccat 4500

gagggggatg cctatgtggt caagtggaag ttcatggtga gcacggcagt gggaagtcgc 4560

cagaagggag agcactcggt gagggcagcc ggcaaagaga agtgcgtcta cttcttctgg 4620

caaggccggc actccaccgt gagtgagaag ggcacgtcgg cgctgatgac ggtggagctg 4680

gacgaggaaa gggggcccca ggtccaggtt ctccagggaa aggagccccc ctgtttcctg 4740

cagtgtttcc agggggggat ggtggtgcac tcggggaggc gggaagagga agaagaaaat 4800

gtgcaaagtg agtggcggct gtactgcgtg cgtggagagg tgcccgtgga agggaatttg 4860

ctggaagtgg cctgtcactg tagcagcctg aggtccagaa cttccatggt ggtgcttaac 4920

gtcaacaagg ccctcatcta cctgtggcac ggatgcaaag cccaggccca cacgaaggag 4980

gtcggaagga ccgctgcgaa caagatcaag gaacaatgtc ccctggaagc aggactgcat 5040

agtagcagca aagtcacaat acacgagtgt gatgaaggct ccgagccact cggattctgg 5100

gatgccttag gaaggagaga caggaaagcc tacgattgca tgcttcaaga tcctggaagt 5160

tttaacttcg cgccccgcct gttcatcctc agcagctcct ctggggattt tgcagccaca 5220

gagtttgtgt accctgcccg agccccctct gtggtcagtt ccatgccctt cctgcaggaa 5280

gatctgtaca gcgcgcccca gccagcactt ttccttgttg acaatcacca cgaggtgtac 5340

ctctggcaag gctggtggcc catcgagaac aagatcactg gttccgcccg catccgctgg 5400

gcctccgacc ggaagagtgc gatggagact gtgctccagt actgcaaagg aaaaaatctc 5460

aagaaaccag cccccaagtc ttaccttatc cacgctggtc tggagcccct gacattcacc 5520

aatatgtttc ccagctggga gcacagagag gacatcgctg agatcacaga gatggacacg 5580

gaagtttcca atcagatcac cctcgtggaa gacgtcttag ccaagctctg taaaaccatt 5640

tacccgctgg ccgacctcct ggccaggcca ctcccggagg gggtcgatcc tctgaagctt 5700

gagatctatc tcaccgacga agacttcgag tttgcactag acatgacgag ggatgaatac 5760

aacgccctgc ccgcctggaa gcaggtgaac ctgaagaaag caaaaggcct gttctgagtg 5820

gggagacgcc agaggagcct cacggtcacg tccaacaaca ccactgcacc agggaaatgg 5880

atatatattt ttggactggt gtttttcaca aagtattttt caatcagagt tttcagaacc 5940

tgacattgtt aaagatactg cttgtcccgg agttgtgtat tttgtaaatg ttcaagggaa 6000

ctgtttggaa acttctttcc accattcagg aggttatcag aattaataaa agtatctgtt 6060

atgtgcactt aagccgcagc tgctatagat agcactgcct tcttgttcca gctaggcaat 6120

gccttttttt tttttttttg aagcagttct ctttataaag tgttattttg atagtttgtg 6180

gattctaaaa tatatatata tttatataaa caccatataa gtcaaatatg tatttaacaa 6240

agcaatatgt attcattcac tttcaagatt tgttttggtg tcaaaataac atgaaaaggt 6300

agatggagtt gcttctgttg aattagctct gccaccaata tgtatcttca tacacgtttg 6360

gaaatgtttc ctgcagcatt aggtatgact tgttctgagt actgcttccg gtgctaaaat 6420

gaacaaagaa tttgtactta atggcatgga ctctggagaa tctatgcgaa tcaacctttc 6480

taccttaata tctccccaaa aatgtatagt gccttgtttt tatgtacagt ttatatacag 6540

aaaagtttgc tctgcatttt tgatgatggt ttggaacatt atctacaatt ttactctcaa 6600

atagtcaaaa taaaaacatc tcaatttcta ataccggttg taaacagtac acatgtcatt 6660

ttgtgatata ggactcccaa ataaaagtat cagaataaac acaacaatta actggtaaaa 6720

aaaaa 6725

<210> 88
<211> 2939
<212> DNA
<213> Homo sapiens

<400> 88
agttaatagg ctggctgtcg cttggggttg ctgcataaat gctaggagca gcagctctat 60

ggttatgagg tggggagaac ggaatgacgt catcgcttgg gagctgctgc aggatggagt 120

ggaaagctgc tgctgatggc attgtttttg tggcagcaag ctgaatgaca gatcctcact 180

acaaagatac ccctttggcc cccgtgtagg cctccttggt tcgggtgttt caccatgcca 240

gcacagcgcc atgagtcctg gatgcatgct gctgtttgtg tttggctttg ttggcggggc 300

ggtggtcatt aattctgcta tcttagtatc tctctctgtt ttgctgcttg tgcacttttc 360

tatttctacc ggtgtgccag ctctgacgca gaacctacca aggatactca gaaaagaacg 420

ccctatatca ttaggaattt tcccattacc tgctggagat ggattgctta cacctgatgc 480

tcagaaagga ggagagaccc ctggatctga gcaatggaaa tttcaggaat taagtcaacc 540

acgttctcat accagcctga aggatgagct ttctgatgtt agccaaggcg gatctaaagc 600

taccactcca gcatcaacag ctaattcaga tgtggcaaca attcctactg atactccctt 660

aaaggaagaa aacgaaggat ttgtgaaggt tacagatgcg ccaaataaat cagagataag 720

caaacacatt gaagtacagg tagcccagga aactagaaat gtatctactg gctctgctga 780

aaatgaagaa aagtcagaag ttcaagcaat catcgaatct actcctgagc tggatatgga 840

caaagatctc agtggatata aaggttcaag cactcccacc aaaggcatag agaacaaagc 900

ttttgatcgc aatacagaat ctctctttga agaaagcagc accttatctc agctccctgg 960

ggataagtcc aaagcctttg atttccttag tgaagaaact gaagctagtt tagcctcacg 1020

cagagaacaa aagagagagc agtatcgtca ggtaaaagca catgttcaga aggaagacgg 1080

tagagtgcag gcttttggct ggagtctgcc tcagaagtac aaacaggtaa ccaatggtca 1140

aggtgaaaat aagatgaaaa atttacctgt gcctgtctat ctcagacctc tggatgaaaa 1200

agatacatca atgaagctgt ggtgtgctgt tggagtcaat ttatctggtg ggaagaccag 1260

agatggtggt tctgttgttg gagcaagtgt attttacaag gatgttgctg gtttggatac 1320

agaaggcagt aaacagcgaa gtgcctctca gagtagttta gataagttag atcaggaact 1380

taaggaacag cagaaggagt taaaaaatca agaagaatta tccagtctag tttggatctg 1440

taccagcact cattcggcta caaaagttct tattattgat gctgttcaac ctggcaacat 1500

cctagacagt ttcactgttt gcaactctca tgttctgtgc attgcaagtg tgccaggtgc 1560

acgagaaaca gactaccctg caggagaaga tctttcagaa tctggtcagg tagacaaagc 1620

atctttatgt ggaagtatga caagcaacag ctcagcagag acagacagcc tgttaggagg 1680

catcacagtg gttggttgtt ctgcagaagg tgtgacggga gctgccactt cccctagtac 1740

aaatggtgct tctccagtga tggataaacc accagaaatg gaagcagaaa atagtgaggt 1800

tgatgaaaat gttccaacag cagaagaagc aactgaagct acagaaggga atgcggggtc 1860

agctgaagac acagtggaca tctcccaaac tggcgtctac acagagcatg tctttacaga 1920

tcctttggga gttcagatcc cagaagacct ctccccagtg tatcagtcga gcaatgactc 1980

agatgcatat aaagatcaaa tatcagtact gccaaatgaa caagacttgg tgagagaaga 2040

agcccagaaa atgagtagtc ttttaccaac tatgtggctt ggagctcaaa atggctgttt 2100

gtatgtccat tcatctgtag cccagtggag gaaatgtctc cattccatta aacttaaaga 2160

ttcgattctc agtattgtac acgtgaaggg aatcgtgtta gtagccctgg ctgacggcac 2220

ccttgcaatc tttcacagag gagtggatgg gcagtgggat ttgtcaaact atcacctctt 2280

agaccttgga cggcctcatc attccatccg ttgcatgact gtggtacatg acaaagtctg 2340

gtgtggctat aggaacaaaa tctatgtggt gcactccagc ctgggtgacg gagcaagacc 2400

ctgtctcaaa acagacaaac aagcaaaaaa tagattaaag tctggatttc actgattttc 2460

ttgcttaata agttttttaa aaccacgatg ctgcaatttt ttccctctca agcttcttga 2520

aaatgtgtga tttacccttt tttatctatt actctaggca aagctaaatg taattttatg 2580

tactgtttta acagtataaa cctatgggat aaaggccccc aatcatcaga aggactactg 2640

aaaagaaaag aaagcaaacg tagatgtcaa attgtctaaa cattcttaag taccactgat 2700

tttttttttc tggactatta tacaaagatg tctgtaaaaa gtataccagt ggcttttatg 2760

catatacacg attaggctag attgtgaagt acatggattt tcatgagcca gaggaggcat 2820

ttggatccat tgggccacat gtgactataa gcacattgtg tacacaataa aattgtagcc 2880

actcatgtat taaaactgtt ttggtaaaag ctgtattaaa agaataattt tttgcttga 2939

<210> 89
<211> 1001
<212> DNA
<213> Homo sapiens

<400> 89
gaaaaagatg gcgccccgcg cccggggggc cccttcctga gcgccccggc ccctccctcc 60

gccgcctccc cctcctcccc gcggcgcccc cgccccgccc cgcccccgcc gagaccccga 120

ccccggcccc acgggcggac actcggccgg gcagccgcgg gccgagcgca gccgcctccg 180

ccaccgatgc gcctggtggc cagactccaa gtgggaccgg cggacacgca gcctcgcgtg 240

tcaggggaag ctgatggaga atcgagctct ggatccaggg actcgggact cctatggtgc 300

caccagccac ctccccaaca agggggccct ggcgaaggtc aagaacaact tcaaagactt 360

gatgtccaaa ctgacggagg gccagtatgt gctgtgccgg tggacagatg gcctgtacta 420

cctcgggaag atcaagaggg tcagcagctc taagcaaagc tgcctcgtga ctttcgaaga 480

taattccaaa tactgggtcc tatggaagga catacagcat gccggtgttc caggagagga 540

gcccaagtgc aacatctgcc tagggaagac atcagggccg ctgaatgaga tcctcatctg 600

cgggaagtgt ggcctgggtt accaccagca gtgccacatc cccatagcgg gcagtgctga 660

ccagcccctg ctcacacctt ggttctgccg acgctgcatc ttcgcactgg ctgtgcgggt 720

gagccttcca tcctccccag tccctgcctc tcctgcctcc tccagtgggg cagaccagag 780

actcccatca cagagtctga gctccaagca gaagggccac acctgggctt tggagacaga 840

tagcgcctct gccactgtcc ttggccagga tttgtagact ccctgagcct cagtttcctc 900

aactgtaaag tggagatggg tttggtgtcg ggaataacgg gaccaataaa tgatgcttta 960

ctattaaaaa agaaaatcca tgtaaataaa aaaaaaaaaa a 1001

<210> 90
<211> 2995
<212> DNA
<213> Homo sapiens

<400> 90
gcgggttcga gtcccgcctc ctgactctgg cctctagtcc ctgagtcccg ggcgggctgc 60

attcgtcggg gaaacctctc ctcgaccagg ggcacctcta ctcgaccagg ggcgacggcg 120

tactttgggc ttcatcatgg aggactacct gcagggttgt cgagctgctc tgcaggagtc 180

ccgacctcta catgttgtgc tgggaaatga agcctgtgat ttggactcca cagtgtctgc 240

tcttgccctg gctttttacc tagcaaagac aactgaggct gaggaagtct ttgtgccagt 300

tttaaatata aaacgttctg aactacctct gcgaggtgac attgtcttct ttcttcagaa 360

ggttcatatt ccagagagta tcttgatttt tcgggatgag attgacctcc atgcattata 420

ccaggctggc caactcaccc tcatccttgt cgaccatcat atcttatcca aaagtgacac 480

agccctagag gaggcagtag cagaggtgct agaccatcga cccatcgagc cgaaacactg 540

ccctccctgc catgtttcag ttgagctggt ggggtcctgt gctaccctgg tgaccgagag 600

aatcctgcag ggggcaccag agatcttgga caggcaaact gcagcccttc tgcatggaac 660

catcatcctg gactgtgtca acatggacct taaaattgga aaggcaaccc caaaggacag 720

caaatatgtg gagaaactag aggccctttt cccagaccta cccaagagaa atgatatatt 780

tgattcccta caaaaggcaa agtttgatgt atcaggactg accactgagc agatgctgag 840

aaaagaccag aagactatct atagacaagg cgtcaaggtg gccattagtg caatatatat 900

ggatttggag gcctttctgc agaggtctaa cctccttgca gatctccatg ctttctgcca 960

ggctcacagc tatgatgtcc tggttgccat gactatcttt ttcaacactc acaatgagcc 1020

agtgcggcag ttggctattt tctgtcccca tgtggcactc caaacaacga tctgtgaagt 1080

cctggaacgc tcccactctc cacccctgaa gctgacccct gcctcaagta cccaccctaa 1140

cctccatgcc tatcttcaag gcaacaccca ggtctctcga aagaaacttc tgcccctgct 1200

ccaggaagcc ctgtcagcat attttgactc catgaagatc ccttcaggac agcctgagac 1260

agcagatgtg tccagggagc aagtggacaa ggaattggac agggcaagta actccctgat 1320

ttctggactg agtcaagatg aggaggaccc tccgctgccc ccgacgccca tgaacagctt 1380

ggtggatgag tgccctctag atcaggggct gcctaaactc tctgctgagg ccgtcttcga 1440

gaagtgcagt cagatctcac tgtcacagtc taccacagcc tccctgtcca agaagtgact 1500

gttgagaggc gaggaggtag tgggtgaggc tacctgactc acttcaaatg catgttttga 1560

gatgtttgga gattcagcaa ttctgtcttc attgctccag gatctggtat actgttctca 1620

taaaactgag aggagaaaaa aagtgaaaga aagcagctgc tttaagaatg gttttccacc 1680

ttttccccct aatctctacc aatcagacac attttattat ttaaatctgc acctctctct 1740

attttatttg ccaggggcac gatgtgacat atctgcagtc ccagcacagt gggacaaaaa 1800

gaatttagac cccaaaagtg tcctcggcat ggatcttgaa cagaaccagt atctgtcatg 1860

gaactgaaca ttcatcgatg gtctccatgt attcatttat tcacttgttc attcaagtat 1920

ttattgaata cctgcctcaa gctagagaga aaagagagtg cgctttggaa atttattcca 1980

gttttcagcc tacagcagat tatcagctcg gtgacttttc tttctgccac catttaggtg 2040

atggtgtttg attcagagat ggctgaattt ctattcttag cttattgtga ctgtttcaga 2100

tctagtttgg gaacagatta gaggccattg tcttctgtcc tgatcaggtg gcctggctgt 2160

ttctttggat ccctctgtcc cagagccacc cagaaccctg actcttgaga atcaagaaaa 2220

cacccagaaa ggccttaatg acctcatagg cactcttcca aaaagacaac agaactggaa 2280

tgagaggcct gggtctgtct cctgccttag caggcctatc aatttcttgt caatctcttt 2340

ttttccttgc tcacattaaa aggaagcatg gagttctaat gctcccataa actatgtatt 2400

ttggcaagac acttcactac tccaggtctc actttcccca tctgtaaaac agggtttgga 2460

ctaggtgttc cctggtattc tgtgatctgc ctcttgctgc cattctttct ctcctctgct 2520

tctctgtatt tttcttctgt tatccctggg ggtgctcagg ttcacttgat tgtctgtatt 2580

tctgtgtggt tgtagcaagg actcagcctc atgtagcacg aataggggtg tggttcatgg 2640

cgtgttgacc cagcagagca ctccctccca ctaacttgtt ctgcatgtgt agagtctccc 2700

catttttttt aacgcaaccc tttccccttt ttcctacccc acagctctgt tccatgtaag 2760

ttgccaacag tttcactgaa cagtggggta tgtgatggtt ttggcatgac atcttcagta 2820

tgagggggac agtttgactt cactttgagg gtgtgatgtc tgtagctatg tggaaggtaa 2880

aaatagtggt gtgatcatga accaaaggaa tttatgtttt gtaacttggg tactttattt 2940

tgcattttgt tatactatta aataattttt tcctgttaaa aaaaaaaaaa aaaaa 2995

<210> 91
<211> 4709
<212> DNA
<213> Homo sapiens

<400> 91
tgcatgctgg atgcagtggc tcatgcctgt aatctcagca ctttgggagg ccaaggtggg 60

aggatcgctt aaactcagga gtttgagacc agcctgggca acatggtgaa aacctgtctc 120

tataaaaaat taaaaaataa taataataac tggtgtggtg ttgtgcacct agagttccaa 180

ctactaggga agctgagatg agaggatacc ttgagctggg gactggggag gcttaggtta 240

cagtaagctg agattgtgcc actgcactcc agcttggaca aaagagcctg atcctgtctc 300

aaaaaaaaga aagataccca gggcaagtta agttcggagg ggcacagagc tcccatgccc 360

tctgttgaac atgcgaccct cccagcatct cctgtgtcca gcaaccctga aagctctgca 420

aacccctttc agggtgttta tggaggcttt attatgcaag catgattgat aaaacctttg 480

gctgttggtg attaagtcag tctccagccc ctcttccccc tggagttcag tgcatgaggc 540

tgaaagttcc aagcctctta ccatgtggtt tcatggtaat cagccctcct cttgaagaaa 600

tttaggagct tgcagtcacc cagtcatctc aacaacatcc ccaaatgcat tcttaccatg 660

ctggagatcc caaagttctt agaggctctt gtgttagaaa cctgggacca agaccaaata 720

ttaaaacaaa agatgctcct gtcacatcta tcactgaggt ctttgtaaga gctttagaag 780

ctctgtgcca ggaaccaggg acagagatta aatatatatt tcttttcttt tttttgagac 840

agaatctccc tgtgccatcc aggctggagt gcagtgatgt gatcatagct cactatagct 900

ttggccttct gagatcaagc aatcctccca tctcaacctc ccaagtagct aggactacac 960

atgcatgtca cccatgccca gatcattttt gtagagtcag agtttcaccg tggtggccag 1020

gttggccatg ttggccaaat ggggtcttct tttgttgccc aggctggcca caagttcctg 1080

ggctcaagtg atcctcccac ctcgtccttg tagagatgag atttagttat gtcgtccagg 1140

ctgatctcaa actcctgggc taaatcgatt gactcacctc agcctctcaa gtatgttatg 1200

aaggttatat gttaggaagg gtcccaggag gtagacccac acagatggga tttgggcata 1260

ggtttggttt cccagggggc agtgctgagc tctttgccag tgggaaatgg gatgctggtg 1320

atttccagta ggtgacctca cagtgactca agctaccact tactgttgat tgtgacgaaa 1380

tgccagctga ggcacatgcc ttgggagcta agtggttgct gcccttgacc actgtgaaga 1440

ctggtgtggg aagggtcgtt ttggatgcac ttgagcaggg gtccccaacc cctgagccat 1500

ggagccgcaa ggagccacac agcaggaggt gagtggtgtc gagtgaggga gtgagggaag 1560

cttcctctgt atttacagcc actccccttt gctcacattc ccgcctgagc tccaccttct 1620

cagatgagca gcagcattag atgctcatag gagaacgcac cctgttgtga accatgcatg 1680

tgagggatct aggttgcgct gtccttatga gagtctaata cctattgatc tgtcactttc 1740

tcccatcacg ctcaggtggg aacatccagt tgcaggaaaa caagcttaac acgcccactg 1800

attctacatt atgctcctac ctcccggcag cctctccagg cccagaactt tctccagtca 1860

gcctctacag accaagctca tgactcaaaa tggcctattt aggcccatac cctacgtcac 1920

ggcagcctcc gcagatgagg ctactgcctc acaacagcct ccacaggcac agctccatcg 1980

ttacaatggc ctctttagac ccagctcctg cctcccagcc ttctctccag gccctgaaca 2040

ttctcaggtc tccctctgtt gtccaaggct ggagtgtagt agtgctatcg cagctgactg 2100

cagcctcaac cttccaggct gaagcgatcc tcccacgtca acctcccacg tggctgagac 2160

tacaggtgct tgccactatg cccaactaat atttggaatt ttcatatacg tggattccag 2220

aggggtgaca gcgaaacgtg ggaacatcca gttgcaggaa aacaagctta acacgcccac 2280

tgattctaca ttatgctcct acctcccggc agcctctcca ggcccagaac tttctccagt 2340

cagcctctac agaccaagct catgactctc aatggcctat ttaggcccat accctacgtc 2400

acggcagcct ccgcagatga ggctactgcc tcacaacagc ctccacaggc acagctccat 2460

cgttacaatg gcctctttag acccagctcc tgcctcccag ccttctctcc aggccctgaa 2520

ctttctcaag ttgacctcac caggcccagc tcatgcttct ttgcagcctc tccaggccca 2580

gctcctgcat cttggtggcc cctccaggcc cagcctctgc ctcccgtcag cctctacagt 2640

cccaacgtct gcctcacagc agattcttca cgcccagctt ctacctcact gtggaccccc 2700

caagccaagc tcccaacctt tcagcagctt ctacacaccc agctcctgcc acccagtggc 2760

ctctttaggc caagctcatg cttcacaagg gcctttccag gcccaacttt tgtctcatgg 2820

caaccttccc tggccagatt cctgcctgtc tcccagcagc ctagacaggc ccaggtcttg 2880

cctcacactg gcctctctac atccagctta tgcctcacgg tggcctctcc acggccaact 2940

cctgtcccag gacatcatct ccgggcccaa aacttactca agtcagactc tctagtccca 3000

actgctgcct cctggtggcc tatgaaggcc caaaatctcc tcaagttgac ctgtccaggc 3060

ccagctcctg cctcctgtca gcgtctacag gcccaacctc tgcctcatgg gggcttctcc 3120

aggcccacct cttcctcttg gctgggtcta caggcacaac tgctgcctca caacagcctt 3180

ttttggccca gttcctgtcc agctaatggc ggccaatgta ggcccaaaac ttcctcaagt 3240

caaactctcc aggcccacct tctgcttccc ggtggcatca acaggcccag ctttgacttg 3300

agaacagcct ctgcaggccc tgctcttgcc tcccaggggc tttttccagg cccagctctt 3360

gcctcatggc agctgcccca ggccaaattt ctgcctgcct gccagcagcc tcaacaggca 3420

cagctcctcc ctcacagtgg cccatttagg cccaacttat gactgtgagg ccatttccag 3480

gcctagtgcc tgcctcgtgg ctgactcttg aagcccaaaa cttcctcaaa tcaggctttt 3540

gcccaacttc tgtctactgt cggactctac aggtcagcct ctgcctcaca gtggaccctc 3600

cagacccaga tggtgtctca ctgtggcatc ctcaggtgaa gctcctgcct ttcggcagcc 3660

tctccaggcc cagctcctcc tgcctcccag tggcctcttt cggcccagcc cagctcatgc 3720

ctcccggcgg ccttcccaag ccccgctttt gactttcggt ggcctctgca ggcctcgaca 3780

aggcccagcc tcctgcctcc cgaaggcctg cacaggccca gcctctgcct cacagcggac 3840

tctccacgcc cagctagctg ttgcttcact gcggcctccc gagtccaaag ctcctgcctc 3900

tcggccgctt cggcaggccc agctcccgcc tgccagtggc ctcttcaggc ccatggggct 3960

cattcctgac aacggccttt ccaggcccag tttttccctt ccagcggcct ctccgggccc 4020

agaacctcct caagtcggcc tctccagacc cacttgcacc ctccgggcgt tctctccggg 4080

cccagctctt cttcctggtt gggtctccag gcccgattcc tgcctctcaa caacctcttt 4140

ggactcagtg cctacccatc tcctggtggc cttggtcggt ccacagcttc ctcaagccaa 4200

gctccccagg cccaggtcag gcctcacggt ggcctctcca ggatgagctc ctgccctccg 4260

atggcatctc caggccccaa atggtctccg gtcggtgggc tcctccacgc caaggttggg 4320

cctcccggcg accgccgcag gcccaagttg tcctgaagtc gggctctcct ggccctgcct 4380

cccagcaagt aagcaagctc ttttggctca actcctgccc agctcccaac cgcctttgta 4440

ggccccgaac tttctccagc caagctctga gggcccacct cctgcctcct ggtggcctgt 4500

acagttctag cactggttgg agaacagcct ctgcaggccc cgcccttgcc tcccaggggc 4560

ctctccaggc ccagctcttg cccccacggc ggcctcccgg ggccaagtcc ctgcctgcct 4620

cccagcagcc cgcgtgcggc ccagctcctc cctcacggtg gcctgttgat gcccaactca 4680

tgcctctggc accctgccca gaggtgtga 4709

<210> 92
<211> 3630
<212> DNA
<213> Homo sapiens

<400> 92
ggtattccag aagtgtttga ggatcccttc catgaaggaa gagaggaaag tttttaagta 60

aacctcccac tcccatgtgt cttcagcttt cttttgcaaa ggagaaaatc cttgaagttt 120

ggtaaagacc gagttagtct atctctcttt gcctatctcg agttgggctg gggagaggag 180

gagataggtt cttttgtctt tttctgtctt ctcccttccc cacttccttc cctccagtcc 240

ccactcactc acatgcacac actaaccttg gagccgatgg gattgagtga ctggcacttg 300

ggaccacaga gaaatgtcag agtgtttggt tacagactca aggaaacctc tcattttaga 360

gtgctcattt ggttttgagc aaaattttgg actgtgaagc aaggcattgg tgaagacaaa 420

atggcctcgc cggctgacag ctgtatccag ttcacccgcc atgccagtga tgttcttctc 480

aaccttaatc gtctccggag tcgagacatc ttgactgatg ttgtcattgt tgtgagccgt 540

gagcagttta gagcccataa aacggtcctc atggcctgca gtggcctgtt ctatagcatc 600

tttacagacc agttgaaatg caaccttagt gtgatcaatc tagatcctga gatcaaccct 660

gagggattct gcatcctcct ggacttcatg tacacatctc ggctcaattt gcgggagggc 720

aacatcatgg ctgtgatggc cacggctatg tacctgcaga tggagcatgt tgtggacact 780

tgccggaagt ttattaaggc cagtgaagca gagatggttt ctgccatcaa gcctcctcgt 840

gaagagttcc tcaacagccg gatgctgatg ccccaagaca tcatggccta tcggggtcgt 900

gaggtggtgg agaacaacct gccactgagg agcgcccctg ggtgtgagag cagagccttt 960

gcccccagcc tgtacagtgg cctgtccaca ccgccagcct cttattccat gtacagccac 1020

ctccctgtca gcagcctcct cttctccgat gaggagtttc gggatgtccg gatgcctgtg 1080

gccaacccct tccccaagga gcgggcactc ccatgtgata gtgccaggcc agtccctggt 1140

gagtacagcc ggccgacttt ggaggtgtcc cccaatgtgt gccacagcaa tatctattca 1200

cccaaggaaa caatcccaga agaggcacga agtgatatgc actacagtgt ggctgagggc 1260

ctcaaacctg ctgccccctc agcccgaaat gccccctact tcccttgtga caaggccagc 1320

aaagaagaag agagaccctc ctcggaagat gagattgccc tgcatttcga gccccccaat 1380

gcacccctga accggaaggg tctggttagt ccacagagcc cccagaaatc tgactgccag 1440

cccaactcgc ccacagagtc ctgcagcagt aagaatgcct gcatcctcca ggcttctggc 1500

tcccctccag ccaagagccc cactgacccc aaagcctgca actggaagaa atacaagttc 1560

atcgtgctca acagcctcaa ccagaatgcc aaaccagagg ggcctgagca ggctgagctg 1620

ggccgccttt ccccacgagc ctacacggcc ccacctgcct gccagccacc catggagcct 1680

gagaaccttg acctccagtc cccaaccaag ctgagtgcca gcggggagga ctccaccatc 1740

ccacaagcca gccggctcaa taacatcgtt aacaggtcca tgacgggctc tccccgcagc 1800

agcagcgaga gccactcacc actctacatg caccccccga agtgcacgtc ctgcggctct 1860

cagtccccac agcatgcaga gatgtgcctc cacaccgctg gccccacgtt ccctgaggag 1920

atgggagaga cccagtctga gtactcagat tctagctgtg agaacggggc cttcttctgc 1980

aatgagtgtg actgccgctt ctctgaggag gcctcactca agaggcacac gctgcagacc 2040

cacagtgaca aaccctacaa gtgtgaccgc tgccaggcct ccttccgcta caagggcaac 2100

ctcgccagcc acaagaccgt ccataccggt gagaaaccct atcgttgcaa catctgtggg 2160

gcccagttca accggccagc caacctgaaa acccacactc gaattcactc tggagagaag 2220

ccctacaaat gcgaaacctg cggagccaga tttgtacagg tggcccacct ccgtgcccat 2280

gtgcttatcc acactggtga gaagccctat ccctgtgaaa tctgtggcac ccgtttccgg 2340

caccttcaga ctctgaagag ccacctgcga atccacacag gagagaaacc ttaccattgt 2400

gagaagtgta acctgcattt ccgtcacaaa agccagctgc gacttcactt gcgccagaag 2460

catggcgcca tcaccaacac caaggtgcaa taccgcgtgt cagccactga cctgcctccg 2520

gagctcccca aagcctgctg aagcatggag tgttgatgct ttcgtctcca gccccttctc 2580

agaatctacc caaaggatac tgtaacactt tacaatgttc atcccatgat gtagtgcctc 2640

tttcatccac tagtgcaaat catagctggg ggttgggggt ggtgggggtc ggggcctggg 2700

ggactgggag ccgcagcagc tccccctccc ccactgccat aaaacattaa gaaaatcata 2760

ttgcttcttc tcctatgtgt aaggtgaacc atgtcagcaa aaagcaaaat cattttatat 2820

gtcaaagcag gggagtatgc aaaagttctg acttgacttt agtctgcaaa atgaggaatg 2880

tatatgtttt gtgggaacag atgtttcttt tgtatgtaaa tgtgcattct tttaaaagac 2940

aagacttcag tatgttgtca aagagagggc tttaattttt ttaaccaaag gtgaaggaat 3000

atatggcaga gttgtaaata tataaatata tatatatata aaataaatat atataaacct 3060

aacaaagata tattaaaaat ataaaactgc gttaaaggct cgattttgta tctgcaggca 3120

gacacggatc tgagaatctt tattgagaaa gagcacttaa gagaatattt taagtattgc 3180

atctgtataa gtaagaaaat attttgtcta aaatgcctca gtgtatttgt atttttttgc 3240

aagtgaaggt ttacaattta caaagtgtgt attaaaaaaa acaaaaagaa caaaaaaatc 3300

tgcagaagga aaaatgtgta attttgttct agttttcagt ttgtatatac ccgtacaacg 3360

tgtcctcacg gtgccttttt tcacggaagt tttcaatgat gggcgagcgt gcaccatccc 3420

tttttgaagt gtaggcagac acagggactt gaagttgtta ctaactaaac tctctttggg 3480

aatgtttgtc tcatcccatt ctgcgtcatg cttgtgttat aactactccg gagacagggt 3540

ttggctgtgt ctaaactgca ttaccgcgtt gtaaaatata gctgtacaaa tataagaata 3600

aaatgttgaa aagtcaaact ggaaaaaaaa 3630

<210> 93
<211> 374
<212> DNA
<213> Homo sapiens

<400> 93
atgtctggcc gtggtaaagg tggaaaaggt ttgggtaagg gaggagctaa gcgtcatcgc 60

aaggttttgc gcgataacat ccagggcatc actaagccag ctatccggcg ccttgctcgt 120

cgcggcggtg tcaagcgaat ttctggcctt atctatgagg agactcgtgg tgttctgaag 180

gtgttcctgg agaacgtgat tcgtgacgct gtcacttaca cagagcacgc caaacgcaag 240

accgtgacag caatggatgt ggtctacgcg ctgaagcgac agggacgcac tctttacggc 300

ttcggtggct aaggctcctg cttgctgcac tcttattttc attttcaacc aaaggccctt 360

ttcagggccg ccca 374

<210> 94
<211> 681
<212> DNA

<213>  Homo sapiens

<220>
<221>  misc_feature
<222>  (637)..(637)
<223>  n is a, c, g, t or u

<400>  94
tttttttttt ttttttttaa ttgtgatatt tgctttatta taatggtccg gaacagaacc      60

tggaatatct ccaaactata ccattgtgct taaggcaata gtatttttgg acaaacttcg      120

ctgaacatct ttacatttat tttactctct gatgttgttt tcattaagga tgacatgtga      180

atgaagcacc acagtcctta agtgtgtagg gcttctaaat atcttaatct tgtcccttgg      240

ggaggagtga gggctacgaa gtaaggtcac agtcttcccc tagatgttga cagctcttat      300

gtactcagga tgtcaatttt cacagtgcac acttcttatt gtcactccat agatactcct      360

ggcagctgtg tgtgtctatg tgtgtgggtt ttctttgctg atgtggtctg aaggccattt      420

ctgagctcta ccagtccagt ccaatttatg tgattctcac ctgtcataat gttgggtttc      480

ttcaacatta gtggtaggtt ttcctcgtct gctctggttg caatatttac tctcaaagcg      540

gagggcttcc caggttccta atcacctctg agctatggat acagccaatc cagatggtcc      600

aggttaaaac aagtcttgga tcaagagtat cattcanagg attttaaccc atgtcagagc      660

tttcctcgtg ccgaattctt g                                                681

<210>  95
<211>  3241
<212>  DNA
<213>  Homo sapiens

<400>  95
ctgcgcgacc gtgattcccc gctcgcgact ccccaccccc cagggctccc taaagagggc      60

cacgagctgc gaaagggcgg gaaaggcagt tggagaagag gtaagcggtt actcactcca      120

tggctgcagc aaggagaggc ggcggcggcc tcggctgaag aaagaagaaa tcttcccaag      180

gctgcagaca ccgacggatt tgctttggga gccagagtag ctgccgccac cagagtccgg      240

agccatgagc gggtttaatt ttggaggcac tggggcccct acaggcgggt tcacgtttgg      300

cactgcaaag acggcaacaa ccacacctgc tacagggttt tctttctcca cctctggcac      360

tggagggttt aattttgggg ctcccttcca accagccaca agtacccctt ccaccggcct      420

gttctcactt gccacccaga ctccggccac acagacgaca ggcttcactt ttggaacagc      480

gactcttgct tcggggggaa ctggattttc tttggggatc ggtgcttcaa agctcaactt      540

gagcaacaca gctgccaccc cagccatggc aaaccccagc ggctttgggc tgggcagcag      600

caacctcact aatgccatat cgagcaccgt cacctccagc cagggcacag cacccaccgg 660

ctttgtgttt ggcccctcca ccacctctgt ggctccagct accacatctg gaggcttctc 720

attcactggt ggaagcacgg cccaaccctc cggtttcaac attggctcag cagggaattc 780

agcccagccc acggcacctg ccacgttgcc cttcactccg gccacgccag cagccaccac 840

agcaggtgcc acacagccag ctgctcccac acccacagcc accatcacca gcactgggcc 900

cagcctcttt gcgtcaatag caactgctcc aacctcatct gccaccactg gactctccct 960

ctgtacccct gtgaccacag cgggcgcccc cactgctggg acacagggct tcagcttaaa 1020

ggcacctgga gcagcttccg gcacctccac aacaacatcc accgctgcca ccgccaccgc 1080

caccaccacc agcagcagca gcaccaccgg ctttgccttg aatttaaaac cactggcgcc 1140

agccgggatc cccagcaata cagcagctgc cgtgaccgct ccacctggcc ctggcgcagc 1200

tgcaggggcg gctgccagct ccgccatgac ctacgcgcag ctggagagcc tgatcaacaa 1260

atggagcctg gagctagagg accaggagcg gcacttcctc cagcaggcca cccaggtcaa 1320

cgcctgggac cgcacgctga tcgagaatgg agaaaagatc accagcctgc accgcgaggt 1380

ggagaaggtg aagctggacc agaagaggct ggaccaggag ctcgacttca tcctgtccca 1440

gcagaaggag ctggaagacc tgctgagccc actggaggag ttggtcaagg agcagagcgg 1500

gaccatctac ctgcagcacg cggatgagga gcgtgagaaa acctacaagc tggctgagaa 1560

catcgatgca cagctcaagc gcatggccca ggatctcaag gacatcatcg agcacctgaa 1620

cacgtccggg gcccccgccg acaccagtga cccactgcag cagatctgca agatcctcaa 1680

tgcgcacatg gactcactgc agtggatcga ccagaactcg gccctgctgc agaggaaggt 1740

ggaggaggtg accaaggtgt gcgagggccg gcgcaaggag caggagcgca gcttccggat 1800

cacctttgac tgagcgacag cagccctggg gcccgcaggt ccctagggag ttcatgaggg 1860

gaatgcgccc tgttgtctgt agtttggggt tgtggcaaga tacttgtttg tttgtttctt 1920

tctttcacat gactgccctt gacatgatcg ctgtgtgctt tgcgtttttc catttaggag 1980

ggtattctgg gccttctgcc caggcagcag cctcatgggt gtggcttctg tggctttcat 2040

ttgagtatct ttggcccctt ttcacctact gcgaccaccc acctcatcct ggctcagcct 2100

ggtgatggag aagtgctgat ggtcttggtc ccagccaggg tcgtgggggc agccactctc 2160

tccaaagcat agtcataggt gtcatgaaaa aataccaaat gtaagagaac ctccaagtca 2220

gggcgcagtg gctcacccct gtaatctcag cactttgggt ggccaaggcg ggcagatgac 2280

ttgaggtcag gagttcgaga ccagcctggc caacatggtg aaaccccgtc tctactaaaa 2340

atacaaaaat tagtcaggtg tggtggacgc ctgtgatctc aatctcagct actcgggagg 2400

ctgaggcagg agaatcactt gaacccagga ggtgttgcag tgaaccaaga tcacaccact 2460

gcactccagc ctaggcaaca gagactctgt ctcaaaaaaa aaaaaaaaaa aaaagaaact 2520

cccaggagac agcagcctag ttttcgagtg tgagcttgtg cttgtgaaag ctaaccatgc 2580

taaccaccaa ggcaaagcag cacagtgtga atagaacaga gcgggatcaa gaatttcaca 2640

gaagacaggt cagctgaggg gcctgcacac acagggtgtt gaggaaccac agatgggcgc 2700

cgagaggcct gccttttgcc tggcccaggc tcacccccac cttgggcctc acctcctcca 2760

ggaagccttc ccagctaccc gaagctcagg tggccttctt gcaggtcccc gtagcaccct 2820

gagcctgtac cttgggtggc acttgttatg ctatcctgtg ctagccgttt gtgcctcgtc 2880

tcgctgttag attgtgagtt cccatgggca gagacccact gtcgttcccc gtgtgtcccc 2940

agcccggtcc ctgtcacatt tgttaaatga aagaacaatg aagcccagtg taacgtcagt 3000

ccacagaaat agccacagct tccagtggtg gccgtagact tggctcggaa cttagtggca 3060

ccagagtaac tctagtcagt tacagtaaaa tccactgtgt gtggaaggca gaagctagcg 3120

gttgtatccc aagcatcttt tgtatttgtc tttatacttt gctgaattct ctgaaatacc 3180

tattactgta tgttgctttt ctaaataaat gtattgtgaa accaaaaaaa aaaaaaaaaa 3240

a 3241

<210> 96
<211> 1537
<212> DNA
<213> Homo sapiens

<400> 96
gaggaagaag aatctgtata tctgtatata ttggctagca aatgtgccct gctctctccc 60

ctcttaaaaa tagcagcaac ccatctttgc aaagaagctt gcctatagag caggcactct 120

gtgaatggac tgtgctttta cgaccctaca gggtatcaag atactgtgca gctcgccaac 180

aaggattaat tgcaaggact ggtagatcga atttactgaa gacttggagc ttgcttctga 240

gaacaaacgc aaaaggacag taaactgtgg accttgaagt tagcagcgtg ggcttcctct 300

aatattacac cgtaaaaggc attgatcacc ataagaagga acatttgtga aggtactcca 360

gtgccagaaa gaggcacaaa gcagacattc gtagagaaac atggatgaaa caggaaatct 420

gacagtatct tctgccacat gccatgacac tattgatgac ttccgcaatc aagtgtattc 480

caccttgtac tctatgatct ctgttgtagg cttctttggc aatggctttg tgctctatgt 540

cctcataaaa acctatcaca agaagtcagc cttccaagta tacatgatta atttagcagt 600

agcagatcta ctttgtgtgt gcacactgcc tctccgtgtg gtctattatg ttcacaaagg 660

catttggctc tttggtgact tcttgtgccg cctcagcacc tatgctttgt atgtcaacct 720

ctattgtagc atcttcttta tgacagccat gagcttttcc cggtgcattg caattgtttt 780

tccagtccag aacattaatt tggttacaca gaaaaaagcc aggtttgtgt gtgtaggtat 840

ttggattttt gtgatttgga ccagttctcc atttctaatg gccaaaccac aaaaagatga 900

gaaaaataat accaagtgct ttgagccccc acaagacaat caaactaaaa atcatgtttt 960

ggtcttgcat tatgtgtcat tgtttgttgg ctttatcatc ccttttgtta ttataattgt 1020

ctgttacaca atgatcattt tgaccttact aaaaaaatca atgaaaaaaa atctgtcaag 1080

tcataaaaag gctataggaa tgatcatggt cgtgaccgct gcctttttag tcagtttcat 1140

gccatatcat attcaacgta ccattcacct tcatttttta cacaatgaaa ctaaaccctg 1200

tgattctgtc cttagaatgc agaagtccgt ggtcataacc ttgtctctgg ctgcatccaa 1260

ttgttgcttt gaccctctcc tatatttctt ttctgggggt aactttagga aaaggctgtc 1320

tacattcaga aagcattctt tgtccagcgt gacttatgta cccagaaaga aggcctcttt 1380

gccagaaaaa ggagaagaaa tatgtaaagt atagtttaaa cccatttcca gtccaaacca 1440

atgaaaatag tttcccaaat aagtatttgg tcaaatcatt tacaaaaaaa ataaaaattt 1500

tacttaatat tttaaaaaaa aaaaaaaaaa aaaaaaa 1537

<210> 97
<211> 2193
<212> DNA
<213> Homo sapiens

<400> 97
gtcttctggt tagggtcata ttcctactgc aagtggccct tactgagctg agagatgtca 60

ttgccacagg gaggacctat aggcacatgt aggttgaatg aaactctagt tccacttgga 120

agcccagaca agggatgggt cagtgagcaa ggctctcttc ctagtctcag gccatgcctg 180

tggcgcccta atcctactct caagatgttg gatctgggca gatgtgacaa attcacacaa 240

ctctgatttt gtctcaattt tgtagatctt gtagatttca tccttcactc taatttcagc 300

gtctgaaatc ctcgctacca tgaacaatct gagtatttga tgagacaggg ctgaatagtg 360

cagtttttct cctagcaacc atttgggggc aattgcttta aatcgattgg aaaaatatgg 420

cataaccatt tgcacaaact tgggacaaat gatattggga taatgatcta ccagaatagg 480

gaattttacc cacagtttct gggacaaaaa ccaaggaatc tctatggtga tcagccttca 540

ggcctcctga ggaatatctc tcacagtgtc ctattctcat gctgaggagc ctgaagtccc 600

tgtgtgagga ttagacagtg gattgttatg tgtgtttagg agaaccagct taatatgtct 660

gtccatgtct gaacttattg cagaaattga aaagtaccaa gaagtggaag aagaccaaga 720

cccatcatgc cccaggctta gcagggagct gctggatgag aaagagcctg aagtcttgca 780

ggactcactg gatagatgtt attcgactcc ttcaggttat cttgaactgc ctgacttagg 840

ccagccctac agcagtgctg tttactcatt ggaggaacag taccttggct tggctcttga 900

cgtggacagt gagtacctta ctgtgaaggt gataagcctc cacgtggtct tccagatagg 960

ggtgatattc ctgttccaag tgccccttac tgacccgaga gacgtcattg ccgcaggcag 1020

gacctatggg cgcatatagg ttgtaatgaa actgtagtct cagttggaag cctagacatg 1080

aaatgggtca gtgagcaagg ctctattcct agtctccagc catgcctgtg gcaacctgag 1140

cccgctctca gcacattgga cccaggcaga tgtaaaaaat tcacagaagt atgatttgga 1200

ctgaagggtt tgtagatttc ctccttcatt ctaatttcag tgtctaaaat tcttgcatcc 1260

atgaacgagc tgggcatttg atgagacagg gctgaatact ttagttttcc tcctggaaat 1320

catctggggc attttctttg aactgatggg aacaataaag cataactgtt tgcacaaact 1380

tgggataaat gattttggga taacgatgta ccagaatggg gatatttcac ccttggttct 1440

gagatgtaaa ccaaagaata tcatgaccag ctttcaggcc tcctgaagta tatctctcac 1500

attgtcctgt tctcatgctg aggagcctga gatccctgtg tggggattag acagtggact 1560

gttatgggtg taggtgaatt ggcttatttt gtctgtccct gtctgaatgt attgcaggaa 1620

ttaaaaagga ccaagaagag gaagaagacc aaggcccacc atgccccagg taactgagca 1680

attgtgaaca gctacttctg tgttgacatc tggagactcc tggttcaggg aaaacagtgc 1740

gggctgacat tatcgattac atcttttcaa ccaagcctga attattccta ctaacattgc 1800

tgttggtttt cattgcagta gatatttagg tttccatttc ttcctcccct tatcatttac 1860

taacctactg taggtggacc agacttcaaa aactgtattc tcatggcgac tgcatggaaa 1920

cttgagcaca ttttatggaa aattattgag cacagtcttt tcatgatccc tgtatgctgt 1980

gtgtcctgag ggcactaact cagagtgtcc tgttactccc tcatcagtgt gtcacctgga 2040

caattcactg agctcgttct ctctctctct ctctctgtgt gtgtgtgtgt gtgtgtgtgt 2100

gtgtgtgtgt gtgtgtgtgt ctatctgtct ttctctttca ttcttttcca tttggccctg 2160

ttctgtccca acatgaaggc aataatttgt tac 2193

<210> 98
<211> 1853
<212> DNA
<213> Homo sapiens

<400> 98
atcgcgcgag acagcggaag gagcaagagt gggaggcgcg cgcggaggcc gcgacggacg 60

caagatggcg acggcgacca tagctctcca ggtcaatggc cagcaaggag gggggtccga 120

gccggcggcg gcggcggcag tggtggcagc gggagacaaa tggaaacctc cacagggcac 180

agactccatc aagatggaga acgggcagag cacagccgcc aagctggggc tgcctcccct 240

gacgcccgag cagcaggagg cccttcagaa ggccaagaag tacgccatgg agcagagcat 300

caagagtgtg ctggtgaagc agaccatcgc gcaccagcag cagcagctca ccaacctgca 360

gatggcggct cagcggcagc gggcgctggc catcatgtgc cgcgtctacg tgggctctat 420

ctactatgag ctgggggagg acaccatccg ccaggccttt gccccctttg gccccatcaa 480

gagcatcgac atgtcctggg actccgtcac catgaagcac aagggctttg ccttcgtgga 540

gtatgaggtc cccgaagctg cacagctggc cttggagcag atgaactcgg tgatgctggg 600

gggcaggaac atcaaggtgg gcagacccag caacataggg caggcccagc ccatcataga 660

ccagttggct gaggaggcac gggccttcaa ccgcatctac gtggcctctg tgcaccagga 720

cctctcagac gatgacatca agagcgtgtt tgaggccttt ggcaagatca agtcctgcac 780

actggcccgg gaccccacaa ctggcaagca caagggctac ggcttcattg agtacgagaa 840

ggcccagtcg tcccaagatg ctgtgtcttc catgaacctc tttgacctgg gtggccagta 900

cttgcgggtg ggcaaggctg tcacaccgcc catgccccta ctcacaccag ccacgcctgg 960

aggcctccca cctgccgctg ctgtggcagc tgctgcagcc actgccaaga tcacagctca 1020

ggaagcagtg gccggagcag cggtgctggg taccctgggc acacctggac tggtgtcccc 1080

agcactgacc ctggcccagc ccctgggcac tttgccccag gctgtcatgg ctgcccaggc 1140

acctggagtc atcacaggtg tgaccccagc ccgtcctcct atcccggtca ccatcccctc 1200

ggtgggagtg gtgaacccca tcctggccag ccctccaacg ctgggtctcc tggagcccaa 1260

gaaggagaag gaagaagagg agctgtttcc cgagtcagag cggccagaga tgctgagcga 1320

gcaggagcac atgagcatct cgggcagtag cgcccgacac atggtgatgc agaagctgct 1380

ccgcaagcag gagtctacag tgatggttct gcgcaacatg gtggacccca aggacatcga 1440

tgatgacctg gaaggggagg tgacagagga gtgtggcaag ttcggggccg tgaaccgcgt 1500

catcatctac caagagaaac aaggcgagga ggaggatgca gaaatcattg tcaagatctt 1560

tgtggagttt tccatagcct ctgagactca taaggccatc caggccctca atggccgctg 1620

gtttgctggc cgcaaggtgg tggctgaagt gtacgaccag gagcgttttg ataacagtga 1680

cctctctgcg tgacagtggt ccctctcccc ggacttgcac ttgttccttg tttcctctgg 1740

gttttatagt gatacagtgg tgtccccggg gccaggcgcg ctctgcccag cccagcctac 1800

agtgcggata aaggtgcgga tgctgctggc cctgaaaaaa aaaaaaaaaa aaa 1853

<210> 99
<211> 3767
<212> DNA
<213> Homo sapiens

<400> 99
aagaaagagc cccgccccta gtcttatgac tcgcactgaa gcgccgattc ctggcttttg 60

caaggctgtg gtcggtggtc atcagtgctc ttgacccagg tccagcgagc cttttccctg 120

gtgttgcagc tgttgttgta ccgccgccgt cgccgccgtc gccgcctgct ctgcggggtc 180

atggtgtgct tccgcctctt cccggttccg ggctcagggc tcgttctggt ctgcctagtc 240

ctgggagctg tgcggtctta tgcattggaa cttaatttga cagattcaga aaatgccact 300

tgcctttatg caaaatggca gatgaatttc acagtacgct atgaaactac aaataaaact 360

tataaaactg taaccatttc agaccatggc actgtgacat ataatggaag catttgtggg 420

gatgatcaga atggtcccaa aatagcagtg cagttcggac ctggcttttc ctggattgcg 480

aattttacca aggcagcatc tacttattca attgacagcg tctcattttc ctacaacact 540

ggtgataaca caacatttcc tgatgctgaa gataaaggaa ttcttactgt tgatgaactt 600

ttggccatca gaattccatt gaatgacctt tttagatgca atagtttatc aactttggaa 660

aagaatgatg ttgtccaaca ctactgggat gttcttgtac aagcttttgt ccaaaatggc 720

acagtgagca caaatgagtt cctgtgtgat aaagacaaaa cttcaacagt ggcacccacc 780

atacacacca ctgtgccatc tcctactaca acacctactc caaaggaaaa accagaagct 840

ggaacctatt cagttaataa tggcaatgat acttgtctgc tggctaccat ggggctgcag 900

ctgaacatca ctcaggataa ggttgcttca gttattaaca tcaaccccaa tacaactcac 960

tccacaggca gctgccgttc tcacactgct ctacttagac tcaatagcag caccattaag 1020

tatctagact ttgtctttgc tgtgaaaaat gaaaaccgat tttatctgaa ggaagtgaac 1080

atcagcatgt atttggttaa tggctccgtt ttcagcattg caaataacaa tctcagctac 1140

tgggatgccc ccctgggaag ttcttatatg tgcaacaaag agcagactgt ttcagtgtct 1200

ggagcatttc agataaatac ctttgatcta agggttcagc ctttcaatgt gacacaagga 1260

aagtattcta cagctgaaga atgttctgct gactctgacc tcaactttct tattcctgtt 1320

gcagtgggtg tggccttggg cttccttata attgttgtct ttatctctta tatgattgga 1380

agaaggaaaa gtcgtactgg ttatcagtct gtgtaatcag ttaaatctag tgtttgtttg 1440

tttttttcaa ttagaagtta cgtttccatt ggctaaaagc caggacatgc tgtgcaatag 1500

attgtttaag atatgcagac taacttcagt gagttcctag ctaacttggg catgagtaca 1560

cttatttaag acaaaatata ttaggaccaa tttttttctg ttttttttct tcctttgtta 1620

aagtataatt aaaagaaaaa ttgtggctta gaatttttta agtaaataat gattttaagc 1680

ccctggatcc aattatgaaa gcatttttgc tgatgtgtaa ttttatatgt tacagttact 1740

tatattttac tactttgatg ttatttgcaa aatcaaaggt gttaaagaat ttaacttgct 1800

tcaggaaata aattcaagaa catagtggat tcattttcat tggtggcaga cacgaaattt 1860

ggttcatgat aagacttcct ttccccacct cctgatcagc attatttaaa tctgtatttt 1920

tctgttagtt aagaaagaaa tggcttcatg atattgtatt taatagcaaa agtttggctg 1980

tcttcttcat tactgttaat agctactata ttttaacaag gagatttctt tttttgttgt 2040

tgttgttcta gagtttggaa tatactgatt atctcagact tgacatttat actgaaggat 2100

gaagtaagac ctccagcttt ttttaaaaaa ggtgttgatt tggaacacct gtatgggtta 2160

tggtttatta aggttatggt ttagaaagtt tttttccctc agagccttaa cttgttaaga 2220

aggttcattt atcctgcact gaaaacaaaa actctatata ctttgtttgt gtgcctcctg 2280

cactctccca ttccctatgt gaatatgctc tagttgatat ttttaatata ttgatttctt 2340

ttttctcaca gcaacaagtg cttactctag aggttagtgg gccctgatat gtcatcagtc 2400

agatgcctgc ctagccaaag ctggactaag attattctgt acatttgttg atcttgatat 2460

agacttatat ccctgtaggg actgctaatg gctccggctt ctggagtaag gtactggaga 2520

ccactcatcc ctgtgtctgc ttgattggtt cagctgttga attgcccttt tatttggaag 2580

cagtgttgaa gttgtctagg gttcaaatgg ctgctttgta cacctgtcat tagtataagg 2640

cagatgttta ttttatcaag ctattttatc tctacattta actaaaaaca aaagttccca 2700

aagatctgcc ttcacttcag aaattttttt tggattaaaa aaattaagcc tgaaccttaa 2760

ataaagtgag ttggttattc attccaagga ttaagtccca atctacctct cagcacaatg 2820

cagaagctca ccactgtatt gctgccatta actcatgcca gaaccctttg ccaataactg 2880

gaattacaaa tttttgttaa agaaaattta tcaagatctt tctttactgc cttctctata 2940

tgtacatctc aaaaacatgt acatctcaaa aactggagta gaaagttaga ttgctcaact 3000

acaactcctc tagaactcta tagctctgac atacagattc acactctcct ctatttgcta 3060

agtatgtaaa gaatgttttc ttttaaaatg ttctcttttg agaacaactg cttatttgtt 3120

ataaaagcat ttggttaaaa tgatgtcatc ataaaaaaca gtggctttgt ttcaatacat 3180

atttttgaga tgattatcta gaagccagat taataaaatc agcttgtgac cttgctaagc 3240

atataaactg gaaattcaga tacattcaaa attatgggtt catttaaaag tgttctacct 3300

tttgggtatg agactaatat cactaattcc tcaatagtta tcatggctct atcttaatta 3360

attagaaaat atgtgtgttt aattctttga gaattaaaat agagaatatt aacagagggt 3420

taaaaactgc ttcaactcca ataagataaa ggaagctcaa aatctatgag ctgagtgttc 3480

aattagcttt gcctactgag ttcaatttta tgtcaataca acagtggatc agacagtacg 3540

actttgaact ggtgaatgta aacaattgtt tttcacctaa gctgctttgg aagaactgat 3600

gcttgctgct aactaaagtt ttggatgtat cgatttagag aaccaattaa tacctgcaaa 3660

ataaagcata ctgtggtact tctgtttgat ctagtatgtg tgattttaga ttgatggatt 3720

aaaaattaat aaagatcata cattccatac caaaaaaaaa aaaaaaa 3767

<210> 100
<211> 4256
<212> DNA
<213> Homo sapiens

<400> 100
gccgcggcgg tggggttggc agggtgtgct ggggcctgga ggaggcgccg cgcggccagg 60

gagccagcgg gaggccgcgc ctggcaggtt tctgaaacag atcgtgagct tcatcaagag 120

aattcaactg gaaaaccaag actggtagac tctctttttc ttcagacaat aggcaggagc 180

caggcagagt ccagggattc ttggaacacc tatcttttct tcggaggaca ctaagttcta 240

tttgaagaca aagttcaata tggcaacagg actgatggga cacgaaggag tcgctaccgt 300

gatttggtga cagttcttca aaacgacagt gtctcaagga aaggtggacc taggaactcc 360

tgaacttttg ggttgcctta agtgagaaat cagcatggct caggcaagtc tcctggcttg 420

tgaaggccta gcaggtgtga gtttggttcc cactgcagcc agcaagaaga tgatgctgag 480

ccagattgcc agcaagcagg ccgagaatgg cgagcgggca ggtagccctg atgtgctgag 540

gtgctcgagt cagggtcacc gaaaagacag tgataagtcc cggagccgca aagatgatga 600

cagcttgtct gaggcctctc attcaaaaaa gactgttaaa aaggtggtgg tagtggaaca 660

aaatggttct tttcaagtaa agattcccaa aaattttgtt tgtgaacact gctttggagc 720

ctttcggagc agttaccacc taaagaggca catccttatt cacactggtg agaagccatt 780

tgaatgcgat atatgtgata tgcgtttcat ccagaagtac cacctggaac gccacaagcg 840

tgtgcacagt ggtgaaaagc cttaccagtg tgaacggtgt catcagtgtt tttctcggac 900

agatcgatta ctcagacaca aacggatgtg ccaagggtgc cagtccaaga cttccgacgg 960

gcagttttct ctataggcgc aaggggcccc gggtggtggg agtgatcaga agaacctgcc 1020
gaagagcaca ccccctctgg tctgatggtc ccaccaccgc cccatgtgaa cctgtcccac 1080
tcctggagga gtggacccag gagaccagaa gagtgcatca ggggacagtg gccccagaac 1140
ctcagctctg taaataatct gagactatga gtatctcggg gaagttctta cagcattcct 1200
gggtagggga gctagtccct ggacccttgg ctgaggtcat aggtggggac tcaaggtaca 1260
ggaccaagac tgaagtgtgg ctcccacaac cttggactcc agctggcagc tgaaatggaa 1320
aagattgggg aaggggattt gtttgttctg ttcttgtttt tgtttatcca aaagcaactt 1380
cagcgggtaa actgtggata caggtaatcc agaggcataa gagtcctggt ccaaagtagg 1440
gactatagct ggtccagctc tccccaaaaa ttgggttttt agttgcagta gctcctcagt 1500
gtttcacaat cctgcacttc gcctcctttt tgaggaagaa taaggtcagg aatggcagct 1560
gagcttactt tggcttcctt atacactgta gggacaaaag gaaaaacggg gtggagcaga 1620
caagggctgg atccaaaaag tggatggcac ctcttccttc ctcaagggaa aaggaagagt 1680
tgtgccagtg ggtgttgtca tggcccatgt cacattggca cctgatggca tcagctcaaa 1740
tgcctttagg aggaaacttg ggtgagagtg gcccatgaag cccttttctt ctttttttgg 1800
ggagtcggtc ttggagatgg tatgtggcta cctcgctgcc attgactcct aaagtgagac 1860
agatgtgttg agcaaggagt catggaagtc aaagtgggtt ctctctcctt gtgccctctt 1920
aggaacaacc caagactggt cccaagttgg ctaacagctg ctcaagaaac aaaggaatct 1980
ggggtcttct gacatgcctg ttgagcagta ggagctgttc tccctcccca agtcctatca 2040
ctgtattcag gtagaggagg tagggagggg tctgtccttt ggtcccaaaa aacaagatct 2100
cggcttgctt cctgatggcc atccctaccc aacagagtgt gggggatggg ggtgggggtg 2160
ctgcagagaa aagactaaag aagaaaccta atgtaggttc cattcttaac catattcttt 2220
gctcctctct gctccaatac caccaccttg tccactccca aaaaagtaat cgggtgtgtg 2280
tgtgtgtgtg agagagagtg tgtgtgagat atgaatgcat gtatttgtat gcttgcagga 2340
gatggcatat tattgagcca aacccttctc tggccctcac cttcagggag gatgagtgga 2400
aaatagcctc ctcttgccca aacatgaagg gtgcttgagg tctcagcagg agctgaagaa 2460
cacaatttgg ggaacagaag ggcttttgtt ttggagggtt ttttttttta agatgaattc 2520
aacagcctgg tggttaggat gtttctaacc caagccaggg cttgtgaaca tgaattttct 2580
aaggtgaaat aaaccttttc tttctgctaa agaaagaatc cttatgggaa gttttggggt 2640
ttggttgacc cccactgtaa cctgacctga cctgtggcgg gtacgggaac tgtcttagta 2700
atcatggaga ctggctgaac ctgagtgcca agctgtcagt agggtctgag atgggaaact 2760

cctgaaagta tgatttcatt tcccagaaaa tgtaaagaat ggataccagc aatgattcct 2820

ggctgttacc tattctggag gggtggggtt ggggaggggg ttgtccatac ctctgtggta 2880

tgagtatttc agggaaaaag aaagcaggca tggcacccat tcgattttcc ctgacagcat 2940

ctgagatcct tttggggaga cgctgaggag tgtttgctgc catgtactct tacagctcta 3000

tgctgacact cccatttgat gtggtccaga acttagacct cagtccttgg atcagccctg 3060

tggtccctgc aagcaggggc atctttctgc atgtgagcca gcccccttcc tgttcaaggg 3120

ttctgctgga tctgggcttt tcctgtcctt cacttctggg atgattcacc ccacatcttc 3180

cagtaccctg taaaccattt taaaatattt agaaaactat cctcccaaaa atgcttttga 3240

aaatgagagc cctctgtccc tgccacttac agctagtctc tttgggatag gggtgtatgt 3300

ggagagattc atgtaagtct cacatgagtg acctgtgccc ctatgtgtac taatgtgtgt 3360

actgggtcag aaggtgccct gggttcccac agaccttggt ttcctgcctg ggtgggtggg 3420

agggagggaa cttcagagag tatgtgcgaa cagccatagc agagccaccc ctgccaagga 3480

cccagtgagg gaaactcagg agcaggtggt tggttcagtt cccgcgctga tgtctggggc 3540

agtgcatgtg tgagaagaga ggcgcaaagc agtgaaagga gtgatttggc atccagaggg 3600

cccgggagcc ccacggatac cccagggaag ccactgactc acaccgctgc agaggctcct 3660

gggcccaatg cccgacccct gctggccagc atggggccta gcactggaag atgcctcagg 3720

tgtgtttgga gtgggggaaa cggaagtagg gcacgctctc agagaccagg atagagagtc 3780

agggaacctg ggaaagagta gtgccagttc cgggtaggct gctgcaccag gccctaccag 3840

ctcaggctat aaacagtctg ctttgcccca agtcttttcc gtccaccccc aacagccttg 3900

tcatcactta gctactgatc acgcccatgg cttgacattg gagggttaca ttagtggagt 3960

ccgccacagc ttcgaaccct ctccccagat gccctttgcc tctgctgtgg ccctggggtt 4020

ttatcaactg tccccttcct atcctcctct ccaccctgtt ttttgttttt ttttttttta 4080

aataatattt tgctatgggg gaggggaata tcaaatggac aagatcactt ttaaatggta 4140

gtttttataa gatgttataa acttgtatct ttttaccatt aaagtgcagt gtatgttcct 4200

tcgtgatata tttaggatat ttaaataaaa ggaaaatggg gcttttctac atacta 4256

<210> 101
<211> 5732
<212> DNA
<213> Homo sapiens

<400> 101
   ttgcgggaaa gagccaaacc ctggcgttgg ggggcccggg cggggagccc ctcccgcggt 60
   ccacagcgac gcctgcccag ccctcctccc cttccggctc cggcacgggg ccccgaggcg 120
   ttcggaggcc aggcgggttt ctgtcaggcc cggggaggag gggcgggcgg ggcggccgct 180
   gcctccccgg gacgggccgt accacgcgga cggggaggac ggggccaggg gactgcaggg 240
   cggctgcacc gcccgggggc ggggtgcgga gcgggccggc gggctccccg gggcggggcg 300
   ggagggcggg gcgtggggcg gacggaacca ccggggcggg gtgggaggta acgggacggg 360
   cgcgaccatg gcgcggtgag ggagcggggg tggggatcgg tccgggggag gcctgaggcc 420
   gctggcttgt gcgctgtctc cgccgccccc ctctttcgcc gccgccgccg ccgccccggg 480
   catgtcgtcc aactgcacca gcaccacggc ggtggcggtg gcgccgctca gcgccagcaa 540
   gaccaagacc aagaagaagc atttcgtgtg ccagaaagtg aagctattcc gggccagcga 600
   gccgatcctc agcgtcctga tgtggggggt gaaccacacg atcaatgagc tgagcaatgt 660
   tcctgttcct gtcatgctaa tgccagatga cttcaaagcc tacagcaaga tcaaggtgga 720
   caatcatctc ttcaataagg agaacctgcc cagccgcttt aagtttaagg agtattgccc 780
   catggtgttc cgaaaccttc gggagaggtt tggaattgat gatcaggatt accagaattc 840
   agtgacgcgc agcgccccca tcaacagtga cagccagggt cggtgtggca cgcgtttcct 900
   caccacctac gaccggcgct ttgtcatcaa gactgtgtcc agcgaggacg tggcggagat 960
   gcacaacatc ttaaagaaat accaccagtt tatagtggag tgtcatggca acacgctttt 1020
   gccacagttc ctgggcatgt accgcctgac cgtggatggt gtggaaacct acatggtggt 1080
   taccaggaac gtgttcagcc atcggctcac tgtgcatcgc aagtatgacc tcaagggttc 1140
   tacggttgcc agagaagcga gcgacaagga gaaggccaag gacttgccaa cattcaaaga 1200
   caatgacttc ctcaatgaag ggcagaagct gcatgtggga gaggagagta aaaagaactt 1260
   cctggagaaa ctgaagcggg acgttgagtt cttggcacag ctgaagatca tggactacag 1320
   cctgctggtg ggcatccacg acgtggaccg ggcagagcag gaggagatgg aggtggagga 1380
   gcgggcagag gacgaggagt gtgagaatga tggggtgggt ggcaacctac tctgctccta 1440
   tggcacacct ccggacagcc ctggcaacct cctcagcttt cctcggttct ttggtcctgg 1500
   ggaattcgac ccctctgttg acgtctatgc catgaaaagc catgaaagtt cccccaagaa 1560
   ggaggtgtat ttcatggcca tcattgatat cctcacgcca tacgatacaa agaagaaagc 1620
   tgcacatgct gccaaaacgg tgaaacacgg ggcaggggcc gagatctcga ctgtgaaccc 1680
   tgagcagtac tccaaacgct tcaacgagtt tatgtccaac atcctgacgt agttctcttc 1740
   taccttcagc cagagccaga gagctggata tggggtcggg gatcgggagt tagggagaag 1800
   ggtgtatttg ggctagatgg gagggtggga gcagagtcgg gtttgggagg gctttagcaa 1860
   tgagactgca gcctgtgaca ccgaaagaga ctttagctga agaggagggg gatgtgctgt 1920
   gtgtgcacct gctcacagga tgtaacccca ccttctgctt acccttgatt ttttctcccc 1980
   atttgacacc caggttaaaa aggggttccc tttttggtac cttgtaacct tttaagatac 2040
   cttggggcta gagatgactt cgtgggttta tttgggtttt gtttctgaaa tttcattgct 2100
   ccaggtttgc tatttataat catatttcat cagcctaccc accctcccca tctttgctga 2160
   gctctcagtt cccttcaatt aaagagatac ccggtagacc cagcacaagg gtccttccag 2220
   aaccaagtgc tatggatgcc agattggaga ggtcagacac ctcgccctgc tgcatttgct 2280
   cttgtctgga ttaactttgt aatttatgga gtattgtgca caacttcctc cacctttccc 2340
   ttggattcaa gtgaaaactg ttgcattatt cctccatcct gtctggaata caccaggtca 2400
   acaccagaga tctcagatca gaatcagaga tctcagaggg gaataagttc atcctcatgg 2460
   gatggtgagg ggcaggaaag cggctgggct cttggacacc tggttctcag agaaccctgt 2520
   gatgatcacc caagccccag gctgtcttag cccctggagt tcagaagtcc tctctgtaaa 2580
   gcctgcctcc cactaggtca agaggaacta gagtaccttt ggatttatca ggaccctcat 2640
   gtttaaatgg ttatttccct ttgggaaaac ttcagaaact gatgtatcaa atgaggccct 2700
   gtgccctcga tctatttcct tcttccttct gacctcctcc caggcactct tacttctagc 2760
   cgaactctta gctctgggca gatctccaag cgcctggagt gctttttagc agagacacct 2820
   cgttaagctc cgggatgacc ttgtaggaga tctgtctccc tgtgcctgga gagttacagc 2880
   cagcaaggtg cccccatctt agagtgtggt gtccaaacgt gaggtggctt cctagttaca 2940
   tgaggatgtg atccaggaaa tccagtttgg aggcttgatg tgggttttga cctggcctca 3000
   gccttggggc tgtttttcct tgttgccccg ctctagactt ttagcagatc tgcagcccac 3060
   aggctttttt ggaaggagtg gcttcctgca ggtgttccac ctgccttcgg agcctgccac 3120
   ccaggccctc agaactgagc cacaggctgc tctggccagg agagaaacag ctctgttgtt 3180
   ctgcattggg ggaggtacat tcctgcatct tctcaccccc tcaaccagga actggggatt 3240
   tgggatgaga tatggtcaga cttgtagata accccaaaga tgtgaagatc gcttgtgaaa 3300
   ccattttgaa tgaatagatt ggtttcctgt ggctccctcc aaacctggcc aagcccagct 3360
   tccgaagcag gaaccagcac tgtctctgtg cctgactcac agcatatagg tcaggaaaga 3420
   atggagacgg cattcttgga cttcactggg gctgctggat tggatgggaa accttctgga 3480
   agaggcagat gggggtcaaa ccactgcctt ggccccagga aggggccata ggtaggtctg 3540
   aacaactgcc gcaagaccac tacatgactt agggaacttg aaaccaactg gctcatggag 3600
   aaaacaaatt tgacttggga aagggattat gtaggaataa tgtttggact tgatttcccc 3660
   acgtcataat gaagaatgga agtttggatc tgctcctcgt caggcgcagc atctctgaag 3720
   cttggaaagc tgtcttccag cagcctccgt ggcctcgggt tcctaccggc ttctctgcat 3780
   ttggtctgct gatcatgttg ccataatgtg tatggaaagt gtaacacatt cttactggtt 3840
   aaagacgact accaggtatc taacttgttt aacattgagt ttgtgtgtgt gtgtgtatgt 3900
   ttgtgtgttt tgtatattgt ttacattttg agaggtagca ttctgtttca aatgcttttt 3960
   gtttttctga cagtattgtt gactgggtca taacattttg agctgtggtt tggtggattt 4020
   tcaatttttt tttttaaagg tcattcgctg tgctatcttc aaaaccttga gtttggcccc 4080
   caatttttgg cattcaaatg tttaaaagct atttatcttg gtttatacaa gtttcctttc 4140
   tcttcttttt gtcatggtat tctatttggt ctgcagtttg aatgtagaga aagtggactg 4200
   atcccccaag cgttgtctgc ccccactctt tcctccttgg gtcccgccat tcttttactg 4260
   ggcagtcgag ggcattggag gggaagtgac tgccctcagc ctcactccct ggggccatga 4320
   agaaaagcta aacagtctca tggcatctca gaataatgtt gggtctccca agaagaaagg 4380
   tgtaagaata acgacatggc tgattaggcg aggccaggat agggctaagg ccaggattcc 4440
   tggctggcat ccagtcaccc cttctcccat ccttccccct cttcttccac aagtccgcag 4500
   ccgagacact gtagtctccc agccacagtg atgagtgccc tggagactcc actgacctct 4560
   agatgaaggc ccctggccct ggttcctgtt aattaacctc tgggtctttg agtcccccag 4620
   cacaaacttc tttcctgtac cctgcggctt ggggtcacag ggcatgccgg gaagccacag 4680
   ctgaggggcg cagactgaag cagtgctcca cctctccttc tttagctcag gggttgctgg 4740
   tctgtggcag gcgccacgag tggcccctgt ggctgttctc agtggcagtc tcttaagttc 4800
   ccaccacagg cagctcttta tcccctctcc ctacttgact ctttctcttg cctgtgcttt 4860
   tggcctcaaa caggcctgct ggtagcgctc agggcgtgag gctacactcc tgccctgcct 4920
   ttcctgtctt catggtctgc cagggcatac cttggggagg tggaccaaag acccaggact 4980
   ttttgcagta gccagtccta ccccccagtt gtctttttac caattcaggg tgggagagaa 5040
   aactgcagca ccccagcatg tgagttactc aggtgttggg ggctagaagg gacagtgcgt 5100
   ttaaacaaca ctcagagctc tggccttaaa cctgtggccc cccaagtcta ggagcctcat 5160
   ctcttcctgg cagtcatgcg ggcaggaggt cctgaaaggg aaaacccatt cagacaactg 5220
   ttccccaatc taccagccat ctgcaggggt cagtgaccgt ggccctctcc ctcctctaga 5280
   atgtgccact tatgaagagt gccccatggg gaaaaggaga ctcagctgtc ccttggcagc 5340
   ttgtgccagt atcccagggc agaagtttcc acaggagcct cttgcccttg cgcagagcca 5400
   ctgtgagagg cggtgggagc caacaccctt gggggagggg gcagtactgc tcggcacatc 5460
   ccagcatcag gtcagatcat tgaaattaaa aaatgtgaat taagttcata tccacctttt 5520
   ggggaagcag gacaaaccac caccccacca agtgtgtgac ttctccatat cccactgcag 5580
   tttccatttt ttaaatggga attttcaatc ccctgtgctt gtctaacgtc tgctttaaaa 5640
   agtttgagac cctgttactg tttgaaaatg catgcatgtt acgatgaatc tccaacctga 5700
   ggaaaaaaat aaaactcaaa aagctttgtg ta 5732

<210> 102
<211> 1982
<212> DNA
<213> Homo sapiens

<400> 102
aatcccagcg ccgctgtcac tgttatggtc ctgtcagggt gccggcgtcg tggtgcttgg 60

gtggtcgcca ccaagaagac tttggtgggg tagtctcggg gcagctcagc ggcccgctgt 120

gcccgtttct ggcctcgctc gcagcttgca cgtcgagact cgtaggccgc accgtagggc 180

gagcgtgcgg gtcgccgccg cggccgcctc ggggtctggg cccagccgca gcctcttcta 240

ccgcggccgg ttgggagtcg ccgcgagatg cagcctccgg gcccgccccc ggcctatgcc 300

cccactaacg gggacttcac ctttgtctcc tcagcagacg cggaagatct cagtggttca 360

atagcatccc cagatgtcaa attaaatctt ggtggagatt ttatcaaaga atctacagct 420

actacatttc tgagacaaag aggttatggc tggcttctgg aagttgaaga tgatgatcct 480

gaagataaca agccactctt ggaagaattg gacattgatc taaaggatat ttactacaaa 540

atccgatgtg ttttgatgcc aatgccatca cttggtttta atagacaagt ggtgagagac 600

aatcctgact tttggggtcc tctggctgtt gttcttttct tttccatgat atcattatat 660

ggacagttta gggtggtctc atggattata accatttgga tatttggttc actaacaatt 720

ttcttactgg ccagagttct tggtggagaa gttgcatatg gccaagtcct tggagttata 780

ggatattcat tacttcctct cattgtaata gcccctgtac ttttggtggt tggatcattt 840

gaagtggtgt ctacacttat aaaactgttt ggtgtgtttt gggctgccta cagtgctgct 900

tcattgttag tgggtgaaga attcaagacc aaaaagcctc ttctgattta tccaatcttt 960

ttattataca tttatttttt gtcgttatat actggtgtgt gatccaagtt atacatgaat 1020

agaaaaagat ggtgttaaat ttgtgtgtag gctgggaatt cttgctgaag gaattggaga 1080

aaacctgttg ctgcaaaatt ttacatgttc cagatggaaa gggaagtcta agcgcttttt 1140

aaaacaattt ttttttgtat ttaattaagc aattgcagtt atctgggatt tttgggtcag 1200

aattttaaat tctgtttgat tctccatatt ccagtgaata aaatacaaaa gcattgtgtt 1260

tttaagattg tgtcgatatt cacctaaaaa cttgtgccaa aagcacctgg attggtaatt 1320

atatttcact taaagggtaa atttgacaat atcttgataa tcaaaagtgc aatttttttc 1380

ttcaaaatgt tttctccagc atcacagatc ctgcagatat atatttatat ttatacatat 1440

atatttatga aataattctt actcacaaaa tatatttctg ataaacatta agatattaaa 1500

tctgatgcac aaacttttta atttggccat taatcttttt tatttaaaaa tttaaatttg 1560

tttttaaaat tgtatatagt ttttaaaatc tcacacatgc ttcgatactt ccttgttaag 1620

aattcttaat aactactaaa actgattttt aatagttgct gatatatatt tggtttgttt 1680

gggtatactt ttcaaaacca tttttgaatg tccaaacatc tgatttaaag tttctgttta 1740

tctttctgac caaaggagca agaggtataa tggatatggc attcattaaa atctttacta 1800

tgtacaaaaa cagtaatatt tacagcatca gtaaatattt ttaagtggta cttctaaatc 1860

ataaaagttg gggaaagaga cctttaaaat cttgtggtgt tgaacaatgt tatatgaagt 1920

agaaaaaata aaatacttcc cagttgtgtg ttttgtttta cagcaaaaaa aaaaaaaaaa 1980

aa 1982

<210> 103
<211> 5877
<212> DNA
<213> Homo sapiens

<400> 103
agagcagcct gggagatgta gtcttcggac cgtctccact agaggctgca cagcccgccc 60

ggaccaacgc ctccgcgcct gccgccgtct ggccggcgcc ctcccgtttc cttcctccag 120

ggggaaaaaa tgtctttcgg gccgctttcc aggaagcagc aggatgcgcc gctccactgt 180

cggtagcaac aagctgcgca aaccatgcag gatgatttac tgatggacaa aagcaaaacc 240

cagccccagc cccagcagca gcagcggcag cagcagcagc cccaacctga gtccagcgta 300

tccgaagccc cgtccacgcc cctctcctca gagaccccca agccggagga aaacagcgca 360

gtgccggccc tcagcccagc cgctgccccc ccggccccca acggcccgga caagatgcag 420

atggaatcac cgctcctgcc aggcttgagt ttccatcagc ctcctcagca gccgccgccg 480

cctcaggagc ccgcggcacc gggcgcgtcg ctgtcgccgt ccttcggcag cacctggtcc 540

acgggcacca ccaacgcggt agaggacagc ttcttccagg ggatcacccc agtcaacggg 600

accatgctct tccagaactt cccgcaccat gtcaacccag tcttcggagg cactttctcc 660

ccgcagatcg gcctggcgca gacccagcac caccagcagc cgccgccgcc tgcgcccgcg 720

ccgcagccgg cacagccagc gcagccacca caggcgcagc ccccgcagca gcgccgctca 780

cccgccagcc ccagccaggc gccctacgcg cagaggagcg ccgccgcggc gtacggccac 840

cagcccatca tgaccagcaa gccgtcctcg tcttcggcgg ttgcagccgc tgctgccgca 900

gccgccgcct cgtcggcctc gtccagctgg aacacgcacc aaagcgtgaa tgcagcctgg 960

agcgcaccgt ccaacccctg gggcggcctg caggcgggcc gggaccctcg ccgggcggtc 1020

ggtgtgggcg tgggtgtggg tgtcggggtg ccttccccgc tcaaccccat ctcgccgctc 1080

aaaaagccct tctccagcaa cgtgatcgcg ccgcccaagt tccctcgcgc ggcccctctc 1140

acttccaagt cctggatgga ggataacgct ttccggaccg ataatggtaa caatctgttg 1200

ccatttcagg accggagtag gccctatgat acttttaact tgcactcgtt ggagaactcc 1260

ttaatggata tgataaggac tgatcatgaa cctctgaaag gtaaacacta ccctcccagt 1320

ggcccaccaa tgagtttcgc tgatataatg tggaggaatc attttgcagg acgcatgggg 1380

ataaatttcc atcatccagg aacagataat attatggcac ttaacaatgc cttcctggat 1440

gatagccatg gtgatcaagc cttgtcatct ggcttaagtt ctcccactcg ctgtcaaaat 1500

ggggaacgag tagaacgcta ctctagaaag gtgtttgttg gaggacttcc tcctgatatt 1560

gatgaagatg agatcactgc cagctttcgc aggtttggac ctctcgtagt agactggcct 1620

cacaaagctg aaagcaagtc ttattttcct cctaaaggct atgcctttct gctgttccaa 1680

gaggaaagct cagtacaagc tttgatagat gcctgcctag aagaagatgg gaaactctac 1740

ctgtgtgtgt caagccccac catcaaggac aagccagtgc aaattcgacc atggaaccta 1800

agtgacagtg actttgtaat ggatggttct cagcctttgg accccagaaa aactatcttt 1860

gttgggggag ttccacgacc ccttcgagct gttgaactgg caatgatcat ggaccgtttg 1920

tacggtggtg tctgctatgc tggcattgat acggacccag agctgaagta ccccaaaggt 1980

gctggccgcg tggcattctc caatcagcag agttacattg cagccatcag cgctcgtttt 2040

gtgcagcttc agcacaatga cattgacaaa cgggttgaag taaagccata tgtgctggat 2100

gatcagatgt gtgatgagtg ccagggcaca cgctgtggtg ggaagtttgc cccgttcttc 2160

tgtgccaacg tcacctgtct gcagtattac tgtgaatact gctgggcgag catacattcc 2220

cgagccgggc gggagttcca caaaccgctg gtgaaggagg gaggcgaccg ccctcgtcac 2280

gtcccgttcc gctggagctg agccacgggc cgacccagcc acaagtactg gagtagataa 2340

caaggaggga aaagagaggg cactgcctca gggcttacag tgttctggaa atctgtgcat 2400

tcgttctcga tttttaaaga agaattgggt ctttttatta ttattattat tatttacagt 2460

catattactg aactcagtgt ccagtataat gcagaattgc agagtgtaga atggtactga 2520

tttgcacttt gattcacacc tttgtctgct tggtacctta attttttata cctgatttgt 2580

cactcgtgac agtatgtaga ctgccattct catcagcggc cgtcaggcta ctgtctgtga 2640

ttcttttgtt tgttgttgtg gtgttgttct gattgttttg aactggagca tgcacttatt 2700

ttcagaaact tagagagttg cccctaggac aagacttacc aaaggtaata ctcgtgagta 2760

ggtggcagat gtagcaaaaa cttcacaaca attcagcaat cattagctcg ggtcacttag 2820

agtaaggata acccttcatg aaaataagcc tttagttgct ctctattttg acttgtaagg 2880

atacctcata agctggatct tttatttttc cttcatgttt gatttttgtt ttgccgagga 2940

ttttggttag atcttttagt attatgtaaa tttatgctgc aatagctttt gctgctatta 3000

aaatggtatt attaatacca taatactcta ttcaggctag gatatcaatt aaacacaaaa 3060

acccaacaac acacttttgt gattttaggg atagagtcag ctgccgaacg gagatcagaa 3120

tagacttcag aaagcttcgg tggaaggtca ctatttctga ctgcatgttt acttaatcag 3180

gttgctgcat gcaataaatt ttatatccaa tgtctagtat aaaacgttcc cacaatgcac 3240

aaattaagaa tctatataag ctataaaaat actcattttt taaaaaaagt ttttttcatt 3300

caaagaattg gtaattgacc ggaggaaggc atgcctcaat aaatggaatg ctctgaatgg 3360

gaagagccca taacagaatg acctatatta caaccgatat aaaacctagg tgtaggatat 3420

tcttcaagca aatttgtgga tggtagatga agtactttgc ggtgctctgt tatatattgt 3480

gaaatttatt ttatatagta aagtgattat gtctaactgt gatcaaactt aactgtttaa 3540

agcctctgtc agacattaat gaacttgaca gttcataact gatatattat aaactaacac 3600

atgagctctt agttacaatc tcttagtgct tgcaccattt tacatagctt cagaccttgt 3660

ccagaaaacc aaagagctca tcttagctta caaacactaa gaatatatac aggatataga 3720

gataagttgt cagattgaca aactaggcac atttttaaga aagttgtgta atggtgatgc 3780

taaagaaatg tctgtacaaa ataagatggc ctgggcagag ttggttgtct gagtgagaaa 3840

ctaactactt aattccctgg atttatcctg ttaagcttga gtagaatgaa ggcctgctag 3900

cactagcgtg gtctagaatg aaggcctgct agcactagcg tggacatttt tctagcattc 3960

acccttgggc tgcagatagt aatatataaa cacacacaat gattgcaaga ctatgtaaat 4020

ctttttttaa tcttcttcct atgttttgga attctgggga caatctgact ggatatgaca 4080

ctgcagaata gatttaagtc gctgtgtttt ctgtgctgtg atgtccttgt actgtcttaa 4140

gttagtgttg cttgtttttg ttttgttctc ccatgtttag ttgcaattac tggctctcaa 4200

gctatagttt gttttcaaaa aggaaaaaca aaatagtttt ttactggatt aaaaatgctt 4260

attttagaat cctccccttt ttgaggcttt ttggtaataa ttgctttttt ccagcccagg 4320

tgtggttttg tttgtttttt tctatgtttg tgggtttttt ttcatctgta caagaaattt 4380

tgttatgcac tactactttt tgtattctag acagttttca aaagttggca gatttttttt 4440

tttgttttta aggaaaaagg catgctttct gactgacatg atcttttgca atacctcaat 4500

tttgaaatat aggaaagaaa atgatatttt ctaagtaagt ttattcagaa aaatatatat 4560

taatttaatt ctgcaagaaa atgatttaac agatgggtaa ctttattttt ttcatgctta 4620

tttgtgtttt ttgaaaatga agaagttaga gctttataac tataatatta aagatcatat 4680

ctaacctaca gaaatctacc taattatgtg aaagaagcaa aactttttga agaagcaccc 4740

ctctttcatg tattaaaata acactgagat ttttttaaaa gctggaagat tgtacagcat 4800

aaagctaaag tgaaggcaaa aaacattgtc ctgaagaata ggttacaaaa aataaactcc 4860

atttggtgct gaaagttgtg aatagatggt ggaaactact ttcccttaat ttgtatattt 4920

ctaatcaagt gttgattgtg cgcgactgac caggattgtg aaagagttct tctgtttgta 4980

tttttttaaa gagaactttt ttagtttatt aaattataac atgttccctt ttgttttgta 5040

aataaatgtg cccccaagtt gttaatgtta tattaaaaga gagggtcctt caaaaaaatt 5100

attttttaaa acacagaggt gttctgacct gcaacttgac tgggaagccc ctgggtaaca 5160

caggtcctgc tgtggccttt ccttggtgtg acacttgaac tagtcgattt tttgaaggct 5220

ataacctaac ctcgactatt tgttgttatg tgcaatactg tttgtactgt ttgtataaaa 5280

aatagaaaaa aaaaagaaaa gaaaaaaggc ataaatcttt attgcagatt tattttcatt 5340

gcaaacttta gacattgtga ttcactgaaa tcatttttct actgtcaaat atgtaccttt 5400

tcttcatgct ggtatttttt caatagtaat gtagcctttg tactgagaca aattttcatt 5460

gttattttta gaaagatttt ttgctaagaa aaaaattaaa catatttaca tatttttcat 5520

tttatttcgt attttcttta aggagtgctt tctcaatcat taatagagtt gtttctggga 5580

gggactttca tatctggtca atgtcataat attattttgt atttttattg gaataaatta 5640

attttatgat gctaattctt taaaagttta tttttttcat tttcagttat ttttgaagct 5700

aaaacctttg taatattgtt actaaagtgt ctagtttttt gaaatgcaaa gctttatgta 5760

ttaacttgct gatgtggttt acaatagtgt gacaacgatg aaaatgcttg gttatgtaaa 5820

gtgattggaa atgtaatgga taattgggta ataaaatgac agaatgagca gaaaaaa 5877

<210> 104
<211> 2953

<212>  DNA
<213>  Homo sapiens

<400>  104
gagttgggct gggccggccg acgaatcacg aggcttcgca cccggatatg gttatgggct      60

cggaaatcta gttcgggaaa agtgtgaggg gctcttcacg tggggaagga acagcaggcg     120

cggaggaggg ggcaagcgtg tgtgagattc agtggtccat gcgtgcgttt gtcgtgtaag     180

ggtcattcct ggggtttgga gtgggggaac aaatcaatgt ggctgttttt ccgtggaaag     240

aattcccact gcagtgtccc ggagcctgcg tgtggtgggc aagctcctca aatggtatct     300

cacagggaat aggggagtct tgaaaacgca gcttcggcag taggaacatg aacctcttac     360

ctaaaagttc cagggagttt ggctccgttg actattggga gaagttcttc cagcagcgag     420

gaaagaaagc tttcgagtgg tatggaacct acctggaact gtgcggggtg ctacataaat     480

atatcaagcc cagggaaaag gtgctggtga ttgggtgtgg caactcagaa ctgagtgagc     540

aactgtatga tgtgggctat cgggatatag tgaacatcga catcagtgag gttgtcatca     600

agcaaatgaa ggaatgtaat gccacccgac ggccccagat gagcttcttg aagatggaca     660

tgacgcagat ggagtttcct gatgcctcgt tccaggtggt gttggacaag ggcaccctgg     720

atgctgtcct gacagatgag gaagagaaga ccttacaaca ggtggacagg atgctggctg     780

aggttggccg tgtcctgcag ggccgggaga ccgagtggct ctttggcatg gatgagggcc     840

ggaaacagct ggcggccagt gctggcttca ggaggttgat tacagtggcc cttcaccgag     900

gtcagcagta tgaaagcatg gaccacatcc aagctgagct gtcggctaga gtcatggagc     960

tggccccagc tgggatgccc acccagcagc aggtcccctt tctgtctgtg ggtggggaca    1020

ttggggtccg gaccgttcag caccaagact gcagcccctt gagcggtgac tatgtcattg    1080

aggatgtgca aggggatgac aagcgatact tccgtcgact gatcttcctc agcaacagga    1140

atgtggtgca gtccgaagcc aggttgctga aggatgtgtc tcacaaagcc cagaagaagc    1200

ggaaaaagga caggaagaag cagcggcctg ctgatgcgga ggacctccct gcagccccgg    1260

ggcagtccat tgataagagt tacctgtgtt gtgaacacca caaagccatg atcgctggcc    1320

ttgccctgct gagaaaccca gagctactcc tagagatccc actggcattg ttggtggtag    1380

gcctgggcgg gggcagcctc cccctctttg tccacgatca ttttccaaag tcctgcattg    1440

atgctgtgga gatcgatccc tccatgttgg aagtggccac ccagtggttt ggcttctccc    1500

agagtgaccg aatgaaggtc cacattgcag atggcctgga ctatatcgcc agcttggcag    1560

gaggaggaga agcacggcct tgctacgatg tcataatgtt tgatgttgac agtaaggacc    1620

caacactggg aatgagttgt ccgcccccag catttgtgga gcaatctttt ctacagaagg    1680

ttaaaagcat cttgactcct gaaggtgttt ttattctcaa ccttgtgtgc cgagacttgg 1740

ggctaaaaga ctcagtgctg gctgggctca aggcagtgtt ccccctccta tatgtccggc 1800

gaattgaggg tgaagtgaat gagatcctgt tctgtcagct gcaccctgag caaaaacttg 1860

ccacaccaga gctcctagaa acagcccagg ctttggagcg gaccctgagg aagcctggga 1920

ggggttggga tgacacgtat gtcttgtcag atatgctcaa gacggtgaaa attgtgtgac 1980

tgcttaggcc aagcagccct cctgcctaga ctgaccttgg actcccagcc tgccagagaa 2040

tgaagaaata caacgcacag tacttttgaa gcttcgtatt tttcttggtt tcacactcag 2100

ctacatgtga cctccagctt ggtgaggttg cctgaagatt agggaaaata aaaatgtcct 2160

tcccatcttg tcctcttcag taccacttgg gttggtttgt ctttgcttcc tacaccacgt 2220

ccttgagtgg agttccctgc tgaagcccct agcacacact gcatgcctta acaagtgtgt 2280

gcaagcccct cagaactcaa gacatccaaa ttttattgcg tctctactta tactggtttg 2340

cttttgattt attcctctat tagttctata ggagtgatct caagtgagat agcagagcaa 2400

gatgccaaaa gaccataaat agagtaaggt ttctatagat gtgagacaga tttgagagag 2460

catttactct gtctccctgt ggatgaaact gctgctgaaa tggttccaat ttttaggaat 2520

ctgcttaccc acttcattat ttgacagctt tccttggtga cccaaacctt gtagcctaag 2580

ccatttgtct ttttctcagt ggagggagtg tatggacctg gccccatggc tttgcatgtt 2640

agagacctgg cagactaaag tctctagtgt ttgtttgctc acatttgctg agtgacagct 2700

atgtgccaga ctgcataaag ggtggtggca gaagtgaaaa tgtttaagaa tgaccaaaaa 2760

cattagtaat gaaagttaat gtgttccagg cattcttcta agtggtttac atgcactgtc 2820

tcatttaatc tgagataaag gatacttaag cccaaactat atctaaaccc aaatctcact 2880

tggctggaaa catcaatctt aaccatttat tcagaaccat taaaccaatg attccaagaa 2940

aaaaaaaaaa aaa 2953

<210> 105
<211> 1321
<212> DNA
<213> Homo sapiens

<400> 105
cttttcccgg cacatgcgca ccgcagcggg tcgcgcgccc taaggagtgg cactttttaa 60

aagtgcagcc ggagaccagc ctacagccgc ctgcatctgt atccagcgcc aggtcccgcc 120

agtcccagct gcgcgcgccc cccagtcccg cacccgttcg gcccaggcta agttagccct 180

caccatgccg gtcaaaggag gcaccaagtg catcaaatac ctgctgttcg gatttaactt 240

catcttctgg cttgccggga ttgctgtcct tgccattgga ctatggctcc gattcgactc 300

tcagaccaag agcatcttcg agcaagaaac taataataat aattccagct tctacacagg 360

agtctatatt ctgatcggag ccggcgccct catgatgctg gtgggcttcc tgggctgctg 420

cggggctgtg caggagtccc agtgcatgct gggactgttc ttcggcttcc tcttggtgat 480

attcgccatt gaaatagctg cggccatctg gggatattcc cacaaggatg aggtgattaa 540

ggaagtccag gagttttaca aggacaccta caacaagctg aaaaccaagg atgagcccca 600

gcgggaaacg ctgaaagcca tccactatgc gttgaactgc tgtggtttgg ctgggggcgt 660

ggaacagttt atctcagaca tctgccccaa gaaggacgta ctcgaaacct tcaccgtgaa 720

gtcctgtcct gatgccatca aagaggtctt cgacaataaa ttccacatca tcggcgcagt 780

gggcatcggc attgccgtgg tcatgatatt tggcatgatc ttcagtatga tcttgtgctg 840

tgctatccgc aggaaccgcg agatggtcta gagtcagctt acatccctga gcaggaaagt 900

ttacccatga agattggtgg gattttttgt ttgtttgttt tgttttgttt gttgtttgtt 960

gtttgttttt ttgccactaa ttttagtatt cattctgcat tgctagataa aagctgaagt 1020

tactttatgt ttgtctttta atgcttcatt caatattgac atttgtagtt gagcgggggg 1080

tttggtttgc tttggtttat attttttcag ttgtttgttt ttgcttgtta tattaagcag 1140

aaatcctgca atgaaaggta ctatatttgc tagactctag acaagatatt gtacataaaa 1200

gaattttttt gtctttaaat agatacaaat gtctatcaac tttaatcaag ttgtaactta 1260

tattgaagac aatttgatac ataataaaaa attatgacaa tgtcctggac tggtaaaaaa 1320

a 1321

<210> 106
<211> 6113
<212> DNA
<213> Homo sapiens

<400> 106
taatacttaa ttaccttcta ataattggag cagaagatga acccaactaa tcctttcagt 60

gggcagcagc ctagtgcttt ttcggcgtct tctagtaatg taggaacact tccatctaag 120

ccgccatttc gatttggtca accttctctt tttggacaaa acagtacctt atctgggaag 180

agctcgggat tttcacaggt atccagcttt ccagcgtctt ctggagtaag tcattcctct 240

tcagtgcaaa cattagggtt cacccaaacc tcaagtgttg gacccttttc tggacttgag 300

cacacttcca cctttgtggc tacctctggg ccttcaagtt catctgtgct gggaaacaca 360

ggatttagtt ttaaatcacc caccagtgtt ggggctttcc caagcacttc tgcttttgga 420

caagaagctg gagaaatagt gaactctggt tttgggaaaa cagaattcag ctttaaacct 480

ctggaaaatg cagtgttcaa accaatactg ggggctgaat ctgagccaga gaaaacccag 540

agccaaattg cttctgggtt ttttacattt tcccacccaa ttagtagtgc acctggaggc 600

ctggcccctt tctcttttcc tcaagtaaca agtagttcag ctaccacttc aaattttacc 660

ttttcaaaac ctgttagtag taataattca ttatctgcct ttacccctgc tttgtcaaac 720

caaaatgtag aggaagagaa gagaggacct aagtcaatat ttggaagttc taataatagc 780

ttcagtagct tccctgtatc atctgcggtt ttgggcgaac ctttccaggc tagcaaagca 840

ggtgtcaggc aggggtgtga agaagctgtt tcccaggtgg aaccacttcc cagcctaatg 900

aaaggactga aaaggaagga ggaccaggat cgctccccaa ggagacatgg ccacgagcca 960

gcagaagatt cggatcctct gtcccggggc gatcatcctc cagacaaacg acctgtccgc 1020

ctgaatcgac ccggggaggt tactttattt ggtcggacga tacaggatgt tttcaaaagc 1080

aataaggaag taggtcgtct gggcaacaag gaggccaaaa aggaaactgg ctttgttgag 1140

tctgcagaaa gtgaccacat ggctatccca ggagggaatc agtctgtcct ggcaccttcc 1200

cggattccag gtgtgaataa agaggaagaa actgaaagta gagagaagaa agaagattct 1260

ctaagaggaa ctccggcgcg tcagagtaac agaagcgaga gcacagacag tcttgggggc 1320

ttgtctccct ctgaagtcac agccatccag tgcaagaaca tccctgacta cctcaacgac 1380

aggaccattc tggagaacca ttttggcaaa attgctaaag tgcagcgcat ctttaccagg 1440

cgcagcaaaa agcttgcagt ggtacatttc tttgatcatg catctgcagc cctggctaga 1500

aagaagggga aaagtttgca taaagacatg gctatctttt ggcacaggaa gaaaataagc 1560

cccaataaga aacccttttc cctgaaggag aagaaaccag gtgacggtga agtcagcccg 1620

agcacagagg atgcaccctt tcagcactct cctcttggca aggccgcagg gaggactggt 1680

gctagcagcc tcctgaataa aagctctcca gtgaagaagc caagtcttct aaaggcccac 1740

caattcgagg gagactcttt tgactcagcc tccgagggct ccgagggcct cgggccatgt 1800

gtgctctccc tcagtaccct gataggcact gtggctgaga catccaagga gaagtaccgc 1860

ctgcttgacc agagagacag gatcatgcgg caagctcggg tgaagagaac cgatctggac 1920

aaagcgagga cttttgttgg cacctgcctg gatatgtgtc ctgagaagga gaggtacatg 1980

cgggagaccc gtagccagct gagcgtgttc gaagtggtcc cagggactga ccaggtggac 2040

cacgcagcag ctgtgaaaga gtacagtcgg tcctcggcgg atcaggagga gcccctgccc 2100

cacgagctgc ggcccttgcc agtgctcagc aggaccatgg actacctggt gacccagatc 2160

atggaccaga aggagggcag cctgcgggat tggtatgact tcgtgtggaa ccgcacgcgt 2220

ggcatacgga aggatatcac gcagcagcac ctctgtgacc ccctgacggt gtccctgatt 2280

gagaagtgca cccggtttca catccactgt gcccacttca tgtgtgagga gcccatgtcc 2340

tcctttgatg ccaagatcaa taatgagaac atgaccaagt gcctgcagag cctgaaggag 2400

atgtaccagg acctgagaaa caaggtgtgc ttctgtgcca gcgaagcgga gttccagggc 2460

tacaatgttc tgctcagtct caacaaggga gacatcctaa gagaagtaca acagttccat 2520

cctgctgtta gaaactcatc tgaggtgaaa tttgctgttc aggcttttgc tgcattgaac 2580

agtaataatt ttgtgagatt tttcaaactg gtccagtcag cttcttacct gaacgcttgt 2640

cttttacact gttacttcag tcagatccgc aaggatgctc tccgggcgct caactttgcg 2700

tacacggtga gcacacagcg atctaccatc tttcccctgg atggtgtggt gcgcatgctg 2760

ctgttcagag actgtgaaga ggccaccgac ttcctcacct gccacggcct caccgtttcc 2820

gacggctgtg tggagctgaa ccggtctgca ttcctggaac cagagggatt atccaagacc 2880

aggaagtcgg tgtttattac taggaagctg acggtgtcag tcggggaaat tgtgaacgga 2940

gggccattgc cccccgtccc tcgtcatacc cctgtgtgca gcttcaactc ccagaacaag 3000

tacatcgggg agagcctggc cgcggagctg cccgtcagca cccagagacc cggctccgac 3060

acagtgggcg gagggagagg agaggagtgt ggtgtagagc cggatgcacc cctgtccagt 3120

ctcccacagt ctctaccagc ccctgcgccc tcaccagtgc ctctgcctcc tgtcctggca 3180

ctgaccccgt ctgtggcgcc cagcctcttc cagctgtctg tgcagcctga accaccgcct 3240

ccagagcccg tgcccatgta ctctgacgag gacctggcgc aggtggtgga cgagctcatc 3300

caggaggccc tgcagaggga ctgtgaggaa gttggctctg cgggtgctgc ctacgcagct 3360

gccgccctgg gtgtttctaa tgctgctatg gaggatttgt taacagctgc aaccacgggc 3420

attttgaggc acattgcagc tgaagaagtg tctaaggaaa gagagcgaag ggagcaggag 3480

aggcagcggg ctgaagagga aaggttgaaa caagagagag agctggtgtt aagtgagctg 3540

agccagggcc tggccgtgga gctgatggaa cgcgtgatga tggagtttgt gagggaaacc 3600

tgctcccagg agttgaagaa tgcagtagag acagaccaga gggtccgtgt ggcccgttgc 3660

tgtgaggatg tctgtgccca cttagtggac ttgtttctcg tggaggaaat cttccagact 3720

gcaaaggaga ccctccagga gcttcagtgc ttctgcaagt atctacagcg gtggagggaa 3780

gctgtcacag cccgcaagaa actgaggcgc caaatgcggg ctttccctgc tgcgccctgc 3840

tgcgtggacg tgagcgaccg gctgagggcg ctggcgccca gcgcagagtg ccccattgct 3900

gaagagaacc tggccagggg cctcctggac ctgggccatg cagggagatt gggcatctcc 3960

tgcaccaggt taaggcggct cagaaacaag acagctcacc agatgaaggt tcagcacttc 4020

taccagcagc tgctgagtga tgtggcatgg gcgtctctgg acctgccatc cctcgtggct 4080

gagcacctcc ctgggaggca ggagcatgtg ttttggaagc tggtgctggt gttgccggat 4140

gtagaggagc agtccccaga gagttgtggc agaattctag caaattggtt aaaagtcaag 4200

ttcatgggag atgaaggctc agtggatgac acatccagcg atgctggtgg gattcagacg 4260

ctttcgcttt tcaactcact tagcagcaaa ggggatcaga tgatttctgt taacgtgtgt 4320

ataaaggtgg cccatggcgc cctcagtgat ggtgccattg atgctgtgga gacacagaag 4380

gacctcctgg gagccagtgg gctcatgctg ctgcttcccc ccaaaatgaa gagtgaggac 4440

atggcagagg aggacgtgta ctggctgtcg gccttgctgc agctcaagca gctcctgcag 4500

gctaagccct tccagcctgc gcttcctctg gtggttcttg tgcctagccc aggaggggac 4560

gccgttgaga aggaagtaga agatggtctg atgctacagg acttggtttc agctaagctg 4620

atttcagatt acactgttac cgagatccct gataccatta atgatctaca aggttcaact 4680

aaggttttgc aagcagtgca gtggctggtt tcccactgcc cccattccct tgacctctgc 4740

tgccagactc tcattcagta cgtcgaagac gggattggcc atgagtttag tggccgcttt 4800

ttccatgaca gaagagagag gcgtctgggc ggtcttgctt ctcaggagcc tggcgccatc 4860

attgagctgt ttaacagtgt gctgcagttc ctggcttctg tggtgtcctc tgaacagctg 4920

tgtgacctgt cctggcctgt cactgagttt gctgaggcag ggggcagccg gctgcttcct 4980

cacctgcact ggaatgcccc agagcacctg gcctggctga agcaggctgt gctcgggttc 5040

cagcttccgc agatggacct tccacccctg gggggcccct ggctccccgt gtgctccatg 5100

gttgtccagt acgcctccca gatccccagc tcacgccaga cacagcctgt cctccagtcc 5160

caggtggaga acctgctcca cagaacctac tgtaggtgga agagcaagag tccctcccca 5220

gtccatgggg caggcccctc ggtcatggag atcccatggg atgatcttat cgccttgtgt 5280

atcaaccaca agctgagaga ctggacgccc ccccggcttc ctgttacatc agaggcgctg 5340

agtgaagatg gtcagatatg tgtgtatttt tttaaaaacg atttgaaaaa atatgatgtt 5400

cctttgtcgt gggaacaagc caggttgcag acgcagaagg agctacagct gagagaggga 5460

cgtttggcaa taaagccttt tcatccttct gcaaacaatt ttcccatacc attgcttcac 5520

atgcaccgta actggaagag gagcacagag tgtgctcaag aggggaggat tcccagcaca 5580

gaggatctga tgcgaggagc ttctgctgag gagctcttgg cgcagtgttt gtcgagcagt 5640

ctgctgctgg agaaagaaga gaacaagagg tttgaagatc agcttcagca atggttgtct 5700

gaagactcag gagcatttac ggatttaact tcccttcccc tctatcttcc tcagactcta 5760

gtgtctcttt ctcacactat tgaacctgtg atgaaaacat ctgtaactac tagcccacag 5820

agtgacatga tgagggagca actgcagctg tcagaggcga caggaacgtg tctaggcgaa 5880

cgactaaagc acctggaaag gctgatccgg agttcaaggg aagaggaagt tgcctctgag 5940

ctccatctct ctgcgctgct agacatggtg gacatttgag cagcctgacc tgtgggaggg 6000

gggtctctcc cgaagagttt ctgtttttac tcaaaataat gttattctca gatgcttgat 6060

gcactgttgg aaatgtgatt aatttaatca tgcagataaa ccatttaaat gtc 6113

<210> 107
<211> 5243
<212> DNA
<213> Homo sapiens

<400> 107
agcgtgagac tcgcgccctc cggcacggaa aaggccaggc gacaggtgtc gcttgaaaag 60

actgggcttg tccttgctgg tgcatgcgtc gtcggcctct gggcagcagg tttacaaagg 120

aggaaaacga cttcttctag attttttttt cagtttcttc tataaatcaa aacatctcaa 180

aatggagacc taaaatcctt aaagggactt agtctaatct cgggaggtag ttttgtgcat 240

gggtaaacaa attaagtatt aactggtgtt ttactatcca aagaatgcta attttataaa 300

catgatcgag ttatataagg tataccataa tgagtttgat tttgaatttg atttgtggaa 360

ataaaggaaa agtgattcta gctggggcat attgttaaag catttttttc agagttggcc 420

aggcagtctc ctactggcac attctcccat tatgtagaat agaaatagta cctgtgtttg 480

ggaaagattt taaaatgagt gacagttatt tggaacaaag agctaataat caatccactg 540

caaattaaag aaacatgcag atgaaagttt tgacacatta aaatacttct acagtgacaa 600

agaaaaatca agaacaaagc tttttgatat gtgcaacaaa tttagaggaa gtaaaaagat 660

aaatgtgatg attggtcaag aaattatcca gttatttaca aggccactga tattttaaac 720

gtccaaaagt ttgtttaaat gggctgttac cgctgagaat gatgaggatg agaatgatgg 780

ttgaaggtta cattttagga aatgaagaaa cttagaaaat taatataaag acagtgatga 840

atacaaagaa gatttttata acaatgtgta aaatttttgg ccagggaaag gaatattgaa 900

gttagataca attacttacc tttgagggaa ataattgttg gtaatgagat gtgatgtttc 960

tcctgccacc tggaaacaaa gcattgaagt ctgcagttga aaagcccaac gtctgtgaga 1020

tccaggaaac catgcttgca aaccactggt aaaaaaaaaa aaaaaaaaaa aaaaaagcca 1080

cagtgacttg cttattggtc attgctagta ttatcgactc agaacctctt tactaatggc 1140

tagtaaatca taattgagaa attctgaatt ttgacaaggt ctctgctgtt gaaatggtaa 1200

atttattatt ttttttgtca tgataaattc tggttcaagg tatgctatcc atgaaataat 1260

ttctgaccaa aactaaattg atgcaatttg attatccatc ttagcctaca gatggcatct 1320

ggtaactttt gactgtttta aaaaataaat ccactatcag agtagatttg atgttggctt 1380

cagaaacatt tagaaaaaca aaagttcaaa aatgttttca ggaggtgata agttgaataa 1440

ctctacaatg ttagttcttt gagggggaca aaaaatttaa aatctttgaa aggtcttatt 1500

ttacagccat atctaaatta tcttaagaaa atttttaaca aagggaatga aatatatatc 1560

atgattctgt ttttccaaaa gtaacctgaa tatagcaatg aagttcagtt ttgttattgg 1620

tagtttgggc agagtctctt tttgcagcac ctgttgtcta ccataattac agaggacatt 1680

tccatgttct agccaagtat actattagaa taaaaaaact taacattgag ttgcttcaac 1740

agcatgaaac tgagtccaaa agaccaaatg aacaaacaca ttaatctctg attatttatt 1800

ttaaatagaa tatttaattg tgtaagatct aatagtatca ttatacttaa gcaatcatat 1860

tcctgatgat ctatgggaaa taactattat ttaattaata ttgaaaccag gttttaagat 1920

gtgttagcca gtcctgttac tagtaaatct ctttatttgg agagaaattt tagattgttt 1980

tgttctcctt attagaagga ttgtagaaag aaaaaaatga ctaattggag aaaaattggg 2040

gatatatcat atttcactga attcaaaatg tcttcagttg taaatcttac cattatttta 2100

cgtacctcta agaaataaaa gtgcttctaa ttaaaatatg atgtcattaa ttatgaaata 2160

cttcttgata acagaagttt taaaatagcc atcttagaat cagtgaaata tggtaatgta 2220

ttattttcct cctttgagtt aggtcttgtg cttttttttc ctggccacta aatttcacaa 2280

tttccaaaaa gcaaaataaa catattctga atatttttgc tgtgaaacac ttgacagcag 2340

agctttccac catgaaaaga agcttcatga gtcacacatt acatctttgg gttgattgaa 2400

tgccactgaa acattctagt agcctggaga agttgaccta cctgtggaga tgcctgccat 2460

taaatggcat cctgatggct taatacacat cactcttctg tgaagggttt taattttcaa 2520

cacagcttac tctgtagcat catgtttaca ttgtatgtat aaagattata caaaggtgca 2580

attgtgtatt tcttccttaa aatgtatcag tataggattt agaatctcca tgttgaaact 2640

ctaaatgcat agaaataaaa ataataaaaa atttttcatt ttggcttttc agcctagtat 2700

taaaactgat aaaagcaaag ccatgcacaa aactacctcc ctagagaaag gctagtccct 2760

tttcttcccc attcatttca ttatgaacat agtagaaaac agcatattct tatcaaattt 2820

gatgaaaagc gccaacacgt ttgaactgaa atacgacttg tcatgtgaac tgtaccgaat 2880

gtctacgtat tccacttttc ctgctggggt tcctgtctca gaaaggagtc ttgctcgtgc 2940

tggtttctat tacactggtg tgaatgacaa ggtcaaatgc ttctgttgtg gcctgatgct 3000

ggataactgg aaaagaggag acagtcctac tgaaaagcat aaaaagttgt atcctagctg 3060

cagattcgtt cagagtctaa attccgttaa caacttggaa gctacctctc agcctacttt 3120

tccttcttca gtaacaaatt ccacacactc attacttccg ggtacagaaa acagtggata 3180

tttccgtggc tcttattcaa actctccatc aaatcctgta aactccagag caaatcaaga 3240

tttttctgcc ttgatgagaa gttcctacca ctgtgcaatg aataacgaaa atgccagatt 3300

acttactttt cagacatggc cattgacttt tctgtcgcca acagatctgg caaaagcagg 3360

cttttactac ataggacctg gagacagagt ggcttgcttt gcctgtggtg gaaaattgag 3420

caattgggaa ccgaaggata atgctatgtc agaacacctg agacattttc ccaaatgccc 3480

atttatagaa aatcagcttc aagacacttc aagatacaca gtttctaatc tgagcatgca 3540

gacacatgca gcccgcttta aaacattctt taactggccc tctagtgttc tagttaatcc 3600

tgagcagctt gcaagtgcgg gtttttatta tgtgggtaac agtgatgatg tcaaatgctt 3660

ttgctgtgat ggtggactca ggtgttggga atctggagat gatccatggg ttcaacatgc 3720

caagtggttt ccaaggtgtg agtacttgat aagaattaaa ggacaggagt tcatccgtca 3780

agttcaagcc agttaccctc atctacttga acagctgcta tccacatcag acagcccagg 3840

agatgaaaat gcagagtcat caattatcca ttttgaacct ggagaagacc attcagaaga 3900

tgcaatcatg atgaatactc ctgtgattaa tgctgccgtg gaaatgggct ttagtagaag 3960

cctggtaaaa cagacagttc agagaaaaat cctagcaact ggagagaatt atagactagt 4020

caatgatctt gtgttagact tactcaatgc agaagatgaa ataagggaag aggagagaga 4080

aagagcaact gaggaaaaag aatcaaatga tttattatta atccggaaga atagaatggc 4140

actttttcaa catttgactt gtgtaattcc aatcctggat agtctactaa ctgccggaat 4200

tattaatgaa caagaacatg atgttattaa acagaagaca cagacgtctt tacaagcaag 4260

agaactgatt gatacgattt tagtaaaagg aaatattgca gccactgtat tcagaaactc 4320

tctgcaagaa gctgaagctg tgttatatga gcatttattt gtgcaacagg acataaaata 4380

tattcccaca gaagatgttt cagatctacc agtggaagaa caattgcgga gactacaaga 4440

agaaagaaca tgtaaagtgt gtatggacaa agaagtgtcc atagtgttta ttccttgtgg 4500

tcatctagta gtatgcaaag attgtgctcc ttctttaaga aagtgtccta tttgtaggag 4560

tacaatcaag ggtacagttc gtacatttct ttcatgaaga agaaccaaaa catcgtctaa 4620

actttagaat taatttatta aatgtattat aactttaact tttatcctaa tttggtttcc 4680

ttaaaatttt tatttattta caactcaaaa aacattgttt tgtgtaacat atttatatat 4740

gtatctaaac catatgaaca tatatttttt agaaactaag agaatgatag gcttttgttc 4800

ttatgaacga aaaagaggta gcactacaaa cacaatattc aatcaaaatt tcagcattat 4860

tgaaattgta agtgaagtaa aacttaagat atttgagtta acctttaaga attttaaata 4920

ttttggcatt gtactaatac cgggaacatg aagccaggtg tggtggtatg tgcctgtagt 4980

cccaggctga ggcaagagaa ttacttgagc ccaggagttt gaatccatcc tgggcagcat 5040

actgagaccc tgcctttaaa aacaaacaga acaaaaacaa aacaccaggg acacatttct 5100

ctgtcttttt tgatcagtgt cctatacatc gaaggtgtgc atatatgttg aatgacattt 5160

tagggacatg gtgtttttat aaagaattct gtgagaaaaa atttaataaa gcaacaaaaa 5220

ttactcttaa aaaaaaaaaa aaa 5243

<210> 108
<211> 2389
<212> DNA
<213> Homo sapiens

<400> 108
atgccggaag ctttcctgcc acgcaagctt tccgagcatg ctaactcgcc ccaagcagct 60

cccatcccgc agcatccact gcagccgctg aggagagtgg cgcagcgcag aaaccagtgc 120

ccagtgccca ggtgggggaa gcggtccgtg ggagtcacag ccgcgccacc cgccgccgct 180

acccccttca aggtctcgct gcagcccccg ggagccgccg gcgccgcgcc cgagcccgag 240

accggtgagt gccagcccgc cgcggccgcc gagcaccggg aagccgccgc tgtccccgcc 300

gccaagatgc ccgccttctc ctcctgcttc gaggtggtgt ccggggccgc cgcgcccgcc 360

tccgccgccg ccggcccgcc cggcgcgtcc tgcaagccgc cgctgccgcc gcactacacg 420

tccaccgcac agatcaccgt gcgggccctg ggcgccgaca ggctcctgct gcacggcccc 480

gatcccgttc ccggcgccgc gggctccgcc gctgccccgc gcggccgctg cctcctgctc 540

gccccggcgc ccgcagcccc ggtcccgcgc ggcggggctc ctcggcctgg ctcctggagg 600

agctgctgcg gcccgactgc cccgagcccg cgggcttgga cgcgacacgg gaggggcccg 660

atcggaactt ccgactgagc gagcaccgcc aggccctggc cgccgccaag caccgaggcc 720

ccgcggcgac cccggggagc cccgatcccg gccccggtcc gtggggcgaa gagcacttgg 780

cggagagggg ccccaggggc tgggagaggg gcggcgatcg ctgcgacgcg cccggtgggg 840

acgcggcgcg gaggcccgac ccagaggccg aggcaccccc cgccgggagc atcgaggccg 900

ccccgagtag tgcggcggag ccggtgatcg tctccaggtc ggatcccaga gacgagaagc 960

tggccctata cctggccgag gtggagaagc aggacaagta tctgcggcag aggaataagt 1020

accgattcca catcattcca gacggcaact gcctctaccg agctgtcagc aagacggtgt 1080

atggggacca gagcctgcac cgggagttga gggagcagac ggtgcactac atcgccgacc 1140

atctcgacca cttcagcccc ctgattgagg gcgacgtggg ggagtttatc atcgctgctg 1200

cccaagacgg ggcatgggcc gggtacccgg agttgctggc catggggcag atgctgaatg 1260

tgaatatcca tttaactact ggagggaggc tggagagtcc cacggtgtct accatgattc 1320

attatttggg cccagaggat tccctgaggc ctagtatttg gctcagttgg ctcagtaacg 1380

gacactatga tgctgtattt gatcactcct atcctaaccc agagtacgac aactggtgca 1440

aacaaactca agtgcaaagg aaacgcgacg aagaacttgc caaatctatg gccatatcct 1500

tgtctaaaat gtatattgaa caaaatgcat gctcttgaaa tgtctcaaaa ccttacaccc 1560

tgggaataat tgcatatata acttgtgttt ggagaatcac atgaacttta atcagggtaa 1620

tagcactttc aaacttgcta gtagatttta ctgtaggtgt aatgccttaa tcatcttttt 1680

gaatgttttc tcagagctgg aggttgctgg gcacctaaat gatgtttcat gatagctttg 1740

ggtgatttta ctgctattta taatttgctg tataaagtga gcattactta atttgcaagc 1800

tgatttctca cagtgtaaat ttgttcattc ctggtagtct attttctata aaaatgtatt 1860

tttgcacaac atttttaaaa actggtgtac cttcatctat gacgtgttcc attttgacaa 1920

acagctttca ggcgtaaatc cagagaagtg ctttatatga aatgtattat tttgaacaga 1980

gtttgtgatt tggtagttat tttatgttgt tgaaatttga atttcacaat tcttagataa 2040

ttatttcaaa tggatattga tgcattcttg ttaccagatg tttggcccat tccattttga 2100

tgaaacagag ctgttgtttt ggaagtcatt atttttctag aaatggcgaa tcttttaaag 2160

aaaattactt aatggaaggt tgtgggaagg tgtttttttg tgtttttttt ttggtttttg 2220

ttttgttttt cctcttttaa gggatagtag caggtcttac ttgaatgaaa gtctgatatt 2280

tgctgatggc agaatgatta ttctgtaccc tggttgatgt gtagagtaga ttgtctggtg 2340

ctctcagttg tttttattta catttgtcac gttgtttcag gatggtctc 2389

<210> 109
<211> 1363
<212> DNA
<213> Homo sapiens

<400> 109
gtcgtggggg cggcactggg agcgccttcc ggagagacgc agtcggctgc caccccggga 60

tgggtcgctg gtgccagacc gtcgcgcgcg ggcagcgccc ccggacgtct gccccctccc 120

gcgccggtgc cctgctgctg ctgcttctgt tgctgaggtc tgcaggttgc tggggcgcag 180

gggaagcccc gggggcgctg tccactgctg atcccgccga ccagagcgtc cagtgtgtcc 240

ccaaggccac ctgtccttcc agccggcctc gccttctctg gcagaccccg accacccaga 300

cactgccctc gaccaccatg gagacccaat tcccagtttc tgaaggcaaa gtcgacccat 360

accgctcctg tggcttttcc tacgagcagg accccaccct caggacccca gaagccgtgg 420

ctcggcggtg gccctggatg gtcagcgtgc gggccaatgg cacacacatc tgtgccggca 480

ccatcattgc ctcccagtgg gtgctgactg tggcccactg cctgatctgg cgtgatgtta 540

tctactcagt gagggtgggg agtccgtgga ttgaccagat gacgcagacc gcctccgatg 600

tcccggtgct ccaggtcatc atgcatagca ggtaccgggc ccagcggttc tggtcctggg 660

tgggccaggc caacgacatc ggcctcctca agctcaagca ggaactcaag tacagcaatt 720

acgtgcggcc catctgcctg cctggcacgg actatgtgtt gaaggaccat tcccgctgca 780

ctgtgacggg ctggggactt tccaaggctg acggcatgtg gcctcagttc cggaccattc 840

aggagaagga agtcatcatc ctgaacaaca aagagtgtga caatttctac cacaacttca 900

ccaaaatccc cactctggtt cagatcatca agtcccagat gatgtgtgcg gaggacaccc 960

acagggagaa gttctgctat gagctaactg gagagccctt ggtctgctcc atggagggca 1020

cgtggtacct ggtgggattg gtgagctggg gtgcaggctg ccagaagagc gaggccccac 1080

ccatctacct acaggtctcc tcctaccaac actggatctg ggactgcctc aacgggcagg 1140

ccctggccct gccagcccca tccaggaccc tgctcctggc actcccactg cccctcagcc 1200

tccttgctgc cctctgactc tgtgtgccct ccctcacttg tgggcccccc ttgcctccgt 1260

gcccaggttg ctgtgggtgc agctgtcaca gccctgagag tcagggtgga gatgaggtgc 1320

tcaattaaac attactgttt tccatgcaaa aaaaaaaaaa aaa 1363

<210> 110
<211> 4047
<212> DNA
<213> Homo sapiens

<400> 110
agaactcgcg cgcctcccct cgaacgaacg cgcacgcgcg cgcagggggc gggtcttctt 60

cccgacaccc actgagcgcc cggcctcctc accccacccc accgaactcc ggccgcccag 120

gctccgcctc cgcgacgggc cgtcgggagg aggcgaccgg aagccactta aagcagagat 180

cgaggtgaca ggcgagctgg ctggactcgg agcgcggtcg aggctttctg cgttcgcggc 240

ggcggaatgg cccgtgcgcg gctcgccgcg tcgcggctct gtggtcccta gacgtcggct 300

cccgccctcg gcgctgatct ccggcgcggg cactgctttc cactcggctc ctgtcgtccg 360

ttctctcagg ctcccgttca ggatttttag actctgagga gcagttggag ctaatccaca 420

ttatggaaat ggaaaccacc gaacctgagc cagactgtgt agtgcagcct ccctctcctc 480

ctgatgactt ttcatgccaa atgagactct ctgagaagat cactccattg aagacttgtt 540

ttaagaaaaa ggatcagaaa agattgggaa ctggaaccct gaggtctttg aggccaatat 600

taaacactct tctagaatct ggctcacttg atggggtttt tagatctagg aaccagagta 660

cagatgagaa cagcttacat gaacctatga tgaagaaagc catggaaatc aattcatcat 720

gcccaccagc agaaaataat atgtctgttc tgattcctga taggacaaat gttggggacc 780

agataccgga agcccatcct tccactgaag ctccagaacg agtggttcca atccaagatc 840

acagctttcc atcagaaacc ctcagtggga cggtggcaga ttccacacca gctcacttcc 900

agactgatct tttgcaccca gtttcaagtg atgttcctac tagtcctgac tgcttagaca 960

aagtcataga ttatgttcca ggcattttcc aagaaaacag ttttacaatc caatacattc 1020

tggacaccag tgataagctg agtactgagc tctttcagga caaaagtgaa gaggcttccc 1080

ttgacctcgt gtttgagctg gtgaaccagt tgcagtacca cactcaccaa gagaacggaa 1140

ttgaaatttg catggacttt ctgcaaggca cttgtattta tggcagggat tgtttgaagc 1200

accacactgt cttgccatat cattggcaga tcaaaaggac aactactcaa aagtggcaga 1260

gtgtattcaa tgattctcag gagcacttgg aaagatttta ctgtaaccca gaaaatgata 1320

gaatgagaat gaagtatgga ggacaagaat tttgggcaga tttgaatgcc atgaacgtgt 1380

atgaaacaac tgaatttgac caactacgaa ggctgtccac accaccctct agcaatgtca 1440

actctattta ccacacagtc tggaaattct tctgtaggga ccactttgga tggagagagt 1500

atcccgagtc tgtcattcga ttgattgaag aagccaactc tcggggtctg aaagaggttc 1560

gatttatgat gtggaataac cactacatcc tccacaattc attcttcagg agagagataa 1620

aaaggagacc cctcttccgc tcctgtttta tactgcttcc atatttacag acacttggtg 1680

gggttcccac acaagctcct ccacctcttg aagcaacttc atcatcacaa attatctgcc 1740

cagatggggt cacttcagca aacttttacc ctgaaacttg ggtttatatg catccatctc 1800

aggacttcat ccaagtccct gtttctgcag aggataaaag ttatcggatc atttacaatc 1860

tttttcataa gactgtgcct gagtttaaat acagaatttt gcagatattg agagtccaaa 1920

accagtttct ttgggagaaa tataaaagga aaaaggaata tatgaacagg aaaatgtttg 1980

gccgtgacag gataataaat gagagacatt tatttcatgg aacatcccag gatgtggtag 2040

atggaatctg caaacacaac tttgaccctc gagtctgtgg aaagcatgct acaatgtttg 2100

gacaaggcag ttattttgca aagaaggcaa gctactctca taacttttct aagaagtcct 2160

ccaaaggagt ccacttcatg tttctggcca aagtgctgac gggcagatac acaatgggca 2220

gtcatggcat gagaaggccc ccgccagtca atcctggcag tgtcaccagt gacctttatg 2280

actcttgtgt ggataatttc tttgagcctc agatttttgt catttttaat gatgaccaga 2340

gttaccctta ttttgttatc caatatgaag aagtcagtaa cactgtttcc atttgaaaaa 2400

tcttggtact gctaaattat ttgatatgaa ctcaatccag catttgtagc aggttttgaa 2460

tgggtgggac tgggtgggga acagcattgg acattaatag ggcacttttc agacccattt 2520

tttaaagtgc tagaaaatgc tttttttaaa aaaaaaatac aagttttaaa atgaccactt 2580

actctttaat tatttactaa ttgctagtgt actcagtgtg gaaaagacta cagattacac 2640

actcttttca ttcacacttg tacatataga cagcaatgtt attaggagca ttaaattaaa 2700

aaactgaaca gcctaattta aatgtggctt gggcctggta gaagtttgac caaatggaat 2760

ggaggctgtg agcaatgtga ggattctatt tatttattta cgtttgataa aacttactgg 2820

aactagtact accatgcgta ttccctgtcc aaagcatcac tgctttggta tagtataagt 2880

tcatgaaatt ctggtgggta gaaagaaatt tttatttcta tcagcagtac taaaatgtat 2940

cagccaacca gagaacatca gtgactttaa cttctgcaga gtttgcccca gaattcagag 3000

ttctatttag aggaagttaa aacaacaaca aaaaacaacc atttgaaaaa tttttgtcac 3060

cagcaaaact tttcactaat tagtgatatg aaatgtgatt tttgtgttgt taaacttcag 3120

ctttggaaaa ctcagtctct ttcattatca tccattccaa tttgaaggag ttgggcagct 3180

aatttggtta aaggcagtct tgagggttag aagtattact tccttttcgg gttccagacc 3240

tagcttgtga ctgaaagttt tagaaaaagg aagtacatct atagccaaat tgataaggtt 3300

attactgtgt tttgcacaaa gtatattagc aaaagtattt gctggaatta ttggtagatg 3360

gcagtcccac tcctacacct gctttgtcag atacagctgg gttccctggc tgactctgta 3420

ccttacttac actacttact taatagaaac acaaacttgg aaattgtgcc agtggtccag 3480

ctggagcaca acgtttggtg aatatgctgt ttcctcagtt cagagaggta gcaactaggt 3540

aaactcctta taaaaagcaa atacctggat ttacaaaagt gaaagtagtt gttcacaaaa 3600

gaattcgcca tggaattctt tcagttacca agctctcctg gtaatgtttg tggttatatc 3660

atttacacaa aacttttcag gaacttctgt gttgtttaag caagatgtat ctgtactgat 3720

gtctcagtga atcagtctgt ttattaagca cttatcaggg cttccacaca cttatttatt 3780

ttgccctagt taatcctgtt gtttgctgcc attggcatga aatggccaac tgtggctgtt 3840

acagttcttt cattcaatta taacttgtaa accagtgact cctaatcttt ttcaagttaa 3900

gacaccttac cattgcttat ttggttttat gagacttgtt cctttttttc tccctaagga 3960

aaaagaaagc tttatgacat atttattttt ttaataaaac taagcaaaaa taaaacttat 4020

ggtaattctt taaaaaaaaa aaaaaaa 4047

<210> 111
<211> 434
<212> DNA
<213> Homo sapiens

<400> 111
tttttttttt ttttttttat tgtttaaaaa ttattttatt taatggtaag tttacataac 60

ttaattttca gttatggcta tttttaacaa ttagactgca aaaactaggc tctcatgagg 120

cactgcaagc ctgcataggc acacgactat ttatgaccca gatattacac gtctaggtat 180

atatccacag aaataggtac atgtgctctc aaaagacgtg cacaacaatt tttattgcag 240

cattttccat tatggtcaaa tagtagaaag actctagagg cccattagtg gtagaatgga 300

aaaataaatt gttacatttt catagcacag aatatgagtc agcaataaaa ttgaacaaat 360

gactgcttca gcacactcaa ggaacagaat attgagccag gtaccaaaga gtatgtactg 420

tccgattcca ttta 434

<210> 112
<211> 564
<212> DNA
<213> Homo sapiens

<400> 112
cattcttttt ctttattcag tggattgtta gttcttctgc tgttaggaag ccactatgtc 60

tggacgtgga aagcaaggcg gcaaagctcg ggcaaaagct aaaacgcgtt cttccagggc 120

cggtcttcag tttccagttg gccgtgtgca ccgcctcctc cgcaaaggca actactccga 180

acgagtcggg gccggcgctc cagtgtacct ggcagcggtg ctggaatatc tgacggccga 240

gatcttagag ctagctggca acgcggctcg cgacaataag aagacccgca tcatcccgcg 300

ccacctgcag ctagccatcc gcaacgacga ggagctaaat aagcttctag gtcgcgtgac 360

catcgcgcag ggcggtgtcc tgcccaacat ccaggccgta ttgctgccta agaagacgga 420

gagccaccat aaggccaagg gcaagtgaaa tgattactag tcaaatccgt cagtgatccc 480

gagtcccaga aaccaaaggc tcttttcaga gccacccacc ttttctgtaa agtgctggaa 540

tacacatacg atgcctgaaa tctc 564

<210>  113
<211>  3667
<212>  DNA
<213>  Homo sapiens

<400>  113
gagcttccgg ggcggccccg ggcaggtcgg cggcggcggc ccgcagtcgt ggaggagcgg      60

tgggagcgtc ggcggccgcg ggcgatgcaa cttccggacg ggactcccct ctgtccgcgc     120

ctcacatctc cccttcctct cgcctagtcc tgtgccgttt tccgtccgcg actcttccgg     180

cccagagctt tcggagtgcg gttgctcagg ggaagccgtc gccgcccccg cctcggggcc     240

gagtgagagt gcccgtcgcg tcgcgccgcg tcgcccccgg ggccgcctcc ttgccgccag     300

tggcgggctc cgttctccct cgaagcactc cccccagctc catgaatgga aatcggctcc     360

gcaggacccg ctggggccca gcccctactc atggtgccca gaagacctgg ctatggcacc     420

atgggcaaac ccattaaact gctggctaac tgttttcaag ttgaaatccc aaagattgat     480

gtctacctct atgaggtaga tattaaacca gacaagtgtc ctaggagagt gaacagggag     540

gtggttgact caatggttca gcattttaaa gtaactatat ttggagaccg tagaccagtt     600

tatgatggaa aaagaagtct ttacaccgcc aatccacttc ctgtggcaac tacaggggta     660

gatttagacg ttactttacc tggggaaggt ggaaaagatc gacctttcaa ggtgtcaatc     720

aaatttgtct ctcgggtgag ttggcaccta ctgcatgaag tactgacagg acggaccttg     780

cctgagccac tggaattaga caagccaatc agcactaacc ctgtccatgc cgttgatgtg     840

gtgctacgac atctgccctc catgaaatac acacctgtgg ggcgttcatt tttctccgct     900

ccagaaggat atgaccaccc tctgggaggg ggcagggaag tgtggtttgg attccatcag     960

tctgttcggc ctgccatgtg gaaaatgatg cttaatatcg atgtttctgc cactgccttc    1020

tacaaagcac aacctgtaat tcagttcatg tgtgaagttc ttgatattca taatattgat    1080

gagcaaccaa gacctctgac tgattctcat cgggtaaaat tcaccaaaga gataaaaggt    1140

ttgaaggttg aagtgactca ttgtggaaca atgagacgga aataccgtgt ttgtaatgta    1200

acaaggaggc ctgccagtca tcaaaccttt cctttacagt tagaaaacgg ccaaactgtg    1260

gagagaacag tagcgcagta tttcagagaa aagtatactc ttcagctgaa gtacccgcac    1320

cttccctgtc tgcaagtcgg gcaggaacag aaacacacct acctgccact agaagtctgt    1380

aatattgtgg cagggcaacg atgtatcaag aagctaacag acaatcagac ttccactatg    1440

atcaaggcaa cagcaagatc tgcaccagat agacaagagg aaattagcag attggtaaga    1500

agtgcaaatt atgaaacaga tccatttgtt caggagtttc aatttaaagt tcgggatgaa    1560

atggctcatg taactggacg cgtacttcca gcacctatgc tccagtatgg aggacggaat    1620

cggacagtag caacaccgag ccatggagta tgggacatgc gagggaaaca attccacaca 1680

ggagttgaaa tcaaaatgtg ggctatcgct tgttttgcca cacagaggca gtgcagagaa 1740

gaaatattga agggtttcac agaccagctg cgtaagattt ctaaggatgc agggatgccc 1800

atccagggcc agccatgctt ctgcaaatat gcacaggggg cagacagcgt agagcccatg 1860

ttccggcatc tcaagaacac atattctggc ctacagctta ttatcgtcat cctgccgggg 1920

aagacaccag tgtatgcgga agtgaaacgt gtaggagaca cacttttggg tatggctaca 1980

caatgtgttc aagtcaagaa tgtaataaaa acatctcctc aaactctgtc aaacttgtgc 2040

ctaaagataa atgttaaact cggagggatc aataatattc ttgtacctca tcaaagacct 2100

tctgtgttcc agcaaccagt gatctttttg ggagccgatg tcactcatcc acctgctggt 2160

gatggaaaga agccttctat tgctgctgtt gtaggtagta tggatgcaca cccaagcaga 2220

tactgtgcca cagtaagagt tcagagaccc cgacaggaga tcatccagga cttggcctcc 2280

atggtccggg aacttcttat tcaattttat aagtcaactc ggttcaagcc tactcgtatc 2340

atcttttatc gggatggtgt ttcagagggg cagtttaggc aggtattata ttatgaacta 2400

ctagcaattc gagaagcctg catcagtttg gagaaagact atcaacctgg aataacctac 2460

attgtagttc agaagagaca tcacactcga ttattttgtg ctgataggac agaaagggtt 2520

ggaagaagtg gcaatatccc agctggaaca acagttgata cagacattac acacccatat 2580

gagttcgatt tttacctctg tagccatgct ggaatacagg gtaccagtcg tccttcacac 2640

tatcatgttt tatgggatga taactgcttt actgcagatg aacttcagct gctaacttac 2700

cagctctgcc acacttacgt acgctgtaca cgatctgttt ctatacctgc accagcgtat 2760

tatgctcacc tggtagcatt tagagccaga tatcatcttg tggacaaaga acatgacagt 2820

gctgaaggaa gtcacgtttc aggacaaagc aatgggcgag atccacaagc tcttgccaag 2880

gctgtacaga ttcaccaaga taccttacgc acaatgtact tcgcttaaat agtccaagta 2940

tattctctga gaggaagtac tgaaagatga attgacatac aacgtatgtt tccagtgaag 3000

tcaattgagt aaggacacct ccagccatac agaaaccaac actgtgtggg ggccaaggtc 3060

tgatccttat gttaatacaa ggaagattgt ttacttcatc aaggaacaca gcatcattat 3120

gcaatatgaa accagccaac tgctttttgt gcggtctcct ataggaagta tcgcaattgt 3180

tttgttttca tttcttgtag tctaaccctt ttaatgcctt tacctcaagt tgcttggcag 3240

cacaactatc tttgcaaaaa aaagtaaaga aaaagtaaat gatggtttaa aaaatacaca 3300

ccttcatgaa taatcaaagt gatttttcag aattatgtgt gcaaaaaatt aatgtgcatt 3360

catatattct tgtaaaaggt gtctgtgtat ttttaaaata tatacatcca tacttcatat 3420

gcatatatat ctagatctgg attgataata gatatatatg tgtctgttat atattttaga 3480

gttcattcca ttggggaatt ttctttccct tttattctac ccccactacc gcctttattt 3540

ctctatttcc cttgccttca tcacctacat ttttttccca gtcctaccag tgacattcaa 3600

atgttgatgt atctggttcg tttgaatata aaatatggca aactaaaaaa aaaaaaaaaa 3660

aaaaaaa 3667

<210> 114
<211> 1771
<212> DNA
<213> Homo sapiens

<400> 114
ggcggccagg ccgggcgcgg agtgggcgcg cggggccgga ggagggccca gcgaccgcgg 60

caccgcctgt gcccgcccgc ccctccgcag ccgctactta agaggctcca gcgccggccc 120

cgccctagtg cgttacttac ctcgactctt agcttgtcgg ggacggtaac cgggacccgg 180

tgtctgctcc tgtcgccttc gcctcctaat ccctagccac tatgcgtgag tgcatctcca 240

tccacgttgg ccaggctggt gtccagattg gcaatgcctg ctgggagctc tactgcctgg 300

aacacggcat ccagcccgat ggccagatgc caagtgacaa gaccattggg ggaggagatg 360

actccttcaa caccttcttc agtgagacgg gcgctggcaa gcacgtgccc cgggctgtgt 420

ttgtagactt ggaacccaca gtcattgatg aagttcgcac tggcacctac cgccagctct 480

tccaccctga gcagctcatc acaggcaagg aagatgctgc caataactat gcccgagggc 540

actacaccat tggcaaggag atcattgacc ttgtgttgga ccgaattcgc aagctggctg 600

accagtgcac cggtcttcag ggcttcttgg ttttccacag ctttggtggg ggaactggtt 660

ctgggttcac ctccctgctc atggaacgtc tctcagttga ttatggcaag aagtccaagc 720

tggagttctc catttaccca gcaccccagg tttccacagc tgtagttgag ccctacaact 780

ccatcctcac cacccacacc accctggagc actctgattg tgccttcatg gtagacaatg 840

aggccatcta tgacatctgt cgtagaaacc tcgatatcga gcgcccaacc tacactaacc 900

ttaaccgcct tattagccag attgtgtcct ccatcactgc ttccctgaga tttgatggag 960

ccctgaatgt tgacctgaca gaattccaga ccaacctggt gccctacccc cgcatccact 1020

tccctctggc cacatatgcc cctgtcatct ctgctgagaa agcctaccat gaacagcttt 1080

ctgtagcaga gatcaccaat gcttgctttg agccagccaa ccagatggtg aaatgtgacc 1140

ctcgccatgg taaatacatg gcttgctgcc tgttgtaccg tggtgacgtg gttcccaaag 1200

atgtcaatgc tgccattgcc accatcaaaa ccaagcgcag catccagttt gtggattggt 1260

gccccactgg cttcaaggtt ggcatcaact accagcctcc cactgtggtg cctggtggag 1320

acctggccaa ggtacagaga gctgtgtgca tgctgagcaa caccacagcc attgctgagg 1380

cctgggctcg cctggaccac aagtttgacc tgatgtatgc caagcgtgcc tttgttcact 1440

ggtacgtggg tgaggggatg gaggaaggcg agttttcaga ggcccgtgaa gatatggctg 1500

cccttgagaa ggattatgag gaggttggtg tggattctgt tgaaggagag ggtgaggaag 1560

aaggagagga atactaatta tccattcctt ttggccctgc agcatgtcat gctcccagaa 1620

tttcagcttc agcttaactg acagacgtta aagctttctg gttagattgt tttcacttgg 1680

tgatcatgtc ttttccatgt gtacctgtaa tatttttcca tcatatctca aagtaaagtc 1740

attaacatca aaaaaaaaaa aaaaaaaaaa a 1771

<210> 115
<211> 2543
<212> DNA
<213> Homo sapiens

<400> 115
cggacccgag gagcaggaag cggcggctcc ttcggccacc caggcagcag ccacagcggg 60

gagtgcgcgg cgcggggaca ggaagagagg ggcaatggct gccgacccca ccgagctgcg 120

gctgggcagc ctccccgtct tcacccgcga cgacttcgag ggcgactggc gcctagtggc 180

cagcggcggc ttcagccagg tgttccaggc gcggcacagg cgctggcgga cggagtacgc 240

catcaagtgc gccccctgcc ttccacccga cgccgccagc tctgatgtga attacctcat 300

tgaagaagct gccaaaatga agaagatcaa gtttcagcac atcgtgtcta tctacggggt 360

gtgcaagcag cccctgggta ttgtgatgga gtttatggcc aacggctccc tggagaaggt 420

gctgtccacc cacagcctct gctggaagct caggttccgc atcatccatg agaccagctt 480

ggccatgaac ttcctgcaca gcattaagcc gcctctgctc cacctggacc tcaagccggg 540

caacatactc ctggacagca acatgcatgt caaaatttca gacttcggcc tgtccaagtg 600

gatggaacag tccacccgga tgcagtacat cgagaggtcg gctctgcggg gcatgctcag 660

ctacatcccc cctgagatgt tcctggagag taacaaggcc ccaggaccta aatatgatgt 720

gtacagcttt gcaattgtca tctgggagct actcactcag aagaaaccat actcaggttt 780

caacatgatg atgattatta tccgagtggc ggcaggcatg cggccctccc tacagcctgt 840

ctctgaccaa tggccaagcg aggcccagca gatggtggac ctgatgaaac gctgctggga 900

ccaggacccc aagaagaggc catgctttct agacattacc atcgagacag acatactgct 960

gtcactgctg cagagtcgtg tggcagtccc agagagcaag gccctggcca ggaaggtgtc 1020

ctgcaagctg tcgctgcgcc agcccgggga ggttaatgag gacatcagcc aggaactgat 1080

ggacagtgac tcaggaaact acctgaagcg ggcccttcag ctctccgacc gtaagaattt 1140

ggtcccgaga gatgaggaac tgtgtatcta tgagaacaag gtcacccccc tccacttcct 1200

ggtggcccag ggcagtgtgg agcaggtgag gttgctgctg gcccacgagg tagacgtgga 1260

ctgccagacg gcctctggat acacgcccct cctgatcgcc gcccaggacc agcaacccga 1320

cctctgtgcc ctgcttttgg cacatggtgc tgatgccaac cgagtggatg aggatggctg 1380

ggccccactg cactttgcag cccagaatgg ggatgacggc actgcgcgcc tgctcctgga 1440

ccacggggcc tgtgtggatg cccaggaacg tgaagggtgg acccctcttc acctggctgc 1500

acagaataac tttgagaatg tggcacggct tctggtctcc cgtcaggctg accccaacct 1560

gcatgaggct gagggcaaga cccccctcca tgtggccgcc tactttggcc atgttagcct 1620

ggtcaagctg ctgaccagcc agggggctga gttggatgct cagcagagaa acctgagaac 1680

accactgcac ctggcagtag agcggggcaa agtgagggcc atccaacacc tgctgaagag 1740

tggagcggtc cctgatgccc ttgaccagag cggctacggc ccactgcaca ctgcagctgc 1800

caggggcaaa tacctgatct gcaagatgct gctcaggtac ggagccagcc ttgagctgcc 1860

cacccaccag ggctggacac ccctgcatct agcagcctac aagggccacc tggagatcat 1920

ccatctgctg gcagagagcc acgcaaacat gggtgctctt ggagctgtga actggactcc 1980

cctgcaccta gctgcacgcc acggggagga ggcggtggtg tcagcactgc tgcagtgtgg 2040

ggctgacccc aatgctgcag agcagtcagg ctggacaccc ctccacctgg cggtccagag 2100

gagcaccttc ctgagtgtca tcaacctcct agaacatcac gcaaatgtcc acgcccgcaa 2160

caaggtgggc tggacacccg cccacctggc cgccctcaag ggcaacacag ccatcctcaa 2220

agtgctggtc gaggcaggcg cccagctgga cgtccaggat ggagtgagct gcacacccct 2280

gcaactggcc ctccgcagcc gaaagcaggg catcatgtcc ttcctagagg gcaaggagcc 2340

gtcagtggcc actctgggtg gttctaagcc aggagccgag atggaaattt agacaacttg 2400

gccagccgtg gtggctcacg tctgtaatcc cagcactttg ggaggctgag gcaggcagat 2460

cacctgatat caagagtttg aggccagcct ggccaacatg gcaaaaccct gtctctgcta 2520

aaaatacaaa atttagctgg gta 2543

<210> 116
<211> 2816
<212> DNA
<213> Homo sapiens

<400> 116
attggggaca atcctgcggg gaggtgctga ggagggcagc tacgacaact ggccccacac 60
caggaaaagc tgggggccgc tgagcccagg ccaccaacgg gagctgtgga cccagcctga 120
cccctggacc gaggtgcttt cagggcacaa gggggatgcg ggagcctgtg gctgctgttg 180
cttctgctct cagttcataa acgcacgctg tgcacatccc ctgtgcttgg caaggggcct 240
ggatagaagg gccagtgagg agatgcccat cctccaggca ctgtgcctcc tcccaaaggt 300
cagcaccccg agcatcactg tgccctcccc acaaaggtca gcagccctga gcatcactgt 360
gccctcccct caaaggtcag cggccccgag catcactgtg ccctccccac aaaggtcagc 420
accccgagca tcactgtgcc ctccccacaa aggtcagcac cccgagcatc actgtgccct 480
ccctcccaaa ggtcagcacc cggagcatca ctgtgccctc cccacaaagg tcagcacccc 540
gagcatcact gtgccctccc cacaaaggtc accacagatg tccctgagct ctgcagcacg 600
tgggtccaat acagatgtgg caggtttgtc tgttggggag tggcctggct ggcagctgtg 660
gggagaaggc caggacgggg cacagcagag gcctcacctg cccagcgggg gctctggggc 720
tggggtggct cctcagagat tgcccaagtc cagagcttgc atcctatgca gccgtcacgg 780
ggcacagggc ccctgggtta ctggcaggtc cgtcagccat agccactgcc ccatccaggg 840
cctgctggat ttgcagaggc cagacttggg aactgactgg gggaggacca ggcccctctg 900
cacccctcag gatttatgtg ggggccggcc tctgccgtcc acctggggcg tgacaatgca 960
tttgattcac tgtctctctg tgtcactgtc tctatgtctg tctttatctc actgtgtcta 1020
ggtttctgtc tctcccactg tctcccttgc tcagctgggt gggaaaggga cattctggaa 1080
ggttccacat ggtcttccct acaggtcagg acaactgggc tattccagtg acgtattggg 1140
gatctgggaa atgacctctg ggagttccgt gagctccgtc tggaaggtcc ccattcattt 1200
cccgttccct gctctgctct atgggggcgc ggcggggctg cagttccctg atgctggcgt 1260
ctgctctgtc cccagcccac tgccctgacc gtttggaccg acccttcctc cccagcgccc 1320
cttgggaggg ccagggggac ccttgcccaa ggcttctgtg catttagggt tctttcttcc 1380
cctctcctgt ctggattctg catctggaac ctgccccagg ggggaggctg cgtgggatgc 1440
tgggtttgct gggcagctgc ctgtggcccc agcctccgtc ttgactgcct tagtggggtg 1500
ggtggagctg ctgcccacct ctcctgcccc cggggcttgg gtgctaccgg ctttcactcc 1560
cacctctgtg gggcaggccc cggtacacca ctcagtctgc tgctcagccc cacaacggcc 1620
ctgccttcct tctgacagtc aggccccctt ctgccatcag gggcccggct ctgtgatggt 1680
gtctggccgc cagccctgcc cacgccgccc ggccacccca gcttccaggg aggctgctgc 1740

tgcccactct tcccagtggc cagtgcaggg tctcctgggc ccccgggagc aggtcagccg 1800

gcagtgtcca gccttacacc acgcctacca gcacggtcac ttctcagggc ctttggtccc 1860

cggcgtgggc tgagctgggc tctcgctctc ctgcgtcact ggcattgctt atgtgctgtg 1920

cctgtctccc ttgacggctc tcagccctgc aggaccatgg acgtcccttc cctctctcag 1980

caggaaaatc tccatgatgc cagcaggcgt gtccacagag gaaggggcga agaaaatgtc 2040

gaatggacag gcgacctgca tcctgcccag ctcggaagag gaggacgtcc tgagattcgc 2100

cacagcctgg aggcgattgc gctcgtgaca aaagccagac acagaaagac aaataccacg 2160

ttctaatttg tgcatgggag ctaaaataaa cccaggtgct ccctgctgga aagccacacg 2220

cggcagagga gagctggcag gaggaaaagc gggttcgagt cagcagcctt tggagacggc 2280

agactgagag tgtcacagag accatctcaa gtctgcacga attccaggct cttttatgtt 2340

aagggcaggg ggacggggag ggggttggga tcaagaggtg acaggtgacc gcacacatgt 2400

gggtgccagc gagggtccga ggaggctggc gatgccttcg tccttggtca ggtcacgagc 2460

acctgtgaat ccacagcaga acagctgttc acagcttccc ctttcatccc ggagtgagtt 2520

tcaaaacctg caccacgact gcctctgtgt attttctccg tcctctagaa gatcctaggc 2580

tccgtgcagg aatgggtgaa ggccccttac acaaaaacaa agtcaggtcc tgagttcttt 2640

tgctgtttct ttgctttctc ctgcaaagtc actcgaaagg tgactggcgg aggtgaggct 2700

gcgataatta gcttgattgt ggtgaccctt ccacaaagca cgtgtatgtc ggcatattca 2760

ctgggtcatg cacctcgaat acatattttt acttgtcaaa tacatgataa taaagg 2816

<210> 117
<211> 2166
<212> DNA
<213> Homo sapiens

<400> 117
agtacagctc cggccgccgc gccgcctggc tttcgtattc gttgttctcg gcgggctgtg 60

gggcctccgc gccgcggccg ttagtcatgt cggattctgg aagttacggt cagtctgggg 120

gtgagcagca aagttattct acctatggaa atccaggcag ccaaggctat ggacaagcat 180

cacaaagcta ttctggctat gggcaaacga ctgattcctc ttatggacag aactacagcg 240

gttactccag ttatggacaa agttattcac agtcctatgg tggttatgag aatcaaaagc 300

agagctcata tagccagcaa ccatataata accagggaca gcagcaaaac atggaatcat 360

caggaagcca aggtggaaga gcaccttcct atgaccagcc agactatggt caacaagatt 420

catatgacca gcagtcaggc tatgatcaac atcaaggctc atatgatgag cagtcaaatt 480

atgatcagca gcatgattcc tatagtcaaa accagcagtc ctatcattca caaaggaaaa 540

actacagcca ccacacacaa gatgaccgtc gtgatgtgag taggtatgga gaagataata 600

gaggatatgg cgggtcacag ggaggaggta gagggcgtgg gggatatgac aaggatggaa 660

gaggtcctat gacaggatca agtggtggtg accgcggtgg cttcaaaaat tttggtggtc 720

acagggatta tggacccaga acagatgctg attcagaatc tgataattca gataacaaca 780

caatctttgt gcaaggactt ggggagggtg tgtctacaga tcaagttggg gagttcttta 840

aacaaatagg aattatcaag acaaataaga agaccggaaa accaatgata aatctttata 900

cagacaagga cacaggaaag ccaaaggggg aggcaacagt gtcatttgat gaccctcctt 960

cagctaaggc agccattgac tggtttgatg gaaaagaatt ccatggcaac atcattaaag 1020

tgtcctttgc cactagaaga cctgaattca tgagaggagg tggaagtgga ggtgggcggc 1080

gaggccgtgg aggatataga ggtcgtggag gctttcaagg gagaggtgga gaccccaaaa 1140

gtggggattg ggtttgccct aatccgtcat gcggaaatat gaactttgct cgaaggaatt 1200

cctgcaatca gtgcaatgag cctagaccag aggactctcg tccctcagga ggagatttcc 1260

gggggagagg ctacggtgga gagaggggct acagaggtcg tgggggcaga ggtggagacc 1320

gaggcggcta tggtggagac agaagtgggg gtggctatgg tggagacaga agcagcggtg 1380

gtggctacag cggagataga agtgggggcg gctatggtgg agacagaagt gggggtggct 1440

atggtgggga cagaggcggc ggctatggtg gggacagagg aggcggctat ggaggagacc 1500

gaggaggtgg ctatggagga gatcgaggtg gctatggagg agaccgaggt ggaggctatg 1560

gtggagaccg aggaggctat ggaggagatc gaggaggtta cggaggagat cgaggaggtt 1620

atggaggaga tcgaggaggc tatggaggag acagaagccg ggggggctat ggaggagacc 1680

gtggtggtgg cagtggctac ggtggagacc gaagtggagg ctatggagga gacaggagtg 1740

gtggcggcta tggaggagac cgaggtgggg gctacggagg agaccgaggt ggctatggag 1800

gcaaaatggg aggaagaaac gactacagaa atgatcagcg caaccgacca tactgatgac 1860

tgttttgaat gttcctttgt ctctgacatg atccatagtg aaattgccag agttttgcct 1920

gctgctttcc tcgtggcctc ttcttgggta gtgaaattaa gtgacatttg gatttttatt 1980

tgggtgggag ggctgggaca gtttttcttc tagaaatgtc tgttgagatt tcccccttta 2040

gtttccaacc ttctccccaa cccttggagc taaatgcgtt gtaaaatatt gccaaaatga 2100

aaagtgtttt gtaatactgc aataaaggct gcttgttttt gtggaaaaaa aaaaaaaaaa 2160

aaaaaa 2166

<210>  118
<211>  2515
<212>  DNA
<213>  Homo sapiens

<400>  118
   ccctatcccg tcttgctctg cggcgcaggg gcaagatggc tgctgagaag caggtcccag      60
   gcggcggcgg cggcggcggc agtggcggcg gcggtggcag tggcggcggc ggtagcggcg     120
   gtggacgtgg tgccggaggg gaagaaaata aagaaaacga acgcccttcg gccggatcga     180
   aggcaaacaa agaatttggg gatagcctga gtttggagat tcttcagatt attaaggaat     240
   cccagcagca gcatggttta cggcatggag attttcagag gtacaggggc tactgttccc     300
   gtagacaaag acgtcttcga aaaacactca acttcaagat gggtaacaga cacaaattca     360
   cagggaagaa agtgactgaa gagcttctga ccgataatag atacttgctt ctggttctga     420
   tggatgctga aagagcctgg agctacgcca tgcagctgaa acaggaagcc aacactgaac     480
   cccgaaaacg gtttcacttg ttatctcgcc tacgcaaagc cgtgaagcat gcagaggaat     540
   tggaacgctt gtgtgagagc aatcgcgtgg atgccaagac caaattagag gctcaggctt     600
   acacagctta cctctcagga atgctacgtt ttgaacatca agaatggaaa gctgccattg     660
   aggcttttaa caaatgcaaa actatctatg agaagctagc cagtgctttc acagaggagc     720
   aggctgtgct gtataaccaa cgtgtggaag agatttcacc caacatccgc tattgtgcat     780
   ataatattgg ggaccagtca gccatcaatg aactcatgca gatgagattg aggtctgggg     840
   gcactgaggg tctcttggct gaaaaattgg aggctttgat cactcagact cgagccaaac     900
   aggcagctac catgagtgaa gtggagtgga gagggagaac ggttccagtg aagattgaca     960
   aagtgcgcat tttcttatta ggactggctg ataacgaagc agctattgtc caggctgaaa    1020
   gcgaagaaac taaggagcgc ctgtttgaat caatgctcag cgagtgtcgg gacgccatcc    1080
   aggtggttcg ggaggagctc aagccagatc agaaacagag agattatatc cttgaaggag    1140
   agccagggaa ggtgtctaat cttcaatact tgcatagcta cctgacttac atcaagctat    1200
   caacggcaat caagcgtaat gagaacatgg ccaaaggtct gcagagggct ctgctgcagc    1260
   agcagccaga ggatgacagc aagcgctcac cccggcccca ggacctgatc cgactctatg    1320
   acatcatctt acagaatctg gtggaattgc tccagcttcc tggtttagag gaagacaaag    1380
   ccttccagaa agagataggc ctcaagactc tggtgttcaa agcttacagg tgttttttca    1440
   ttgctcagtc ctatgtgctg gtgaagaagt ggagcgaagc ccttgtcctg tatgacagag    1500
   tcctgaaata tgcaaatgaa gtaaattctg atgctggcgc cttcaagaac agcctaaagg    1560
   acctgcctga tgtgcaagag ctcatcactc aagtgcggtc agagaagtgc tccctgcagg 1620
   ccgcagccat ccttgatgca aacgacgctc atcaaacaga gacctcctcc tcccaagtca 1680
   aggacaataa gcctctggtt gaacggtttg agacattctg cctggaccct tcccttgtca 1740
   ccaagcaagc caaccttgtg cacttcccac caggcttcca gcccattccc tgcaagcctt 1800
   tgttctttga cctggccctc aaccatgtgg ctttcccacc ccttgaggac aagttggaac 1860
   agaagaccaa gagtggcctc actggataca tcaagggcat ctttggattc aggagctaac 1920
   caggctcttc ctcgggggcg ggggagattc tgactcttaa tctgtattgt gagaaaatcc 1980
   cagcaagttc catgatatta aatccaggtc tgcattggcc cggggcaaga gtttaacatc 2040
   ttcggccctg cattcctaca tcttgtgtct gtacacgttc ttaagcagcg tgtcaggaga 2100
   gcaccctgtt gtcttctggt aaatgtgtgc agggtcatcc tgtctcctgt acctcctggg 2160
   aaaggggccg ctgctgtctg gtgccctgtg agctgtgatt gattgccttt ggtcagtaat 2220
   gcgttcagga gtccacacca ggcacagatg gggccttgaa acgctttgtc atgcttcttc 2280
   agtaccatgg atttgaaatg aactcatcct tgctgtgagc atccaggagc ccttgagaag 2340
   tttatctatg actatgaaac tggcaacgtc accccagaat tacggtcagc cttattcccc 2400
   ttcacctccc agtgaacgct aagaagtttc agacaagcag agagctctat ttttagaaga 2460
   aatatgttac actcagaaat gatgaaacca aatcttatat taaaaggcaa agatg 2515

<210> 119
<211> 10515
<212> DNA
<213> Homo sapiens

<400> 119
   ggaggaggga gagcacaggc tttgaccgat agtaacctct gcgctcggtg cagccgaatc 60
   tataaaagga actagtcccg gcaaaaaccc cgtaattgcg agcgagagtg agtggggccg 120
   ggacccgcag agccgagccg acccttctct cccgggctgc ggcagggcag ggcggggagc 180
   tccgcgcacc aacagagccg gttctcaggg cgctttgctc cttgtttttt ccccggttct 240
   gttttctccc cttctccgga aggcttgtca aggggtagga gaaagagacg caaacacaaa 300
   agtggaaaac agttaatgac cagccacggc gtccctgctg tgagctctgg ccgctgcctt 360
   ccagggctcc cgagccacac gctgggggtg ctggctgagg gaacatggct tgttggcctc 420
   agctgaggtt gctgctgtgg aagaacctca ctttcagaag aagacaaaca tgtcagctgc 480
   tgctggaagt ggcctggcct ctatttatct tcctgatcct gatctctgtt cggctgagct 540
   acccacccta tgaacaacat gaatgccatt ttccaaataa agccatgccc tctgcaggaa 600
   cacttccttg ggttcagggg attatctgta atgccaacaa cccctgtttc cgttacccga 660
   ctcctgggga ggctcccgga gttgttggaa actttaacaa atccattgtg gctcgcctgt 720
   tctcagatgc tcggaggctt cttttataca gccagaaaga caccagcatg aaggacatgc 780
   gcaaagttct gagaacatta cagcagatca agaaatccag ctcaaacttg aagcttcaag 840
   atttcctggt ggacaatgaa accttctctg ggttcctgta tcacaacctc tctctcccaa 900
   agtctactgt ggacaagatg ctgagggctg atgtcattct ccacaaggta tttttgcaag 960
   gctaccagtt acatttgaca agtctgtgca atggatcaaa atcagaagag atgattcaac 1020
   ttggtgacca agaagtttct gagctttgtg gcctaccaag ggagaaactg gctgcagcag 1080
   agcgagtact tcgttccaac atggacatcc tgaagccaat cctgagaaca ctaaactcta 1140
   catctccctt cccgagcaag gagctggctg aagccacaaa aacattgctg catagtcttg 1200
   ggactctggc ccaggagctg ttcagcatga gaagctggag tgacatgcga caggaggtga 1260
   tgtttctgac caatgtgaac agctccagct cctccaccca aatctaccag gctgtgtctc 1320
   gtattgtctg cgggcatccc gagggagggg ggctgaagat caagtctctc aactggtatg 1380
   aggacaacaa ctacaaagcc ctctttggag gcaatggcac tgaggaagat gctgaaacct 1440
   tctatgacaa ctctacaact ccttactgca atgatttgat gaagaatttg gagtctagtc 1500
   ctctttcccg cattatctgg aaagctctga agccgctgct cgttgggaag atcctgtata 1560
   cacctgacac tccagccaca aggcaggtca tggctgaggt gaacaagacc ttccaggaac 1620
   tggctgtgtt ccatgatctg gaaggcatgt gggaggaact cagccccaag atctggacct 1680
   tcatggagaa cagccaagaa atggaccttg tccggatgct gttggacagc agggacaatg 1740
   accacttttg ggaacagcag ttggatggct tagattggac agcccaagac atcgtggcgt 1800
   ttttggccaa gcacccagag gatgtccagt ccagtaatgg ttctgtgtac acctggagag 1860
   aagctttcaa cgagactaac caggcaatcc ggaccatatc tcgcttcatg gagtgtgtca 1920
   acctgaacaa gctagaaccc atagcaacag aagtctggct catcaacaag tccatggagc 1980
   tgctggatga gaggaagttc tgggctggta ttgtgttcac tggaattact ccaggcagca 2040
   ttgagctgcc ccatcatgtc aagtacaaga tccgaatgga cattgacaat gtggagagga 2100
   caaataaaat caaggatggg tactgggacc ctggtcctcg agctgacccc tttgaggaca 2160
   tgcggtacgt ctgggggggc ttcgcctact tgcaggatgt ggtggagcag gcaatcatca 2220
   gggtgctgac gggcaccgag aagaaaactg gtgtctatat gcaacagatg ccctatccct 2280
   gttacgttga tgacatcttt ctgcgggtga tgagccggtc aatgcccctc ttcatgacgc 2340
   tggcctggat ttactcagtg gctgtgatca tcaagggcat cgtgtatgag aaggaggcac 2400
   ggctgaaaga gaccatgcgg atcatgggcc tggacaacag catcctctgg tttagctggt 2460
   tcattagtag cctcattcct cttcttgtga gcgctggcct gctagtggtc atcctgaagt 2520
   taggaaacct gctgccctac agtgatccca gcgtggtgtt tgtcttcctg tccgtgtttg 2580
   ctgtggtgac aatcctgcag tgcttcctga ttagcacact cttctccaga gccaacctgg 2640
   cagcagcctg tgggggcatc atctacttca cgctgtacct gccctacgtc ctgtgtgtgg 2700
   catggcagga ctacgtgggc ttcacactca agatcttcgc tagcctgctg tctcctgtgg 2760
   cttttgggtt tggctgtgag tactttgccc tttttgagga gcagggcatt ggagtgcagt 2820
   gggacaacct gtttgagagt cctgtggagg aagatggctt caatctcacc acttcggtct 2880
   ccatgatgct gtttgacacc ttcctctatg gggtgatgac ctggtacatt gaggctgtct 2940
   ttccaggcca gtacggaatt cccaggccct ggtattttcc ttgcaccaag tcctactggt 3000
   ttggcgagga aagtgatgag aagagccacc ctggttccaa ccagaagaga atatcagaaa 3060
   tctgcatgga ggaggaaccc acccacttga agctgggcgt gtccattcag aacctggtaa 3120
   aagtctaccg agatgggatg aaggtggctg tcgatggcct ggcactgaat ttttatgagg 3180
   gccagatcac ctccttcctg ggccacaatg gagcggggaa gacgaccacc atgtcaatcc 3240
   tgaccgggtt gttccccccg acctcgggca ccgcctacat cctgggaaaa gacattcgct 3300
   ctgagatgag caccatccgg cagaacctgg gggtctgtcc ccagcataac gtgctgtttg 3360
   acatgctgac tgtcgaagaa cacatctggt tctatgcccg cttgaaaggg ctctctgaga 3420
   agcacgtgaa ggcggagatg gagcagatgg ccctggatgt tggtttgcca tcaagcaagc 3480
   tgaaaagcaa aacaagccag ctgtcaggtg gaatgcagag aaagctatct gtggccttgg 3540
   cctttgtcgg gggatctaag gttgtcattc tggatgaacc cacagctggt gtggaccctt 3600
   actcccgcag gggaatatgg gagctgctgc tgaaataccg acaaggccgc accattattc 3660
   tctctacaca ccacatggat gaagcggacg tcctggggga caggattgcc atcatctccc 3720
   atgggaagct gtgctgtgtg ggctcctccc tgtttctgaa gaaccagctg ggaacaggct 3780
   actacctgac cttggtcaag aaagatgtgg aatcctccct cagttcctgc agaaacagta 3840
   gtagcactgt gtcatacctg aaaaaggagg acagtgtttc tcagagcagt tctgatgctg 3900
   gcctgggcag cgaccatgag agtgacacgc tgaccatcga tgtctctgct atctccaacc 3960
   tcatcaggaa gcatgtgtct gaagcccggc tggtggaaga catagggcat gagctgacct 4020
   atgtgctgcc atatgaagct gctaaggagg gagcctttgt ggaactcttt catgagattg 4080
   atgaccggct ctcagacctg ggcatttcta gttatggcat ctcagagacg accctggaag 4140
   aaatattcct caaggtggcc gaagagagtg gggtggatgc tgagacctca gatggtacct 4200
   tgccagcaag acgaaacagg cgggccttcg gggacaagca gagctgtctt cgcccgttca 4260
   ctgaagatga tgctgctgat ccaaatgatt ctgacataga cccagaatcc agagagacag 4320
   acttgctcag tgggatggat ggcaaagggt cctaccaggt gaaaggctgg aaacttacac 4380
   agcaacagtt tgtggccctt ttgtggaaga gactgctaat tgccagacgg agtcggaaag 4440
   gattttttgc tcagattgtc ttgccagctg tgtttgtctg cattgccctt gtgttcagcc 4500
   tgatcgtgcc accctttggc aagtacccca gcctggaact tcagccctgg atgtacaacg 4560
   aacagtacac atttgtcagc aatgatgctc ctgaggacac gggaaccctg gaactcttaa 4620
   acgccctcac caaagaccct ggcttcggga cccgctgtat ggaaggaaac ccaatcccag 4680
   acacgccctg ccaggcaggg gaggaagagt ggaccactgc cccagttccc cagaccatca 4740
   tggacctctt ccagaatggg aactggacaa tgcagaaccc ttcacctgca tgccagtgta 4800
   gcagcgacaa aatcaagaag atgctgcctg tgtgtccccc aggggcaggg gggctgcctc 4860
   ctccacaaag aaaacaaaac actgcagata tccttcagga cctgacagga agaaacattt 4920
   cggattatct ggtgaagacg tatgtgcaga tcatagccaa aagcttaaag aacaagatct 4980
   gggtgaatga gtttaggtat ggcggctttt ccctgggtgt cagtaatact caagcacttc 5040
   ctccgagtca agaagttaat gatgccatca aacaaatgaa gaaacaccta aagctggcca 5100
   aggacagttc tgcagatcga tttctcaaca gcttgggaag atttatgaca ggactggaca 5160
   ccaaaaataa tgtcaaggtg tggttcaata acaagggctg gcatgcaatc agctctttcc 5220
   tgaatgtcat caacaatgcc attctccggg ccaacctgca aaagggagag aaccctagcc 5280
   attatggaat tactgctttc aatcatcccc tgaatctcac caagcagcag ctctcagagg 5340
   tggctctgat gaccacatca gtggatgtcc ttgtgtccat ctgtgtcatc tttgcaatgt 5400
   ccttcgtccc agccagcttt gtcgtattcc tgatccagga gcgggtcagc aaagcaaaac 5460
   acctgcagtt catcagtgga gtgaagcctg tcatctactg gctctctaat tttgtctggg 5520
   atatgtgcaa ttacgttgtc cctgccacac tggtcattat catcttcatc tgcttccagc 5580
   agaagtccta tgtgtcctcc accaatctgc ctgtgctagc ccttctactt ttgctgtatg 5640
   ggtggtcaat cacacctctc atgtacccag cctcctttgt gttcaagatc cccagcacag 5700
   cctatgtggt gctcaccagc gtgaacctct tcattggcat taatggcagc gtggccacct 5760
   ttgtgctgga gctgttcacc gacaataagc tgaataatat caatgatatc ctgaagtccg 5820
   tgttcttgat cttcccacat ttttgcctgg gacgagggct catcgacatg gtgaaaaacc 5880
   aggcaatggc tgatgccctg gaaaggtttg gggagaatcg ctttgtgtca ccattatctt 5940
   gggacttggt gggacgaaac ctcttcgcca tggccgtgga aggggtggtg ttcttcctca 6000
   ttactgttct gatccagtac agattcttca tcaggcccag acctgtaaat gcaaagctat 6060
   ctcctctgaa tgatgaagat gaagatgtga ggcgggaaag acagagaatt cttgatggtg 6120
   gaggccagaa tgacatctta gaaatcaagg agttgacgaa gatatataga aggaagcgga 6180
   agcctgctgt tgacaggatt tgcgtgggca ttcctcctgg tgagtgcttt gggctcctgg 6240
   gagttaatgg ggctggaaaa tcatcaactt tcaagatgtt aacaggagat accactgtta 6300
   ccagaggaga tgctttcctt aacaaaaata gtatcttatc aaacatccat gaagtacatc 6360
   agaacatggg ctactgccct cagtttgatg ccatcacaga gctgttgact gggagagaac 6420
   acgtggagtt ctttgccctt ttgagaggag tcccagagaa agaagttggc aaggttggtg 6480
   agtgggcgat tcggaaactg ggcctcgtga agtatggaga aaaatatgct ggtaactata 6540
   gtggaggcaa caaacgcaag ctctctacag ccatggcttt gatcggcggg cctcctgtgg 6600
   tgtttctgga tgaacccacc acaggcatgg atcccaaagc ccggcggttc ttgtggaatt 6660
   gtgccctaag tgttgtcaag gaggggagat cagtagtgct tacatctcat agtatggaag 6720
   aatgtgaagc tctttgcact aggatggcaa tcatggtcaa tggaaggttc aggtgccttg 6780
   gcagtgtcca gcatctaaaa aataggtttg gagatggtta tacaatagtt gtacgaatag 6840
   cagggtccaa cccggacctg aagcctgtcc aggatttctt tggacttgca tttcctggaa 6900
   gtgttctaaa agagaaacac cggaacatgc tacaatacca gcttccatct tcattatctt 6960
   ctctggccag gatattcagc atcctctccc agagcaaaaa gcgactccac atagaagact 7020
   actctgtttc tcagacaaca cttgaccaag tatttgtgaa ctttgccaag gaccaaagtg 7080
   atgatgacca cttaaaagac ctctcattac acaaaaacca gacagtagtg gacgttgcag 7140
   ttctcacatc ttttctacag gatgagaaag tgaaagaaag ctatgtatga agaatcctgt 7200
   tcatacgggg tggctgaaag taaagaggaa ctagactttc ctttgcacca tgtgaagtgt 7260
   tgtggagaaa agagccagaa gttgatgtgg gaagaagtaa actggatact gtactgatac 7320
   tattcaatgc aatgcaattc aatgcaatga aaacaaaatt ccattacagg ggcagtgcct 7380
   ttgtagccta tgtcttgtat ggctctcaag tgaaagactt gaatttagtt ttttacctat 7440
   acctatgtga aactctatta tggaacccaa tggacatatg ggtttgaact cacacttttt 7500
   tttttttttt tgttcctgtg tattctcatt ggggttgcaa caataattca tcaagtaatc 7560
   atggccagcg attattgatc aaaatcaaaa ggtaatgcac atcctcattc actaagccat 7620
   gccatgccca ggagactggt ttcccggtga cacatccatt gctggcaatg agtgtgccag 7680
   agttattagt gccaagtttt tcagaaagtt tgaagcacca tggtgtgtca tgctcacttt 7740
   tgtgaaagct gctctgctca gagtctatca acattgaata tcagttgaca gaatggtgcc 7800
   atgcgtggct aacatcctgc tttgattccc tctgataagc tgttctggtg gcagtaacat 7860
   gcaacaaaaa tgtgggtgtc tccaggcacg ggaaacttgg ttccattgtt atattgtcct 7920
   atgcttcgag ccatgggtct acagggtcat ccttatgaga ctcttaaata tacttagatc 7980
   ctggtaagag gcaaagaatc aacagccaaa ctgctggggc tgcaagctgc tgaagccagg 8040
   gcatgggatt aaagagattg tgcgttcaaa cctagggaag cctgtgccca tttgtcctga 8100
   ctgtctgcta acatggtaca ctgcatctca agatgtttat ctgacacaag tgtattattt 8160
   ctggcttttt gaattaatct agaaaatgaa aagatggagt tgtattttga caaaaatgtt 8220
   tgtacttttt aatgttattt ggaattttaa gttctatcag tgacttctga atccttagaa 8280
   tggcctcttt gtagaaccct gtggtataga ggagtatggc cactgcccca ctatttttat 8340
   tttcttatgt aagtttgcat atcagtcatg actagtgcct agaaagcaat gtgatggtca 8400
   ggatctcatg acattatatt tgagtttctt tcagatcatt taggatactc ttaatctcac 8460
   ttcatcaatc aaatattttt tgagtgtatg ctgtagctga aagagtatgt acgtacgtat 8520
   aagactagag agatattaag tctcagtaca cttcctgtgc catgttattc agctcactgg 8580
   tttacaaata taggttgtct tgtggttgta ggagcccact gtaacaatac tgggcagcct 8640
   tttttttttt ttttttaatt gcaacaatgc aaaagccaag aaagtataag ggtcacaagt 8700
   ctaaacaatg aattcttcaa cagggaaaac agctagcttg aaaacttgct gaaaaacaca 8760
   acttgtgttt atggcattta gtaccttcaa ataattggct ttgcagatat tggatacccc 8820
   attaaatctg acagtctcaa atttttcatc tcttcaatca ctagtcaaga aaaatataaa 8880
   aacaacaaat acttccatat ggagcatttt tcagagtttt ctaacccagt cttatttttc 8940
   tagtcagtaa acatttgtaa aaatactgtt tcactaatac ttactgttaa ctgtcttgag 9000
   agaaaagaaa aatatgagag aactattgtt tggggaagtt caagtgatct ttcaatatca 9060
   ttactaactt cttccacttt ttccagaatt tgaatattaa cgctaaaggt gtaagacttc 9120
   agatttcaaa ttaatctttc tatatttttt aaatttacag aatattatat aacccactgc 9180
   tgaaaaagaa aaaaatgatt gttttagaag ttaaagtcaa tattgatttt aaatataagt 9240
   aatgaaggca tatttccaat aactagtgat atggcatcgt tgcattttac agtatcttca 9300
   aaaatacaga atttatagaa taatttctcc tcatttaata tttttcaaaa tcaaagttat 9360
   ggtttcctca ttttactaaa atcgtattct aattcttcat tatagtaaat ctatgagcaa 9420
   ctccttactt cggttcctct gatttcaagg ccatatttta aaaaatcaaa aggcactgtg 9480
   aactattttg aagaaaacac aacattttaa tacagattga aaggacctct tctgaagcta 9540
   gaaacaatct atagttatac atcttcatta atactgtgtt accttttaaa atagtaattt 9600
   tttacatttt cctgtgtaaa cctaattgtg gtagaaattt ttaccaactc tatactcaat 9660
   caagcaaaat ttctgtatat tccctgtgga atgtacctat gtgagtttca gaaattctca 9720
   aaatacgtgt tcaaaaattt ctgcttttgc atctttggga cacctcagaa aacttattaa 9780
   caactgtgaa tatgagaaat acagaagaaa ataataagcc ctctatacat aaatgcccag 9840
   cacaattcat tgttaaaaaa caaccaaacc tcacactact gtatttcatt atctgtactg 9900
   aaagcaaatg ctttgtgact attaaatgtt gcacatcatt cattcactgt atagtaatca 9960
   ttgactaaag ccatttgtct gtgttttctt cttgtggttg tatatatcag gtaaaatatt 10020
   ttccaaagag ccatgtgtca tgtaatactg aaccactttg atattgagac attaatttgt 10080
   acccttgtta ttatctacta gtaataatgt aatactgtag aaatattgct ctaattcttt 10140
   tcaaaattgt tgcatccccc ttagaatgtt tctatttcca taaggattta ggtatgctat 10200
   tatcccttct tataccctaa gatgaagctg tttttgtgct ctttgttcat cattggccct 10260
   cattccaagc actttacgct gtctgtaatg ggatctattt ttgcactgga atatctgaga 10320
   attgcaaaac tagacaaaag tttcacaaca gatttctaag ttaaatcatt ttcattaaaa 10380
   ggaaaaaaga aaaaaaattt tgtatgtcaa taactttata tgaagtatta aaatgcatat 10440
   ttctatgttg taatataatg agtcacaaaa taaagctgtg acagttctgt tggtctacag 10500
   aaaaaaaaaa aaaaa 10515

<210> 120
<211> 812
<212> DNA
<213> Homo sapiens

<400> 120
agcacatccc aaaagaataa catttcaaat gtaactagaa aaaagtatgc tgaagttcgc 60

aatacagaaa taattattaa taagatagct ttaaagccct gctcagcttt tgaatgttgg 120

gaattgaccc agaggtggct gtaacctaag atggttcctt cagtaatgac cattttttct 180

ttttcaagat gatgattatt ccccaccttc taagagacaa agaccaacga gccaccacag 240

ccaccagtcc cagaacccgc caatgctggg gaacggaaaa tgagggagtt caactctggc 300

cctcacaatc cagtggagga gacgaaactc atctgcctct gtccctctgg gcacgcctca 360

tgccaggtgc atctgtggac aggggccatg ctcctgggct tccaaagttg gagaaagctg 420

ccaggctcag gtctgaaggc cagaattcta cagtaagtcc tactgagtca aggtgggagc 480

agggtcggta gcttccgagg ctctgcggga gaatccgttt cctggccgta gaggtggcct 540

gcactcctca gcttgtgctg cccgtctcga atgactggag tttcctgctt ctgtcactac 600

acctcccacc ctctccatca cctgctctgc tcttacaagg atccgaagaa atggaatcat 660

cgtatcgctg atctacgtaa acaaactgaa gaattgtctg aaagaaaata tggtatgtct 720

aaactggaaa agtcttgtaa tcttatgttc atgggcgttt acacagtgga gttactgttc 780

atcatggggg taccgtggac aagcccaggg ct 812

<210> 121
<211> 2094
<212> DNA
<213> Homo sapiens

<400> 121
gcgcggatct tgcatctgaa actgagcaga gcagggcgcc gggcagggcc ggcgggccac 60

gtgataagcc cggaaacagc tccgccccct cgcttcctga gccgccacat cccggcagcc 120

ctcctacctg cgcacgtggt gccgccgctg ctgcctcccg ctcgccctga acccagtgcc 180

tgcagccatg gctcccggcc agctcgcctt atttagtgtc tctgacaaaa ccggccttgt 240

ggaatttgca agaaacctga ccgctcttgg tttgaatctg gtcgcttccg gagggactgc 300

aaaagctctc agggatgctg gtctggcagt cagagatgtc tctgagttga cgggatttcc 360

tgaaatgttg gggggacgtg tgaaaacttt gcatcctgca gtccatgctg gaatcctagc 420

tcgtaatatt ccagaagata atgctgacat ggccagactt gatttcaatc ttataagagt 480

tgttgcctgc aatctctatc cctttgtaaa gacagtggct tctccaggtg taactgttga 540

ggaggctgtg gagcaaattg acattggtgg agtaacctta ctgagagctg cagccaaaaa 600

ccacgctcga gtgacagtgg tgtgtgaacc agaggactat gtggtggtgt ccacggagat 660

gcagagctcc gagagtaagg acacctcctt ggagactaga cgccagttag ccttgaaggc 720

attcactcat acggcacaat atgatgaagc aatttcagat tatttcagga aacagtacag 780

caaaggcgta tctcagatgc ccttgagata tggaatgaac ccacatcaga cccctgccca 840

gctgtacaca ctgcagccca agcttcccat cacagttcta aatggagccc ctggatttat 900

aaacttgtgc gatgctttga acgcctggca gctggtgaag gaactcaagg aggctttagg 960

tattccagcc gctgcctctt tcaaacatgt cagcccagca ggtgctgctg ttggaattcc 1020

actcagtgaa gatgaggcca aagtctgcat ggtttatgat ctctataaaa ccctcacacc 1080

catctcagcg gcatatgcaa gagcaagagg ggctgatagg atgtcttcat ttggtgattt 1140

tgttgcattg tccgatgttt gtgatgtacc aactgcaaaa attatttcca gagaagtatc 1200

tgatggtata attgccccag gatatgaaga agaagccttg acaatacttt ccaaaaagaa 1260

aaatggaaac tattgtgtcc ttcagatgga ccaatcttac aaaccagatg aaaatgaagt 1320

tcgaactctc tttggtcttc atttaagcca gaagagaaat aatggtgtcg tcgacaagtc 1380

attatttagc aatgttgtta ccaaaaataa agatttgcca gagtctgccc tccgagacct 1440

catcgtagcc accattgctg tcaagtacac tcagtctaac tctgtgtgct acgccaagaa 1500

cgggcaggtt atcggcattg gagcaggaca gcagtctcgt atacactgca ctcgccttgc 1560

aggagataag gcaaactatt ggtggcttag acaccatcca caagtgcttt cgatgaagtt 1620

taaaacagga gtgaagagag cagaaatctc caatgccatc gatcaatatg tgactggaac 1680

cattggcgag gatgaagatt tgataaagtg gaaggcactg tttgaggaag tccctgagtt 1740

actcactgag gcagagaaga aggaatgggt tgagaaactg actgaagttt ctatcagctc 1800

tgatgccttc ttccctttcc gagataacgt agacagagct aaaaggagtg gtgtggcgta 1860

cattgcggct ccctccggtt ctgctgctga caaagttgtg attgaggcct gcgacgaact 1920

gggaatcatc ctcgctcata cgaaccttcg gctcttccac cactgatttt accacacact 1980

gttttttggc ttgcttatgt gtaggtgaac agtcacgcct gaaactttga ggataacttt 2040

ttaaaaaaat aaaacagtat ctcttaatca ctggatccat agttaaaaaa aaaa 2094

<210> 122
<211> 2021
<212> DNA
<213> Homo sapiens

<400> 122
ggcccggacg ggacgtgcgc gctcaaaggt tgcccgtctc tgacgcccgc atttcctggt 60

ctggagccgg ctgagccaca gcagggtcgc cgcggggtcc cggggccgtg ctcccctgcc 120

cctcccggga gcgcgcgggg cggggcgggg cggggcggga ccaggcgggc gagctgggcc 180

ctcgcccctc cctcgggcgg tcacctgggc acgggcgctg caggtgtcgg ggcctcaacc 240

ttgcggagcc gacagccatc gatcctcggg tggcctcgag gtggtggcag ggccgccccc 300

tgcagtccgg agacgaacgc acggaccggg cctccggagg caggttcggc tggaaggaac 360

cgctctcgct tcgtcctaca cttgcgcaaa tgtctccgag cttactcaca tagcatattg 420

gtatatcaaa atgaaatgca aggaaccaaa aataacataa ttgaaggcag taaaagtgaa 480

attaaatagg aagatcatca gtcaaggaag acccactgga gaggacagaa aatgaagcag 540

tgttttatca tgtgtatttc agcaggtctt cttgaaattt aactaaaaat atgactgctc 600

tctcttcaga gaactgctct tttcagtacc agttacgtca aacaaaccag cccctagatg 660

ttaactatct gctattcttg atcatacttg ggaaaatatt attaaatatc cttacactag 720

gaatgagaag aaaaaacacc tgtcaaaatt ttatggaata tttttgcatt tcactagcat 780

tcgttgatct tttacttttg gtaaacattt ccattatatt gtatttcagg gattttgtac 840

ttttaagcat taggttcact aaataccaca tctgcctatt tactcaaatt atttccttta 900

cttatggctt tttgcattat ccagttttcc tgacagcttg tatagattat tgcctgaatt 960

tctctaaaac aaccaagctt tcatttaagt gtcaaaaatt attttatttc tttacagtaa 1020

ttttaatttg gatttcagtc cttgcttatg ttttgggaga cccagccatc taccaaagcc 1080

tgaaggcaca gaatgcttat tctcgtcact gtcctttcta tgtcagcatt cagagttact 1140

ggctgtcatt tttcatggtg atgattttat ttgtagcttt cataacctgt tgggaagaag 1200

ttactacttt ggtacaggct atcaggataa cttcctatat gaatgaaact atcttatatt 1260

ttcctttttc atcccactcc agttatactg tgagatctaa aaaaatattc ttatccaagc 1320

tcattgtctg ttttctcagt acctggttac catttgtact acttcaggta atcattgttt 1380

tacttaaagt tcagattcca gcatatattg agatgaatat tccctggtta tactttgtca 1440

atagttttct cattgctaca gtgtattggt ttaattgtca caagcttaat ttaaaagaca 1500

ttggattacc tttggatcca tttgtcaact ggaagtgctg cttcattcca cttacaattc 1560

ctaatcttga gcaaattgaa aagcctatat caataatgat ttgttaatat tattaattaa 1620

aagttacagc tgtcataaga tcataatttt atgaacagaa agaactcagg acatattaaa 1680

aaataaactg aactaaaaca acttttgccc cctgactgat agcatttcag aatgtgtctt 1740

ttgaagggct atgataccag ttattaaata gtgttttatt ttaaaaacaa aataattcca 1800

agaagttttt atagttattc agggacacta tattacaaat attactttgt tattaacaca 1860

aaaagtgata agagttaaca tttggctata ctgatgtttg tgttactcaa aaaaactact 1920

ggatgcaaac tgttatgtaa atctgagatt tcactgacaa ctttaagata tcaacctaaa 1980

catttttatt aaatgttcaa atgaaagcaa gaaaaaaaaa a 2021

<210> 123
<211> 4957
<212> DNA
<213> Homo sapiens

<400> 123
taatgcaggt aatctaaaaa ggacggagga acagcaaggt ttggatgaag ggtttgactc 60

tcttacagat cagagcactg gagagactga tcctgatgat gtatctggag tgcagcctaa 120

aggtcatatt aatacgaaga aaaatcttac agctctcagt acttccaaag tagatcacaa 180

tgtcccaagg catcgttgta tgttatgtaa caaggaattt ttaggtggtc acattgtaag 240

gcatgcccag gctcatcaga aaaaaggcag ttttgcatgt gtaatatgtg gtaggaaatt 300

tagaaacaga ggacttatgc agaagcattt gaagaatcat gttaagaaga tacagaggca 360

gcaaattgct gcagctcaac aggatgatca ggaagtcact gctttggaag aaataaattg 420

ttctagttct tccatttcat ttgaaaatgg gaattctgat agtaaggatt tggaagtgga 480

gacacttact gcttctagtg aaggaaacaa agaagtcatc cctgagcatg tggctgaatt 540

cattgaaatt cccataagtg taccagaaga tgttattgaa aatgttattg aaaatggcag 600

tcctaataat tctttaaata atgttttcaa gcctttaact gaatgtgggg atgattatga 660

ggaggaagag gatgaagaag gtgattatga agaagatgat tatgacctga atcaagaaac 720

ttcagtaatt cataaaatca atggaactgt gtgccatcca aaagacatat atgccacaga 780

tcaagaagga aactttaagt gtcctgctct tggttgtgtc cggatattta aaagaattgg 840

gtttctaaat aaacatgcaa tgaccgtaca tccaaccgat ttaaatgtgc gacaaacagt 900

aatgaagtgg agcaaaggaa aatgcaaatt ttgtcaaagg caatttgaag attctcaaca 960

ttttatagac caccttaata gacatagcta tccaaatgtg tatttttgtt tgcattttaa 1020

ttgcaacgag tcgtttaagc tgccgttcca gcttgcccag cacacaaaaa gtcacaggat 1080

atttcaggct cagtgtagtt ttccagaatg ccatgagctt tttgaagatc ttcctctgct 1140

gtatgaacat gaagctcaac actatttaag taaaacacca gagtcatctg cacaaccaag 1200

tgaaacaatt ctttgggatg ttcagacaga ctcaaatcct aatcaggaaa aagactcatc 1260

tagtaatgag aaacaaacta ttagtctgcc agtttctact agcaaatcaa ggaaagagtc 1320

tacagaacca aagacatgta tagaaagtat ggaaaagaaa acagacagtt tagttcagaa 1380

tggaaacgaa cgttctgatg acactgtttc aaatataagc ttgatagacc aaaagatgcc 1440

tgacatagag ccaaattctg aaaataattg tagtagtagt gatatagtca atggacacag 1500

tgaaatagag caaacacctt tagtttcatc agatcctgct ttgaaaattg atacaaacag 1560

aatcaggaca gaaaatggtt ccattttgcc cagtgttgta ccacaagaac acaacacctt 1620

gccagtatct caggcacctt ccaaaccaaa tctgacaagt gaacatactt catatggctt 1680

aattttaaca aaaccatacg tcagaccatt gcctcccagt taccttgatg aacggtatct 1740

tagtatgcca aaacgcagaa aatttctgac tgatagagta gatgcctgtt ctgatcaaga 1800

taacgtgtat aaaaaatcag tgaaaagatt aagatgtggc aaatgcctga ccacctactg 1860

taatgcagaa gcacttgagg ctcatcttgc acaaaagaaa tgtcagacac tctttggatt 1920

tgattcagat gatgaaagtg cctgatgaaa acggttcaga aagatctgtc aatcaagcag 1980

tagtgtgaaa aaagcactat aagaaaatgc atcatcagtt tgctatttcc ctgatggcct 2040

taattttaga gtggtcttgg attactaaag ataaagacaa agcacatttt ctagaatgaa 2100

ctcacagaga tgtgctggct tagactccaa aaggattata aaactcccaa agtaccagtt 2160

ttccagaaaa ccacatttta cagtttatga tgattaatag cagcagcatg ttcagtttcg 2220

cttatatgag aaacatgttt aggcaactag tcagaaaacc agctatggcc ttacagaaag 2280

ggaaaaatta acccattatt aaaaagtgtg tgtggggagg ggggctggtt tacaatatta 2340

aaccatttaa agtattttat tctacaaatg gggtttgttt tcttgtcatg cataatcaga 2400

ttatgtcccc tcccttctgg ttaaacatgg tttaaggaac cgctgctgtt tggtttgtga 2460

ttgatttaga aacttataca ttaacttacc acagtgctat caattaaaga ttgtaacagg 2520

tttccagatg attaattagg tcaaagttca atgaatggca cgccagcatt gaaaattaaa 2580

aaacaaaaca aaaaaaacca cagtgctttg ctaataagct cttgatagaa cctcccacta 2640

accacttttg tggtacaaat tagttaacat ttaaaggcca gcagtgtcaa taaaatagaa 2700

aaaaaccgat cagtactctg ggagagatgg aaaggaatct aagactttac aggctttgta 2760

gttgaaggaa aacactgaat tgcaaattat tcaatgtgaa taatggtaaa agcacactgg 2820

gatacttctg tttgtgtata tgttgggaca ttgcttgatg attcatagta aggtccttta 2880

gacattaatg tgagttatct aagtacagtc ctattaaaat aactggctca aaaaatcagc 2940

taagaatcag gttgagaaat cattcatttt attctattag gagaggtaat tccaaaataa 3000

gccatactat tcctgttttt ttaattcatt agtgatgact ctggtctgaa cagtaatttt 3060

tatatgtaaa aggaagtatt tacacaaaac atttgcacaa gaacacagta agagatacat 3120

tcaagcattg ttttccttag tgatgttatt ttcctaagaa gattttaact taataaagtg 3180

ttaaaacaat aaatattttt aaaaataccc tctcagatgt tttccatttg tccattattg 3240

aaaggaagat gcttaaagac ttccttttac taatagtgtg taaatctaaa acaggattta 3300

aaataacaaa aaggcagtgt ttcaaaacag atctcctaat gtcccaatgt caaatattcc 3360

agttattcta aataataaaa tctttgttca aggcagataa tctcatcaga ccctattaga 3420

gcttcttgaa tgaggctatt acggaagtgg ggaatttctt ccaggtagca tccatatttt 3480

attaaattag gatttccaga tcatgataga tcagatgatt tttttttaat ttcccggttt 3540

tattggttta tcatcagcat tcactactat gcaatgatgg tcataagtcc attccaaatt 3600

taaatttgtt tctgttatgt agcctatatt aggctgtgga atttacattt aggtaactaa 3660

acacagctca taattaaaat cttttcttta aggtcagcta aaaaaaatgt ttcctgtatt 3720

tcttgatact aaaaatgtaa tgatttttat tttagttcat tctaaaagta tgtcttgtaa 3780

taaaaagtct gtttggaaac tggttcattt ggacagtgaa gtcttttgaa atatttctgc 3840

tttgtacatt ttccagaagt ttaatatttt atcacatttt ccacaagttg taacaattga 3900

tataaatcca tgcccacaaa gtattccatt ttcattactt ttactgttca tgccaagatg 3960

tttcaaatat attccatttg taatctgtct ttaacttgag gtttatttct acttttgtgt 4020

tgctaaaatt atttctgcaa gactgagatc aaactggttt atatataact aaatcaagga 4080

atgttttata aaattctatt tgaccacact gtcttaacct cggtttatat acttctcaaa 4140

tagctattga aagaatgtgt tcattttatt acatctgtct tttaaaacat aaacctacat 4200

cattttgctt ttatagggtg cataaacttc caacagttct aatttgagcg cttcagaaga 4260

aaaggaagct tgcttcttat tcctcagatt cttctttttt tctgttttga ggatgtattc 4320

atcctacatg gaccaagttt caaatctttg tttttaaata aaaatttcct tcaaaaaatt 4380

aaatgttaaa tttctgctgt gtgtcagtaa ggtgaagaaa atagcaaatc tgaatttttg 4440

ctgctcagat attatggagg aggtaaaaag gggaatctcc gtggacaagg acactggtac 4500

tttgggcttt agccatattc atttttttga aaccaccaat tatataatac ttaaataatt 4560

tatatcattg aacttgtgat tcttttcaaa tcacactgaa ggttcagccg tactcctttc 4620

atgtttggtt taatactgtt gtatggatga aaaacctaaa agggatgata gtgtttattt 4680

tttaatttat ttggtactgt aaaacacctt ttcagaatga gtaaatgctg catatgcatt 4740

ttggagttgt taatatgtga aagatattac agattcagca agaaaggaaa tttgtgcttc 4800

cttgttgaat gttaaattat tttactttgc attttataag agtacaaatt aaaactgagc 4860

cattgaactg caataaatca acaatagtgt ctcatttcaa gaaatttttt gtactgtaca 4920

tagatttgtt caataaaacg tccttgttgg taatcaa 4957

<210> 124
<211> 5101
<212> DNA
<213> Homo sapiens

<400> 124
cggaacccgg tcgaaagggt cctctctggc gtccttctgc ttccgtgggt ttctggatag 60

gctgcgtttt gttctcaggg gatgcaagtt cttcctttca gggttgaagc gtgagagagg 120

ttggtttttg aaacgggagt gtttcaggtt attgaggtcc aaaaagaaat aaagtttttg 180

tttacgtctg tatggcttgt aagatatctc ccggggccaa ttcagcatct ctacctggcc 240

atcctaacaa ggtgatttgt gaaagggtga gacttcagag cctgttccct ctcctcccaa 300

gtgatcagaa cactaccgtt caagaggatg ctcacttcaa agctttcttc cagagtgaag 360

atagtccaag tcctaagaga cagcgcctct ctcattcagt ctttgattat acatcagcat 420

caccagctcc ctcaccacca atgcgaccat gggagatgac atcaaatagg cagccccctt 480

cagttcgacc aagccaacat cacttctcag gggaacgatg caacacacct gcacgcaaca 540

gaagaagtcc tcctgtcagg cgccagagag gaagaaggga tcgtctgtct cgacataatt 600

ccattagtca agatgaaaac tatcaccatc tcccttacgc acagcagcaa gcaatagagg 660

agcctcgagc cttccaccct ccgaatgtat ctccccgtct gctacatcct gctgctcatc 720

caccccagca gaatgcagtc atggttgaca tacatgatca gctccatcaa ggaacagtcc 780

ctgtttctta cacagtaaca acagtggcac cacatgggat tccactctgc acaggccagc 840

acatccctgc ttgtagtaca cagcaggtcc caggatgctc tgtggttttc agtggacagc 900

acctccctgt ctgtagtgtg cctcctccaa tgcttcaggc atgttcagtt cagcacttac 960

cagtaccata tgctgcattc ccacccctta tttctagtga tccatttctt atacatcctc 1020

ctcacctttc tccccatcat cctcctcatt tgccaccacc aggccagttt gtccctttcc 1080

aaacacagca atcacgatcg cctctgcaaa ggatagaaaa tgaagtggaa ctcttaggag 1140

aacatcttcc agtaggaggt tttacttacc ctccatcagc ccacccccca acattacctc 1200

catcagctcc cttgcagttc ttaacacatg atcctttgca tcaggaggtg tcctttggag 1260

taccttatcc tccatttatg cctcggaggc ttacaggacg tagtagatac cgatcccagc 1320

agccaatacc acctccccct tatcatccca gcttactgcc atatgtgtta tcaatgcttc 1380

cagtgccacc tgcagtgggc ccaactttca gctttgaatt agatgtagaa gatggagaag 1440

tagaaaatta cgaggccctg ttaaacctgg cagagcgact gggagaggca aagcctcgtg 1500

gactgactaa agcagatatt gaacaacttc cttcttatcg gttcaatcct aacaaccacc 1560

agtcagaaca gactttgtgt gtagtatgca tgtgtgattt tgagtcaagg cagctactta 1620

gagtcttacc ctgtaaccac gagttccatg ccaagtgtgt tgacaaatgg cttaaggcaa 1680

atcgtacttg cccaatttgc cgagctgatg cttcagaagt gcatcgggat tcagaatgac 1740

caacctaaga agcacaaatt tagtttgggt gttcctcatc acatgtatat acggactatc 1800

cattgaactt aatctgtgtg gcttccagcc ctccctttac caaaagggtc aatggacctt 1860

tctttgcact gtgtgactta atcaactata aaagcttaca attagtcttc acagttatgg 1920

gattgttata ctaaccgtgt gattggaact ccaaagactt tttctttagc ttaattttgt 1980

gtgtgcacta acattccctg gtttttgtgt gatcattccg agtgttgctg caagattaca 2040

gtggacgtga tcttttagca tgtgctttta taaaaagtgg tagctacaga tgatatagca 2100

atctacctta tatagagcct tcagaaactg tgagtggaaa tgaatgcgca gcgtatgact 2160

gttggtgata agtgtatctg tgtgagtgtg tgcaactact tgtgtgaggg catggtagct 2220

aacgtgtgta tgagatccta tataccagca tgtaccaaga atgtgtgtgt agttttaatt 2280

atgctgcaat gtataatctg gtgtgttatt taaacagcac tagtactgta cactggtttt 2340

tttcccttgt gtttgctgtt gcacactgat tgctgagggt gcatcaatta caagcattga 2400

atactccatc cccttccctc ccagtgaatg gaatgagaaa agccttcttc ctttgcttca 2460

ggcagctgtc accttctctt tattggtggt cctaagtggg tcacttaaga aaaaaaagtg 2520

taacaaattc tcgctccatg aacctggttt ttttttattc tgttgtggaa atttttaaat 2580

ctccaacttg ctgtttccaa aaagaaggtg caatatcata tatcatcagt tagcaatatt 2640

agcatttcaa tcagtgattt gaatgttgat actttttttt cctttacatt tccagtaagt 2700

ttcttacaga aagtacctgt gatttttttt aatgtttaga tttgtagtct agtctattct 2760

gaagatattt gattaatata tttctaagaa gaccactagt cccatgaagt ctcaccttgt 2820

tgacttccac cagaacaaaa cctgttaatt cagcttgact tctcatatgt gttctgtgtt 2880

caagctcctg tgggcaactc aattgtttta agaaaactct atttctgttc atattaattc 2940

acttgccaga ctccaattct gaaagttgtc tagttcttcc ttcatcacac gtgcttctca 3000

tcgaaaccct ggtttcagga tggatcaagt gctgttacag ctcatgttcc ttagacctga 3060

tttgttttga tattttactg cctttccttt taatttttgt ttgaatgaga aaagtttaat 3120

acaacaggtg tccagaatac ttgcagtata aatgaagatt tggtttttgt ataaaaaaat 3180

aatgctgtta aacaggagtt gaccttcatt tagttgatgt aatacatagc tgtatggggt 3240

tttttttagt aagttctttg agcataattc actttaaagt catttttcca gcaatgttta 3300

aattactttc tcattctttt agtgtattca acattgtctg cctcttcctg cagttgatgt 3360

aattgctttg tttgcaatag cacaagctgc attattccag tcaggactgt gataacttgc 3420

tgccagcccc actcaacttt cagttggctc tgtgtcagtt ttccattcag tgttaactac 3480

ttgttactgc catgctgctt gccctccctt gaagtgtcta taagctcatc acagcctaga 3540

gttaagtaaa gtcaattcac agaagcacaa ttttgccctt tgcgagacat tgttgcctct 3600

atctagtcct acaagtaggg ttttgcatac tgtgtttgcc cctagggttg tcagtgcatc 3660

agaaatactt ctaaatagtg gtaaaaatgc acatggttaa tgcacatgtt acttttaaat 3720

cattaggata tccctcacct gttcctgatg aataaaaagt gtgttaaaga ccaaaattct 3780

tggcataata atcagctaca tacaaatcac atatagttta atctttttta atgaaaaaaa 3840

atcatgttta aaatggcaaa agcccatctt atacactttt atatagctgc aaaaaattta 3900

tatctgtaca gatctaacac tacgacactc agtattcatt ttattgaagc atgcaagtaa 3960

agcacttttt ctaatttata tagaggtatc taattaacac agcacattgt actaatgact 4020

aggagtagca gctttttctt ctctccctct atgaattctt ataatgtccc tttttctgta 4080

agtttttgag aggcaattgg caatttagga ggcagcaggg tctgttttgg tcaaatcttg 4140

aatttcgttg ttgcactcta gtgactgatc tttgggaatg tcttgtcttt gcatggggct 4200

catagagatg tgtgcagact tgcttattgt ggttagtgtg tatcaggaac acacacacag 4260

gtgttctgac cagctcaggc ttgccacagt gagcaactct gtggctagca aaagagaagt 4320

ttatttgtgc ccagccattg gtcaccttgg gtgatgcacc agatagcagg cagatgttgg 4380

ttcattggcc ttcgtcctct ttcctcctaa aataatattg gctttaccat cttaactcag 4440

ctgtgggttt tttgtgggtt cttgtttgtt ttttggcatg aattgtcatc tttggtgttt 4500

ttttaacccc cagcccctca aaaaaataag gcctccaggt atcaagatct catattagga 4560

ttttctgtcc ttaatttttt gagcaaaatc tggaaaatgt gaaagcatat ttagatttta 4620

tatactatct gaaatgtgat ttgttaagat tcttaaattt gggcctctta gaataatttt 4680

gaatgagatc taccgactca cttgtgagaa tatttttcac agattatctt tgggcctttt 4740

cattagaaag ctgtttgttt gtccccctgt tggtacattt ggttacctca ttttgccgtt 4800

tcagattgtg aaagctcaca ggggtgtttt ttggaatcat ttgctgagtc attttctcaa 4860

atcatattcc attgtatcag ttaacatata gttttaaatg tatgtattat aaatatctgt 4920

aaccaaatca tttgaaggct tgataaattt ttaacaaagt ttgtacattt tttatgaaag 4980

ttactagtaa tgctttacta agtagtgcaa tgaattttta tttttaatcc ctgtgcccaa 5040

ttttggagtt gagagggttg ttggtaataa atgtatgatg tacacttaaa aaaaaaaaaa 5100

a 5101

<210> 125
<211> 2882
<212> DNA
<213> Homo sapiens

<400> 125
atctgaaaaa ttaataattc cttaattatc aaatatccat tatttaaatt tataattgtg 60

tcataaatat tgtcataaat agatttgctg ttttaaagct tgttccttca ttttctctgt 120

tttgttttag ataaacattg tcataaatag atttgttgtt ttaaagcttg ttccttcatt 180

ttctctgatt gttttgtttt agattcagag gttacttatg cttgtttgtt acatggatgt 240

tacatgtgta atgggggata ttggacttct agtgtactca tcacccatat actgaacact 300

gtactcaaaa gggattgaaa gaaactagga aacttggcag gaagatcatt cttaagccag 360

gaaaaaaatt tttaatgctc acatgtgaac atgtgatggt cataccagaa ggagcaccca 420

cctccctccc tctgtgacag acacattttc ttagccttca cctttccttc tttcaagttg 480

ctgaaaatcc acagtgtttc tgttcatttg ttactttcat tctcacctat cttctctctt 540

gctccatcta ccagaacaat aattccccat ataatacttc tcacttcact tttcaacgca 600

ggacctcttg ttgggtctga tctgtttgtc tgtccgcttt atcaatatta tcagatgtaa 660

gtttacatga atacacacac atattcacta aactgagggg aaaaaatgcc ttgtaggtca 720

taaaaaagca gggaaattcc caacaattca tatttgatcc ctggatccag gggtggcagc 780

aataagcctg ctttagatat ttactcccca ttttatgatc cggtggtttg gtttttcaaa 840

tgatgatatg gctcctttcg caatgacttg atgtttagga ggtgtgcttc aataaataca 900

ttttaaaatc aacaatcaag ttagagttgt acaaatggct ctgaaatgtc ccactacact 960

gttagaccaa gggcacagat tgtgcttctg tactatttat cctagtatcc ctcggcatat 1020

attaactgct ctaaaaatct ccttggctac acgctgcatc aaatcaaagt taaatgttat 1080

accacctttc tattctattt ttaatattca aagagggtgc tcagatttta gaacaaattt 1140

caatgtttaa gtacacacaa aaaaatcatt aactcatata tttcaagagt aggaaatggg 1200

aactggtgtt aaaactctta taacaaatgt cactgtctta agggacagtg tttaaaaacg 1260

catacctggc cgggcgcggt ggctcatgcc tgtaatccca gcactttggg aggccgaggc 1320

cggcggatca caaggaaaac aaactcagga agaaaaagaa aagcagaagt gatcaaggag 1380

agcgctcgag ttgcaatatt ttcctttggc tgctgacagg cagttactat aaagcattgt 1440

gcatggacac catcttcttg tattatacaa gaaaggagtg tacctatcac acacaggggg 1500

aaaaatgctc ttttgggtgc taggcctcct aatcctctgt ggttttctgt ggactcgtaa 1560

aggaaaacta aagattgaag acatcactga taagtacatt tttatcactg gatgtgactc 1620

gggctttgga aacttggcag ccagaacttt tgataaaaag ggatttcatg taatcgctgc 1680

ctgtctgact gaatcaggat caacagcttt aaaggcagaa acctcagaga gacttcgtac 1740

tgtgcttctg gatgtgaccg acccagagaa tgtcaagagg actgcccagt gggtgaagaa 1800

ccaagttggg gagaaaggtc tctggggtct gatcaataat gctggtgttc ccggcgtgct 1860

ggctcccact gactggctga cactagagga ctacagagaa cctattgaag tgaacctgtt 1920

tggactcatc agtgtgacac taaatatgct tcctttggtc aagaaagctc aagggagagt 1980

tattaatgtc tccagtgttg gaggtcgcct tgcaatcgtt ggagggggct atactccatc 2040

caaatatgca gtggaaggtt tcaatgacag cttaagacgg gacatgaaag cttttggtgt 2100

gcacgtctca tgcattgaac caggattgtt caaaacaaac ttggcagatc cagtaaaggt 2160

aattgaaaaa aaactcgcca tttgggagca gctgtctcca gacatcaaac aacaatatgg 2220

agaaggttac attgaaaaaa gtctagacaa actgaaaggc aataaatcct atgtgaacat 2280

ggacctctct ccggtggtag agtgcatgga ccacgctcta acaagtctct tccctaagac 2340

tcattatgcc gctggaaaag atgccaaaat tttctggata cctctgtctc acatgccagc 2400

agctttgcaa gactttttat tgttgaaaca gaaagcagag ctggctaatc ccaaggcagt 2460

gtgactcagc taaccacaaa tgtctcctcc aggctatgaa attggccgat ttcaagaaca 2520

catctccttt tcaaccccat tccttatctg ctccaacctg gactcattta gatcgtgctt 2580

atttggattg caaaagggag tcccaccatc gctggtggta tcccagggtc cctgctcaag 2640

ttttctttga aaaggagggc tggaatggta catcacatag gcaagtcctg ccctgtattt 2700

aggctttgcc tgcttggtgt gatgtaaggg aaattgaaag acttgcccat tcaaaatgat 2760

ctttaccgtg gcctgcccca tgcttatggt ccccagcatt tacagtaact tgtgaatgtt 2820

aagtatcatc tcttatctaa atattaaaag ataagtcaaa cattaaaaaa aaaaaaaaaa 2880

aa 2882

<210> 126
<211> 1834
<212> DNA
<213> Homo sapiens

<400> 126
actttgacag tcttctcatg ctgcctctgc caccttctct gccagaagat accatttcaa 60

ctttaacaca gcatgatcga aacatacaac caaacttctc cccgatctgc ggccactgga 120

ctgcccatca gcatgaaaat ttttatgtat ttacttactg tttttcttat cacccagatg 180

attgggtcag cactttttgc tgtgtatctt catagaaggt tggacaagat agaagatgaa 240

aggaatcttc atgaagattt tgtattcatg aaaacgatac agagatgcaa cacaggagaa 300

agatccttat ccttactgaa ctgtgaggag attaaaagcc agtttgaagg ctttgtgaag 360

gatataatgt taaacaaaga ggagacgaag aaagaaaaca gctttgaaat gcaaaaaggt 420

gatcagaatc ctcaaattgc ggcacatgtc ataagtgagg ccagcagtaa aacaacatct 480

gtgttacagt gggctgaaaa aggatactac accatgagca acaacttggt aaccctggaa 540

aatgggaaac agctgaccgt taaaagacaa ggactctatt atatctatgc ccaagtcacc 600

ttctgttcca atcgggaagc ttcgagtcaa gctccattta tagccagcct ctgcctaaag 660

tcccccggta gattcgagag aatcttactc agagctgcaa atacccacag ttccgccaaa 720

ccttgcgggc aacaatccat tcacttggga ggagtatttg aattgcaacc aggtgcttcg 780

gtgtttgtca atgtgactga tccaagccaa gtgagccatg gcactggctt cacgtccttt 840

ggcttactca aactctgaac agtgtcacct tgcaggctgt ggtggagctg acgctgggag 900

tcttcataat acagcacagc ggttaagccc accccctgtt aactgcctat ttataaccct 960

aggatcctcc ttatggagaa ctatttatta tacactccaa ggcatgtaga actgtaataa 1020

gtgaattaca ggtcacatga aaccaaaacg ggccctgctc cataagagct tatatatctg 1080

aagcagcaac cccactgatg cagacatcca gagagtccta tgaaaagaca aggccattat 1140

gcacaggttg aattctgagt aaacagcaga taacttgcca agttcagttt tgtttctttg 1200

cgtgcagtgt ctttccatgg ataatgcatt tgatttatca gtgaagatgc agaagggaaa 1260

tggggagcct cagctcacat tcagttatgg ttgactctgg gttcctatgg ccttgttgga 1320

gggggccagg ctctagaacg tctaacacag tggagaaccg aaaccccccc cccccccccg 1380

ccaccctctc ggacagttat tcattctctt tcaatctctc tctctccatc tctctctttc 1440

agtctctctc tctcaacctc tttcttccaa tctctctttc tcaatctctc tgtttccctt 1500

tgtcagtctc ttccctcccc cagtctctct tctcaatccc cctttctaac acacacacac 1560

acacacacac acacacacac acacacacac acacacacac agagtcaggc cgttgctagt 1620

cagttctctt ctttccaccc tgtccctatc tctaccacta tagatgaggg tgaggagtag 1680

ggagtgcagc cctgagcctg cccactcctc attacgaaat gactgtattt aaaggaaatc 1740

tattgtatct acctgcagtc tccattgttt ccagagtgaa cttgtaatta tcttgttatt 1800

tattttttga ataataaaga cctcttaaca ttaa 1834

<210> 127
<211> 1297
<212> DNA
<213> Homo sapiens

<400> 127
ggggcggggc ttcgggcgag agggagggca gcaggagaaa ggataagaaa gggagtggga 60

ctggcgccta cggtggccga agtgggacgc gccgagccgg aggctgcagg atgatgcggt 120

tcatgctatt attcagccgg cagggaaaac tgcggctgca aaaatggtac ctggccactt 180

cggacaagga acggaagaag atggtgcgcg agctcatgca ggttgtcctg gctcgaaagc 240

ccaagatgtg cagcttcctg gagtggaggg acctcaaagt tgtctataag agatatgcca 300

gcctctactt ctgctgcgcc atcgagggcc aagacaatga gctcatcaca ctggagctga 360

tccaccgata cgtggagctc ttagacaaat actttggcag tgtgtgcgag ctggacatca 420

tcttcaactt tgagaaggcc tacttcatcc tggatgagtt tttgatgggg ggggatgtcc 480

aggacacctc caagaagagt gtgctgaaag ccatcgagca ggctgaccta ctgcaagagg 540

aggatgagtc gccacggagt gtgctggagg agatgggttt ggcatagccc ctgctgggcc 600

ggggtgtggc gatggggtcc tggcagcgtg gcgggaacgg ctgcttctcc tctgcccagg 660

gccctgttct tggtgggact cggctgcccc tcctctgctg cctcaccttt cggagtgagc 720

tgtgggctca ggcccttcaa acattccctc cctccacccc ctacctccac tttccccttt 780

tcccactgaa ggttttagaa gctaggaggc aggaaaatgt gacccagatg ggggtgctat 840

ttggctttta ttccctgcct ttgcagaact gatgtcaccc cagatgtcct tccctcccta 900

ataactgtaa atatataaat atgtcaggtt aaagggaaaa ggtgttcagg gcacttcttg 960

tcctctctgt cccataacct acctccaccc tccccctagc cagccaggca gcttctctgc 1020

ctgggagggg agcctggacc cccctctttc tccttggctg cagtggggcc tttatccagt 1080

gccagggagg aacaacatag ttaatttttt tctaaccttg ccactttgag ggaaggaagg 1140

gttgggggaa gggcaagctt tatgggaccc tggtcctggc cctggccttt cactccagtt 1200

ctgggtgagg caggagctgg gaggggtggg gagggggagg gggaagtgtc tgcctttatg 1260

tcctttcttc tgaaataaaa gtaaaagcat ttctgga 1297

<210> 128
<211> 7412
<212> DNA
<213> Homo sapiens

<400> 128
   cgcttctgct caacctagac cagccccagc ttcagcctca gctcccctcc ttcctggatc 60
   gagcgcccgc actcccggcc ctgcagccac ccgagtcccg ctcgctgtcg cctgcacgcg 120
   agtcccccct ggcacgcgct cccacatccc gggatcgtcc caacggcccc tgcgcccttc 180
   ctgggatcac tccgactgcc ccgcgcgccc tgggatcggt ccatctaccc cgcgtggccc 240
   cagctgcttg cccggagcgc cagctagcgc tccccgctct ccgctccccg gcactctcgg 300
   ggggcccgcc cgccctgcac cctggagctc cgggccgcga gcctctgcca actcctctgg 360
   accctcgcgg ccgtgggcag cggctgccgc gcctgtctgc ccgagggagg accttcgcct 420
   ctgcatttgt ccagtaactc tggctgtgcc ggatactgct tgggtaaaac gggcacccca 480
   ggaacatggc agacgaagat ctcatcttcc gcctggaagg cgttgatggc ggccagtccc 540
   cccgagctgg ccatgatggt gattctgatg gggacagcga cgatgaggaa ggttacttca 600
   tctgccccat cacggatgac ccaagctcga accagaatgt caattccaag gttaataagt 660
   actacagcaa cctaacaaaa agtgagcggt atagctccag cgggtccccg gcaaactcct 720
   tccacttcaa ggaagcctgg aagcacgcaa tccagaaggc caagcacatg cccgacccct 780
   gggctgagtt ccacctggaa gatattgcca ccgaacgtgc tactcgacac aggtacaacg 840
   ccgtcaccgg ggaatggctg gatgatgaag ttctgatcaa gatggcatct cagcccttcg 900
   gccgaggagc aatgagggag tgcttccgga cgaagaagct ctccaacttc ttgcatgccc 960
   agcagtggaa gggcgcctcc aactacgtgg cgaagcgcta catcgagccc gtagaccggg 1020
   atgtgtactt tgaggacgtg cgtctacaga tggaggccaa gctctggggg gaggagtata 1080
   atcggcacaa gccccccaag caggtggaca tcatgcagat gtgcatcatc gagctgaagg 1140
   acagaccggg caagcccctc ttccacctgg agcactacat cgagggcaag tacatcaagt 1200
   acaactccaa ctctggcttt gtccgcgatg acaacatccg cctgacgccg caggccttca 1260
   gccacttcac ttttgagcgt tccggccatc agctgatagt ggtggacatc cagggagttg 1320
   gggatctcta cactgaccca cagatccaca cggagacggg cactgacttt ggagacggca 1380
   acctaggtgt ccgcgggatg gcgctcttct tctactctca tgcctgcaac cggatttgcg 1440
   agagcatggg ccttgctccc tttgacctct cgccccggga gagggatgca gtgaatcaga 1500
   acaccaagct gctgcaatca gccaagacca tcttgagagg aacagaggaa aaatgtggga 1560
   gcccccgagt aaggaccctc tctgggagcc ggccacccct gctccgtccc ctttcagaga 1620
   actctggaga cgagaacatg agcgacgtga ccttcgactc tctcccttct tccccatctt 1680
   cggccacacc acacagccag aagctagacc acctccattg gccagtgttc agtgacctcg 1740
   ataacatggc atccagagac catgatcatc tagacaacca ccgggagtct gagaatagtg 1800
   gggacagcgg ataccccagt gagaagcggg gtgagctgga tgaccctgag ccccgagaac 1860
   atggccactc atacagtaat cggaagtacg agtctgacga agacagcctg ggcagctctg 1920
   gacgggtatg tgtagagaag tggaatctcc tcaactcctc ccgcctccac ctgccgaggg 1980
   cttcggccgt ggccctggaa gtgcaaaggc ttaatgctct ggacctcgaa aagaaaatcg 2040
   ggaagtccat tttggggaag gtccatctgg ccatggtgcg ctaccacgag ggtgggcgct 2100
   tctgcgagaa gggcgaggag tgggaccagg agtcggctgt cttccacctg gagcacgcag 2160
   ccaacctggg cgagctggag gccatcgtgg gcctgggact catgtactcg cagttgcctc 2220
   atcacatcct agccgatgtc tctctgaagg agacagaaga gaacaaaacc aaaggatttg 2280
   attacttact aaaggccgct gaagctggcg acaggcagtc catgatccta gtggcgcgag 2340
   cttttgactc tggccagaac ctcagcccgg acaggtgcca agactggcta gaggccctgc 2400
   actggtacaa cactgccctg gagatgacgg actgtgatga gggcggtgag tacgacggaa 2460
   tgcaggacga gccccggtac atgatgctgg ccagggaggc cgagatgctg ttcacaggag 2520
   gctacgggct ggagaaggac ccgcagagat caggggactt gtatacccag gcagcagagg 2580
   cagcgatgga agccatgaag ggccgactgg ccaaccagta ctaccaaaag gctgaagagg 2640
   cctgggccca gatggaggag taaccaggaa aatcactgcc ggctagtccc aagcaaacgg 2700
   gctaggagga aagattaaaa aaacaacaac aacaacttat ttagtttggg gaggggaagc 2760
   atttttaagt gtgttgtaaa atcaaatttt atatttcatt ttttgactct tgaaaaatgt 2820
   ctttgctcct tggcagctac cagcagagac tctatagctg tctcttaggg cagtattttg 2880
   gggaagtggg gcttgaagaa gcagcctaat gaaccaacat accgttttgt gtgtggtttt 2940
   ttttgtttgt ttgtttgttt gttttgagac agagtcttgc tctgtcaccc aggctggagt 3000
   gcagtgacat gatcttagct cactgcaacc tccgcctcct gggttcaagt gattctcctg 3060
   cctcagcctc ccaagtagct gggattactg gtgcacacca ccacactcag ctaatttttg 3120
   catttttagt agagatgggg tttcaccatg ttggccaggc tggtctcgaa ctcctaacct 3180
   caggtgatcc acctgcctca gcctcccaaa gtgctgggat tacaggtgtg agccaccatg 3240
   cctgcccatt ttgtggttct attttcattt ttatttcttt tttttttttt gtcacgagat 3300
   ataagaaagt gctttttgcc ttgaatggac aattttaggg ctgtgctcac tagtcttttc 3360
   aggctggact gaaatgtcgg gcccatggag ccctgtgttt tgtgcatcgg gatgagaaat 3420
   gaagcacttc acgctggctt tcctaagtca cggggcgtgt attgccgtgg cttagtgcaa 3480
   agcattcttt ctcagagcat ttagaggcat gcgtggcatt ttttcagtgg gtgtgagatt 3540
   gcacaatacc caggctccct tctactgtgg ggaagggcct gcatgttggc tgttttttaa 3600
   acttctagtt caatttcctt ccataatgct actgattttc tggcatacag ccgaattcca 3660
   tcttttaagc atgctttcta cggtgggctt ttcaaaacag gtttgagttt tgtatgcaca 3720
   cgtttactac ctctaactcc tacatcagct agtgtggaag agggtgcacc tcaaagcttt 3780
   tacacgtaag gacagcggct tggaatgtga gagccttttc tccaagcaga cccacactct 3840
   gcatctcagt ggcagctccc acaacgtgac tgcaatgtct cttatacagt attccttggt 3900
   gttttcttag tgtctggatg ttcttacgtg aaatctgctc cccagccctg gtccttggca 3960
   ttttctgctt gaagctgggc tgattttctt gtaatttaca gcaggacgct ttcagcagca 4020
   gtctcttggg attttatcta agatgtttga ggatgagagg gcaagaacta taaacactca 4080
   ttaattctag tagtctcccc atggccagac aatggcgatt gttatttaat gagcttttcc 4140
   tttcaatgga attcagctct cacattagta tgatttcatt tgatgtttca aatagcaaag 4200
   atgctaggtg cggtggctcc cgcctgtaat ctcagcactt tgaggagggc caaggtggga 4260
   ggattgcttg agctcaggac ttcaagacca gcctgggtaa acatggcgag accctgtctc 4320
   taccaaaaca acaaaaaaaa gacagactgc tttgatcaac cctaaatgca aaagcagcct 4380
   atttttcttt gtttaaaagt caaaacataa aaaagcagag tataacatac aaaccattct 4440
   taactattca ttaaaatggg tccttcaaca ccttagtggg gtttgttgtt gttgcttatg 4500
   cagagagatt attttctttt tattatttta taatttttga aatagagatg gggtctcact 4560
   gtgttgccca ggctggtctc gaactcctgg acttaagtga gcctcccgcc tcagtctccc 4620
   aaagcgctgg gattacaggc aggagccact gagcccagcc aagacttcag tgttgactgc 4680
   tttggaggca caaacccatg caagcgttag ttccaaagtt cagtgtgtac ccttaaatga 4740
   acaatgaagc aggtaaaatt acccttgaaa aaaatccctt ggaccaccca taaatgacag 4800
   tgactttttc aatatggact catcatagcc agttttcctt ttgaagttgg aactgatcac 4860
   ccttttgtca tctgtaccag atcagtagtt ggcttgtgtt acattttgtg tgtgtgtgtg 4920
   cgtgttttaa accagtgcat ataaattgta tgttaaatgt aagtaacttt aagttgactt 4980
   atctcttcac agtaatcaag cctcacgtaa ttcatgcttt ttaaattcag ccagcccccc 5040
   ctctctgaaa ttttattatg taaataattt gtgttccctg atcactcgtt taagttctta 5100
   gttgtatgtc atctcttctc tagcaggaat tggcaaactt ttttgtaaag gggtagaaag 5160
   tgaagatttt aggctttgca ggccatatag cctctgctgc aaatgctcag ccctgctgtt 5220
   gtaatgtaaa agctgccaca gacactacat gaacacgaat gagtgtggct ggtgttccaa 5280
   taaaacttta tttacacaaa cgggtggccc ttttgggctg tagtttgtca acccttgctc 5340
   taacccttga ctgagagcta ctttattaag ccctgagggc aggagctatc ccaattttgt 5400
   gttccccagg gcaccaaaac acagtgcttt ggcatagagt aggcactcaa caagtgtgtg 5460
   aacagatgga gagccagccc tagtcagtgc actcaccctt tgaggctctg gttcctccaa 5520
   acaatgattc gttgtctgga ttggctggaa ctgtcacccc cgcaattcta ctccccaccc 5580
   acccatgtga ccttaatgtc agttgctggt ctgttgctct tcggggaggg agagatggcc 5640
   tggatacaga gctaagcaaa tgcttcttta agggccctta aaagtgaaaa gtaacttgca 5700
   agaggttgag atccttctga gctaggagaa cttattccac cttcaaaacc tagtttgggc 5760
   tgggtgcagt ggctcacgcc actttgggag gctgaggtgg gcggatcacc tgaagtcagc 5820
   tactcgggag gctgaggcag gagaatcgct tgaacccagg aggcagaggt tgcagtgagc 5880
   cgagattgcg tcactgcact ccagcctggg cgacagagtg agactccgtc tcgaaaccaa 5940
   aaacaagaaa aacccctagt tttgtgcctt ctgaagatag ttaggacatc ttctttttcc 6000
   gcaaatgctg gacatgccaa aaacctaacg caaaacccag gacattccag cccaactggg 6060
   acgatatcag agaccatctt caagtgcaag aagcaggtga aagcccagga aattggcaga 6120
   gccaggatta tcatccagta ctttcatttc acaaatggag aaaccgaggt tctgccagct 6180
   agattttttt tcacagtgtc actgctagca agccactaag ctggagctgg gatttgagag 6240
   ctgctgctat ttagaggatc ttgggaataa aatttaaact ggagtttaat ggccctttca 6300
   gttttgctat aggcaagaga ataaaatgaa tgaatggata ggtggcttta tgggtgtaag 6360
   aaagaagcga aaaaaactcc caaaccccag tgttcctgaa tatctgttct ccccctgacc 6420
   actctgggaa tttatcaaat gcagctttga cctccaagcc aagtaaactg ctcttgttgc 6480
   tattttagtg gtttttgttt ttttaagaca caaggtctca ctttgttact caggctggag 6540
   tgcagtggca tgatcataac tcattgtagg ctcaacctcc tgggccccag tgatcctcct 6600
   gcctcagcct ctcaagtagc taaaactaca gatgtgcacc atcacatctg gctaattttt 6660
   tttttttttt ttttgaggtg gagtctcgct ctgttgccca gattcaagtg caatggcacg 6720
   ttttggctca ctgcaacctc tgcctcccag gttcaagcga ttctccctgt ctcggcctcc 6780
   cgagtagctg ggactacagg cacctgccac cacgcgcagc taatgtttgt atttttagta 6840
   gagacggggg tttcaccatg ttggccaggc tggtttcaaa ctcctgacat taggtgatcc 6900
   acctgcctcg gcctcccaaa gtgctgggat tacaggcatg agccaccgtg ccaagccacg 6960
   cctggctaat tttttaaaat tattttttgc agagacagag tttcactatg ttgcccaggc 7020
   tggtcttgaa ctccttggcc tcaagtgatc ctcccacctc agcctcccaa agcattgggg 7080
   ttacaggcgt gagccactgc acctggccct gttttcatgg cttttatttc ctttcatccc 7140
   acacatttgt ctgcagtact agacatgttt tacaaatcaa caagtttaca caagtatatg 7200
   cagcttgttt tggggggaaa ggagacaaaa tatgcatatt ttctgtgtgg atttgtgcct 7260
   cttaattgtg tttctagtct ctaaggtgac cctttaacct actcaagatg gggcccagag 7320
   aagtggcctg cgttacagat ttattttggc atatgtacta agttccattt tctctttaca 7380
   aataaagtgt tttctttctt ttctgtctca ga 7412

<210> 129
<211> 3387
<212> DNA
<213> Homo sapiens

<400>  129
acttcccaga aggcaccgag tccctgccgt tctcctcaac tggcggcggc gcgaacgaat      60

agtcgccggc gacctgtgag ggcactcgga agggcgaggg gagggctcga ccgctcgcgc     120

ctagtttttc tatctctccc ggagcctgag tctctgagcc gtccccagca aacgctcagg     180

ggctgcagag gccccgagag gtgaggggct ccgtgagggc gggaaccagg ctgaggccgc     240

ctctggggag cggagcgtgt ccgttgctga gggagcaagg ccgggtaggg agcctggtga     300

gcgcctcagg caggggcgca cgctgagctt tacggtaaag gtgttccttg accagcggaa     360

gaggccccag agtgagcctg gcccggcggt ccttagtggg atgtcgcctg ccgctctcag     420

cagagctttg acggcggaga ggagtcggca ggcggtgtgt ggacacctcc tcggccttgc     480

atctgctccc cgggagagtc accaaccgcc tccccgccca aagggcaccg gagggagctt     540

cggttcgagg gcttggctct ctggcagatt tcctctagta agaggtggct ctggaggccc     600

cgcgaaacga gtgtggtgtg tggttgcaag gcatgatggc tgcaaaagtg gttcctatgc     660

cccaaaagcc aaagcagtcc tttatactga gagttccgcc agactccaag ctgggccaag     720

acctacttcg agatgccact aacgggccca agaccatcca ccagctagtg ctggagcact     780

tcctcacctt cttgcccaag ccaagcctgg tccagcccag tcagaaagtc aaggagacct     840

tggttattat gaaagatgtg agctcaagcc ttcagaacag agtgcatcct cgtcccttgg     900

tgaagcttct gcccaaagga gtccaaaagg aacaagagac agtgtctctg tatttgaaag     960

ctaaccctga ggagctggtg gtctttgagg atttgaatgt atttcactgc caggaagaat    1020

gtgtgagctt ggatcctact caacaactca cgtcagagaa ggaagatgac agcagtgtcg    1080

gggaaatgat gttactggca gtcaatggca gtaatcctga aggtgaagat cctgagaggg    1140

aacctgtaga aaatgaagat tatagagaaa agtcttcaga tgatgatgaa atggattctt    1200

ccttggtctc tcagcagcct cccgataacc aggaaaagga acgactaaat acatccattc    1260

cacaaaaaag gaaaatgaga aatctgttag ttaccattga gaatgatact cctctagagg    1320

aactctcaaa atatgtagac atcagtatta ttgcccttac tcgaaatcgg aggacaagga    1380

gatggtacac ttgtccactg tgtgggaaac agtttaatga aagttcttac ctcatttccc    1440

accagaggac ccacactgga gaaaaaccct atgactgtaa tcactgtggg aaaagcttca    1500

atcataaaac aaacctcaat aaacatgagc gaattcatac aggagagaaa ccttattcct    1560

gttctcagtg tggaaaaaac ttccgtcaga attctcatcg gagtcgtcat gaaggaatcc    1620

atataaggga gaagatattt aagtgtccag aatgtgggaa aaccttccca aagaatgagg    1680

agtttgtgct tcatctgcag agtcatgagg ctgagagacc atatggttgc aaaaaatgtg    1740

ggagaagatt tggtcggctg tcaaactgta cccggcatga gaaaacccac tcagcctgta 1800

agacccgaaa gcagaagtaa tactgggaac cctttctggg tctgatggtg ctgcctcaac 1860

ctgagagctt tcataagtag ttctgaattc ccaagctgcc taaaaaggta taaatgtgta 1920

aaaatctcat tattgccaaa attggataaa tgcccatctt agctaaaacc tcaaattgct 1980

agaaaattca cagggaagaa aacatttcaa gggctatacc tcagcatcta ggctttttgg 2040

actaaggagc tttccttttt gaagttatat gataatgtac aggtcacaga tcccctttcc 2100

caacactttg aagatgaatc tggagtctgc ttacttggaa ggcaaagagt gacttgtgtc 2160

tattgaaagt atatccgttt tccccccaca tggggattca tacttgagaa atagtgcaaa 2220

gatgcttatc tggaactgtg ttctggtgaa agaaccaaat tactggcttg ttagccaaca 2280

gcttctgata gcaattcata taaccctcta agaatacctg tttaagtctt gagtgttgaa 2340

aggaattgtt tactttggaa tataggaaaa cagttgaatg tcagactctc atttgtatgt 2400

gatctaaatt tgcaatcaat ttcaataata tttacaattt gtgataaaac tgacttttac 2460

agattccttt tcacaacata atttaggtgt ctactgttct tattgtattt tgttctgctg 2520

ttgatctctc cagcagccgt ctcatgcttc tcccttgcta aaagaagttt ggattactca 2580

ggcagggcca tccagcccca ccactagaaa agctcttcag aatcttgtcc ctctgttgag 2640

cccagatctc atgtgctacg aaggaaaccc caagacccag agaggaaggg tcaacctgga 2700

ggcaggaaaa agttggcttg gatccatgtc tcatcaataa ccttaccata tgcttaggtc 2760

ccctctatgc tgtcatcaga cctttggcaa tggggtggtc actacctcac aaggcaaagt 2820

gttgtatgat tagaaattac gtctccagtg gttagctcac attgcctctc aagagacagg 2880

tttccaggtg tcttcattgt agtgggtatt aattgtcttc agcctcttga tatccatacc 2940

ttcctgtcct ctgcctagaa gcaaggccag cggtgccttt acggactgat cgtgtggtgc 3000

gatttaggga ttcttcagtt ttgcttgctt taggtttcca aaagttatac attggtgttt 3060

tgattggaat aaagaaatcc tataagctat ttgggaaaaa ttatagtgta tgtttcccat 3120

ccagaaacat gcctttctat ttattagagt attatattcc tgtgaaaatt tttctaattt 3180

tcttcacttg ttttacacaa ttttgttatt gtagtttttt ccattatatt tttatagttg 3240

attattgctt ttacatggga aagttatttt taattatata tttgtatagt catctcactg 3300

ttgttaattt tcaatagttt gttggtttag ttctgttaac ttttggtaaa atgacaccat 3360

ctacaaagaa tagtttttgt ttcttgc 3387

<210> 130
<211> 7042

<212>  DNA
<213>  Homo sapiens

<400>  130
aagaagaggt agcgagtgga cgtgactgct ctatcccggg caaaagggat agaaccagag      60

gtggggagtc tgggcagtcg gcgacccgcg aagacttgag gtgccgcagc ggcatccgga     120

gtagcgccgg gctccctccg gggtgcagcc gccgtcgggg gaagggcgcc acaggccggg     180

aagacctcct ccctttgtgt ccagtagtgg ggtccaccgg agggcggccc gtgggccggg     240

cctcaccgcg gcgctccggg actgtggggt caggctgcgt tgggtggacg cccacctcgc     300

caaccttcgg aggtccctgg gggtcttcgt gcgccccggg gctgcagaga tccaggggag     360

gcgcctgtga ggcccggacc tgccccgggg cgaagggtat gtggcgagac agagccctgc     420

acccctaatt cccggtggaa aactcctgtt gccgtttccc tccaccggcc tggagtctcc     480

cagtcttgtc ccggcagtgc cgccctcccc actaagacct aggcgcaaag gcttggctca     540

tggttgacag ctcagagaga gaaagatctg agggaagatg gatgcaaaag ctcgaaattg     600

tttgcttcaa catagagaag ctctggaaaa ggacatcaag acatcctaca tcatggatca     660

catgattagt gatggatttt taacaatatc agaagaggaa aaagtaagaa atgagcccac     720

tcaacagcaa agagcagcta tgctgattaa aatgatactt aaaaaagata atgattccta     780

cgtatcattc tacaatgctc tactacatga aggatataaa gatcttgctg cccttctcca     840

tgatggcatt cctgttgtct cttcttccag tgtaaggaca gtcctgtgtg aaggtggagt     900

accacagagg ccagttgttt ttgtcacaag gaagaagctg gtgaatgcaa ttcagcagaa     960

gctctccaaa ttgaaaggtg aaccaggatg ggtcaccata catggaatgg caggctgtgg    1020

gaagtctgta ttagctgcag aagctgttag agatcattcc cttttagaag gttgtttccc    1080

agggggagtg cattgggttt cagttgggaa acaagacaaa tctgggcttc tgatgaaact    1140

gcagaatctt tgcacacggt tggatcagga tgagagtttt tcccagaggc ttccacttaa    1200

tattgaagag gctaaagacc gtctccgcat tctgatgctt cgcaaacacc caaggtctct    1260

cttgatcttg gatgatgttt gggactcttg ggtgttgaaa gcttttgaca gtcagtgtca    1320

gattcttctt acaaccagag acaagagtgt tacagattca gtaatgggtc ctaaatatgt    1380

agtccctgtg gagagttcct taggaaagga aaaaggactt gaaattttat ccctttttgt    1440

taatatgaag aaggcagatt tgccagaaca agctcatagt attataaaag aatgtaaagg    1500

ctctcccctt gtagtatctt taattggtgc acttttacgt gattttccca atcgctggga    1560

gtactacctc aaacagcttc agaataagca gtttaagaga ataaggaaat cttcgtctta    1620

tgattatgag gctctagatg aagccatgtc tataagtgtt gaaatgctca gagaagacat    1680

caaagattat tacacagatc tttccatcct tcagaaggac gttaaggtgc ctacaaaggt 1740

gttatgtatt ctctgggaca tggaaactga agaagttgaa gacatactgc aggagtttgt 1800

aaataagtct cttttattct gtgatcggaa tggaaagtcg tttcgttatt atttacatga 1860

tcttcaagta gattttctta cagagaagaa ttgcagccag cttcaggatc tacataagaa 1920

gataatcact cagtttcaga gatatcacca gccgcatact ctttcaccag atcaggaaga 1980

ctgtatgtat tggtacaact ttctggccta tcacatggcc agtgccaaga tgcacaagga 2040

actttgtgct ttaatgtttt ccctggattg gattaaagca aaaacagaac ttgtaggccc 2100

tgctcatctg attcatgaat ttgtggaata cagacatata ctagatgaaa aggattgtgc 2160

agtcagtgag aattttcagg agtttttatc tttaaatgga caccttcttg gacgacagcc 2220

atttcctaat attgtacaac tgggtctctg tgagccggaa acttcagaag tttatcagca 2280

agctaagctg caggccaagc aggaggtcga taatggaatg ctttacctgg aatggataaa 2340

caaaaaaaac atcacgaatc tttcccgctt agttgtccgc ccccacacag atgctgttta 2400

ccatgcctgc ttttctgagg atggtcagag aatagcttct tgtggagctg ataaaacctt 2460

acaggtgttc aaagctgaaa caggagagaa acttctagaa atcaaggctc atgaggatga 2520

agtgctttgt tgtgcattct ctacagatga cagatttata gcaacctgct cagtggataa 2580

aaaagtgaag atttggaatt ctatgactgg ggaactagta cacacctatg atgagcactc 2640

agagcaagtc aattgctgcc atttcaccaa cagtagtcat catcttctct tagccactgg 2700

gtcaagtgac tgcttcctca aactttggga tttgaatcaa aaagaatgtc gaaataccat 2760

gtttggtcat acaaattcag tcaatcactg cagattttca ccagatgata agcttttggc 2820

tagttgttca gctgatggaa ccttaaagct ttgggatgcg acatcagcaa atgagaggaa 2880

aagcattaat gtgaaacagt tcttcctaaa tttggaggac cctcaagagg atatggaagt 2940

gatagtgaag tgttgttcgt ggtctgctga tggtgcaagg ataatggtgg cagcaaaaaa 3000

taaaatcttt ttgtggaata cagactcacg ttcaaaggtg gctgattgca gaggacattt 3060

aagttgggtt catggtgtga tgttttctcc tgatggatca tcatttttga catcttctga 3120

tgaccagaca atcaggctct gggagacaaa gaaagtatgt aagaactctg ctgtaatgtt 3180

aaagcaagaa gtagatgttg tgtttcaaga aaatgaagtg atggtccttg cagttgacca 3240

tataagacgt ctgcaactca ttaatggaag aacaggtcag attgattatc tgactgaagc 3300

tcaagttagc tgctgttgct taagtccaca tcttcagtac attgcatttg gagatgaaaa 3360

tggagccatt gagattttag aacttgtaaa caatagaatc ttccagtcca ggtttcagca 3420

caagaaaact gtatggcaca tccagttcac agccgatgag aagactctta tttcaagttc 3480

tgatgatgct gaaattcagg tatggaattg gcaattggac aaatgtatct ttctacgagg 3540

ccatcaggaa acagtgaaag actttagact cttgaaaaat tcaagactgc tttcttggtc 3600

atttgatgga acagtgaagg tatggaatat tattactgga aataaagaaa aagactttgt 3660

ctgtcaccag ggtacagtac tttcttgtga catttctcac gatgctacca agttttcatc 3720

tacctctgct gacaagactg caaagatctg gagttttgat ctccttttgc cacttcatga 3780

attgaggggc cacaacggct gtgtgcgctg ctctgccttc tctgtggaca gtaccctgct 3840

ggcaacggga gatgacaatg gagaaatcag gatatggaat gtctcaaacg gtgagcttct 3900

tcatttgtgt gctccgcttt cagaagaagg agctgctacc catggaggct gggtgactga 3960

cctttgcttt tctccagatg gcaaaatgct tatctctgct ggaggatata ttaagtggtg 4020

gaacgttgtc actggggaat cctcacagac cttctacaca aatggaacca atcttaagaa 4080

aatacacgtg tcccctgact tcaaaacata tgtgactgtg gataatcttg gtattttata 4140

tattttacag actttagaat aaaatagtta agcattaatg tagttgaact tttaaatttt 4200

ttgaattgga aaaaaattct aatgaaaccc tgatatcaac tttttataaa gctcttaatt 4260

gttgtgcagt attgcattca ttacaaaagt gtttgtggtt ggatgaataa tattaatgta 4320

gctttttccc aaatgaacat acctttaatc ttgtttttca tgatcatcat taacagtttg 4380

tccttaggat gcaaatgaaa atgtgaatac ataccttgtt gtactgttgg taaaattctg 4440

tcttgatgca ttcaaaatgg ttgacataat taatgagaag aatttggaag aaattggtat 4500

tttaatactg tctgtattta ttactgttat gcaggctgtg cctcagggta gcagtggcct 4560

gctttttgaa ccacacttac cccaaggggg ttttgttctc ctaaatacaa tcttagaggt 4620

tttttgcact ctttaaattt gctttaaaaa tattgtgtct gtgtgcatag tctgcagcat 4680

ttcctttaat tgactcaata agtgagtctt ggatttagca ggccccccca cctttttttt 4740

ttgtttttgg agacagagtc ttgctttgtt gccaggctgg agtgcagtgg cgcgatctcg 4800

gctcaccaca atcgctgcct cctgggttca agcaattctc ctgcctcagc ctcccgagta 4860

gctgggacta caggtgtgcg cacatgccag gctaattttt gtatttttag tagagacggg 4920

gtttcaccat gttggccggg atggtctcga tctcttgacc tcatgatcta cccgccttgg 4980

cctcccaaag tgctgagatt acaggcgtga gccaccgtgc ctggccaggc cccttctctt 5040

ttaatggaga cagggtcttg cactatcacc caggctggag tgcagtggca taatcatacc 5100

tcattgcagc ctcagactcc tgggttcaag caatcctcct gcctcagcct cccaagtagc 5160

tgagactaca ggcacgagcc accacaccca gctaattttt aagttttctt gtagagacag 5220

ggtctcacta tgttgtctag gctggtcttg aactcttggc ctcaagtaat cctcctgcct 5280

cagcctccca aagtgttggg attgcagata tgagccactg gcctggcctt cagcagttct 5340

ttttgtgaag taaaacttgt atgttggaaa gagtagattt tattggtcta cccttttctc 5400

actgtagctg ctggcagccc tgtgccatat ctggactcta gttgtcagta tctgagttgg 5460

acactattcc tgctccctct tgtttcttac atatcagact tcttacttga atgaaacctg 5520

atctttccta atcctcactt ttttcttttt taaaaagcag tttctccact gctaaatgtt 5580

agtcattgag gtggggccaa ttttaatcat aagccttaat aagatttttc taagaaatgt 5640

gaaatagaac aattttcatc taattccatt tacttttaga tgaatggcat tgtgaatgcc 5700

attcttttaa tgaatttcaa gagaattctc tggttttctg tgtaattcca gatgagtcac 5760

tgtaactcta gaagattaac cttccagcca acctattttc ctttcccttg tctctctcat 5820

cctcttttcc ttccttcttt cctttctctt cttttatctc caaggttaat caggaaaaat 5880

agcttttgac aggggaaaaa actcaataac tagctatttt tgacctcctg atcaggaact 5940

ttagttgaag cgtaaatcta aagaaacatt ttctctgaaa tatattatta agggcaatgg 6000

agataaatta atagtagatg tggttcccag aaaatataat caaaattcaa agattttttt 6060

tgtttctgta actggaacta aatcaaatga ttactagtgt taatagtaga taacttgttt 6120

ttattgttgg tgcatattag tataactgtg gggtaggtcg gggagagggt aagggaatag 6180

atcactcaga tgtattttag ataagctatt tagcctttga tggaatcata aatacagtga 6240

atacaatcct ttgcattgtt aaggaggttt tttgttttta aatggtgggt caaggagcta 6300

gtttacaggc ttactgtgat ttaagcaaat gtgaaaagtg aaaccttaat tttatcaaaa 6360

gaaatttctg taaatggtat gtctccttag aatacccaaa tcataatttt atttgtacac 6420

actgttaggg gctcatctca tgtaggcaga gtataaagta ttaccttttg gaattaaaag 6480

ccactgactg ttataaagta taacaacaca catcaggttt taaaaagcct tgaatggccc 6540

ttgtcttaaa aagaaattag gagccaggtg cggtggcacg tgcctgtaat cccagctcct 6600

tgggaggcta agacaggagg attccttgag ccctggagtt tgagtccagc ctgggtgaca 6660

tagcaagacc ctgtcttaaa agaaaaatgg gaagaaagac aaggtaacat gaagaaagaa 6720

gagataccta gtatgatgga gctgcaaatt tcatggcagt tcatgcagtc ggtcaagagg 6780

aggattttgt tttgtagttt gcagatgagc atttctaaag cattttccct tgctgtattt 6840

ttttgtatta taaattacat tggacttcat atatataatt tttttttaca ttatatgtct 6900

cttgtatgtt ttgaaactct tgtatttatg atatagctta tatgattttt ttgccttggt 6960

atacatttta aaatatgaat ttaaaaaatt tttgtaaaaa taaaattcac aaaattgttt 7020

tgaaaaacaa aaaaaaaaaa aa 7042

<210> 131
<211> 7852
<212> DNA
<213> Homo sapiens

<400> 131
ggcgctgagc gagctcggag cccgcgctgt gcgcctgcgg ccggggcgcc ccgccgagcg 60

ccggtgcccc ggctcccggg ccgccttcgc cgcgcgggaa ggattcttca aaattaacag 120

aaaccaattc gggccagctg aagagaaaaa ataaaggtgg ctcccggctg cctctgctgc 180

agttcagagc aacttcagga gcttcccagc cgagagcttc aggacgcctt tcctgtccca 240

ctggcccagt tgccacaaca aacaacagag aagacggtga ccatggggga tgtgaagctg 300

gttgcctcgt cacacatttc caaaacctcc ctcagtgtgg atccctcaag agttgactcc 360

atgcccctga cagaggcccc tgctttcatt ttgccccctc ggaacctctg catcaaagaa 420

ggagccaccg ccaagttcga agggcgggtc cggggttacc cagagcccca ggtgacatgg 480

cacagaaacg ggcaacccat caccagcggg ggccgcttcc tgctggattg cggcatccgg 540

gggactttca gccttgtgat tcatgctgtc catgaggagg acaggggaaa gtatacctgt 600

gaagccacca atggcagtgg tgctcgccag gtgacagtgg agttgacagt agaaggaagt 660

tttgcgaagc agcttggtca gcctgttgtt tccaaaacct taggggatag attttcagct 720

ccagcagtgg agacccgtcc tagcatctgg ggggagtgcc caccaaagtt tgctaccaag 780

ctgggccgag ttgtggtcaa agaaggacag atgggacgat tctcctgcaa gatcactggc 840

cggccccaac cgcaggtcac ctggctcaag ggaaatgttc cactgcagcc gagtgcccgt 900

gtgtctgtgt ctgagaagaa cggcatgcag gttctggaaa tccatggagt caaccaagat 960

gacgtgggag tgtacacgtg cctggtggtg aacgggtcgg ggaaggcctc gatgtcagct 1020

gaactttcca tccaaggttt ggacagtgcc aataggtcat ttgtgagaga aacaaaagcc 1080

accaattcag atgtcaggaa agaggtgacc aatgtaatct caaaggagtc gaagctggac 1140

agtctggagg ctgcagccaa aagcaagaac tgctccagcc cccagagagg tggctcccca 1200

ccctgggctg caaacagcca gcctcagccc ccaagggagt ccaagctgga gtcatgcaag 1260

gactcgccca gaacggcccc gcagaccccg gtccttcaga agacttccag ctccatcacc 1320

ctgcaggccg caagagttca gccggaacca agagcaccag gcctgggggt cctatcacct 1380

tctggagaag agaggaagag gccagctcct ccccgtccag ccaccttccc caccaggcag 1440

cctggcctgg ggagccaaga tgttgtgagc aaggctgcta acaggagaat ccccatggag 1500

ggccagaggg attcagcatt ccccaaattt gagagcaagc cccaaagcca ggaggtcaag 1560

gaaaatcaaa ctgtcaagtt cagatgtgaa gtttccggga ttccaaagcc tgaagtggcc 1620

tggttcctgg aaggcacccc cgtgaggaga caggaaggca gcattgaggt ttatgaagat 1680

gctggctccc attacctctg cctgctgaaa gcccggacca gggacagtgg gacatacagc 1740

tgcactgctt ccaacgccca aggccagctg tcctgtagct ggaccctcca agtggaaagg 1800

cttgccgtga tggaggtggc cccctccttc tccagtgtcc tgaaggactg cgctgttatt 1860

gagggccagg attttgtgct gcagtgctcc gtacggggga ccccagtgcc ccggatcact 1920

tggctgctga atgggcagcc catccagtac gctcgctcca cctgcgaggc cggcgtggct 1980

gagctccaca tccaggatgc cctgccggag gaccatggca cctacacctg cctagctgag 2040

aatgccttgg ggcaggtgtc ctgcagcgcc tgggtcaccg tccatgaaaa gaagagtagc 2100

aggaagagtg agtaccttct gcctgtggct cccagcaagc ccactgcacc catcttcctg 2160

cagggcctct ctgatctcaa agtcatggat ggaagccagg tcactatgac tgtccaagtg 2220

tcagggaatc caccccctga agtcatctgg ctgcacaatg ggaatgagat ccaagagtca 2280

gaggacttcc actttgaaca gagaggaact cagcacagcc tttgtatcca ggaagtgttc 2340

ccggaggaca cgggcacgta cacctgcgag gcctggaaca gcgctggaga ggtccgcacc 2400

caggccgtgc tcacggtaca agagcctcac gatggcaccc agccctggtt catcagtaag 2460

cctcgctcag tgacagcctc cctgggccag agtgtcctca tctcctgcgc catagctggt 2520

gacccctttc ctaccgtgca ctggctcaga gatggcaaag ccctctgcaa agacactggc 2580

cacttcgagg tgcttcagaa tgaggacgtg ttcaccctgg ttctaaagaa ggtgcagccc 2640

tggcatgccg gccagtatga gatcctgctc aagaaccggg ttggcgaatg cagttgccag 2700

gtgtcactga tgctacagaa cagctctgcc agagcccttc cacgggggag ggagcctgcc 2760

agctgcgagg acctctgtgg tggaggagtt ggtgctgatg gtggtggtag tgaccgctat 2820

gggtccctga ggcctggctg gccagcaaga gggcagggtt ggctagagga ggaagacggc 2880

gaggacgtgc gaggggtgct gaagaggcgc gtggagacga ggcagcacac tgaggaggcg 2940

atccgccagc aggaggtgga gcagctggac ttccgagacc tcctggggaa gaaggtgagt 3000

acaaagaccc tatcggaaga cgacctgaag gagatcccag ccgagcagat ggatttccgt 3060

gccaacctgc agcggcaagt gaagccaaag actgtgtctg aggaagagag gaaggtgcac 3120

agcccccagc aggtcgattt tcgctctgtc ctggccaaga aggggacttc caagaccccc 3180

gtgcctgaga aggtgccacc gccaaaacct gccaccccgg attttcgctc agtgctgggt 3240

ggcaagaaga aattaccagc agagaatggc agcagcagtg ccgagaccct gaatgccaag 3300

gcagtggaga gttccaagcc cctgagcaat gcacagcctt cagggccctt gaaacccgtg 3360

ggcaacgcca agcctgctga gaccctgaag ccaatgggca acgccaagcc tgccgagacc 3420

ctgaagccca tgggcaatgc caagcctgat gagaacctga aatccgctag caaagaagaa 3480

ctcaagaaag acgttaagaa tgatgtgaac tgcaagagag gccatgcagg gaccacagat 3540

aatgaaaaga gatcagagag ccaggggaca gccccagcct tcaagcagaa gctgcaagat 3600

gttcatgtgg cagagggcaa gaagctgctg ctccagtgcc aggtgtcttc tgacccccca 3660

gccaccatca tctggacgct gaacggaaag accctcaaga ccaccaagtt catcatcctc 3720

tcccaggaag gctcactctg ctccgtctcc atcgagaagg cactgcctga ggacagaggc 3780

ttatacaagt gtgtagccaa gaatgacgct ggccaggcgg agtgctcctg ccaagtcacc 3840

gtggatgatg ctccagccag tgagaacacc aaggccccag agatgaaatc ccggaggccc 3900

aagagctctc ttcctcccgt gctaggaact gagagtgatg cgactgtgaa aaagaaacct 3960

gcccccaaga cacctccgaa ggcagcaatg ccccctcaga tcatccagtt ccctgaggac 4020

cagaaggtac gcgcaggaga gtcagtggag ctgtttggca aagtgacagg cactcagccc 4080

atcacctgta cctggatgaa gttccgaaag cagatccagg aaagcgagca catgaaggtg 4140

gagaacagcg agaatggcag caagctcacc atcctggccg cgcgccagga gcactgcggc 4200

tgctacacac tgctggtgga gaacaagctg ggcagcaggc aggcccaggt caacctcact 4260

gtcgtggata agccagaccc cccagctggc acaccttgtg cctctgacat tcggagctcc 4320

tcactgaccc tgtcctggta tggctcctca tatgatgggg gcagtgctgt acagtcctac 4380

agcatcgaga tctgggactc agccaacaag acgtggaagg aactagccac atgccgcagc 4440

acctctttca acgtccagga cctgctgcct gaccacgaat ataagttccg tgtacgtgca 4500

atcaacgtgt atggaaccag tgagccaagc caggagtctg aactcacaac ggtaggagag 4560

aaacctgaag agccgaagga tgaagtggag gtgtcagatg atgatgagaa ggagcccgag 4620

gttgattacc ggacagtgac aatcaatact gaacaaaaag tatctgactt ctacgacatt 4680

gaggagagat taggatctgg gaaatttgga caggtctttc gacttgtaga aaagaaaact 4740

cgaaaagtct gggcagggaa gttcttcaag gcatattcag caaaagagaa agagaatatc 4800

cggcaggaga ttagcatcat gaactgcctc caccacccta agctggtcca gtgtgtggat 4860

gcctttgaag aaaaggccaa catcgtcatg gtcctggaga tcgtgtcagg aggggagctg 4920

tttgagcgca tcattgacga ggactttgag ctgacggagc gtgagtgcat caagtacatg 4980

cggcagatct cggagggagt ggagtacatc cacaagcagg gcatcgtgca cctggacctc 5040

aagccggaga acatcatgtg tgtcaacaag acgggcacca ggatcaagct catcgacttt 5100

ggtctggcca ggaggctgga gaatgcgggg tctctgaagg tcctctttgg caccccagaa 5160

tttgtggctc ctgaagtgat caactatgag cccatcggct acgccacaga catgtggagc 5220

atcggggtca tctgctacat cctagtcagt ggcctttccc ccttcatggg agacaacgat 5280

aacgaaacct tggccaacgt tacctcagcc acctgggact tcgacgacga ggcattcgat 5340

gagatctccg acgatgccaa ggatttcatc agcaatctgc tgaagaaaga tatgaaaaac 5400

cgcctggact gcacgcagtg ccttcagcat ccatggctaa tgaaagatac caagaacatg 5460

gaggccaaga aactctccaa ggaccggatg aagaagtaca tggcaagaag gaaatggcag 5520

aaaacgggca atgctgtgag agccattgga agactgtcct ctatggcaat gatctcaggg 5580

ctcagtggca ggaaatcctc aacagggtca ccaaccagcc cgctcaatgc agaaaaacta 5640

gaatctgaag aagatgtgtc ccaagctttc cttgaggctg ttgctgagga aaagcctcat 5700

gtaaaaccct atttctctaa gaccattcgc gatttagaag ttgtggaggg aagtgctgct 5760

agatttgact gcaagattga aggataccca gaccccgagg ttgtctggtt caaagatgac 5820

cagtcaatca gggagtcccg ccacttccag atagactacg atgaggacgg gaactgctct 5880

ttaattatta gtgatgtttg cggggatgac gatgccaagt acacctgcaa ggctgtcaac 5940

agtcttggag aagccacctg cacagcagag ctcattgtgg aaacgatgga ggaaggtgaa 6000

ggggaagggg aagaggaaga agagtgaaac aaagccagag aaaagcagtt tctaagtcat 6060

attaaaagga ctatttctct aaaactcaaa aaaaaaaaaa aaactcaaga tagtaaaagc 6120

acctagtgtg atagattatc ggttaggtca tttgtgggtt gattcttcag aaacagcagt 6180

tgatacctag cagcgttatt gatgggcatt aatctatgtt agttggcacc ttaagatact 6240

agtgcagcta gatttcattt agggaaatca ccagtaactt gactgaccaa ttgattttag 6300

agagaaagta accaaaccaa atatttatct gggcaaagtc ataaattctc cacttgaatg 6360

cgctcatgaa aaataaggcc aaaacaagag ttctggccca cagctcagcc cagagggttc 6420

ctggggatgg gaggcctctc tctccccacc ccctgactct agagaactgg gttttctccc 6480

agtactccag caattcattt ctgaaagcag ttgagccact ttattccaaa gtacactgca 6540

gatgttcaaa ctctccattt ctctttcccc ttccacctgc cagttttgct gactctcaac 6600

ttgtcatgag tgtaagcatt aaggacatta tgcttcttcg attctgaaga caggtccctg 6660

ctcatggatg actctggctt ccttaggaaa atatttttct tccaaaatca gtaggaaatc 6720

taaacttatc ccctctttgc agatgtctag cagcttcaga catttggtta agaacccatg 6780

ggaaaaaaaa aatccttgct aatgtggttt cctttgtaaa ccaggattct tatttgtgct 6840

gttatagaat atcagctctg aacgtgtggt aaagattttt gtgtttgaat ataggagaaa 6900

tcagtttgct gaaaagttag tcttaattat ctattggcca cgatgaaaca gatttcaact 6960

gataaagagc tggagaactc catgtacttt ggaatctcct ccaagatagc cagagtttaa 7020

tacatcttca ttctcaacac tctccaaaga acttgaccta ccttatgggt tccatatttt 7080

tcttcttaaa tgtgcatcaa tcatgccttg cccccaacct ttaaatatat tcttagacct 7140

ggtaaatgca ctcagacttg cgtctttagg aatttttaac tttctttcac tacattggca 7200

cttaaatttt ttctttataa agctttttga aggtcataaa caaagaccat aattgatgat 7260

agacctaata catttcctct gtgtgtgtgt gtaacattcc aaatactttt tttttctttt 7320

ccactgtttg taaggtgcaa caatttaata tttttaaggg actttttaag agttccttaa 7380

gaaccaattt aaaattactt cagtgcaatc ctacacagta tcaacattag aattttgata 7440

ttagtcttat gttatcttcc attctatttt tatctgcttt ttgctgctag tttcaaactg 7500

ccagtatttt tccttttgct tttaaaatag ttacaatatt tttcatgata gccacagtat 7560

tgccacagtt tattataata aagggttttt atttgattta gcgcattcaa agcttttttc 7620

tatcactttt gtgttcagaa tataaccttt gtgtgcgtgt atgttgtgtg tgtgcatgtg 7680

tggcgtatat gtgtgttaca ggttaatgcc ttcttggaat tgtgttaatg ttctcttggt 7740

ttattatgcc atcagaatgg taaatgagaa cactacaact gtagtcagct cacaattttt 7800

aaataaagga taccacagtg catgctgttt gttcaaaaaa aaaaaaaaaa aa 7852

<210> 132
<211> 1074
<212> DNA
<213> Homo sapiens

<400> 132
tggaagcggc actcaagatg ctgcgccgcg aggcccgcct gcgccgcgag tacctgtacc 60

gcaaggcccg ggaggaggcg cagcgctcag cccaggagag gaaggagcgg ctgcggcgcg 120

cgctggaaga aaaccgcctg attcccactg agttacgccg agaggctctg gccttacagg 180

ggtccctgga gtttgatgat gctggaggtg aaggtgtgac cagccacgtg gatgatgaat 240

accgatgggc aggagtcgag gatcccaagg ttatgatcac tacctcccga gaccccagtt 300

cccgcctcaa gatgtttgca aaggagctga agctggtgtt cccgggcgcc cagcgaatga 360

accgaggtcg acatgaagtg ggggcactgg tgcgagcctg caaagccaac ggcgtcaccg 420

atctgctggt cgttcacgag catcggggca cacctgtggg gctcatcgtc agccacctgc 480

cctttggtcc tactgcctac ttcacgctgt gcaatgtggt catgcggcat gacatcccag 540

acctgggcac catgtcggag gccaagcccc acctcatcac acacggcttc tcctcccgcc 600

tgggcaagcg ggtctctgac atcctccgat acctatttcc cgtgcccaaa gatgacagcc 660

accgggtcat caccttcgca aaccaggacg actacatatc attccggcac catgtgtata 720

agaagacaga ccaccgcaac gtggagctca ctgaggtcgg gccccgcttt gagctgaagc 780

tgtacatgat ccgtctgggc acgctggagc aggaggccac agcagacgtg gagtggcgct 840

ggcaccctta caccaatacc gcacgcaaga gagtcttcct gagcaccgag tgagcacact 900

caccactcag tcaggacatg gacttggaac tcaggatggg gctgtcatag acagacccac 960

cagtaggaac tgtcacagaa tggcctgctg aactgggatg tggaactgtg gcgggtggag 1020

aggtctgaat aaaccgtctg tgtcatggca aaaaaaaaaa aaaaaaaaaa aaaa 1074

<210> 133
<211> 3220
<212> DNA
<213> Homo sapiens

<400> 133
ccgcgccggc tagcggagga gagtaaatac aacaggagcg caaaatggcg gaaccgccga 60

gccccgtgca ctgtgtcgct gccgcggccc ccaccgccac cgtctcggag aaagaaccgt 120

ttggcaagct gcaactctcc tcccgggacc ctccgggttc tctgtccgcc aagaaggtcc 180

ggactgagga gaagaaggca ccgcggagag tgaacggaga agggggcagc ggcgggaaca 240

gcaggcagct gcagccgccg gcagcacctt cgcctcagag ctatggcagc cccgcgtctt 300

ggagctttgc ccctctgtct gctgctccct ccccgtcctc ttctcggagc agtttctctt 360

tctccgctgg cacggccgtt ccctcctcag cctccgcttc cttgtctcag ccggtgccgc 420

gcaaactgct ggtccctcct acgctgctgc acgctcagcc tcaccatctc ctcctgcccg 480

ccgccgccgc cgctgcctcg gctaacgcca agtcgcgcag acctaaggag aagcgggaga 540

aggagaggag gaggcacggt ctcggtgggg cccgagaggc cggcggggcc tcccgggagg 600

agaacgggga ggtgaagccg ctgccccgag ataaaatcaa agacaaaatt aaagagagag 660

acaaagaaaa agaaagagaa aaaaagaaac ataaagtaat gaatgagatc aagaaagaga 720

atggagaagt aaagattttg ctgaaaagtg ggaaggagaa accaaaaaca aatatagaag 780

acttacaaat taaaaaggta aagaagaaaa agaaaaagaa acacaaagag aatgaaaaac 840

ggaagcgtcc gaaaatgtat agcaaatcta ttcagaccat ctgctcagga ttgctaactg 900

atgttgaaga tcaagcagcc aaaggcatcc taaatgataa cataaaagat tacgttggga 960

agaatttgga taccaagaac tatgattcca aaattccaga gaacagtgag tttccatttg 1020

tctcattaaa ggagccacga gttcagaata acctcaaaag gttggacact ttggaattta 1080

aacaactcat tcatatagag caccagccta atggaggtgc atcggttatc catgcctaca 1140

gtaacgaact ctcccacctg tctcctatgg agatggagag gtttgcagaa gagtttgtgg 1200

gtctagtgtt cagtgaaaat gaaaactctg cagctttcta cgtgatgggt attgttcatg 1260

gggcagctac ttatttacct gactttttag actatttttc atttaatttt cccaattcac 1320

cagtgaaaat ggagatattg ggaaagaaag atatagagac aacgactatg tccaattttc 1380

atgctcaggt aaaaagaacg tattctcatg gtacttacag agctggccca atgagacaaa 1440

taagcttggt gggagcagtt gatgaagaag taggagatta tttccctgag ttccttgaca 1500

tgttggaaga gtcaccattt ttaaaatgta cactgccatg ggggacgcta tctagtctaa 1560

aattacagag tcgaaaagat agtgatgatg gtcccatcat gtgggttcgt ccaggagaac 1620

aaatgatccc tgtggctgat atgccaaagt cacctttcaa aaggaaaaga actaccaatg 1680

aaataaaaaa tcttcagtac ctacctcgaa caagtgagcc ccgtgagatg ctctttgaag 1740

acaggacaag agctcatgca gatcatatag gacaaggttt tgaacgacag actacagctg 1800

ctgttggagt gctgaaggct gtgcactgtg gagagtggcc tgatcaaccc cgtataacca 1860

aagatgtaat ttgttttcat gctgaagatt tcttagaagt agttcaacga atgcagttag 1920

atttacatga acctccactg tcccagtgtg tccaatgggt tgatgatgca aaactgaatc 1980

aactgaggag ggaaggcatt cgctatgcca ggattcagct atatgataat gacatttatt 2040

ttattccaag gaatgttgtt catcagttca agacagtttc agctgtatgc agtttagcat 2100

ggcatattcg gctcaaatta tatcactcag aggaggacac ttctcagaat acagctactc 2160

atgaaacagg cacatcatca gattccacat catctgttct tggacctcac actgacaaca 2220

tgatttgtgc tgtaagcaaa gcctccttgg attctgtttt ttcagataaa cttcattcta 2280

aatatgaatt acagcagatt aaacatgaac ctattgcatc tgtaagaatc aaggaagaac 2340

ctgtgaatgt taatattcct gaaaagacta cagcactgaa taatatggat ggcaagaatg 2400

ttaaagcaaa attggatcat gttcaatttg cagaatttaa gattgacatg gattctaaat 2460

ttgaaaatag caacaaagat ttaaaggaag aattgtgccc tggaaatcta agtctagttg 2520

atacaaggca acacagttca gcacattcaa atcaagataa aaaagacgat gacattttgt 2580

gctaaatttg catataccat ctaaaatcct tttttaaaaa aatttaatgt aataaagatt 2640

catgaattct gaaagcaagc caaggacttg ctcctatgtc tgttacaaaa catagtttat 2700

gtagctttgt aacattcctc agtgcctgtc cataactgtg aagtattaag cacttagggc 2760

cagatgcact gtaaacattg caggtttaaa cataaaggag tctttaaaaa aaaatcattt 2820

acgttggaat tttaggtttt agaatagagc tgacattaac atatatatat atatataaat 2880

atatatatat attttgtaat atgagccaga attctttttc aacaatttaa agcttttcca 2940

tagagcttat ttatatcctt ttttttcatt ttaaatgtgt cagcactgta gtgtaaatag 3000

cttttaaata tctttttagt gtgatttata ctgaaatgtg agccacttaa taaaggttca 3060

tatgttcata ttaataaata tgttttctgt tgagtctgta aagacaaact gacaattcat 3120

ttaagtcatt tgataatgat tctctgctgg tatatctatt tagtgtggta ttgtagtgca 3180

aatgtacgta attcaaatct agtaaatatt taagttcctc 3220

<210> 134
<211> 534
<212> DNA
<213> Homo sapiens

<400> 134
atgacaaaaa tctaccaact caaccacgta cctttctccg gcttcactct agaggaacca 60

tggaggaatt cactccggtg tatatccgaa atgaataggc tgttttctgg aaaagcagac 120

ataaccaagc cagggtacga tccctgcaat ctattggttg acctggatga catcagagac 180

ctgtcttctg ggttcagcaa ataccgagac ttcatccgtt acctgcccat ccacctctcc 240

aagtacattc taagaatgct ggatagacac accctgaaca agtgcgcctc tgtgagccag 300

cactgggccg ccatggctca acaggtcaag atggacttgt cagcgcacgg cttcattcag 360

aaccagatta ccttcttgca ggggtcctac acaagaggaa ttgatcctaa ttatgccaat 420

aaggtttcta tcccagttcc taaaatggta gatgacggga agagcatgcg tgtgaaacat 480

ccgaagtgga agttgagaac gaaggtgggt tccaacagca tctggggcaa gtag 534

<210> 135
<211> 1709
<212> DNA
<213> Homo sapiens

<400> 135
cattggcaga gctggaaggt ctgtgcaagc tggctcctgc tggggaatca ataaaggagg 60

cagagcaacg cttttttttt ttttcgccgt ggccagggca gggttctgcc agggctctat 120

cgccaatgga gcaggggtcc acctggggcc taggacgcct gcagatagcc ctctcttagc 180

cccaacggcc gcgggcttca gcgctccccg gctccctcgc tctgcagggt cttcccggta 240

ccggcaggcc gcggcgggag ctgctttcgc tcttaacctt gcaggtgcgt gtgccgcggg 300

cgcccggggc aggcaggacg cagaggagga ggcgcccgcc atgcccggcc tgcggctccc 360

cgcctcggct tcccgtccgc cgcccccgcg gaacggcctg ctgcagcccc tgtgaagagg 420

aagaggcggc ggcggcggcg gaggaggagg aggcgccgcc cccttggcaa gctccccgac 480

ccggcggcct cggcaggacc catggcggat tcctcgcccg cactgtccct gcgggaaggc 540

ggcccccgcg cgccgcggcc ctcggccccc tcgccgccgc cgcgctcgcg ctccggctcc 600

gagtccgagg aggccgagct gtcgctgagc ctggcccgca ccaagacccg ctcgtacggc 660

agcaccgcca gcgtgcgggc gccgctgggc gccggcgtca tcgagcgcca tgtggagcac 720

cgggtccgcg cgggcgacac gctgcagggc atcgcgctca agtacggtgt cacgatggaa 780

cagattaaaa gggccaataa actgtttacc aatgattgta tatttctgaa gaaaactttg 840

aacatcccag ttatatcaga gaagcctttg ttgtttaatg gacttaactc cattgattct 900

ccagaaaatg aaactgctga taacagtttt tctcaggaag aggagccagt ggtggccggg 960

gaagacctcc ctcctcccag tcctcaagaa tctgatgttc agcctgtgca gcctgaggaa 1020

gtgtcagcca gagatttcct gcagagactt gacttgcaga ttaagttatc aacacaggca 1080

gccaagaagc taaaagaaga gagcagagat gaagaaagtc cctatgcaac ttccctctat 1140

cacagttagg tgatttgggg gcataactct aaccaaaatt aagatatgtt tggaccataa 1200

agaatccaac aactgaagat tcaaaaacat cccttctgga ttcctatagt gtccatctta 1260

aaatcataac taggtagttc tacctgctgc agttgcactg aagtgattag ttccctctgg 1320

ctttctataa ttttaagtct ttattatggc atgatggcaa ggttgtagcc taaagctcat 1380

cacttttgta ggtggtgtta gtttttagac tagcttttat aaatactaat tgacataaag 1440

catcaaagat tatttatata tttaggctaa aaataatgtt tatctcctta tgcaatcctg 1500

attatgtgaa aatatggttg ctgtttaagt gatgctgaat ttgctgtgtg tttataactt 1560

aagtgctata tatatatttc gatatgtctt acatattctc tattaatata aagaaccaaa 1620

ttttgtctgc tattttgtaa aaataaacta caaaagcctg aatgagaaaa taaactatgt 1680

tttcaaactt gaaaaaaaaa aaaaaaaaa 1709

<210> 136
<211> 3318
<212> DNA
<213> Homo sapiens

<400> 136
gactgctaag agggggttaa agggggacga tgtgaaggag agaacctgtg gtccttcaga 60

aggcgaagaa gaaagaaagg ggaagcagtg aagaaaggga cggagatact gggacaggga 120

gaaaaaagtt gtggagagta gcttttaagg agtcatttgg tggccatgga tccaacgtgc 180

tcttctgagt gcatttataa cctcataccc agtgacttga aggagcctcc ccagcctcct 240

aggtacatat ccatttttaa ggcaactgta aaagatgaca tgcaaaaagc taaaactgca 300

atgaaaacta tgggaccagc aaaagttgaa gtaccttctc caaaggattt cctaaagaaa 360

cattcaaagg agaaaactct accacccaaa aaaaactttg atcggaacgt gcccaaaaag 420

cctgctgtgc cattgaagac tgatcatcct gtcatgggaa tacagagtgg aaaaaatttt 480

ataaatacaa atgcagctga tatcatcatg ggagtggcta aaaagcctaa accaatttat 540

gttgataaaa gaactggaga caagcatgat cttgagcctt caggactagt tccaaagtac 600

atcaataaaa aggattatgg tgtcacacct gaatacatat gtaagcgaaa cgaggaaata 660

aagaaagccc aagaagacta tgatcgttat atccaggaaa accttaagaa agcagctatg 720

aaaaggctct ccgatgaaga aagggaggca gttttgcagg ggctgaaaaa gaactgggaa 780

gaggtgcata aagaattcca gtccctctcg gtctttatag attctatacc aaagaagatc 840

cgcaagcaga ggctggaaga agaaatgaaa caactagaac acgacattgg cataattgaa 900

aagcacaaga ttatttatat tgccaataac gcatgatacc aacagcacaa ctttgaaaac 960

tgttaagtat tttgaactgg aaatagtaac acgtgccttc ttgctactct aaactgtctc 1020

caacacattg tttctgagct ccacaaatgc aaaagatgta ttttcctgtg gcccatgaca 1080

tttgcagtta ttcccagtga gaaatatttt cagtattcgt aacactgtat atgatgaaag 1140

gcttctattt ggaggaagaa aaattaagaa gacaacttac aaaatcaaga tgacaactta 1200

taaaggcatc attgaggatg attaaggaga atagcctcct ttctagcata ccaacctcga 1260

tactcttatt ttattccaca actcactatg catatcttat ttaaatgttt ttttcctttt 1320

ggtcctttct acttatctat tttgtgaatt ctaaagttac atttgtaaga ttacataatc 1380

tattaagcaa taagtttatg ttagtagagc tgttttcaac ccaaaaataa gaaaaaatag 1440

ctctaaatat ctaaaaggaa aaggttattt tatgaaattt taaattagaa aaatttggta 1500

aaatatatgc aactataaaa tatcagagaa ataacaattt ctactgctta cactggtttt 1560

tatcctctgg acaacataaa aaacaaaata tgggagtctt tcaggctttt ttcacaaaca 1620

cagaatttgg aagccttata gaaaagctta ttttggttac ttaaagaaaa gtagtttata 1680

tacaattatt ttttgaaaat tcacttgaaa cttgtccata tgagtatctt tattccttta 1740

ttaacttagg gcagatataa aataaaggat aatatatgac aaattctcac ttaccaagaa 1800

tggttggttg ctatttaggg taggctaaca gctgtgacag tacactccaa aatgtataat 1860

tacgtaaaca catattcaat tcttgctaac atagcagtac tttgctgtga gcaggtcagc 1920

aggaaccctt ttctgcagtc actcagggac ccagacttac agagtgggta ggatgcatct 1980

gatccagagt tagggagagg cacgtacagg ccaggcctag aaatgatgca tatcattccc 2040

gtttgcatcg ctaagtaagc aacttaagtt gctttaacaa actaactcca aatctcagtt 2100

tcctgataaa gatacatttt tcatttacat cactgtgcaa tgtgactaag ggtaggggta 2160

taagtctgtt ccatgcagtc actcagggac ccagtctcct tttgtctggt gactctgttg 2220

atccaggcag tctttctctg ccaattcagg atgagatgtt taggtaagta ttttatttgt 2280

gaaatgattg ctatacatac tcatttttaa ataattcaaa atcagtagca atgcaaaaat 2340

attctgatta ctttagcctt atggatatta tgctttgaag tcatgagtgg gagttgcctt 2400

aaatgttaaa atgaaaactt tgtggtttta acagctaatt cttttgcttt ctgtaaatgt 2460

ctactaaaga atctagactt ttctctatgg tctacagtgt tgtcctagat gaacattgtt 2520

gcctttcttt cacagagcct tcagagggaa acattcccat ccattgttcc tggaaaggtg 2580

atccctacca cgggatccca aacttggata gagccagttc aacctccccc aatccataca 2640

catacatacc taggtcatcc tatcacccaa aagagggaca aacatctgac ttggacagct 2700

gaccagtgac tgatcagatt ctctctctct tgagagcatg aattaagaga catggagact 2760

gcagtcactt gggagtagac acctgaaact aaaaagtcaa acttcattat gcagctagtt 2820

tgacttggtt tctcttcctg ttcaaaggaa gaatgggtag aataaagatt tgatgtggtc 2880

tggccccttg tctatgatct ttttagcttg atttcttgaa atgctgcttc ttccctaccc 2940

actgtaactg gcattcccat agtactgagg tactagagac ctctgagatc tgcacacatg 3000

gttccctcca cccagaaaac tgcccttttc tttctctcat cctacatagc tgcctggcaa 3060

atccctagtc ttcatcagct cacatcacat ttccatgaca atcccctttg attcctcaga 3120

atctggcctt cctttatagc actggtcata ttgtattgtg catatctggt tatttgcctg 3180

ttttccatga acatttcaag cttatggggg agaagacact gtatacattt tcatctgcat 3240

tccttagaat ctgcataaca gctggtatag agaaggtact caaaaaatcc ttaatgaata 3300

aagtaatgga tcgtaccc 3318

<210> 137
<211> 5144
<212> DNA
<213> Homo sapiens

<400> 137
catgagggac cccagactgc gttaggcagc agatgaccgc ctgaatctag atggagagaa 60

acccaccatt tttctaggcc ctggattatg tagaagattt agatactaag cctcaagaga 120

cgtctttcct ctcatttacg gtcttcctgc atatttaaaa tttctaatac ggtagacaga 180

gtcggtgtct ttgaagctgt taatcaggca atttcctggg cactccagct gcgggtcctc 240

gcggccttct cggcttctcc agcttcggta ggagaggatc cggcgccgaa tcactgactg 300

gcacaggtgt tgggaaaatg atccacagtc tatttctcat aaactgttcc ggtgacatat 360

ttctagagaa gcactggaag agcgttgtga gccagtctgt ctgtgattat ttctttgaag 420

ctcaagagaa agctgctgat gttgaaaatg taccacctgt catttcaaca cctcaccact 480

acctcatcag tatctaccgg gataagctct tctttgtatc tgtcatacag accgaagtgc 540

cacctctctt tgtaattgag ttcctacatc gagttgctga cacttttcag gactactttg 600

gtgagtgttc agaggctgca attaaggata atgtggtcat agtatatgaa ctcttagaag 660

aaatgttaga caatggattt ccactggcta ccgaatctaa cattttgaaa gaattgatta 720

aaccaccaac aattctacgc tctgttgtca actctattac aggcagtagt aatgttgggg 780

acacactccc caccgggcag ctgtccaaca taccatggcg tcgggcaggg gtaaagtaca 840

caaacaatga agcctatttt gatgttgttg aagaaataga cgcaattata gataaatcag 900

gatctacagt ctttgcagaa attcaggggg tcattgatgc ttgcattaaa ctatctggaa 960

tgcctgatct ctccctttct ttcatgaacc ctaggcttct ggatgatgtc agctttcacc 1020

cctgcatccg gttcaagcgt tgggaatctg aaagagtttt gtcatttatt cctccagatg 1080

gaaatttccg actcatatca taccgtgtca gctcacaaaa tctagtggca ataccagtgt 1140

atgtgaaaca tagtatcagc tttaaggaga acagttcttg cggcagattt gatataacaa 1200

ttggaccaaa gcagaatatg gggaaaacta ttgaaggaat tacagtgaca gttcacatgc 1260

caaaagttgt gctgaacatg aacctgacac ccacacaagg cagctataca tttgatccag 1320

tcaccaaggt actaacatgg gatgtgggaa aaattactcc acaaaagctc ccaagtctta 1380

aaggactggt aaatttacag tctggagccc ccaaaccaga agagaatccg agcctcaaca 1440

tacagtttaa gatccagcag cttgctattt caggcttaaa agtaaaccgt ttggacatgt 1500

atggggagaa atataagcca tttaaaggag tcaaatacgt cacgaaagct ggaaagttcc 1560

aagtgaggac atgagaagag gccaaaattc ctcaggacca gtttgttttc caagtgtcat 1620

tacgatgtat cactattagg taccaagtga gtgggaatac atattctagt taaagcattt 1680

gtgtctagct acacaccgct aacaaagtta cttagttatc aatgtaggat tcttaaggag 1740

ctttaagcta aggaaacctt ttagtgactt agcttatttt gtatcttttc acttaggaag 1800

attttggagg tgattttttt ccataggagg ataccatctg gcggctgcac attgtaacag 1860

taaaggcaga aagctgtagt gataacctct ctcctaaaag agttaactgg tctcatccag 1920

cagaagctat cttaaatctg tgatgtgtca ggtgcagcca aatatcacac cttctgatct 1980

tagccatccc aaaccagtat ctgtcccgag aggaaattcc ccccaccccc agaagtttac 2040

agaaaactgc ctcttcaagt gtttgcctta ttcagctttt cacttgtgcc attaagcaag 2100

cactgtagca aaagccactt ccacatggcc ctggcaggga gcactgctgc tccatgctcc 2160

attctcactg tacttggtat tgtatttttt ataaataaga tttttatgta aagcttagaa 2220

tttgatttac agggaccttg ctgcagtaaa taccatctca attttgtgcc actggttcag 2280

ctgttagcac agtaaaaaaa tcatttgtat caaaggggca aatgctttat taaggtagta 2340

aaagggaaca ttacttctgc ttttaggaag ttactgcaag cacaagcatt tgtgctttta 2400

agcaaattaa agtagtaaaa gaaaaactta agtgaaacct ttgccatctt catgttttat 2460

aatataaagc ttacccaaca ccagttaagc catggttaac ctaaatgcct catgccccag 2520

ttcagcaaaa ggaggaaaat gtgcctgcct cacagtcatc agtcttttta aatctttttt 2580

gttgttgttc ttaagggttt gaatttgtct gcattccttg tctttagggg aaattccctt 2640

ttcatattgt gtgcttccca aagctatagt catagatttc ttccagaaac tattgtcata 2700

attgtcactg gagtgcttaa atatacgtac tatactgaca aaatacatgg aagtgagtta 2760

taatgaggca gaaacaaaat cctcggtaac attgatgata ctctaccgat caccgtggtt 2820

ttggaaagtc agtcaacagt tgtattattg cactcaattt cattgtgaca ttttatttaa 2880

cttcttcatc ttggtggtcc ttgcccagtt attttgcctc attagacatc aagaaatgga 2940

gaaagactga aagttaatat cttaagtgct tgttcttcat gtttccttct tgttatttat 3000

gctattctct ttgtggctcc attcttcttt caatcttctc agcttataac cgtctttccc 3060

ttatgctaag gatagccctt acactcatcc catctatgct gtcaagggct gctggttggt 3120

gctggtacaa ggagcccact cagcagtttt cttacctttg cctgccctgc ctttcatgga 3180

ataagaaagg caacgttttg cagcttccaa atttctgaag aaactaatct cagattggca 3240

gttaaagtca aaatgttgcc aaatatttat tccttttgcc taagtttggc tacccggttc 3300

aattgctttt tatttttaat gtcttgactc ttcagagttc gtacctcaaa agaacaatga 3360

gaacatttgc tttgctttct gctgaatccc taatctcaac aatctatacc tggactgtcc 3420

agttctcctc ctgtgctatc ttctcttcta tccaagtaga atgtacgcca ggagctcctt 3480

ccctctagca atttctacta aaatgtccaa gtagaatgtt tccttttaca atcaaattac 3540

tgtatttatt aatttgctag aatccagtaa atcattttgg tagctctggc tgtgctatca 3600

ataaaaagat gaaagcaaca agttgatttt ttttaggtct actattgatt gaacattctc 3660

caatgggcaa ggctcattgt caaagcaggg tgaacatgat cagattttct tctgcatcat 3720

cacacaagct gcgagtgttg ctgcaaagca cttctcagta ctccttccct aacctcagta 3780

tgttaatagc cattcatact tggcttaagc atcaccattc tggccacagt ctgagagatt 3840

agtgatcaaa gctcccttcc ctatcactac caccacctac tgataacaaa gatttctgaa 3900

tacttagtag tagtaaaata tttttcaagc acagagttac agctaatcct ggatcacttg 3960

ttctaaattc ataagggagt atatcaagag caaatgaagt aaatgattct ttcctgcgtg 4020

tgggagtcac catcatttta ttcaaaggga gttcctgtga taggttcatt ccttaagtgg 4080

tgctacagaa ggaccctagg aaaatgctga tcttgtcctg agaggcaact gatttgagaa 4140

aatgattata ctacagcagg aaggcagcac cagtgaacat atggtaaatg gcaaaaacaa 4200

gacagtttta gaaactgcta attcagtagc agccttggcc tttcccttaa ctcaagcagg 4260

ctggaatctg tctttatagt cggcaacttt ccaagataaa gcagtgataa tagcagttgg 4320

caacactaaa tatcaatatc atcacccttt aatgacatgt tacccctttc agatctagaa 4380

aatcagggaa aaagtgatgt cccaactcta ctctaaaaac ctgctcctac tattttagtc 4440

agtgtttctt taaggaagaa gaggactatg tccccaaaag ctaactctgt tttatagccc 4500

tggtggatcc ctgaaaaccc tatttattct atactgggct ttggaatatg aaaaatggac 4560

ctgtgtggcc gggcgtggtg gatcacacct gtaatcccag cactttggga ggtcaaggtg 4620

ggcggatcac ttgaggtcag gagttcgaga ccagcctggc caacatggtg aaactccatc 4680

tctactaaaa atacaaaaat tagccaggca tggtggcgca cacctgtaat cccagctaca 4740

tgggaggctg aggcaggaga attgcttgaa cccgggaggc agaggttgca gtgagctgag 4800

attgcaccac tgcactgcag cctgggcaac agagcaagtc tctgtcttgc agcaggggga 4860

aggacctgtg gtactcctac aaacttaaca actttagctt ttcggggaac agtggtggtc 4920

tccacagata gctagaatct tctaagatga aaataaactg tttcatccag ctgctttctg 4980

cctgttaacc tttgccactt ccattccttc aagtgtgtaa tccccagggt tgtcttgtat 5040

tacacacatc cttgttaagt aaatatacca ctgttgattt cttcttccag tgcaacattt 5100

ggattaaata aaaatcttta gtttccaaaa aaaaaaaaaa aaaa 5144

<210> 138
<211> 1844
<212> DNA
<213> Homo sapiens

<400> 138

ctctcgagtc tccgccagat ccggggcggg tccgcggccg cggcgggaac atggaggagc 60

tgctgaggcg cgagctgggc tgcagctctg tcagggccac gggccactcg gggggcgggt 120

gcatcagcca gggccggagc tacgacacgg atcaaggacg agtgttcgtg aaagtgaacc 180

ccaaggcgga ggccagaaga atgtttgaag gtgagatggc aagtttaact gccatcctga 240

aaacaaacac ggtgaaagtg cccaagccca tcaaggttct ggatgcccca ggcggcggga 300

gcgtgctggt gatggagcac atggacatga ggcatctgag cagtcatgct gcaaagcttg 360

gagcccagct ggccgattta caccttgata acaagaagct tggagagatg cgcctgaagg 420

aggcgggcac agtggggaga ggaggtgggc aggaggaacg gccctttgtg gcccggtttg 480

gatttgacgt ggtgacgtgc tgtggatacc tcccccaggt gaatgactgg caggaggact 540

gggtcgtgtt ctatgcccgg cagcgcattc agccccagat ggacatggtg gagaaggagt 600

ctggggacag ggaggccctc cagctttggt ctgctctgca gttaaagatc cctgacctgt 660

tccgtgacct ggagatcatc ccagccttac tccacgggga cctctggggt ggaaacgtag 720

cagaggattc ctctgggccg gtgatttttg acccagcttc tttctacggc cactcggaat 780

atgagctggc aatagctggc atgtttgggg gctttagcag ctccttttac tccgcctacc 840

acggcaaaat ccccaaggcc ccaggattcg agaagcgcct tcagttgtat cagctctttc 900

actacttgaa ccactggaat cattttggat cggggtacag aggatcctcc ctgaacatca 960

tgaggaatct ggtcaagtga gcgggcctta ctctggaagg aggcctcaga ggtttctcca 1020

cagtcctctt ctgggcaaat tcttgtttct tcacatgccg gactagctta agaccaatgc 1080

agtagcttat ttccaagcct tgcaaagtat ataatatcta agaggaaagg ttttgtcatc 1140

ccagcgttgt ccactttgtg gggctttgta ggtagacgga gccacactac aggcagggta 1200

tgagcagagg gatgtatgga gtgtgggtga ctctgagcct cactgctgct gcaaggtggg 1260

gaaactgtaa gtgaacccct gtgggtgcgg gggagggtat ccggtgcgca gggaggtggc 1320

cagcgccccc gggcactgct gctcataggt acctttccac tgcctcctcc ctgctctcct 1380

gtgcaggaat gtctctgagc tgttcacgtt gatgcttctt ggttggcaag acttgggtgt 1440

agacatgaaa ccatcttact aaaagcgtct taaaatgacc aattccagaa tcaagcgtat 1500

tccgttttct tcctgcatga tccctgggcc ctcccgcagg ctgagcaagt ctgtaaactg 1560

attctgggag aaaccaagct gctggccata gggtgtcctt gggtacatcc aggagtcttc 1620

attgcttctg ttattacccc gtctcctctg ccattttcta cagcttgctg agttgtcatt 1680

cctttgcaac attaaaatac atgctgaact catatttttc cttccttcac tgttgtagta 1740

aagagacata tttcatgaat ggcattgatg ctaataaatc ctttgcacaa aaatttgaat 1800

aaacttccag tggtttcgat aatgtaaaaa aaaaaaaaaa aaaa 1844

<210> 139
<211> 9479
<212> DNA
<213> Homo sapiens

<400> 139
tgaactgagg cgaactccgc cgccaagtga gtgaggagga ggaggaggag aaggaggagg 60

aggaagagga ggagcgcagt cggagcgcgg cggcagcggc agaggcgccg cggcggggac 120

cagcccagag agaccccccg agcccgcggc acaggcggcg gcggcgctga gcgggctcga 180

ccagccgagc gagcggcggc cccggctcct cctcgtccgg cgccagcagc ccgcgccccg 240

cgcccgcact cggccagccg agacccgcct cccgcccgcc cgccggcctc gtgagggacg 300

cgccgagccg ggcggccgag agcggcggcg gctgggagcg cgtcgggccc gggcggaaag 360

tgcctgcggg gggcgggcga gcgcgcggtg ccggccgccc cggggctcgg cgcgggagcc 420

agagcgggag ccggcgcgga gcgggacgcc gggtcccgag cggcccgcgg ccggggctcg 480

cccccgcccc tccctcctct ccggcggcgg cggcggcggc ggcgggcgga gtgagctgcg 540

gagcctggaa tatggtcggg gaaatggaaa cgaaggagaa gccgaagccc accccagatt 600

acctgatgca gctgatgaac gacaagaagc tcatgagcag cctgcccaac ttctgcggga 660

tcttcaacca cctcgagcgg ctgctggacg aagaaattag cagagtacgg aaagacatgt 720

acaatgacac attaaatggc agtacagaga aaaggagtgc agaattgcct gatgctgtgg 780

gacctattgt tcagttacaa gagaaacttt atgtgcctgt aaaagaatac ccagatttta 840

attttgttgg gagaatcctt ggacctagag gacttacagc caaacaactt gaagcagaaa 900

ccggatgtaa aatcatggtc cgaggcaaag gctcaatgag ggataaaaaa aaggaggagc 960

aaaatagagg caagcccaat tgggagcatc taaatgaaga tttacatgta ctaatcactg 1020

tggaagatgc tcagaacaga gcagaaatca aattgaagag agcagttgaa gaagtgaaga 1080

aattattggt acctgcagca gaaggagaag acagcctgaa gaagatgcag ctgatggagc 1140

ttgcgattct gaatggcacc tacagagatg ccaacattaa atcaccagcc cttgcctttt 1200

ctcttgcagc aacagcccag gctgctccaa ggatcattac tgggcctgcg ccggttctcc 1260

caccagctgc cctgcgtact cctacgccag ctggccctac cataatgcct ttgatcagac 1320

aaatacagac cgctgtcatg ccaaacggaa ctcctcaccc aactgctgca atagttcctc 1380

cagggcccga agctggttta atctatacac cctatgagta cccctacaca ttggcaccag 1440

ctacatcaat ccttgagtat cctattgaac ctagtggtgt attaggtgcg gtggctacta 1500

aagttcgaag gcacgatatg cgtgtccatc cttaccaaag gattgtgacc gcagaccgag 1560

ccgccaccgg caactaacct atgaccttct gacctctgaa ctcttcaccc aatgatgacc 1620

tgaccatgcc tgcctgctga tcagttaact ggtaatcgcc tttgcttgcc tgtcgtcagt 1680

gcagcgagct gaggcacttg tccgttcgtc ttaccatcta accaaacaaa agacaaagaa 1740

attgttgtcc tccaactcag cttttttttt ttttttttcc tgtttgggtg aaagtggttc 1800

tagaaactgc actgaatagt agtaaagcaa taaggcccaa ttcatcccac agcactgatc 1860

atcttttaat atcccaccct aagcgaacgg taagaaggcc tctcttaaga aggggagaca 1920

gatggtcctt aactactcaa tgacagaggc agttactgtg agagacttct aggaatcttt 1980

ttcttctcat agcgaagtca aagctctctc tgaatgtact gtgtgatgat gcatcatgca 2040

tgaaccttcg gtcagggata tcattggtga agtgatttca aaaagtattc aaaatttgat 2100

atgctgttta gtcactacag tgccctcaaa gggcagaagt tgcagccttt tttatattgc 2160

ctgccaaaat ttgaagtatt agaagaaagt gtgccatgag agaaaaactt aaggagtttt 2220

gaaaagtaat gcaaataaca aaactgcaac actattttta aaaagataaa tatctgagtt 2280

aaaattactg aatctttatt ttacacctaa aaaaatatga gaacaaggta catgcattat 2340

gtgtcacatt actgggcaaa ctgttcaagt attttttttt aaacctccct gtatagaaaa 2400

aaatcattaa ggatgtaaaa gccatgcttg cctatttgct gtatacatgt aatgaaattg 2460

tagataaagt gtagtgcatt gaaacaaatg aacaaaaagt agatactttt actatacaag 2520

ggtgctggtg cagaaaaaaa tatatatatt tttggaaatg tagcatttta tactttcaag 2580

tgttataaaa aaaaagaaaa agaacaaaga aaccctttat ttcattaaat gattttttct 2640

ggtggttgtc aagaaacaaa agaccaaaag agcctttttg tctttctttt ttatttttta 2700

agtattggaa taagtctaaa gaaaagaaag tgccttattt tccttcatgg tcattcagtc 2760

aagtgtctca taaagatcag ctcctccctc agaatacaag ttaatgcatg ttactcagtc 2820

gcccagtatg tgaaagaaga tatgagggag acaaatgagt tgatagtttg aattatagga 2880

tttttgccac gttaccttga tgcaaatttg ccaaatctga tactgtgaaa agatttgata 2940

acttttctaa tgcctgacta gtaagtttta aaacatcatt ctttaaaaga atagtttaat 3000

acttttggtt attccaatct tcaatgttaa cccaaaggat tctgagataa aaattatgtg 3060

tcatctaaac ttcagtttac agacaaaatt agagctttgt tttatttgga gaagggggat 3120

gcattcattt gattttgttt tttgttttta attaattgaa tataatagga tacctagcaa 3180

atgttttcac aaaagtgaat ctttgtgatt ttcattctag aaaacaatgt tttaaatcac 3240

ccaaatttaa tcacatgtct ttatatcata ccagataact ggtgtagtgt atccttttac 3300

tgtttcaata acctctgcct cccccacccc ttaactttaa aagaatgtga aaaactagca 3360

tcatagtgca tataaatact caaccacatt tcagcttaag cttctaattt ctcaagaagt 3420

tgaaacagtt agttatgtag ttgaagcaat ttgtgtgcaa agcctacatc taaacacttg 3480

agtgaataat cattaactgc tcagtaccag acgtggcaaa tctcaagtga cagtggaccc 3540

ccccccccgc ccagcttatc aactcgtccc ctcttccaca ccttcccagc atcacagtca 3600

gaggtcagca ggaagctgag tcattacctt cttggtgacc tcccccacca tcttgtctca 3660

cttggaaacc gttgtcagtt tatttactat gcaatagaca ttcattgttt tgtatccagc 3720

tagctttggt ttaatatgcc actgctttgt ctttctgtta cacatacagt tttgatttat 3780

ttacaatata tgctgtgcca ttttgatttt tatattgttt ggttggttga ataaatttgc 3840

gacactcaaa cacttgaggt gatagttgtt aaaaacaata ccagtatttg atccagtttc 3900

atctcaacta tgttatacct ggacatttta gatgtttatc aaaggtcttt tatggggaag 3960

aaagtaaatg tatgaaataa taaatgtgtg acatttatag ggaatgttgg ctctgaacaa 4020

acttttgaaa acttggttga caaaattttg gatcaactga aaaaagcatg acttttgaaa 4080

tctctgaatg ccttggttct cagtattatc attctttatt gaatttattt cttattaaaa 4140

tatgtagttt ttaagacttt ttttctgaca gtattatgta attttttagc gtgggtagat 4200

gggagtgtcg cttgtatgtt atcgtacagc tgacatgtat tttgtctatt tctttattat 4260

cttagtttca tgctatgtat gtaccataac caacctattg cctatgagaa acatgtaaga 4320

taatgtattt acagccattg ttacaagttt ataatgtatt tttctatctt gttttatatg 4380

tatgttatat aacattcaaa aagaattttt ttcttgattg agaaaaggat acaaaatgca 4440

aaatccacaa ttttgataac tgaaaattgc caattgtttt gcagtacttt attatattgg 4500

gtgtcttgtc ttttttgggc ttttagttag ctaatgaatg aatacattag acacttttgg 4560

gttttagttg ggattttaca tagcttgcat tttaattctt tggttctttg ctgtttctat 4620

taacccacag cattatttta ataaatgtta taataccaac ctactaactc tggactttgt 4680

ctgtttatta acctttatat gtttaattaa aataaacaaa taaagacaaa agaatgtaaa 4740

gtcttgagtt actggactaa ccctgaagaa cgtgaccaca gataacatag tgtgacttgt 4800

ttttatgttg tttgtcagct gacagttttg cacactactg ttaaaatggc tttggttatt 4860

ctggtctttt agcttggggg ccggggcggg tgtgggtgta ggtgcagtag agatgggaac 4920

aaagtagcct ttgtttgagg cattcttctc ttgccacttg cctttcagca tctgcattac 4980

taattccaca ccttattttt ccttctccaa aataatcact gtctgctcac agcatcgagt 5040

aaaaagatac attgtcatgg tatatacctg gagaaaccag tttgattctt tgttgaacca 5100

tttcttttaa tttctgtaaa taagtaattt tatggtcaca ttgtaaagca gagaaactgt 5160

tgatatttta cctttattta gatttttgtt ttaacttgac ctttttcctt tggaaagcgt 5220

tgaacttgtt tagctaacta ctcctattag gggagcttaa cttgggtaaa gtcaaaggcc 5280

cactggctgt ttctcatggt ggcctgaagc cccgctgctt tccagtgagg aaaagaaccc 5340

tcagcacgtt actgagcttt cctcaggtat ttttttctct gtcacctact ttacaaatac 5400

agtcagacta aaacattaaa caagaagttt aagggggaat aacattggga tttgaaaagt 5460

tgtatgtgat taatttcttg catgcaaatc aatttaagat ttcttaaata tcttcatgct 5520

tttcttggct ggaacttggg taaattaacc tgtaattatg aattatttag tttaactcaa 5580

ggtgcatttt tatttcacag attgatactt gcttttttct gtgaaacagt tctcctagat 5640

accaaaatcc tcaggatgaa tttttgcatt tgtaaataac aatttatctt tgtaaattac 5700

attttattta tttagagttc taaaattgag ggctgaaatg aatgacatgt aatagaagac 5760

aaaactgtac caatacgctg ttaaccaatc agactgtcca ggtattctca tttcataaat 5820

atgtatacac acatataaat acacagagta gtgttagctg atgtgagtga taacagcgaa 5880

tcaccattga gatggtcatt tggatcatgt aaacaaaccg tgtcatgtct ctgatgggag 5940

cggtgggtag tgtactgtta ggtggacagg tcttttaaca tgctactatt tgtaaaagtt 6000

tccgttgcca taacagttca tgtagtagtc tgtgaaaatt cgaagctgtc tgtcaagctg 6060

ctaatataca agttgtcatg gtaccatgta acttaaattg atgaatttct atgcaattta 6120

ctattctctg aagcctatga atgcagcctc atctcatttt cagttaaggg tactttatgt 6180

ataattatgt cattgctgcc ttttatgtag gaaatgtaaa aaagttttaa aaggcgaaaa 6240

taacattaca gatttcattt ttatacaggt tacaatatta aacatgaaac taccaaattc 6300

caagaatagt aaaattctag agtcagttga ttgattgaaa ctatttgaca aaacagttga 6360

aagctgagct ttcagtcaag aagttggtga aattaatgtc cttaaacttt tcctttaggc 6420

aaaatgtcag ggaaaactta acatgaaagt agttactccc atacgcacca gtgcagtagc 6480

tccaggtgta agaggtcacg aaggcaccct gcccataaaa ccaggatgtg catacgtaca 6540

cacaatcggt gtctggttat ggttttctaa acactacata aggatctgaa ttttttagtg 6600

tgccaaaagc aaacgatagg tttaaatgaa attgctcact accaaatcaa gactcttcat 6660

atatatacgt gtgtgtatat atatatgtca taacactttc acgtgtgaca gcctaaggtt 6720

tctttagttt taggatgggg tggggtggga gtggttttgc atgtttgacc aggatgtgat 6780

gcctgtcttt attccttgat aagaagtagc tgttacagca tttcaagcaa attattttag 6840

gtagaataga acttactgcc aaatgattat ttatttagca aaaattattc tccaactttt 6900

cgaattcaca atttttctaa gtgcatagag tagtttttta cattgtcacg aattccttaa 6960

tacctttatt taaaatggaa aaaatatgga caatatttta gaaatatgtg ctacacatct 7020

aattttatct gtagtttaag tgatctaaat tgagatgcct ttgatatgaa gagatttacc 7080

aacattatat gcactcgtgc cttcaatgtg gaatcaaact ggttaacctc agcacagtcc 7140

tctcgtttct gtgtttctgc tgcactaatt gccagggggt gggggagaag gcgaatgaat 7200

taattcatag tagaaggagg cgataggtgc agcaaagggc agccacagtg ttgattccac 7260

attataatgt tgttgcctct tcttggcaaa agacaccaca ttgtgggaag atctcagctt 7320

ccagggtaaa agttaattta taacttaaaa gtgctattaa gtttttatta ccaaatatat 7380

cttttatggt ttatattgta gtggtatgta tgaaacattt aaaattttac tgtggaaatt 7440

gtgtatatat atatatatag tcgaaatagg tgttcacagg tcacatgtga acggagaact 7500

gcatgaccgt acatgaaatg caataaacca actggaaaaa gtgcatgtgc ttcatcctct 7560

caagccaact gcagctggaa agtgctgctt atcctccacc cccagaaaat gcatgtatca 7620

atatgagaat aaagaacgca cactttcaat tttattgagg ctttcaacac tatttaaaag 7680

aaaatgtaag aatttgacat tctggagtta ttataacatt agaaaatgag cataacattc 7740

actctgattt tagccattaa gggagattag taaacagact gctacagtgt tccatagttg 7800

gactgtgcat ccaaaacatt tttttatctt taataaatgg tacagttttt atgtagtttt 7860

cgaatgtaag aagaaaggaa tgctgaccaa aacttgattt catcagcttc atgaaaagga 7920

ctagtgtcat taacctgttg aacagaattg gtttattaaa aaaatcattt ccagtagtgt 7980

gaaaccttta cgagtcttta acatctaaat gttatgactc cttgtacctt aagttttcca 8040

gtctttctta tttatatcat ctccaagtac ctctggctcc tttcctcttg ctcaccggaa 8100

ccttagtttt cctcaacaga atgctttgtt aaagtagccc acagttgcag gatccatagc 8160

accgtcgtgc agactagcag cccaaaggtg tgtttggttt ggcttatacg gtgttttgct 8220

ttttaaacta cttgccataa tttaaaagtg gcaacactag acttaaaaaa aaaaaagtct 8280

gattgcccat attagatttt ttttttaatt cttcacaaaa tctgctcttc ctgaaagatc 8340

aaagtgtcta gaaagcccaa acatgtattc ttaacatagt aggcaccagc tgaaactgag 8400

taattaaacg gtcccctgaa gccaagtatt ccctggttag tcgccacccc accactcctt 8460

cctgtctcta gtgtcacact tgggctgttg attttcttac tcttctcttg ctttaatcct 8520

tccccccggc tctctggctc ctgtggatat ctgtgcttgt ttcctggtcc aggatggtga 8580

tctgactttc aaaccagctt ctcaaaaggg gtgacataaa atcagttttg atgtttttcc 8640

tcctgaaaaa tcagatgaat attttagtca ctgttactca tgcacattgt gttcttatgt 8700

ttacagaagt gcttaagtga atggaagcac tagacatttg gatttccttc ctaacgtaat 8760

ttttaatgat tacccctttt atacagtaat ttgtggtctt tagaaagcag ttaaactaat 8820

tgaccatcta atagttgtac tatacatatg tctaaaataa tagtcatggt aagtttggca 8880

tcatatcttc cccaaaaaat gtttattaaa attagattat tccagtttaa tgctattttg 8940

tgaactgtat accctctgaa agcgcttatt tttacatgct acacaacagt tccaatttta 9000

aggagtgtct cctaaaattg ggatgaaaat ctactgtagt ctgttttaaa gtatgctata 9060

ctatgttcat tggttactta actggatatt aatataaagg ttattacaag aaaaatgatg 9120

aagagcaaaa ggagaagaaa atatttgcaa gtgaatccac aattcttgca gaactatttg 9180

agttgatact aaagatttta tgttcactcc tttaccttag aactgtcaag cttgggaatg 9240

aggaaagtgc cttggctgtg cttgaaacct gaattttagt gcttccctta ttacattcat 9300

tgttttcaat gattgattta taaaattaag acatactggt agtacaagtt gaaagttggt 9360

ttgaatacat tttaattaaa tgataatatg ttaatatgct tttgttcatt gctttctcac 9420

tgaggtaaaa cagcattaaa aagttgtcca gaatttaaac tgaaaaaaaa aaaaaaaaa 9479

<210> 140
<211> 5083
<212> DNA
<213> Homo sapiens

<400> 140
agaagagcga tgggaggggg tgacgtggca gtgacaggcg cataaactga aatgaatggc 60

ggaaacagca gccaatcggc gatcagattg gccgggcatt ccgcattctc ccgtcctccc 120

tgagcgctgg aaacttcagg aaagcaccaa tgagcttccc caaaagtctg attggccttt 180

atctgaacca atcaaaggtg ggtctggcgg acgcgtccca ccccaggctc tcactgggcg 240

ctgtagaatg acaggcccga gagaaatgcc aatatccgcg gggtcttcct caagtgacac 300

tcagcggcac caatcgacgt ccttcttccc ggcggccgag ccctcctccc cgcggctggc 360

cgggcggggc ggggacgctg cgcggcggtg gctgatgcgg tagccgtgtg gggcgctccg 420

ggcggcgacg gcggctctcg taggcggttc cggtcttgta tctccaggcg gcggcggctc 480

atggcggcga cggagctgag aggagtggtg gggccaggcc cggcagccat tgcagcactt 540

ggcggcggcg gcgccggtcc cccagtggtg ggaggaggcg gcggccgcgg agatgcgggg 600

ccaggctccg gggccgcgtc agggacggtg gtcgcggcgg cggcgggagg cccgggcccg 660

ggggccgggg gagtggcggc ggctggcccg gcccctgcgc cgccgactgg gggctcgggc 720

ggctcgggcg ctgggggttc gggctcggct cgagagggct ggctcttcaa atggaccaat 780

tatatcaaag gctaccagcg gcgatggttc gtgctgagca acgggctcct gagctactac 840

agatcaaagg cagaaatgag acatacctgc cgtggtacca tcaacctcgc cacagccaac 900

atcaccgtgg aggactcctg caacttcatc atttccaatg gggtgctcag gacctaccat 960

ctgaaagcta gttcagaagt tgagcggcag cgctgggtga cggccctgga actggccaag 1020

gccaaagctg tgaagatgct ggcagagtca gatgaatcag gagatgaaga gtctgtctca 1080

caaactgaca agactgagct gcagaatacc cttcggaccc tctctagcaa agtagaggac 1140

ttgagcacgt gcaatgactt gatagctaag catggcacag ctctgcagcg ttctctcagt 1200

gagctggagt ccctgaagtt gcctgctgag agcaatgaaa agatcaaaca ggtcaacgaa 1260

cgagccacac tctttaggat aacatccaat gccatgatca acgcctgcag agatttcctc 1320

atgttagccc agacccatag taaaaaatgg caaaagtcac tacagtatga aagagaccag 1380

cgtatccgac tggaagaaac cctcgagcag ctggcgaagc agcataatca cctggagagg 1440

gccttccgag gagccacggt gctgccggca aacactcctg gcaatgtggg ttctggtaaa 1500

gatcagtgct gctctggcaa aggggacatg agcgatgaag atgatgagaa tgaatttttt 1560

gatgcacctg agatcatcac catgcctgaa aatttgggcc acaaacgtac tggcagcaat 1620

atcagtggag ccagcagtga catcagcctt gatgaacagt acaagcatca gctggaggag 1680

accaaaaagg aaaagagaac cagaatacca tacaagccaa actatagcct caatttatgg 1740

agcatcatga agaactgcat tggaaaagaa ctctctaaga tccccatgcc ggtaaacttt 1800

aatgagccct tgtccatgct tcagcgcctt actgaagatc tggaatacca tgagctgtta 1860

gaccgagctg caaaatgtga gaattctcta gaacagctct gttatgttgc agctttcacc 1920

gtgtcctcct actccactac tgtcttccgc accagtaagc cattcaaccc actgcttggg 1980

gagacctttg agctggaccg attagaggag aatgggtacc gatccctctg tgaacaggtg 2040

agtcatcatc cccctgctgc tgcgcaccat gctgagtcca aaaatggctg gacattgcgt 2100

caggaaatca aaatcaccag caagtttcga ggcaaatacc tctccattat gcccctcggt 2160

accattcatt gtattttcca tgcaactggg caccactaca cttggaagaa agttaccaca 2220

actgtacaca acattattgt gggcaagttg tggatagatc agtctggcga aattgatatt 2280

gtgaatcaca agacaggaga caagtgtaat cttaaatttg ttccttatag ctacttctct 2340

cgggatgtag caagaaaggt gacgggggaa gtgacagatc catcaggaaa agtccacttt 2400

gctcttctgg ggacgtggga tgagaaaatg gaatgtttca aagtacagcc agtcattggg 2460

gaaaatgggg gtgatgctcg acagagaggc catgaagcag aggaaagcag ggtcatgctg 2520

tggaaaagga atcctttacc gaagaatgca gaaaacatgt actacttctc agagcttgct 2580

ctgactctca atgcttggga aagtggcact gcccccacag acagccggtt acgacctgac 2640

cagagactga tggaaaatgg acgctgggat gaagcaaatg cggagaagca gcgcctggag 2700

gaaaaacaaa gactttccag aaagaagaga gaagcggaag ctatgaaagc cacagaggat 2760

ggcacaccat atgatcccta taaggcactg tggtttgagc ggaagaagga ccctgttacc 2820

aaggagttaa cccatattta taggggagaa tactgggagt gtaaagaaaa acaggactgg 2880

agctcatgcc cggacatttt ctgaaacggc agtaacaaaa aagaggagca tataatggag 2940

aagaggacag aggatgtgtg ggaaagctgg aagttgtgac tctcttacca agtgctttcc 3000

tcaagtttgt ctctcttgac caatcatttt ttccaaatca atcaccagaa ggagacacca 3060

gtggtggtga ttggctggtt gccttagctg attgaaactg aaatcgacat aggagatact 3120

atgtctgtaa gggagaggtt tagtggccga aggttagtgt tattccacat ccacgaagtc 3180

aagtattctt ggctcttctt tctccctctg cccccattta agacaatgtc actcaccctc 3240

cccttgtagt atcctgctca gccaagatct gcctaagatt atgattcagg ggcattttgg 3300

tgctgttaaa agcaagagcc gaatttagaa gttcccttaa aaagaaacac ataagactta 3360

ccaatggagg cattgtgaat ggttgcagtg gagcttaggt ataacatcat caagtgtgtt 3420

cacacgcggg gtcggtttaa tggagtgtcc acgcggagat aactgcgata ttggaacacc 3480

tgtgagagag attgttctat agggctggaa tattcagagt tacattcttg gaagtttctg 3540

tttttacttg catcaaacac ccctctgttg ttctccatca tctttaatag caactggaga 3600

ccactttggt cattggtaag ggggtgcatt ctcctcacaa aggggtttta tggacttcct 3660

caggcggaga gcttctgaga acacaggcag gatggaaaaa gactactagc cacttttgct 3720

ttcccaaccc cccttaatgc catccttcat tgtctttctg gcttctcttc ttctggcaca 3780

gtaccatttt gggtctgtgc cccagtgtgg agcaaaacat tgcctgtccc attctgatat 3840

acttcagaat ttgagagcag aagttaatgt ggaacaaaag ttttcaccat ctctcaagcc 3900

ccaaggactg gagccacctc tggaataatg tgttaaatat ctgtatatta tatatatgta 3960

gagaaaaatc taattttttg gtttgatttc cttcccatta agaatcttgt tttttgatac 4020

ccttgtctcc ctgagactct ttcttggaca cgtgttgatg tggatggcac tcctgctctc 4080

catcttttat gggcagagag atttcaaaac ctgagcagga tcatgtctgg cagttactaa 4140

acagggactt tgtccatggt ttgtatcatg gggtgcctac tgctgtctaa actctgctct 4200

cctaattagc tgactcttga aaatgaaatc ctaacctctt tcctgcctct tttctttctc 4260

ttttcaggaa gagtgtgggt agaaagtccg gagggcaact tccaatttaa ttctgctctg 4320

tccggcgcat aggctgaatt ccaaaagaac tttcttccta ggatgtgggg cttaaagcac 4380

cgtcatgtgg ggtggatcag ataattgctc ctgtggggcc ctggcttcct tctctgtttg 4440

tgcagtattt gtttttgttg tcttgttgag aaatatcccc tgtgcaaccc acccttggtt 4500

gaggatcaca aattgaggtg ccttccttgt atgctttttg ttagtatttt ttgtttttgt 4560

tccttaactg ctgagcctca gccagcgtgg gtcctggggg aaataccatt ccaaaggaag 4620

ccatccctat ggggaaattg gttttactgg gtaaattaat acttttattt cttcccctat 4680

cccttccttc ctagttgtga taaacatggg agaggagtga ctttgactgc tggggaattt 4740

cctcctaaga accttattcc caaaataagc cagggattat gtcaaggctt ctttcaagga 4800

ggctcataaa agcagtgacc tcatccgggg ctttggatag agtgagaatt ccccaactaa 4860

attggacttc tctgctttat ccccttctct tccttcacca tctgcactac atttctggct 4920

gatcccaatc agattcccgc taatggaaga agtttagaat ctttcaggtg gaataaagtc 4980

acatgaaaac aaaacacaac tatatatatt ttcagttttt ttgccttatt gatttttttc 5040

caaaaaaaaa aaactactaa attaaataat tttttaaaag tca 5083

<210> 141
<211> 7005
<212> DNA
<213> Homo sapiens

<400> 141
   ggcctggcgc tccggccgct ttgtcgaaag ccggcccgac tggagcagga cgaaggggga 60
   gggtctcgag gccgagtcct gttcttctga gggacggacc ccagctgggg tggaaaagca 120
   gtaccagaga gcctccgagg cgcgcggtgc caaccatgga gcgggccggc cccagcttcg 180
   ggcagcagcg acagcagcag cagccccagc agcagaagca gcagcagagg gatcaggact 240
   cggtcgaagc atggctggac gatcactggg actttacctt ctcatacttt gttagaaaag 300
   ccaccagaga aatggtcaat gcatggtttg ctgagagagt tcacaccatc cctgtgtgca 360
   aggaaggtat cagaggccac accgaatctt gctcttgtcc cttgcagcag agtcctcgtg 420
   cagataacag tgcccctgga acaccaacca ggaaaatctc tgcctctgaa tttgaccggc 480
   ctcttagacc cattgttgtc aaggattctg agggaactgt gagcttcctc tctgactcag 540
   aaaagaagga acagatgcct ctaacccctc caaggtttga tcatgatgaa ggggaccagt 600
   gctcaagact cttggaatta gtgaaggata tttctagtca tttggatgtc acagccttat 660
   gtcacaaaat tttcttgcat atccatggac tgatatctgc tgaccgctat tccctgttcc 720
   ttgtctgtga agacagctcc aatgacaagt ttcttatcag ccgcctcttt gatgttgctg 780
   aaggttcaac actggaagaa gtttcaaata actgtatccg cttagaatgg aacaaaggca 840
   ttgtgggaca tgtggcagcg cttggtgagc ccttgaacat caaagatgca tatgaggatc 900
   ctcggttcaa tgcagaagtt gaccaaatta caggctacaa gacacaaagc attctttgta 960
   tgccaattaa gaatcatagg gaagaggttg ttggtgtagc ccaggccatc aacaagaaat 1020
   caggaaacgg tgggacattt actgaaaaag atgaaaagga ctttgctgct tatttggcat 1080
   tttgtggtat tgttcttcat aatgctcagc tctatgagac ttcactgctg gagaacaaga 1140
   gaaatcaggt gctgcttgac cttgctagtt taatttttga agaacaacaa tcattagaag 1200
   taattttgaa gaaaatagct gccactatta tctctttcat gcaagtgcag aaatgcacca 1260
   ttttcatagt ggatgaagat tgctccgatt ctttttctag tgtgtttcac atggagtgtg 1320
   aggaattaga aaaatcatct gatacattaa caagggaaca tgatgcaaac aaaatcaatt 1380
   acatgtatgc tcagtatgtc aaaaatacta tggaaccact taatatccca gatgtcagta 1440
   aggataaaag atttccctgg acaactgaaa atacaggaaa tgtaaaccag cagtgcatta 1500
   gaagtttgct ttgtacacct ataaaaaatg gaaagaagaa taaagttata ggggtttgcc 1560
   aacttgttaa taagatggag gagaatactg gcaaggttaa gcctttcaac cgaaatgacg 1620
   aacagtttct ggaagctttt gtcatctttt gtggcttggg gatccagaac acgcagatgt 1680
   atgaagcagt ggagagagcc atggccaagc aaatggtcac attggaggtt ctgtcgtatc 1740
   atgcttcagc agcagaggaa gaaacaagag agctacagtc gttagcggct gctgtggtgc 1800
   catctgccca gacccttaaa attactgact ttagcttcag tgactttgag ctgtctgatc 1860
   tggaaacagc actgtgtaca attcggatgt ttactgacct caaccttgtg cagaacttcc 1920
   agatgaaaca tgaggttctt tgcagatgga ttttaagtgt taagaagaat tatcggaaga 1980
   atgttgccta tcataattgg agacatgcct ttaatacagc tcagtgcatg tttgctgctc 2040
   taaaagcagg caaaattcag aacaagctga ctgacctgga gatacttgca ttgctgattg 2100
   ctgcactaag ccacgatttg gatcaccgtg gtgtgaataa ctcttacata cagcgaagtg 2160
   aacatccact tgcccagctt tactgccatt caatcatgga acaccatcat tttgaccagt 2220
   gcctgatgat tcttaatagt ccaggcaatc agattctcag tggcctctcc attgaagaat 2280
   ataagaccac gttgaaaata atcaagcaag ctattttagc tacagaccta gcactgtaca 2340
   ttaagaggcg aggagaattt tttgaactta taagaaaaaa tcaattcaat ttggaagatc 2400
   ctcatcaaaa ggagttgttt ttggcaatgc tgatgacagc ttgtgatctt tctgcaatta 2460
   caaaaccctg gcctattcaa caacggatag cagaacttgt agcaactgaa ttttttgatc 2520
   aaggagacag agagagaaaa gaactcaaca tagaacccac tgatctaatg aacagggaga 2580
   agaaaaacaa aatcccaagt atgcaagttg ggttcataga tgccatctgc ttgcaactgt 2640
   atgaggccct gacccacgtg tcagaggact gtttcccttt gctagatggc tgcagaaaga 2700
   acaggcagaa atggcaggcc cttgcagaac agcaggagaa gatgctgatt aatggggaaa 2760
   gcggccaggc caagcggaac tgagtggcct atttcatgca gagttgaagt ttacagagat 2820
   ggtgtgttct gcaatatgcc tagtttctta cacactgtct gtatagtgtc tgtatttggt 2880
   atatactttg ccactgctgt atttttattt ttgcacaact tttgagagta tagcatgaat 2940
   gtttttagag gactattaca tattttttgt atatttgttt tatgctactg aactgaaagg 3000
   atcaacaaca tccactgtta gcacattgat aaaagcattg tttgtgatat ttcgtgtact 3060
   gcaaagtgta tgcagtattc ttgcactgag gtttttttgc ttggggatta ttttaaataa 3120
   ttggtttttg tgttttctga attaccattt tttcaagaat gtttggaatc tttccttttt 3180
   caaaagtagg ttaggagcaa attatcatac attctgtgac atttaaagcc tttataggat 3240
   agtgaaaaat gctggctgag tggattttaa gagaaataat tgtatttgtt aacagtgtct 3300
   ttttttaaaa agttaaggca ctctgaaaca aatggaaagt cctatgaaac tgtattgtaa 3360
   agaaaacatt atttaattga tatgctgttt tgtgagagaa caggcaagac agaactttgt 3420
   cacttcagtg cagtacattt ttctgaaagc tacccataaa atcactttca tctcacctac 3480
   ctgatgcaaa gcaggtgaaa ccttaggaga tgatccagtc actgacttga ttgagggata 3540
   agtgtgattt agaaatggaa tggccttgga tgtctatcag tgaagaaaaa tgttctgtta 3600
   gaagatctct ctaagagttt ttttccttct gagcttcctt ttcaaaataa aagtgacaat 3660
   tgtagcattg acttgaagtg agacatggtt atagataaga gagtacaaaa tgactctttt 3720
   tcctgtcaat tgaaatttaa agaaaagttt taattatata aatagcaaag ggctattgcc 3780
   aatactaggg tcaaaaatga atttgaggga acagtgggta agaaacttta tgcctgaata 3840
   acatttagca gtattgtgat tgaaaaattg ccatattttg atgtatagga caagtcaact 3900
   gagatccaga gaatcctgga tgtgaatgct aaacactggc ccttaactca cattcaatgt 3960
   attttcttcc cataacattt agtatagtta atattttctt agaatttgag cccatttaag 4020
   tggattaata ttctacatgt gtgcccctaa agacacattt actcaatatt ggagaagtag 4080
   ataatgaatt aagcaactgg tctaggaaag gaaaatttgt ttcaaatatg caggaatgtt 4140
   tggatttggg gagagtagaa ggagagattt gcttgatttg ttaacttcta cctccaaccc 4200
   acaaaaaaga tatttgatct gagtttctat cactaatttg gatagaaaat ttctaaggga 4260
   catggtaatc cagcattctc aaggaccttt cgccaaaatg tgttttccat ctatgtcccg 4320
   attcccctaa attttgccta aaattcagta tgttccttaa gtttttaaaa ttctgagtgt 4380
   gtacaaatat cttgacataa tgcagtttta tttttatcat tctggtaaaa aaacaaaaaa 4440
   tagaagcaaa acacattgta ttgccattat tttgtatttg gtaaaggtta atctaggaag 4500
   ttaccaactg tttaatgcta tatgtattgt atacttgtat tttcaggata ttttattttt 4560
   tttgccatac agataaaatt tgtaaggttg cccctttgtg gcactggtgt gtaaaataca 4620
   cagactatca ctaaaataat agttatatat acatacaggt gtatacttat gcatgcatac 4680
   ataaatcctt agtatagaaa aattgcataa agaatagcaa tctttaataa acctttttat 4740
   tacattgtga tttagcagtt atgctaaaat atgtacttat gctttagtag tttgtttggt 4800
   cccctctagt atgtgtcact gagaaatttt ttaaagacat ggtagatcgt gtttagaggc 4860
   tttgtatgtg tgtcatttta ataagcaaga agatatattt agattagaaa tggtttggtc 4920
   tgcctttgaa tattgtttat tttactttac tagttgagac attaaaggaa gctgggcaat 4980
   gcctatttta tttctttgtt ggatatttta gttcatacaa agcagagtac ttctttaggg 5040
   ctggttaatt ggttcaaata atttttaatt tcctttctag tatcttctca agttggaaaa 5100
   atatacatac agtcctcctt caccttactc tgtatttata ttacccataa ctagcaagaa 5160
   gttcttgttc tagatttttt gtttgtttag ttataacaga gtaacatacc atttaattac 5220
   aatttttagc cagaaaagtc cccactattt tactaacttg ttaaaagata tctatataat 5280
   tgcctggcct tatatttttc agtagattag accgtgccaa tcacaatcct gggtggattt 5340
   gtgtaagtca cttaacctct gtgtgcctaa acaagttgtg cttttttaaa aggagttatg 5400
   tttgggcaaa gcctttgtct tcaagcagaa tgtcacagaa ggcagctact ttataagccc 5460
   caatgggcca tggagaccac tgtcagaaat gggatattag tctagagaga aggtgatcta 5520
   ttcccacatg tcatttctaa tgttgagttt ccatgactga acaaagagaa tatatttatt 5580
   cagcttcact tgcagatcac tagtgaatgt gagatttaga gctcattgag tatattgctt 5640
   caaggtacaa acccaggatg atgatgttgt caccactgtc tcttaatttt gaataatagt 5700
   ttcctttaat aggagtatta gagataagaa agtatatgaa aatatactgg aaatattgga 5760
   ttcttggaga aaactgttca gtcacagata tattcttgcc tagcagtgaa gtgcctttat 5820
   tttcagcata gcaaataaat attagacctg ttccaatttg atctacaatt tttttctgtg 5880
   tttttcacca gattgtactc ctaaaactta acaggccatc acaagcaatt gtcttttgtt 5940
   tacaagattg atttaatatg agaggataca aaatgtcatc gttatcctct cttatgaaca 6000
   actgtagtca aaataaggtg gcacaattta attgttttgt atcagaaata cactgaccca 6060
   ccttttattg agtcctgcca catgttaggt accgtgctct gctgtggaga cagagcagtg 6120
   accccaagga gctcacggtc cctgaaggag gtgctagaga agagacttag cttctgatac 6180
   tgccaattta atgtgagaac atggggtata ctgcatcatt tccattttca tcaataacat 6240
   atgttttatg caccttcttt acctgaaact tactaagaat ctaccagtaa acaaacatcc 6300
   tgtctttttg caagtatgaa tcacttaacc tgctgatagt tgaagaacac tttaggagtt 6360
   ttgtattctt gtatatagtt tattttttcc atgtgctagc caggtaaaga ttacacagtt 6420
   cttctggact gttaaattgt gcatggtttt ggaccccttc tgctctacta cagagagtga 6480
   agaagaaagt attaaagctc actttaccat tccatatact tactaaaagc ctgtgtaaac 6540
   atgtcttaat gaatgttgtt gaaagcaatg taaatagttg aaaatataaa tttatattac 6600
   agtttaagaa aaccttatga ggcatcacta gccactgtta atatctattt gtattcttat 6660
   accttttcaa tatatttgaa caaatatagt ttctggcact atttttatac taggaaaagg 6720
   agttactatg tatattatgc ttagctttta aggcatttta aataaccatg aatgttgatt 6780
   tcattacttt cctttcctcc atcacgagag tcatttcaga tgactctttc atgacaaaat 6840
   cactttaaag gaacacttac ctccgattcc tgtataaagt catgagatgg tcaaggtggt 6900
   tttccattgt gcaaattctt cacctgtcag tggtttcctc attttgccat gctttgtaaa 6960
   aataaaaaga atgatcaagt aggtatgaaa aaaaaaaaaa aaaaa 7005

<210> 142
<211> 1895
<212> DNA
<213> Homo sapiens

<400> 142
atgacaggaa gtgacccgtt aaggaagcag cacatcgctg cattcggctg gttttcaggg 60

tcttgttccc aatcagtttc cagccaacac cagggtgtcc tagtccgcag aggtgtgggg 120

gacacactcc ataatctcta cttttctttt tgtgcagctg agtcatggag ctttcagccc 180

cagcacatgg ctcctcctta actgcgtctg ctcaacctcc ctcagccctg tgaacagcat 240

ccccgcacac agacgcagag caggactctc tctgctgcca cttcaccttc ctgagagagg 300

accagcggcc agagcctcag tgactgccac cctggaggac agggcacaac aaccgtttct 360

ggagagaatg ggaggattcc agaggggcaa atatggaact atggctgaag gtagatcaga 420

agataacttg tctgcaacac caccggcatt gaggattatc ctagtgggca aaacaggctg 480

cgggaaaagt gccacaggga acagcatcct tggccagccc gtgtttgagt ccaagctgag 540

ggcccagtca gtgaccagga cgtgccaggt gaaaacagga acatggaacg ggaggaaagt 600

cctggtggtt gacacgccct ccatctttga gtcacaggcc gatacccaag agctgtacaa 660

gaacatcggg gactgctacc tgctctctgc cccgggcccc cacgtcctgc ttctggtgat 720

ccagctgggg cgtttcactg ctcaggacac agtggccatc aggaaggtga aagaggtctt 780

tgggacaggg gccatgagac atgtggtcat cctcttcacc cacaaagagg acttaggggg 840

ccaggccctg gatgactatg tagcaaacac ggacaactgc agcctgaaag acctggtgcg 900

ggagtgtgag agaaggtact gtgccttcaa caactggggc tctgtggagg agcagaggca 960

gcagcaggca gagctcctgg ctgtgattga gaggctgggg agggagcgag agggctcctt 1020

ccacagcaat gacctcttct tggatgccca gctgctccaa agaactggag ctggggcctg 1080

ccaggaagac tacaggcagt accaggccaa agtggaatgg caggtggaga agcacaagca 1140

agagctgagg gagaacgaga gtaactgggc atacaaggcg ctcctcagag tcaaacactt 1200

gatgcttttg cattatgaga tttttgtttt tctattgttg tgcagcatac tttttttcat 1260

tatttttctg ttcatctttc attacattta aatctctgga ccctggagca cttctaatgt 1320

atcaccccat ggagtcattg ttctaataat caccaattca gactcagatc ctcgtggtct 1380

atggagcatg ctgcttgctg tctgtgcagc tcccatttcc ccttcttcct gatagacttg 1440

gagctgtgtg cctccactcc aaggctgcct gcctgctgta aacactattc cactctgtct 1500

gccaacaact gcttcaggaa tgggcctgag atcccatgca ggtccctgag aagtgagtaa 1560

aagtccgcag aggtggggat ggaagatctc tccttagata gaacctgtct tcctccctgg 1620

cattgtgggg tctgggcgtg acactgggac tctcagcagc tttgtgctgc caacctgaga 1680

ttgaaggcag tgcctcagag cagcacagag agttggggcc ccctgagccc tgagccacca 1740

gccctgcagc ctgccctatc tccgcatttc cagttgtatt agccaataga tttcctactt 1800

atttaagcta tttgagctcc gggtctcttc tacctgcatt ctaaaacatt caaagtaata 1860

aaaatttctc cacattcaaa aaaaaaaaaa aaaaa 1895

<210> 143
<211> 4743
<212> DNA
<213> Homo sapiens

<400> 143
cgcttcatgt ggcccggccc gcgacggccg gcggctggga gcggcgaggc ggcggcggcg 60

gcgagtggcg gcccgcgagg cccgggaggc ggtggccgag gcccaggcgg tggcggcggc 120

ggcccaggag gcggcggacg gggagctgcg ggagcaggcc cggcctggct ctctagcggc 180

cgcctggctg cagcatgcgc gcccgccggg ggctgctgcg gctgccgcgc cgctcgctgc 240

tcgccgcgct cttcttcttt tctctctcgt cctcgctgct gtacttcgtc tatgtggcgc 300

ccggcatagt gaacacctac ctcttcatga tgcaagccca aggcattctg atccgggaca 360

acgtgagaac aatcggtgct caggtttatg agcaggtgct tcggagtgct tatgccaaga 420

ggaacagcag tgtaaatgac tcagattatc ctcttgactt gaaccacagt gaaaccttcc 480

tgcaaactac aacatttctt cctgaagact tcacctactt tgcaaaccat acctgccctg 540

aaagactccc ttccatgaag ggcccaatag acataaacat gagtgaaatt ggaatggatt 600

acattcatga actcttctcc aaagacccaa ccatcaagct cggaggtcac tggaagcctt 660

ctgattgcat gcctcggtgg aaggtggcga tccttatccc cttccggaac cgccacgagc 720

acctcccagt cctgttcaga cacctgcttc ccatgctcca gcgccagcgc ttgcagtttg 780

cattttatgt ggttgaacaa gttggtaccc aaccctttaa tcgagccatg cttttcaacg 840

ttggctttca agaggcaatg aaagacttgg attgggactg tttgattttt catgatgtag 900

atcacatacc ggaaagtgat cgcaactatt atggatgtgg acagatgccg aggcattttg 960

caaccaaatt ggataagtat atgtatctgc ttccttatac cgagttcttt ggcggagtga 1020

gtggcttaac agtggaacaa tttcggaaaa tcaatggctt tcctaatgct ttctggggtt 1080

ggggtggaga agatgacgac ctctggaaca gagtacagaa tgcaggctat tctgtgagcc 1140

ggccagaggg tgacacagga aagtacaagt ccattcctca tcaccatcga ggagaagtcc 1200

agtttcttgg aaggtatgct ctgctgagga agtcaaaaga acggcaaggg ctggatggcc 1260

tcaacaacct gaactacttt gcaaacatca catacgacgc cttgtataaa aacataactg 1320

tcaacctgac acccgagctg gctcaggtga acgagtactg agaggagaga atgtacgttt 1380

gctttaccca ccgccaccaa gaaagcagtc cgatgagatt tttttttgga gggggagggg 1440

tctacacagc aagagaacag aaatactgtg tctcatgaag gatcacagag ttcaggggga 1500

aaatgtgaca gcacacgcac aaacgccttc actggatcag ccgctggaac tgagggagtg 1560

agcttgggga cttccttcgt cagcactggc tttctgtttt cacaagacag acgtctgtcc 1620

cgctgctctc tccccatctc ctaccccaca tcctgtctta gccgcagtct ccagaaccca 1680

tgatgaactg tgatctgccg tggtcctgcc gtggtcctgc cgtggagcct gtccctacac 1740

atgaccttgg agcctcttgg ccttcagagc agaggcaaac ccaccacagg gcagctgcgt 1800

tttaggaaga gcaaatgaaa ctccacacca ttcttctaga tctctggtgt tctctttggt 1860

ttcatttttt taaaaaatta ccttctttgg gtggggattg agggtggagg ggagggtgtt 1920

tgggaaagat aaatagacat aaatatataa caatcacttc ttgaagaagt ataattgtaa 1980

ataagccatg taaaatgcct ttttaaaatt taattttcta gctggctcca attcaaattg 2040

aggatttatg tattaggcca cttacttggt tggcaagtgc aggaactcag ttaaaatgca 2100

gttgaagaat gtcatctccc gaattgctgt cactttggcg agggagtgga tatagggcat 2160

gtcacaaaag aacaaaataa cccgaccttt attgctggga gctggcttct gtccctttct 2220

tccccccccc acgagtcttg cccttgactt ctgctctgga ttcactcttc cctgtcggcc 2280

gcgcatgtgc tcatcccact ctccgctaag cgggaggctg ctgttagagc aggctgcttc 2340

ctgcctaaag caggcccttc ggggctcgct gcacacacat ctctggctct ccaggcttcg 2400

tgttctgtct tttcatcagc atggcggggc gggggcgggg gggcgggggt gtgtatggga 2460

atccctcccc ctcttacttt ttctcttgtg gaacttggcc acagtttctg aacaatgtgc 2520

ctacattacc agctggcttc agtgattcct ctgtgtccct ttttggtttc tggaaagatt 2580

ctttgtcaac attagtaact gatacataga accaaggagc actcaaatag ggagccagga 2640

gccagggagc tggtgacact tgtgtgctgt ggggcagctg ggatccaggt aagaccggat 2700

tgaagctttg aaattagact aacaaagctc cagacagcaa gagcccaggt gcactgctca 2760

cacccccacc tgcattttga agtcatatta ttttttgttt tgttttttaa gacggtctgg 2820

ctctgtcgcc taagctggag tgtggtggca cgatcacagc tcactgcagc ctccatctcc 2880

taggctcaag ccattttccc acctcagcct cccgagtagc tgggactaca ggtgcacacc 2940

accacacctg gctaattttt tgtattttta gtagagacag ggtttcttcc catgttgccc 3000

aggctggtct cgaactcctg gactcaagca atccgcccac cttgacttcc caaagtgctg 3060

ggattatggg cgggtgtgag ccattgcgcc cagccttgaa gtcatgttct aaattgtatt 3120

tgaatttgtg cctctttgtt tttccccaaa ccaaagccct caaattgtag tctctgtcgg 3180

cttctgcaga attctggaaa atgccagttt tcctcccccg cccttgtttt ccataaaaca 3240

tatttatata ttgtgatgag gagtactttc tgaagagtac ttcgtatttt tttttaattg 3300

ccttgtttgc cttcaacttc cttgattttc atagtttaca tgggtgtgtg taggggtgtg 3360

tgtgtgtatg tgtgtgggtt agggcttttt tcgttgcatg tgatggttct gtggacatat 3420

gatccccaca aactgtggga gtgattggcc aggccttgtt ttgtttgttt gtttgtttgt 3480

gtttttgttc ttttgaagaa tagagtggta tttagaaaat aaattgcatt gcaaagctct 3540

tatcggctca tatgagagag caggttcctg cccttgaaaa tgccggtaag ctatagcata 3600

tgttttttaa gacttaagca tttcatgctt taaaatacct tcacaagtga acattacaca 3660

cagaagttca tttggttttc ctttgtttta tggtgcatat agcaataaag acccccctcc 3720

accctgcaac ccccatcccc caccgggcct ttgtccctgc cttggctttt ctccccttct 3780

cattctcctc tcccctttcc tcactgaagg ctgtgagttg ctttcaatgt gacaacacta 3840

tgatgtcatt tggaaggatt tgccaggaca gactgattct gagtcctggg tgccgtatgt 3900

gtatgcggca gtgttgtcag gcgatcttgt ttgaagctct atgttgccat aattaccatc 3960

aagtacacac tgttggcaaa aggctaacac ctgactttag aaaatgctga tttgagaaca 4020

aaaggaaagg tcttttttca ctgcttaaag tggggtcact ttgatacctt tgcggtcatg 4080

tctgtgtctg atgagtgtag aatctctgga tgtgcactgt cagtcatgtg tccaccaggc 4140

ctcgaatatc atatgggaaa tgtcatagtt aaaaacgtac agccaggccc gtgtgctgtt 4200

aatagtgtga aattgtcatg ttaaaaaaaa aaacaggaac caaatgtgac cttgtgcata 4260

tattggtagc tgaaaatctt caaggctact gatgggtggc cccttaatct tgtctttgat 4320

tgctgtgtgc agggaaaggt gtccccgttt gttcatgctg ttttgggggg tgggggggta 4380

tttgcaagaa tactcatttt gacataatag gtcctcttgt cagagatcct ctaccacaga 4440

cattaatagc tgagcaggag ccacatggat tgattgtatc cactcaccat tgacgatggc 4500

attgagcgta gctagcttat ttccatcact acgtgttttt gagcttgctc ttacgtttta 4560

agaggtgcca ggggtacatt tttgcactga aatctaaaga tgttttaaaa aacacttttc 4620

acaaaaatag tcctttgtca ttacattatt tactcatgtg tttgtacatt tttgtatgtt 4680

aatttatgaa tgattttttc agtaaaaaat acatattcaa gaaccaaaaa aaaaaaaaaa 4740

aaa 4743

<210> 144
<211> 2439
<212> DNA
<213> Homo sapiens

<400> 144
gagagcagcg gccgggaagg ggcggtgcgg gaggcggggt gtggggcggt agtgtgggcc 60

ctgttcctgc ccgcgcggtg ttccgcattc tgcaagcctc cggagcgcac gtcggcagtc 120

ggctccctcg ttgaccgaat caccgacctc tctccccagc tgtatttcca aaatgtcgct 180

ttctaacaag ctgacgctgg acaagctgga cgttaaaggg aagcgggtcg ttatgagagt 240

cgacttcaat gttcctatga agaacaacca gataacaaac aaccagagga ttaaggctgc 300

tgtcccaagc atcaaattct gcttggacaa tggagccaag tcggtagtcc ttatgagcca 360

cctaggccgg cctgatggtg tgcccatgcc tgacaagtac tccttagagc cagttgctgt 420

agaactcaaa tctctgctgg gcaaggatgt tctgttcttg aaggactgtg taggcccaga 480

agtggagaaa gcctgtgcca acccagctgc tgggtctgtc atcctgctgg agaacctccg 540

ctttcatgtg gaggaagaag ggaagggaaa agatgcttct gggaacaagg ttaaagccga 600

gccagccaaa atagaagctt tccgagcttc actttccaag ctaggggatg tctatgtcaa 660

tgatgctttt ggcactgctc acagagccca cagctccatg gtaggagtca atctgccaca 720

gaaggctggt gggtttttga tgaagaagga gctgaactac tttgcaaagg ccttggagag 780

cccagagcga cccttcctgg ccatcctggg cggagctaaa gttgcagaca agatccagct 840

catcaataat atgctggaca aagtcaatga gatgattatt ggtggtggaa tggcttttac 900

cttccttaag gtgctcaaca acatggagat tggcacttct ctgtttgatg aagagggagc 960

caagattgtc aaagacctaa tgtccaaagc tgagaagaat ggtgtgaaga ttaccttgcc 1020

tgttgacttt gtcactgctg acaagtttga tgagaatgcc aagactggcc aagccactgt 1080

ggcttctggc atacctgctg gctggatggg cttggactgt ggtcctgaaa gcagcaagaa 1140

gtatgctgag gctgtcactc gggctaagca gattgtgtgg aatggtcctg tgggggtatt 1200

tgaatgggaa gcttttgccc ggggaaccaa agctctcatg gatgaggtgg tgaaagccac 1260

ttctaggggc tgcatcacca tcataggtgg tggagacact gccacttgct gtgccaaatg 1320

gaacacggag gataaagtca gccatgtgag cactgggggt ggtgccagtt tggagctcct 1380

ggaaggtaaa gtccttcctg gggtggatgc tctcagcaat atttagtact ttcctgcctt 1440

ttagttcctg tgcacagccc ctaagtcaac ttagcatttt ctgcatctcc acttggcatt 1500

agctaaaacc ttccatgtca agattcagct agtggccaag agatgcagtg ccaggaaccc 1560

ttaaacagtt gcacagcatc tcagctcatc ttcactgcac cctggatttg catacattct 1620

tcaagatccc atttgaattt tttagtgact aaaccattgt gcattctaga gtgcatatat 1680

ttatattttg cctgttaaaa agaaagtgag cagtgttagc ttagttctct tttgatgtag 1740

gttattatga ttagctttgt cactgtttca ctactcagca tggaaacaag atgaaattcc 1800

atttgtaggt agtgagacaa aattgatgat ccattaagta aacaataaaa gtgtccattg 1860

aaaccgtgat tttttttttt ttcctgtcat actttgttag gaagggtgag aatagaatct 1920

tgaggaacgg atcagatgtc tatattgctg aatgcaagaa gtggggcagc agcagtggag 1980

agatgggaca attagataaa tgtccattct ttatcaaggg cctactttat ggcagacatt 2040

gtgctagtgc ttttattcta acttttattt ttatcagtta cacatgatca taatttaaaa 2100

agtcaaggct tataacaaaa aagccccagc ccattcctcc cattcaagat tcccactccc 2160

cagaggtgac cactttcaac tcttgagttt ttcaggtata tacctccatg tttctaagta 2220

atatgcttat attgttcact tctttttttt ttatttttta aagaaatcta tttcatacca 2280

tggaggaagg ctctgttcca catatatttc cacttcttca ttctctcggt atagttttgt 2340

cacaattata gattagatca aaagtctaca taactaatac agctgagcta tgtagtatgc 2400

tatgattaaa tttacttatg taaaaaaaaa aaaaaaaaa 2439

<210> 145
<211> 819
<212> DNA
<213> Homo sapiens

<400> 145
cggttccgga atcccccaga accgtgagtc ctcctgagga agccgtcggg ggcctggccc 60

tgttaggcac tgtctcccag agtccccctt tcccactagc cgcctccgcg tagcccgaga 120

ccgcgtcagg agttgcgtct tcctggctgc gcctttttcg ggactgtccc ctgcgccgtc 180

cgccgcctct cactgtcccc ggagcgcgcg ccacccgccc gcttcccaca ggcggtttca 240

cgcgggtccc cggcgccgcg cgcggctccg gccctggcga ctcttgggac acccgagtag 300

ttgcgtgata gcggggctgt cctgaggatt cggcgtgcca ggttgagagc ccgtcgcgtg 360

ccggggactc gcggccactg tcgcttcccg tccgcccctc ttctcctggg atttggggag 420

gtgggcggga aagagaactg gtcggcggcc acccttgccg actcctcctc agcaggtcgc 480

agttgaaagg tcatccttct agagcccact cccctggccc ggcgggcaac ggataggtct 540

ccaagtccca ccctctcaca gcgctcggtt ctgttccact ttagaacagg tcgcagttgc 600

cactacgtaa acaagctccc catagagaga gtatttattc attccgattg ctccgtcttt 660

tatctagaac agagtctggt acaatggctc tcgaaatgtg gtctggtgac ctcagggtcg 720

cctgagaccc ttctagggaa ttcacaaagt caaaacacca ttgataacgt tctaacgtgt 780

tatttgccag ttttaccctc attctgttgg cacctgtca 819

<210> 146
<211> 2217
<212> DNA
<213> Homo sapiens

<400> 146
ttgcgggtcc tagaagtcgc ctccccgcct tgccggccgc ccttgcagcc ccgagccgag 60

cagcaaagtg agacattgtg cgcctgccag atccgccggc cgcggaccgg ggctgcctcg 120

gaaacacaga ggggtcttct ctcgccctgc atataattag cctgcacaca aagggagcag 180

ctgaatggag gttgtcactc tctggaaaag gatttctgac cgagcgcttc caatggacat 240

tctccagtct ctctggaaag attctcgcta atggatttcc tgctgctcgg tctctgtcta 300

tactggctgc tgaggaggcc ctcgggggtg gtcttgtgtc tgctgggggc ctgctttcag 360

atgctgcccg ccgcccccag cgggtgcccg cagctgtgcc ggtgcgaggg gcggctgctg 420

tactgcgagg cgctcaacct caccgaggcg ccccacaacc tgtccggcct gctgggcttg 480

tccctgcgct acaacagcct ctcggagctg cgcgccggcc agttcacggg gttaatgcag 540

ctcacgtggc tctatctgga tcacaatcac atctgctccg tgcaggggga cgcctttcag 600

aaactgcgcc gagttaagga actcacgctg agttccaacc agatcaccca actgcccaac 660

accaccttcc ggcccatgcc caacctgcgc agcgtggacc tctcgtacaa caagctgcag 720

gcgctcgcgc ccgacctctt ccacgggctg cggaagctca ccacgctgca tatgcgggcc 780

aacgccatcc agtttgtgcc cgtgcgcatc ttccaggact gccgcagcct caagtttctc 840

gacatcggat acaatcagct caagagtctg gcgcgcaact ctttcgccgg cttgtttaag 900

ctcaccgagc tgcacctcga gcacaacgac ttggtcaagg tgaacttcgc ccacttcccg 960

cgcctcatct ccctgcactc gctctgcctg cggaggaaca aggtggccat tgtggtcagc 1020

tcgctggact gggtttggaa cctggagaaa atggacttgt cgggcaacga gatcgagtac 1080

atggagcccc atgtgttcga gaccgtgccg cacctgcagt ccctgcagct ggactccaac 1140

cgcctcacct acatcgagcc ccggatcctc aactcttgga agtccctgac aagcatcacc 1200

ctggccggga acctgtggga ttgcgggcgc aacgtgtgtg ccctagcctc gtggctcaac 1260

aacttccagg ggcgctacga tggcaacttg cagtgcgcca gcccggagta cgcacagggc 1320

gaggacgtcc tggacgccgt gtacgccttc cacctgtgcg aggatggggc cgagcccacc 1380

agcggccacc tgctctcggc cgtcaccaac cgcagtgatc tggggccccc tgccagctcg 1440

gccaccacgc tcgcggacgg cggggagggg cagcacgacg gcacattcga gcctgccacc 1500

gtggctcttc caggcggcga gcacgccgag aacgccgtgc agatccacaa ggtggtcacg 1560

ggcaccatgg ccctcatctt ctccttcctc atcgtggtcc tggtgctcta cgtgtcctgg 1620

aagtgtttcc cagccagcct caggcagctc agacagtgct ttgtcacgca gcgcaggaag 1680

caaaagcaga aacagaccat gcatcagatg gctgccatgt ctgcccagga atactacgtt 1740

gattacaaac cgaaccacat tgagggagcc ctggtgatca tcaacgagta tggctcgtgt 1800

acctgccacc agcagcccgc gagggaatgc gaggtgtgat tgtcccagtg gctctcaacc 1860

catgcgctac caaatacgcc tgggcagccg ggacggccgg gcgggcacca ggctggggtc 1920

tccttgtctg tgctctgata tgctccttga ctgaaacttt aaggggatct ctcccagaga 1980

cttgacattt tagctttatt gtgtcttaaa aacaaaagcg aattaaaaca caacaaaaaa 2040

ccccacccca caaccttcag gacagtctat cttaaatttc atatgagaac tccttcctcc 2100

ctttgaagat ctgtccatat tcaggaatct gagagtgtaa aaaaggtacc aatcattgat 2160

tttttttttt tttgtaaact aaaatgttta aaataaaata gcatttacag ttaaaaa 2217

<210> 147
<211> 4074
<212> DNA
<213> Homo sapiens

<400> 147
gacaggcctc tcggcgagcc ggtgccccat cgggtaccgc acggctgccg cggcggcctt 60

accctgccgc gagcgcctgt gacagcggcg ccgctgtgct cgcgaccccg gctccgggcc 120

tctgccgacc tcaggggcag gaaagagtcg cccggcggga tggcggggga ggctgggtgc 180

gcggcggccg tgggtgccga gggccgcgtg aagagcctgg gtctggtgtt cgaggacgag 240

cgcaagggct gctattccag cggcgagaca gtggccgggc acgtgctgct ggaggcgtcc 300

gagccggtgg ccctgcgcgc gctgcgcctg gaggcccagg ggcgcgccac cgccgcctgg 360

ggcccgagca cctgcccccg cgcctcggcc agcaccgcgg ccctggctgt cttctcggag 420

gtggagtacc tgaacgtgcg cctcagcctg cgggagcccc cggccggtga aggcatcatt 480

ttattacagc ctggaaaaca tgaatttcca tttcgctttc aacttccatc tgaacctttg 540

gtcacctcgt ttactgggaa atatggaagc attcagtact gtgtgcgggc agtgttggaa 600

cgacccaagg tacctgatca gagtgtaaag cgggaactcc aggttgttag tcatgtcgat 660

gtcaacacac cagcattatt aacccctgta ttgaaaactc aagagaaaat ggttggctgt 720

tggtttttca cttctggtcc agtctcgctg agtgccaaaa ttgaaagaaa gggatactgt 780

aatggagaag ctattccaat ctatgcagaa atagaaaatt gttcctctcg tctgattgtt 840

ccaaaggctg ctattttcca aacgcagaca tatttggcta gtggaaaaac aaagaccatt 900

cgacacatgg tcgccaatgt gcgaggaaac cacatcgctt ctgggagcac agacacatgg 960

aatggaaaaa cgctaaaaat cccacctgtt actccatcca tcctggattg ctgcattatc 1020

agagtggact attccttagc tgtatacatt cacattcctg gtgctaaaaa attgatgctc 1080

gaactgccat tagtgatcgg tacaattcca tataatggtt ttggcagcag aaactccagc 1140

attgccagcc agttcagtat ggatatgagc tggttgacac tgaccctgcc agagcagcct 1200

gaagcaccac caaattatgc agatgtggta tcagaggaag aattctctag acacattcct 1260

ccttaccctc aaccccctaa ctgtgaggga gaagtgtgct gtcctgtgtt tgcctgtata 1320

caagaattcc ggtttcaacc cccacctctt tattcagagg ttgacccaca tcctagcgac 1380

gtagaagaga gccagcctgt ttccttcatt ctctgaacgt atttcagaaa tcactgtgtt 1440

catcatcaaa ttagaatgtt ggttcttttc cttctgcctt tttgggaaag agacaggaaa 1500

gattcacttg aaaacataaa tgaacgtcaa gactgaaggc aatagaaatt aaagaatgtg 1560

agaaagttct ggtgggccgg caggattgcc gcacaagttt atatgatggt cgtatatata 1620

tccctgttaa aaactgggat gaagatgtgc aaagtcacag aatgtaatgg aagtcctgat 1680

ggttacagag taagtgaaag ggtgcctgcg ctgacgtgag agaaaggaat ctgtaaacag 1740

tggaaacact gtgggagttt cccatggtga agagtggaac gaaggcgata tgaactgaag 1800

gggtgaagac ttgattttgg agagggcaac aaaacaaggg tgtgtgtgca taggagaatg 1860

gcccactcca aatacgaagt gagatcctga gtctttgggt gcttcatgat ttcctaccat 1920

attcaggcct aaagacattg aaaaagcatc ttttcttgag atcatggtca tatgaggtcc 1980

taatgaagta ctacagtttt cattctttca agggtagact aaaatatagt ttataaatcg 2040

gcagtacggt attatgaaac caagaaaggg tttcttgaaa agcttgtcgg ttcaaagagg 2100

aaagatgaat ttcaatgtga aaacacgttt tgttgagggc tgtacttttt acccccttta 2160

agtgctttaa caggatatac gtttgatttt cctcatatct tatttaccta ggagcatgta 2220

agagaaagaa gggagagaaa aggttgcatc tgcaggatgc cttgataact acacagtccc 2280

aaataaaagg cctttttcta acctacctct aatggggtta tcagatatgt ttttaaatct 2340

ctcgccctga gtactcttct tgggagttgc tgctgtttac agccacagtc tcatctatca 2400

ctaaatatga aactgggcca aggggaaaat tcagtaagtt agcgttaaat ggaagtaaat 2460

atatgtatac ctaagagaat gtcaaaataa acaccaaacc aaaagaaggt aggagtgcca 2520

tttttagaaa agacacaact ttaattcctg tcaagagacc taggtctact gatggcagtc 2580

actgcattgt acattattcc agttcttcag taattctgtt taaactgtga ttttctttat 2640

tgtgttggtg caatatttta ttatcattta acctcaggat attcagacca acaggattgt 2700

gttgctgctg aaagtctgag cagtgtttta ccgtccccag cccccctccc acatggtcac 2760

atgttcactt gcttttcctt ccatctgttg gttttgtgag ctgagagcat ctctggacac 2820

atggaaggga gccagggatt cctgtgatac agattttgaa ccagcagagt actttggttt 2880

tcttcatgtc ctaagggttt gatccagaaa ccctggtgct tcccctgcag aatactgtac 2940

ttcatttaac agaatgattg atttttcttt caatcagcat gttcaccaaa aatagaaaag 3000

ttcttctaag gttaaatttt gtatttaaaa gttagttggc catttgatca gggaattttc 3060

tacacacatt aggcaaatgt atgcttttca tcctctgggc ttacctctct cctctgcaat 3120

cctggaagca tcgtgcatag ttctttcatt gtaagccgct atttaaattc ccacaatgca 3180

aaactgttag accagtaatt gtgctactga aattgtcaca aaacgttatt ccttgatttg 3240

gtttttaaat tttgctttcg tgtgtgtgtc tttaaacagg taatgtgtat ttaagtgttg 3300

tctaaacagt tgatttttac tttagaaaag atcatcttca actgtttatt gacttctgag 3360

ttatatagtt catgccctgc caaggcattg ttcgactcca tgatactaaa acataatgaa 3420

agaaacaaac tccagtatgg agaagagaaa gtttaattat ttggttccat ctagaaaagc 3480

aacaacttaa aggaatcctt cactaggatt gtcttatcac ctttattaga taaatcatga 3540

agtgtccctg tgaaattgaa gtgaaactat ctctgtagga cttaagagaa tagctaaaag 3600

gtgtgacttg ccttattgaa atgatactgg catatctgac tgtaagcagt aggttgaaga 3660

tatcatttta tgaatgtgga gaattctaca ttgaaacaga aaatacctgg gaatgaagat 3720

taaaaatgta gctgctgtta tttgcttggt tattcccctc ttgctcttct ttagtttgaa 3780

aaaacaaaaa cgtggccttg gaattttcat tttgatgcag aaattttgaa attgaaaatg 3840

tgcatgtttt ggtgcacaaa atccttctgt gggcaaaact ttgtttttgt tttgcacagt 3900

aagaaacaat aggcaagcgt tatgtttttg gtaagttaac tatgaaagct ttcttatttt 3960

tattattaaa aatgtaacaa tttaacccac aggaaaaaaa attactttgt atgcttgttt 4020

gaaccctatg ggttcgtttt attaataaaa ttattacact aatgctaaaa aaaa 4074

<210> 148
<211> 1609
<212> DNA
<213> Homo sapiens

<400> 148
gggattggag tgctctgctc cggacgcact acgtaggtgg gcctgcggga gttctgagtg 60

cgacggcgca ggtctgcacc atggcggccc ggcttgtcag ccgatgcggg gctgtgcgtg 120

cagctcccca cagcggcccg ctggtgtcct ggcgcaggtg gtccggcgcc tcaacagaca 180

ccgtgtatga cgtggtggtg tcgggtggag gcctggtggg cgctgccatg gcctgtgcct 240

tgggatatga tattcacttt catgacaaga aaatcctgtt gctcgaagca ggtccaaaga 300

aagtactgga gaaattgtca gaaacttaca gcaacagggt cagctccatt tcccctggct 360

ctgcaacgct tctcagtagt tttggtgcct gggaccatat ctgcaacatg agatacagag 420

cctttcggcg aatgcaggtg tgggacgcct gctcagaggc cctgataatg tttgataagg 480

ataatttaga tgacatgggc tatatcgtgg agaatgatgt catcatgcat gctctcacta 540

agcagttgga ggctgtgtct gaccgagtga cggttctcta caggagcaaa gccattcgct 600

atacctggcc ttgtccattt cctatggccg actccagccc ttgggttcat attaccctag 660

gtgatggcag caccttccag accaaattgt tgataggtgc agatggtcac aactccggag 720

tacggcaggc tgttggaatc cagaatgtga gctggaacta tgaccagtct gctgttgtgg 780

ctactctgca tttatcagag gccacagaaa acaacgtagc ctggcagaga tttcttccct 840

ctgggcctat tgctctgctc ccgctctcag acaccttgag ttccttggtt tggtccacgt 900

cccatgaaca tgcagcagag ctagttagca tggatgagga aaaatttgtg gatgccgtta 960

actctgcctt ttggagtgat gctgaccaca cggacttcat cgacacagct ggtgccatgc 1020

tgcagtatgc tgtcagcctt ctgaagccca ctaaggtctc ggctcgccag ctgcccccaa 1080

gcgtagccag ggtggatgcc aaaagccgag ttctgtttcc tcttgggttg ggacatgctg 1140

ctgagtacgt caggcctcgg gtggcgctca ttggggatgc agcccacaga gtccatccgc 1200

ttgcaggaca gggtgtcaac atgggctttg gggatatctc cagcttggcc catcacctca 1260

gtacggcagc cttcaatggg aaggacttag gttccgtgag ccacctcaca ggttatgaaa 1320

cagaaagaca gcgtcacaac actgctcttc tggctgctac agacttacta aaaaggctct 1380

attctaccag tgcctccccg cttgtgttgc tcaggacgtg gggcttgcag gccacaaatg 1440

cagtgtctcc actcaaagaa cagattatgg cctttgcaag caaatgagta ctcctctcct 1500

aaagaaagat tacgttgatg aaaaagaaca tcctgcccag gacccatcat acatattttc 1560

aagatcttat ttaatttaat aaacttactt tacattaaaa aaaaaaaaa 1609

<210> 149
<211> 5821
<212> DNA
<213> Homo sapiens

<400> 149
tgaggtccgg aagcggctgc cgggcagcaa aggaggatgg cgaggggctg atactgaacc 60

cgggaagggt gggctgtgct gaagccagag ccggagccgg agctggggcc agaacccgag 120

cagtgagttc ctccactggc tgccggctgg cggcgctcgc cgccttgggc aggacccacc 180

tcgccttcct cccggcgtgg cagatgctcc agctgccatg tccacgcaga gacttcggaa 240

tgaagactac cacgactaca gctccacgga cgtgagccct gaggagagcc cgtcggaagg 300

cctcaacaac ctctcctccc cgggctccta ccagcgcttt ggtcaaagca atagcacaac 360

atggttccag accttgatcc acctgttaaa aggcaacatt ggcacaggac tcctgggact 420

ccctctggcg gtgaaaaatg caggcatcgt gatgggtccc atcagcctgc tgatcatagg 480

catcgtggcc gtgcactgca tgggtatcct ggtgaaatgt gctcaccact tctgccgcag 540

gctgaataaa tcctttgtgg attatggtga tactgtgatg tatggactag aatccagccc 600

ctgctcctgg ctccggaacc acgcacactg gggaagacgt gttgtggact tcttcctgat 660

tgtcacccag ctgggattct gctgtgtcta ttttgtgttt ctggctgaca actttaaaca 720

ggtgatagaa gcggccaatg ggaccaccaa taactgccac aacaatgaga cggtgattct 780

gacgcctacc atggactcgc gactctacat gctctccttc ctgcccttcc tggtgctgct 840

ggttttcatc aggaacctcc gagccctgtc catcttctcc ctgttggcca acatcaccat 900

gctggtcagc ttggtcatga tctaccagtt cattgttcag aggatcccag accccagcca 960

cctccccttg gtggcccctt ggaagaccta ccctctcttc tttggcacag cgattttttc 1020

atttgaaggc attggaatgg ttctgcccct ggaaaacaaa atgaaggatc ctcggaagtt 1080

cccactcatc ctgtacctgg gcatggtcat cgtcaccatc ctctacatca gcctggggtg 1140

tctggggtac ctgcaatttg gagctaatat ccaaggcagc ataaccctca acctgcccaa 1200

ctgctggttg taccagtcag ttaagctgct gtactccatc gggatctttt tcacctacgc 1260

actccagttc tacgtcccgg ctgagatcat catccccttc tttgtgtccc gagcgcccga 1320

gcactgtgag ttagtggtgg acctgtttgt gcgcacagtg ctggtctgcc tgacatgcat 1380

cttggccatc ctcatccccc gcctggacct ggtcatctcc ctggtgggct ccgtgagcag 1440

cagcgccctg gccctcatca tcccaccgct cctggaggtc accaccttct actcagaggg 1500

catgagcccc ctcaccatct ttaaggacgc cctgatcagc atcctgggct tcgtgggctt 1560

tgtggtgggg acctatgagg ctctctatga gctgatccag ccaagcaatg ctcccatctt 1620

catcaattcc acctgtgcct tcatataggg atctgggttc gtctctgcag ctgcctaccc 1680

ctgccccatg tgtcccccgt tacctgtcct cagagcctca ggtatggtcc aggctctgag 1740

gaaagtcagg gttgctgtgt gggaacccct ctgcctggca cctggatacc ctgggccagg 1800

taacctgagg gcaggggaga ggtggggtgg cagacacgca gaagtgctac tagtgacagg 1860

gctgccatcg ctcacctgta cctatttaca cccagaactt tccagctccc cctcatcatg 1920

cctcctcctt cctacctgcc tcccctctgc tggtgcacct cgcccaactc attcttactg 1980

cacagttcac tttatttaac aattttcatg tcccccacct catgttttca ccttttctgg 2040

gccaggcata gattaagtaa ctgggaacgc cccctcttta taaagctggg cttctttctc 2100

atctctctcc caaatgttgt atctcagtat tcttcctatt cgagtctcca gggggtggct 2160

ggacctacct ggtcatttga aacaggcccc caagctggag tttttaatct ggactctctg 2220

gcttgctgtg acccctaagg caatgcttct cttccctgga ttccttagtg tgggtcacag 2280

tactgtgttc ttagttgctt tagctcttaa aacatacgaa gtgttgccta aactgaaaat 2340

atttatcttt tatttaaaat cagatttttg tttttagact gtcttagatc tggggctatt 2400

acgaatcact tcttcttcag taaactttga ctcaacttct cctgctgaaa agaagctcgc 2460

tccagatgtc tgcatgggtc ctcggcactc ttggctgagg actcaaaggt tttaatcagg 2520

atcgtctaaa aatgtacctc ggtgaggagg cacagatttt gcctcctgtt gaccagcctg 2580

gtttcatacc gaaaagacat tgaaggactg cagaaatgta tgggtgcacc gggccgaggg 2640

aagggtggct gagtgagagg cgtataaaat ggggctgtgt gcatgcaggc ccatgtttca 2700

gcctcagccc acgccaggtg aaaggatcag caatgctctg ttgccatcgt gctgggacga 2760

caccagctct attgccaccg atgagtagct gaggtcagtg tgcacagagt ttgaaattaa 2820

gttaatagac tttacagcag ctggtctgac actacgcgca gtgctcggtt gtttacaatc 2880

agtggggaaa agggcagaac cagtgcccgg ccccacactg cctctgtggc ctggactttg 2940

aaaggaaccc actgaacact aattatgagc cctgtctttc ccccagaatg cctccctggg 3000

tttcacaaac agccttgagg ttggccctcc tcaaggtcag ccttcagatt tgggagcaaa 3060

cttcagagaa ggcagaggaa gatacattgc cttgctgtgg gctgcctctt ctttcctctt 3120

ggtgtgcgaa gtatttcaga aggccattga tgaattcccc ctctttagct gtgtatttgt 3180

gcacgtgtgt gtgtacgtgc gtgtgtgtgt gtgttcctgt gtaagtaaca gaccagactc 3240

cttttctctt ctgtcccgtc accaggctct tgcttcactg cagatacagt tcactctgaa 3300

agctggttga aggagagcag caaaaatgta tcaggggttt tgcttctgtg tttcgccaaa 3360

gctcataagg gctgtgaccc acccatatgg ccccagtttt ttctgtctct tctgttccaa 3420

agccaggaga gctgacttcc aggtgaaggg atgggaaaag tggactctca ttgtagtgac 3480

tcccaaccta cctaataatt tgttaactta ggaatatgct atcattgttg acttgttctt 3540

ccttaggaga aggacgattt tcacccaccc tttctgttct atggtggact cttaacaggt 3600

gctatgtgac caggaatcta gccgggagta gcagaggccc tgtcttctga agtctcaggc 3660

ttagaagtta ccaaagtggg ctcagaaact gtcatctcct ggttccaagt tcgggctctg 3720

gcagcccagc cgctatctta gctgtctttc ccagcggtgc taagagtggt ctcagtgaga 3780

aggtagatgc caactggagg gccagacctg tgtcctgccc atgtcctcct tggtggacgt 3840

ttctgtttac tcagagctgc tagagaccat cctgcccatc cgagttctga gattgggact 3900

gtgatgttgg gacctgagga ctggatggta gaatactggg gtcccccagc tcttagcagg 3960

atgcaggcta ttgcttccac acccctggcc gtgagaacgt ggtatgtagg agagttggct 4020

gtagctttag gatttctgga agccaatttg gcatggccta tttgatctct ggcttgtgct 4080

cctgctacac tgacagactg attgcgtggc tctttcagaa gacccaggaa gggcagctcc 4140

agccagagga ccttcctggc tatgcaggtg cacagctctt aggcatcaag caaagggtca 4200

gccagtcagt agtagtggga ggaagccctt cctcctctta tgcaagcagc tcgcagccag 4260

cccagaatct cttatgcagc ccaaagggct tcttgaggta gagagccctc cccagtgtct 4320

tcccaggata agtagaaata tgatcacaga gcaacggagc aaaggctttc gggagtgtga 4380

ggctgcatct gctggagcaa agggaaaccg tgggcttttc cggccagata ctcttgagct 4440

ctgtgaccct gctcctgtca ccccaatttc tccaagccag aggtagcttt ctcagagccc 4500

cttggtggtt ctgtcctcca aatgctgctg ttgggagcgg gcttccagct ctcagtgggc 4560

agtgcacctc ctcctaaatg caggcacttg ctaggaagag tgtgagctgg cgtcttctgt 4620

tcaccctgcc tagcagttgt caccattcac aagtggcatt atttatgttg tgctgctgtc 4680

cagctgctaa gatcccttca tctgcacaag ccctggctag atatgtgtga atgtgtggca 4740

tcatttcact tcagccgccc aattccatct ctcctctgca gccagattca ctggctgatg 4800

ctcactgctc actgtctatc cccacgaaat ttagttttat cagtagtctt aaagtgttga 4860

tctcaaacaa gtacattaga aaaatcatgt ttcttctctc tcatcttact ttttcttctc 4920

agatttctcc cttcctagaa cattctctct gtttagcact aatgttcacc tcgtattttt 4980

tggaagtgca aaaatctcaa tttgtgtctg tttacagctc tctctcctca ctgctcacag 5040

caaggggttc tgtatcagtg gatttcattt tgtagctgct gagatgttaa ggcaagcctc 5100

agcatctgcc cctgctgggt gcacaatgct gcttcctcga agagaagaca cagagtccaa 5160

gtggcaggac ttgaggttgg cttccactct gccttagaag ttaattttcc aaagtacatt 5220

acaaatctct gaggccatta ggggaaaagg aaggggtgtg gtttgtcttt gaaattacgg 5280

ttaatacttt tagacagtaa gtccggctgg ttgcagggct atttgccccg acagcatcag 5340

cctgtaacat ttcttctctt tcctttgtgc cactgagtcg ttccctggcc agaggacata 5400

aatggtgctg gtaggaggtt atcagagtaa ggaaggtagc agatataggt gcagggtgcc 5460

tgtcattcac tgtgttattt ggtttaaatc aaagtgattc tgggggaagc tatgctcttt 5520

cagtggataa taaaattggt aactctattg taaaacatgt caatggtgtg tgaagaaaaa 5580

tcaaccaatc tgtaggtgtt gataactaga cagtactgtg tatgttacgt gcctgtgtgg 5640

atgtgcactt ccagcatggt atgtgtagcg atgtggatca tgccagagtt cgtagatcct 5700

gttttggggt ttgcacatgg atcgtatgtt aagctttttc ttttcaataa atgaatttta 5760

tttttatttt tgaaaaaaaa aaaaaaaaaa aaaaaaaaaa aaaaaaaaaa aaaaaaaaaa 5820

a 5821

<210>  150
<211>  7589
<212>  DNA
<213>  Homo sapiens

<400>  150
   gccgcgcgcg agagcctcgg cctggccgcg ctgcgcccgc cgccgccgcc gccccctccc      60
   cgcctgggcc ctaccgccgc acggccccgg ccccctccca gcctgccgct ccggagagcc     120
   gcccgccgag gatgcgacgc accgcagtgt tctaagaacg gaagcatctg ggctggatgg     180
   aatttagcat caagcagagt cccctttctg ttcagagtgt tgtaaagtgc ataaagatga     240
   agcaggcacc agaaatcctc ggcagtgcca acgggaagac tccgagctgc gaggtgaacc     300
   gcgagtgttc tgtgttcctc agcaaagccc agctctccag tagcctgcag gagggggtca     360
   tgcagaagtt taacggccac gacgccctgc cctttattcc agccgacaag ctgaaagatc     420
   ttacttcccg ggtgtttaat ggagaacccg gcgcacacga tgccaaactg cgttttgagt     480
   cccaggaaat gaaagggatt gggacacccc ctaacactac ccctatcaaa aatggctctc     540
   cagaaattaa gctgaaaatc accaaaacat acatgaatgg gaagcctctc tttgaatctt     600
   ccatttgtgg tgacagtgct gctgatgtgt ctcagtcaga agaaaatgga caaaaaccag     660
   aaaacaaggc gagaaggaac aggaagagga gcataaaata tgactccttg ctggagcagg     720
   gccttgtcga agcagctctt gtgtctaaga tctcaagtcc ttcagataaa aagattccag     780
   ctaagaaaga gtcttgtcca aacactggaa gagacaaaga ccacctgttg aaatacaacg     840
   ttggtgattt ggtgtggtcc aaagtgtcgg gttacccttg gtggccttgc atggtttctg     900
   cagatccact ccttcacagc tataccaaac ttaaaggtca gaaaaagagt gcacgccagt     960
   atcacgtaca gttctttggt gacgccccag aaagagcttg gatatttgag aagagcctcg    1020
   tagcttttga aggagaagga cagtttgaaa aattatgcca ggaaagtgcc aagcaggcac    1080
   ccacgaaagc tgagaaaatt aagctattga aaccaatttc agggaaattg agggcccagt    1140
   gggaaatggg cattgttcaa gcagaagaag ctgcaagcat gtcagtggag gagcggaaag    1200
   ccaagttcac ctttctctat gtgggggacc agcttcatct caaccctcaa gtagccaagg    1260
   aggctggcat tgctgcagag tctttgggag aaatggcaga atcctcagga gtcagtgaag    1320
   aagctgctga aaaccccaag tctgtgagag aagagtgcat tcccatgaag agaaggcgga    1380
   gggccaaact gtgtagctct gcagagaccc tggagagtca ccccgacata gggaagagta    1440
   ctcctcaaaa gacggcagag gctgacccca gaagaggagt agggtctcct cctgggagga    1500
   agaagaccac agtctccatg ccacgaagca ggaagggaga tgcagcatcc cagtttttgg    1560
   tcttctgtca aaaacacagg gatgaggtgg tagctgagca cccagatgct tcaggtgagg 1620
   agattgaaga gctgctcagg tcacagtgga gtctgctgag tgagaagcag agagcacgct 1680
   acaacaccaa gtttgccctg gtggcccctg tccaggctga agaagactct ggtaatgtaa 1740
   atgggaaaaa aagaaaccac acaaagagga tacaggaccc tacagaagat gctgaagctg 1800
   aggacacacc caggaaaaga ctcaggacgg acaagcacag tcttcggaag agagacacaa 1860
   tcactgacaa aacggccaga acaagctctt acaaggccat ggaggcagcc tcctcgctca 1920
   agagccaggc agcaacgaaa aatctgtctg atgcatgtaa accactgaag aagcgaaatc 1980
   gggcttccac ggcagcatct tcagctcttg ggtttagcaa aagttcatct ccttctgcat 2040
   ccttaactga gaatgaggtc tcggacagcc cgggagacga gccctcggag tccccatacg 2100
   aaagtgcaga cgaaacacaa actgaagtat ctgtctcatc caaaaagtct gagcgaggag 2160
   tgactgccaa aaaggagtat gtgtgccagc tgtgtgagaa gccgggcagc ctcctgctct 2220
   gtgaaggacc ctgctgcgga gctttccacc tcgcctgcct tgggctttcc cggaggccag 2280
   aagggaggtt cacctgcagc gagtgtgcct cagggattca ctcatgtttc gtgtgtaaag 2340
   agagcaagac agatgttaag cgctgtgtgg taactcagtg tggaaaattt taccatgagg 2400
   cttgtgtgaa aaaataccct ctgactgtat ttgagagccg aggtttccgc tgccccctcc 2460
   acagctgtgt gagctgccat gcttccaacc cttcaaaccc aaggccgtca aaaggtaaaa 2520
   tgatgcggtg tgtccgctgc cccgttgcct atcacagcgg ggatgcttgt ctggcagcag 2580
   gatgctcagt gatcgcctcc aacagcatca tctgcactgc ccacttcact gctcggaagg 2640
   ggaagcgaca ccacgcccac gtcaacgtga gctggtgctt cgtgtgctcc aaagggggga 2700
   gccttctgtg ctgtgagtcc tgcccagcgg ccttccaccc tgactgcctg aacatcgaga 2760
   tgcctgacgg cagctggttc tgcaatgact gcagggctgg gaagaagctg cacttccagg 2820
   atatcatttg ggtgaaactt gggaactaca gatggtggcc ggcagaagtt tgccatccca 2880
   aaaatgttcc cccaaatatt cagaaaatga agcacgagat tggagaattc cctgtgtttt 2940
   tctttgggtc taaagattat tactggacgc atcaggcgcg agtgttcccg tacatggagg 3000
   gggaccgggg cagccgctac cagggggtca gagggatcgg aagagtcttc aaaaacgcac 3060
   tgcaagaagc tgaagctcgt tttcgtgaaa ttaagcttca gagggaagcc cgagaaacac 3120
   aggagagcga gcgcaagccc ccaccataca agcacatcaa ggtgaataag ccttacggga 3180
   aagtccagat ctacacagcg gatatttcag aaatccctaa gtgcaactgc aagcccacag 3240
   atgagaatcc ttgtggcttt gattcggagt gtctgaacag gatgctgatg tttgagtgcc 3300
   acccgcaggt gtgtcccgcg ggcgagttct gccagaacca gtgcttcacc aagcgccagt 3360
   acccagagac caagatcatc aagacagatg gcaaagggtg gggcctggtc gccaagaggg 3420
   acatcagaaa gggagaattt gttaacgagt acgttgggga gctgatcgac gaggaggagt 3480
   gcatggcgag aatcaagcac gcacacgaga acgacatcac ccacttctac atgctcacta 3540
   tagacaagga ccgtataata gacgctggcc ccaaaggaaa ctactctcga tttatgaatc 3600
   acagctgcca gcccaactgt gagaccctca agtggacagt gaatggggac actcgtgtgg 3660
   gcctgtttgc cgtctgtgac attcctgcag ggacggagct gacttttaac tacaacctcg 3720
   attgtctggg caatgaaaaa acggtctgcc ggtgtggagc ctccaattgc agtggattcc 3780
   tcggggatag accaaagacc tcgacgaccc tttcatcaga ggaaaagggc aaaaagacca 3840
   agaagaaaac gaggcggcgc agagcaaaag gggaagggaa gaggcagtca gaggacgagt 3900
   gcttccgctg cggtgatggc gggcagctgg tgctgtgtga ccgcaagttc tgcaccaagg 3960
   cctaccacct gtcctgcctg ggccttggca agcggccctt cgggaagtgg gaatgtcctt 4020
   ggcatcattg tgacgtgtgt ggcaaacctt cgacttcatt ttgccacctc tgccccaatt 4080
   cgttctgtaa ggagcaccag gacgggacag ccttcagctg caccccggac gggcggtcct 4140
   actgctgtga gcatgactta ggggcggcat cggtcagaag caccaagact gagaagcccc 4200
   ccccagagcc agggaagccg aaggggaaga ggcggcggcg gaggggctgg cggagagtca 4260
   cagagggcaa atagcgccag gcggccgctt ggccggatcc aggggcggtg cagggcggcc 4320
   ggccctgcct gcgggagagg gcgagcatga actggcccgg aggacccagc tcgagccgcc 4380
   aggacacaga cgtacaggcc tcctcgggag ggagcgcctc cccaccactg agccatcctc 4440
   agcagcgtcc gctgcgtctg cactgatgac cgtctgagcc cagctcagcg ttcctggaca 4500
   aacagcctca ctcctcagcg ttaccgccac acttgaattt ctccgaatgt caaggttccc 4560
   tcccactcta tttttttagg ttaaagttaa ttggcatatg gaatgtttta atctcctctg 4620
   aaatgtgtag cgtaggcttt tcccaagggt cgctagaaac tcgtcttcgc gttgccccct 4680
   ttctggctct cagcgccgtc gccactcggg agaggctggg tgaggcccgt gtgaggactg 4740
   accctggatt cctcgaaact gccattgtga tcattactct gctctttgga aatggctgta 4800
   tcattttttt gtactaatgt gaattgttcc tcagaaacgc ttcttttcca tcctagtgag 4860
   aagctggccc tgcaggtggt ggcagcaatg gtgttgtaag atttcctccc gtagtttttt 4920
   ctcctcatgg atttgaatga aatgccaata acacgtccac tttcaacgtg tagtttacgc 4980
   ggagcacttt cgaggcctgg ccgggttggg cctacttctc acctgggcct atcttctgaa 5040
   ctcgctaggt tcttatcaac atttggggga taactttgta tatttttttc atttggcttt 5100
   tctttaccag tttctgattt ttattctcaa tatatttttg ctaaacctat ttcacaaatc 5160
   accaccgact gaagtgtgtg tttactgatg cggccctgag ctccatggcg aaaggagtga 5220
   ctttgcaggg cgtgagaccg cagtctgctt agagcacagg aagtgacaac ttagggagcc 5280
   ccgtagggcg ctgcaggccc cggggacccc agcacgtggg tctaaagaga gacggagtct 5340
   agctctcctg ccacccagag tggcttccat ctcagcactc tgtgggtctg gtgatggaag 5400
   atgcagtctc tgctgatcac atgtgccctc tgccagggca cctactgaga ggtgcggtcc 5460
   tgggggtgga ggcctgcctg gcaggtgtgc gtgcctcgta cgtgtgttat gggcactggt 5520
   ctaggccagg tatgacaccc actctcctgt gagatttcac tttagttttt aaaaggtcca 5580
   gttctacaga gtgagaccta tctatctgag tactacatat gttttaagac ttggttcttt 5640
   ttttgaggga tccttgaccc tgggaagtct ggagcaccct gagaaggggg caccatgtgt 5700
   gcctttgccc acgtgtcctg aggggctgct tgtctgggag ggagggagag aacattcagc 5760
   agcaggtgct tttttatggc cttttcttaa aataacctaa gggggacaca tccatcttgc 5820
   agagaagttt acagaactcc ccttgaaaac tgctgctgag gctcctgtta aattttctgt 5880
   ggcatctttt atgccttggt aaaaactgca gtgtctttgg acctgagagt ggctactccg 5940
   tggttttgtg acctgtaagc gtggggttca ggggtgtgtg gccctgcagg gtcccacgcc 6000
   tccctgagca ctgactggaa gtttcactgg ctggtggctg tcccttctcc catcagggtc 6060
   cccagcaaag ttaactacac agaggaccca ggggaaacga gctgtgtagc cactgacttg 6120
   ctcgcgcggc cgtggcctct gaggggcact cgccggttaa gacagggtgg gagtagtgct 6180
   ttccagttca gactctaact tctcccaaag tgtcctaaga aaatactgga tcggctcata 6240
   gatttatgct ccttatgatg ccctaacttg gaaggttgtt ctagggacag gccgggcagt 6300
   gtccccacac acaccttaga gtcgaaggcc ccagggcccc gctgtcactt gcccaaaaga 6360
   tcccttccgg caggtaaggg actaccaatg cttacgtcaa aacagcagaa tcggctttgc 6420
   agtgcacttt ggggagcaga tattaactta tttttgtgtt ggacagtagt gaaatcttgt 6480
   gatttttaat cgctttgata atacttccaa attttatgat ttttctgaag gaaataatgc 6540
   aaacatttta aatatgtttc tccccctttc caaaaactgt taaactaatg agcaagtaac 6600
   actaactttg aatgtctcta caatacccgt tgataactca gtggagccag gctttggggt 6660
   agcggccctg agcttgcagg gtttctcgcc actggggctg accacgcccc cagctgtgac 6720
   cgtgggtgtg gctggctctc ggccctgccc agctttgttc tgaggacgtg gtgacttcct 6780
   gaacatcagc ttcaatcctc catcattaat gtgaagcaaa acacaaaaac cgccccaatc 6840
   cctcaggatt ccttggcatc cgaaaccagc atctgcacct aaacccatac ccacccgtgt 6900
   gcgcccacag ggggatgtgt ccgaatgggc agcttaaaat gtggtcacct gtgggggaaa 6960
   ctcttcaggc acctgaagtg agaacccagc tgtccgtcct caggccggcc tttcttccgg 7020
   cgacacccgt ccatggctgg ctgggtcccc ttcgcagtgt ttgtctgtct tgacatctaa 7080
   accccggcgt gtgcagtgcc catcttccag gactacctta ttttccagaa ttaaacctgt 7140
   tttataattc aagttaatgc aaatgactgt cagttgccaa atatcttgat cctatgagtg 7200
   tagttgatga ctgtttgtta gtcagtagag taaaatgctg tgtccacggg gtgtcacagc 7260
   ctcaccatac cctgttgagg tgtgaaatgc cccgtcagaa attaaataca aacttaaatg 7320
   tgcctattgg tgtctaaact tcatacaatg taaggtcaga ttccttttag gaatactggg 7380
   tgctgtcacc aggtttgata gttagactta aaaacttgaa attcactttt tggggggagg 7440
   gatatactga aatagagagt tgagacttgc cagttggggg aaaatagcat ttaaaatgga 7500
   aagctgtgtt tggaaaattg tgtatgagta tttttgtatt aaaaacattt taaaggcttt 7560
   tttcttaact taaaaaaaaa aaaaaaaaa 7589

<210> 151
<211> 8704
<212> DNA
<213> Homo sapiens

<400> 151
gcgcgaccga agcggataag ggacgcggcg gcggttggct gtgtctgttg agagagtaca 60

ggacagcccg gactgcgaga ggcggcggcg gaggcagtag ttgcaccggc agaggcggcc 120

gctggcagcg gttgaggcgg cggtccaacg cgtgctctct gggcggggtg cggcggggga 180

tgtgcctgct caggccgggc ggagtctctc tgacagggcg ggggatgcgc cgcggccgct 240

cgcccggacc ctgaggctgc tgggcccacc ctcccggaac cgtccgaccc tcggtggcct 300

cggcctcttc tgccatctcc ggtcctaccc tggggcggag ggtggaaggc agcttccgtc 360

gaagaggagg gggctgcggt ggccaccgcg gcggagcccg agttatttta ccaagaaaat 420

ggtttgcacg actttgaaca tatactatcc atgctgatgg gacaggatcc aatatgaata 480

taaatgatgg aggaagacga cgctttgaag ataatgaaca tacattacgg atatatcctg 540

gggctatttc agaaggacaa atctactgtc cgattcctgc cagaaaaaac tccacagctg 600

ctgaggtgat tgagtctctt ataaacaaac ttcatcttga caaaacaaaa tgttatgttc 660

tagcagaggt aaaggaattt ggtggagaag aatggattct caatccaaca gattgtccag 720

ttcagcgaat gatgctgtgg ccccgaatgg ctctggaaaa tcgcttaagt ggagaggact 780

accgcttcct tctgagagag aaaaaccttg atggatcaat ccattatggt agcctgcagt 840

catggctacg ggtaacagaa gaacgtcgca ggatgatgga acggggtttt cttccacagc 900

ctcaacagaa agactttgat gatttatgta gtttacctga tttgaatgag aaaactctct 960

tagaaaacct acgaaatcgc tttaagcatg aaaaaattta tacctatgtt ggcagtattc 1020

taatagttat taacccattc aagtttcttc ctatttataa ccccaaatat gtcaaaatgt 1080

atgataacca ccaactggga aaacttgagc cccacattta tgctgtggct gatgtagctt 1140

atcatgccat gcttcagcgc aaaaagaatc agtgcatcgt gatttcagga gagagtggtt 1200

ctgggaagac tcaaagcaca aactttctta ttcaccacct tactgctctc agtcagaaag 1260

gatttgccag tggagtagaa cagattattc ttggagctgg accagtactt gaggcctttg 1320

gaaatgcaaa gacagctcat aataacaatt caagtcgttt tgggaagttt attcaagtaa 1380

attaccagga aacaggcact gtacttggtg cctatgttga aaaatatcta ctggagaagt 1440

ccagactcgt ttatcaggag cataatgaac ggaactatca tgtattctat tacctcctgg 1500

caggagcaag tgaagatgag agatcagcat tccatcttaa gcaaccagag gaatatcatt 1560

atctcaatca gataacaaag aaacccctca gacagagctg ggatgattat tgctatgact 1620

ctgagccgga ttgcttcacg gtggaaggag aagatttgag acatgacttt gagcgcctac 1680

aacttgccat ggaaatggta ggatttcttc ccaagacacg aagacagatt ttctctcttc 1740

tctcagccat actacatttg ggtaatatct gttacaaaaa gaagacatac cgggatgact 1800

ccattgatat ctgtaatcct gaagttctgc ctattgtctc agaattatta gaggttaaag 1860

aagagatgct atttgaagca ttagttacaa ggaagacggt gacagtggga gaaaagctta 1920

ttttgccata caagttggca gaggctgtga cagtgaggaa ctccatggct aagtctctgt 1980

atagtgccct gtttgactgg atagtttttc gaattaatca tgcacttctg aatagtaaag 2040

atttagagca taataccaag acattgtcta ttggtgttct tgatattttt gggtttgaag 2100

attatgaaaa taacagcttt gaacagttct gtattaattt tgctaatgaa cgtttacagc 2160

actactttaa tcagcatatc tttaaattgg aacaagagga atatagaact gaaggtatca 2220

gctggcacaa catagattac attgataata cctgctgcat aaatcttatt agcaaaaaac 2280

caacaggact gcttcatctt ttggatgaag aaagcaactt tccacaggct acaaatcaaa 2340

cattgctaga caagtttaag catcaacatg aagataattc ttacatcgaa tttccagccg 2400

tgatggagcc tgctttcatt ataaaacatt atgctggaaa agtaaaatat ggggtaaagg 2460

atttccggga aaaaaataca gatcatatgc gcccagacat tgtagctctt ctgagaagca 2520

gcaagaatgc atttatctct gggatgattg gaattgatcc tgtagctgtt ttccgatggg 2580

caattctccg agcttttttc agagccatgg ttgctttcag ggaagctggg aaaagaaaca 2640

ttcacagaaa aactggacat gatgatacag cgccatgtgc aattttgaaa agtatggata 2700

gttttagctt tctccaacac ccagtccacc agaggagctt agagattctg cagagatgca 2760

aggaagagaa gtacagtata acccggaaaa atcccagaac acctctttct gatctccagg 2820

gcatgaatgc tctaaatgaa aaaaaccaac atgatacatt tgatattgcc tggaatggca 2880

gaactgggat tcgccagagc agactatcaa gtggcacctc cttgcttgat aaagatggaa 2940

tatttgctaa ttcaactagc agcaaactcc tggagagagc ccatggaatt ctcacgagaa 3000

acaaaaattt caaatccaag cctgcccttc caaagcactt gctagaagta aattctttaa 3060

agcacctgac aagactgaca ctacaagatc gcattaccaa gtctcttctt catttacaca 3120

agaagaaaaa acctcccagc atcagtgccc agtttcaggc atcattaagc aagctaatgg 3180

aaacacttgg tcaagcagaa ccatattttg taaaatgcat tcgctctaat gctgaaaagc 3240

tgcccttaag gttcagtgat gtcttggtac ttagacagct tcgatacacc gggatgctgg 3300

aaacagttcg aattcgccaa tcaggataca gctccaaata ttctttccag gattttgtga 3360

gccacttcca tgtacttctt ccccgaaata ttattccatc caaatttaac attcaggatt 3420

tcttcaggaa aataaatctt aatccagata attatcaagt tggaaaaacc atggtctttc 3480

taaaggagca ggaacgacag cacttacaag atctgcttca ccaagaggtg ctccgcagaa 3540

tcatattgtt gcagcgatgg ttcagggtct tgctgtgtag gcagcatttc ctccatctga 3600

gacaagcatc tgttattatc cagagattct ggaggaatta cctaaatcag aagcaagtca 3660

gagatgcagc tgtgcagaag gatgcttttg ttatggctag tgcagctgct cttctccaag 3720

cttcctggcg tgctcactta gagaggcagc ggtacttgga gttacgggct gcagccatcg 3780

ttatccagca gaaatggaga gattactata ggcgcaggca catggctgct atttgcatac 3840

aagcaagatg gaaagcctac agggaaagta aaaggtacca agaacaaagg aaaaaaatta 3900

tccttttgca atcaacatgt agaggattca gagcaagaca aagatttaaa gctttaaaag 3960

aacaaaggct aagagaaaca aagccagaag ttggattggt gaatattaag ggatatggat 4020

ctctggaaat tcagggttca gacccttcag gatgggagga ttgttctttt gacaacagaa 4080

taaaagccat agaggaatgt aaatctgtaa tagagagtaa tcgaattagc cgtgaaagtt 4140

cagtggactg cttgaaggag tcaccaaaca agcagcagga gagagcccaa agccagagtg 4200

gtgtggactt gcaggaagat gtgcttgtaa gagagagacc cagatccttg gaggatctcc 4260

atcagaaaaa agtaggccgg gctaagagag aaagtaggag aatgagagaa ctagagcaag 4320

ctatatttag cttagaattg ctgaaagttc gttctcttgg tggtatttct ccttcagagg 4380

atcgcagatg gtctacagaa ttggtgcctg aaggccttca gtctccacgg ggtacacctg 4440

atagtgagag ctctcaagga agcttggaac ttctgagcta tgaggaaagc caaaagagca 4500

aactagagtc tgtcatttca gatgaaggag acttgcagtt tccatcacct aagatatcca 4560

gcagtccaaa atttgattca cgggacaatg ccctcagtgc ctcaaatgag actagcagtg 4620

cagagcattt gaaggatgga actatgaagg aaatggtggt ctgcagttct gagtctatta 4680

cctgtaaacc acagctgaaa gactccttca tttcaaatag tctacctact tttttttata 4740

tcccccaaca agacccactg aaaacaaatt cccaactaga cacaagtatc caaagaaaca 4800

aactattgga aaatgaagac acagcggggg aagctcttac tttggatatc aacagggaaa 4860

ctagaaggta tcactgctca ggaaaagatc agattgttcc ttctttgaat acagagtctt 4920

ctaatcctgt gcttaagaag ttagaaaagc taaacactga gaaggaagaa aggcaaaaac 4980

agttgcagca acagaatgaa aaagagatga tggaacagat tcgccagcaa acagatattt 5040

tagagaagga gcgcaaagcc ttcaagacaa ttgaaaagcc aagaattgga gagtgtttgg 5100

tggcaccatc ttcctatcag tcaaagcaaa gagtagagag gccatcctct ctcctcagct 5160

taaatacctc aaataaggga gaacttaatg tactggggtc cctatcatta aaagatgcag 5220

ctcttgccca aaaagacagt tcctctgctc acttaccccc aaaggaccga cctgtcaccg 5280

tgttctttga aagaaaagga agtccatgcc aatctagtac tgtcaaggaa ttatccaaga 5340

cagacagaat gggcacccag ctgaatgtag cctgtaaact ctcaaataat cgcatttcaa 5400

aaagagaaca ctttaggcca actcagtctt acagccacaa ttctgatgac ctttccagag 5460

agggaaatgc taggcccatt ttcttcactc caaaggacaa tatgagtatt ccccttgtca 5520

gcaaagaagc cttaaacagt aaaaatcctc aactccataa agaagatgaa ccagcatgga 5580

aacctgtgaa gttagctggg ccaggccaaa gagagacatc acagcgattt tcgtcagttg 5640

atgaacaagc aaaacttcat aagactatgt ctcaaggaga gattaccaag ttggcagtga 5700

gacagaaggc ttcagattca gatataagac ctcagagagc taagatgaga ttctgggcca 5760

aagggaaaca aggggagaag aagactacca gagtgaaacc tactacccag tcagaggttt 5820

cgccactctt tgcaggcaca gatgtgattc cagctcatca gtttccagat gaattagctg 5880

catatcaccc aacacctcct ttgagcccag aactgcccgg cagttgccgg aaggaattca 5940

aagagaacaa agaaccttct ccaaaggcta agcgcaagcg aagtgtgaag attagcaacg 6000

tggctttgga ttctatgcat tggcaaaatg actctgtcca gatcatagca agtgtcagtg 6060

atttaaaaag catggatgaa tttcttctga aaaaggtgaa tgacctagat aatgaagaca 6120

gcaagaagga tacactagtg gatgttgtat ttaaaaaagc cctgaaggaa tttcggcaga 6180

atatcttcag cttttattca tctgcattgg cgatggatga tgggaaaagc atacggtata 6240

aagacctcta tgcactattt gaacagattc tggaaaagac gatgaggctt gagcagcgtg 6300

attcactggg tgaatctcca gtgagagttt gggtcaacac ttttaaagtg tttttagatg 6360

aatatatgaa tgaattcaag acttcagatt gcacagccac aaaggtgcca aaaacagaaa 6420

gaaagaaaag aaggaaaaag gaaactgatt tggtggaaga acacaatggt cacatcttta 6480

aagccaccca atatagcatc cctacatact gtgaatactg ttcttctttg atatggataa 6540

tggaccgagc ctctgtttgc aaattatgca agtatgcttg ccataagaag tgctgtctga 6600

aaaccacagc caagtgctct aaaaagtatg atccagagct gtcatctcga caatttgggg 6660

ttgaactgtc ccgtttgacc agtgaagacc gaactgttcc tttagtagtg gaaaagctca 6720

taaactacat tgaaatgcat ggactgtata cagaaggtat ttatcgaaag tctggttcga 6780

ctaataaaat caaggagctt cggcagggtc tagatacaga tgctgagagt gtaaatctag 6840

atgactataa catacacgtc attgcaagtg tattcaaaca atggcttcga gatttgccca 6900

atcctctcat gacctttgaa ctctatgagg aatttcttcg agctatgggc cttcaggaga 6960

ggaaggagac aatccgtggt gtatactctg tgattgatca actctcccga actcatctca 7020

atacactgga acgcctcatc tttcatctag tcaggattgc tctgcaggaa gacactaatc 7080

gaatgtctgc taatgctttg gccattgtgt ttgcgccctg cattctccgc tgccctgaca 7140

ccactgaccc actacaaagt gtacaggaca tcagtaagac taccacttgt gtggaactga 7200

ttgttgtgga acaaatgaat aaatacaagg ctcgtctcaa agatatcagt agcttggaat 7260

ttgctgagaa taaggcaaag accaggttgt cactgattcg tagatcaatg ggaaaggggc 7320

gtattcgtcg aggaaactat ccaggtccat cgtctcctgt tgtagttcgg ttgccttctg 7380

tgtctgatgt ctcagaggag accttgacta gtgaggcagc catggagact gacatcacag 7440

aacagcagca agcagctatg cagcaggagg agagagtact gactgagcag attgagaacc 7500

tacagaagga gaaggaggag ctaacatttg agatgcttgt actggaaccc cgtgcctctg 7560

atgatgaaac ccttgagtct gaggcctcca ttgggactgc tgatagctca gagaatttga 7620

atatggagtc tgaatatgct atctctgaga aatcagaaag aagcttagcc cttagctccc 7680

tgaagacagc tggcaagtct gaaccttcca gcaagttgcg aaagcaactt aaaaagcagc 7740

aagactcttt agatgtcgtg gactcttcgg tctcctcttt atgtctgtct aacacggcat 7800

catctcatgg gaccagaaaa ctatttcaga tttattccaa atctccattc taccgagctg 7860

cctcaggtaa tgaggccctg ggaatggaag gaccattggg ccagaccaaa ttcctggaag 7920

acaagcctca gttcatcagc agaggaacct tcaacccgga aaggggcaaa caaaaattaa 7980

agaatgtgaa aaactcacct cagaaaacca aagagacccc agaggggaca gtcatgtctg 8040

gccgcagaaa aactgtggac ccagactgca cctccaacca acagctagca ctctttggaa 8100

ataatgaatt tatggtctga accggcagat gtgtgtccct ccgtggctac agagtggtaa 8160

acaaatctca cctttggggc tgcgtttcat cacctcgtcc acaatagtca atcctaattg 8220

tggtcctgcc tcttttctaa gcatatggct aagactgtat gtgctgaatt cctgggcctc 8280

ctgcagaagc agaaagcctg ctggggatgg tgccagctgt gccttggctg ttgtatttga 8340

attgagattt ttactataca aagccaccta gggcctgggg atttgggtca gttgtagttg 8400

cctctccccc accctctttt cccttcccaa aggtgggtgt tgaactaggg gggatattgc 8460

tgtcctgagg gaccctctca tttctgacat ttgaagaaaa cgtataaatc tttcttaacc 8520

gtgaaagcaa aagcctttgg gtttattttg ggatagttag gagctagggt agaatataat 8580

ttttttccaa aaacttactt acaaacaaaa agcctaatcc ctctatttta agatttctga 8640

aaaaacactc catgttatat tctggggaaa gcaaaaacaa aaaaaaaaaa aaaaaaaaaa 8700

aaaa 8704

<210> 152
<211> 6416
<212> DNA
<213> Homo sapiens

<400> 152
gggtatcctg gtctccgcgt gtgtgagtac cgcggcgcga ggttagtggt agctgggtgc 60

agacgccgtg gcgctggaga cccaaacatg gcaaacctgg aagaaagctt cccccgagga 120

ggtacaagaa agatccacaa accagagaaa gctttccagc agtcagttga acaagacaac 180

ttatttgata tttctactga agagggatcc accaaaagaa aaaagagcca gaaggggcca 240

gcaaaaacaa aaaagttgaa aatcgaaaag agagaaagca gcaagtccgc aagagagaag 300

tttgaaatcc ttagtgttga gtccctgtgt gagggaatgc gtattttggg ttgcgtgaaa 360

gaggtgaatg aactggaact ggtgattagt ctccccaatg gcctccaggg ctttgtgcaa 420

gtcactgaaa tctgtgatgc ctacaccaaa aagctgaatg agcaggtgac acaagaacaa 480

cctctgaagg acctacttca cttgcctgaa cttttctcac ctggaatgct ggtaagatgt 540

gtggtgagca gtctgggcat cacagacagg ggcaagaaga gtgtcaagct gtctctgaac 600

cccaaaaatg tcaacagagt gctgagtgct gaggccctga agcctggcat gctacttaca 660

ggtaccgtat ccagcctgga agaccatggc tacctagtgg acattggtgt tgatgggacc 720

agagcttttc tgccactgct gaaagcccag gagtacatca gacagaagaa caaaggtgct 780

aaactaaagg tgggtcagta cctgaactgc attgttgaaa aggtgaaagg caacggagga 840

gttgttagtc tgtctgttgg tcactcagag gtttctacgg ccattgctac tgaacagcag 900

agctggaacc ttaataactt gctaccagga ctggtggtca aagctcaggt acagaaggtg 960

actccatttg gccttacgct aaacttcctc acattcttca cgggcgtggt tgactttatg 1020

cacctggatc ccaagaaagc tggaacatat ttctcaaatc aggcagtgag ggcctgcatc 1080

ctttgcgtcc atcctcgaac cagagttgtg cacctgagcc tgcgccccat cttcctacag 1140

cctggacgcc cactcacccg actctcttgc cagaaccttg gagcagtgct ggatgatgtt 1200

cctgtccagg gttttttcaa aaaggctggg gccaccttta ggctgaagga tggggttctg 1260

gcctatgccc ggctcagcca tctctctgat tctaagaacg tcttcaatcc tgaggccttc 1320

aagccaggga acactcacaa gtgtagaatt attgactaca gccaaatgga tgaactggcc 1380

ttgctctctc tacgaacgtc tattattgaa gctcagtacc ttagatatca tgacatcgaa 1440

cctggggcag tggtaaaggg cacagtgcta accataaagt catatgggat gctggtgaag 1500

gtgggcgagc agatgagggg cctggtacct cccatgcacc tggctgacat cctgatgaag 1560

aatccggaga agaagtacca catcggggat gaggtcaagt gccgggtttt gctttgtgac 1620

cctgaagcca agaagctgat gatgaccctg aaaaaaaccc tgattgagtc caaactacct 1680

gtcattacct gctatgccga tgccaagcct ggtctgcaga cacatggctt catcatcagg 1740

gtcaaggact atggctgcat tgtgaagttc tacaacaatg tgcagggact ggtgcccaag 1800

catgagctca gtactgagta tatccctgac ccggagagag ttttttacac tggccaggtg 1860

gtgaaggttg tcgtattgaa ctgtgagcca tccaaagaga ggatgctctt atccttcaag 1920

ctgtcgagtg atccagagcc aaagaaagag cctgcaggac acagtcagaa gaaaggaaaa 1980

gccattaaca ttgggcagtt ggtagatgtg aaggttttag agaagaccaa agatgggctg 2040

gaggtggctg tcctgcccca caacatccgt gctttcctcc ccacatctca tctgtcggac 2100

cacgttgcca acggcccatt gttacatcat tggctccagg caggtgacat ccttcaccga 2160

gtcctgtgtc tgagccagag cgaggggcgt gttcttcttt gcaggaagcc agccttggtc 2220

tccacagtag aaggtggcca ggatcccaag aacttctcag aaatccatcc tggaatgctg 2280

ctcattggtt ttgtgaagag catcaaggac tatggcgtgt tcatccagtt cccctcaggt 2340

cttagcggac tggccccaaa agctatcatg agtgacaaat ttgtgacctc cacaagtgac 2400

cactttgttg agggccagac agtagcggca aaggtgacca atgtggatga ggagaagcag 2460

cggatgctgc tgtcactgcg gctgtcggac tgtggtctgg gggacttggc tatcaccagc 2520

ctcctcctcc tgaatcagtg cctggaggag ctgcagggcg tgcgcagcct tatgagcaac 2580

cgagactctg tgttgatcca gacgctggcc gagatgaccc caggaatgtt ccttgaccta 2640

gtggtgcagg aggtgttgga agatggctct gtggtattca gtgggggtcc agtgcccgac 2700

ctggtcctga aagccagcag ataccatcgc gcagggcagg aggtggaatc tgggcagaaa 2760

aagaaggttg ttatcttaaa tgttgatctt ttgaagttgg aagtgcacgt ttcccttcac 2820

caggacttgg tgaatagaaa agctagaaag ctgaggaaag gcagcgaaca ccaggcgatt 2880

gtgcagcact tggagaagtc ctttgccatt gcctccttgg tagagacggg ccacctggca 2940

gctttctccc tgacctctca cctcaacgac accttccgct ttgactcaga gaaattgcag 3000

gtgggacagg gtgtctccct aaccctcaag accacagaac caggagtgac tggccttctt 3060

ttggctgtgg aggggccggc tgccaagagg accatgaggc cgacccagaa ggactctgag 3120

acagttgatg aggatgaaga agtggatcca gctctgactg tagggaccat aaagaagcac 3180

accctctcca tcggggacat ggtcacaggg actgtcaagt ccattaagcc tacccatgtg 3240

gttgtgactc tggaagatgg cattattggc tgtatccatg cctcccacat tctagatgat 3300

gttccagagg gcacctctcc tactaccaag ctgaaggttg ggaagacggt cactgcccga 3360

gtgattggcg ggcgagacat gaagacattc aagtatctcc caataagtca ccccagattc 3420

gttcgaacca tcccggagct gagtgttcgg ccaagtgagc tggaggatgg ccacactgct 3480

cttaacactc actctgttag ccccatggag aagattaaac agtaccaggc cggccagact 3540

gttacttgct tcttaaagaa atacaatgtg gtgaagaaat ggcttgaggt ggagattgcc 3600

ccagacatcc gggggagaat tcccttattg ctcacttctc tgagcttcaa ggttctgaag 3660

catccagata agaagttccg ggttggccag gccctgaggg ccaccgttgt tggcccagat 3720

tcctccaaga ccctcttatg tctgtccctc acaggtcctc acaagcttga ggaaggggaa 3780

gtggccatgg gccgagtggt gaaggtgact cccaacgagg ggctgaccgt ctccttcccc 3840

tttgggaaga taggaacagt cagtatattt cacatgagtg actcctactc cgagacgccc 3900

ctggaagact tcgtccccca gaaggttgtc agatgttaca tcctgtccac tgcagacaac 3960

gtattgactt tgtcgctgcg atcatccaga acaaacccgg agacgaaaag caaagtagaa 4020

gatccagaga ttaactccat ccaggacatt aaggaagggc agcttctgag gggctatgta 4080

gggtccatcc agccacacgg tgtgttcttt cgccttggcc cctccgttgt gggtttggct 4140

cggtactccc atgtctccca gcacagcccg tccaagaaag ccctttataa caaacacctc 4200

cctgaaggga agctgctcac agccagggtc ctacgcctta accaccagaa gaacctggta 4260

gagctgtctt tcctccccgg agacactggg aagccagacg tgctttctgc ttccttggaa 4320

gggcaactta caaagcaaga ggagaggaaa acagaggctg aggagagaga ccaaaaaggg 4380

gaaaagaaaa atcagaaaag gaacgagaag aagaaccaga aggggcagga ggaggtggag 4440

atgcccagca aggagaagca acagccccag aagccacagg cgcagaagcg gggcgggcgg 4500

gagtgccggg agtctgggag tgagcaggaa agagtgagca agaagccaaa gaaagccggc 4560

ctgtcagagg aggacgacag ccttgtggac gtgtactatc gggagggaaa agaggaggca 4620

gaagagacga atgtgctgcc caaggagaag caaaccaagc cagcagaagc gccccggctg 4680

cagctgtctt caggcttcgc ttggaatgtg ggactagact ctctgacccc ggccttgcca 4740

cctctagcag agagctcaga cagcgaggag gatgagaagc cacaccaagc cacgataaag 4800

aaaagcaaga aagaaaggga gttggagaag cagaaggcag agaaggaact gtcccgcatt 4860

gaggaggcgc tgatggatcc tgggcggcag ccagagtccg cggatgattt tgaccgactg 4920

gtgctgagct cccccaacag ctccattctg tggctgcagt acatggcttt ccacctgcag 4980

gccacggaga tcgagaaggc ccgtgccgtg gctgagaggg cccttaagac catctccttc 5040

agagaggagc aggagaagct gaacgtgtgg gtggctctgc tgaacctgga gaacatgtac 5100

ggctctcagg agtccctgac caaggtcttt gagcgagccg tgcagtacaa cgagcctctc 5160

aaagtctttc tccacctggc tgacatctac gccaagtcag agaaattcca ggaagctggt 5220

gaactctaca accggatgct gaagcgtttc cggcaggaga aagctgtgtg gatcaaatac 5280

ggcgccttcc ttctgcggag gagccaggct gcagccagtc accgcgtgct gcagcgagcc 5340

ctggagtgcc tgcctagcaa ggagcatgtg gatgtcattg ccaagtttgc ccagcttgag 5400

tttcagctgg gggatgcaga gcgggccaaa gccatttttg agaacacgct gagcacctac 5460

ccaaagcgca cagatgtctg gtcggtctat atcgacatga ccatcaagca cggcagccag 5520

aaggacgtcc gggacatctt tgagcgggtc attcatctga gcttggcccc caagagaatg 5580

aagttcttct tcaagcgcta cctggactac gagaagcagc atggcactga gaaggatgtg 5640

caggcagtca aggccaaggc cctggagtat gtggaggcca agagctcagt gctagaggac 5700

tagtggcagg ctggctctgt gggacactgt caacaatggg ccagcccggc cccgcctcga 5760

gtgcctgggc actcggaaaa ctgttacctc aggactctat ttaaatgctg ctttttctgc 5820

agcacgcttg gggaaatcct gtcaggatga aaaggaagtt gagatttttt aaatccctct 5880

tcgcttgctt tattttcagt accaacttgt tatctttttc cttatctgag gctacctggg 5940

gattgtgggc agcaggcccc tggactccca gaaatgctga gggtccctct tccaggggag 6000

ttccctgggg agcaagagta gaggggctat tcccgagggt cctgtggtca gggtcctgct 6060

ttgtccctgg caggctgtca gtccagctgg agggggtcag taggctccag ggagtgggcc 6120

ctcccctccg atcttggagt ctctgggtgt tccttcagcc tcagcctcac tggtaccttc 6180

tgccctttag gggaggctat gagtctggga caggaggaca aattcagagc ccagaggggg 6240

aaatggagaa acctgggctg ctgaggaaag ccacgggtgt tggcgtgaac tgatgatgtc 6300

tcatcccacg cccactatgg ccacattacc taacctctcc atatctgggc tattcaaggt 6360

cttggaggcc agcacagacc cagctagttg ttgaagccag ttcttttttg tcttgg 6416

<210> 153
<211> 3137
<212> DNA
<213> Homo sapiens

<400> 153
agcaccgtct gggctgtgga agcggagggg gtggggacac tctggcccgg ttctcggtgg 60

tgcgggagcg ggcgggagca gcggccgctc tggtcggcgg acgtgctgcc gagtagtccc 120

ggaagcgaag cagcgatggc ggagagtccg actgaggagg cggcaacggc gggcgccggg 180

gcggcgggcc ccggggcgag cagcgttgct ggtgttgttg gcgttagcgg cagcggcggc 240

gggttcgggc cgcctttcct gccggatgtg tgggcggcgg cggcggcagc gggcggggcc 300

gggggcccgg ggagcggcct ggctccgctg cccgggctcc cgccctcagc cgctgcccac 360

ggggccgcgc tgcttagcca ctgggacccc acgctcagct ccgactggga cggcgagcgc 420

accgcgccgc agtgtctact ccggatcaag cgggatatca tgtccattta taaggagcct 480

cctccaggaa tgttcgttgt acctgatact gttgacatga ctaagattca tgcattgatc 540

acaggcccat ttgacactcc ttatgaaggg ggtttcttcc tgttcgtgtt tcggtgtccg 600

cccgactatc ccatccaccc acctcgggtc aaactgatga caacgggcaa taacacagtg 660

aggtttaacc ccaacttcta ccgcaatggg aaagtctgct tgagtattct aggtacatgg 720

actggacctg cctggagccc agcccagagc atctcctcag tgctcatctc tatccagtcc 780

ctgatgactg agaaccccta tcacaatgag cccggctttg aacaggagag acatccagga 840

gacagcaaaa actataatga atgtatccgg cacgagacca tcagagttgc agtctgtgac 900

atgatggaag gaaagtgtcc ctgtcctgaa cccctacgag gggtgatgga gaagtccttt 960

ctggagtatt acgacttcta cgaggtggcc tgcaaagatc gcctgcacct tcaaggccaa 1020

actatgcagg acccttttgg agagaagcgg ggccactttg actaccagtc cctcttgatg 1080

cgcctgggac tgatacgtca gaaagtgctg gagaggctcc ataatgagaa tgcagaaatg 1140

gactctgata gcagttcatc tgggacagag acagaccttc atgggagcct gagggtttag 1200

accctgctcc catctcccct tcccccactc aagagtccca gcagaatccc ttccccccac 1260

cccagggatg gagaggcact gtgtatctcc ctccagactc gaagtcatcc tgcaagatgg 1320

caagaaccaa gcaagctccg atcccagggt gtgggagtgg gggcctgttc ccggtctgac 1380

ctccttggca ctggagcatc tggggcttcg ttcatccatt catcccgtat caggggccaa 1440

ggtaccttta caggagcacc tagagcgagg gcctttggca aaaacaaaac aaccaacaca 1500

cctctccaca gggccagctc cttagggata agtggaagat ggaaattgca attccaagag 1560

ggagtgtgcc caaatgattt atggggatac ctggaaggga gcttggggtg ggggctgtct 1620

gtgacactta agcagtctgg gtggttgtct atttgtctgt cttcagtctt gaagcagggc 1680

ttcccaatgc ccttttcctc cctgccttcc ttcccccatt atttcccaca ggccagcata 1740

attttgtttt tcctaattta tagtcactgt tctagacaga ccaaagagaa ggaacagtgg 1800

tggagtctag gctgctgatc agtaagcttt acctagcacc tgagcacctt tctcccctcc 1860

cctctttcct cacccttttc tagatgtaag acagaaagta aatgtgactg ggacttaacc 1920

aaggtcttgg taaagcctgc atggcaccgt aagaagctga aaatactgtt tgttcccgca 1980

atcactgatt tgaaaagttc ccaacacagg cagctgctgt gtatatggga ttagagccac 2040

tacatagaat agtctcttac agattttcat aaatactagt cacaataagg gtatttttct 2100

tgggggtgga gtaaggggga gactgatgct agtccttgtt gtattttgtt gggctgtcct 2160

tgtgtatttt caccccagcc tgtagtcctc ctcacttcaa ccccagggat ttttggggag 2220

caagggtagc caatggcaga gggggttggg gctgggactc tggaggctcc tccccttctt 2280

tctcttcctt ccgcctcccc cgtgccccca gctgctcttg tcactgtctc tgatgggtat 2340

ttgcctggct ttgttgcttc tctatctgta tttagctgca gtgatccttt agctggttgg 2400

ctcagaaaaa aaaaaatgtg ctttaggtgc cctgtaatcc tgggcatcaa gggaatccat 2460

ccttcccctt tttgatatgt tctccccgta cttccagatt tattgttatg gctcccagtg 2520

ggtattggcg attcttgtga tgcagggcct cagtcagtgt ccagccatgc ataaggagag 2580

ggatagtgtg tacctgccct gccctctgct atgaaggtct ctgccttgtg gatcatggga 2640

ctccccttgg aggatctgtg caaagggggg ctgggcacaa aggagaatgt cctatttggg 2700

agggcaggaa gcaaaggaac tggacaggga ttggtgggct tggggaacgg aagtttatct 2760

tggataccct tgaagaggct gggtctcttc acatgaagat cgaaaaggga ccctgcttcc 2820

aatttccctc ttccattcct cgagctactc cagggcttag aagaatgctc ttggtctgtg 2880

ggtccagtgt tgtctgtcat ccatttaagt gttcccactt tcaagtgaca atcctctcct 2940

tggccctgcc atagggcaga gcatgtctgg catagcagcc tgacttttat gccctaatct 3000

tgagttgagg aaatatatgc acaggagtca aagagatgtc tttatatctg actgtatata 3060

aatgaagttt ttttgttttt tttgttttcc tttttggtgc aataaagttt gttttggcag 3120

aaggagaaaa aaaaaaa 3137

<210> 154
<211> 1961
<212> DNA
<213> Homo sapiens

<400> 154
atgaccgcaa acggcaccaa gccattcgtg agggatttgc cctcacgacc cacagacctc 60

cccgcagccc tgcctcccag cactggcata ctggggatca acatgagttt tggtggggat 120

ggacaaaatt ctgagaacaa taactccaca atggcgtcgg cctcggaggg tgaaatggag 180

tgtgggcagg agctgaagga ggaagggggc ccgtgcttgt tcccgggctc agacagttgg 240

caagaaaacc ccgaggagcc ctgttccaaa gcctcctgga ccgtccaaga atgcatctgt 300

gctctgagca caagccaagg cattcgtgtg gaggcccggg gcgccgggag cctggagctg 360

cttcttcgtc ttcccacacc ctcctgccca tgggccctgg aggcagacgg tgctgggaag 420

ctggtcagtg cccacatgtg ggtgtccctg cctgggcctt cccaagagga ggtggcccct 480

gagggagcta catcagaggt tttggtagat gctgctgtag acctcatatc cgatgaatgg 540

gaagctgcta atgccatacc cagcaagaga aggaagcagg atgcagcccc gcttgaggcc 600

gccagcgtgc cttctgcaga ctgtgagcag ggggtcggtt ccaggaccgc ggaggacgcc 660

aaaatccaag gatgcccagt cccagatatg aaacggcgtg gcattgccac cacgccactg 720

gagggtgacg gcctctccgc gcccaccgag gttggcgaca gccccctgca ggcccaggct 780

ttgggagagg caggagtggc cacaggaagt gaggctcaga gcagcccgca attccaggac 840

cacacggaag gggaggacca ggacgcttcc atcccctctg gggcagaggg cctgtcccag 900

gaggggaccg gtccccccac ctctgctggt gaaggccatt ctaggactga agatgctgcc 960

caggagctcc tgttgcctga gtcaaaagga ggcagctctg agcccgggac agaactgcag 1020

accaccgagc aacaggcagg ggcctcagcc tctatggcag ttgatgctgt agctgagcca 1080

gccaatgcag ttaaaggggc cgggaaggaa atgaaagaga agacccagag aatgaaacag 1140

ccaccagcaa ccactcctcc tttcaaaaca cactgccagg aagctgagac caagaccaag 1200

gacgagatgg ctgctgctga agaaaaagtc ggtaagaatg aacaagggga gcctgaagac 1260

ctcaagaagc cagaggggaa gaacagaagt gcagctgctg tgaaaaacga gaaggagcaa 1320

aaaaaccagg aagcagatgt ccaggaagtg aaggcaagca cgctgagccc gggtggagga 1380

gtcaccgtgt tcttccacgc catcatctct cttcatttcc cattcaatcc tgacctccat 1440

aaagtcttca tcagaggagg agaagaattt ggggagtcaa aatgggacag caatatctgt 1500

gagctgcact acaccagaga cttgggtcat gaccgcgttc ttgttgaagg cattgtctgc 1560

atttccaaga agcacctaga taaatacatt ccttacaagt acgtcattta taatggggaa 1620

tcttttgagt atgagttcat ttacaagcac cagcagaaga agggcgagta cgtcaaccgc 1680

tgtctgttca taaaatcttc acttctgggc tcaggaggta agtcgtggca gcaggctgtc 1740

tgctcacccc tccggagtgc aggtgccaat cccgcggtac tggacgcgtc tctctggaga 1800

gcacagactt tgcctgggca gtgaggtcct ctttgctctg cattgacccc gccccagcct 1860

ccaggttcag tgacactgac tctgggttgt gtctctggtt ggttaaagat gtgttggctt 1920

ctcctttgag ctcagtgtga cttacctact cacttgcagg a 1961

<210> 155
<211> 6675
<212> DNA
<213> Homo sapiens

<400> 155
tgctttgcga ggctgggtgt tgagtcgagc cgcgggaaag gcgcgtgtcg gcctctcact 60

ggcgcagcct gcactgccgc tgccgcctcg ccccgccctg ccctggcgtt gtctctggca 120

ctgtggcgga ctgaccacgg cccgggcatg ggctgcaagg gagacgcgag cggtgcgtgt 180

gccgcgggtg cgctgccggt gacaggtacc caggggtggc gtgaggaggc ctcgtggaca 240

aagagagggt ttggggcaca aggcgaccct gtgcccctgt cacattgagc ttccgcctct 300

accgtgactc agtttaccct ctggtgtcct atcccttccc ctattcgcga gcgccccgag 360

ttgccctcag tggtcttctt ggcaaggagt ttgctataaa atgggagttc tggttgtact 420

cactgttctg tggctgttct cctcagtaaa ggccgactca aaagccatta caacctctct 480

tacaacaaaa tggttttcca ctccattgtt gttagaagcc agtgagtttt tagcagaaga 540

cagtcaagag aaattttgga atttgtagaa agccagtcaa aatattggat catcagatca 600

tgacggtacc gattattcct actatcatgc aatattggag gctgcatttc agtttctgtc 660

acccctccag cagaatttgt ttaaattttg tctgtccctt cgttcttact cagctacaat 720

ccaagccttc cagcagatag cagctgatga acctccacca gaaggatgta attcgttttt 780

ttcagtgcat ggaaagaaga cttgtgaatc tgataccctt gaggctcttc tactgacagc 840

ctctgaaaga cccaaacctt tattgttcaa aggagatcac agatatccct cgtctaatcc 900

tgaaagccct gtggtgattt tctactctga gattggctct gaggaatttt ccaattttca 960

ccgccagctt atatcaaaaa gcaatgcagg caaaatcaat tatgtattca gacattatat 1020

atttaatccc aggaaggagc ctgtttacct ctctggctat ggcgtggaat tggccattaa 1080

gagcactgag tacaaggcca aggatgatac tcaggtgaaa ggaactgagg taaacaccac 1140

agtgattggt gaaaatgatc ctattgatga ggttcagggg ttcctctttg gaaaattaag 1200

agatctgcac cccgacctgg agggacagtt gaaagaactc agaaagcatc ttgtagagag 1260

caccaatgaa atggcacctt taaaggtttg gcagttgcaa gatctcagtt tccagactgc 1320

tgctcgaatc ttggcttctc ctgttgagtt ggctttggtt gtcatgaagg atcttagtca 1380

gaattttcct accaaagcca gagcaataac aaaaacagct gtgagctcag aacttagaac 1440

cgaagtggaa gagaatcaga agtatttcaa gggaacttta ggattacaac ctggagattc 1500

agccctcttc atcaatggac ttcacatgga tttagataca caggatatat tcagtctgtt 1560

tgatgtgttg aggaatgaag ctcgggtaat ggagggtctg catagattgg gaatagaagg 1620

cctttctctg cataatgttt tgaagctgaa catccagccc tctgaggcag actatgccgt 1680

agacatccgg agtcctgcta tttcatgggt caacaacctg gaggttgata gcagatataa 1740

ttcgtggcct tctagtttac aagagttgct tcgacccacc tttcctggtg ttattcggca 1800

gatcaggaaa aacttacata atatggtttt catagttgat cctgcacatg agaccacagc 1860

agagttgatg aacacagctg agatgttcct tagtaatcat ataccactaa gaattggttt 1920

tatctttgtg gttaatgact ctgaagatgt tgatgggatg caagatgctg gagtggctgt 1980

tcttagagca tataattatg ttgcccaaga agtggatgat tatcatgcct tccagactct 2040

gacacatatc tataacaagg tgaggactgg agaaaaagtg aaagttgaac atgtggtcag 2100

tgtcctggag aagaaatatc cgtatgtaga agtgaatagc attttgggga ttgattctgc 2160

ttatgatcgg aatcggaagg aagcaagagg ctactatgag cagactggag ttggacctct 2220

gcccgttgtg ctgttcaatg gaatgccctt tgaaagggaa cagctagacc ctgatgagtt 2280

agaaaccatc acaatgcata aaatcctgga gaccaccacc ttcttccaaa gagcggtgta 2340

cttgggtgaa ctgccccatg atcaagatgt ggtagagtat atcatgaatc agccaaatgt 2400

tgttccacga atcaattcta ggattttgac agctgaacga gactacctgg atttaacagc 2460

gagtaataac ttctttgtgg atgattatgc tagatttact atcttggatt cccaaggcaa 2520

gactgctgct gtagccaata gtatgaacta tctgacaaag aaaggaatgt cctccaagga 2580

aatctatgat gattctttta ttaggccagt aactttttgg attgttgggg attttgatag 2640

cccttctgga cggcagttac tgtatgatgc catcaaacat cagaaatcca gtaacaatgt 2700

tagaataagc atgatcaata atcctgccaa agagataagc tatgagaaca ctcagatctc 2760

cagagcaatc tgggcagctc tccaaactca gacttccaac gctgctaaga acttcatcac 2820

caaaatggcc aaggaggggg ctgcagaggc cctggctgca ggagctgaca ttgcggagtt 2880

ctctgttggg ggaatggatt tcagtctttt taaagaggtc tttgagtctt ccaaaatgga 2940

tttcattttg tctcatgccg tgtactgcag ggatgttctg aagctgaaga agggacagag 3000

ggcagtgatc agcaatggaa ggatcattgg gccactggag gatagtgagc tctttaatca 3060

agacgatttc cacctcctcg aaaatatcat cttaaaaacc tcaggacaga aaataaaatc 3120

tcatattcaa cagcttcggg tagaagaaga tgtggcaagc gacttggtaa tgaaggtgga 3180

tgctcttctg tcagcgcaac caaaaggaga tccaagaatc gagtaccagt tttttgaaga 3240

cagacacagt gcaatcaaac tgaggccgaa ggaaggggag acatactttg atgttgtggc 3300

tgtcgttgac cctgtcacca gagaagcaca gagacttgct cctttgctct tggttttggc 3360

tcagctgata aacatgaatc tgagagtatt tatgaactgc caatccaaac tttctgacat 3420

gcctttaaaa agcttttacc gttatgtctt agaaccagag atttctttca cttcagacaa 3480

tagttttgct aagggtccaa tcgcaaaatt tttggatatg cctcagtctc cactgttcac 3540

tctgaatttg aacacacctg agagctggat ggtagaatct gtcagaacac catatgatct 3600

tgataatatt tatttagaag aggtggacag tgtagtggct gctgagtatg agctggaata 3660

cctgttactg gaaggtcatt gctacgacat caccacaggc cagcctccac ggggactaca 3720

gtttacctta ggaacttcag ccaacccggt cattgtggac accattgtta tggccaatct 3780

gggctacttt cagctgaaag ccaacccagg agcttggatc ctcagactta ggaagggacg 3840

ctctgaagat atttatagaa tttacagcca cgatggcact gattctcccc ctgatgctga 3900

tgaggtggtt atcgtcctca acaacttcaa aagcaaaatt attaaagtga aggttcagaa 3960

gaaggcagat atggtgaacg aagacttgct gagtgatgga acgagtgaga atgaatctgg 4020

attttgggat tccttcaaat ggggctttac aggacagaag actgaggaag tgaagcaaga 4080

taaagatgac ataattaata ttttctccgt tgcatctggt catctctacg aaagatttct 4140

tcgcataatg atgctatccg tgctgaagaa taccaagact cctgtgaaat tctggttctt 4200

gaagaattac ttgtccccca catttaagga gtttatacct tacatggcaa atgaatacaa 4260

tttccagtat gagcttgttc agtacaaatg gccccggtgg cttcatcaac aaactgaaaa 4320

acagcgtatc atctggggtt acaagatcct cttcctggat gtacttttcc cactagttgt 4380

tgacaagttc ctgtttgtgg atgctgatca gattgtacga acagatctga aagagttaag 4440

agatttcaat ttggatggtg ctccttatgg ttacactcct ttctgtgaca gccgaagaga 4500

aatggacggc tacaggttct ggaagtcagg gtactgggcc agtcatttag ccgggcgaaa 4560

gtatcatatc agtgcactat atgttgtgga tctgaagaag tttaggaaaa tagctgctgg 4620

tgacagactc aggggacagt accaaggtct gagtcaggac cctaacagcc tttcaaatct 4680

tgatcaagat ctgcccaata acatgattca tcaggtgcca attaaatccc tccctcaaga 4740

atggctttgg tgtgaaacgt ggtgtgatga cgcctctaag aaaagggcaa aaaccattga 4800

tttgtgtaat aatccgatga ccaaagagcc gaaactggaa gcagctgtgc ggattgtccc 4860

ggagtggcag gactacgacc aagagatcaa acagctacag atccgctttc agaaggagaa 4920

agaaacggga gcactgtaca aagagaagac aaaagaacca agccgagaag gtcctcagaa 4980

acgtgaagaa ttatgatctc tggagaagga caggaaatca ccccatttga aaaacagttt 5040

ttataataaa tgctagtttt ttctgatctg tctatacaac tgctgataag ccggctgggc 5100

aggagtgcca cacctttgga ttctgagcat ttgattctga cttctgtact ctggtggcca 5160

ctggatcttt gggattaaag ctctgttgga tttgtacctc agaggaagac caagtggctg 5220

atcctttgga ctctgtaaag agcattcttc tagtcagagg gtggaatggc agcagcaact 5280

ggaagaaaat gagtttttgg gtgcccacac ccaagagcac acacatgctg cactgtctcg 5340

gaaagcaggg ccagctagag ccaccatgtt cttccttacc tcagtttacc tgcggcctgc 5400

gctgcactgc agatgcccac cctgccctgg gtctggccgg cggaagctct gtccaaggtc 5460

cacacacctc caggtttacg ccaacatcct tgtgccctcc ccaccttctc ttccaacgca 5520

ttaggtgcat tgtttaattg aaatccaacc aacaattgtg tgtcaaggct ggtttggtgc 5580

agtggctggg caaattaatt ttgggccagg atgggggtgg gttgcagtga gggtagggaa 5640

aatgtcagga gtaggaaggt tcgggggtta agggaaggga aggaagacca gaactggcca 5700

tcctctttta taatccatta gtagcaccat ggctcatttg aaatgaaaat attacactta 5760

ttccccaccc aaccgcagtg aactttctag gtaattgttt tgaaaacaat ttttgtatct 5820

gtgaaagtct ttgctttttc tttccacctt ctagaaaagt ctgctaccag tttccttact 5880

gaatacagcc atactcagcc cctctcgcat ccagcccgtc agggtcaggg tcagggtcag 5940

gctccctcaa gacgagcacc gcattgtctg ccctcttttg cgtaggattt ttctctcaga 6000

cccaggggac attgccttgg acttttctct acaaatgccc tcagatgtaa gaccagaaat 6060

gattctgctt gtggaactcg tggctttctg tcctattttc cttttatgtg agccctgcag 6120

gttgcctttg gcgcccacac cagttctgtc ttcatgtcgc tgcatcgtga agccccattt 6180

gcctttagtc cctgctttgt gtgtcttcac ttccacagcc agtgttgccc ctcagggcat 6240

actttgctgg tgaacgggct gtagagtctt gagtatgtta ttggcctaag tgggcatgag 6300

gccccagcat ccgactccat ctgccaggct gcagggcaca gcagcactgg catagacagg 6360

cacgctctgt cttccaacgt gcaccagcct tttcttacat attttgcctg tttttaaaaa 6420

atacgaggag agggtgcaga tggatactgt gccctcaccc tcctaagcca ggtaatatat 6480

tgatgtgcag acttttaaga aatcttagtc ttatcacaaa tatcaagtaa tttatttttt 6540

ccattttcaa atgcaaaagt tagcctagga agcctcccag tcatcctgaa ggaccttttg 6600

actttgcttt tataattctg ttaaaaattg aggaggggat taatgagtac aatgaaaaca 6660

aaacaaaaca aaaaa 6675

<210> 156
<211> 3189
<212> DNA
<213> Homo sapiens

<400> 156
attgcgcctg cgcagggagc ccaaggcaag agccgctagg ctgccctgcc cgaagggctc 60

aactgtcagt gagcctgcgc aggaggccaa taggctgcca atactccttg gactccccgc 120

cagggccctg ctgtcagtgc gcctgcgcgc gggtccggcg ccgaggttct tgactgctgt 180

gccggacgcc aggtgtagcc atgcagcgag ccgattccga gcagccctcc aagcgtcccc 240

gttgcgatga cagcccgaga accccctcaa acaccccttc cgcagaggca gactggtccc 300

cgggcctgga actccatccc gactacaaga catggggtcc ggagcaggtg tgctccttcc 360

tcaggcgcgg tggctttgaa gagccggtgc tgctgaagaa catccgagaa aatgaaatca 420

caggcgcatt actgccttgt cttgatgagt ctcgttttga aaatcttgga gtaagttcct 480

tgggggagag gaagaagctg cttagttata tccagcgatt ggttcaaatc cacgttgata 540

caatgaaggt aattaatgat cctatccatg gccacattga gctccaccct ctcctcgtcc 600

gaatcattga tacacctcaa tttcaacgtc ttcgatacat caaacagctg ggaggtggtt 660

actatgtttt tccaggagct tcacacaatc gatttgagca tagtctaggg gtggggtatc 720

tagcaggatg tctagttcac gcactgggtg aaaaacaacc agagctgcag ataagtgaac 780

gagatgttct ctgtgttcag attgctggac tttgtcatga tctcggtcat gggccatttt 840

ctcacatgtt tgatggacga tttattccac ttgctcgccc ggaggtgaaa tggacgcatg 900

aacaaggctc agttatgatg tttgagcacc ttattaattc taatggaatt aagcctgtca 960

tggaacaata tggtctcatc cctgaagaag atatttgctt tataaaggaa caaattgtag 1020

gaccacttga atcacctgtc gaagattcat tgtggccata taaagggcgt cctgaaaaca 1080

aaagcttcct ttatgagata gtatctaata aaagaaatgg cattgatgtg gacaaatggg 1140

attattttgc cagggactgc catcatcttg gaatccaaaa taattttgat tacaagcgct 1200

ttattaagtt tgcccgtgtc tgtgaagtag acaatgagtt gcgtatttgt gctagagata 1260

aggaagttgg aaatctgtat gacatgttcc acactcgcaa ctctttacac cgtagagctt 1320

atcaacacaa agttggcaac attattgata caatgattac agatgctttc ctcaaagcag 1380

atgactacat agagattaca ggtgctggag gaaaaaagta tcgcatttct acagcaattg 1440

acgacatgga agcctatact aagctgacag ataacatttt tctggagatt ttatactcta 1500

ctgatcccaa attgaaagac gcacgagaga ttttaaaaca aattgaatac cgtaatctat 1560

tcaagtatgt gggtgagacg cagccaacag gacaaataaa gattaaaagg gaggactatg 1620

aatctcttcc aaaagaggtt gccagtgcta aacccaaagt attgctagac gtgaaactga 1680

aggctgaaga ttttatagtg gatgttatca acatggatta tggaatgcaa gaaaagaatc 1740

caattgatca tgttagcttc tattgtaaga ctgcccccaa cagagcaatc aggattacta 1800

aaaaccaggt ttcacaactt ctgccagaga aatttgcaga gcagctgatt cgagtatatt 1860

gtaagaaggt ggacagaaag agtttgtatg ccgcaagaca atattttgtt cagtggtgtg 1920

cagacagaaa tttcaccaag ccgcaggatg gcgatgttat agccccactc ataacacctc 1980

aaaaaaagga atggaacgac agtacttcag tccaaaatcc aactcgcctc cgagaagcat 2040

ccaaaagcag agtccagctt tttaaagatg acccaatgtg aatgtctgta gtcagttgtt 2100

tacaaactcc ctctcctgca caattcattt agaggcttca atcatagaat tctgcaaatt 2160

aatgacaact catgctttaa ttttgtattt tgaatgtaca cgcatgctga agctaagtaa 2220

cttttaatca aagaaataag atggtattag gcaaatctta ctatactatg aaaagcatta 2280

ccttgcctat ttttaatatt attaaagcct ttctccttca gtagtctatt ttcttagaat 2340

aacaactctt ttatctattc tgaactctat ttttttcttt ttttaagaga caaggttttg 2400

ctctgttgcc cagcttggac tcgaactttc ctgggctcaa gcgaccctcc tgcctcagcc 2460

ccccaagtag ctgggactaa agtcatgtgc caccacaccc agcttactct gaacttttat 2520

gacagatgat tgttttttgt ttttaatgta gaaatgagac aagggtacaa attggaacta 2580

gaaattgaca ttgtcattga caaacatggc taaaaacaaa acatcaaatc ctgcccccgt 2640

gaagagttcc ctgtcacctc aagtttgagg atagtcctct aagagtgacc taagcataag 2700

tgaaagacac ctcccctcac ccttctagcc ccctacaagg tgccaggttg gggtaaagag 2760

ttggagatga tggccaggag tggcctccaa cacgctggtg agaggcctga ttaggttttg 2820

gggaagatct gagagctctg gcctcttcgt gagtggaaca taaagccgcc tcttgttggg 2880

agatcctacc ccagtgacag aggaatcccc caaactaggc tgtgccctgg ctccgtggcg 2940

gctccagacc cgggtagtgc ctttgtcccc tgaatactca ctcccccggt ccagagggcc 3000

ttcccactgc ccagcctgga gaaggcctcc cctgacctgc tctctcagta tcctggagag 3060

ctggccagag gccatcacag gcatcatcct cagagctcct cagacctggg actttgtttt 3120

tgctggttca gtgcattttg tgtatttaag agcaaacact agccaggcgt ggcggcgtgt 3180

gcctgtagt 3189

<210> 157
<211> 5159
<212> DNA
<213> Homo sapiens

<400> 157
gagggcggag accaaggctg cgcaggcgtg gttggccggt cgttgcgttg ggatggatca 60

ggcggaattc cagaagagaa aataatccct gtctagatgc agaggacttg gttcctattc 120

cagtgctcct ccggtagcag ggaggcgaca gaaggtgtcc aactatccat taccaagaag 180

aaatctattc gtttgagcct gagacactct ttgaggtaaa aaattagaat gaaagaacct 240

ttggatggtg aatgtggcaa agcagtggta ccacagcagg agcttctgga caaaattaaa 300

gaagaaccag acaatgctca agagtatgga tgtgtccaac agccaaaaac tcaagaaagt 360

aaattgaaaa ttggtggtgt gtcttcagtt aatgagagac ctattgccca gcagttgaac 420

ccaggctttc agctttcttt tgcatcatct ggcccaagtg tgttgcttcc ttcagttcca 480

gctgttgcta ttaaggtttt ttgttctggt tgtaaaaaaa tgctttataa gggccaaact 540

gcatatcata agacaggatc tactcagctc ttctgctcca cacgatgcat caccagacat 600

tcttcacctg cctgcctgcc acctcctccc aagaaaacct gcacaaactg ctcgaaagac 660

attttaaatc ctaaggatgt gatcacaact cgctttgaga attcctatcc tagcaaagat 720

ttctgcagcc aatcatgctt gtcatcttat gagctaaaga aaaaacctgt tgttaccata 780

tataccaaaa gcatttcaac taagtgcagt atgtgtcaga agaatgctga tactcgattt 840

gaagttaaat atcaaaatgt ggtacatggt ctttgtagtg atgcctgttt ttcaaaattt 900

cactctacaa acaacctcac catgaactgt tgtgagaact gtgggagcta ttgctatagt 960

agctctggtc cttgccaatc ccagaaggtt tttagttcaa caagtgtcac ggcatacaag 1020

cagaattctg cccaaattcc tccatatgcc ctggggaagt cattgaggcc ctcagctgaa 1080

atgattgaga ctacaaatga ttcaggaaaa acagagcttt tctgctctat taattgctta 1140

tctgcttaca gagttaagac tgttacttct tcaggtgtcc aggtttcatg tcatagttgt 1200

aaaacctcag caatccctca gtatcaccta gccatgtcaa atggaactat atacagcttc 1260

tgcagctcca gttgtgtggt tgctttccag aatgtattta gcaagccaaa aggaacaaac 1320

tcttcggcgg tgcccctgtc tcagggccaa gtggttgtaa gcccgccctc ctccaggtca 1380

gcagtgtcaa taggaggagg taacacctct gccgtttccc ccagctccat ccgtggctct 1440

gctgcagcca gcctccaacc tcttgctgaa caatcccagc aagttgcttt aacccataca 1500

gttgttaaac tcaagtgtca gcactgtaac catctatttg ccacaaaacc agaacttctt 1560

ttttacaagg gtaaaatgtt tctgttttgt ggcaagaatt gctctgatga atacaagaag 1620

aaaaataaag ttgtggcaat gtgtgactac tgtaaactgc agaaaattat aaaggagact 1680

gtgcgattct caggggttga taagccattc tgtagtgaag tttgcaaatt cctctctgcc 1740

cgtgactttg gagaacgatg gggaaactac tgtaagatgt gcagctactg ttcacagaca 1800

tccccaaatt tggtagaaaa tcgattggag ggcaagttag aagagttttg ttgtgaagat 1860

tgtatgtcca aatttacagt tctgttttat cagatggcca agtgtgatgg ttgtaaacga 1920

cagggtaaac taagcgagtc cataaagtgg cgaggcaaca ttaaacattt ctgtaaccta 1980

ttttgtgtct tggagttttg tcatcagcaa attatgaatg actgtcttcc acaaaataaa 2040

gtaaatattt ctaaagcaaa aactgctgtg acggagctcc cttctgcaag gacagataca 2100

acaccagtta taaccagtgt gatgtcattg gcaaaaatac ctgctacctt atctacaggg 2160

aacactaaca gtgttttaaa aggtgcagtt actaaagagg cagcaaagat cattcaagat 2220

gaaagtacac aggaagatgc tatgaaattt ccatcttccc aatcttccca gccttccagg 2280

cttttaaaga acaaaggcat atcatgcaaa cccgtcacac agaccaaggc cacttcttgc 2340

aaaccacata cacagcacaa agaatgtcag acagatttac ctatgcctaa tgaaaaaaat 2400

gatgcagaac ttgattctcc accttcaaag aaaaaaagat taggtttttt ccagacttat 2460

gatacagaat atttaaaagt tggttttatt atctgtcctg gatcaaaaga aagttcacca 2520

aggccacagt gtgtcatttg tggagagatc ttatccagtg aaaacatgaa gccagcaaat 2580

ctttctcatc atttgaagac aaaacattca gaattagaaa acaaaccagt agattttttt 2640

gaacaaaaat ctttagaaat ggaatgtcaa aatagttctt taaaaaagtg tttactagtt 2700

gaaaagtcac ttgtgaaagc ttcttattta attgctttcc aaactgctgc aagcaagaag 2760

ccattctcca ttgctgaaga attaattaaa ccatatttag tagaaatgtg ttcagaagtt 2820

ttgggttcaa gtgctggaga caaaatgaaa actattccac tttctaatgt tacaattcaa 2880

cacaggattg atgaactatc tgcagacatt gaagaccagc tgattcaaaa ggtcagagag 2940

tcaaagtggt ttgcccttca gatagatgag tcatcagaaa tctcaaatat cacacttctt 3000

ttgtgctata ttcgtttcat tgattatgat tgtcgtgatg taaaagaaga attattattt 3060

tgcattgaaa tgcctactca aataactggc tttgaaatat ttgaactaat aaataaatat 3120

attgatagta aatctctgaa ttggaaacat tgtgttggtc tctgtaccga tggggctgca 3180

agcatgactg gcaggtattc tggtttaaaa gcaaaaattc aagaagttgc catgaataca 3240

gcggcattta cacattgttt tattcaccgt gaacgtttag tggcagaaaa gttgtctcca 3300

tgtttacata aaattctttt gcagtcagca caaattttaa gttttataaa gagcaatgca 3360

ttaaattcac gtatgttaac aattttgtgt gaagagatgg gatctgagca tgtgagttta 3420

ccgcttcatg ctgaagtacg ttggatatca agagggagaa tgttaaaaag attatttgaa 3480

ttacgacatg agattgaaat atttttaagt caaaagcatt cagatttggc caagtatttt 3540

catgatgagg aatgggttgg aaagctggcc tacttatcag atatattttc acttataaat 3600

gaattaaatt taagtctcca aggaactttg actactttct tcaatttgtg taataaaatt 3660

gatgtattta agagaaagtt aaaaatgtgg ttgaagcgca cacaagagaa tgattatgac 3720

atgttccctt catttctgaa attctcaaat tcatcaggct taaatatgac agacatcaca 3780

aggattattt ttgagcacct ggaaggactt tctcaagtgt tcagtgactg ttttccacca 3840

gaacaagact tgcgttcagg aaatttgtgg ataattcacc cttttatgaa tcaccaaaat 3900

aataatctca ccgacttcga agaagaaaag ctaacagagc tatcttcaga tttaggatta 3960

caagcactat ttaaatcagt gtctgtaact cagttttgga taaatgcaaa gacaagttac 4020

ccagaactcc atgaaagggc aatgaaattt ttattaccct tttcaactgt ttatttatgt 4080

gatgctgcct tttcagcttt gactgagtca aaacaaaaaa atctgttggg ttctggccct 4140

gccctaagac ttgcagtcac atctttaatt ccaaggatag aaaaattagt gaaggagaaa 4200

gagtagcaat atgcacattg cttaacagtg aagtcaataa tcctgtgtta agttttgtat 4260

aagtatccta aaagataatt tcctaatgtg gatttgtgtt ttcagtgatt aaatgtttta 4320

ataatttttc cctttttgtt gaggaattta ataattatga ttgttataaa taattatgta 4380

taattataat ctagggtaga aaatttagtt atttcattaa atttggacta gtgacaaaga 4440

ctgcaggtaa tgagaagccc agtttataat gtaacagcac aacctggatg ttgaaacagc 4500

ttggccttta gaagcaagtg gaacatttca gtcttctagc caaccagcta cttacccctg 4560

caaggttgtg agtaggtgga aaagatagat cttttttttg agatggagtt ttgctcttgt 4620

tgcccaggct ggagtgcaat ggcgtgatct tggctcactg gaacctctgc ctcctaggtt 4680

caaacaattc tcctgcctca gccttccaag tagctggggt tacaggcatg tgccaccatg 4740

cccgtctatt gtattttttt tagtagagac ggggtttcac catattggtc aggctggtct 4800

ctatctcctg acctcaagtg atccacctgc ctcggcctcc caaagtgctg ggattacaag 4860

tgtgagccaa cgtgcccagc tggaaaaaga tagatctaag cagcttgggt aactataaat 4920

ggaagcagca gggggatttg agacccctgc ttggggcctt tctggcccaa tataccaggg 4980

cttagcaaac tttttaaata aagagccaga tagtaaatat tttcagcttt gcaggccata 5040

ctgtcacaac tactcaattc tgcagttgca gtgcaaaagc agccatagac aatatgcaga 5100

taagtgtggc tgtttttaaa taaaacttat tcatgaacac tgaaaaaaaa aaaaaaaaa 5159

<210> 158
<211> 2625
<212> DNA
<213> Homo sapiens

<400> 158
gcggatgtct gtttcctggg atattgtgag ggcgggcgga gatgtagacc cggtagtgtt 60

gtgccttgtg gtgacaactg gcggcagcgc gccgcgggcc cgagacttag tctcgggccg 120

ccatggccag cgtccacgag agcctctact tcaatcccat gatgaccaat ggggttgtgc 180

acgccaatgt gttcggcatc aaggactggg tgacgccgta caagatcgcg gtgctggtgc 240

tgctgaacga gatgagccgc acaggcgagg gcgccgtcag cctcatggag cggcggaggc 300

tcaaccagct gctcctgccc ctgctgcagg gcccagatat tacactgtca aaactttaca 360

agttaattga agagtcttgt ccacagctgg caaattcagt gcagatcaga atcaaactga 420

tggctgaagg cgagttgaag gatatggaac agttttttga tgacctttca gattctttct 480

ctggaactga accagaggtt cacaaaacaa gtgtagtagg tttgtttctg cgtcacatga 540

tcttggccta cagtaagctt tctttcagcc aagtgtttaa actgtacact gcccttcagc 600

agtacttcca gaatggtgag aaaaagacag tggaggatgc tgatatggaa ctgaccagta 660

gagatgaggg tgaaagaaaa atggaaaaag aagaacttga tgtatctgta agagaagagg 720

aggtatcttg cagtgggcct ctgtcccaaa aacaagcaga atttttcctt tctcaacagg 780

cttctttgct aaagaatgat gagactaagg ccctcactcc agcttccttg cagaaggaat 840

taaacaattt gttgaaattt aatcctgatt ttgctgaagc gcattatctc agctacttaa 900

acaacctccg tgtccaagat gttttcagtt caacacacag tctcctccat tattttgatc 960

gtctgattct taccggagcc gaaagcaaaa gtaatgggga agagggctat ggccggagct 1020

tgagatacgc cgctctgaat cttgccgccc tgcactgccg cttcggtcac tatcaacagg 1080

cagagctcgc cctgcaggag gcaattagga ttgcccagga gtccaacgat cacgtgtgtc 1140

tccagcactg tttgagctgg ctttatgtgc tggggcagaa gagatccgat agctatgttc 1200

tgctggagca ttctgtgaag aaggcagtac attttgggtt accgtacctc gcctccctgg 1260

gaatacagtc ccttgttcaa cagagagctt ttgctgggaa gacggcaaac aagctgatgg 1320

atgccctaaa ggactccgac ctcctgcact ggaaacacag cctgtcagag ctcatcgata 1380

tcagcatcgc acagaaaacg gccatctgga ggctgtatgg ccgcagcacc atggcactgc 1440

aacaggccca gatgttgctg agcatgaaca gcctggaggc ggtgaatgcg ggcgtgcagc 1500

agaacaacac agagtccttt gctgtcgcac tctgccacct cgcagagcta cacgcggagc 1560

agggctgttt tgctgcagct tctgaagtgt taaagcactt gaaggaacga tttccgccta 1620

atagtcagca cgcccagtta tggatgctat gtgatcaaaa aatacagttt gacagagcaa 1680

tgaatgatgg caaatatcat ttggctgatt cacttgttac aggaatcaca gctctcaata 1740

gcatagaggg tgtttatagg aaagcggttg tattacaagc tcagaaccaa atgtcagagg 1800

cacataagct tttacaaaaa ttgttggttc attgtcagaa actgaagaac acagaaatgg 1860

tgatcagtgt cctactgtcc gtggcagagc tgtactggcg atcttcctcc cctaccatcg 1920

cgctgcccat gctcctgcag gctctggccc tctccaagga gtaccggtta cagtacttgg 1980

cctctgaaac agtgctgaac ttggcttttg cgcagctcat tcttggaatc ccagaacagg 2040

ccttaagtct tctccacatg gccatcgagc ccatcttggc tgacggggct atcctggaca 2100

aaggtcgtgc catgttctta gtggccaagt gccaggtggc ttcagcagct tcctacgatc 2160

agccgaagaa agcagaagct ctggaggctg ccatcgagaa cctcaatgaa gccaagaact 2220

attttgcaaa ggttgactgc aaagagcgca tcagggacgt cgtttacttc caggccagac 2280

tctaccatac cctggggaag acccaggaga ggaaccggtg tgcgatgctc ttccggcagc 2340

tgcatcagga gctgccctct catggggtac ccttgataaa ccatctctag agaggacatc 2400

cctgctgggc tgctgtgcag agtataagat tttggacttg ttcatgtccc ctctctccct 2460

ataaatgatg tatttgtgac accctatctt gtcaataaac agcattctga ttagtttgtc 2520

ttattttgtt gctagtaact acgtatttgt tttattcccc ttttcttccc ttttggtagc 2580

aaaggacacc aactttttct taataaatgg gtattttgct aacaa 2625

<210> 159
<211> 1351
<212> DNA
<213> Homo sapiens

<400>  159
agctccggct ccccctatat aaatcggcca tttgcttcgc tccgccccgc agcgccggag      60

tcaaagccgg ttcccggccc agtcccgtcc tgcagcagtc tgcctcctct ttcaacatga     120

cagatgccgc tgtgtccttc gccaaggact tcctggcagg tggagtggcc gcagccatct     180

ccaagacggc ggtagcgccc atcgagcggg tcaagctgct gctgcaggtg cagcatgcca     240

gcaagcagat cactgcagat aagcaataca aaggcattat agactgcgtg gtccgtattc     300

ccaaggagca gggagttctg tccttctggc gcggtaacct ggccaatgtc atcagatact     360

tccccaccca ggctcttaac ttcgccttca aagataaata caagcagatc ttcctgggtg     420

gtgtggacaa gagaacccag ttttggctct actttgcagg gaatctggca tcgggtggtg     480

ccgcaggggc cacatccctg tgttttgtgt accctcttga ttttgcccgt acccgtctag     540

cagctgatgt gggtaaagct ggagctgaaa gggaattccg aggcctcggt gactgcctgg     600

ttaagatcta caaatctgat gggattaagg gcctgtacca aggctttaac gtgtctgtgc     660

agggtattat catctaccga gccgcctact tcggtatcta tgacactgca aagggaatgc     720

ttccggatcc caagaacact cacatcgtca tcagctggat gatcgcacag actgtcactg     780

ctgttgccgg gttgacttcc tatccatttg acactgttcg ccgccgcatg atgatgcagt     840

cagggcgcaa aggaactgac atcatgtaca caggcacgct tgactgctgg cggaagattg     900

ctcgtgatga aggaggcaaa gcttttttca agggtgcatg gtccaatgtt ctcagaggca     960

tgggtggtgc ttttgtgctt gtcttgtatg atgaaatcaa gaagtacaca taagttattt    1020

cctaggattt ttccccctgt gaacaggcat gttgtattat ataacatatc ttgagcattc    1080

ttgacagact cctggctgtc agtttctcag tggcaactat ttactggttg aaaatgggaa    1140

gcaataatat tcatctgacc agttttctct taaagccatt tccatgatga tgatgatggg    1200

actcaattgt attttttatt tcagtcactc ctgataaata acaaatttgg agaaataaaa    1260

atatctaaaa taaattttgt ctgcagtata ttttcatata aaaatgcata tttgagtgct    1320

acattcgaat aaatactacc tttttagtga a                                   1351

<210>  160
<211>  4009
<212>  DNA
<213>  Homo sapiens

<400>  160
ggccggcggc cacgtgaccc accgcggcgg ggccggcggc ccagaaatag cagcggcggc      60

ggttccgccc gcgggcggcg cgagcgaggg gcagaggcga gagacgccgg cggggcgcgg     120

gcgcggcggc cccggaggat gctgctgagc cccggcactg cctggctgcg agcacatgat 180

ggcgatacgg gagctcaaag tgtgccttct cggggacact ggggttggga aatcaagcat 240

cgtgtgtcga tttgtccagg atcactttga ccacaacatc agccctacta ttggggcatc 300

ttttatgacc aaaactgtgc cttgtggaaa tgaacttcac aagttcctca tctgggacac 360

tgctggtcag gaacggtttc attcattggc tcccatgtac tatcgaggct cagctgcagc 420

tgttatcgtg tatgatatta ccaagcagga ttcattttat accttgaaga aatgggtcaa 480

ggagctgaaa gaacatggtc cagaaaacat tgtaatggcc atcgctggaa acaagtgcga 540

cctctcagat attagggagg ttcccctgaa ggatgctaag gaatacgctg aatccatagg 600

tgccatcgtg gttgagacaa gtgcaaaaaa tgctattaat atcgaagagc tctttcaagg 660

aatcagccgc cagatcccac ccttggaccc ccatgaaaat ggaaacaatg gaacaatcaa 720

agttgagaag ccaaccatgc aagccagccg ccggtgctgt tgacccaagg gccgtggtcc 780

acggtacttg aagaagccag agcccacatc ctgtgcactg ctgaaggacc ctacgctcgg 840

tggcctggca cctcactttg agaagagtga gcacactggc tttgcatcct ggaagacctg 900

cagggggcgg ggcaggaaat gtacctgaaa aggattttag aaaaccctgg gaaaacccac 960

cacaccacca caaaatggcc tttagtgtat gaaatgcaca tggaggggat gtagttgcat 1020

ttttgctaaa aaaaaaaaaa aacctttaaa aattgttgga tgtgtacaaa agtcttactg 1080

ccttattatg tgtatgggat tctaaagtgg cattccactt ggatttcctg tgctacctat 1140

ccaaattcca gtaactactt cagtgtcatt gcctttgtta cctaaccaac cttcactgaa 1200

aggcaaattt agttcaggag gttagttttt agctagcttt ggaagtaagc ctttatttat 1260

tactttttgg aggaaatcag agaagtgtca atggaccgtc actcagactg agacttgagt 1320

tattacagaa gccaggaaaa gtgtattaga aactgttgtc tacaccactt ttaattggtg 1380

aacaattttt ctaagttatg gtcatatata cccaaacaaa ccaaatcaaa ctaaattact 1440

gcatataatt ttgggattgg gtggcctagt ttgaaagagt gatttaagta atcactatgt 1500

aagtggtgag agatgcagga catacacatt attcaagaga ccacctgaca tgcatctcct 1560

ccgcaggaat acattcgtcc tctcttagag aagtttaacg cacatagtat tattttacta 1620

agagaatatc tcttggtgtc atatctaggg gaagagaatt aactagaatt aaatttaatg 1680

tttgaatcta aatcattggg caaacttcta ataataacaa ttaataatag gttacaggaa 1740

agccagccag aggaagtgtc agcactttaa aattctagac cccaaaaaac tacaaaatca 1800

gaaaaagtat ttttatgttt ctagcttgag gagaaggcct ttagggctaa ccagaggtct 1860

gaccctagaa tgccaaggaa ctgagaatgg gctccgatga aaaccttcct tttcagattc 1920

cctgtctgct caattaaaga tgtttgaatc caaaggaagt caaggaagaa aaagcatgga 1980

aaggaagaga actgattcct actgaaaatt caaattctat taccattcta actttcataa 2040

aaagttggga tcaagaagca gctgatttcc tgccagggct tatattaggg ggtgattctt 2100

aaaggacatt aggattggtg ctcagaaatg gttaatcatg ctgtgtgcta gccagggcca 2160

gctggtacct tctttgccat gagcattcaa gggacggcta acctttattg acaatctata 2220

tcgcaaaagt caggaaagag gttgtgagct gattggatta aagacctggc acttcagtaa 2280

ctcagcacgc ttccacttca ctcaacttaa gagagttcat tgacagtgtt aggatgtgaa 2340

ggctgggaaa cacttatttt gcttcaagag ttccacttgg ctctcccaaa taggtacctc 2400

aaaaactgtt agcaagcggc atttggatgt cttgacaggg gctttgcagg gatttttagg 2460

gttttttcca cattgtccac attaatggtt ggcatgattg tgcttgcagg ccaagaaatg 2520

atcatacccc ttgccaaagg taaaaaaaaa aaaaaaaaaa tgagttgaaa attgaagtga 2580

cctctttcca gctgagttgc aggcttattt tgtaaccttt cctcatccag ttttccctga 2640

gaacctgggt ttatctctag atagctgttc aggtttttta gctgaggggt aagtatccta 2700

gctgagagtt ttgcatcttt gggctgggtt tgcagtggtt gtgttttgca taaaatgtct 2760

agtctttgcc acagatagtg agctacccac taatgagccc atggttttat ttcagaagca 2820

catgagggtg tgaaaccact ctgttacctt tctgtattgt cttagctatt caagccagtc 2880

agaggataat atatatattc tcatcagcac tcagagtagt cagtgaagag agtagatcac 2940

acttgggcac accaggattc acataaacat tgtatcttct ctgtggatgc tcaggccttg 3000

tctacaatga ggctttacaa ccttcctttg ttttggctcg ggattacttc ctggctgtct 3060

aataattgaa ccataaccat gtaatattat gtaaaggcct ggaaattact gttgctaaaa 3120

aaagtcatgt agtttcatgt agtgtagcat ccttggcatc gttttccaaa atttgttcct 3180

tctccctttt tttttctttt cgtgtgtggc atgagtgtgt atctgtgtaa atatgattgt 3240

atatgtgtta ctccgatatg taatccattt cactggctga gtttggcccc tagccatgtg 3300

ttaatataaa gtaggcatgg cttcccaatg gaaatctctg agaatgacag tggagttgtg 3360

caagcatttt acattgccac ataattgact tgccatttta tggttaaaaa cggcacatta 3420

ggcagttgaa tatgacgtta ccttgcagac taaaaggttg aaggcccgaa actaactttt 3480

agctaacaat aagggctgtg ccccaatgga aactgagttc attttctgag aaaggtttgg 3540

atgactgaaa tatttcctct acagtcaagg actttggcat gtggtggctg aaactgagct 3600

tttttgtgtg ggctccagtt ctcactgttc tgcaatgctc atggcaagtt gaatggtgag 3660

ctagcttata aattaaagag ctctgaactg tattcagacc gactgggtat ctagcttact 3720

gttttaacat cattgttgaa accagaccct gtagtccagt ggtgctgccc tgttgtgcaa 3780

actgctcctt tttctcgtgt ttttgtaaag agcttccatc tgggctggac ccagttcttg 3840

cacatacaag acaccgctgc agtcagctag gacctttccg ccatgtattc tattctgtag 3900

taaagcattt ccatcaacaa tgcctaattg tatctgttat ttttggttta acacacactg 3960

attcatacta ataaatattt tcagttttac cgttaaaaaa aaaaaaaaa 4009

<210> 161
<211> 3233
<212> DNA
<213> Homo sapiens

<400> 161
ggctggagag gcgtgataat agaaccagtg cttcaacagc aacttctctt ttgcaatcct 60

tgctggtctg acaggctcct gcagaattac atcacttcct aaaaaacagg cacacacctc 120

tccttcaagt cagcaccgac ccaaacaagc aggaaacagg aaatgttcac gtttcagaga 180

ggctgcagcc cggcgcagca tcctgagcgc gcctctgccg aggcgagcgg acatgcaggc 240

tccccgcgca gccctagtct tcgccctggt gatcgcgctc gttcccgtcg gccggggtaa 300

ttatgaggaa ttagaaaact caggagatac aactgtggaa tctgaaagac caaataaagt 360

gactattcca agcacatttg ctgcagtgac catcaaagaa acattaaatg caaatataaa 420

ttctaccaac tttgctccgg atgaaaatca gttagagttt atactgatgg tgttaatccc 480

attgatttta ttggtcctct tacttttatc cgtggtattc cttgcaacat actataaaag 540

aaaaagaact aaacaagaac cttctagcca aggatctcag agtgctttac agacatatga 600

actgggaagt gaaaacgtga aagtccctat ttttgaggaa gatacaccct ctgttatgga 660

aattgaaatg gaagagcttg ataaatggat gaacagcatg aatagaaatg ccgactttga 720

atgtttacct accttgaagg aagagaagga atcaaatcac aacccaagtg acagtgaatc 780

ctaaacctga atggcgctca tgttttccaa gagaagcagc ccctgaggga gtctgctgag 840

gctgccaaca gaggatgaag aggatacaaa tttaattaat ttcaaatcaa catagacaca 900

agaacctttt gctgtttctt ccaacgccca ctcttcctaa tgatggcatc acttgcactt 960

ggaagaatgt gcaattgaga agtactagga aaaggcctgg ctgccatcca tcgctgcctc 1020

tgagggtgga gaaggaggcg ggtgatgtgc tcacttctga tcaacatgtg ttgcctcctc 1080

tcagccaact tctagctcac tgcactcact ctggtcatga taaatgttcg tcacctttct 1140

gcttcattcc ttagggccta aatcaggaag ctgttttatc gatggtttcc ttttgggtca 1200

gtaaccagct ttggataatt tcctctgatt attcaagtcg tgggacaggt aaactacatt 1260

cagcaggaac ttttctcgag gagtgttatg tcatggaaaa gacaccaaac acagcaagta 1320

ttttaatgaa tacaccatcc cagggggtca gtaagctctg cctgccaaga agacacagtg 1380

agaggggtcc acagtcctga tgaggtggcg tttggtaact tgtagaccct agcatggcca 1440

ggtctggtca cccttaagaa cttctcagag aaactaggaa tcttcagtga aagaactaat 1500

gttctcctca gctgaaattc ccttgcttgt cagcatttct gcaaagctca cacttgtttc 1560

accatacctc ccttggatgt gacatgtagg taggaagtat gtgcaggtgg gagtcatctg 1620

tcagccttct atgtttcaga gatcctgaag gtggtttgaa acaaacagaa gaggagcagg 1680

aaatatccgt gcctgtggca gatctcactc atcatgctta gcattctctc ccgccaagct 1740

gggataagcc tcatgtccta acacagcaca acaggaggtc tctgtcagtc catcagagat 1800

gacattctat gtgatatttt tgacatcctt gtgctaaaag caatggcaca aaatggaaaa 1860

gggcctattg accacaccta ctccagtaaa attgttcttc atttattcct taattttcta 1920

aatctgaccc ctttaaagca atctagcaaa ttgagaatcc tcagctctcc ttggatacct 1980

gatattttat ttcaagaaag agacaaagaa ggaaaatttt atttatttta ctacccacat 2040

ataaaccgaa gggagatggg actacccaaa catttgctgc tcaattttgt gtcttgtgct 2100

tgaaagtctg ccctaatgca taacaaaaac tacttgtctc ctaccttttg ggatcccttt 2160

aacaagtatt tgccttctga actacgtgga taatttcaaa ggcagagttc cagaccagag 2220

aggtctttcc atacaatgaa agctataact agctggttgt gtttaaccac tgctatcacg 2280

ctatcctggg actgcataga actttgacaa aagacagact tcatggtaca caacttcaac 2340

agattttctg tcatattctc accagcacat ctgaatgagg ctttgtgttt tccttgctct 2400

cttgcatgtt ccttttcaac tcatggccca cagtgactct cagagattta tgccaaaatt 2460

gcatacaatt gttttctgaa tcataacttg tctatttttc tgcctatgtg tgctactttc 2520

agtttgtttc tcatcaacat tttgactctc agaagagcct ccatttgccc ctttctctct 2580

ttaggtatct aagatctttg aacacctgga cctttacatt tgatccaacc ctatcaaata 2640

atgaacttct cagagaggca tctggggtcc tggaacttca tgttgatgaa gtcatattat 2700

ataatatgat aaaaatattc tcatgcagta ttttaaataa tttcaaattc tagaagaagc 2760

aaatttcagc gacatgtcat tgagttttta tttggtaaag ctataattgt gcagtgtaca 2820

aagcactttt taaaaagata gtttattctg tcagggtata tgaagttagt atacagccag 2880

aacagccaag cctcaattct tgtaccttgt gtctttttat tactgtttaa tcaatagata 2940

tcatatgttt atgacagttt caagaattgt ttttaaaccc aaacttaatt ttatgtttca 3000

gactattgtt agaaaaacaa aacaaaaaac aaaaaaaccc tcactaattt gccctaattg     3060

gatagggcaa tcagaacaaa atctgacttt cgaatattta aaagatatgt aagtttgatt     3120

gcattttcgt acattttaag caaactaggt taacaacaac atagcctagt caaacttctc     3180

aggaaacttg ttttaataaa tatgtaaaaa tacccaaaaa aaaaaaaaaa aaa            3233